# EUROPEAN PATENT APPLICATION

(11) **EP 1 777 235 A1**
(43) Date of publication of application: **25.04.2007**
(21) Application number: 06021944.1
(22) Date of filing: 29.06.2005
(51) Int. Cl.: C07K 16/24, A61K 39/395, A61P 19/02

(54) **Compositions and methods for treating inflammatory disorders**

(30) Priority: 30.06.2004 WO PCT/GB2004/002829; 24.08.2004 US 925366; 04.04.2005 US 98758
(62) Divisional of application: 05755389.3
(71) Applicant: Domantis Limited, Cambridge Cambridgeshire, CB4 OWG (GB)
(72) Inventor: Ignatovich, Olga, Cambridge CB4 0WG (GB); de Wildt, Rudolph, Cambridge CB4 0WG (GB); Woolven, Benjamin, Cambridge CB4 0WG (GB); Grant, Steven, Cambridge CB4 0WG (GB); Jones, Philip, Cambridge CB4 0WG (GB); Basran, Amrik, Cambridge CB4 0WG (GB); Brewis, Neil, Cambridge CB4 0WG (GB)
(74) Representative: Lock, Graham James

(57) **Abstract**

The invention relates to compositions and methods for treating inflammatory disorders. More specifically, the invention relates to antibody compositions and their use in the treatment of inflammatory disorders.

## Description

The present invention relates to methods of treatment of diseases, including rheumatoid arthritis using antibody polypeptide constructs including single domain antibody ligands and dual specific ligands, compositions of such ligands, and methods of making and using such ligands. In particular, the invention provides methods for the preparation of single domain antibodies that bind inflammatory cytokines including TNF-α and VEGF. Also disclosed are dual- specific ligands comprising a first single immunoglobulin variable domain which binds to a first antigen or epitope, and a second single immunoglobulin variable domain which binds to a second antigen or epitope. More particularly, the invention relates to dual-specific ligands wherein binding to at least one of the first and second antigens or epitopes acts to increases the half-life of the ligand in vivo. Open and closed conformation ligands comprising more than one binding specificity are described. Methods for treating rheumatoid arthritis are disclosed that use single domain antibody constructs and dual specific ligands that bind first and second antigens that can comprise any combination of, for example, TNF-α, VEGF and HSA.

### TNF-α:

As the name implies, Tumor Necrosis Factor-a (TNF-α) was originally described as a molecule having anti-tumor properties, but the molecule was subsequently found to play key roles in other processes, including a prominent role in mediating inflammation and autoimmune disorders. TNF-α is a key proinflammatory cytokine in inflammatory conditions including, for example, rheumatoid arthritis (RA), Crohn's disease, ulcerative colitis and other bowel disorders, psoriasis, toxic shock, graft versus host disease and multiple sclerosis.

The pro-inflammatory actions of TNF-α result in tissue injury, such as inducing procoagulant activity on vascular endothelial cells (Pober, et al., J. Immunol. 136:1680 (1986)), increasing the adherence of neutrophils and lymphocytes (Pober, et al., J. Immunol. 138:3319 (1987)), and stimulating the release of platelet activating factor from macrophages, neutrophils and vascular endothelial cells (Camussi, et al., J. Exp. Med. 166:1390 (1987)).

TNF-α is synthesized as a 26 kD transmembrane precursor protein with an intracellular tail that is cleaved by a TNF-α-converting metalloproteinase enzyme and then secreted as a 17 kD soluble protein. The active form consists of a homotrimer of the 17 kD monomers which interacts with two different cell surface receptors, p55 TNFRI and p75 TNFR2. There is also evidence that the cell surface bound precursor form of TNF-α can mediate some biological effects of the factor. Most cells express both p55 and p75 receptors which mediate different biological functions of the ligand. The p75 receptor is implicated in triggering lymphocyte proliferation, and the p55 receptor is implicated in TNF-mediated cytotoxicity, apoptosis, antiviral activity, fibroblast proliferation and NF-κB activation (see Locksley et al., 2001, Cell 104: 487-501).

The TNF receptors are members of a family of membrane proteins including the NGF receptor, Fas antigen, CD27, CD30, CD40, Ox40 and the receptor for the lymphotoxin α/β heterodimer. Binding of receptor by the homotrimer induces aggregation of receptors into small clusters of two or three molecules of either p55 or p75. TNF-α is produced primarily by activated macrophages and T lymphocytes, but also by neutrophils, endothelial cells, keratinocytes and fibroblasts during acute inflammatory reactions.

TNF-α is at the apex of the cascade of pro-inflammatory cytokines (Reviewed in Feldmann & Maini, 2001, Ann. Rev. Immunol. 19: 163). This cytokine induces the expression or release of additional proinflammatory cytokines, particularly IL-1 and IL-6 (see, for example, Rutgeerts et al., 2004, Gastroenterology 126: 1593-1610). Inhibition of TNF-α inhibits the production of inflammatory cytokines including IL-1, IL-6, IL-8 and GM-CSF (Brennan et al., 1989, Lancet 2: 244).

Because of its role in inflammation, TNF-α has emerged as an important inhibition target in efforts to reduce the symptoms of inflammatory disorders. Various approaches to inhibition of TNF-α for the clinical treatment of disease have been pursued, including particularly the use of soluble TNF-α receptors and antibodies specific for TNF-α. Commercial products approved for clinical use include, for example, the antibody products Remicade^{™} (Infliximab; Centocor, Malvern, PA; a chimeric monoclonal IgG antibody bearing human IgG4 constant and mouse variable regions), Humira^{™} (adalimumab or D2E7; Abbott Laboratories, described in U.S. patent No. 6,090,382) and the soluble receptor product Enbrel^{™} (etanercept, a soluble p75 TNFR2 Fc fusion protein; Immunex).

The role of TNF-α in inflammatory arthritis is reviewed in, for example, Li & Schwartz, 2003, Sringer Semin. Immunopathol. 25: 19-33. In RA, TNF-α is highly expressed in inflamed synovium, particularly at the cartilage-pannus junction (DiGiovine e tal., 1988, Ann. Rheum. Dis. 47: 768; Firestein et al., 1990, J. Immunol. 144: 3347; and Saxne et al., 1988, Atrhritis Rheum. 31: 1041). In addition to evidence that TNF-α increases the levels of inflammatory cytokines IL-1, IL-6, IL-8 and GM-CSF, TNF-α can alone trigger joint inflammation and proliferation of fibroblast-like synoviocytes (Gitter et al., 1989, Immunology 66: 196), induce collagenase, thereby triggering cartilage destruction (Dayer et al., 1985, J. Exp. Med. 162: 2163; Dayer et al., 1986, J. Clin. Invest. 77: 645), inhibit proteoglycan synthesis by articular chondrocytes (Saklatvala, 1986, Nature 322: 547; Saklatvala et al., 1985, J. Exp. Med. 162: 1208) and can stimulate osteoclastogenesis and bone resorption (Abu-Amer et al., 2000, J. Biol. Chem. 275: 27307; Bertolini et al., 1986, Nature 319: 516). TNF-α induces increased release of CD14+ monocytes by the bone marrow. Such monocytes can infiltrate joints and amplify the inflammatory response via the RANK (Receptor Activator or NF-κB)-RANKL signaling pathway, giving rise to osteoclast formation during arthritic inflammation (reviewed in Anandarajah & Richlin, 2004, Curr. Opin. Rheumatol. 16: 338-343).

TNF-α is an acute phase protein which increases vascular permeability through its induction of IL-8, thereby recruiting macrophage and neutrophils to a site of infection. Once present, activated macrophages continue to produce TNF-α, thereby maintaining and amplifying the inflammatory response.

Titration of TNF-α by the soluble receptor construct etanercept is effective for the treatment of RA, but not for treatment of Crohn's disease. In contrast, the antibody TNF-α antagonist infliximab is effective to treat both RA and Crohn's disease. Thus, the mere neutralization of soluble TNF-α is not the only mechanism involved in anti-TNF-based therapeutic efficacy. Rather, the blockade of other pro-inflammatory signals or molecules that are induced by TNF-α also plays a role (Rutgeerts et al., supra). For example, the administration of infliximab apparently decreases the expression of adhesion molecules, resulting in a decreased infiltration of neutrophils to sites of inflammation. Also, infliximab therapy results in the disappearance of inflammatory cells from previously inflamed bowel mucosa in Crohn's disease. This disappearance of activated T cells in the lamina propria is mediated by apoptosis of cells carrying membrane-bound TNF-α following activation of caspases 8, 9 and then 3 in a Fas dependent manner (see Lugering et al., 2001, Gastroenterology 121: 1145-1157). Thus, membrane- or receptor-bound TNF-α is an important target for anti-TNF-α therapeutic approaches. Others have shown that infliximab binds to activated peripheral blood cells and lamina propria cells and induces apoptosis through activation of caspase 3 (see Van den Brande et al., 2003, Gastroenterology 124: 1774-1785).

Intracellularly, the binding of trimeric TNF-α to its receptor triggers a cascade of signaling events, including displacement of inhibitory molecules such as SODD (silencer of death domains) and binding of the adaptor factors FADD, TRADD, TRAF2, c-IAP, RAIDD and TRIP plus the kinase RIP1 and certain caspases (reviewed by Chen & Goeddel, 2002, Science 296: 1634-1635, and by Muzio & Saccani in :Methods in Molecular Medicine: Tumor Necrosis Factor, Methods and Protocols," Corti and Ghezzi, eds. (Humana Press, New Jersey), pp. 81-99. The assembled signaling complex can activate either a cell survival pathway, through NF-κB activation and subsequent downstream gene activation, or an apoptotic pathway through caspase activation.

Similar extracellular downstream cytokine cascades and intracellular signal transduction pathways can be induced by TNF-α in other diseases. Thus, for other diseases or disorders in which the TNF-α molecule contributes to the pathology, inhibition of TNF-α presents an approach to treatment.

### VEGF:

Angiogenesis plays an important role in the active proliferation of inflammatory synovial tissue. RA synovial tissue, which is highly vascularized, invades the periarticular cartilage and bone tissue and leads to joint destruction.

Vascular endothelial growth factor (VEGF) is the most potent angiogenic cytokine known. VEGF is a secreted, heparin-binding, homodimeric glycoprotein existing in several alternate forms due to alternative splicing of its primary transcript (Leung et al., 1989, Science 246: 1306). VEGF is also known as vascular permeability factor (VPF) due to its ability to induce vascular leakage, a process important in inflammation. The identification of VEGF in synovial tissues of RA patients highlighted the potential role of VEGF in the pathology of RA (Fava et al., 1994, J. Exp. Med. 180: 341: 346; Nagashima et al., 1995, J. Rheumatol. 22: 1624-1630). A role for VEGF in the pathology of RA was solidified following studies in which anti-VEGF antibodies were administered in the murine collagen-induced arthritis (CIA) model. In these studies, VEGF expression in the joints increased upon induction of the disease, and the administration of anti-VEGF antisera blocked the development of arthritic disease and ameliorated established disease (Sone et al., 2001, Biochem. Biophys. Res. Comm. 281: 562-568; Lu et al., 2000, J. Immunol. 164: 5922-5927).

### Antibody Polypeptides:

Antibodies are highly specific for their binding targets and although they are derived from nature's own defense mechanisms, antibodies face several challenges when applied to the treatment of disease in human patients. Conventional antibodies are large multi-subunit protein molecules comprising at least four polypeptide chains. For example, human IgG has two heavy chains and two light chains that are disulfide bonded to form the functional antibody. The size of a conventional IgG is about 150 kD. Because of their relatively large size, complete antibodies (e.g., IgG, IgA, IgM, etc.) are limited in their therapeutic usefulness due to problems in, for example, tissue penetration. Considerable efforts have focused on identifying and producing smaller antibody fragments that retain antigen binding function and solubility.

The heavy and light polypeptide chains of antibodies comprise variable (V) regions that directly participate in antigen interactions, and constant (C) regions that provide structural support and function in non-antigen-specific interactions with immune effectors. The antigen binding domain of a conventional antibody is comprised of two separate domains: a heavy chain variable domain (V_{H}) and a light chain variable domain (V_{L}: which can be either V_{κ} or V_{λ}). The antigen binding site itself is formed by six polypeptide loops: three from the V_{H} domain (H1, H2 and H3) and three from the V_{L} domain (L1, L2 and L3). *In vivo,* a diverse primary repertoire of V genes that encode the V_{H} and V_{L} domains is produced by the combinatorial rearrangement of gene segments. C regions include the light chain C regions (referred to as C_{L} regions) and the heavy chain C regions (referred to as C_{H}1, C_{H}2 and C_{H}3 regions).

A number of smaller antigen binding fragments of naturally occurring antibodies have been identified following protease digestion. These include, for example, the "Fab fragment" (V_{L}-C_{L}-C_{H}1-V_{H}), "Fab' fragment" (a Fab with the heavy chain hinge region) and "F(ab')₂ fragment" (a dimer of Fab' fragments joined by the heavy chain hinge region). Recombinant methods have been used to generate even smaller antigen-binding fragments, referred to as "single chain Fv" (variable fragment) or "scFv," consisting of V_{L} and V_{H} joined by a synthetic peptide linker.

### Single Domain Antibodies:

While the antigen binding unit of a naturally-occurring antibody (e.g., in humans and most other mammals) is generally known to be comprised of a pair of V regions (V_{L}/V_{H}), camelid species express a large proportion of fully functional, highly specific antibodies that are devoid of light chain sequences. The camelid heavy chain antibodies are found as homodimers of a single heavy chain, dimerized via their constant regions. The variable domains of these camelid heavy chain antibodies are referred to as V_{H}H domains and retain the ability, when isolated as fragments of the V_{H} chain, to bind antigen with high specificity ((Hamers-Casterman et al., 1993, Nature 363: 446-448; Gahroudi et al., 1997, FEBS Lett. 414: 521-526). Antigen binding single V_{H} domains have also been identified from, for example, a library of murine V_{H} genes amplified from genomic DNA from the spleens of immunized mice and expressed in *E*. *coli* (Ward et al., 1989, Nature 341: 544-546). Ward et al. named the isolated single V_{H} domains "dAbs," for "domain antibodies." The term "dAb" will refer herein to a single immunoglobulin variable domain (V_{H}, V_{HH} or V_{L}) polypeptide that specifically binds antigen. A "dAb" binds antigen independently of other V domains; however, as the term is used herein, a "dAb" can be present in a homo- or heteromultimer with other V_{H} or V_{L} domains where the other domains are not required for antigen binding by the dAb, i.e., where the dAb binds antigen independently of the additional V_{H}, V_{HH} or V_{L} domains.

Single immunoglobulin variable domains, for example, V_{H}H, are the smallest antigen-binding antibody unit known. For use in therapy, human antibodies are preferred, primarily because they are not as likely to provoke an immune response when administered to a patient. As noted above, isolated non-camelid V_{H} domains tend to be relatively insoluble and are often poorly expressed. Comparisons of camelid V_{H}H with the V_{H} domains of human antibodies reveals several key differences in the framework regions of the camelid V_{H}H domain corresponding to the V_{H}/V_{L} interface of the human V_{H} domains. Mutation of these residues of human V_{H}3 to more closely resemble the V_{H}H sequence (specifically Gly 44→Glu, Leu 45→Arg and Trp 47→Gly) has been performed to produce "camelized" human V_{H} domains that retain antigen binding activity (Davies & Riechmann, 1994, FEBS Lett. 339: 285-290) yet have improved expression and solubility. (Variable domain amino acid numbering used herein is consistent with the Kabat numbering convention (Kabat et al., 1991, Sequences of Immunological Interest, 5th ed. U.S. Dept. Health & Human Services, Washington, D.C.)) WO 03/035694 (Muyldermans) reports that the Trp 103→Arg mutation improves the solubility of non-camelid V_{H} domains. Davies & Riechmann (1995, Biotechnology N.Y. 13: 475-479) also report production of a phage-displayed repertoire of camelized human V_{H} domains and selection of clones that bind hapten with affinities in the range of 100-400 nM, but clones selected for binding to protein antigen had weaker affinities.

The antigen binding domain of an antibody comprises two separate regions: a heavy chain variable domain (V_{H}) and a light chain variable domain (V_{L}: which can be either V_{κ} or V_{λ}). The antigen binding site itself is formed by six polypeptide loops: three from V_{H} domain (H1, H2 and H3) and three from V_{L} domain (L1, L2 and L3). A diverse primary repertoire of V genes that encode the V_{H} and V_{L} domains is produced by the combinatorial rearrangement of gene segments. The V_{H} gene is produced by the recombination of three gene segments, V_{H}, D and J_{H}. In humans, there are approximately 51 functional V_{H} segments (Cook and Tomlinson (1995) Immunol Today, 16: 237), 25 functional D segments (Corbett et al. (1997) J. Mol. Biol., 268: 69) and 6 functional J_{H} segments (Ravetch et al. (1981) Cell, 27: 583), depending on the haplotype. The V_{H} segment encodes the region of the polypeptide chain which forms the first and second antigen binding loops of the V_{H} domain (H 1 and H2), whilst the V_{H}, D and J_{H} segments combine to form the third antigen binding loop of the VH domain (H3). The V_{L} gene is produced by the recombination of only two gene segments, V_{L} and J_{L}. In humans, there are approximately 40 functional V_{κ} segments (Schable and Zachau (1993) Biol. Chem. Hoppe- Seyler, 374: 1001), 31 functional V_{λ} segments (Williams et al. (1996) J. Mol. so Biol., 264: 220; Kawasaki et al. (1997) Genome Res., 7: 250), 5 functional J_{κ} segments (Hieter et al. (1982) J. Biol. Chef,., 257: 1516) and 4 functional J_{λ} segments ( Vasicek and Leder (1990) J. Exp. Med., 172: 609), depending on the haplotype. The V_{L} segment encodes the region of the polypeptide chain which forms the first and second antigen binding loops of the V_{L} domain (L1 and L2), whilst the V_{L} and J_{L} segments combine to form the third antigen binding loop of the V_{L} domain (L3). Antibodies selected from this primary repertoire are believed to be sufficiently diverse to bind almost all antigens with at least moderate affinity. High affinity antibodies are produced by "affinity maturation" of the rearranged genes, in which point mutations are generated and selected by the immune system on the basis of improved binding.

Analysis of the structures and sequences of antibodies has shown that five of the six antigen binding loops (H1, H2, L1, L2, L3) possess a limited number of main-chain conformations or canonical structures (Chothia and Lesk (1987) d: Mol. Biol., 196: 901; Chothia et al. (1989) Nature, 342: 877). The main-chain conformations are determined by (i) the length of the antigen binding loop, and (ii) particular residues, or types of residue, at certain key position in the antigen binding loop and the antibody framework. Analysis of the loop lengths and key residues has enabled us to the predict the main-chain conformations of H1, H2, L1, L2 and L3 encoded by the majority of human antibody sequences (Chothia et al. (1992) J. Mol. Biol., 227: 799; Tomlinson et al. (1995) EMBO J., 14: 4628; Williams et al. (1996) J. Mol. Biol., 264: 220). Although the H3 region is much more diverse in terms of sequence, length and structure (due to the use of D segments), it also forms a limited number of main- chain conformations for short loop lengths which depend on the length and the presence of particular residues, or types of residue, at key positions in the loop and the antibody framework (Martin et al. (1996) J. Mol. Biol., 263: 800; Shirai et al. (1996) FEBS Letters, 399: 1.

### Bispecific Antibodies:

Bispecific antibodies comprising complementary pairs of V_{H} and V_{L} regions are known in the art. These bispecific antibodies must comprise two pairs of V_{H} and V_{L}s, each V_{H}/V_{L} pair binding to a single antigen or epitope. Methods described involve hybrid hybridomas (Milstein & Cuello, Nature 305:537-40), minibodies (Hu et al., (1996) Cancer Res 30 56:3055- 3061;), diabodies (Holliger et al., (1993) Proc. Natl. Acad. Sci. USA 90, 6444 6448; WO 94/13804), chelating recombinant antibodies (CRAbs; (Neri et al., (1995) J. Mol. Biol. 246, 367-373), biscFv (e.g. Atwell et al., (1996) Mol. Immunol. 33, 1301 1312), "knobs in holes" stabilized antibodies (Carter et al., (1997) Protein Sci. 6, 781 788). In each case, each antibody species comprises two antigen-binding sites, each fashioned by a complementary pair of V_{H} and V_{L} domains. Each antibody is thereby able to bind to two different antigens or epitopes at the same time, with the binding to EACH antigen or epitope mediated by a V_{H} and its complementary V_{L} domain. Each of these techniques presents its particular disadvantages; for instance in the case of hybrid hybridomas, inactive V_{H}/V_{L} pairs can greatly reduce the fraction of bispecific IgG.

Furthermore, most bispecific approaches rely on the association of the different V_{H}/V_{L} pairs or the association of V_{H} and V_{L} chains to recreate the two different V_{H}/V_{L} binding sites. It is therefore impossible to control the ratio of binding sites to each antigen or epitope in the assembled molecule and thus many of the assembled molecules will bind to one antigen or epitope but not the other. In some cases it has been possible to engineer the heavy or light chains at the sub-unit interfaces (Carter et al., 1997) in order to improve the number of molecules which have binding sites to both antigens or epitopes, but this never results in all molecules having binding to both antigens or epitopes. ,

There is some evidence that two different antibody binding specificities might be incorporated into the same binding site, but these generally represent two or more specificities that correspond to structurally related antigens or epitopes or to antibodies that are broadly cross- reactive. For example, cross-reactive antibodies have been so described, usually where the two antigens are related in sequence and structure, such as hen egg white lysozyme and turkey lysozyme (McCafferty et al., WO 92/01047) or to free hapten and to hapten conjugated to carrier (Griffiths AD et al. EMBO J 1994 13:14 3245-60). In a further example, WO 02/02773 (Abbott Laboratories) describes antibody molecules with "dual specificity". The antibody molecules referred to are antibodies raised or selected against multiple antigens, such that their specificity spans more than a single antigen. Each complementary V_{H}/N_{L} pair in the antibodies of WO 02/02773 specifies a single binding specificity for two or more structurally related antigens; the V_{H} and V_{L} domains in such complementary pairs do not each possess a separate specificity.

The antibodies thus have a broad single specificity which encompasses two antigens, which are structurally related. Furthermore natural autoantibodies have been described that are polyreactive (Casali & Notkins, Ann. Rev. Immunol. 7, 515-531), reacting with at least two (usually more) different antigens or epitopes that are not structurally related. It has also been shown that selections of random peptide repertoires using phage display technology on a monoclonal antibody will identify a range of peptide sequences that fit the antigen binding site. Some of the sequences are highly related, fitting a consensus sequence, whereas others are very different and have been termed mimotopes (Lane & Stephen, Current Opinion in Immunology, 1993, 5, 268-271). It is therefore clear that a natural four-chain antibody, comprising associated and complementary V_{H} and V_{L} domains, has the potential to bind to many different antigens from a large universe of known antigens. It is less clear how to create a binding site to two given antigens in the same antibody, particularly those which are not necessarily structurally related.

Protein engineering methods have been suggested that may have a bearing on this. For example, it has also been proposed that a catalytic antibody could be created with a binding activity to a metal ion through one variable domain, and to a hapten (substrate) through contacts with the metal ion and a complementary variable domain (Barbas et al., 5 1993 Proc. Natl. Acad. Sci USA 90, 6385-6389). However in this case, the binding and catalysis of the substrate (first antigen) is proposed to require the binding of the metal ion (second antigen). Thus the binding to the V_{H}/V_{L} pairing relates to a single but multi component antigen.

Methods have been described for the creation of bispecific antibodies from camel antibody heavy chain single domains in which binding contacts for one antigen are created in one variable domain, and for a second antigen in a second variable domain. However the variable domains were not complementary. Thus a first heavy chain variable domain is selected against a first antigen, and a second heavy chain variable domain against a second antigen, and then both domains are linked together on the same chain to give a bispecific antibody fragment (Conrath et al., J. Biol. Chem. 270, 27589-27594). However the camel heavy chain single domains are unusual in that they are derived from natural camel antibodies which have no light chains, and indeed the heavy chain single domains are unable to associate with camel light chains to form complementary V_{H} and V_{L} pairs.

Single heavy chain variable domains have also been described, derived from natural antibodies which are normally associated with light chains (from monoclonal antibodies or from repertoires of domains; see EP-A-0368684). These heavy chain variable domains have been shown to interact specifically with one or more related antigens but have not been combined with other heavy or light chain variable domains to create a ligand with a specificity for two or more different antigens. Furthermore, these single domains have been shown to have a very short in vivo half-life. Therefore, such domains are of limited therapeutic value.

It has been suggested to make bispecific antibody fragments by linking heavy chain variable domains of different specificity together (as described above). The disadvantage with this approach is that isolated antibody variable domains may have a hydrophobic interface that normally makes interactions with the light chain and is exposed to solvent and may be "sticky" allowing the single domain to bind to hydrophobic surfaces. Furthermore, in the absence of a partner light chain, the combination of two or more different heavy chain variable domains and their association, possibly via their hydrophobic interfaces, may prevent them from binding to one or both of the ligands they are able to bind in isolation. Moreover, in this case the heavy chain variable domains would not be associated with complementary light chain variable domains and thus may be less stable and readily unfold (Worn & Pluckthun, 1998 Biochemistry 37, 13120-7).

### SUMMARY OF THE INVENTION

The inventors have described, in their copending international patent application WO 03/002609 as well as in copending unpublished UK patent application 0230203.2, dual specific immunoglobulin ligands which comprise immunoglobulin single variable domains where each variable domain may have a different specificity. The domains may act in competition with each other or independently to bind antigens or epitopes on target molecules.

The present invention describes methods of treating a TNF-α-related inflammatory disorder in an individual suffering from such a disorder. The method comprises administering a therapeutically effective amount of a single domain antibody polypeptide construct, preferably a human single domain antibody construct, to such an individual, wherein the single domain antibody polypeptide construct binds human TNF-α, and whereby the TNF-α-related disorder is treated.

In one aspect, the inflammatory disorder is rheumatoid arthritis, and the method comprises the use of one or more single domain antibody polypeptide constructs, wherein one or more of the constructs antagonizes human TNFα's binding to a receptor. The present invention describes compositions comprising one or more single domain antibody polypeptide constructs that antagonize human TNFα's binding to a receptor, and dual specific ligands in which one specificity of the ligand is directed toward TNFα and a second specificity is directed to VEGF or HSA. The present invention further describes dual specific ligands in which one specificity of the ligand is directed toward VEGF and a second specificty is directed to HSA.

Also encompassed herein is the use of a polypeptide construct as described herein in the preparation of a medicament for the treatment of disease, particularly in the preparation of a medicament for the treatment of rheumatoid arthritis.

In one aspect, the invention encompasses a method of treating rheumatoid arthritis, the method comprising administering to an individual in need thereof a therapeutically effective amount of a composition comprising a single domain antibody polypeptide construct that antagonizes human TNFα's binding to a receptor, whereby the rheumatoid arthritis is treated.

In one embodiment, the composition prevents an increase in arthritic score when administered to a mouse of the Tg197 transgenic mouse model of arthritis.

In another embodiment, the administration of the composition to a Tg197 transgenic mouse comprises the following steps: a) administer weekly intraperitoneal injections of the composition to a heterozygous Tg197 transgenic mouse, b) weigh the mouse of step a) weekly, and c) score the mouse weekly for macrophenotypic signs of arthritis according to the following system: 0 = no arthritis (normal appearance and flexion), 1 = mild arthritis (joint distortion), 2 = moderate arthritis (swelling, joint deformation), 3 = heavy arthritis (severely impaired movement).

In another embodiment, the composition is administered to the mouse before the onset of arthritic symptoms is manifested. In another embodiment, the composition is first administered when the mouse is three weeks of age. In another embodiment, the composition is first administered when the mouse is six weeks of age.

In another embodiment, the composition has an efficacy in the Tg197 transgenic mouse arthritis assay that is greater or equal, within the realm of statistical significance, to that of an equivalent dose (on a mg/kg basis) of an agent selected from the group consisting of Etanercept, Infliximab and D2E7.

In another embodiment, the composition has an efficacy in the Tg197 transgenic mouse arthritis assay , such that the treatment results in an arthritic score of 0 to 0.5. In another embodiment, the composition has an efficacy in the Tg197 transgenic mouse arthritis assay , such that the treatment results in an arthritic score of 0 to 1.0. In another embodiment, the composition has an efficacy in the Tg197 transgenic mouse arthritis assay , such that the treatment results in an arthritic score of 0 to 1.5. In another embodiment, the composition has an efficacy in the Tg197 transgenic mouse arthritis assay , such that the treatment results in an arthritic score of 0 to 2.0.

In another embodiment, the treating comprises inhibiting the progression of the rheumatoid arthritis. In another embodiment, the treating comprises preventing or delaying the onset of rheumatoid arthritis.

In another embodiment, the administering results in a statistically significant change in one or more indicia of RA. In another embodiment, the one or more indicia of RA comprise one or more of erythrocyte sedimentation rate (ESR), Ritchie articular index and duration of morning stiffness, joint mobility, joint swelling, x ray imaging of one or more joints, and histopathological analysis of fixed sections of one or more joints.

In another embodiment, the one or more indicia of RA comprises a decrease in the macrophenotypic signs of arthritis in a Tg197 transgenic mouse, wherein the composition is administered to a Tg197 transgenic mouse, wherein the Tg197 transgenic mouse is scored for the macrophenotypic signs of arthritis, and wherein the macrophenotypic signs of arthritis are scored according to the following system: 0 = no arthritis (normal appearance and flexion), 1 = mild arthritis (joint distortion), 2 = moderate arthritis (swelling, joint deformation), 3 = heavy arthritis (severely impaired movement).

In another embodiment, the one or more indicia of RA comprises a decrease in the histopathological signs of arthritis in a Tg197 transgenic mouse, wherein the composition is administered to a Tg197 transgenic mouse, wherein the Tg197 transgenic mouse is scored for the histopathological signs of arthritis, and wherein the histopathological signs of arthritis are performed on a joint and scored using the following system: 0= no detectable pathology, 1= hyperplasia of the synovial membrane and presence of polymorphonuclear infiltrates, 2= pannus and fibrous tissue formation and focal subchondral bone erosion, 3= articular cartilage destruction and bone erosion, 4= extensive articular cartilage destruction and bone erosion.

In another embodiment, the single domain antibody polypeptide construct comprises a human single domain antibody polypeptide. In another embodiment, the human single domain antibody polypeptide binds TNFα. In another embodiment, the single domain antibody polypeptide construct binds human TNFα with a Kd of < 100 nM. In another embodiment, the single domain antibody polypeptide construct binds human TNFα with a K_{d} in the range of 100 nM to 50 pM. In another embodiment, the single domain antibody polypeptide construct binds human TNFα with a K_{d} of 30 nM to 50 pM. In another embodiment, the single domain antibody polypeptide construct binds human TNFα with a K_{d} of 10 nM to 50 pM. In another embodiment, the single domain antibody polypeptide construct binds human TNFα with a K_{d} in the range of 1 nM to 50 pM.

In another embodiment, the single domain antibody polypeptide construct antagonizes human TNFα as measured in a standard L929 cytotoxicity cell assay.

The invention further encompasses a method of treating rheumatoid arthritis, the method comprising administering to an individual in need thereof a therapeutically effective amount of a composition comprising a single domain antibody polypeptide construct that antagonizes human TNFα's binding to a receptor, wherein the single domain antibody polypeptide construct inhibits the binding of human TNFα to a TNFα receptor, and whereby the rheumatoid arthritis is treated.

In one embodiment, the single domain antibody polypeptide construct specifically binds to human TNF-α which is bound to a cell surface receptor.

In another embodiment, the single domain antibody polypeptide construct has an in vivo tα-half life in the range of 15 minutes to 12 hours. In another embodiment, the single domain antibody polypeptide construct has an in vivo tβ-half life in the range of 1 to 6 hours. In another embodiment, the single domain antibody polypeptide construct has an in vivo tβ-half life in the range of 2 to 5 hours. In another embodiment, the single domain antibody polypeptide construct has an in vivo tβ-half life in the range of 3 to 4 hours. In another embodiment, the single domain antibody polypeptide construct has an in vivo tβ-half life in the range of 12 to 60 hours. In another embodiment, the single domain antibody polypeptide construct has an in vivo tβ-half life in the range of 12 to 48 hours. In another embodiment, the single domain antibody polypeptide construct has an in vivo tβ-half life in the range of 12 to 26 hours.

In another embodiment, the single domain antibody polypeptide construct has an in vivo AUC half-life value of 15 mg.min/ml to 150 mg.min/ml. In another embodiment, the single domain antibody polypeptide construct has an in vivo AUC half-life value of 15 mg.min/ml to 100 mg.min/ml. In another embodiment, the single domain antibody polypeptide construct has an in vivo AUC half-life value of 15 mg.min/ml to 75 mg.min/ml. In another embodiment, the single domain antibody polypeptide construct has an in vivo AUC half-life value of 15 mg.min/ml to 50 mg.min/ml.

In another embodiment, the single domain antibody polypeptide construct is linked to a PEG molecule. In another embodiment, the PEG-linked single domain antibody polypeptide construct has a hydrodynamic size of at least 24 kDa, and wherein the total PEG size is from 20 to 60 kDa. In another embodiment, the PEG-linked single domain antibody polypeptide construct has a hydrodynamic size of at least 200 kDa and a total PEG size of from 20 to 60 kDa. In another embodiment, the PEGylated proteins of the invention may be linked, on average, to 1, 2, 3, 4, 5, 6, 7, 8, 9, 10, 12, 15, 17, 20, or more polyethylene glycol molecules.

In another embodiment, the antibody construct comprises two or more single immunoglobulin variable domain polypeptides that bind human TNFα. In another embodiment, the antibody construct comprises a homodimer of a single immunoglobulin variable domain polypeptide that binds human TNFα. In another embodiment, the antibody construct comprises a homotrimer of a single immunoglobulin variable domain polypeptide that binds human TNFα. In another embodiment, the antibody construct comprises a homotetramer of a single immunoglobulin variable domain polypeptide that binds human TNFα.

In another embodiment, the construct further comprises an antibody polypeptide specific for an antigen other than TNFα. In another embodiment, the antibody polypeptide specific for an antigen other than TNFα comprises a single domain antibody polypeptide. In another embodiment, the binding of the antigen other than TNFα by the antibody polypeptide specific for an antigen other than TNFα increases the in vivo half-life of the antibody polypeptide construct. In another embodiment, the antigen other than TNFα comprises a serum protein. In another embodiment, the serum protein is selected from the group consisting of fibrin, α-2 macroglobulin, serum albumin, fibrinogen A, fibrinogen, serum amyloid protein A, heptaglobin, protein, ubiquitin, uteroglobulin and β-2- microglobulin. In another embodiment, the antigen other than TNFα comprises HSA.

In another embodiment, the treating further comprises administration of at least one additional therapeutic agent.

In another embodiment, the single domain antibody polypeptide construct comprises the amino acid sequence of CDR3 of an antibody polypeptide selected from the group consisting of clones TAR1-2m-9, TAR1-2m-30,TAR1-2m-1,TAR1-2m-2, TAR1-5, TAR1-27, TAR1-261, TAR1-398, TAR1-701,TAR1-5-2, TAR1-5-3, TAR1-5-4, TAR1-5-7, TAR1-5-8, TAR1-5-10, TAR1-5-11, TAR1-5-12, TAR1-5-13, TAR1-5-19, TAR1-5-20, TAR1-5-21, TAR1-5-22, TAR1-5-23, TAR1-5-24, TAR1-5-25, TAR1-5-26, TAR1-5-27, TAR1-5-28, TAR1-5-29, TAR1-5-34, TAR1-5-35, TAR1-5-36, TAR1-5-464, TAR1-5-463, TAR1-5-460, TAR1-5-461, TAR1-5-479, TAR1-5-477, TAR1-5-478, TAR1-5-476, TAR 1-5-490, TAR1h-1, TAR1h-2, TAR1h-3, TAR1-100-29, TAR1-100-35, TAR1-100-43, TAR1-100-47, TAR1-100-52, TAR1-109, TAR1-100, TAR1-100-34, TAR1-100-36, TAR1-100-38, TAR1-100-39, TAR1-100-40, TAR1-100-41, TAR1-100-45, TAR1-100-60, TAR1-100-62, TAR1-100-64, TAR1-100-65, TAR1-100-75, TAR1-100-76, TAR1-100-77, TAR1-100-78, TAR1-100-80, TAR1-100-82, TAR1-100-83, TAR1-100-84, TAR1-100-89, TAR1-100-90, TAR1-100-91, TAR1-100-92, TAR1-100-93, TAR1-100-94, TAR1-100-95, TAR1-100-96, TAR1-100-97, TAR1-100-98, TAR1-100-99, TAR1-100-100, TAR1-100-101, TAR1-100-102, TAR1-100-103, TAR1-100-105, TAR1-100-106, TAR1-100-107, TAR1-100-108, TAR1-100-109, TAR1-100-110, TAR1-100-111, TAR1-100-112, TAR1-100-113 and TAR1-5-19.

In another embodiment, the single domain antibody polypeptide construct comprises the amino acid sequence of an antibody polypeptide selected from the group consisting of clones TAR1-2m-9, TAR1-2m-30, TAR1-2m-1, TAR1-2m-2, TAR1-5, TAR1-27, TAR1-261, TAR1-398, TAR1-701, TAR1-5-2, TAR1-5-3, TAR1-5-4, TAR1-5-7, TAR1-5-8, TAR1-5-10, TAR1-5-11, TAR1-5-12, TAR1-5-13, TAR1-5-19, TAR1-5-20, TAR1-5-21, TAR1-5-22, TAR1-5-23, TAR1-5-24, TAR1-5-25, TAR1-5-26, TAR1-5-27, TAR1-5-28, TAR1-5-29, TAR1-5-34, TAR1-5-35, TAR1-5-36, TAR1-5-464, TAR1-5-463, TAR1-5-460, TAR1-5-461, TAR1-5-479, TAR1-5-477, TAR1-5-478, TAR1-5-476, TAR1-5-490, TAR1h-1, TAR1h-2, TAR1h-3, TAR1-100-29, TAR1-100-35, TAR1-100-43, TAR1-100-47, TAR1-100-52, TAR1-109, TAR1-100, TAR1-100-34, TAR1-100-36, TAR1-100-38, TAR1-100-39, TAR1-100-40, TAR1-100-41, TAR1-100-45, TAR1-100-60, TAR1-100-62, TAR1-100-64, TAR1-100-65, TAR1-100-75, TAR1-100-76, TAR1-100-77, TAR1-100-78, TAR1-100-80, TAR1-100-82, TAR1-100-83, TAR1-100-84, TAR1-100-89, TAR1-100-90, TAR1-100-91, TAR1-100-92, TAR1-100-93, TAR1-100-94, TAR1-100-95, TAR1-100-96, TAR1-100-97, TAR1-100-98, TAR1-100-99, TAR1-100-100, TAR1-100-101, TAR1-100-102, TAR1-100-103, TAR1-100-105, TAR1-100-106, TAR1-100-107, TAR1-100-108, TAR1-100-109, TAR1-100-110, TAR1-100-111, TAR1-100-112, TAR1-100-113 and TAR1-5-19 or an amino acid sequence at least 85% identical thereto.

In another embodiment, the single domain antibody polypeptide construct comprises the amino acid sequence of an antibody polypeptide selected from the group consisting of clones TAR1-2m-9, TAR1-2m-30,TAR1-2m-1,TAR1-2m-2, TAR1-5, TAR1-27, TAR1-261, TAR1-398, TAR1-701,TAR1-5-2, TAR1-5-3, TAR1-5-4, TAR1-5-7, TAR1-5-8, TAR1-5-10, TAR1-5-11, TAR1-5-12, TAR1-5-13, TAR1-5-19, TAR1-5-20, TAR1-5-21, TAR1-5-22, TAR1-5-23, TAR1-5-24, TAR1-5-25, TAR1-5-26, TAR1-5-27, TAR1-5-28, TAR1-5-29, TAR1-5-34, TAR1-5-35, TAR1-5-36, TAR1-5-464, TAR1-5-463, TAR1-5-460, TAR1-5-461, TAR1-5-479, TAR1-5-477, TAR1-5-478, TAR1-5-476, TAR 1-5-490, TAR1h-1, TAR1h-2, TAR1h-3, TAR1-100-29, TAR1-100-35, TAR1-100-43, TAR1-100-47, TAR1-100-52, TAR1-109, TAR1-100, TAR1-100-34, TAR1-100-36, TAR1-100-38, TAR1-100-39, TAR1-100-40, TAR1-100-41, TAR1-100-45, TAR1-100-60, TAR1-100-62, TAR1-100-64, TAR1-100-65, TAR1-100-75, TAR1-100-76, TAR1-100-77, TAR1-100-78, TAR1-100-80, TAR1-100-82, TAR1-100-83, TAR1-100-84, TAR1-100-89, TAR1-100-90, TAR1-100-91, TAR1-100-92, TAR1-100-93, TAR1-100-94, TAR1-100-95, TAR1-100-96, TAR1-100-97, TAR1-100-98, TAR1-100-99, TAR1-100-100, TAR1-100-101, TAR1-100-102, TAR1-100-103, TAR1-100-105, TAR1-100-106, TAR1-100-107, TAR1-100-108, TAR1-100-109, TAR1-100-110, TAR1-100-111, TAR1-100-112, TAR1-100-113 and TAR1-5-19 or an amino acid sequence at least 90% identical thereto.

In another embodiment, the single domain antibody polypeptide construct comprises the amino acid sequence of an antibody polypeptide selected from the group consisting of clones TAR1-2m-9, TAR1-2m-30,TAR1-2m-1,TAR1-2m-2, TAR1-5, TAR1-27, TAR1-261, TAR1-398, TAR1-701,TAR1-5-2, TAR1-5-3, TAR1-5-4, TAR1-5-7, TAR1-5-8, TAR1-5-10, TAR1-5-11, TAR1-5-12, TAR1-5-13, TAR1-5-19, TAR1-5-20, TAR1-5-21, TAR1-5-22, TAR1-5-23, TAR1-5-24, TAR1-5-25, TAR1-5-26, TAR1-5-27, TAR1-5-28, TAR1-5-29, TAR1-5-34, TAR1-5-35, TAR1-5-36, TAR1-5-464, TAR1-5-463, TAR1-5-460, TAR1-5-461, TAR1-5-479, TAR1-5-477, TAR1-5-478, TAR1-5-476, TAR1-5-490, TAR1h-1, TAR1h-2, TAR1h-3, TAR1-100-29, TAR1-100-35, TAR1-100-43, TAR1-100-47, TAR1-100-52, TAR1-109, TAR1-100, TAR1-100-34, TAR1-100-36, TAR1-100-38, TAR1-100-39, TAR1-100-40, TAR1-100-41, TAR1-100-45, TAR1-100-60, TAR1-100-62, TAR1-100-64, TAR1-100-65, TAR1-100-75, TAR1-100-76, TAR1-100-77, TAR1-100-78, TAR1-100-80, TAR1-100-82, TAR1-100-83, TAR1-100-84, TAR1-100-89, TAR1-100-90, TAR1-100-91, TAR1-100-92, TAR1-100-93, TAR1-100-94, TAR1-100-95, TAR1-100-96, TAR1-100-97, TAR1-100-98, TAR1-100-99, TAR1-100-100, TAR1-100-101, TAR1-100-102, TAR1-100-103, TAR1-100-105, TAR1-100-106, TAR1-100-107, TAR1-100-108, TAR1-100-109, TAR1-100-110, TAR1-100-111, TAR1-100-112, TAR1-100-113 and TAR1-5-19 or an amino acid sequence at least 92% identical thereto.

In another embodiment, the single domain antibody polypeptide construct comprises the amino acid sequence of an antibody polypeptide selected from the group consisting of clones TAR1-2m-9, TAR1-2m-30,TAR1-2m-1,TAR1-2m-2, TAR1-5, TAR1-27, TAR1-261, TAR1-398, TAR1-701,TAR1-5-2, TAR1-5-3, TAR1-5-4, TAR1-5-7, TAR1-5-8, TAR1-5-10, TAR1-5-11, TAR1-5-12, TAR1-5-13, TAR1-5-19, TAR1-5-20, TAR1-5-21, TAR1-5-22, TAR1-5-23, TAR1-5-24, TAR1-5-25, TAR1-5-26, TAR1-5-27, TAR1-5-28, TAR1-5-29, TAR1-5-34, TAR1-5-35, TAR1-5-36, TAR1-5-464, TAR1-5-463, TAR1-5-460, TAR1-5-461, TAR1-5-479, TAR1-5-477, TAR1-5-478, TAR1-5-476, TAR1-5-490, TAR1h-1, TAR1h-2, TAR1h-3, TAR1-100-29, TAR1-100-35, TAR1-100-43, TAR1-100-47, TAR1-100-52, TAR1-109, TAR1-100, TAR1-100-34, TAR1-100-36, TAR1-100-38, TAR1-100-39, TAR1-100-40, TAR1-100-41, TAR1-100-45, TAR1-100-60, TAR1-100-62, TAR1-100-64, TAR1-100-65, TAR1-100-75, TAR1-100-76, TAR1-100-77, TAR1-100-78, TAR1-100-80, TAR1-100-82, TAR1-100-83, TAR1-100-84, TAR1-100-89, TAR1-100-90, TAR1-100-91, TAR1-100-92, TAR1-100-93, TAR1-100-94, TAR1-100-95, TAR1-100-96, TAR1-100-97, TAR1-100-98, TAR1-100-99, TAR1-100-100, TAR1-100-101, TAR1-100-102, TAR1-100-103, TAR1-100-105, TAR1-100-106, TAR1-100-107, TAR1-100-108, TAR1-100-109, TAR1-100-110, TAR1-100-111, TAR1-100-112, TAR1-100-113 and TAR1-5-19 or an amino acid sequence at least 94% identical thereto.

In another embodiment, the single domain antibody polypeptide construct comprises the amino acid sequence of an antibody polypeptide selected from the group consisting of clones TAR1-2m-9, TAR1-2m-30,TAR1-2m-1,TAR1-2m-2, TAR1-5, TAR1-27, TAR1-261, TAR1-398, TAR1-701,TAR1-5-2, TAR1-5-3, TAR1-5-4, TAR1-5-7, TAR1-5-8, TAR1-5-10, TAR1-5-11, TAR1-5-12, TAR1-5-13, TAR1-5-19, TAR1-5-20, TAR1-5-21, TAR1-5-22, TAR1-5-23, TAR1-5-24, TAR1-5-25, TAR1-5-26, TAR1-5-27, TAR1-5-28, TAR1-5-29, TAR1-5-34, TAR1-5-35, TAR1-5-36, TAR1-5-464, TAR1-5-463, TAR 1-5-460, TAR 1-5-461, TAR1-5-479, TAR 1-5-477, TAR 1-5-478, TAR 1-5-476, TAR1-5-490, TAR1h-1, TAR1h-2, TAR1h-3, TAR1-100-29, TAR1-100-35, TAR1-100-43, TAR1-100-47, TAR1-100-52, TAR1-109, TAR1-100, TAR1-100-34, TAR1-100-36, TAR1-100-38, TAR1-100-39, TAR1-100-40, TAR1-100-41, TAR1-100-45, TAR1-100-60, TAR1-100-62, TAR1-100-64, TAR1-100-65, TAR1-100-75, TAR1-100-76, TAR1-100-77, TAR1-100-78, TAR1-100-80, TAR1-100-82, TAR1-100-83, TAR1-100-84, TAR1-100-89, TAR1-100-90, TAR1-100-91, TAR1-100-92, TAR1-100-93, TAR1-100-94, TAR1-100-95, TAR1-100-96, TAR1-100-97, TAR1-100-98, TAR1-100-99, TAR1-100-100, TAR1-100-101, TAR1-100-102, TAR1-100-103, TAR1-100-105, TAR1-100-106, TAR1-100-107, TAR1-100-108, TAR1-100-109, TAR1-100-110, TAR1-100-111, TAR1-100-112, TAR1-100-113 and TAR1-5-19 or an amino acid sequence at least 96% identical thereto.

In another embodiment, the single domain antibody polypeptide construct comprises the amino acid sequence of an antibody polypeptide selected from the group consisting of clones TAR1-2m-9, TAR1-2m-30,TAR1-2m-1,TAR1-2m-2, TAR1-5, TAR1-27, TAR1-261, TAR1-398, TAR1-701,TAR1-5-2, TAR1-5-3, TAR1-5-4, TAR1-5-7, TAR1-5-8, TAR1-5-10, TAR1-5-11, TAR1-5-12, TAR1-5-13, TAR1-5-19, TAR1-5-20, TAR1-5-21, TAR1-5-22, TAR1-5-23, TAR1-5-24, TAR1-5-25, TAR1-5-26, TAR1-5-27, TAR1-5-28, TAR1-5-29, TAR1-5-34, TAR1-5-35, TAR1-5-36, TAR1-5-464, TAR1-5-463, TAR1-5-460, TAR1-5-461, TAR1-5-479, TAR1-5-477, TAR1-5-478, TAR1-5-476, TAR1-5-490, TAR1h-1, TAR1h-2, TAR1h-3, TAR1-100-29, TAR1-100-35, TAR1-100-43, TAR1-100-47, TAR1-100-52, TAR1-109, TAR1-100, TAR1-100-34, TAR1-100-36, TAR1-100-38, TAR1-100-39, TAR1-100-40, TAR1-100-41, TAR1-100-45, TAR1-100-60, TAR1-100-62, TAR1-100-64, TAR1-100-65, TAR1-100-75, TAR1-100-76, TAR1-100-77, TAR1-100-78, TAR1-100-80, TAR1-100-82, TAR1-100-83, TAR1-100-84, TAR1-100-89, TAR1-100-90, TAR1-100-91, TAR1-100-92, TAR1-100-93, TAR1-100-94, TAR1-100-95, TAR1-100-96, TAR1-100-97, TAR1-100-98, TAR1-100-99, TAR1-100-100, TAR1-100-101, TAR1-100-102, TAR1-100-103, TAR1-100-105, TAR1-100-106, TAR1-100-107, TAR1-100-108, TAR1-100-109, TAR1-100-110, TAR1-100-111, TAR1-100-112, TAR1-100-113 and TAR1-5-19 or an amino acid sequence at least 98% identical thereto.

In another embodiment, the single domain antibody polypeptide construct comprises the amino acid sequence of an antibody polypeptide selected from the group consisting of clones TAR1-2m-9, TAR1-2m-30,TAR1-2m-1,TAR1-2m-2, TAR1-5, TAR1-27, TAR 1-261, TAR 1-398, TAR1-701,TAR1-5-2, TAR1-5-3, TAR1-5-4, TAR1-5-7, TAR1-5-8, TAR1-5-10, TAR1-5-11, TAR1-5-12, TAR1-5-13, TAR1-5-19, TAR1-5-20, TAR1-5-21, TAR1-5-22, TAR1-5-23, TAR1-5-24, TAR1-5-25, TAR1-5-26, TAR1-5-27, TAR1-5-28, TAR1-5-29, TAR1-5-34, TAR1-5-35, TAR1-5-36, TAR1-5-464, TAR1-5-463, TAR1-5-460, TAR1-5-461, TAR1-5-479, TAR1-5-477, TAR1-5-478, TAR1-5-476, TAR1-5-490, TAR1h-1, TAR1h-2, TAR1h-3, TAR1-100-29, TAR1-100-35, TAR1-100-43, TAR1-100-47, TAR1-100-52, TAR1-109, TAR1-100, TAR1-100-34, TAR1-100-36, TAR1-100-38, TAR1-100-39, TAR1-100-40, TAR1-100-41, TAR1-100-45, TAR1-100-60, TAR1-100-62, TAR1-100-64, TAR1-100-65, TAR1-100-75, TAR1-100-76, TAR1-100-77, TAR1-100-78, TAR1-100-80, TAR1-100-82, TAR1-100-83, TAR1-100-84, TAR1-100-89, TAR1-100-90, TAR1-100-91, TAR1-100-92, TAR1-100-93, TAR1-100-94, TAR1-100-95, TAR1-100-96, TAR1-100-97, TAR1-100-98, TAR1-100-99, TAR1-100-100, TAR1-100-101, TAR1-100-102, TAR1-100-103, TAR1-100-105, TAR1-100-106, TAR1-100-107, TAR1-100-108, TAR1-100-109, TAR1-100-110, TAR1-100-111, TAR1-100-112, TAR1-100-113 and TAR1-5-19 or an amino acid sequence at least 99% identical thereto.

The invention further encompasses a method of treating rheumatoid arthritis, the method comprising administering to an individual in need thereof, a therapeutically effective amount of a composition comprising a single domain antibody polypeptide construct that antagonizes human TNFα's binding to a receptor, wherein the composition prevents an increase in arthritic score when administered to a mouse of the Tg197 transgenic mouse model of arthritis, wherein the single domain antibody polypeptide construct binds human TNFα with a Kd of < 100 nM, wherein the single domain antibody polypeptide construct neutralizes human TNFα as measured in a standard L929 cell assay, and wherein the rheumatoid arthritis is treated.

The invention further encompasses a composition comprising a single domain antibody polypeptide construct that antagonizes human TNFα's binding to a receptor, and that prevents an increase in arthritic score when administered to a mouse of the Tg197 transgenic mouse model of arthritis, wherein the single domain antibody polypeptide construct neutralizes human TNFα as measured in a standard L929 cell assay.

The invention further encompasses a composition comprising a single domain antibody polypeptide construct that antagonizes human TNFα's binding to a receptor, that prevents an increase in arthritic score when administered to a mouse of the Tg197 transgenic mouse model of arthritis, wherein the single domain antibody polypeptide construct inhibits the progression of the rheumatoid arthritis.

The invention further encompasses a composition comprising a single domain antibody polypeptide construct that antagonizes human TNFα's binding to a receptor, that prevents an increase in arthritic score when administered to a mouse of the Tg197 transgenic mouse model of arthritis, wherein the single domain antibody polypeptide construct binds human TNFα with a Kd of < 100 nM.

The invention further encompasses a composition comprising a single domain antibody polypeptide construct that antagonizes human TNFα's binding to a receptor, that prevents an increase in arthritic score when administered to a mouse of the Tg197 transgenic mouse model of arthritis, wherein the single domain antibody polypeptide construct neutralizes human TNFα as measured in a standard L929 cell assay, wherein the single domain antibody polypeptide construct inhibits the progression of the rheumatoid arthritis, wherein the single domain antibody polypeptide construct binds human TNFα with a Kd of < 100 nM.

In a further embodiment of the preceding 3 embodiments, the single domain antibody polypeptide construct comprises the amino acid sequence of CDR3 of an antibody polypeptide selected from the group consisting of clones TAR1-2m-9, TAR1-2m-30,TAR1-2m-1,TAR1-2m-2, TAR1-5, TAR1-27, TAR1-261, TAR1-398, TAR1-701,TAR1-5-2, TAR1-5-3, TAR1-5-4, TAR1-5-7, TAR1-5-8, TAR1-5-10, TAR1-5-11, TAR1-5-12, TAR1-5-13, TAR1-5-19, TAR1-5-20, TAR1-5-21, TAR1-5-22, TAR1-5-23, TAR1-5-24, TAR1-5-25, TAR1-5-26, TAR1-5-27, TAR1-5-28, TAR1-5-29, TAR1-5-34, TAR1-5-35, TAR1-5-36, TAR1-5-464, TAR1-5-463, TAR1-5-460, TAR1-5-461, TAR1-5-479, TAR1-5-477, TAR1-5-478, TAR1-5-476, TAR1-5-490, TAR1h-1, TAR1h-2, TAR1h-3, TAR1-100-29, TAR1-100-35, TAR1-100-43, TAR1-100-47, TAR1-100-52, TAR1-109, TAR1-100, TAR1-100-34, TAR1-100-36, TAR1-100-38, TAR1-100-39, TAR1-100-40, TAR1-100-41, TAR1-100-45, TAR1-100-60, TAR1-100-62, TAR1-100-64, TAR1-100-65, TAR1-100-75, TAR1-100-76, TAR1-100-77, TAR1-100-78, TAR1-100-80, TAR1-100-82, TAR1-100-83, TAR1-100-84, TAR1-100-89, TAR1-100-90, TAR1-100-91, TAR1-100-92, TAR1-100-93, TAR1-100-94, TAR1-100-95, TAR1-100-96, TAR1-100-97, TAR1-100-98, TAR1-100-99, TAR1-100-100, TAR1-100-101, TAR 1-100-102, TAR1-100-103, TAR1-100-105, TAR1-100-106, TAR1-100-107, TAR1-100-108, TAR1-100-109, TAR1-100-110, TAR1-100-111, TAR1-100-112, TAR1-100-113 and TAR1-5-19.

In a further embodiment the single domain antibody polypeptide construct comprises the amino acid sequence of an antibody polypeptide selected from the group consisting of clones TAR1-2m-9, TAR1-2m-30, TAR1-2m-1, TAR1-2m-2, TAR1-5, TAR1-27, TAR1-261, TAR1-398, TAR1-701,TAR1-5-2, TAR1-5-3, TAR1-5-4, TAR1-5-7, TAR1-5-8, TAR1-5-10, TAR1-5-11, TAR1-5-12, TAR1-5-13, TAR1-5-19, TAR1-5-20, TAR1-5-21, TAR1-5-22, TAR1-5-23, TAR1-5-24, TAR1-5-25, TAR1-5-26, TAR1-5-27, TAR1-5-28, TAR1-5-29, TAR1-5-34, TAR1-5-35, TAR1-5-36, TAR1-5-464, TAR1-5-463, TAR1-5-460, TAR1-5-461, TAR1-5-479, TAR1-5-477, TAR1-5-478, TAR1-5-476, TAR 1-5-490, TAR1h-1, TAR1h-2, TAR1h-3, TAR1-100-29, TAR1-100-35, TAR1-100-43, TAR1-100-47, TAR1-100-52, TAR1-109, TAR1-100, TAR1-100-34, TAR1-100-36, TAR1-100-38, TAR1-100-39, TAR1-100-40, TAR1-100-41, TAR1-100-45, TAR1-100-60, TAR1-100-62, TAR1-100-64, TAR1-100-65, TAR1-100-75, TAR1-100-76, TAR1-100-77, TAR1-100-78, TAR1-100-80, TAR1-100-82, TAR1-100-83, TAR1-100-84, TAR1-100-89, TAR1-100-90, TAR1-100-91, TARI-100-92, TAR1-100-93, TAR1-100-94, TAR1-100-95, TAR1-100-96, TAR1-100-97, TAR1-100-98, TAR1-100-99, TAR1-100-100, TAR1-100-101, TAR1-100-102, TAR1-100-103, TAR1-100-105, TAR1-100-106, TAR1-100-107, TAR1-100-108, TAR1-100-109, TAR1-100-110, TAR1-100-111, TAR1-100-112, TAR1-100-113 and TAR1-5-19 or an amino acid sequence at least 85% identical thereto.

In a further embodiment the single domain antibody polypeptide construct comprises the amino acid sequence of an antibody polypeptide selected from the group consisting of clones TAR1-2m-9, TAR1-2m-30, TAR1-2m-1, TAR1-2m-2, TAR1-5, TAR1-27, TAR1-261, TAR1-398, TAR1-701, TAR1-5-2, TAR1-5-3, TAR1-5-4, TAR1-5-7, TAR1-5-8, TAR1-5-10, TAR1-5-11, TAR1-5-12, TAR1-5-13, TAR1-5-19, TAR1-5-20, TAR1-5-21, TAR1-5-22, TAR1-5-23, TAR1-5-24, TAR1-5-25, TAR1-5-26, TAR1-5-27, TAR1-5-28, TAR1-5-29, TAR1-5-34, TAR1-5-35, TAR1-5-36, TAR1-5-464, TAR1-5-463, TAR1-5-460, TAR1-5-461, TAR1-5-479, TAR1-5-477, TAR1-5-478, TAR1-5-476, TAR1-5-490, TAR1h-1, TAR1h-2, TAR1h-3, TAR1-100-29, TAR1-100-35, TAR1-100-43, TAR1-100-47, TAR1-100-52, TAR1-109, TAR1-100, TAR1-100-34, TAR1-100-36, TAR1-100-38, TAR1-100-39, TAR1-100-40, TAR1-100-41, TAR1-100-45, TAR1-100-60, TAR1-100-62, TAR1-100-64, TAR1-100-65, TAR1-100-75, TAR1-100-76, TAR1-100-77, TAR1-100-78, TAR1-100-80, TAR1-100-82, TAR1-100-83, TAR1-100-84, TAR1-100-89, TAR1-100-90, TAR1-100-91, TAR1-100-92, TAR1-100-93, TAR1-100-94, TAR1-100-95, TAR1-100-96, TAR1-100-97, TAR1-100-98, TAR1-100-99, TAR1-100-100, TAR1-100-101, TAR1-100-102, TAR1-100-103, TAR1-100-105, TAR1-100-106, TAR1-100-107, TAR1-100-108, TAR1-100-109, TAR1-100-110, TAR1-100-111, TAR1-100-112, TAR1-100-113 and TAR1-5-19 or an amino acid sequence at least 90% identical thereto.

In a further embodiment the single domain antibody polypeptide construct comprises the amino acid sequence of an antibody polypeptide selected from the group consisting of clones TAR1-2m-9, TAR1-2m-30,TAR1-2m-1,TAR1-2m-2, TAR1-5, TAR1-27, TAR1-261, TAR1-398, TAR1-701,TAR1-5-2, TAR1-5-3, TAR1-5-4, TAR1-5-7, TAR1-5-8, TAR1-5-10, TAR1-5-11, TAR1-5-12, TAR1-5-13, TAR1-5-19, TAR1-5-20, TAR1-5-21, TAR1-5-22, TAR1-5-23, TAR1-5-24, TAR1-5-25, TAR1-5-26, TAR1-5-27, TAR1-5-28, TAR1-5-29, TAR1-5-34, TAR1-5-35, TAR1-5-36, TAR1-5-464, TAR1-5-463, TAR1-5-460, TAR1-5-461, TAR1-5-479, TAR1-5-477, TAR1-5-478, TAR1-5-476, TAR1-5-490, TAR1h-1, TAR1h-2, TAR1h-3, TAR1-100-29, TAR1-100-35, TAR1-100-43, TAR1-100-47, TAR1-100-52, TAR1-109, TAR1-100, TAR1-100-34, TAR1-100-36, TAR1-100-38, TAR1-100-39, TAR1-100-40, TAR1-100-41, TAR1-100-45, TAR1-100-60, TAR1-100-62, TAR1-100-64, TAR1-100-65, TAR1-100-75, TAR1-100-76, TAR1-100-77, TAR1-100-78, TAR1-100-80, TAR1-100-82, TAR1-100-83, TAR1-100-84, TAR1-100-89, TAR1-100-90, TAR1-100-91, TAR1-100-92, TAR1-100-93, TAR1-100-94, TAR1-100-95, TAR1-100-96, TAR1-100-97, TAR1-100-98, TAR1-100-99, TAR1-100-100, TAR1-100-101, TAR1-100-102, TAR1-100-103, TAR1-100-105, TAR1-100-106, TAR1-100-107, TAR1-100-108, TAR1-100-109, TAR1-100-110, TAR1-100-111, TAR1-100-112, TAR1-100-113 and TAR1-5-19 or an amino acid sequence at least 92% identical thereto.

In a further embodiment the single domain antibody polypeptide construct comprises the amino acid sequence of an antibody polypeptide selected from the group consisting of clones TAR1-2m-9, TAR1-2m-30,TAR1-2m-1,TAR1-2m-2, TAR1-5, TAR1-27, TAR1-261, TAR1-398, TAR1-701,TAR1-5-2, TAR1-5-3, TAR1-5-4, TAR1-5-7, TAR1-5-8, TAR1-5-10, TAR1-5-11, TAR1-5-12, TAR1-5-13, TAR1-5-19, TAR1-5-20, TAR1-5-21, TAR1-5-22, TAR1-5-23, TAR1-5-24, TAR1-5-25, TAR1-5-26, TAR1-5-27, TAR1-5-28, TAR1-5-29, TAR1-5-34, TAR1-5-35, TAR1-5-36, TAR1-5-464, TAR1-5-463, TAR1-5-460, TAR1-5-461, TAR1-5-479, TAR1-5-477, TAR1-5-478, TAR1-5-476, TAR1-5-490, TAR1h-1, TAR1h-2, TAR1h-3, TAR1-100-29, TAR1-100-35, TAR1-100-43, TAR1-100-47, TAR1-100-52, TAR1-109, TAR1-100, TAR1-100-34, TAR1-100-36, TAR1-100-38, TAR1-100-39, TAR1-100-40, TAR1-100-41, TAR1-100-45, TAR1-100-60, TAR1-100-62, TAR1-100-64, TAR1-100-65, TAR1-100-75, TAR1-100-76, TAR1-100-77, TAR1-100-78, TAR1-100-80, TAR1-100-82, TAR1-100-83, TAR1-100-84, TAR1-100-89, TAR1-100-90, TAR1-100-91, TAR1-100-92, TAR1-100-93, TAR1-100-94, TAR1-100-95, TAR1-100-96, TAR1-100-97, TAR1-100-98, TAR1-100-99, TAR1-100-100, TAR1-100-101, TAR1-100-102, TAR1-100-103, TAR1-100-105, TAR1-100-106, TAR1-100-107, TAR1-100-108, TAR1-100-109, TAR1-100-110, TAR1-100-111, TAR1-100-112, TAR1-100-113 and TAR1-5-19 or an amino acid sequence at least 94% identical thereto.

In a further embodiment the single domain antibody polypeptide construct comprises the amino acid sequence of an antibody polypeptide selected from the group consisting of clones TAR1-2m-9, TAR1-2m-30,TAR1-2m-1,TAR1-2m-2, TAR1-5, TAR1-27, TAR1-261, TAR1-398, TAR1-701,TAR1-5-2, TAR1-5-3, TAR1-5-4, TAR1-5-7, TAR1-5-8, TAR1-5-10, TAR1-5-11, TAR1-5-12, TAR1-5-13, TAR1-5-19, TAR1-5-20, TAR1-5-21, TAR1-5-22, TAR1-5-23, TAR1-5-24, TAR1-5-25, TAR1-5-26, TAR1-5-27, TAR1-5-28, TAR1-5-29, TAR1-5-34, TAR1-5-35, TAR1-5-36, TAR1-5-464, TAR1-5-463, TAR1-5-460, TAR1-5-461, TAR1-5-479, TAR1-5-477, TAR1-5-478, TAR1-5-476, TAR1-5-490, TAR1h-1, TAR1h-2, TAR1h-3, TAR1-100-29, TAR1-100-35, TAR1-100-43, TAR1-100-47, TAR1-100-52, TAR1-109, TAR1-100, TAR1-100-34, TAR1-100-36, TAR1-100-38, TAR1-100-39, TAR1-100-40, TAR1-100-41, TAR1-100-45, TAR1-100-60, TAR1-100-62, TAR1-100-64, TAR1-100-65, TAR1-100-75, TAR1-100-76, TAR1-100-77, TAR1-100-78, TAR1-100-80, TAR1-100-82, TAR1-100-83, TAR1-100-84, TAR1-100-89, TAR1-100-90, TAR1-100-91, TAR1-100-92, TAR1-100-93, TAR1-100-94, TAR1-100-95, TAR1-100-96, TAR1-100-97, TAR1-100-98, TAR1-100-99, TAR1-100-100, TAR1-100-101, TAR1-100-102, TAR1-100-103, TAR1-100-105, TAR1-100-106, TAR1-100-107, TAR1-100-108, TAR1-100-109, TAR1-100-110, TAR1-100-111, TAR1-100-112, TAR1-100-113 and TAR1-5-19 or an amino acid sequence at least 96% identical thereto.

In a further embodiment the single domain antibody polypeptide construct comprises the amino acid sequence of an antibody polypeptide selected from the group consisting of clones TAR1-2m-9, TAR1-2m-30,TAR1-2m-1,TAR1-2m-2, TAR1-5, TAR1-27, TAR1-261, TAR1-398, TAR1-701,TAR1-5-2, TAR1-5-3, TAR1-5-4, TAR1-5-7, TAR1-5-8, TAR1-5-10, TAR1-5-11, TAR1-5-12, TAR1-5-13, TAR1-5-19, TAR1-5-20, TAR1-5-21, TAR1-5-22, TAR1-5-23, TAR1-5-24, TAR1-5-25, TAR1-5-26, TAR1-5-27, TAR1-5-28, TAR1-5-29, TAR1-5-34, TAR1-5-35, TAR1-5-36, TAR1-5-464, TAR1-5-463, TAR1-5-460, TAR1-5-461, TAR1-5-479, TAR1-5-477, TAR1-5-478, TAR1-5-476, TAR1-5-490, TAR1h-1, TAR1h-2, TAR1h-3, TAR1-100-29, TAR1-100-35, TAR1-100-43, TAR1-100-47, TAR1-100-52, TAR1-109, TAR1-100, TAR1-100-34, TAR1-100-36, TAR1-100-38, TAR1-100-39, TAR1-100-40, TAR1-100-41, TAR1-100-45, TAR1-100-60, TAR1-100-62, TAR1-100-64, TAR1-100-65, TAR1-100-75, TAR1-100-76, TAR1-100-77, TAR1-100-78, TAR1-100-80, TAR1-100-82, TAR1-100-83, TAR1-100-84, TAR1-100-89, TAR1-100-90, TAR1-100-91, TAR1-100-92, TAR1-100-93, TAR1-100-94, TAR1-100-95, TAR1-100-96, TAR1-100-97, TAR1-100-98, TAR1-100-99, TAR1-100-100, TAR1-100-101, TAR1-100-102, TAR1-100-103, TAR1-100-105, TAR1-100-106, TAR1-100-107, TAR1-100-108, TAR1-100-109, TAR1-100-110, TAR1-100-111, TAR1-100-112, TAR1-100-113 and TAR1-5-19 or an amino acid sequence at least 98% identical thereto.

In a further embodiment the single domain antibody polypeptide construct comprises the amino acid sequence of an antibody polypeptide selected from the group consisting of clones TAR1-2m-9, TAR1-2m-30,TAR1-2m-1,TAR1-2m-2, TAR1-5, TAR1-27, TAR1-261, TAR1-398, TAR1-701,TAR1-5-2, TAR1-5-3, TAR1-5-4, TAR1-5-7, TAR1-5-8, TAR1-5-10, TAR1-5-11, TAR1-5-12, TAR1-5-13, TAR1-5-19, TAR1-5-20, TAR1-5-21, TAR1-5-22, TAR1-5-23, TAR1-5-24, TAR1-5-25, TAR1-5-26, TAR1-5-27, TAR1-5-28, TAR1-5-29, TAR1-5-34, TAR1-5-35, TAR1-5-36, TAR1-5-464, TAR1-5-463, TAR1-5-460, TAR1-5-461, TAR1-5-479, TAR1-5-477, TAR 1-5-478, TAR1-5-476, TAR1-5-490, TAR1h-1, TAR1h-2, TAR1h-3, TAR1-100-29, TAR1-100-35, TAR1-100-43, TAR1-100-47, TAR1-100-52, TAR1-109, TAR1-100, TAR1-100-34, TAR1-100-36, TAR1-100-38, TAR1-100-39, TAR1-100-40, TAR1-100-41, TAR1-100-45, TAR1-100-60, TAR1-100-62, TAR1-100-64, TAR1-100-65, TAR1-100-75, TAR1-100-76, TAR1-100-77, TAR1-100-78, TAR1-100-80, TAR1-100-82, TAR1-100-83, TAR1-100-84, TAR1-100-89, TAR1-100-90, TAR1-100-91, TAR1-100-92, TAR1-100-93, TAR1-100-94, TAR1-100-95, TAR1-100-96, TAR1-100-97, TAR1-100-98, TAR1-100-99, TAR1-100-100, TAR1-100-101, TAR1-100-102, TAR1-100-103, TAR1-100-105, TAR1-100-106, TAR1-100-107, TAR1-100-108, TAR1-100-109, TAR1-100-110, TAR1-100-111, TAR1-100-112, TAR1-100-113 and TAR1-5-19 or an amino acid sequence at least 98% identical thereto.

In a further embodiment the single domain antibody polypeptide construct comprises the amino acid sequence of an antibody polypeptide selected from the group consisting of clones TAR1-2m-9, TAR1-2m-30,TAR1-2m-1,TAR1-2m-2, TAR1-5, TAR1-27, TAR1-261, TAR1-398, TAR1-701,TAR1-5-2, TAR1-5-3, TAR1-5-4, TAR1-5-7, TAR1-5-8, TAR1-5-10, TAR1-5-11, TAR1-5-12, TAR1-5-13, TAR1-5-19, TAR1-5-20, TAR1-5-21, TAR1-5-22, TAR1-5-23, TAR1-5-24, TAR1-5-25, TAR1-5-26, TAR1-5-27, TAR1-5-28, TAR1-5-29, TAR1-5-34, TAR1-5-35, TAR1-5-36, TAR1-5-464, TAR1-5-463, TAR1-5-460, TAR1-5-461, TAR1-5-479, TAR1-5-477, TAR1-5-478, TAR1-5-476, TAR1-5-490, TAR1h-1, TAR1h-2, TAR1h-3, TAR1-100-29, TAR1-100-35, TAR1-100-43, TAR1-100-47, TAR1-100-52, TAR1-109, TAR1-100, TAR1-100-34, TAR1-100-36, TAR1-100-38, TAR1-100-39, TAR1-100-40, TAR1-100-41, TAR1-100-45, TAR1-100-60, TAR1-100-62, TAR1-100-64, TAR1-100-65, TAR1-100-75, TAR1-100-76, TAR1-100-77, TAR1-100-78, TAR1-100-80, TAR1-100-82, TAR1-100-83, TAR1-100-84, TAR1-100-89, TAR1-100-90, TAR1-100-91, TAR1-100-92, TAR1-100-93, TAR1-100-94, TAR1-100-95, TAR1-100-96, TAR1-100-97, TAR1-100-98, TAR1-100-99, TAR1-100-100, TAR1-100-101, TAR1-100-102, TAR1-100-103, TAR1-100-105, TAR1-100-106, TAR1-100-107, TAR1-100-108, TAR1-100-109, TAR1-100-110, TAR1-100-111, TAR1-100-112, TAR1-100-113 and TAR1-5-19 or an amino acid sequence at least 99% identical thereto.

The invention further encompasses a method of treating rheumatoid arthritis, the method comprising administering to an individual in need thereof a therapeutically effective amount of a composition comprising a single domain antibody polypeptide construct that antagonizes human VEGF's binding to a receptor, whereby the rheumatoid arthritis is treated.

In one embodiment the composition prevents an increase in arthritic score when administered to a mouse from a collagen induced arthritis (CIA) mouse model. Immunization of DBA/1 mice with murine type II collagen induces a chronic relapsing polyarthritis that provides a strong model for human autoimmune arthritis. The model is described, for example, by Courtenay et al., 1980, Nature 282 :666-668, Kato et al., 1996? Ann. Rheum. Dis. 55 :535-539 and Myers et al., 1997, Life Sci. 61 :1861-1878, each of which is incorporated herein by reference.

In one embodiment the administration of the composition to the mouse comprises the following steps: a) administer weekly intraperitoneal injections of the composition to the CIA mouse, b) weigh the mouse of step a) weekly, and c) score the mouse weekly for macrophenotypic signs of arthritis according to the following system: 0 = no arthritis (normal appearance and flexion), 1 = mild arthritis (joint distortion), 2 = moderate arthritis (swelling, joint deformation), 3 = heavy arthritis (severely impaired movement).

In one embodiment the treating comprises inhibiting the progression of the rheumatoid arthritis.

In one embodiment the treating comprises preventing or delaying the onset of rheumatoid arthritis.

In one embodiment the administering results in a statistically significant change in one or more indicia of RA. The change is preferably by at least 10% or more.

In one embodiment the one or more indicia of RA comprise one or more of erythrocyte sedimentation rate (ESR), Ritchie articular index (described in Ritchie et al., 1968, Q. J. Med. 37: 393-406) and duration of morning stiffness, joint mobility, joint swelling, analysis by x ray imaging of one or more joints, and histopathological indications by analysis of fixed sections of one or more joints. Disease activity and change effected with treatment can also be evaluated using the disease activity score (DAS) and/or the chronic arthritis systemic index (CASI), see Carotti et al., 2002, Ann. Rheum. Dis. 61:877-882, and Salaffi et al., 2000, Rheumatology 39: 90-96.

In one embodiment the one or more indicia of RA comprises a decrease in the macrophenotypic signs of arthritis in a mouse from a collagen induced arthritis mouse model, wherein the composition is administered to the mouse, wherein the mouse is scored for the macrophenotypic signs of arthritis, and wherein the macrophenotypic signs of arthritis are scored according to the following system: 0 = no arthritis (normal appearance and flexion), 1 = mild arthritis (joint distortion), 2 = moderate arthritis (swelling, joint deformation), 3 = heavy arthritis (severely impaired movement).

In one embodiment the one or more indicia of RA comprises a decrease in the histopathological signs of arthritis in a mouse from a collagen induced arthritis mouse model, wherein the composition is administered to the mouse, wherein the mouse is scored for the histopathological signs of arthritis, and wherein the histopathological signs of arthritis are performed on a joint and scored using the following system: 0= no detectable pathology, 1= hyperplasia of the synovial membrane and presence of polymorphonuclear infiltrates, 2= pannus and fibrous tissue formation and focal subchondral bone erosion, 3= articular cartilage destruction and bone erosion, 4= extensive articular cartilage destruction and bone erosion.

In one embodiment the single domain antibody polypeptide construct comprises a human single domain antibody polypeptide.

In one embodiment the human single domain antibody polypeptide binds VEGF.

In one embodiment the single domain antibody polypeptide construct binds human VEGF with a Kd of<100 nM.

In one embodiment the single domain antibody polypeptide construct binds human VEGF with a Kd in the range of 100 nM to 50 pM.

In one embodiment the single domain antibody polypeptide construct binds human VEGF with a Kd of 30 nM to 50 pM.

In one embodiment the single domain antibody polypeptide construct binds human VEGF with a Kd of 10 nM to 50 pM.

In one embodiment the single domain antibody polypeptide construct binds human VEGF with a Kd in the range of 1 nm to 50 pM.

In one embodiment the single domain antibody polypeptide construct neutralizes human VEGF as measured in a VEGF receptor 1 assay or a VEGF receptor 2 assay.

The invention further encompasses a method of treating rheumatoid arthritis, the method comprising administering to an individual in need thereof a therapeutically effective amount of a composition comprising a single domain antibody polypeptide construct that antagonizes human VEGF's's binding to a receptor, wherein the single domain antibody polypeptide construct inhibits the binding of human VEGF to a VEGF receptor, and whereby the rheumatoid arthritis is treated.

In one embodiment the single domain antibody polypeptide construct specifically binds to human VEGF which is bound to a cell surface receptor.

In one embodiment the single domain antibody polypeptide construct is linked to a PEG molecule.

In one embodiment the PEG-linked single domain antibody polypeptide construct has a hydrodynamic size of at least 24 kDa, and wherein the total PEG size is from 20 to 60 kDa.

In one embodiment the PEG-linked single domain antibody polypeptide construct has a hydrodynamic size of at least 200 kDa and a total PEG size of from 20 to 60 kDa.

In one embodiment the PEGylated proteins of the invention may be linked, on average, to 1, 2, 3, 4, 5, 6, 7, 8, 9, 10, 12, 15, 17, 20, or more polyethylene glycol molecules.

In one embodiment the antibody construct comprises two or more single immunoglobulin variable domain polypeptides that bind human VEGF.

In one embodiment the antibody construct comprises a homodimer of a single immunoglobulin variable domain polypeptide that binds human VEGF.

In one embodiment the antibody construct comprises a homotrimer of a single immunoglobulin variable domain polypeptide that binds human VEGF.

In one embodiment the antibody construct comprises a homotetramer of a single immunoglobulin variable domain polypeptide that binds human VEGF.

In one embodiment the construct further comprises an antibody polypeptide specific for an antigen other than VEGF.

In one embodiment the antibody polypeptide specific for an antigen other than VEGF comprises a single domain antibody polypeptide.

In one embodiment the binding of the antigen other than VEGF by the antibody polypeptide specific for an antigen other than VEGF increases the in vivo half-life of the antibody polypeptide construct.

In one embodiment the antigen other than VEGF comprises a serum protein.

In one embodiment the serum protein is selected from the group consisting of fibrin, α-2 macroglobulin, serum albumin, fibrinogen A, fibrinogen, serum amyloid protein A, heptaglobin, protein, ubiquitin, uteroglobulin and β-2- microglobulin.

In one embodiment the antigen other than VEGF comprises HSA.

In one embodiment, the single domain antibody polypeptide construct has an in vivo tα-half life in the range of 15 minutes to 12 hours. In another embodiment, the single domain antibody polypeptide construct has an in vivo tβ-half life in the range of 1 to 6 hours.

In another embodiment, the single domain antibody polypeptide construct has an in vivo tβ-half life in the range of 2 to 5 hours. In another embodiment, the single domain antibody polypeptide construct has an in vivo tβ-half life in the range of 3 to 4 hours.

In another embodiment, the single domain antibody polypeptide construct has an in vivo tβ-half life in the range of 12 to 60 hours. In another embodiment, the single domain antibody polypeptide construct has an in vivo tβ-half life in the range of 12 to 48 hours.

In another embodiment, the single domain antibody polypeptide construct has an in vivo tβ-half life in the range of 12 to 26 hours. In another embodiment, single domain antibody polypeptide construct has an in vivo AUC half-life value of 15 mg.min/ml to 150 mg.min/ml. In another embodiment, the single domain antibody polypeptide construct has an in vivo AUC half-life value of 15 mg.min/ml to 100 mg.min/ml. In another embodiment, the single domain antibody polypeptide construct has an in vivo AUC half-life value of 15 mg.min/ml to 75 mg.min/ml. In another embodiment, the single domain antibody polypeptide construct has an in vivo AUC half-life value of 15 mg.min/ml to 50 mg.min/ml.

In one embodiment the treating further comprises administration of at least one additional therapeutic agent.

In one embodiment the therapeutic agent is selected from the group consisting of Etanercept, inflixmab and D2E7.

In one embodiment the therapeutic agent is selected from the group consisting of Corticosteroids, Proteolytic enzymes, non-steroidal anti-inflammatory drugs (NTHES), Acetylsalicylic acid, pyrazolones, fenamate, diflunisal, acetic acid derivatives, propionic acid derivatives, oxicams, mefenamic acid, Ponstel, meclofenamate, Meclomen, phenylbutazone, Butazolidin, diflunisal, Dolobid, diclofenac, Voltaren, indomethacin, Indocin, sulindac, Clinoril, etodolac, Lodine, ketorolac, Toradol, nabumetone, Relafen, tolmetin, Tolectin, ibuprofen, Motrin, fenoprofen, Nalfon, flurbiprofen, Anthe, carprofen, Rimadyl, ketoprofen, Orudis, naproxen, Anaprox, Naprosyn, piroxicam and Feldene.

In one embodiment the single domain antibody polypeptide construct comprises the amino acid sequence of CDR3 of an antibody polypeptide selected from the group consisting of clones TAR15-1, TAR15-3, TAR15-4, TAR15-9, TAR15-10, TAR15-11, TAR15-12, TAR15-13, TAR15-14, TAR15-15, TAR15-16, TAR15-17, TAR15-18, TAR15-19, TAR 15-20, TAR 15-22, TAR15-5, TAR 15-6, TAR 15-7, TAR15-8, TAR15-23, TAR15-24, TAR15-25, TAR15-26, TAR15-27, TAR15-29, and TAR15-30.

In one embodiment the single domain antibody polypeptide construct comprises the amino acid sequence of an antibody polypeptide selected from the group consisting of clones TAR15-1, TAR15-3, TAR15-4, TAR15-9, TAR15-10, TAR15-11, TAR15-12, TAR15-13, TAR15-14, TAR15-15, TAR15-16, TAR15-17, TAR15-18, TAR15-19, TAR15-20, TAR 15-22, TAR15-5, TAR15-6, TAR15-7, TAR15-8, TAR15-23, TAR15-24, TAR15-25, TAR15-26, TAR15-27, TAR15-29, and TAR15-30 or an amino acid sequence at least 85% identical thereto.

In one embodiment the single domain antibody polypeptide construct comprises the amino acid sequence of an antibody polypeptide selected from the group consisting of clones TAR15-1, TAR15-3, TAR15-4, TAR15-9, TAR15-10, TAR15-11, TAR15-12, TAR15-13, TAR15-14, TAR15-15, TAR15-16, TAR15-17, TAR15-18, TAR15-19, TAR15-20, TAR 15-22, TAR15-5, TAR15-6, TAR15-7, TAR15-8, TAR15-23, TAR15-24, TAR15-25, TAR15-26, TAR15-27, TAR15-29, and TAR15-30 or an amino acid sequence at least 90% identical thereto.

In one embodiment the single domain antibody polypeptide construct comprises the amino acid sequence of an antibody polypeptide selected from the group consisting of clones TAR15-1, TAR15-3, TAR15-4, TAR15-9, TAR15-10, TAR15-11, TAR15-12, TAR15-13, TAR15-14, TAR15-15, TAR15-16, TAR15-17, TAR15-18, TAR15-19, TAR15-20, TAR 15-22, TAR15-5, TAR15-6, TAR15-7, TAR15-8, TAR15-23, TAR15-24, TAR15-25, TAR15-26, TAR15-27, TAR15-29, and TAR15-30 or an amino acid sequence at least 92% identical thereto.

In one embodiment the single domain antibody polypeptide construct comprises the amino acid sequence of an antibody polypeptide selected from the group consisting of clones TAR15-1, TAR15-3, TAR15-4, TAR15-9, TAR15-10, TAR15-11, TAR15-12, TAR15-13, TAR15-14, TAR15-15, TAR15-16, TAR15-17, TAR15-18, TAR15-19, TAR15-20, TAR 15-22, TAR15-5, TAR15-6, TAR15-7, TAR15-8, TAR15-23, TAR15-24, TAR15-25, TAR15-26, TAR15-27, TAR15-29, and TAR15-30 or an amino acid sequence at least 94% identical thereto.

In one embodiment the single domain antibody polypeptide construct comprises the amino acid sequence of an antibody polypeptide selected from the group consisting of clones TAR15-1, TAR15-3, TAR15-4, TAR15-9, TAR15-10, TAR15-11, TAR15-12, TAR15-13, TAR15-14, TAR15-15, TAR15-16, TAR15-17, TAR15-18, TAR15-19, TAR15-20, TAR 15-22, TAR15-5, TAR15-6, TAR15-7, TAR15-8, TAR15-23, TAR15-24, TAR15-25, TAR15-26, TAR15-27, TAR15-29, and TAR15-30 or an amino acid sequence at least 96% identical thereto.

In one embodiment the single domain antibody polypeptide construct comprises the amino acid sequence of an antibody polypeptide selected from the group consisting of clones TAR15-1, TAR15-3, TAR15-4, TAR15-9, TAR15-10, TAR15-11, TAR15-12, TAR15-13, TAR15-14, TAR15-15, TAR15-16, TAR15-17, TAR15-18, TAR15-19, TAR15-20, TAR 15-22, TAR15-5, TAR15-6, TAR15-7, TAR15-8, TAR15-23, TAR15-24, TAR15-25, TAR15-26, TAR15-27, TAR15-29, and TAR15-30 or an amino acid sequence at least 98% identical thereto.

In one embodiment the single domain antibody polypeptide construct comprises the amino acid sequence of an antibody polypeptide selected from the group consisting of clones TAR15-1, TAR15-3, TAR15-4, TAR15-9, TAR15-10, TAR15-11, TAR15-12, TAR15-13, TAR15-14, TAR15-15, TAR15-16, TAR15-17, TAR15-18, TAR15-19, TAR15-20, TAR 15-22, TAR15-5, TAR15-6, TAR15-7, TAR15-8, TAR15-23, TAR15-24, TAR15-25, TAR15-26, TAR15-27, TAR15-29, and TAR15-30 or an amino acid sequence at least 99% identical thereto.

The invention further encompasses a composition comprising a single domain antibody polypeptide construct that antagonizes human VEGF binding to a receptor,
wherein the single domain antibody polypeptide construct comprises a CDR3 sequence selected from the group consisting of TAR15-1, TAR15-3, TAR15-4, TAR15-9, TAR15-10, TAR15-11, TAR15-12, TAR15-13, TAR15-14, TAR15-15, TAR15-16, TAR15-17, TAR15-18, TAR15-19, TAR15-20, TAR 15-22, TAR15-5, TAR15-6, TAR15-7, TAR15-8, TAR15-23, TAR15-24, TAR15-25, TAR15-26, TAR15-27, TAR15-29, and TAR15-30.

The invention further encompasses a composition comprising a single domain antibody polypeptide construct that antagonizes human VEGF binding to a receptor,
wherein the single domain antibody polypeptide construct comprises an amino acid sequence selected from the group consisting of TAR15-1, TAR15-3, TAR15-4, TAR15-9, TAR15-10, TAR15-11, TAR15-12, TAR15-13, TAR15-14, TAR15-15, TAR15-16, TAR15-17, TAR15-18, TAR15-19, TAR15-20, TAR 15-22, TAR15-5, TAR15-6, TAR15-7, TAR15-8, TAR15-23, TAR15-24, TAR15-25, TAR15-26, TAR15-27, TAR15-29, and TAR15-30 or a sequence at least 85% identical thereto.

The invention further encompasses a composition comprising a single domain antibody polypeptide construct that antagonizes human VEGF binding to a receptor,
wherein the single domain antibody polypeptide construct comprises an amino acid sequence selected from the group consisting of TAR15-1, TAR15-3, TAR15-4, TAR15-9, TAR15-10, TAR15-11, TAR15-12, TAR15-13, TAR15-14, TAR15-15, TAR15-16, TAR15-17, TAR15-18, TAR15-19, TAR15-20, TAR 15-22, TAR15-5, TAR15-6, TAR15-7, TAR15-8, TAR15-23, TAR15-24, TAR15-25, TAR15-26, TAR15-27, TAR15-29, and TAR15-30 or a sequence at least 90% identical thereto.

The invention further encompasses a composition comprising a single domain antibody polypeptide construct that antagonizes human VEGF binding to a receptor,
wherein the single domain antibody polypeptide construct comprises an amino acid sequence selected from the group consisting of TAR15-1, TAR15-3, TAR15-4, TAR15-9, TAR15-10, TAR15-11, TAR15-12, TAR15-13, TAR15-14, TAR15-15, TAR15-16, TAR15-17, TAR15-18, TAR15-19, TAR15-20, TAR 15-22, TAR15-5, TAR15-6, TAR15-7, TAR15-8, TAR15-23, TAR15-24, TAR15-25, TAR15-26, TAR15-27, TAR15-29, and TAR15-30 or a sequence at least 92% identical thereto.

The invention further encompasses a composition comprising a single domain antibody polypeptide construct that antagonizes human VEGF binding to a receptor,
wherein the single domain antibody polypeptide construct comprises an amino acid sequence selected from the group consisting of TAR15-1, TAR15-3, TAR15-4, TAR15-9, TAR15-10, TAR15-11, TAR15-12, TAR15-13, TAR15-14, TAR15-15, TAR15-16, TAR15-17, TAR15-18, TAR 15-19, TAR 15-20, TAR 15-22, TAR15-5, TAR15-6, TAR15-7, TAR15-8, TAR15-23, TAR15-24, TAR15-25, TAR15-26, TAR15-27, TAR15-29, and TAR15-30 or a sequence at least 94% identical thereto.

The invention further encompasses a composition comprising a single domain antibody polypeptide construct that antagonizes human VEGF binding to a receptor,
wherein the single domain antibody polypeptide construct comprises an amino acid sequence selected from the group consisting of TAR15-1, TAR15-3, TAR15-4, TAR15-9, TAR15-10, TAR15-11, TAR15-12, TAR15-13, TAR15-14, TAR15-15, TAR15-16, TAR15-17, TAR15-18, TAR15-19, TAR15-20, TAR 15-22, TAR15-5, TAR15-6, TAR15-7, TAR15-8, TAR15-23, TAR15-24, TAR15-25, TAR15-26, TAR15-27, TAR15-29, and TAR15-30 or a sequence at least 96% identical thereto.

The invention further encompasses a composition comprising a single domain antibody polypeptide construct that antagonizes human VEGF binding to a receptor,
wherein the single domain antibody polypeptide construct comprises an amino acid sequence selected from the group consisting of TAR15-1, TAR15-3, TAR15-4, TAR15-9, TAR15-10, TAR15-11, TAR15-12, TAR15-13, TAR15-14, TAR15-15, TAR15-16, TAR15-17, TAR15-18, TAR15-19, TAR15-20, TAR 15-22, TAR15-5, TAR15-6, TAR15-7, TAR15-8, TAR15-23, TAR15-24, TAR15-25, TAR15-26, TAR15-27, TAR15-29, and TAR15-30 or a sequence at least 98% identical thereto.

The invention further encompasses a composition comprising a single domain antibody polypeptide construct that antagonizes human VEGF binding to a receptor,
wherein the single domain antibody polypeptide construct comprises an amino acid sequence selected from the group consisting of TAR15-1, TAR15-3, TAR15-4, TAR15-9, TAR15-10, TAR15-11, TAR15-12, TAR15-13, TAR15-14, TAR15-15, TAR15-16, TAR15-17, TAR15-18, TAR15-19, TAR15-20, TAR 15-22, TAR15-5, TAR15-6, TAR15-7, TAR15-8, TAR15-23, TAR15-24, TAR15-25, TAR15-26, TAR15-27, TAR15-29, and TAR15-30 or a sequence at least 99% identical thereto.

The invention further encompasses a method of treating rheumatoid arthritis, the method comprising administering to an individual in need thereof a therapeutically effective amount of a composition, wherein the composition comprises a single domain antibody polypeptide construct that antagonizes human TNFα's binding to a receptor and antagonizes human VEGF's binding to a receptor, whereby the rheumatoid arthritis is treated.

In one embodiment, the composition prevents an increase in arthritic score when administered to a mouse of the Tg197 transgenic mouse model of arthritis.

In another embodiment, the administration of the composition to a Tg197 transgenic mouse comprises the following steps: a) administer weekly intraperitoneal injections of the composition to a heterozygous Tg197 transgenic mouse, b) weigh the mouse of step a) weekly, and c) score the mouse weekly for macrophenotypic signs of arthritis according to the following system: 0 = no arthritis (normal appearance and flexion), 1 = mild arthritis (joint distortion), 2 = moderate arthritis (swelling, joint deformation), 3 = heavy arthritis (severely impaired movement).

In another embodiment, the composition has an efficacy in the Tg197 transgenic mouse arthritis assay that is greater than or equal, within the realm of statistical significance, to that of an agent selected from the group consisting of Etanercept, Infliximab and D2E7.

In another embodiment, the treating comprises inhibiting the progression of the rheumatoid arthritis.

In another embodiment, the treating comprises preventing or delaying the onset of rheumatoid arthritis.

In another embodiment, the administering results in a statistically significant change in one or more indicia of RA.

In another embodiment, the one or more indicia of RA comprise one or more of erythrocyte sedimentation rate (ESR), Ritchie articular index and duration of morning stiffness, joint mobility, joint swelling, x ray imaging of one or more joints, and histopathological analysis of fixed sections of one or more joints.

In another embodiment, the one or more indicia of RA comprises a decrease in the macrophenotypic signs of arthritis in a Tg197 transgenic mouse, wherein the composition is administered to a Tg197 transgenic mouse, wherein the Tg197 transgenic mouse is scored for the macrophenotypic signs of arthritis, and wherein the macrophenotypic signs of arthritis are scored according to the following system: 0 = no arthritis (normal appearance and flexion), 1 = mild arthritis (joint distortion), 2 = moderate arthritis (swelling, joint deformation), 3 = heavy arthritis (severely impaired movement).

In another embodiment, the one or more indicia of RA comprises a decrease in the histopathological signs of arthritis in a Tg197 transgenic mouse, wherein the composition is administered to a Tg197 transgenic mouse, wherein the Tg197 transgenic mouse is scored for the histopathological signs of arthritis, and wherein the histopathological signs of arthritis are performed on a joint and scored using the following system: 0= no detectable pathology, 1= hyperplasia of the synovial membrane and presence of polymorphonuclear infiltrates, 2= pannus and fibrous tissue formation and focal subchondral bone erosion, 3= articular cartilage destruction and bone erosion, 4= extensive articular cartilage destruction and bone erosion.

In another embodiment, the single domain antibody polypeptide construct comprises a human single domain antibody polypeptide.

In another embodiment, the human single domain antibody polypeptide binds TNFα and VEGF.

In another embodiment, the single domain antibody polypeptide construct neutralizes human TNFα as measured in a standard L929 cell assay.

In another embodiment, the single domain antibody polypeptide construct is linked to a PEG molecule.

In another embodiment, the PEG-linked single domain antibody polypeptide construct has a hydrodynamic size of at least 24 kDa, and wherein the total PEG size is from 20 to 60 kDa.

In another embodiment, the PEG-linked single domain antibody polypeptide construct has a hydrodynamic size of at least 200 kDa and a total PEG size of from 20 to 60 kDa.

In another embodiment, the antibody polypeptide construct is linked, on average, to 1, 2, 3, 4, 5, 6, 7, 8, 9, 10, 12, 15, 17, 20, or more polyethylene glycol molecules.

In another embodiment, the antibody construct comprises two or more single immunoglobulin variable domain polypeptides that bind human TNFα and/or two or more single immunoglobulin variable domain polypeptides that bind human VEGF.

In another embodiment, the antibody construct comprises a homodimer of a single immunoglobulin variable domain polypeptide that binds human TNFα and/or a homodimer of a single immunoglobulin variable domain polypeptide that binds human VEGF.

In another embodiment, the antibody construct comprises a homotrimer of a single immunoglobulin variable domain polypeptide that binds human TNFα and/or a homotrimer of a single immunoglobulin variable domain polypeptide that binds human VEGF.

In another embodiment, the antibody construct comprises a homotetramer of a single immunoglobulin variable domain polypeptide that binds human TNFα and/or a homotetramer of a single immunoglobulin variable domain polypeptide that binds human VEGF.

In another embodiment, the construct further comprises an antibody polypeptide specific for an antigen other than TNFα or VEGF.

In another embodiment, the antibody polypeptide specific for an antigen other than TNFα or VEGF comprises a single domain antibody polypeptide.

In another embodiment, the binding of the antigen other than TNFα or VEGF by the antibody polypeptide specific for an antigen other than TNFα or VEGF increases the in vivo half-life of the antibody polypeptide construct.

In another embodiment, the antigen other than TNFα or VEGF comprises a serum protein.

In another embodiment, the serum protein is selected from the group consisting of fibrin, α-2 macroglobulin, serum albumin, fibrinogen A, fibrinogen, serum amyloid protein A, heptaglobin, protein, ubiquitin, uteroglobulin and β-2- microglobulin.

In another embodiment, the antigen other than TNFα comprises HSA.

In another embodiment, the treating further comprises administration of at least one additional therapeutic agent.

The invention further encompasses a composition comprising a single domain antibody polypeptide construct that antagonizes human TNFα's binding to a receptor and that antagonizes human's VEGF's binding to a receptor, that prevents an increase in arthritic score when administered to a mouse of the Tg197 transgenic mouse model of arthritis, wherein the single domain antibody polypeptide construct inhibits the progression of the rheumatoid arthritis.

The invention further encompasses a composition comprising a single domain antibody polypeptide construct that antagonizes human TNFα's binding to a receptor and that antagonizes human's VEGF's binding to a receptor, that prevents an increase in arthritic score when administered to a mouse of the Tg197 transgenic mouse model of arthritis, wherein the single domain antibody polypeptide construct binds human TNFα with a Kd of < 100 nM.

The invention further encompasses a composition comprising a single domain antibody polypeptide construct that antagonizes human TNFα's binding to a receptor and that antagonizes human's VEGF's binding to a receptor, that prevents an increase in arthritic score when administered to a mouse of the Tg197 transgenic mouse model of arthritis, wherein the single domain antibody polypeptide construct neutralizes human TNFα as measured in a standard L929 cell assay, wherein the single domain antibody polypeptide construct inhibits the progression of the rheumatoid arthritis, wherein the single domain antibody polypeptide construct binds human TNFα with a Kd of < 100 nM.

Another aspect is a method for selecting a single domain antibody polypeptide construct that antagonizes human TNFα's binding to a receptor, that prevents an increase in arthritic score when administered to a mouse of the Tg197 transgenic mouse model of arthritis, wherein said single domain antibody polypeptide construct neutralizes human TNFα as measured in a standard L929 cell assay, wherein said single domain antibody polypeptide construct inhibits the progression of said rheumatoid arthritis, and wherein said single domain antibody polypeptide construct binds human TNFα with a Kd of < 100 nM, comprising the following steps: (1) mutating nucleic acid encoding several hypervariable region sites of said single domain antibody polypeptide construct, so that all possible amino substitutions are generated at each site, (2) introducing nucleic acid encoding the mutated hypervariable region sites generated in step (1) into a phagemid display vector, to form a large population of display vectors each capable of expressing one of said mutated hypervariable region sites displayed on a phagemid surface display protein; (3) expressing the mutated hypervariable region sites on the surface of a filamentous phage particle so that the mutated hypervariable region sites thus generated are displayed in a monovalent fashion from filamentous phage particles as fusions to the gene III product of M 13 packaged within each particle, (4) screening the surface-expressed phage particle for the ability to bind TNFa, (5) isolating those surface-expressed phage particle able to bind TNFa, (6) selecting a surface-expressed phage particle from step (5) that is able to bind TNFa, that also prevents an increase in arthritic score when administered to a mouse of the Tg197 transgenic mouse model of arthritis, and neutralizes human TNFα as measured in a standard L929 cell assay, and inhibits the progression of said rheumatoid arthritis, and binds human TNFα with a Kd of < 100 nM, thereby selecting one or more species of phagemid containing a display protein containing a single domain antibody polypeptide construct that antagonizes human TNFα's binding to a receptor.

Another aspect is a method of treating rheumatoid arthritis, the method comprising administering to an individual in need thereof a therapeutically effective amount of a composition comprising a single domain antibody polypeptide construct that antagonizes human VEGF's binding to a receptor, whereby said rheumatoid arthritis is treated,
wherein said single domain antibody polypeptide construct has an in vivo tα-half life in the range of 15 minutes to 12 hours, 1 to 6 hours, 2 to 5 hours, or 3 to 4 hours.

Another embodiment is a method of treating rheumatoid arthritis, the method comprising administering to an individual in need thereof a therapeutically effective amount of a composition comprising a single domain antibody polypeptide construct that antagonizes human VEGF's binding to a receptor, whereby said single domain antibody polypeptide construct has an in vivo tβ-half life in the range of 12 to 60 hours, 12 to 48 hours, or 12 to 26 hours.

Another embodiment is method of treating rheumatoid arthritis, the method comprising administering to an individual in need thereof a therapeutically effective amount of a composition comprising a single domain antibody polypeptide construct that antagonizes human VEGF's binding to a receptor, whereby said single domain antibody polypeptide construct has an in vivo AUC half-life value of 15 mg.min/ml to 150 mg.min/ml, 15 mg.min/ml to 100 mg.min/ml, 15 mg.min/ml to 75 mg.min/ml, or 15 mg.min/ml to 50 mg.min/ml.

Another embodiment is a method of treating rheumatoid arthritis, the method comprising administering to an individual in need thereof a therapeutically effective amount of a composition, wherein said composition comprises a single domain antibody polypeptide construct that antagonizes human TNFα's binding to a receptor and antagonizes human VEGF's binding to a receptor, whereby said rheumatoid arthritis is treated, and wherein said composition prevents an increase in arthritic score when administered to a mouse of the Tg197 transgenic mouse model of arthritis, and wherein said single domain antibody polypeptide construct binds human TNFα and VEGF each with a Kd of < 100 nM, wherein said single domain antibody polypeptide construct binds human TNFα and VEGF each with a Kd in the range of 100 nM to 50 pM, wherein said single domain antibody polypeptide construct binds human TNFα and VEGF each with a K_{d} of 30 nM to 50 pM, wherein said single domain antibody polypeptide construct binds human TNFα and VEGF each with a Kd of 10 nM to 50 pM, or wherein said single domain antibody polypeptide construct binds human TNFα and VEGF each with a K_{d} in the range of 1 nm to 50 pM.

Another embodiment is a method of treating rheumatoid arthritis, the method comprising administering to an individual in need thereof a therapeutically effective amount of a composition comprising a single domain antibody polypeptide construct that antagonizes human TNFα's binding to a receptor and antagonizes VEGF's binding to a receptor, wherein said single domain antibody polypeptide construct inhibits the binding of human TNFα to a TNFα receptor and of hyman VEGF to a VEGF receptor, and whereby said rheumatoid arthritis is treated.

Another embodiment is a method of treating rheumatoid arthritis, the method comprising administering to an individual in need thereof a therapeutically effective amount of a composition comprising a single domain antibody polypeptide construct that antagonizes human TNFα's binding to a receptor and antagonizes VEGF's binding to a receptor, wherein said single domain antibody polypeptide construct inhibits the binding of human TNFα to a TNFα receptor and of hyman VEGF to a VEGF receptor, and whereby said rheumatoid arthritis is treated, wherein said single domain antibody polypeptide construct specifically binds to human TNFα which is bound to a cell surface receptor.

Another embodiment is a method of treating rheumatoid arthritis, the method comprising administering to an individual in need thereof a therapeutically effective amount of a composition, wherein said composition comprises a single domain antibody polypeptide construct that antagonizes human TNFα's binding to a receptor and antagonizes human VEGF's binding to a receptor, whereby said rheumatoid arthritis is treated, and wherein said single domain antibody polypeptide construct specifically binds to human TNFα which is bound to a cell surface receptor.

Another embodiment of the invention is a method of treating rheumatoid arthritis comprising the administration of an antibody construct specific for TNFα, wherein the sequence of the antibody construct comprises, or consists of, a sequence with a percentage identity which is greater than or equal to 85, 90, 95, 96, 97, 98, 99 or 100% to the sequence of any one of the anti-TNF-α clones recited herein.

Another embodiment of the invention is a composition comprising an antibody construct specific for TNFα, wherein the sequence of the antibody construct comprises, or consists of, a sequence with a percentage identity which is greater than or equal to 85, 90, 95, 96, 97, 98, 99 or 100% to the sequence of any one of the anti-TNF-α clones recited herein.

Another embodiment of the invention is a method of treating rheumatoid arthritis comprising the administration of an antibody construct specific for VEGF, wherein the sequence of the antibody construct comprises, or consists of a sequence with a percentage identity which is greater than or equal to 85, 90, 95, 96, 97, 98, 99 or 100% to the sequence of any one of the anti-VEGF clones recited herein.

Another embodiment of the invention is a composition comprising an antibody construct specific for VEGF, wherein the sequence of the antibody construct comprises, or consists of, a sequence with a percentage identity which is greater than or equal to 85, 90, 95, 96, 97, 98, 99 or 100% to the sequence of any one of the anti-VEGF clones recited herein.

In another embodiment, there are provided tetravalent, dual-specific antigen-binding polypeptide constructs comprising two copies of a V_{H} or V_{L} single domain antibody that binds a first antigen or epitope; and two copies of a V_{H} or V_{L} single domain antibody that binds a second antigen or epitope. The first and second epitopes can be present on the same antigen or, alternatively, on different antigens. Each of the two copies of the single domain antibody that binds the first antigen or epitope is fused to a respective IgG heavy chain constant domain, and each of the two copies of the single domain antibody that binds the second antigen or epitope is fused to a respective light chain constant domain. These tetravalent, dual-specific polypeptide constructs are IgG-like in that they have two antigen-binding arms joined by heavy and light chain constant domains. They are different from naturally-occurring IgG in that, by virtue of the presence of two different antigen-specific single domain antibody polypeptides on each arm, each arm can bind two different antigens or epitopes, making the construct tetravalent and dual-specific. In one embodiment, the first and second epitopes are the same, such that there are four specific binding sites for that epitope present on the polypeptide construct. In another embodiment, the first and second epitopes are different, being present on the same or different antigens.

Dual-specific, tetravalent polypeptide constructs as described herein can include single domain antibody sequences specific for any two antigens or epitopes, but particularly those specific for human TNF-α and VEGF, and more particularly, any of those single domain antibody sequences described herein. In other embodiments, C_{κ} or C_{λ} light chain constant domains can be used, and IgG heavy chain constant domains other than IgG1 can also be used.

Also encompassed are constructs of this sort comprising single domain anti-TNF-α antibody clones that prevent an increase in arthritic score when administered as a monomer to a mouse of the Tg197 transgenic mouse model of arthritis, and single domain anti-VEGF antibody clones that prevent an increase in arthritic score when administered as a monomer to a mouse of a collagen-induced arthritis mouse model. In a further embodiment, the single domain anti-TNF-α antibody clone used neutralizes human TNF-α in the L929 cell cytotoxicity assay described herein when used as a monomer, and the single domain anti-VEGF antibody clone used antagonizes VEGF receptor binding in an assay of VEGF Receptor 2 binding as described herein when used a monomer. In a further embodiment, the single domain antibody clones used bind their respective antigens or epitopes with a K_{d} of < 100 nM. In a further embodiment, the dual-specific, bi-valent constructs bind the respective antigens or epitopes with a K_{d} of < 100 nM and prevent an increase in arthritic score in either or both of the Tg197 and CIA models of arthritis described herein.

Such tetravalent, dual specific constructs can be used for the treatment of rheumatoid arthritis in a manner similar to the other constructs described herein, in terms of administration, dosage and monitoring of efficacy. The half-life of the construct can be modified as described herein above, e.g., by addition of a PEG moiety, or by further fusion of a binding moiety (e.g., a further single domain antibody) specific for a protein that increases circulating half-life, e.g., a serum protein such as HSA.

In one aspect, then, there is described a dual-specific antigen-binding polypeptide comprising a first antibody single domain polypeptide that binds TNF-α and a second antibody single domain polypeptide that binds VEGF. Such polypeptides can be used, for example, for the treatment of rheumatoid arthritis.

In another aspect, then, there is described a tetravalent, dual-specific antigen binding polypeptide construct comprising: a) a first copy of a first fusion protein comprising a single domain antibody polypeptide that binds a first epitope, fused to an IgG heavy chain constant domain; b) a second copy of said first fusion protein; c) a first copy of a second fusion protein comprising a single domain antibody polypeptide that binds a second epitope, fused to a light chain constant domain; d) a second copy of said second fusion protein; wherein said first and said second copies of said first fusion protein are disulfide bonded to each other via their respective IgG heavy chain constant domains, and wherein said light chain constant domain of said first copy of said second fusion protein is disulfide bonded to the IgG heavy chain constant domain of said first copy of said first fusion protein, and wherein said light chain constant domain of said second copy of second fusion protein is disulfide bonded to the IgG heavy chain constant domain of said second copy of said first fusion protein, and wherein said polypeptide construct binds said first and said second epitopes.

In one embodiment of such tetravalent, dual-specific antigen binding polypeptide construct, the single domain antibody that binds a first epitope is a V domain selected from V_{H} and V_{L}.

In another embodiment, the single domain antibody that binds a second epitope is a V domain selected from V_{H} and V_{L}.

In another embodiment, the IgG heavy chain constant domain is an IgG 1 heavy chain constant domain.

In another embodiment, the light chain constant domain is a C_{κ} or C_{λ} light chain constant domain. In another embodiment, the light chain constant domain is a C_{κ} light chain constant domain.

In another embodiment, the first and second epitopes are present on the same antigen.

In another embodiment, the first and second epitopes are present on different first and second antigens.

In another embodiment, one or both of said single domain antibody that binds a first epitope and said single domain antibody that binds a second epitope are human single domain antibodies.

In another embodiment, the single domain antibody polypeptide that binds said first epitope and/or the single domain antibody polypeptide that binds said second epitope comprises one or more of a FW1 encoded by a human germline VH gene sequence, a FW2 encoded by a human germline VH gene sequence, a FW3 encoded by a human germline VH gene sequence and a FW4 encoded by a human germline VH gene sequence.

In another embodiment, both the single domain antibody polypeptide that binds a first epitope and the single domain antibody that binds a second epitope are human single domain antibodies.

In another embodiment, the IgG heavy chain constant domain is a human IgG heavy chain constant domain.

In another embodiment, the IgG heavy chain constant domain comprises a C_{H}1 domain.

In another embodiment, the light chain constant domain is a human C_{κ} light chain constant domain.

In another embodiment, the construct binds TNF-α and VEGF. In another embodiment, a single domain antibody that binds VEGF is fused to an IgG 1 heavy chain constant domain and a single domain antibody that binds TNF-α is fused to a C_{κ} light chain constant domain.

In another embodiment, the single domain antibody that binds VEGF prevents an increase in arthritic score when administered to a mouse of a collagen-induced arthritis mouse model.

In another embodiment, the single domain antibody polypeptide moiety that antagonizes an activity of human VEGF binds human VEGF with a K_{d} of <100 nM.

In another embodiment, the single domain antibody polypeptide moiety that antagonizes an activity of human VEGF neutralizes human VEGF as measured in a VEGF receptor 1 assay or a VEGF receptor 2 assay.

In another embodiment, the single domain antibody polypeptide moiety that antagonizes an activity of human VEGF specifically binds to human VEGF which is bound to a cell surface receptor.

In another embodiment of this aspect, the single domain antibody that binds TNF-α or antagonizes an activity of human TNF-α prevents an increase in arthritic score when administered to a mouse of the Tg197 transgenic mouse model of arthritis. In another embodiment, the single domain antibody antagonizes the activity of human TNF-α as measured in a standard L929 cell cytotoxicity assay. In another embodiment, the single domain antibody binds human TNF-α with a K_{d} of < 100 nM.

In any embodiment of this aspect, the single domain antibody polypeptide moiety that antagonizes an activity of human TNF-α can comprise, for example, the amino acid sequence of CDR3 of an antibody polypeptide selected from the group consisting of clones TAR1-2m-9, TAR1-2m-30,TAR1-2m-1,TAR1-2m-2, TAR1-5, TAR1-27, TAR1-261, TAR1-398, TAR1-701,TAR1-5-2, TAR1-5-3, TAR1-5-4, TAR1-5-7, TAR1-5-8, TAR1-5-10, TAR1-5-11, TAR1-5-12, TAR1-5-13, TAR1-5-19, TAR1-5-20, TAR1-5-21, TAR1-5-22, TAR1-5-23, TAR1-5-24, TAR1-5-25, TAR1-5-26, TAR1-5-27, TAR1-5-28, TAR1-5-29, TAR1-5-34, TAR1-5-35, TAR1-5-36, TAR1-5-464, TAR1-5-463, TAR1-5-460, TAR1-5-461, TAR1-5-479, TAR1-5-477, TAR1-5-478, TAR1-5-476, TAR1-5-490, TAR1h-1, TAR1h-2, TAR1h-3, TAR1-100-29, TAR1-100-35, TAR1-100-43, TAR1-100-47, TAR1-100-52, TAR1-109, TAR1-100, TAR1-100-34, TAR1-100-36, TAR1-100-38, TAR1-100-39, TAR1-100-40, TAR1-100-41, TAR1-100-45, TAR1-100-60, TAR1-100-62, TAR1-100-64, TAR1-100-65, TAR1-100-75, TAR1-100-76, TAR1-100-77, TAR1-100-78, TAR1-100-80, TAR1-100-82, TAR1-100-83, TAR1-100-84, TAR1-100-89, TAR1-100-90, TAR1-100-91, TAR1-100-92, TAR1-100-93, TAR1-100-94, TAR1-100-95, TAR1-100-96, TAR1-100-97, TAR1-100-98, TAR1-100-99, TAR1-100-100, TAR1-100-101, TAR1-100-102, TAR1-100-103, TAR1-100-105, TAR1-100-106, TAR1-100-107, TAR1-100-108, TAR1-100-109, TAR1-100-110, TAR1-100-111, TAR1-100-112, TAR1-100-113 and TAR1-5-19, or an amino acid sequence that is, for example, at least 85%, but alternatively at least 90%, 92%, 94%, 96%, 98%, 99% or 100% identical to a said sequence.

In another embodiment of this aspect, a single domain antibody polypeptide moiety that antagonizes an activity of human TNF-α can comprise, for example, the amino acid sequence of an antibody polypeptide selected from the group consisting of clones TAR1-2m-9, TAR1-2m-30,TAR1-2m-1,TAR1-2m-2, TAR1-5, TAR1-27, TAR1-261, TAR1-398, TAR1-701,TAR1-5-2, TAR1-5-3, TAR1-5-4, TAR1-5-7, TAR1-5-8, TAR1-5-10, TAR1-5-11, TAR1-5-12, TAR1-5-13, TAR1-5-19, TAR1-5-20, TAR1-5-21, TAR1-5-22, TAR1-5-23, TAR1-5-24, TAR1-5-25, TAR1-5-26, TAR1-5-27, TAR1-5-28, TAR1-5-29, TAR1-5-34, TAR1-5-35, TAR1-5-36, TAR1-5-464, TAR1-5-463, TAR1-5-460, TAR1-5-461, TAR1-5-479, TAR1-5-477, TAR1-5-478, TAR1-5-476, TAR1-5-490, TAR1h-1, TAR1h-2, TAR1h-3, TAR1-100-29, TAR1-100-35, TAR1-100-43, TAR1-100-47, TAR1-100-52, TAR1-109, TAR1-100, TAR1-100-34, TAR1-100-36, TAR1-100-38, TAR1-100-39, TAR1-100-40, TAR1-100-41, TAR1-100-45, TAR1-100-60, TAR1-100-62, TAR1-100-64, TAR1-100-65, TAR1-100-75, TAR1-100-76, TAR1-100-77, TAR1-100-78, TAR1-100-80, TAR1-100-82, TAR1-100-83, TAR1-100-84, TAR1-100-89, TAR1-100-90, TAR1-100-91, TAR1-100-92, TAR1-100-93, TAR1-100-94, TAR1-100-95, TAR1-100-96, TAR1-100-97, TAR1-100-98, TAR1-100-99, TAR1-100-100, TAR1-100-101, TAR1-100-102, TAR1-100-103, TAR1-100-105, TAR1-100-106, TAR1-100-107, TAR1-100-108, TAR1-100-109, TAR1-100-110, TAR1-100-111, TAR1-100-112, TAR1-100-113 and TAR1-5-19, or an amino acid sequence that is, for example, at least 85%, but alternatively at least 90%, 92%, 94%, 96%, 98%, 99% or 100% identical to a said sequence.

In any embodiment of this aspect, the single domain antibody polypeptide moiety that antagonizes the binding of human VEGF to a VEGF receptor can comprise, for example, the amino acid sequence of CDR3 of an antibody polypeptide selected from the group consisting of clones TAR15-1, TAR15-3, TAR15-4, TAR15-9, TAR15-10, TAR15-11, TAR15-12, TAR15-13, TAR15-14, TAR15-15, TAR15-16, TAR15-17, TAR15-18, TAR15-19, TAR15-20, TAR 15-22, TAR15-5, TAR15-6, TAR15-7, TAR15-8, TAR15-23, TAR15-24, TAR15-25, TAR15-26, TAR15-27, TAR15-29, and TAR15-30, or an amino acid sequence that is, for example, at least 85%, but alternatively at least 90%, 92%, 94%, 96%, 98%, 99% or 100% identical to a said sequence.

In another embodiment of this aspect, a single domain antibody polypeptide moiety that antagonizes the binding of human VEGF to a VEGF receptor can comprise the amino acid sequence of an antibody polypeptide selected from the group consisting of clones TAR15-1, TAR15-3, TAR15-4, TAR15-9, TAR15-10, TAR15-11, TAR15-12, TAR15-13, TAR15-14, TAR15-15, TAR15-16, TAR15-17, TAR15-18, TAR15-19, TAR15-20, TAR 15-22, TAR15-5, TAR15-6, TAR15-7, TAR15-8, TAR15-23, TAR15-24, TAR15-25, TAR15-26, TAR15-27, TAR15-29, and TAR15-30 or an amino acid sequence that is, for example, at least 85%, but alternatively at least 90%, 92%, 94%, 96%, 98%, 99% or 100% identical to a said sequence.

### Definitions

Unless defined otherwise, all technical and scientific terms used herein have the same meaning as commonly understood by one of ordinary skill in the art (e.g., in cell culture, molecular genetics, nucleic acid chemistry, hybridization techniques and biochemistry). Standard techniques are used for molecular, genetic and biochemical methods (see generally, Sambrook et al., Molecular Cloning: A Laboratory Manual, 2d ed. (1989) Cold Spring Harbor Laboratory Press, Cold Spring Harbor, N.Y. and Ausubel et al., Short Protocols in Molecular Biology (1999) 4th Ed, John Wiley & Sons, Inc. which are incorporated herein by reference) and chemical methods.

As used herein, the term "domain" refers to a folded protein structure which retains its tertiary structure independently of the rest of the protein. Generally, domains are responsible for discrete functional properties of proteins, and in many cases may be added, removed or transferred to other proteins without loss of function of the remainder of the protein and/or of the domain.

By "single immunoglobulin variable domain" or "single domain antibody polypeptide" is meant a folded polypeptide domain which comprises sequences characteristic of immunoglobulin variable domains and which specifically binds an antigen (i.e., dissociation constant of 500 nM or less). A "single domain antibody polypeptide" therefore includes complete antibody variable domains as well as modified variable domains, for example in which one or more loops have been replaced by sequences which are not characteristic of antibody variable domains or antibody variable domains which have been truncated or comprise N- or C-terminal extensions, as well as folded fragments of variable domains which retain a dissociation constant of 500 nM or less (e.g., 450 nM or less, 400 nM or less, 350 nM or less, 300 nM or less, 250 nM or less, 200 nM or less, 150 nM or less, 100 nM or less) and the target antigen specificity of the full-length domain. Preferably an antibody single variable domain is selected from the group of V_{H} and V_{L}, including Vₖₐₚₚₐ and V_{lambda}.

The phrase "single domain antibody polypeptide construct" encompasses not only an isolated single domain antibody polypeptide, but also larger polypeptide constructs that comprise one or more monomers of a single immunoglobulin variable domain polypeptide sequence. It is stressed, that a single domain antibody polypeptide that is part of a larger construct is capable, on its own, of specifically binding a target antigen. Thus, a single domain antibody polypeptide construct that comprises more than one single domain antibody polypeptide does not encompass, for example, a construct in which a V_{H} and a V_{L} domain are cooperatively required to form the binding site necessary to specifically bind a single antigen molecule. The linkage between single domain antibody polypeptides in a single domain antibody polypeptide construct can be peptide or polypeptide linkers, or, alternatively, can be other chemical linkages, such as through linkage of polypeptide monomers to a multivalent PEG. The linked single domain antibody polypeptides can be identical or different, and the target specificities of the constituent polypeptides can likewise be the same or different.

Complementary: Two immunoglobulin domains are "complementary" where they belong to families of structures which form cognate pairs or groups or are derived from such families and retain this feature. For example, a VH domain and a VL domain of an antibody are complementary; two VH domains are not complementary, and two V domains are not complementary. Complementary domains may be found in other members of the immunoglobulin superfamily, such as the V_{α} and V_{β} (or γ and δ) domains of the T-cell receptor. In the context of the second configuration of the invention, non-complementary domains do not bind a target molecule cooperatively, but act independently on different target epitopes which may be on the same or different molecules. Domains which are artificial, such as domains based on protein scaffolds which do not bind epitopes unless engineered to do so, are non- complementary. Likewise, two domains based on (for example) an immunoglobulin domain and a fibronectin domain are not complementary.

Immunoglobulin: This refers to a family of polypeptides which retain the immunoglobulin fold characteristic of antibody molecules, which contains two β sheets and, usually, a conserved disulphide bond. Members of the immunoglobulin superfamily are involved in many aspects of cellular and non-cellular interactions in vivo, including widespread roles in the immune system (for example, antibodies, T-cell receptor molecules and the like), involvement in cell adhesion (for example the ICAM molecules) and intracellular signalling (for example, receptor molecules, such as the PDGF receptor).

The present invention is applicable to all immunoglobulin superfamily molecules which possess binding domains. Preferably, the present invention relates to antibodies.

Combining: Variable domains according to the invention are combined to form a group of domains; for example, complementary domains may be combined, such as V_{L} domains being combined with VH domains. Non- complementary domains may also be combined. Domains may be combined in a number of ways, involving linkage of the domains by covalent or non-covalent means.

Closed conformation multi-specific ligand: The phrase describes a multi-specific ligand as herein defined comprising at least two epitope binding domains as herein deemed. The term 'closed conformation' (multi-specific ligand) means that the epitope binding domains of the ligand are arranged such that epitope binding by one epitope binding domain competes with epitope binding by another epitope binding domain. That is, cognate epitopes may be bound by each epitope binding domain individually but not simultaneously. The closed conformation of the ligand can be achieved using methods herein described.

Antibody: An antibody (for example IgG, IgM, IgA, IgD or IgE) or fragment (such as a Fab, F(ab')2, Fv, disulphide linked Fv, scFv, closed conformation multispecific antibody, disulphide-linked scFv, diabody) whether derived from any species naturally producing an antibody, or created by recombinant DNA technology; whether isolated from serum, B- cells, hybridomas, transfectomas, yeast or bacteria).

Dual-specific ligand: A ligand comprising a first immunoglobulin single variable domain and a second immunoglobulin single variable domain as herein defined, wherein the variable regions are capable of binding to two different antigens or two epitopes on the same antigen which are not normally bound by a monospecific immunoglobulin. For example, the two epitopes may be on the same hapten, but are not the same epitope or sufficiently adjacent to be bound by a monospecific ligand. The dual specific ligands according to the invention are composed of variable domains which have different specificities, and do not contain mutually complementary variable domain pairs which have the same specificity.

Antigen: A molecule that is bound by a ligand according to the present invention. Typically, antigens are bound by antibody ligands and are capable of raising an antibody response in vivo. It may be a polypeptide, protein, nucleic acid or other molecule. Generally, the dual specific ligands according to the invention are selected for target specificity against a particular antigen. In the case of conventional antibodies and fragments thereof, the antibody binding site defined by the variable loops (L1, L2, L3 and H1, H2, H3) is capable of binding to the antigen.

Epitope: A unit of structure conventionally bound by an immunoglobulin VH/VL pair. Epitopes define the minimum binding site for an antibody, and thus represent the target of specifcity of an antibody. In the case of a single domain antibody, an epitope represents the unit of structure bound by a variable domain in isolation.

Generic ligand: A ligand that binds to all members of a repertoire. Generally, not bound through the antigen binding site as defined above. Non-limiting examples include protein A, protein L and protein G.

Selecting: Derived by screening, or derived by a Darwinian selection process, in which binding interactions are made between a domain and the antigen or epitope or between an antibody and an antigen or epitope. Thus a first variable domain may be selected for binding to an antigen or epitope in the presence or in the absence of a complementary variable domain.

Universal framework: A single antibody framework sequence corresponding to the regions of an antibody conserved in sequence as defined by Kabat ("Sequences of Proteins of Immunological Interest", US Department of Health and Human Services) or corresponding to the human germline immunoglobulin repertoire or structure as defined by Chothia and Lesk, (1987) J. Mol. Biol. 196:910-917. The invention provides for the use of a single framework, or a set of such frameworks, which has been found to permit the derivation of virtually any binding specificity though variation in the hypervariable regions alone.

Homogeneous immunoassay: An immunoassay in which analyte is detected without need for a step of separating bound and un-bound reagents.

Substantially identical (or "substantially homologous"): A first amino acid or nucleotide sequence that contains a sufficient number of identical or equivalent (e.g., with a similar side chain, e.g., conserved amino acid substitutions) amino acid residues or nucleotides to a second amino acid or nucleotide sequence such that the first and second amino acid or nucleotide sequences have similar activities. In the case of antibodies, the second antibody has the same binding specificity and has at least 50% of the affinity of the same.

A "domain antibody" or "dAb" is equivalent to a "single immunoglobulin variable domain polypeptide" or a "single domain antibody polypeptide" as the term is used herein.

As used herein, the phrase "specifically binds" refers to the binding of an antigen by an immunoglobulin variable domain with a dissociation constant (K_{d}) of 1 µM or lower as measured by surface plasmon resonance analysis using, for example, a BIAcore™ surface plasmon resonance system and BIAcore™ kinetic evaluation software (e.g., version 2.1). The affinity or K_{d} for a specific binding interaction is preferably about 500 nM or lower, more preferably about 300 nM or lower.

As used herein, the term "high affinity binding" refers to binding with a K_{d} of less than or equal to 100 nM.

As used herein, the phrase "human single domain antibody polypeptide" refers to a polypeptide having a sequence derived from a human germline immunoglobulin V region. A sequence is "derived from a human germline V region" when the sequence is either isolated from a human individual, isolated from a library of cloned human antibody gene sequences (or a library of human antibody V region gene sequences), or when a cloned human germline V region sequence was used to generate one or more diversified sequences (by random or targeted mutagenesis) that were then selected for binding to a desired target antigen. At a minimum, a human immunoglobulin variable domain has at least 85% amino acid similarity (including, for example, 87%, 90%, 93%, 95%, 97%, 99% or higher similarity) to a naturally-occurring human immunoglobulin variable domain sequence.

Alternatively, or in addition, "a human immunoglobulin variable domain" is a variable domain that comprises four human immunoglobulin variable domain framework regions (W1-FW4), as framework regions are set forth by Kabat et al. (1991, supra). The "human immunoglobulin variable domain framework regions" encompass a) an amino acid sequence of a human framework region, and b) a framework region that comprises at least 8 contiguous amino acids of the amino acid sequence of a human framework region. A human immunoglobulin variable domain can comprise amino acid sequences of FW1-FW4 that are the same as the amino acid sequences of corresponding framework regions encoded by a human germline antibody gene segment, or it can also comprise a variable domain in which FW1-FW4 sequences collectively contain up to 10 amino acid sequence differences, up to 9 amino acid sequence differences, up to 8 amino acid sequence differences, up to 7 amino acid sequence differences, up to 6 amino acid sequence differences, up to 5 amino acid sequence differences, up to 4 amino acid sequence differences, up to 3 amino acid sequence differences, up to 2 amino acid sequence differences, or up to 1 amino acid sequence differences, relative to the amino acid sequences of corresponding framework regions encoded by a human germline antibody gene segment.

A "human immunoglobulin variable domain" as defined herein has the capacity to specifically bind an antigen on its own, whether the variable domain is present as a single immunoglobulin variable domain alone, or as a single immunoglobulin variable domain in association with one or more additional polypeptide sequences. A "human immunoglobulin variable domain" as the term is used herein *does not* encompass a "humanized" immunoglobulin polypeptide, i.e., a non-human (e.g., mouse, camel, etc.) immunoglobulin that has been modified in the constant regions to render it less immunogenic in humans.

As used herein, the phrase "sequence characteristic of immunoglobulin variable domains" refers to an amino acid sequence that is homologous, over 20 or more, 25 or more, 30 or more, 35 or more, 40 or more, 45 or more, or even 50 or more contiguous amino acids, to a sequence comprised by an immunoglobulin variable domain sequence.

As used herein, the term "bi-valent" means that an antigen-binding antibody polypeptide has two antigen-specific binding sites. The epitopes recognized by the antigen-binding sites can be the same or different. When the antibody polypeptide binds two different epitopes (present on different antigens, or, alternatively, on the same antigen) via the respective two antigen-specific binding sites, the antibody polypeptide is termed "dual-specific."

As used herein the term "tetravalent" means that an antigen-binding polypeptide has four antigen-specific binding sites. The epitopes recognized by the antigen-binding sites can be the same or different. A "dual-specific" tetravalent antibody polypeptide has two binding sites for one epitope or antigen and two binding sites for a different epitope or antigen.

As used herein, a "tetravalent, dual-specific antigen-binding polypeptide construct" has a structure analogous to a naturally occurring IgG, in that it has two antigen-binding arms joined by heavy and light chain constant domains. However, unlike naturally-occurring IgG, each ann has two antigen-binding domains, one specific for a first antigen and one specific for a second antigen. In the tetravalent, dual-specific antigen-binding polypeptide constructs described herein, each of the antigen-binding domains is a single domain antibody, i.e., the antigen-binding domains do not pair together to form a single binding site, e.g., as in scFvs.

As used herein, the term "IgG format" refers to an artificial antigen-binding polypeptide with a structure analogous to a naturally-occurring IgG in that the construct has two antigen-binding arms joined by heavy and light chain constant domains that associate with each other. As described herein, an antigen-binding polypeptide in the IgG format is comprised of four polypeptide chains: two copies of a first fusion protein comprising a single-domain antibody polypeptide that binds a first antigen or epitope, fused to an IgG heavy chain constant domain (e.g., one comprising C_{H}1-C_{H}2-C_{H}3); and two copies of a second fusion protein comprising a single domain antibody polypeptide that binds a second antigen, fused to a light chain constant domain (e.g., C_{λ} or C_{κ}). In this format, when co-expressed in a cell, the heavy chain constant domains disulfide bond to each other, and each of these heavy chain constant domains also disulfide bonds to a light chain constant domain. Antigen-binding polypeptides in the IgG format are tetravalent as the term is used herein; the single domain antibodies fused to the constant domains can be selected to bind different antigens (e.g., dAb1, fused to heavy chain constant domain, binds one antigen, dAb2, fused to light chain constant domain binds another antigen), different epitopes on the same antigen (e.g., dAb1, fused to heavy chain constant domain, binds one epitope on an antigen, dAb2, fused to light chain constant domain binds another epitpoe on the same antigen), or, alternatively, all four can bind the same epitope on the same antigen (dAb 1 and dAb2 bind the same epitope on the same antigen).

As used herein, the term "Fab format" refers to a bi-valent antibody polypeptide construct in which one single-domain antibody is fused to a light chain constant domain C_{L} (e.g., C_{λ} or C_{κ}), another single domain antibody is fused to a heavy chain C_{H}1 constant domain, and the respective C_{H}1 and C_{L} constant domains are disulfide bonded to each other. The single domain antibodies can be selected to bind different antigens (generating a dual-specific Fab format), different epitopes on the same antigen (also dual-specific) or the same epitope on the same antigen. An example of a Fab format dual-specific antibody polypeptide comprises, e.g., an anti-TNF-α single domain antibody described herein, fused, for example, to a C_{λ} light chain, and an anti-VEGF single domain antibody as described herein, fused to human heavy chain C_{H}1 constant domain, wherein the two fusion proteins are disulfide bonded to each other via their respective constant domains.
In antibody polypeptide constructs of this format, the antigen-binding domains do not pair together to form a single binding site, e.g., as in scFvs; rather, each single domain antibody can bind antigen on its own, making the construct bi-valent.

By "rheumatoid arthritis" (RA) is meant a disease which involves inflammation in the lining of the joints and/or other internal organs. RA typically affects many different joints. It is typically chronic, and can be a disease of flare-ups. RA is a systemic disease that affects the entire body and is one of the most common forms of arthritis. It is characterized by the inflammation of the membrane lining the joint, which causes pain, stiffness, warmth, redness and swelling. The inflamed joint lining, the synovium, can invade and damage bone and cartilage. Inflammatory cells release enzymes that may digest bone and cartilage. The involved joint can lose its shape and alignment, resulting in pain and loss of movement. Symptoms include inflammation of joints, swelling, difficulty moving and pain. Other symptoms include loss of appetite, fever, loss of energy, anemia. Other features include lumps (rheumatoid nodules) under the skin in areas subject to pressure (e.g., back of elbows). Rheumatoid arthritis is clinically scored on the basis of several clinically accepted scales, such as those described in U.S. 5,698,195, which is incorporated herein by reference. Briefly, clinical response studies can assess the following parameters:
1. Number of tender joints and assessment of pain/tenderness
   The following scoring is used:
   0=No pain/tenderness
   1=Mild pain. The patient says it is tender upon questioning.
   2=Moderate pain. The patient says it is tender and winces.
   3=Severe pain. The patient says it is tender and winces and withdraws.
2. Number of swollen joints
   Both tenderness and swelling are evaluated for each joint separately.
3. Duration of morning stiffness (in minutes)
4. Grip strength
5. Visual analog pain scale (0-10 cm)
6. Patients and blinded evaluators are asked to assess the clinical response to the drug. Clinical response is assessed using a subjective scoring system as follows:
   5=Excellent response (best possible anticipated response)
   4=Good response (less than best possible anticipated response)
   3=Fair response (definite improvement but could be better)
   2=No response (no effect)
   1=Worsening (disease worse)

The cause of rheumatoid arthritis is not yet known. However, it is known that RA is an autoimmune disease, resulting in the immune system attacking healthy joint tissue and causing inflammation and subsequent joint damage. Many people with RA have a certain genetic marker called HLA-DR4.

As used herein, the phrase "TNF-α related disorder" refers to a disease or disorder in which the administration of an agent that neutralizes or antagonizes the function of TNF-α is effective, alone or in conjunction with one or more additional agents or treatments, to treat such disorder as the term "treatment" is defined herein.

As used herein, the terms "treating" or "treatment" refer to a prevention of the onset of disease or a symptom of disease, inhibition of the progression of a disease or a symptom of a disease, or the reversal of disease or a disease symptom.

As used herein, the phrase "prevention of the onset of disease" means that one or more symptoms or measurable parameters of a given disease, e.g., rheumatoid arthritis, does not occur in an individual predisposed to such disease.

As used herein, the phrase "inhibition of the progression of disease" means that treatment with an agent either halts or slows the increase in severity of symptoms of a disease which has already manifested itself in the individual being treated, relative to progression in the absence of such treatment.

As used herein, the phrase "reversal of disease" means that one or more symptoms or measurable parameters of disease improves following administration of an agent, relative to that symptom or parameter prior to such administration. An "improvement" in a symptom or measurable parameter is evidenced by a statistically significant, but preferably at least a 10%, favorable difference in such a measurable parameter.

Measurable parameters can include, for example, both those that are directly measurable as well as those that are indirectly measurable. Non-limiting examples of directly measurable parameters include joint size, joint mobility, arthritic and histopathological scores or indicia and serum levels of an indicator, such as a cytokine. Indirectly measurable parameters include, for example, patient perception of discomfort or lack of mobility or a clinically accepted scale for rating disease severity.

As used herein, an "increase" in a parameter, e.g., an arthritic score or other measurable parameter, refers to a statistically significant increase in that parameter. Alternatively, an "increase" refers to at least a 10% increase. Similarly, a "decrease" in such a parameter refers to a statistically significant decrease in the parameter, or alternatively, to at least a 10% reduction.

As used herein, the term "antagonizes" means that an agent interferes with an activity. Where the activity is that of, for example, TNF-α, VEGF or another biologically active molecule or cytokine, the term encompasses inhibition (by at least 10%) of an activity of that molecule or cytokine, including as non-limiting examples, binding to or interaction with a receptor (in vitro or on a cell surface in culture or in vivo), intracellular signaling, cytotoxicity, mitogenesis, or other downstream effect or process (e.g., gene activation) mediated by that molecule or cytokine. Antagonism encompasses interference with receptor binding by the factor, e.g., TNF, VEGF, etc., as well as interference with the activity of the factor when the factor is bound to a cell-surface receptor.

As used herein, the term "greater than or equal to" means that a value is either equal to another or is greater than that value in a statistically significant manner (p <0.1, preferably p<0.05, more preferably p<0.01). Where efficacy of a composition is compared to that of another composition in, for example, disease treatment or antagonism of receptor binding, the comparison should be made on an equimolar basis.

As used herein, "linked" refers to the attachment of a polymer moiety, such as PEG to an amino acid residue of an antibody polypeptide, e.g., a single domain antibody as described herein. Attachment of a PEG polymer to an amino acid residue of an antibody polypeptide, such as a single domain antibody, is referred to as "PEGylation" and may be achieved using several PEG attachment moieties including, but not limited to N-hydroxylsuccinimide (NHS) active ester, succinimidyl propionate (SPA), maleimide (MAL), vinyl sulfone (VS), or thiol. A PEG polymer, or other polymer, can be linked to an antibody polypeptide at either a predetermined position, or may be randomly linked to the antibody molecule. It is preferred, however, that the PEG polymer be linked to an antibody polypeptide at a predetermined position. A PEG polymer may be linked to any residue in an antibody polypeptide, however, it is preferable that the polymer is linked to either a lysine or cyseine, which is either naturally occurring in an antibody polypeptide, or which has been engineered into an antibody polypeptide, for example, by mutagenesis of a naturally occurring residue in an antibody polypeptide to either a cysteine or lysine. As used herein, "linked" can also refer to the association of two or more antibody single variable domain monomers to form a dimer, trimer, tetramer, or other multimer. dAb monomers can be linked to form a multimer by several methods known in the art including, but not limited to expression of the dAb monomers as a fusion protein, linkage of two or more monomers via a peptide linker between monomers, or by chemically joining monomers after translation either to each other directly or through a linker by disulfide bonds, or by linkage to a di-, tri- or multivalent linking moiety (e.g., a multi-arm PEG).

As used herein, the phrase "directly linked" with respect to a polymer "directly linked" to an antibody polypeptide, e.g., a single variable domain polypeptide, refers to a situation in which the polymer is attached to a residue which naturally part of the variable domain, e.g., not contained within a constant region, hinge region, or linker peptide. Conversely, as used herein, the phrase "indirectly linked" to an antibody polypeptide refers to a linkage of a polymer molecule to an antibody single variable domain wherein the polymer is not attached to an amino acid residue which is part of the naturally occurring variable region (e.g., can be attached to a hinge region). A polymer is "indirectly linked" if it is linked to the antibody polypeptide via a linking peptide, that is the polymer is not attached to an amino acid residue which is a part of the antibody itself. Alternatively a polymer is "indirectly linked" to an antibody polypeptide if it is linked to a C-terminal hinge region of the polypeptide, or attached to any residues of a constant region which may be present as part of the antibody polypeptide.

As used herein, the terms "homology" or "similarity" refer to the degree with which two nucleotide or amino acid sequences structurally resemble each other. As used herein, sequence "similarity" is a measure of the degree to which amino acid sequences share similar amino acid residues at corresponding positions in an alignment of the sequences. Amino acids are similar to each other where their side chains are similar. Specifically, "similarity" encompasses amino acids that are conservative substitutes for each other. A "conservative" substitution is any substitution that has a positive score in the blosum62 substitution matrix (Hentikoff and Hentikoff, 1992, Proc. Natl. Acad. Sci. USA 89: 10915-10919). By the statement "sequence A is n% similar to sequence B" is meant that n% of the positions of an optimal global alignment between sequences A and B consists of identical amino acids or conservative substitutions. Optimal global alignments can be performed using the following parameters in the Needleman-Wunsch alignment algorithm:
For polypeptides:
   Substitution matrix: blosum62.
   Gap scoring function: -A -B*LG, where A=11 (the gap penalty), B=1 (the gap length penalty) and LG is the length of the gap.
For nucleotide sequences:
   Substitution matrix: 10 for matches, 0 for mismatches.
   Gap scoring function: -A -B*LG where A=50 (the gap penalty), B=3 (the gap length penalty) and LG is the length of the gap.

Typical conservative substitutions are among Met, Val, Leu and lle; among Ser and Thr; among the residues Asp, Glu and Asn; among the residues Gln, Lys and Arg; or aromatic residues Phe and Tyr.

As used herein, two sequences are "homologous" or "similar" to each other where they have at least 85% sequence similarity to each other when aligned using either the Needleman-Wunsch algorithm or the "BLAST 2 sequences" algorithm described by Tatusova & Madden, 1999, FEMS Microbiol Lett. 174:247-250. Where amino acid sequences are aligned using the "BLAST 2 sequences algorithm," the Blosum 62 matrix is the default matrix.

"Identity," as known in the art, is a relationship between two or more polypeptide sequences or two or more polynucleotide sequences, as determined by comparing the sequences. In the art, "identity" also means the degree of sequence relatedness between polypeptide or polynucleotide sequences, as the case may be, as determined by the match between strings of such sequences. Percentage identity can be readily calculated by known methods, including but not limited to those described in (Computational Molecular Biology, Lesk, A. M., ed., Oxford University Press, New York, 1988; Biocomputing: Informatics and Genome Projects, Smith, D. W., ed., Academic Press, New York, 1993; Computer Analysis of Sequence Data, Part I, Griffin, A. M., and Griffin, H. G., eds., Humana Press, New Jersey, 1994; Sequence Analysis in Molecular Biology, von Heinje, G., Academic Press, 1987; and Sequence Analysis Primer, Gribskov, M. and Devereux, J., eds., M Stockton Press, New York, 1991; and Carillo, H., and Lipman, D., SIAM J. Applied Math., 48: 1073 (1988). Preferred methods to determine identity are designed to give the largest match between the sequences tested. Methods to determine identity are codified in publicly available computer programs. Preferred computer program methods to determine percentage identity between two sequences include, but are not limited to, the GCG program package (Devereux, J., et al., Nucleic Acids Research 12(1): 387 (1984)), BLASTP, BLASTN, and FASTA (Atschul, S. F. et al., J. Molec. Biol. 215: 403-410 (1990). The BLAST X program is publicly available from NCBI and other sources (BLAST Manual, Altschul, S., et al., NCBI NLM NIH Bethesda, Md. 20894; Altschul, S., et al., J. Mol. Biol. 215: 403-410 (1990). As an illustration, by a polynucleotide having a nucleotide sequence having at least, for example, 95% "identity" to a reference nucleotide sequence of "SEQ ID NO: A" it is intended that the nucleotide sequence of the polynucleotide is identical to the reference sequence except that the polynucleotide sequence may include up to five point mutations per each 100 nucleotides of the reference nucleotide sequence of "SEQ ID NO: A." In other words, to obtain a polynucleotide having a nucleotide sequence at least 95% identical to a reference nucleotide sequence, up to 5% of the nucleotides in the reference sequence may be deleted or substituted with another nucleotide, or a number of nucleotides up to 5% of the total nucleotides in the reference sequence may be inserted into the reference sequence. These mutations of the reference sequence may occur at the 5 or 3 terminal positions of the reference nucleotide sequence or anywhere between those terminal positions, interspersed either individually among nucleotides in the reference sequence or in one or more contiguous groups within the reference sequence. Analogously, by a polypeptide having an amino acid sequence having at least, for example, 95% identity to a reference amino acid sequence of "SEQ ID NO:B" is intended that the amino acid sequence of the polypeptide is identical to the reference sequence except that the polypeptide sequence may include up to five amino acid alterations per each 100 amino acids of the reference amino acid of "SEQ ID NO:
B." In other words, to obtain a polypeptide having an amino acid sequence at least 95% identical to a reference amino acid sequence, up to 5% of the amino acid residues in the reference sequence may be deleted or substituted with another amino acid, or a number of amino acids up to 5% of the total amino acid residues in the reference sequence may be inserted into the reference sequence. These alterations of the reference sequence may occur at the amino or carboxy terminal positions of the reference amino acid sequence or anywhere between those terminal positions, interspersed either individually among residues in the reference sequence or in one or more contiguous groups within the reference sequence.

As used herein, the terms "low stringency," "medium stringency," "high stringency," or "very high stringency conditions" describe conditions for nucleic acid hybridization and washing. Guidance for performing hybridization reactions can be found in Current Protocols in Molecular Biology, John Wiley & Sons, N.Y. (1989), 6.3.1-6.3.6, which is incorporated herein by reference in its entirety. Aqueous and nonaqueous methods are described in that reference and either can be used. Specific hybridization conditions referred to herein are as follows: (1) low stringency hybridization conditions in 6X sodium chloride/sodium citrate (SSC) at about 45°C, followed by two washes in 0.2X SSC, 0.1% SDS at least at 50°C (the temperature of the washes can be increased to 55°C for low stringency conditions); (2) medium stringency hybridization conditions in 6X SSC at about 45°C, followed by one or more washes in 0.2X SSC, 0.1% SDS at 60°C; (3) high stringency hybridization conditions in 6X SSC at about 45°C, followed by one or more washes in 0.2X SSC, 0.1% SDS at 65°C; and preferably (4) very high stringency hybridization conditions are 0.5M sodium phosphate, 7% SDS at 65°C, followed by one or more washes at 0.2X SSC, 1% SDS at 65°C.

As used herein, the phrase "at a concentration of" means that a given polypeptide is dissolved in solution (preferably aqueous solution) at the recited mass or molar amount per unit volume. A polypeptide that is present "at a concentration of X" or "at a concentration of at least X" is therefore exclusive of both dried and crystallized preparations of a polypeptide.

As used herein, the term "repertoire" refers to a collection of diverse variants, for example polypeptide variants which differ in their primary sequence. A library used in the present invention will encompass a repertoire of polypeptides comprising at least 1000 members.

As used herein, the term "library" refers to a mixture of heterogeneous polypeptides or nucleic acids. The library is composed of members, each of which have a single polypeptide or nucleic acid sequence. To this extent, *library* is synonymous with *repertoire.* Sequence differences between library members are responsible for the diversity present in the library. The library may take the form of a simple mixture of polypeptides or nucleic acids, or may be in the form of organisms or cells, for example bacteria, viruses, animal or plant cells and the like, transformed with a library of nucleic acids. Preferably, each individual organism or cell contains only one or a limited number of library members. Advantageously, the nucleic acids are incorporated into expression vectors, in order to allow expression of the polypeptides encoded by the nucleic acids. In a preferred aspect, therefore, a library may take the form of a population of host organisms, each organism containing one or more copies of an expression vector containing a single member of the library in nucleic acid form which can be expressed to produce its corresponding polypeptide member. Thus, the population of host organisms has the potential to encode a large repertoire of genetically diverse polypeptide variants.

As used herein, "polymer" refers to a macromolecule made up of repeating monomeric units, and can refers to asynthetic or naturally occurring polymer such as an optionally substituted straight or branched chain polyalkylene, polyalkenylene, or polyoxyalkylene polymer or a branched or unbranched polysaccharide. A "polymer" as used herein, specifcally refers to an optionally substituted or branched chain poly(ethylene glycol), poly(propylene glycol), or poly(vinyl alcohol) and derivatives thereof.

As used herein, "PEG" or "PEG polymer" refers to polyethylene glycol, and more specifically can refer to a derivitized form of PEG, including, but not limited to N-hydroxylsuccinimide (NHS) active esters of PEG such as succinimidyl propionate, benzotriazole active esters, PEG derivatized with maleimide, vinyl sulfones, or thiol groups. Particular PEG formulations can include PEG-O-CH₂CH₂CH₂-CO₂-NHS; PEG-O-CH₂-NHS; PEG-O-CH₂CH₂-CO₂-NHS; PEG-S-CH₂CH₂-CO-NHS; PEG-O₂CNH-CH(R)-CO₂-NHS; PEG-NHCO-CH₂CH₂-CO-NHS; and PEG-O-CH₂-CO₂-NHS; where R is (CH₂)₄)NHCO₂(mPEG). PEG polymers useful in the invention may be linear molecules, or may be branched wherein multiple PEG moieties are present in a single polymer. Some particularly preferred PEG conformations that are useful in the invention include, but are not limited to the following:

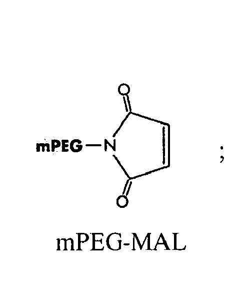

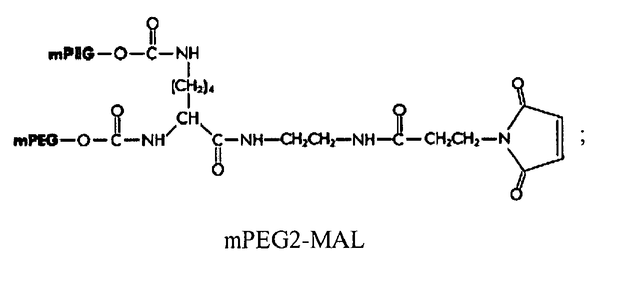

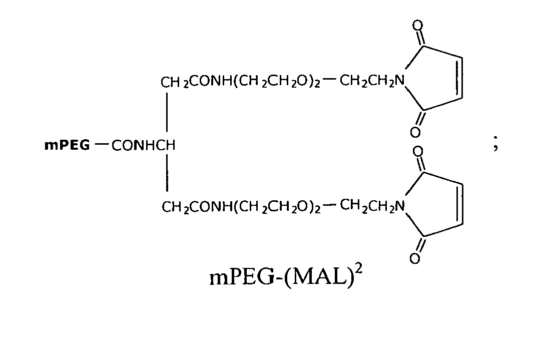

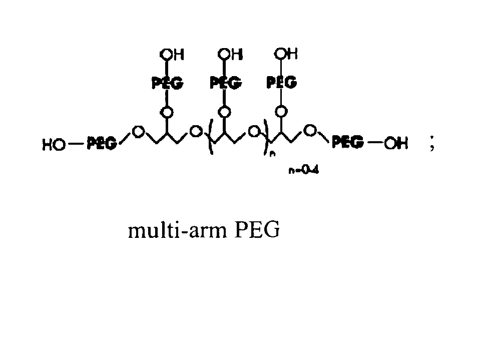

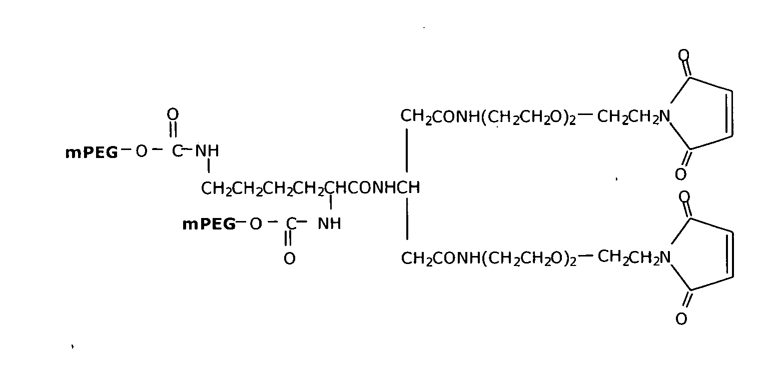

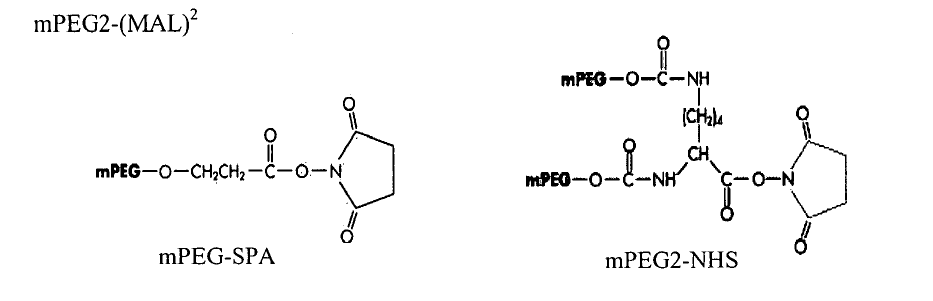

As used herein, a "sulfhydryl-selective reagent" is a reagent which is useful for the attachment of a PEG polymer to a thiol-containing amino acid. Thiol groups on the amino acid residue cysteine are particularly useful for interaction with a sulfhydryl-selective reagent. Sulfhydryl-selective reagents which are useful in the invention include, but are not limited to maleimide, vinyl sulfone, and thiol. The use of sulfhydryl-selective reagents for coupling to cysteine residues is known in the art and may be adapted as needed according to the present invention (See Eg., Zalipsky, 1995, Bioconjug. Chem. 6:150; Greenwald et al., 2000, Crit. Rev. Ther. Drug Carrier Syst. 17:101; Herman et al., 1994, Macromol. Chem. Phys. 195:203).

As used herein, the term "antigen" refers to a molecule that is bound by an antibody or a binding region (e.g., a variable domain) of an antibody. Typically, antigens are capable of raising an antibody response *in vivo.* An antigen can be a peptide, polypeptide, protein, nucleic acid, lipid, carbohydrate, or other molecule. Generally, an immunoglobulin variable domain is selected for target specificity against a particular antigen.

As used herein, the term "epitope" refers to a unit of structure conventionally bound by an immunoglobulin V_{H}/V_{L} pair. Epitopes define the minimum binding site for an antibody, and thus represent the target of specificity of an antibody. In the case of a single domain antibody, an epitope represents the unit of structure bound by a variable domain in isolation.

As used herein, the term "neutralizing," when used in reference to a single immunoglobulin variable domain polypeptide as described herein, means that the polypeptide interferes with a measurable activity or function of the target antigen. A polypeptide is a "neutralizing" polypeptide if it reduces a measurable activity or function of the target antigen by at least 50%, and preferably at least 60%, 70%, 80%, 90%, 95% or more, up to and including 100% inhibition (i.e., no detectable effect or function of the target antigen). This reduction of a measurable activity or function of the target antigen can be assessed by one of skill in the art using standard methods of measuring one or more indicators of such activity or function. As an example, where the target is TNF-α, neutralizing activity can be assessed using a standard L929 cell killing assay or by measuring the ability of a single immunoglobulin variable domain to inhibit TNF-α-induced expression of ELAM-1 on HUVEC, which measures TNF-α-induced cellular activation. Analogous to "neutralizing" as used herein, "inhibit cell cytotoxicity" as used herein refers to a decrease in cell death as measured, for example, using a standard L929 cell killing assay, wherein cell cytotoxicity is inhibited were cell death is reduced by at least 10% or more.

As used herein, a "measurable activity or function of a target antigen" includes, but is not limited to, for example, cell signaling, enzymatic activity, binding activity, ligand-dependent internalization, cell killing, cell activation, promotion of cell survival, and gene expression. One of skill in the art can perform assays that measure such activities for a given target antigen. Preferably, "activity", as used herein, is defined by (1) ND50 in a cell-based assay; (2) affinity for a target ligand, (3) ELISA binding, or (4) a receptor binding assay. Methods for performing these tests are known to those of skill in the art and are described in further detail herein below.

As used herein, "retains activity" refers to a level of activity of the PEG-linked antibody polypeptide, e.g., a single variable domain, which is at least 10% of the level of activity of a non-PEG-linked antibody polypeptide, preferably at least 20%, 30%, 40%, 50%, 60%, 70%, 80% and up to 90%, preferably up to 95%, 98%, and up to 100% of the activity of a non-PEG-linked antibody polypeptide of the same sequence, wherein activity is determined as described herein. More specifically, the activity of a PEG-linked antibody polypeptide compared to a non-PEG linked antibody polypeptide should be determined on an antibody molar basis; that is equivalent numbers of moles of each of the PEG-linked and non-PEG-linked antibody polypeptides should be used in each trial. In determining whether a particular PEG-linked antibody polypeptide "retains activity", it is preferred that the activity of a PEG-linked antibody polypeptide be compared with the activity of the same antibody polypeptide in the absence of PEG.

As used herein, the terms "homodimer," "homotrimer", "homotetramer", and "homomultimer" refer to molecules comprising two, three or more (e.g., four, five, etc.) monomers of a given single immunoglobulin variable domain polypeptide sequence, respectively. For example, a homodimer would include two copies of the same V_{H} sequence. A "monomer" of a single immunoglobulin variable domain polypeptide is a single V_{H} or V_{L} sequence that specifically binds antigen. The monomers in a homodimer, homotrimer, homotetramer, or homomultimer can be linked either by expression as a fusion polypeptide, e.g., with a peptide linker between monomers, or, by chemically joining monomers after translation either to each other directly or through a linker by disulfide bonds, or by linkage to a di-, tri- or multivalent linking moiety. In one embodiment, the monomers in a homodimer, trimer, tetramer, or multimer can be linked by a multi-arm PEG polymer, wherein each monomer of the dimer, trimer, tetramer, or multimer is linked as described above to a PEG moiety of the multi-arm PEG.

As used herein, the terms "heterodimer," "heterotrimer", "heterotetramer", and "heteromultimer" refer to molecules comprising two, three or more (e.g., four, five, six, seven and up to eight or more) monomers of two or more different single immunoglobulin variable domain polypeptide sequence, respectively. For example, a heterodimer would include two V_{H} sequences, such as V_{H1} and V_{H2}, or may alternatively include a combination of V_{H} and V_{L}. Similar to a homodimer, trimer, or tetramer, the monomers in a heterodimer, heterotrimer, heterotetramer, or heteromultimer can be linked either by expression as a fusion polypeptide, e.g., with a peptide linker between monomers, or, by chemically joining monomers after translation either to each other directly or through a linker by disulfide bonds, or by linkage to a di-, tri- or multivalent linking moiety. In one embodiment, the monomers in a heterodimer, trimer, tetramer, or multimer can be linked by a multi-arm PEG polymer, wherein each monomer of the dimer, trimer, tetramer, or multimer is linked as described above to a PEG moiety of the multi-arm PEG.

As used herein, the term "half-life" refers to the time taken for the serum concentration of a ligand (e.g., an antibody polypeptide, such as a single immunoglobulin variable domain) to reduce by 50%, *in vivo,* for example due to degradation of the ligand and/or clearance or sequestration of the ligand by natural mechanisms. The antibody polypeptides are stabilised *in vivo* and their half-life increased by binding to molecules which resist degradation and/or clearance or sequestration, such as PEG. The half-life of an antibody polypeptide, e.g., a dAb) is increased if its functional activity persists, *in vivo,* for a longer period than a similar dAb which is not linked to a PEG polymer. Typically, the half life of a PEGylated dAb is increased by 10%, 20%, 30%, 40%, 50% or more relative to a non-PEGylated dAb. Increases in the range of 2x, 3x, 4x, 5x, 10x, 20x, 30x, 40x, 50x or more of the half life are possible. Alternatively, or in addition, increases in the range of up to 30x, 40x, 50x, 60x, 70x, 80x, 90x, 100x, 150x of the half life are possible. According to the invention, a PEG-linked antibody single variable domain has a half-life of between 0.25 and 170 hours, preferably between 1 and 100 hours, more preferably between 30 and 100 hours, and still more preferably between 50 and 100 hours, and up to 170, 180, 190, and 200 hours or more.

As used herein, "resistant to degradation" or "resists degradation" when used with respect to a PEG or other polymer linked dAb monomer or multimer means that the PEG- or other polymer-linked dAb monomer or multimer is degraded by no more than 10% when exposed to pepsin at pH 2.0 for 30 minutes and preferably not degraded at all. With specific reference to a PEG- or other polymer-linked dAb multimer (e.g., hetero- or homodimer, trimer, tetramer, etc) a molecule that is resistant to degradation is degraded by less than 5%, and is preferably not degraded at all in the presence of pepsin at pH 2.0 for 30 minutes.

As used herein, "hydrodynamic size" refers to the apparent size of a molecule (e.g., a protein molecule) based on the diffusion of the molecule through an aqueous solution. The diffusion, or motion of a protein through solution can be processed to derive an apparent size of the protein, where the size is given by the "Stokes radius" or "hydrodynamic radius" of the protein particle. The "hydrodynamic size" of a protein depends on both mass and shape (conformation), such that two proteins having the same molecular mass may have differing hydrodynamic sizes based on the overall conformation of the protein. The hydrodynamic size of a PEG-linked antibody polypeptide, e.g., a single variable domain (including antibody variable domain multimers as described herein), can be in the range of 24 kDa to 500 kDa; 30 to 500 kDa; 40 to 500 kDa; 50 to 500 kDa; 100 to 500 kDa; 150 to 500 kDa; 200 to 500 kDa; 250 to 500 kDa; 300 to 500 kDa; 350 to 500 kDa; 400 to 500 kDa and 450 to 500 kDa. Preferably the hydrodynamic size of a PEGylated dAb of the invention is 30 to 40 kDa; 70 to 80 kDa or 200 to 300 kDa. Where an antibody variable domain multimer is desired for use in imaging applications, the multimer should have a hydrodynamic size of between 50 and 100 kDa. Alternatively, where an antibody single domain multimer is desired for therapeutic applications, the multimer should have a hydrodynamic size of greater than 200 kDa.

### BRIEF DESCRIPTION OF THE FIGURES

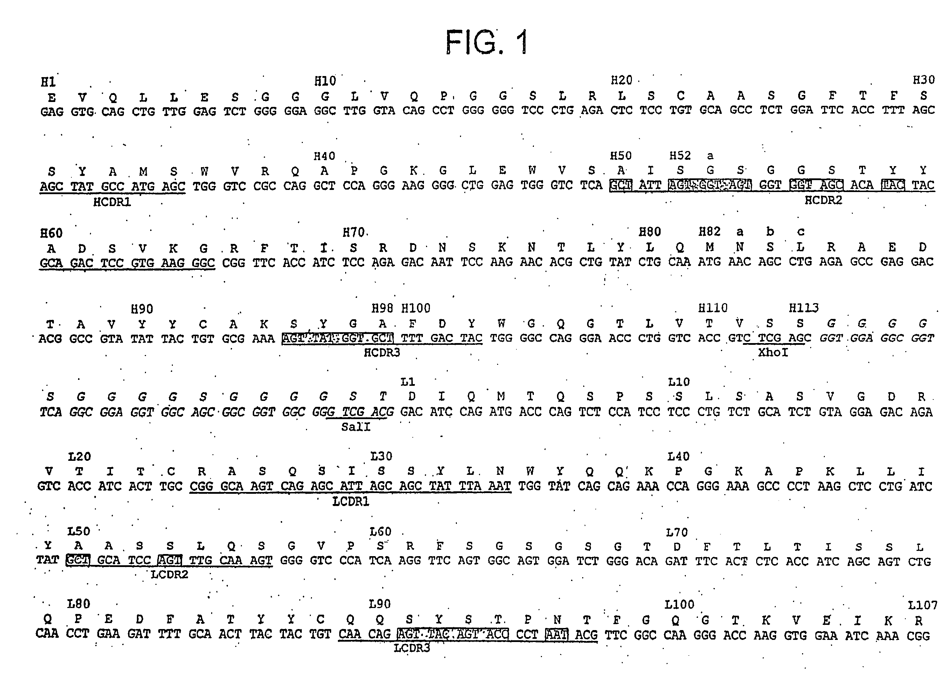

**Figure 1** shows the diversification of VH/HSA at positions H50, H52, H52a, H53, H55, H56, H58, H95, H96, H97, H98 (DVT or NNK encoded respectively) which are in the antigen binding site of VH HSA. The sequence of VK is diversified at positions L50, L53. Amino acid sequence, SEQ ID NO: 219; nucleotide sequence, top strand, SEQ ID NO: 220.

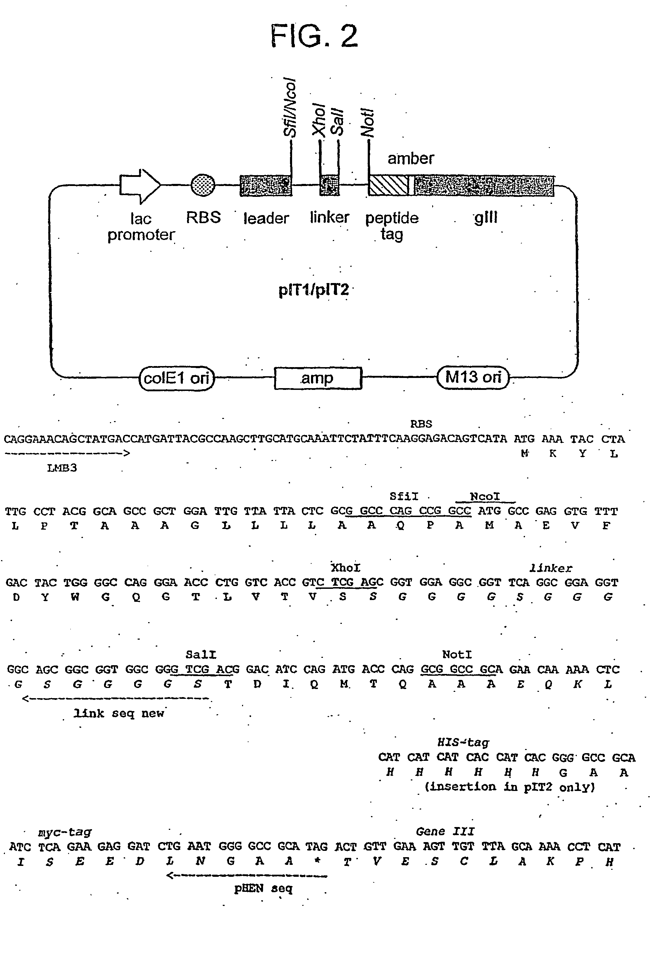

**Figure 2** shows Library 1: Germline VK/DVT VH; Library 2: Germline VK/NNK VH; Library 3: Germline VH/DVT VK; Library 4: Germline VH/NNK VK. In phage display/ScFv format. These libraries were pre-selected for binding to generic ligands protein A and protein L so that the majority of the clones and selected libraries are functional. Libraries were selected on HSA (first round) and β-gal (second round) or HSA β-gal selection or on β-gal (first round) and HSA (second round) β-gal HSA selection. Soluble scFv from these clones of PCR are amplified in the sequence. One clone encoding a dual specific antibody K8 was chosen for further work. Nucleotide sequence:
   SEQ ID NO: 221; Amino acid sequence: SEQ ID NO: 222.

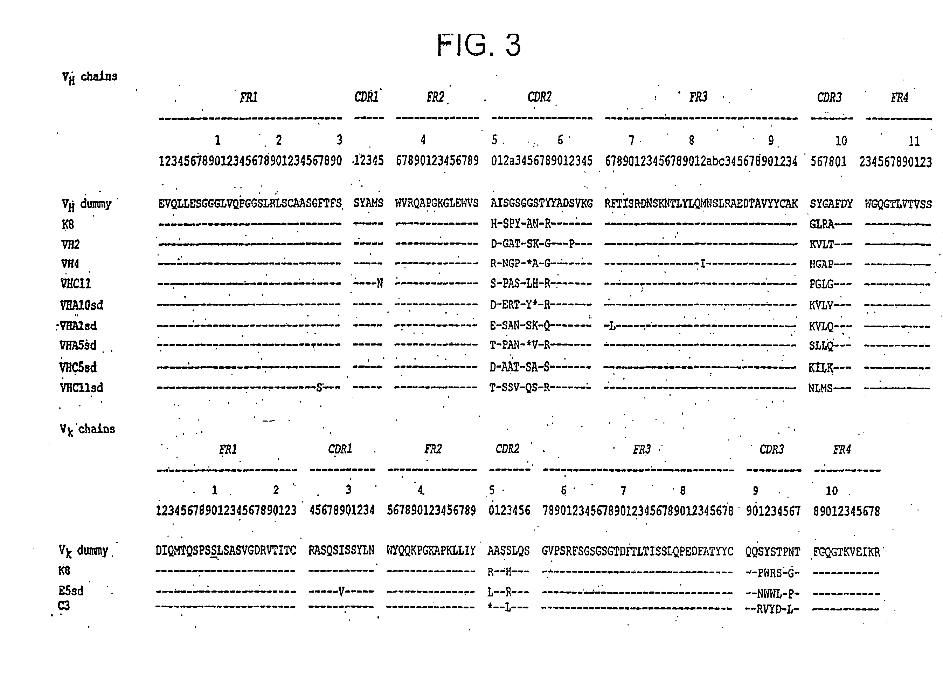

**Figure 3** shows an alignment of VH chains and VK chains. V_{H} dummy, SEQ ID NO: 1; K8, SEQ ID Nos 223 (VH) and 232 (VK); VH2, SEQ ID NO: 224; VH4, SEQ ID NO: 225; VHC11, SEQ ID NO: 226; VHA10sd, SEQ ID NO: 227; VHA1sd, SEQ ID NO: 228; VHA5sd, SEQ ID NO: 229; VHC5sd, SEQ ID NO: 230; VHC11sd, SEQ ID NO: 231; V_{κ} dummy, SEQ ID NO: 3; E5sd, SEQ ID NO: 233; C3, SEQ ID NO: 234.

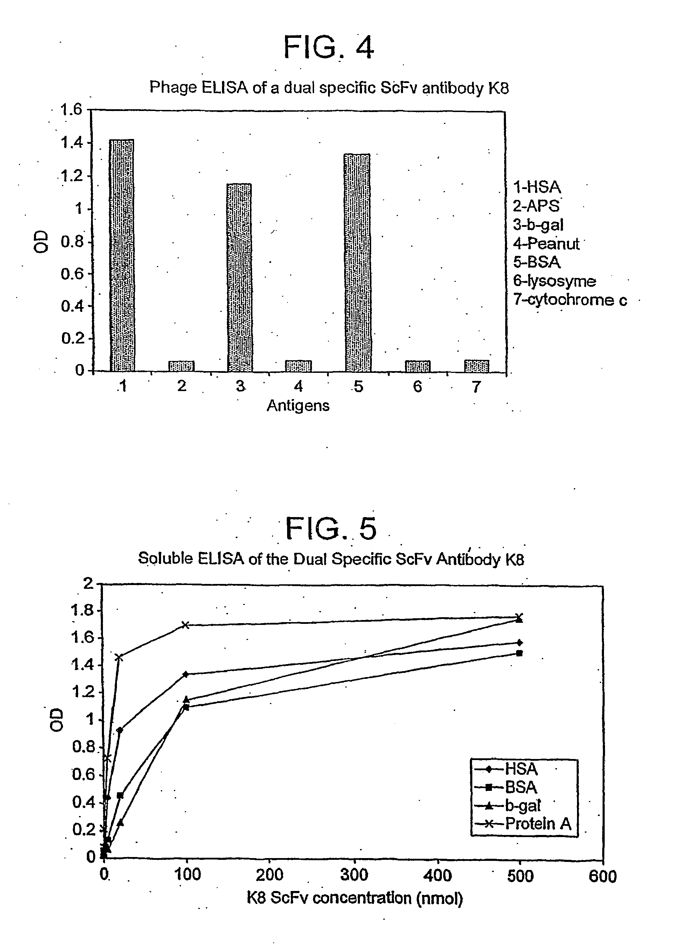

**Figure 4** shows the characterization of the binding properties of the K8 antibody, the binding properties of the K8 antibody characterized by monoclonal phage ELISA, the dual specific K8 antibody was found to bind HSA and β-gal and displayed on the surface of the phage with absorbent signals greater than 1.0. No cross reactivity with other proteins was detected.
**Figure 5** shows soluble scFv ELISA performed using known concentrations of the K8 antibody fragment. A 96-well plate was coated with 100µg of HSA, BSA and β-gal at 10µg/ml and 100µg/ml of Protein A at 1µg/ml concentration. 50µg of the serial dilutions of the K8 scFv was applied and the bound antibody fragments were detected with Protein L-HRP. ELISA results confirm the dual specific nature of the K8 antibody.

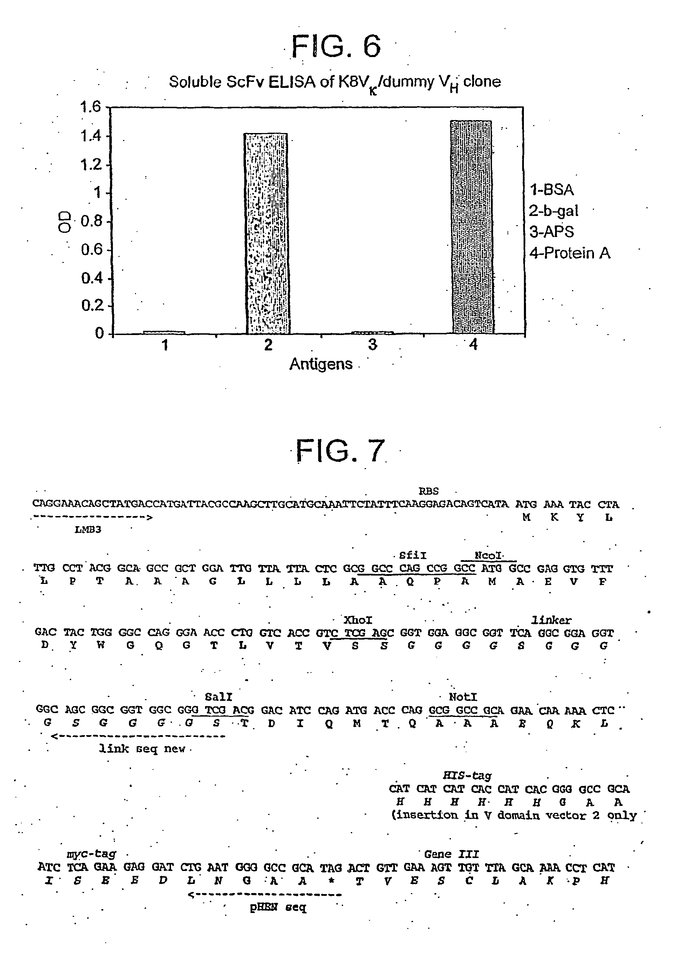

**Figure 6** shows the binding characteristics of the clone K8VK/dummy VH analysed using soluble scFv ELISA. Production of the soluble scFv fragments was induced by IPTG as described by Harrison et al, Methods Enzymol. 1996;267:83-109 and the supernatant containing scFv assayed directly. Soluble scFv ELISA is performed as described in example 1 and the bound scFvs were detected with Protein L-HRP. The ELISA results revealed that this clone was still able to bind β-gal, whereas binding BSA was abolished.
**Figure 7** shows the sequence of variable domain vectors 1 and 2. Nucleotide sequence: SEQ ID NO: 221; Amino acid sequence: SEQ ID NO: 222.

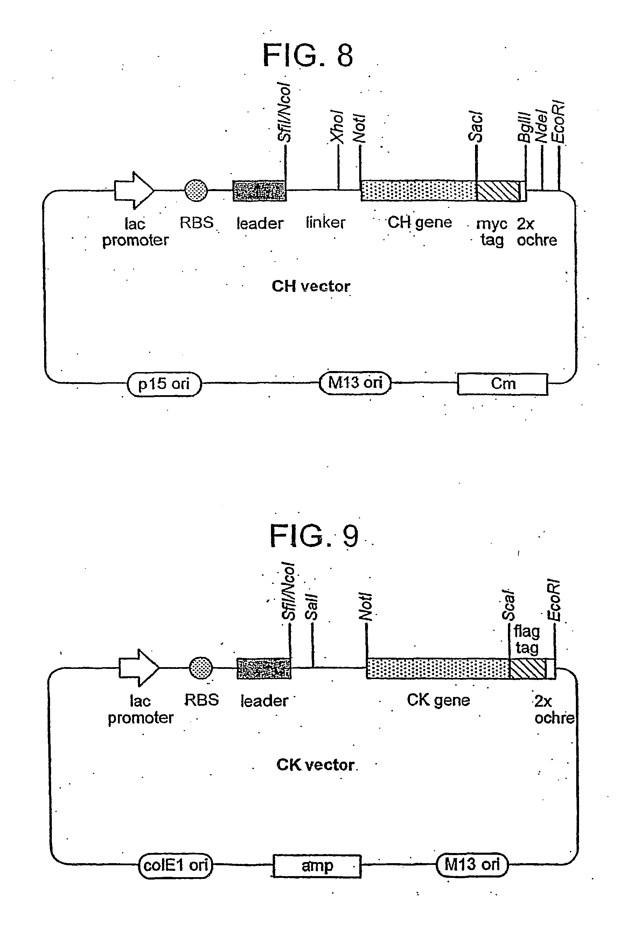

**Figure 8** is a map of the CH vector used to construct a VH1/VH2 multipsecific ligand.
**Figure 9** is a map of the VK vector used to construct a VK1/VK2 multispecific ligand.

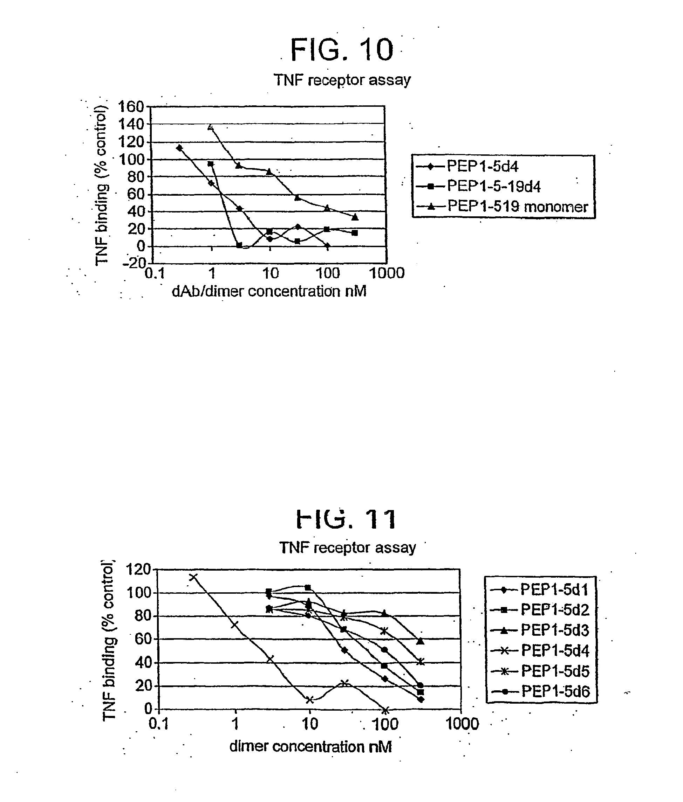

**Figure 10** TNF receptor assay comparing TAR1-5 dimer 4, TAR1-5-19 dimer 4 and TAR1-5-19 monomer.
**Figure 11** TNF receptor assay comparing TAR1-5 dimers 1-6. All dimers have been FPLC purified and the results for the optimal dimeric species are shown.

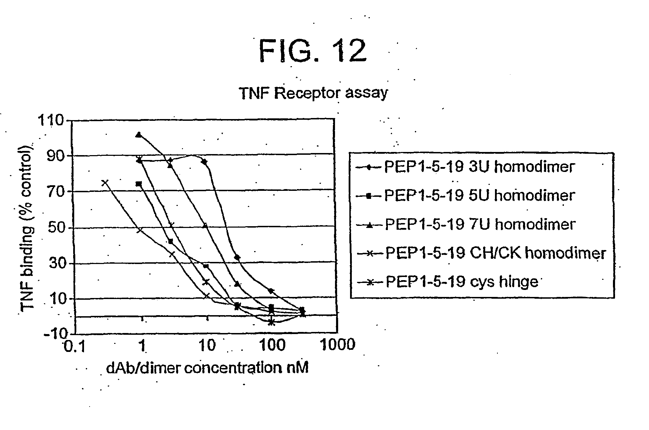

**Figure 12** TNF receptor assay of TAR1-5 19 homodimers in different formats: dAb linker-dAb format with 3U, 5U or 7U linker, Fab format and cysteine hinge linker format.

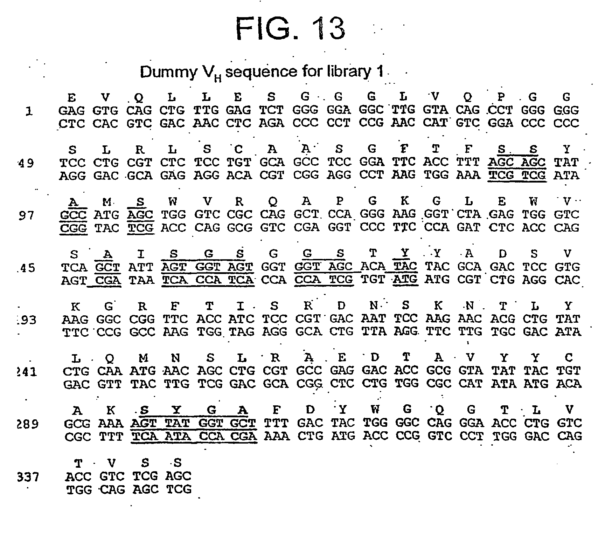

**Figure 13** Dummy VH sequence for library 1. The sequence of the VH framework based on germline sequence DP47 - JH4b. Positions where NNK randomisation (N=A or T or C or G nucleotides; K = G or T nucleotides) has been incorporated into library 1 are indicated in bold underlined text. Amino acid sequence, SEQ ID NO: 1; nucleotide sequence, top strand, SEQ ID NO: 2.

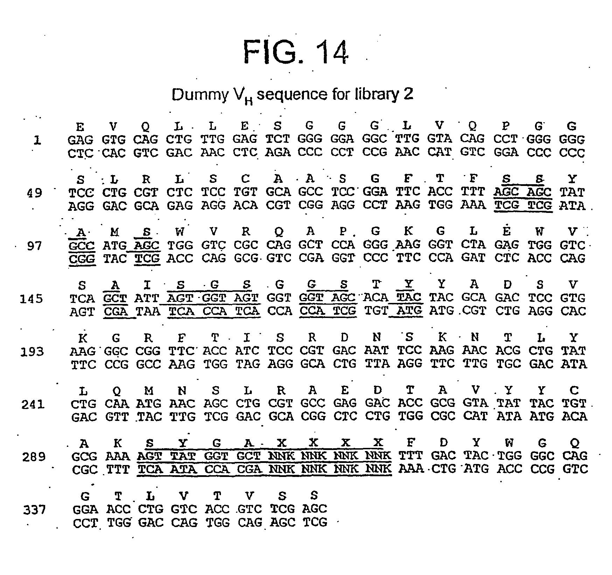

**Figure 14** Dummy VH sequence for library 2. The sequence of the VH framework based on germline sequence DP47 - JH4b. Positions where NNK randomization (N=A or T or C or G nucleotides; K = G or T nucleotides) has been incorporated into library 2 are indicated in bold underlined text. Amino acid sequence, SEQ ID NO: 235; nucleotide sequence, top strand, SEQ ID NO: 236.

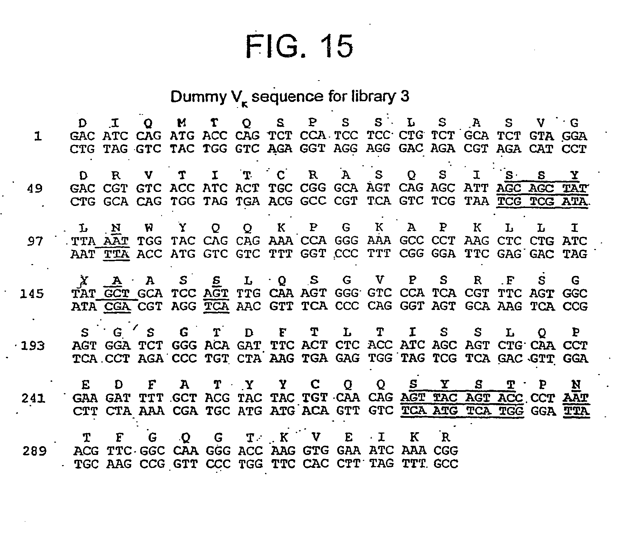

**Figure 15** Dummy VK sequence for library 3. The sequence of the VK framework 5 based on germline sequence DPK9 - J K1. Positions where NNK randomization (N=A or T or C or G nucleotides; K = G or T nucleotides) has been incorporated into library 3 are indicated in bold underlined text. Amino acid sequence, SEQ ID NO: 3; nucleotide sequence, SEQ ID NO: 4.

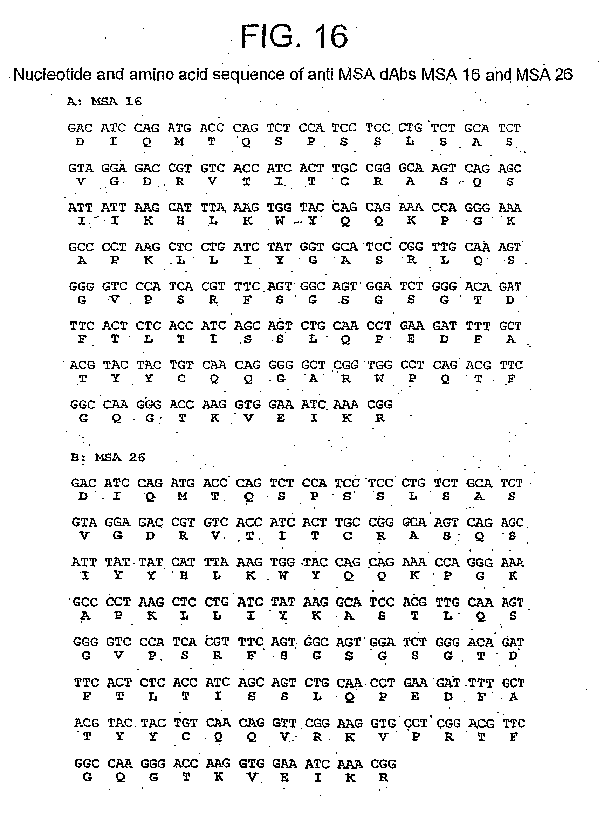

**Figure 16** Nucleotide and amino acid sequence of anti MSA dAbs MSA 16 and MSA 26. MSA 16: Amino acid sequence, SEQ ID NO: 237; nucleotide sequence, SEQ ID NO: 238. MSA 26: Amino acid sequence, SEQ ID NO: 239; nucleotide sequence, SEQ ID NO: 240.

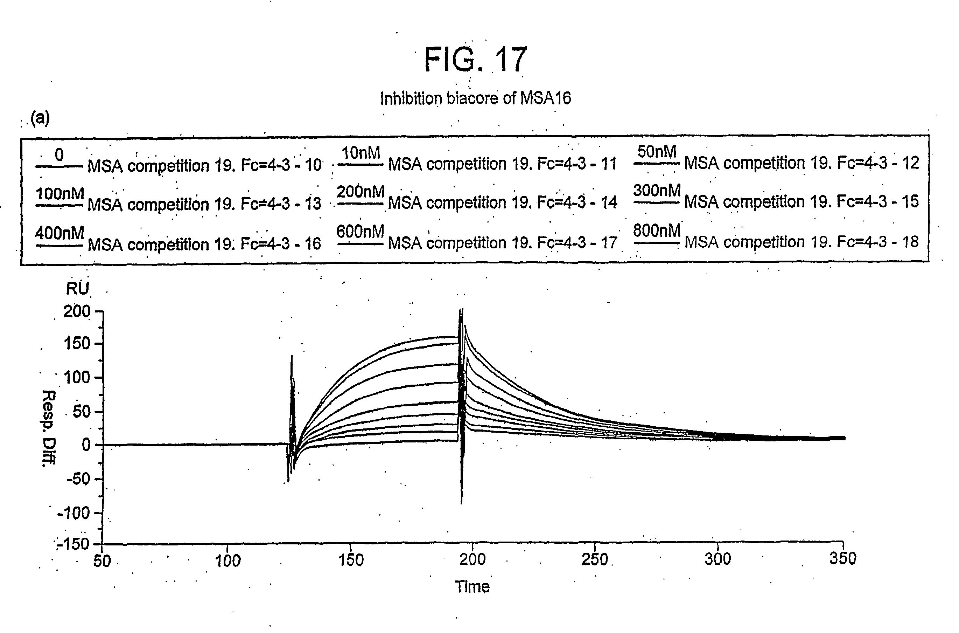

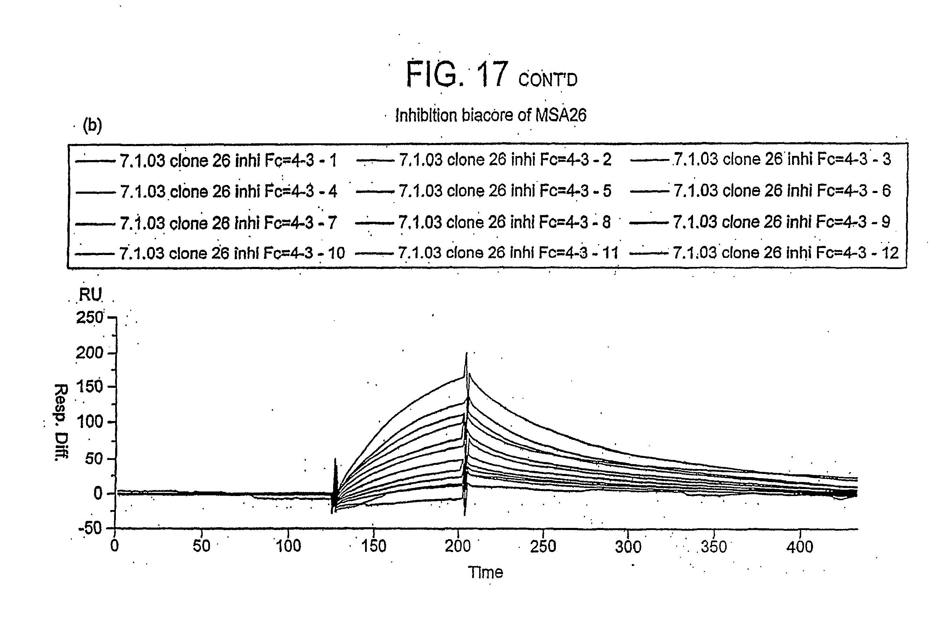

**Figure 17** Inhibition Biacore of MSA 16 and 26. Purified dAbs MSA16 and MSA26 were analysed by inhibition biacore to determine K_{d}. Briefly, the dAbs were tested to determine the concentration of dAb required to achieve 200RUs of response on a biacore CM5 chip coated with a high density of MSA. Once the required concentrations of dAb had been determined, MSA antigen at a range of concentrations around the expected K_{d} was premixed with the dAb and incubated overnight. Binding to the MSA coated biacore chip of dAb in each of the premixes was then measured at a high flow-rate of 30µl/minute.

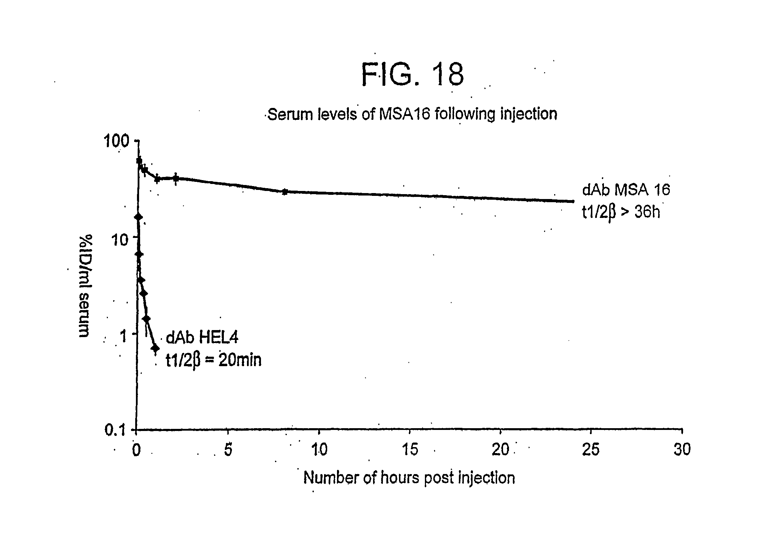

**Figure 18** Serum levels of MSA16 following injection. Serum half life of the dAb MSA16 was determined in mouse. MSA16 was dosed as single i.v. injections at approx 1.5mg/kg into CD1 mice. Modelling with a 2 compartment model showed MSA16 had a t1/2β of 0.98hr, a t1/2β of 36. 5hr and an AUC of 913hr.mg/ml. MSA16 had a considerably lengthened half life compared with HEL4 (an anti-hen egg white lysozyme dAb) which had a t1/2β of 0.06hr and a t1/2β of 0.34hr.

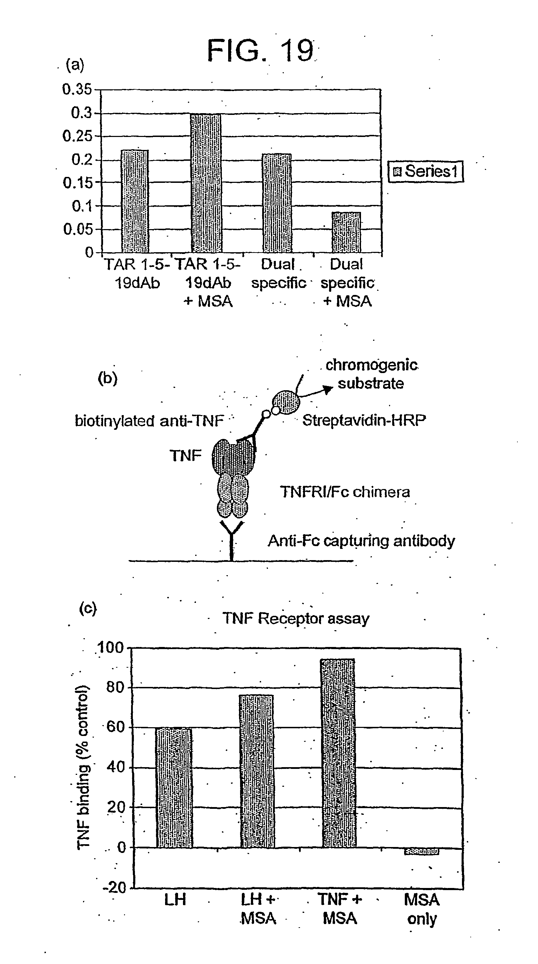

**Figure 19** ELISA (a) and TNF receptor assay (c) showing inhibition of TNF binding with a Fab-like fragment comprising MSA26Ck and TAR1-5-19CH. Addition of MSA with the Fab-like fragment reduces the level of inhibition. An ELISA plate coated with 1ug/ml TNF-α was probed with dual specific VK CH and VK CK Fab like fragment and also with a control TNF-α binding dAb at a concentration calculated to give a similar signal on the ELISA. Both the dual specific and control dAb were used to probe the ELISA plate in the presence and in the absence of 2mg/ml MSA. The signal in the dual specific well was reduced by more than 50% but the signal in the dAb well was not reduced at all (see Figure 19a). The same dual specific protein was also put into the receptor assay with and without MSA and competition by MSA was also shown (see Figure 19c). This demonstrates that binding of MSA to the dual specific is competitive with binding to TNF-α.

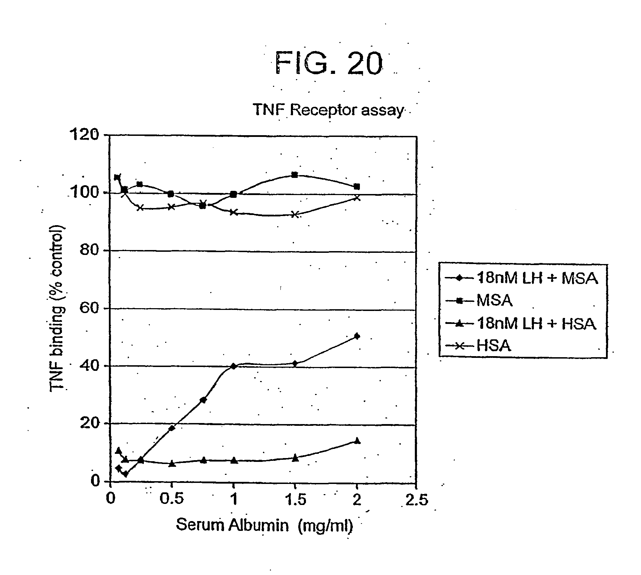

**Figure 20** TNF receptor assay showing inhibitor of TNF binding with a disulphide bonded heterodimer of TAR1-5-19 dAb and MSA16 dAb. Addition of MSA with the dimer reduces the level of inhibitor in a dose dependent manner. The TNF receptor assay was conducted in the presence of a constant concentration of heterodimer (18 nM) and a dilution series of MSA and HSA. The presence of HSA at a range of concentrations (up to 2 mg/ml) did not cause a reduction in the ability of the dimer to inhibit TNF-α. However, the addition of MSA caused a dose dependent reduction in the ability of the dimer to inhibit TNF-α.This demonstrates that MSA and TNF-α compete for binding to the cys bonded TAR1-5-19, MSA16 dimer. MSA and HSA alone did not have an effect on the TNF binding level in the assay.

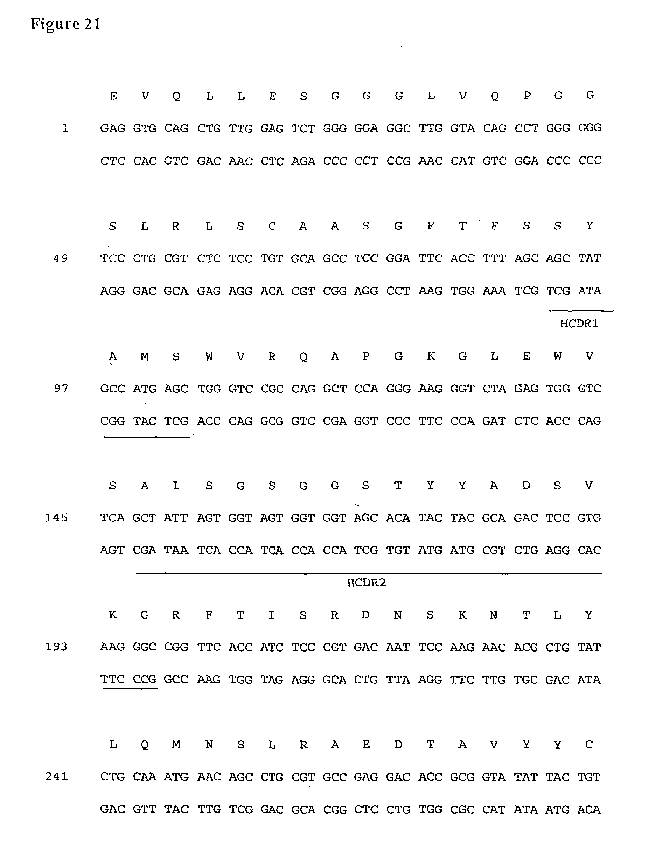

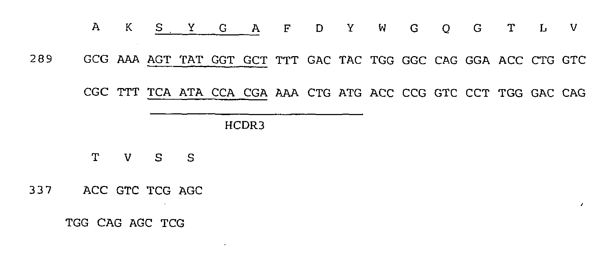

**Figure 21** Shows the polynucleotide and amino acid sequences of human germline framework segment DP47 (see also Figure 1). Amino acid sequence is SEQ ID NO: 1; polynucleotide sequence of top strand is SEQ ID NO: 2.

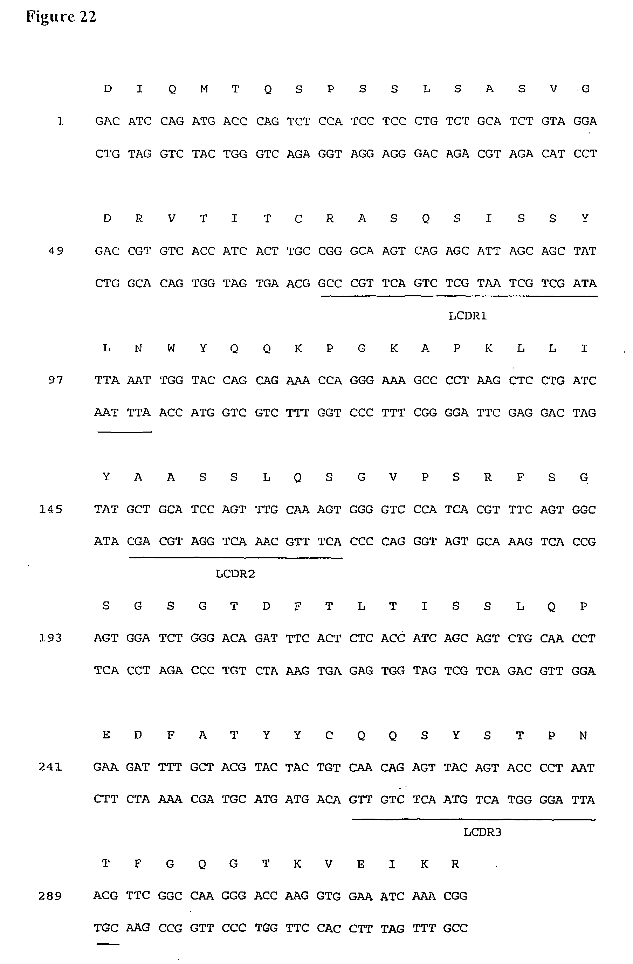

**Figure 22** Shows the polynucleotide and amino acid sequences of human germline framework segment DPK9. Amino acid sequence is SEQ ID NO: 3; polynucleotide sequence of top strand is SEQ ID NO: 4.

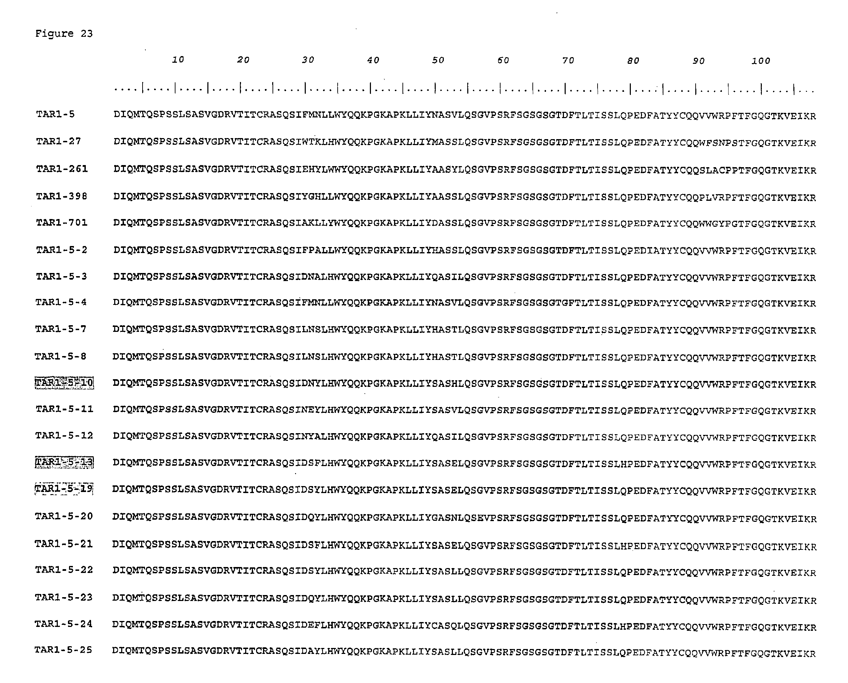

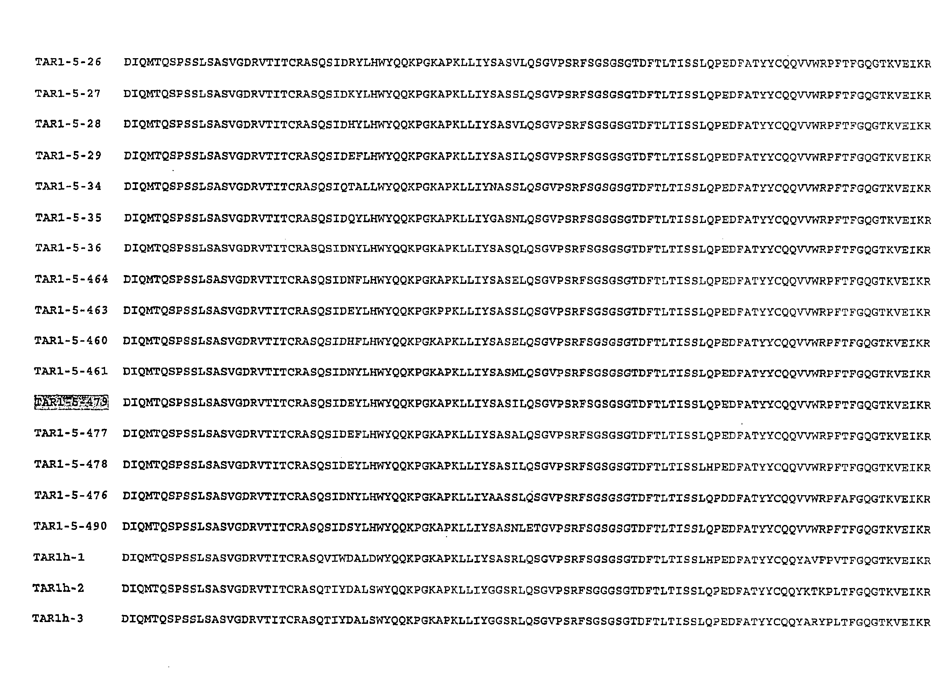

**Figure 23** Shows amino acid sequences for the TAR1 clones described herein (see, e.g., Example 13). TAR1-5, SEQ ID NO: 241; TAR1-27, SEQ ID NO: 242; TAR1-261, SEQ ID NO: 243; TAR1-398, SEQ ID NO: 244; TAR1-701, SEQ ID NO: 245; TAR1-5-2, SEQ ID NO: 246; TAR1-5-3, SEQ ID NO: 247; TAR1-5-4, SEQ ID NO: 248; TAR1-5-7, SEQ ID NO: 249; TAR1-5-8, SEQ ID NO: 250; TAR1-5-10, SEQ ID NO: 251; TAR1-5-11, SEQ ID NO: 252; TAR1-5-12, SEQ ID NO: 253; TAR1-5-13, SEQ ID NO: 254; TAR1-5-19, SEQ ID NO: 191; TAR1-5-20, SEQ ID NO: 255; TAR1-5-21, SEQ ID NO: 256; TAR1-5-22, SEQ ID NO: 257; TAR1-5-23, SEQ ID NO: 258; TAR1-5-24, SEQ ID NO: 259; TAR1-5-25, SEQ ID NO: 260; TAR1-5-26, SEQ ID NO: 261; TAR1-5-27, SEQ ID NO: 262; TAR1-5-28, SEQ ID NO: 263; TAR1-5-29, SEQ ID NO: 264; TAR1-5-34, SEQ ID NO: 265; TAR1-5-35, SEQ ID NO: 266; TAR1-5-36, SEQ ID NO: 267; TAR1-5-464, SEQ ID NO: 268; TAR1-5-463, SEQ ID NO: 269; TAR1-5-460, SEQ ID NO: 270; TAR1-5-461, SEQ ID NO: 271; TAR1-5-479, SEQ ID NO: 272; TAR1-5-477, SEQ ID NO: 273; TAR1-5-478, SEQ ID NO: 274; TAR1-5-476, SEQ ID NO: 275; TAR1-5-490, SEQ ID NO: 276; TAR1h-1, SEQ ID NO: 277; TAR1h-2, SEQ ID NO: 278; TAR1h-3, SEQ ID NO: 279.

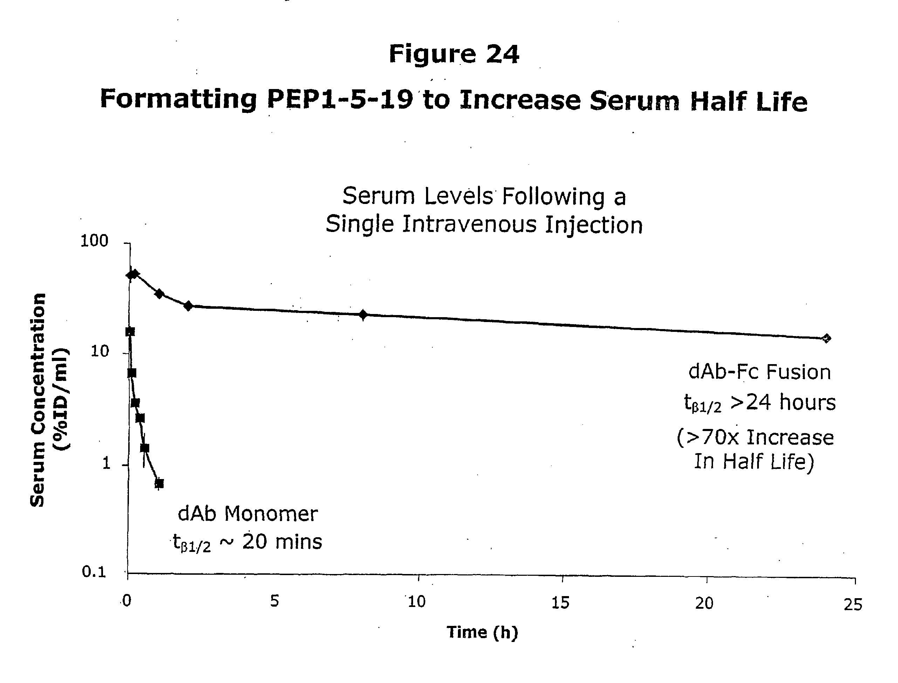

**Figure 24** Shows a comparison of serum half lives of TAR1-5-19 in either dAb monomer format or Fc fusion format following a single intravenous injection.

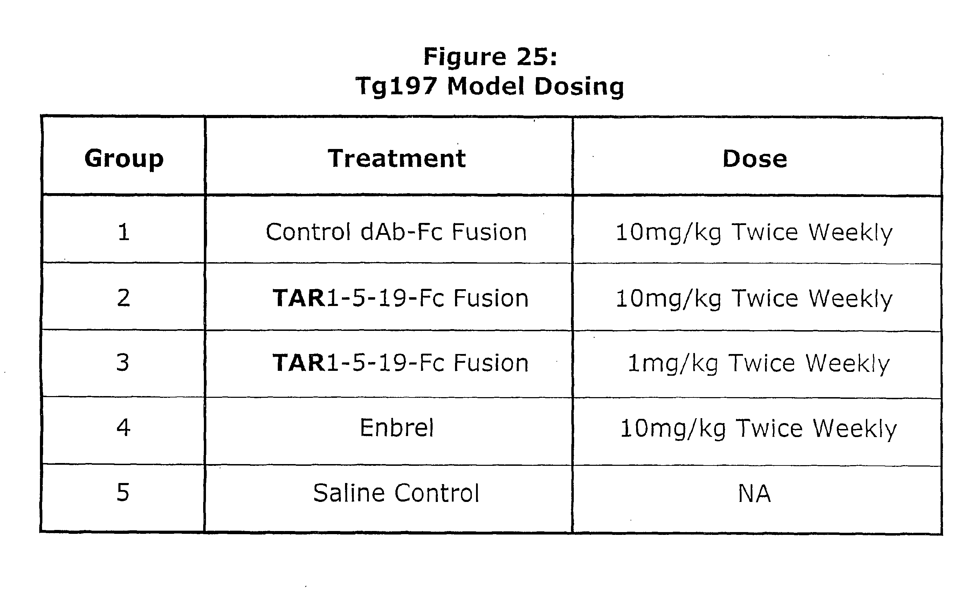

**Figure 25** Summarizes the dosages and timing of dAb constructs administered in a series of Tg197 model trials using TAR1-5-19.

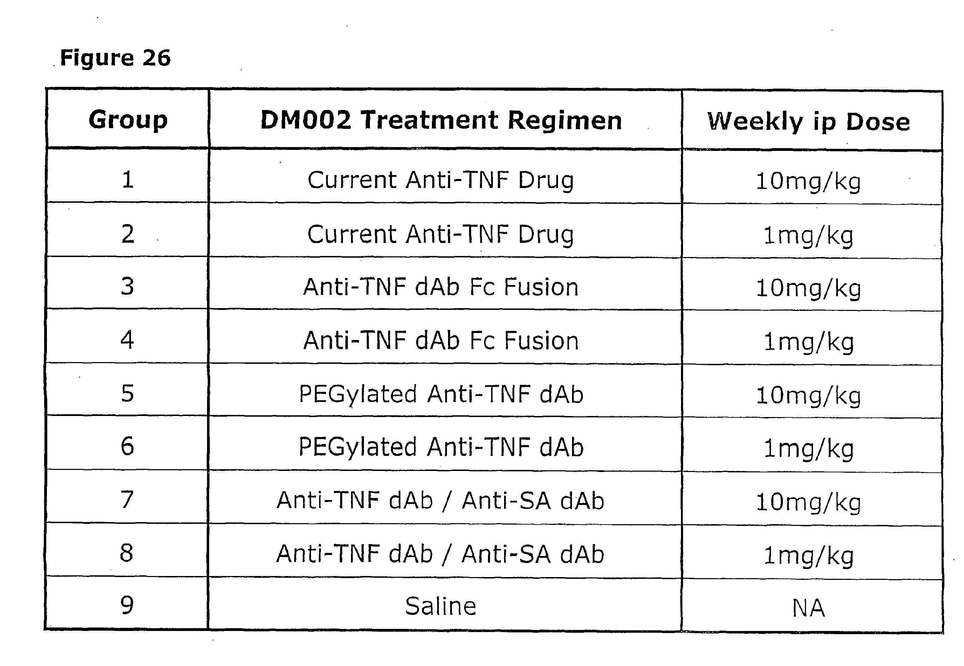

**Figure 26** Summarizes the weekly dosages of differing formats of the TAR1-5-19 dAb (Fc fusion, PEGylated, Anti-TNF/Anti-SA dual specific) used in studies in the Tg197 mouse RA model.

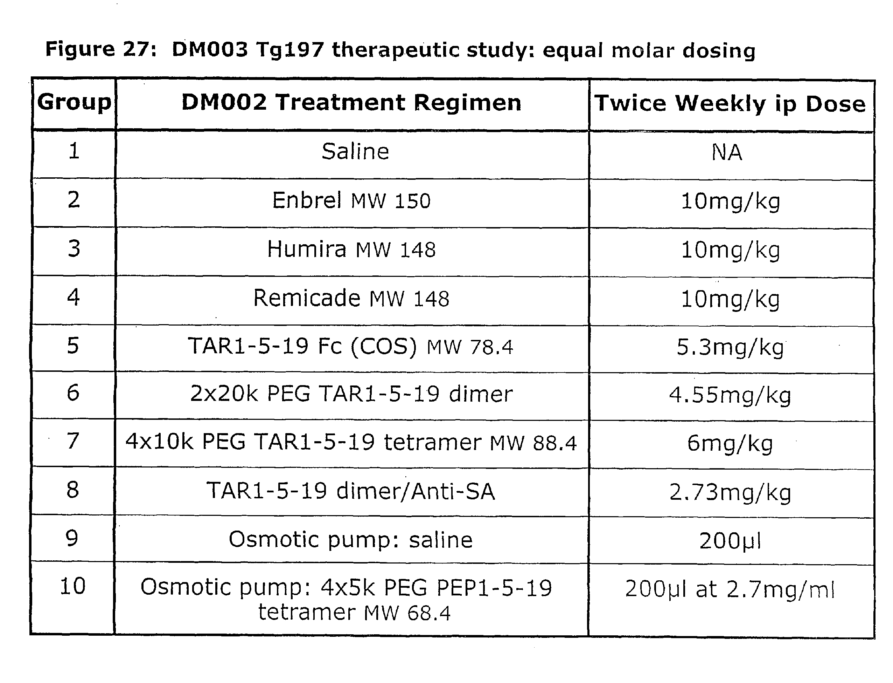

**Figure 27** Summarizes the format (Fc fusion, PEG dimer, PEG tetramer, Anti-TNF/Anti-SA dual specific), delivery mode and dosage of anti-TNF dAb construct administered in a Tg197 mouse RA model study comparing the efficacy of the anti-TNF dAb constructs to the efficacy of the current anti-TNF products.

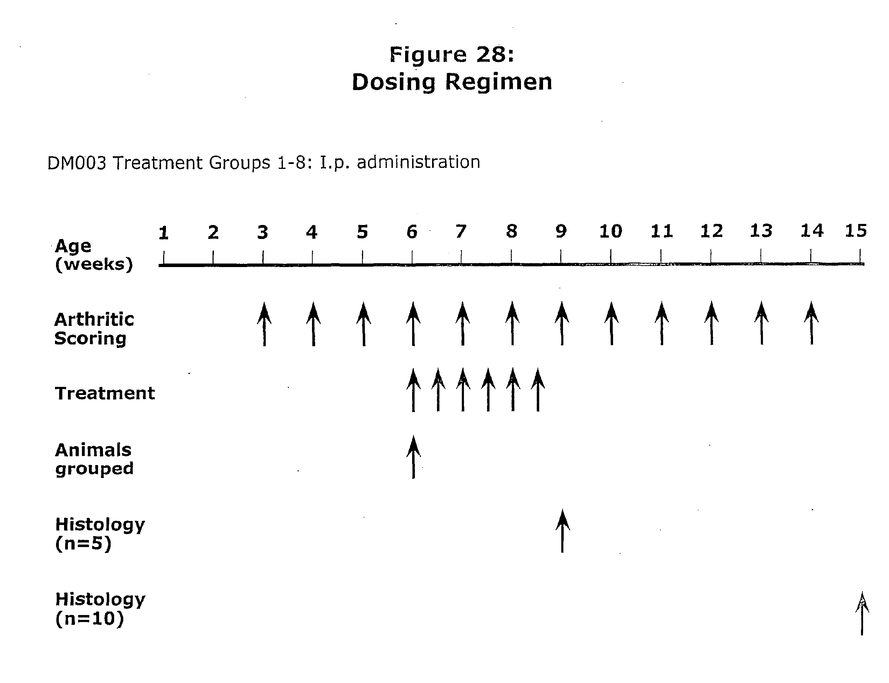

**Figure 28** Shows the dosing and scoring regimen for a study examining the efficacy of anti-TNF dAbs against established disease symptoms in the Tg197 mouse RA model.

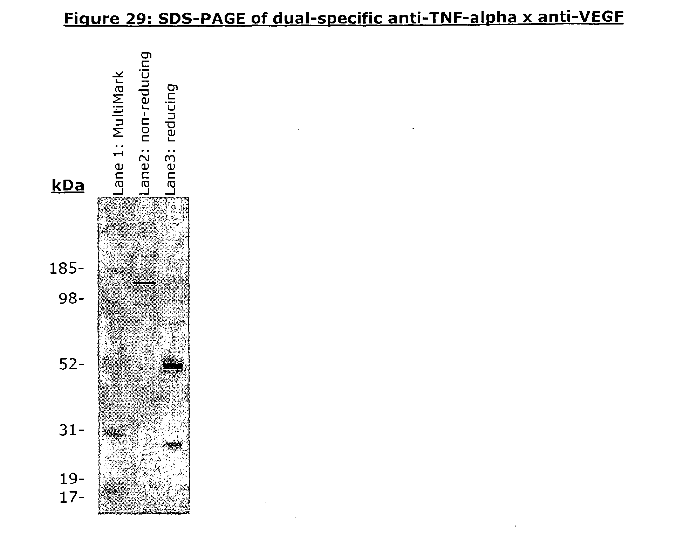

**Figure 29** Shows an SDS PAGE gel analysis for an IgG-like dual specific antibody comprising a V_{κ} variable domain specific for human VEGF fused to human IgG1 constant domain and a V_{κ} variable domain specific for human TNF-α fused to human C_{κ} constant domain. Lane 1: InVitrogen Multimark MW markers. Lane 2: anti-TNF x anti-VEGF dual specific antibody in 1X non-reducing loading buffer. Lane 3: anti-TNF x anti-VEGF dual specific antibody in 1X loading buffer with 10 mM β-mercaptoethanol.

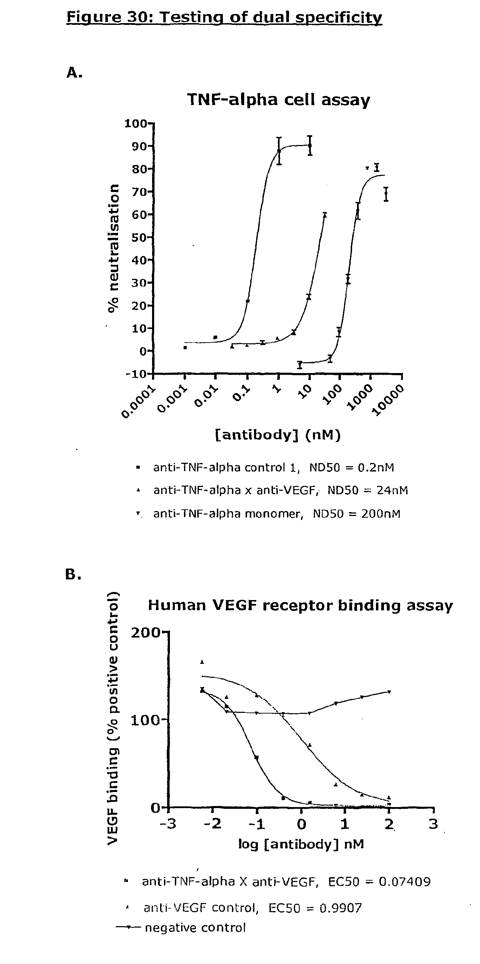

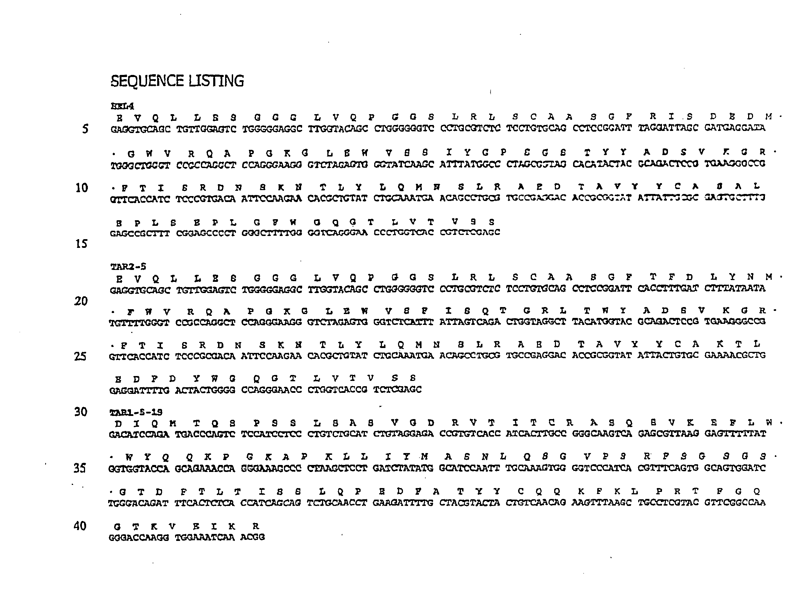

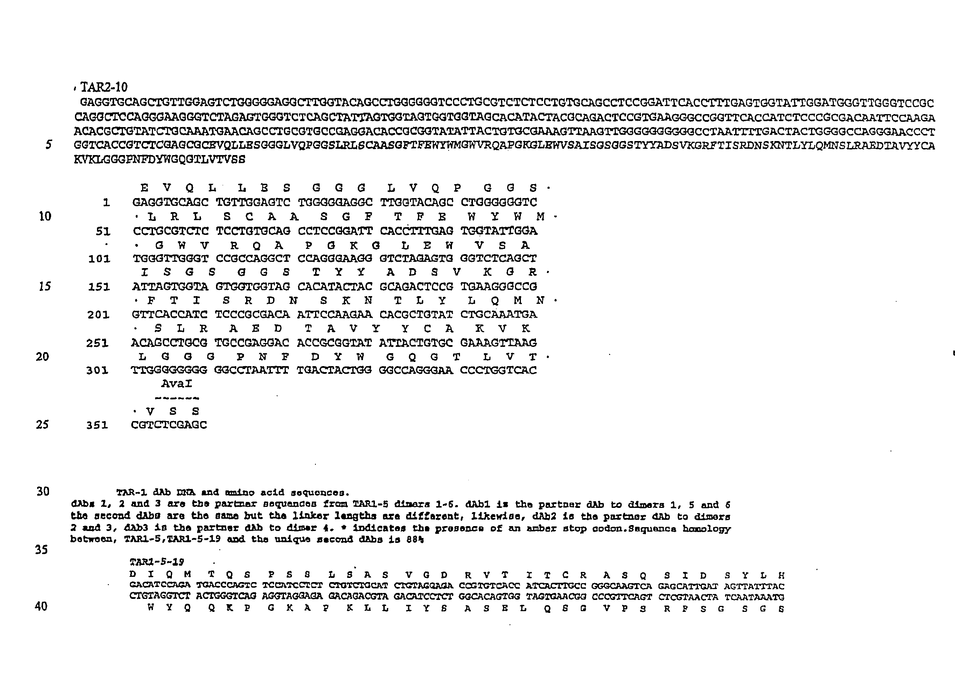

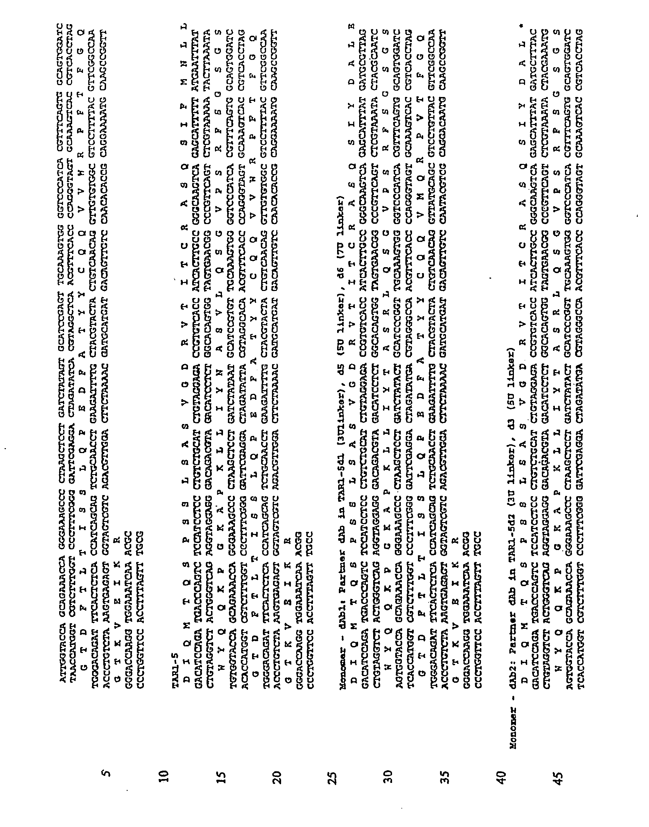

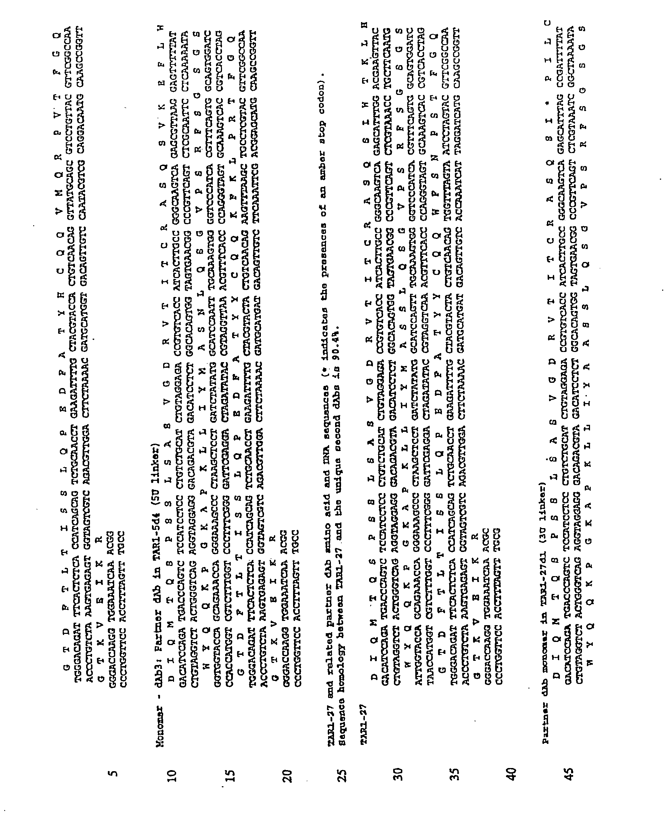

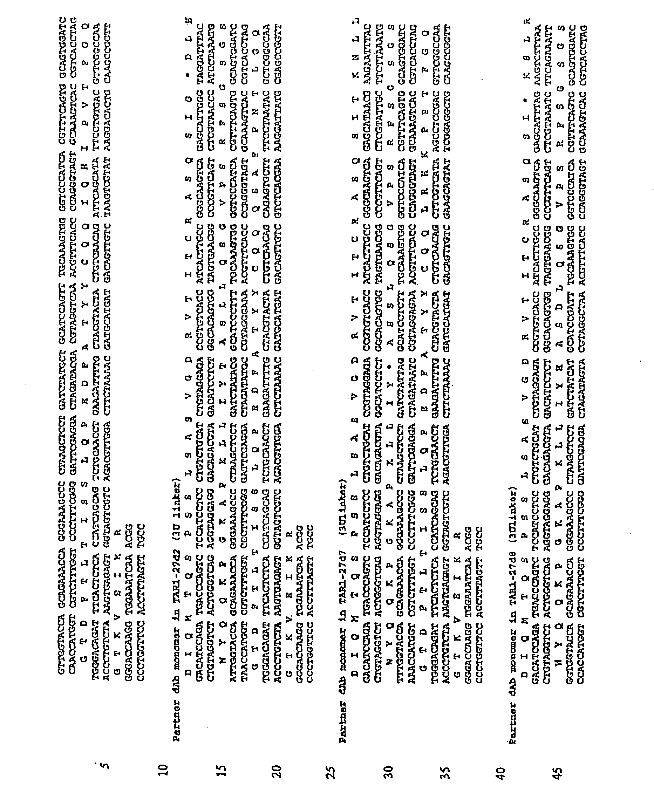

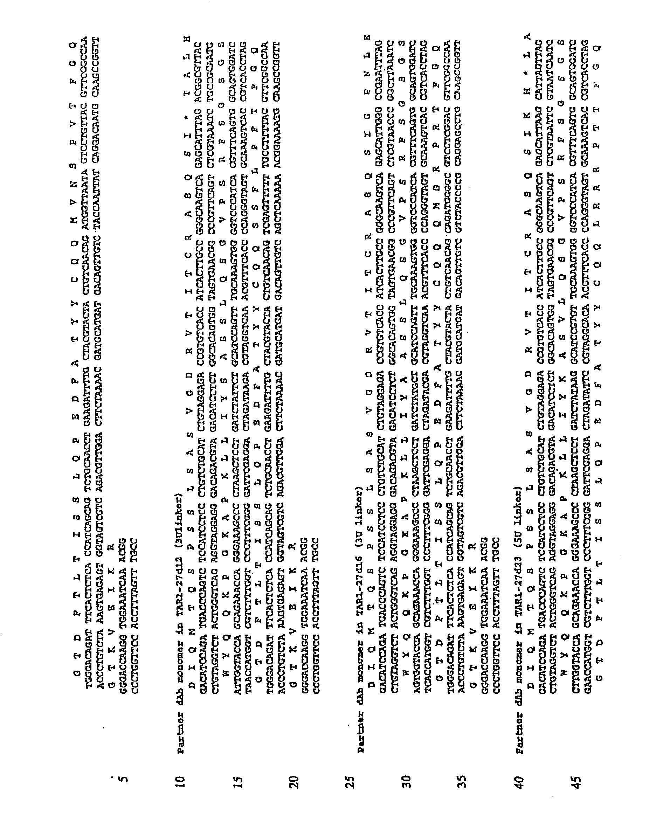

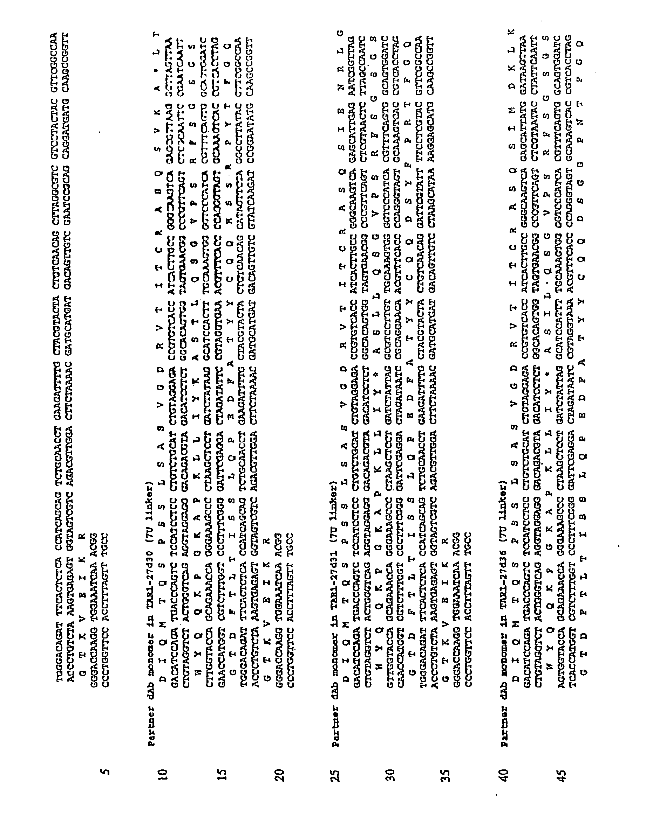

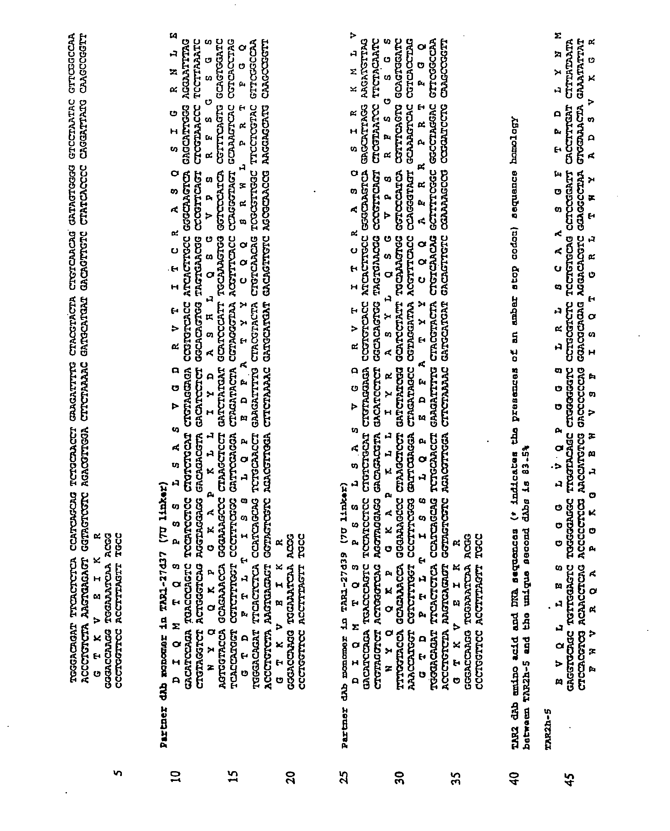

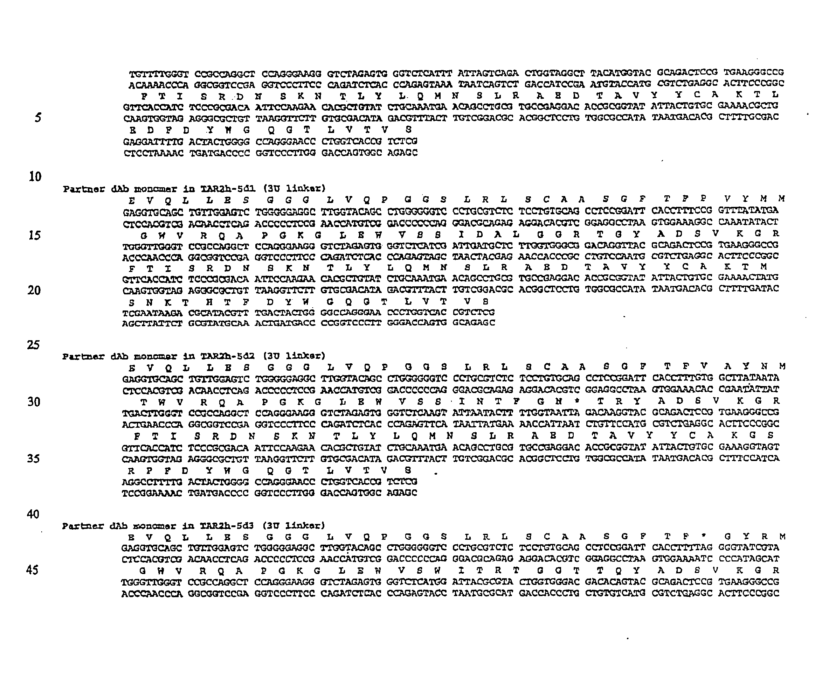

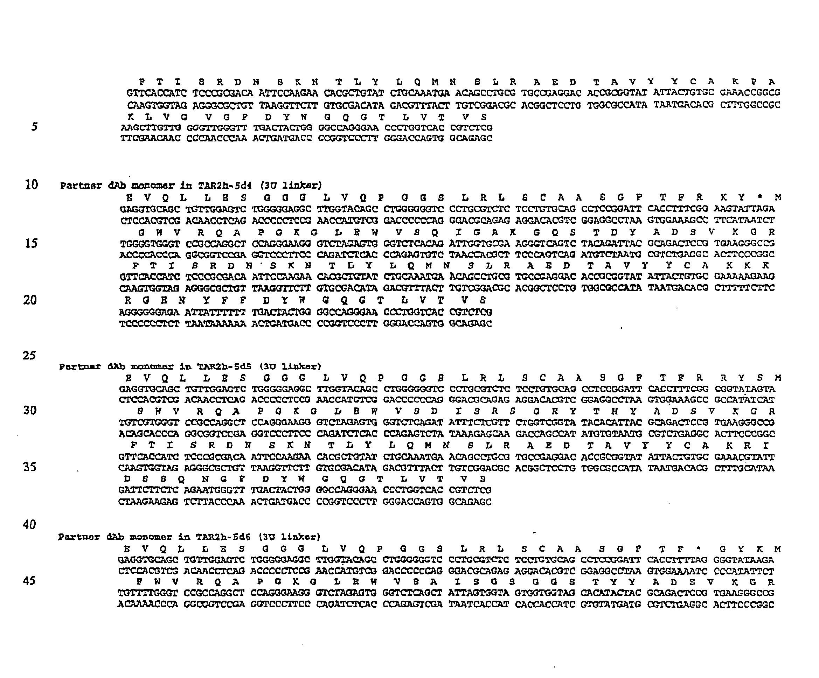

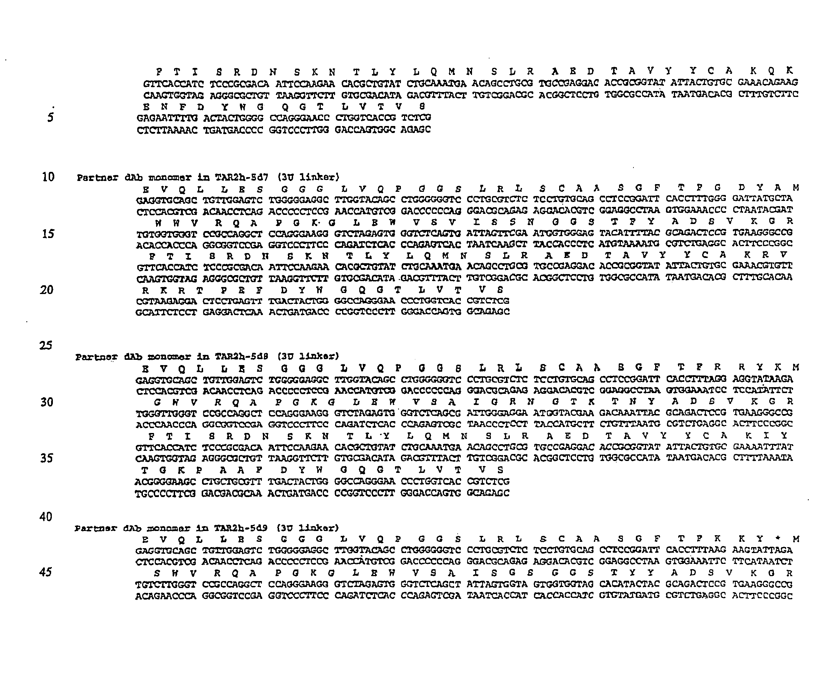

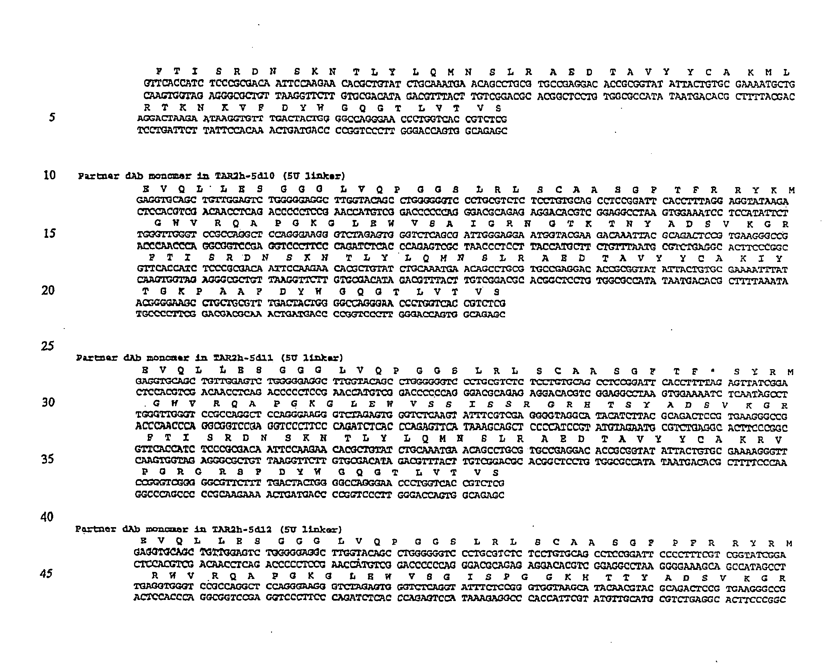

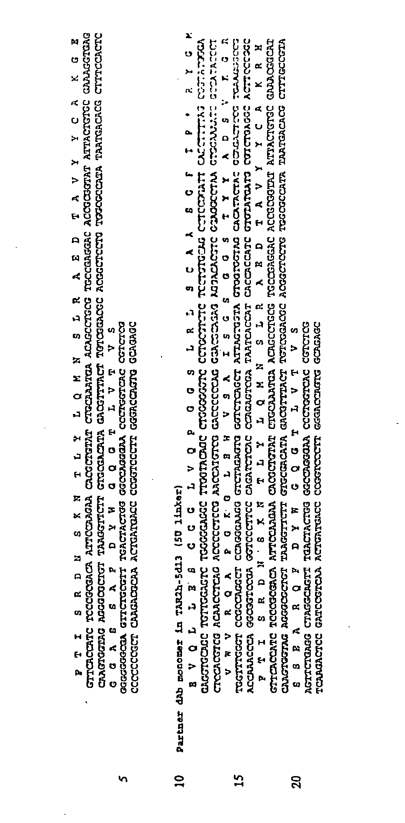

**Figure 30, A and B**. Shows the results of assays examining the inhibitory effects of anti-TNF x anti-VEGF dual specific antibody in assays of TNF-α activity and VEGF receptor binding. **A**. Results of L929 TNF-α cytotoxicity neutralization assays. Curve showing data points as squares, control anti-TNF-α antibody. Curve showing data points as upward-pointing triangles, anti-TNF x anti-VEGF dual specific antibody. Curve showing data points as downward-pointing triangles, anti-TNF-α monomer. **B**. Results of human VEGF Receptor 2 binding assays. Curve showing data points as squares, anti-TNF x anti-VEGF dual specific antibody. Curve showing data points as upward-pointing triangles, anti-VEGF control. Curve showing data points as downward-pointing triangles, negative control.

### DETAILED DESCRIPTION

### Dual-Specific Antibody Polypeptides:

The inventors have described, in their international patent application WO 2004/003019 a further improvement in dual specific ligands in which one specificity of the ligand is directed towards a protein or polypeptide present in vivo in an organism which can act to increase the half-life of the ligand by binding to it. WO 2004/003019 describes a dual-specific ligand comprising a first immunoglobulin single variable domain having a binding specificity to a first antigen or epitope and a second complementary immunoglobulin single variable domain having a binding activity to a second antigen or epitope, wherein one or both of said antigens or epitopes acts to increase the half-life of the ligand in vivo and wherein said first and second domains lack mutually complementary domains which share the same specificity, provided that said dual specific ligand does not consist of an anti-HSA VH domain and an anti-p galactosidase VK domain.

Antigens or epitopes which increase the half-life of a ligand as described herein are advantageously present on proteins or polypeptides found in an organism in vivo.

Examples include extracellular matrix proteins, blood proteins, and proteins present in various tissues in the organism. The proteins act to reduce the rate of ligand clearance from the blood, for example by acting as bulking agents, or by anchoring the ligand to a desired site of action. Examples of antigens/epitopes which increase half-life in vivo are given in Annex 1 below.

Increased half-life is useful in in vivo applications of immunoglobulins, especially antibodies and most especially antibody fragments of small size. Such fragments (Fvs, disulphide bonded Fvs, Fabs, scFvs, dAbs) suffer from rapid clearance from the body; thus, whilst they are able to reach most parts of the body rapidly, and are quick to produce and easier to handle, their in vivo applications have been limited by their only brief persistence in vivo. The invention solves this problem by providing increased half-life of the ligands in vivo and consequently longer persistence times in the body of the functional activity of the ligand.

Methods for pharmacokinetic analysis and determination of ligand half- life will be familiar to those skilled in the art. Details may be found in Kenneth, A et al: Chemical Stability of Pharmaceuticals: A Handbook for Pharmacists and in Peters et al, Pharmacokinetc analysis: A Practical Approach (1996). Reference is also made to "Pharmacokinetics", M Gibaldi & D Perron, published by Marcel Dekker, 2nd Rev. Edition (1982), which describes pharmacokinetic parameters such as t alpha and t beta half lives and area under the curve (AUC).

Half lives (T1/2 alpha and T1/2 beta) and AUC can be determined from a curve of serum concentration of ligand against time. The WinNonlin analysis package (available from Pharsight Corp., Mountain View, CA, USA) can be used, for example, to model the curve. In a first phase (the alpha phase) the ligand is undergoing mainly distribution in the patient, with some elimination. A second phase (beta phase) is the terminal phase when the ligand has been distributed and the serum concentration is decreasing as the ligand is cleared from the patient. The t alpha half life is the half life of the first phase and the t beta half life is the half life of the second phase. Thus, advantageously, the present invention provides a ligand or a composition comprising a ligand according to the invention having a tα half-life in the range of 15 minutes or more. In one embodiment, the lower end of the range is 30 minutes, 45 minutes, 1 hour, 2 hours, 3 hours, 4 hours, 5 5 hours, 6 hours, 7 hours, 10 hours, 11 hours or 12 hours. In addition, or alternatively,a ligand or composition according to the invention will have a tα half life in the range of up to and including 12 hours. In one embodiment, the upper end of the range is 11, 10, 9, 8, 7, 6 or 5 hours. An example of a suitable range is 1 to 6 hours, 2 to 5 hours or 3 to 4 hours. Advantageously, the present invention provides a ligand or a composition comprising a ligand according to the invention having a tβ half-life in the range of 2.5 hours or more.

In one embodiment, the lower end of the range is 3 hours, 4 hours, 5 hours, 6 hours, 7 hours, 10 hours, 11 hours, or 12 hours. In addition, or alternatively, a ligand or composition according to the invention has a tβ half-life in the range of up to and including 21 days. In one embodiment, the upper end of the range is 12 hours, 24 hours, 2 days, 3 days, 5 days, 10 days, 15 days or 20 days. Advantageously a ligand or composition according to the invention will have a tβ half life in the range 12 to 60 hours.

In a further embodiment, it will be in the range 12 to 48 hours. In a further embodiment still, it will be in the range 12 to 26 hours.

In addition, or alternatively to the above criteria, the present invention provides a ligand or a composition comprising a ligand according to the invention having an AUC value (area under the curve) in the range of 1 mg.min/ml or more. In one embodiment, the lower end of the range is 5, 10, 15, 20, 30, 100, 200 or 300mg.min/ml. In addition, or alternatively, a ligand or composition according to the invention has an AUC in the range of up to 600 mg.min/ml.

In one embodiment, the upper end of the range is 500, 400, 300, 200, 150, 100, 75 or 50 mg.min/ml. Advantageously a ligand according to the invention will have a AUC in the range selected from the group consisting of the following: 15 to 150 mg.min/ml, 15 to 100 mg.min/ml, 15 to 75 mg. min/ml, and 15 to 50mg.min/ml.

In a first embodiment, the dual specific ligand comprises two complementary variable domains, i.e. two variable domains that, in their natural environment, are capable of operating together as a cognate pair or group even if in the context of the present invention they bind separately to their cognate epitopes. For example, the complementary variable domains may be immunoglobulin heavy chain and light chain variable domains (VH and VL). VH and VL domains are advantageously provided by scFv or Fab antibody fragments. Variable domains may be linked together to form multivalent ligands by, for example: provision of a hinge region at the C-terminus of each V domain and disulphide bonding between cysteines in the hinge regions; or provision of dAbs each with a cysteine at the C-terminus of the domain, the cysteines being disulphide bonded together; or production of V-CH & V-CL to produce a Fab format; or use of peptide linkers (for example Gly4Ser linkers discussed hereinbelow) to produce dimers, trimers and further multimers. The inventors have found that the use of complementary variable domains allows the two domain surfaces to pack together and be sequestered from the solvent. Furthermore the complementary domains are able to stabilise each other. In addition, it allows the creation of dual- specific IgG antibodies without the disadvantages of hybrid hybridomas as used in the prior art, or the need to engineer heavy or light chains at the sub-unit interfaces.

The dual-specific ligands of the first aspect of the invention have at least one VH/VL pair. A bispecific IgG according to this invention will therefore comprise two such pairs, one pair on each arm of the Y-shaped molecule. Unlike conventional bispecific antibodies or diabodies, therefore, where the ratio of chains used is determinative in the success of the preparation thereof and leads to practical difficulties, the dual specific ligands of the invention are free from issues of chain balance. Chain imbalance in conventional bi-specific antibodies results from the association of two different VL chains with two different VH chains, where VL chain 1 together with VH chain 1 is able to bind to antigen or epitope 1 and VH chain 2 together with VH chain 2 is able to bind to antigen or epitope 2 and the two correct pairings are in some way linked to one another. Thus, only when VL chain 1 is paired with VH chain 1 and VL chain 2 is paired with VH chain 2 in a single molecule is bi- specificity created. Such bi-specific molecules can be created in two different ways. Firstly, they can be created by association of two existing VH/VL pairings that each bind to a different antigen or epitope (for example, in a bi-specific IgG). In this case the VHNL pairings must come all together in a 1:1 ratio in order to create a population of molecules all of which are bi-specific. This never occurs (even when complementary CH domain is enhanced by "knobs into holes" engineering) leading to a mixture of bi-specific molecules and molecules that are only able to bind to one antigen or epitope but not the other. The second way of creating a bi-specific antibody is by the simultaneous association of two different VH chain with two different VL chains (for example in a bi-specific diabody). In this case, although there tends to be a preference for VL chain 1 to pair with VH chain 1 and VL chain 2 to pair with VH chain 2 (which can be enhanced by "knobs into holes" engineering of the VL and VH domains), this paring is never achieved in all molecules, leading to a mixed formulation whereby incorrect pairings occur that are unable to bind to either antigen or epitope.

Bi-specific antibodies constructed according to the dual-specific ligand approach according to the first aspect of the present invention overcome all of these problems because the binding to antigen or epitope 1 resides within the VH or VL domain and the binding to antigen or epitope 2 resides with the complementary VL or VH domain, respectively. Since VH and VL domains pair on a 1:1 basis all VHNL pairings will be bi-specific and thus all formats constructed using these VHNL pairings (Fv, scFvs, Fabs, minibodies, IgGs etc) will have 100% bi-specific activity.

In the context of the present invention, first and second "epitopes" are understood to be epitopes which are not the same and are not bound by a single monospecific ligand. In the first configuration of the invention, they are advantageously on different antigens, one of which acts to increase the half-life of the ligand in vivo. Likewise, the first and second antigens are advantageously not the same.

The dual specific ligands of the invention do not include ligands as described in WO 02/02773. Thus, the ligands of the present invention do not comprise complementary VHNL pairs which bind any one or more antigens or epitopes co-operatively. Instead, the ligands according to the first aspect of the invention comprise a VHNL complementary pair, wherein the V domains have different specificities.

Moreover, the ligands according to the first aspect of the invention comprise VHNL complementary pairs having different specificities for non-structurally related epitopes or antigens. Structurally related epitopes or antigens are epitopes or antigens which possess sufficient structural similarity to be bound by a conventional VH/VL complementary pair which acts in a co-operative manner to bind an antigen or epitope, in the case of structurally related epitopes, the epitopes are sufficiently similar in structure that they "fit" into the same binding pocket formed at the antigen binding site of the VH/VL dimer.

In a second aspect, the present invention provides a ligand comprising a first immunoglobulin variable domain having a first antigen or epitope binding specificity and a second immunoglobulin variable domain having a second antigen or epitope binding specificity wherein one or both of said first and second variable domains bind to an antigen which increases the half-life of the ligand in vivo, and the variable domains are not complementary to one another.

In one embodiment, binding to one variable domain modulates the binding of the ligand to the second variable domain.

In this embodiment, the variable domains may be, for example, pairs of VH domains or pairs of VL domains. Binding of antigen at the first site may modulate, such as enhance or inhibit, binding of an antigen at the second site. For example, binding at the first site at least partially inhibits binding of an antigen at a second site. Such an embodiment, the ligand may for example be maintained in the body of a subject organism in vivo through binding to a protein which increases the half-life of the ligand until such a time as it becomes bound to the second target antigen and dissociates from the half-life increasing protein.

Modulation of binding in the above context is achieved as a consequence of the structural proximity of the antigen binding sites relative to one another. Such structural proximity can be achieved by the nature of the structural components linking the two or more antigen binding sites, eg by the provision of a ligand with a relatively rigid structure that holds the antigen binding sites in close proximity. Advantageously, the two or more antigen binding sites are in physically close proximity to one another such that one site modulates the binding of antigen at another site by a process which involves steric hindrance and/or confirmational changes within the immunoglobulin molecule.

The first and the second antigen binding domains may be associated either covalently or non-covalently. In the case that the domains are covalently associated, then the association may be mediated for example by disulphide bonds or by a polypeptide linker such as (Gly4Ser)n, where n = from 1 to 8, eg, 2, 3, 4, 5 or 7.

Ligands according to this aspect of the invention may be combined into non-immunoglobulin multi ligand structures to form multivalent complexes, which bind target molecules with the same antigen, thereby providing superior avidity, while at least one variable domain binds an antigen to increase the half life of the multimer. For example natural bacterial receptors such as SpA have been used as scaffolds for the grafting of CDRs to generate ligands which bind specifically to one or more epitopes. Details of this procedure are described in US 5,531,012. Other suitable scaffolds include those based on fibronectin and affibodies. Details of suitable procedures are described in WO 98/58965. Other suitable scaffolds include lipocallin and CTLA4, as described in van den Beuken et al., J. Mol. Biol. (2001) 310, 591-601, and scaffolds such as those described in W00069907 (Medical Research Council), which are based for example on the ring structure of bacterial GroEL or other chaperone polypeptides.

Protein scaffolds may be combined, for example, CDRs may be grafted on to a CTLA4 scaffold and used together with immunoglobulin V_{H} or V_{L} domains to form a ligand.

Likewise, fibronectin, lipocallin and other scaffolds may be combined.

In the case that the variable domains are selected from V-gene repertoires selected for instance using phage display technology as herein described, then these variable domains can comprise a universal framework region, such that they may be recognised by a specific generic ligand as herein defined. The use of universal frameworks, generic ligands and the like is described in WO99/20749. In the present invention, reference to phage display includes the use of both phage and/or phagemids.

Where V-gene repertoires are used variation in polypeptide sequence is preferably located within the structural loops of the variable domains. The polypeptide sequences of either variable domain may be altered by DNA shuffling or by mutation in order to enhance the interaction of each variable domain with its complementary pair.

In a preferred embodiment of the invention the 'dual-specific ligand' is a single chain Fv fragment. In an alternative embodiment of the invention, the 'dual-specific ligand' consists of a Fab region of an antibody. The term "Fab region" includes a Fab-like region where two VH or two VL domains are used.

The variable regions may be derived from antibodies directed against target antigens or epitopes. Alternatively they may be derived from a repertoire of single antibody domains such as those expressed on the surface of filamentous bacteriophage. Selection may be performed as described herein below and in the Examples.

### Preparation of dAbs:

An aspect of the invention relates not only to dual-specific ligands in general, but also to various constructs of ligands that bind TNF-α alone, TNF-α and HSA or other half-life-extending polypeptide in the dual-specific format, and ligands that bind TNF-α and VEGF in the dual specific format. Ligands that bind VEGF and HSA or other half-life-extending polypeptide can also be prepared. The dual-specific TNF-α/VEGF construct can additionally comprise a binder for HSA or another half-life-extending molecule. In each of these embodiments, the individual ligands, i.e., those that bind TNF-α, HSA or VEGF, can be and are preferably, dAbs. The generation of such dAbs is discussed below and in the Examples.

In various aspects, the dAbs disclosed herein can be present in monomeric form, dimeric form, trimeric form, tetrameric form, or even in higher multimeric forms. In addition to the heterodimeric forms such as the dual specific constructs, multimeric constructs can be homomultimeric, i.e., homodimer, homotrimer, homotetramer, etc. Heterotrimers, heterotetramers and higher order heteromultimers are also specifically contemplated. Each of the various dAb conformations can additionally be complexed with additional moieties, such as polyethylene glycol (PEG) in order to further prolong the serum half-life of the polypeptide construct. PEGylation is known in the art and described herein.

Single immunoglobulin variable domains or dAbs are prepared in a number of ways. In a preferred aspect, the dAbs are human single immunoglobulin variable domains. For each of these approaches, well-known methods of preparing (e.g., amplifying, mutating, etc.) and manipulating nucleic acid sequences are applicable.

One means of preparing dAbs is to amplify and express the V_{H} or V_{L} region of a heavy chain or light chain gene for a cloned antibody known to bind the desired antigen. The boundaries of V_{H} and V_{L} domains are set out by Kabat et al. (1991, supra). The information regarding the boundaries of the V_{H} and V_{L} domains of heavy and light chain genes is used to design PCR primers that amplify the V domain from a cloned heavy or light chain coding sequence encoding an antibody known to bind a given antigen. The amplified V domain is inserted into a suitable expression vector, e.g., pHEN-1 (Hoogenboom et al., 1991, Nucleic Acids Res. 19: 4133-4137) and expressed, either alone or as a fusion with another polypeptide sequence. The expressed V_{H} or V_{L} domain is then screened for high affinity binding to the desired antigen in isolation from the remainder of the heavy or light chain polypeptide. For all aspects of the present invention, screening for binding is performed as known in the art or as described herein below.

A repertoire of V_{H} or V_{L} domains is screened by, for example, phage display, panning against the desired antigen. Methods for the construction of bacteriophage display libraries and lambda phage expression libraries are well known in the art, and taught, for example, by: McCafferty et al., 1990, Nature 348: 552; Kang et al., 1991, Proc. Natl. Acad. Sci. U.S.A., 88: 4363; Clackson et al., 1991, Nature 352: 624; Lowman et al., 1991, Biochemistry 30: 10832; Burton et al., 1991, Proc. Natl. Acad. Sci U.S.A. 88: 10134; Hoogenboom et al., 1991, Nucleic Acids Res. 19: 4133; Chang et al.,1991, J. Immunol. 147: 3610; Breitling et al., 1991, Gene 104: 147; Marks et al., 1991, J. Mol. Biol. 222: 581; Barbas et al., 1992, Proc. Natl. Acad. Sci. U.S.A. 89: 4457; Hawkins and Winter (1992) J. Immunol., 22: 867; Marks et al. (1992) J. Biol. Chem., 267: 16007; and Lerner et al. (1992) Science, 258: 1313. scFv phage libraries are taught, for example, by Huston et al., 1988, Proc. Natl. Acad. Sci U.S.A. 85: 5879-5883; Chaudhary et al., 1990, Proc. Natl. Acad. Sci U.S.A. 87: 1066-1070; McCafferty et al., 1990, supra; Clackson et al., 1991, supra; Marks et al., 1991, supra; Chiswell et al., 1992, Trends Biotech. 10: 80; and Marks et al., 1992, supra. Various embodiments of scFv libraries displayed on bacteriophage coat proteins have been described. Refinements of phage display approaches are also known, for example as described in WO96/0621 and WO92/01047 (Medical Research Council et al.) and WO97/08320 (Morphosys, supra).

The repertoire of V_{H} or V_{L} domains can be a naturally-occurring repertoire of immunoglobulin sequences or a synthetic repertoire. A naturally-occurring repertoire is one prepared, for example, from immunoglobulin-expressing cells harvested from one or more individuals. Such repertoires can be "naïve," i.e., prepared, for example, from human fetal or newborn immunoglobulin-expressing cells, or rearranged, i.e., prepared from, for example, adult human B cells. Natural repertoires are described, for example, by Marks et al., 1991, J. Mol. Biol. 222: 581 and Vaughan et al., 1996, Nature Biotech. 14: 309. If desired, clones identified from a natural repertoire, or any repertoire, for that matter, that bind the target antigen are then subjected to mutagenesis and further screening in order to produce and select variants with improved binding characteristics.

Synthetic repertoires of single immunoglobulin variable domains are prepared by artificially introducing diversity into a cloned V domain. Synthetic repertoires are described, for example, by Hoogenboom & Winter, 1992, J. Mol. Biol. 227: 381; Barbas et al., 1992, Proc. Natl. Acad. Sci. U.S.A. 89: 4457; Nissim et al., 1994, EMBO J. 13: 692; Griffiths et al., 1994, EMBO J. 13: 3245; DeKriuf et al., 1995, J. Mol. Biol. 248: 97; and WO 99/20749.

The antigen binding domain of a conventional antibody comprises two separate regions: a heavy chain variable domain (V_{H}) and a light chain variable domain (V_{L}: which can be either V_{κ} or V_{λ}). The antigen binding site of such an antibody is formed by six polypeptide loops: three from the V_{H} domain (H1, H2 and H3) and three from the V_{L} domain (L1, L2 and L3). The boundaries of these loops are described, for example, in Kabat et al. (1991, supra). A diverse primary repertoire of V genes that encode the V_{H} and V_{L} domains is produced *in vivo* by the combinatorial rearrangement of gene segments. The V_{H} gene is produced by the recombination of three gene segments, V_{H}, D and J_{H}. In humans, there are approximately 51 functional V_{H} segments (Cook and Tomlinson (1995) Immunol Today 16: 237), 25 functional D segments (Corbett et al. (1997) J. Mol. Biol. 268: 69) and 6 functional J_{H} segments (Ravetch et al. (1981) Cell 27: 583), depending on the haplotype. The V_{H} segment encodes the region of the polypeptide chain which forms the first and second antigen binding loops of the V_{H} domain (H1 and H2), while the V_{H}, D and J_{H} segments combine to form the third antigen binding loop of the V_{H} domain (H3).

The V_{L} gene is produced by the recombination of only two gene segments, V_{L} and J_{L}. In humans, there are approximately 40 functional V_{κ} segments (Schäble and Zachau (1993) Biol. Chem. Hoppe-Seyler 374: 1001), 31 functional V_{λ} segments (Williams et al. (1996) J. Mol. Biol. 264: 220; Kawasaki et al. (1997) Genome Res. 7: 250), 5 functional J_{κ} segments (Hieter et al. (1982) J. Biol. Chem. 257: 1516) and 4 functional J_{λ} segments (Vasicek and Leder (1990) J. Exp. Med. 172: 609), depending on the haplotype. The V_{L} segment encodes the region of the polypeptide chain which forms the first and second antigen binding loops of the V_{L} domain (L1 and L2), while the V_{L} and J_{L} segments combine to form the third antigen binding loop of the V_{L} domain (L3). Antibodies selected from this primary repertoire are believed to be sufficiently diverse to bind almost all antigens with at least moderate affinity. High affinity antibodies are produced *in vivo* by "affinity maturation" of the rearranged genes, in which point mutations are generated and selected by the immune system on the basis of improved binding.

Analysis of the structures and sequences of antibodies has shown that five of the six antigen binding loops (H1, H2, L1, L2, L3) possess a limited number of main-chain conformations or canonical structures (Chothia and Lesk (1987) J. Mol. Biol. 196: 901; Chothia et al. (1989) Nature 342: 877). The main-chain conformations are determined by (i) the length of the antigen binding loop, and (ii) particular residues, or types of residue, at certain key position in the antigen binding loop and the antibody framework. Analysis of the loop lengths and key residues has enabled us to the predict the main-chain conformations of H1, H2, L1, L2 and L3 encoded by the majority of human antibody sequences (Chothia et al. (1992) J. Mol. Biol. 227: 799; Tomlinson et al. (1995) EMBO J. 14: 4628; Williams et al. (1996) J. Mol. Biol. 264: 220). Although the H3 region is much more diverse in terms of sequence, length and structure (due to the use of D segments), it also forms a limited number of main-chain conformations for short loop lengths which depend on the length and the presence of particular residues, or types of residue, at key positions in the loop and the antibody framework (Martin et al. (1996) J. Mol. Biol. 263: 800; Shirai et al. (1996) FEBS Letters 399: 1.

While, according to one embodiment of the invention, diversity can be added to synthetic repertoires at any site in the CDRs of the various antigen-binding loops, this approach results in a greater proportion of V domains that do not properly fold and therefore contribute to a lower proportion of molecules with the potential to bind antigen. An understanding of the residues contributing to the main chain conformation of the antigen-binding loops permits the identification of specific residues to diversify in a synthetic repertoire of V_{H} or V_{L} domains. That is, diversity is best introduced in residues that are not essential to maintaining the main chain conformation. As an example, for the diversification of loop L2, the conventional approach would be to diversify all the residues in the corresponding CDR (CDR2) as defined by Kabat et al. (1991, supra), some seven residues. However, for L2, it is known that positions 50 and 53 are diverse in naturally occurring antibodies and are observed to make contact with the antigen. The preferred approach would be to diversify only those two residues in this loop. This represents a significant improvement in terms of the functional diversity required to create a range of antigen binding specificities.

In one aspect, synthetic variable domain repertoires are prepared in V_{H} or V_{κ} backgrounds, based on artificially diversified germline V_{H} or V_{κ} sequences. For example, the V_{H} domain repertoire is based on cloned germline V_{H} gene segments V3-23/DP47 (Tomlinson et al., 1992, J. Mol. Biol. 227: 7768) and JH4b (see Figures 1 and 2). The V_{κ} domain repertoire is based, for example, on germline V_{κ} gene segments O2/O12/DPK9 (Cox et al., 1994, Eur. J. Immunol. 24: 827) and J_{κ}1 (see Figure 3). Diversity is introduced into these or other gene segments by, for example, PCR mutagenesis. Diversity can be randomly introduced, for example, by error prone PCR (Hawkins, et al., 1992, J. Mol. Biol. 226: 889) or chemical mutagenesis. As discussed above, however it is preferred that the introduction of diversity is targeted to particular residues. It is further preferred that the desired residues are targeted by introduction of the codon NNK using mutagenic primers (using the IUPAC nomenclature, where N = G, A, T or C, and K = G or T), which encodes all amino acids and the TAG stop codon. Other codons which achieve similar ends are also of use, including the NNN codon (which leads to the production of the additional stop codons TGA and TAA), DVT codon ((A/G/T) (A/G/C)T), DVC codon ((A/G/T)(A/G/C)C), and DVY codon ((A/G/T)(A/G/C)(C/T). The DVT codon encodes 22% serine and 11 % tyrosine, asgpargine, glycine, alanine, aspartate, threonine and cysteine, which most closely mimics the distribution of amino acid residues for the antigen binding sites of natural human antibodies. Repertoires are made using PCR primers having the selected degenerate codon or codons at each site to be diversified. PCR mutagenesis is well known in the art; however, considerations for primer design and PCR mutagenesis useful in the methods of the invention are discussed below in the section titled "PCR Mutagenesis."

In one aspect, diversity is introduced into the sequence of human germline V_{H} gene segments V3-23/DP47 (Tomlinson et al., 1992, J. Mol. Biol. 227: 7768) and JH4b using the NNK codon at sites H30, H31, H33, H35, H50, H52, H52a, H53, H55, H56, H58, H95, H97 and H98, corresponding to diversity in CDRs 1, 2 and 3, as shown in **Figure 1.**

In another aspect, diversity is also introduced into the sequence of human germline V_{H} gene segments V3-23/DP47 and JH4b, for example, using the NNK codon at sites H30, H31, H33, H35, H50, H52, H52a, H53, H55, H56, H58, H95, H97, H98, H99, H100, H100a and H100b, corresponding to diversity in CDRs 1, 2 and 3, as shown in **Figure 2.**

In another aspect, diversity is introduced into the sequence of human germline V_{κ} gene segments O2/O12/DPK9 and J_{κ}1, for example, using the NNK codon at sites L30, L31, L32, L34, L50, L53, L91, L92, L93, L94 and L96, corresponding to diversity in CDRs 1, 2 and 3, as shown in Figure 3.

Diversified repertoires are cloned into phage display vectors as known in the art and as described, for example, in WO 99/20749. In general, the nucleic acid molecules and vector constructs required for the performance of the present invention are available in the art and are constructed and manipulated as set forth in standard laboratory manuals, such as Sambrook et al. (1989). Molecular Cloning: A Laboratory Manual, Cold Spring Harbor, USA.

The manipulation of nucleic acids in the present invention is typically carried out in recombinant vectors. As used herein, "vector" refers to a discrete element that is used to introduce heterologous DNA into cells for the expression and/or replication thereof. Methods by which to select or construct and, subsequently, use such vectors are well known to one of skill in the art. Numerous vectors are publicly available, including bacterial plasmids, bacteriophage, artificial chromosomes and episomal vectors. Such vectors may be used for simple cloning and mutagenesis; alternatively, as is typical of vectors in which repertoire (or pre-repertoire) members of the invention are carried, a gene expression vector is employed. A vector of use according to the invention is selected to accommodate a polypeptide coding sequence of a desired size, typically from 0.25 kilobase (kb) to 40 kb in length. A suitable host cell is transformed with the vector after *in vitro* cloning manipulations. Each vector contains various functional components, which generally include a cloning (or "polylinker") site, an origin of replication and at least one selectable marker gene. If a given vector is an expression vector, it additionally possesses one or more of the following: enhancer element, promoter, transcription termination and signal sequences, each positioned in the vicinity of the cloning site, such that they are operatively linked to the gene encoding a polypeptide repertoire member according to the invention.

Both cloning and expression vectors generally contain nucleic acid sequences that enable the vector to replicate in one or more selected host cells. Typically in cloning vectors, this sequence is one that enables the vector to replicate independently of the host chromosomal DNA and includes origins of replication or autonomously replicating sequences. Such sequences are well known for a variety of bacteria, yeast and viruses. The origin of replication from the plasmid pBR322 is suitable for most Gram-negative bacteria, the 2 micron plasmid origin is suitable for yeast, and various viral origins (*e.g*. SV 40, adenovirus) are useful for cloning vectors in mammalian cells. Generally, the origin of replication is not needed for mammalian expression vectors unless these are used in mammalian cells able to replicate high levels of DNA, such as COS cells.

Advantageously, a cloning or expression vector also contains a selection gene also referred to as selectable marker. This gene encodes a protein necessary for the survival or growth of transformed host cells grown in a selective culture medium. Host cells not transformed with the vector containing the selection gene will therefore not survive in the culture medium. Typical selection genes encode proteins that confer resistance to antibiotics and other toxins, e.g. ampicillin, neomycin, methotrexate or tetracycline, complement auxotrophic deficiencies, or supply critical nutrients not available in the growth media.

Because the replication of vectors according to the present invention is most conveniently performed in *E. coli,* an *E. coli*-selectable marker, for example, the β-lactamase gene that confers resistance to the antibiotic ampicillin, is of use. These can be obtained from *E*. *coli* plasmids, such as pBR322 or a pUC plasmid such as pUC18 or pUC19.

Expression vectors usually contain a promoter that is recognized by the host organism and is operably linked to the coding sequence of interest. Such a promoter may be inducible or constitutive. The term "operably linked" refers to a juxtaposition wherein the components described are in a relationship permitting them to function in their intended manner. A control sequence "operably linked" to a coding sequence is ligated in such a way that expression of the coding sequence is achieved under conditions compatible with the control sequences.

Promoters suitable for use with prokaryotic hosts include, for example, the β-lactamase and lactose promoter systems, alkaline phosphatase, the tryptophan (trp) promoter system and hybrid promoters such as the tac promoter. Promoters for use in bacterial systems will also generally contain a Shine-Dalgarno sequence operably linked to the coding sequence.

In libraries or repertoires as described herein, the preferred vectors are expression vectors that enable the expression of a nucleotide sequence corresponding to a polypeptide library member. Thus, selection is performed by separate propagation and expression of a single clone expressing the polypeptide library member or by use of any selection display system. As described above, a preferred selection display system uses bacteriophage display. Thus, phage or phagemid vectors can be used. Preferred vectors are phagemid vectors, which have an *E. coli* origin of replication (for double stranded replication) and also a phage origin of replication (for production of single-stranded DNA). The manipulation and expression of such vectors is well known in the art (Hoogenboom and Winter (1992) *supra*; Nissim *et al.* (1994) *supra*). Briefly, the vector contains a β-lactamase or other selectable marker gene to confer selectivity on the phagemid, and a lac promoter upstream of a expression cassette that consists (N to C terminal) of a pelB leader sequence (which directs the expressed polypeptide to the periplasmic space), a multiple cloning site (for cloning the nucleotide version of the library member), optionally, one or more peptide tags (for detection), optionally, one or more TAG stop codons and the phage protein pIII. Using various suppressor and non-suppressor strains of *E*. *coli* and with the addition of glucose, iso-propyl thio-β-D-galactoside (IPTG) or a helper phage, such as VCS M13, the vector is able to replicate as a plasmid with no expression, produce large quantities of the polypeptide library member only, or produce phage, some of which contain at least one copy of the polypeptide-pIII fusion on their surface.

An example of a preferred vector is the pHEN1 phagemid vector (Hoogenboom et al., 1991, Nucl. Acids Res. 19: 4133-4137; sequence is available, e.g., as SEQ ID NO: 7 in WO 03/031611), in which the production of pIII fusion protein is under the control of the LacZ promoter, which is inhibited in the presence of glucose and induced with IPTG. When grown in suppressor strains of E. coli, e.g., TG1, the gene III fusion protein is produced and packaged into phage, while growth in non-suppressor strains, e.g., HB2151, permits the secretion of soluble fusion protein into the bacterial periplasm and into the culture medium. Because the expression of gene III prevents later infection with helper phage, the bacteria harboring the phagemid vectors are propagated in the presence of glucose before infection with VCSM13 helper phage for phage rescue.

Construction of vectors according to the invention employs conventional ligation techniques. Isolated vectors or DNA fragments are cleaved, tailored, and re-ligated in the form desired to generate the required vector. If desired, sequence analysis to confirm that the correct sequences are present in the constructed vector is performed using standard methods. Suitable methods for constructing expression vectors, preparing *in vitro* transcripts, introducing DNA into host cells, and performing analyses for assessing expression and function are known to those skilled in the art. The presence of a gene sequence in a sample is detected, or its amplification and/or expression quantified by conventional methods, such as Southern or Northern analysis, Western blotting, dot blotting of DNA, RNA or protein*, in situ* hybridization, immunocytochemistry or sequence analysis of nucleic acid or protein molecules. Those skilled in the art will readily envisage how these methods may be modified, if desired.

### PCR Mutagenesis:

The primer is complementary to a portion of a target molecule present in a pool of nucleic acid molecules used in the preparation of sets of nucleic acid repertoire members encoding polypeptide repertoire members. Most often, primers are prepared by synthetic methods, either chemical or enzymatic. Mutagenic oligonucleotide primers are generally 15 to 100 nucleotides in length, ideally from 20 to 40 nucleotides, although oligonucleotides of different length are of use.

Typically, selective hybridization occurs when two nucleic acid sequences are substantially complementary (at least about 65% complementary over a stretch of at least 14 to 25 nucleotides, preferably at least about 75%, more preferably at least about 85% or 90% complementary). See Kanehisa, 1984, Nucleic Acids Res. 12: 203, incorporated herein by reference. As a result, it is expected that a certain degree of mismatch at the priming site is tolerated. Such mismatch may be small, such as a mono-, di- or tri-nucleotide. Alternatively, it may comprise nucleotide loops, which are defined herein as regions in which mismatch encompasses an uninterrupted series of four or more nucleotides.

Overall, five factors influence the efficiency and selectivity of hybridization of the primer to a second nucleic acid molecule. These factors, which are (i) primer length, (ii) the nucleotide sequence and/or composition, (iii) hybridization temperature, (iv) buffer chemistry and (v) the potential for steric hindrance in the region to which the primer is required to hybridize, are important considerations when non-random priming sequences are designed.

There is a positive correlation between primer length and both the efficiency and accuracy with which a primer will anneal to a target sequence; longer sequences have a higher melting temperature (T_{M}) than do shorter ones, and are less likely to be repeated within a given target sequence, thereby minimizing promiscuous hybridization. Primer sequences with a high G-C content or that comprise palindromic sequences tend to self-hybridize, as do their intended target sites, since unimolecular, rather than bimolecular, hybridization kinetics are generally favored in solution; at the same time, it is important to design a primer containing sufficient numbers of G-C nucleotide pairings to bind the target sequence tightly, since each such pair is bound by three hydrogen bonds, rather than the two that are found when A and T bases pair. Hybridization temperature varies inversely with primer annealing efficiency, as does the concentration of organic solvents, *e.g*. formamide, that might be included in a hybridization mixture, while increases in salt concentration facilitate binding. Under stringent hybridization conditions, longer probes hybridize more efficiently than do shorter ones, which are sufficient under more permissive conditions. Stringent hybridization conditions for primers typically include salt concentrations of less than about 1M, more usually less than about 500 mM and preferably less than about 200 mM. Hybridization temperatures range from as low as 0°C to greater than 22°C, greater than about 30°C, and (most often) in excess of about 37°C. Longer fragments may require higher hybridization temperatures for specific hybridization. As several factors affect the stringency of hybridization, the combination of parameters is more important than the absolute measure of any one alone.

Primers are designed with these considerations in mind. While estimates of the relative merits of numerous sequences may be made mentally by one of skill in the art, computer programs have been designed to assist in the evaluation of these several parameters and the optimization of primer sequences. Examples of such programs are "PrimerSelect" of the DNAStar^{™} software package (DNAStar, Inc.; Madison, WI) and OLIGO 4.0 (National Biosciences, Inc.). Once designed, suitable oligonucleotides are prepared by a suitable method, e.g. the phosphoramidite method described by Beaucage and Carruthers, 1981, Tetrahedron Lett. 22: 1859) or the triester method according to Matteucci and Caruthers, 1981, J. Am. Chem. Soc. 103: 3185, both incorporated herein by reference, or by other chemical methods using either a commercial automated oligonucleotide synthesizer or, for example, VLSIPS^{™} technology.

PCR is performed using template DNA (at least 1fg; more usefully, 1-1000 ng) and at least 25 pmol of oligonucleotide primers; it may be advantageous to use a larger amount of primer when the primer pool is heavily heterogeneous, as each sequence is represented by only a small fraction of the molecules of the pool, and amounts become limiting in the later amplification cycles. A typical reaction mixture includes: 2 µl of DNA, 25 pmol of oligonucleotide primer, 2.5 µl of 10X PCR buffer 1 (Perkin-Elmer), 0.4 µl of 1.25 µM dNTP, 0.15 µl (or 2.5 units) of Taq DNA polymerase (Perkin Elmer) and deionized water to a total volume of 25 µl. Mineral oil is overlaid and the PCR is performed using a programmable thermal cycler.

The length and temperature of each step of a PCR cycle, as well as the number of cycles, is adjusted in accordance to the stringency requirements in effect. Annealing temperature and timing are determined both by the efficiency with which a primer is expected to anneal to a template and the degree of mismatch that is to be tolerated; obviously, when nucleic acid molecules are simultaneously amplified and mutagenized, mismatch is required, at least in the first round of synthesis. In attempting to amplify a population of molecules using a mixed pool of mutagenic primers, the loss, under stringent (high-temperature) annealing conditions, of potential mutant products that would only result from low melting temperatures is weighed against the promiscuous annealing of primers to sequences other than the target site. The ability to optimize the stringency of primer annealing conditions is well within the knowledge of one of skill in the art. An annealing temperature of between 30°C and 72°C is used. Initial denaturation of the template molecules normally occurs at between 92°C and 99°C for 4 minutes, followed by 20-40 cycles consisting of denaturation (94-99°C for 15 seconds to 1 minute), annealing (temperature determined as discussed above; 1-2 minutes), and extension (72°C for 1-5 minutes, depending on the length of the amplified product). Final extension is generally for 4 minutes at 72°C, and may be followed by an indefinite (0-24 hour) step at 4°C.

### Screening dAbs for Antigen Binding:

Following expression of a repertoire of dAbs on the surface of phage, selection is performed by contacting the phage repertoire with immobilized target antigen, washing to remove unbound phage, and propagation of the bound phage, the whole process frequently referred to as "panning." Alternatively, phage are pre-selected for the expression of properly folded member variants by panning against an immobilized generic ligand (e.g., protein A or protein L) that is only bound by folded members. This has the advantage of reducing the proportion of non-functional members, thereby increasing the proportion of members likely to bind a target antigen. Pre-selection with generic ligands is taught in WO 99/20749. The screening of phage antibody libraries is generally described, for example, by Harrison et al., 1996, Meth. Enzymol. 267: 83-109.

Screening is commonly performed using purified antigen immobilized on a solid support, for example, plastic tubes or wells, or on a chromatography matrix, for example Sepharose^{™} (Pharmacia). Screening or selection can also be performed on complex antigens, such as the surface of cells (Marks et al., 1993, BioTechnology 11: 1145; de Kruif et al., 1995, Proc. Natl. Acad. Sci. U.S.A. 92: 3938). Another alternative involves selection by binding biotinylated antigen in solution, followed by capture on streptavidin-coated beads.

In a preferred aspect, panning is performed by immobilizing antigen (generic or specific) on tubes or wells in a plate, e.g., Nunc MAXISORP^{™} immunotube 8 well strips. Wells are coated with 150 µl of antigen (100 µg/ml in PBS) and incubated overnight.
The wells are then washed 3 times with PBS and blocked with 400 µl PBS-2% skim milk (2%MPBS) at 37°C for 2 hr. The wells are rinsed 3 times with PBS and phage are added in 2%MPBS. The mixture is incubated at room temperature for 90 minutes and the liquid, containing unbound phage, is removed. Wells are rinsed 10 times with PBS-0.1% tween 20, and then 10 times with PBS to remove detergent. Bound phage are eluted by adding 200 µl of freshly prepared 100 mM triethylamine, mixing well and incubating for 10 min at room temperature. Eluted phage are transferred to a tube containing 100 µl of 1M Tris-HCl, pH 7.4 and vortexed to neutralize the triethylamine. Exponentially-growing E. coli host cells (e.g., TG1) are infected with, for example, 150 ml of the eluted phage by incubating for 30 min at 37°C. Infected cells are spun down, resuspended in fresh medium and plated in top agarose. Phage plaques are eluted or picked into fresh cultures of host cells to propagate for analysis or for further rounds of selection. One or more rounds of plaque purification are performed if necessary to ensure pure populations of selected phage. Other screening approaches are described by Harrison et al., 1996, *supra.*

Following identification of phage expressing a single immunoglobulin variable domain that binds a desired target, if a phagemid vector such as pHEN1 has been used, the variable domain fusion protein are easily produced in soluble form by infecting non-suppressor strains of bacteria, e.g., HB2151 that permit the secretion of soluble gene III fusion protein. Alternatively, the V domain sequence can be sub-cloned into an appropriate expression vector to produce soluble protein according to methods known in the art.

### Purification and Concentration of dAbs:

dAb polypeptides secreted into the periplasmic space or into the medium of bacteria are harvested and purified according to known methods (Harrison et al., 1996, supra). Skerra & Pluckthun (1988, Science 240: 1038) and Breitling et al. (1991, Gene 104: 147) describe the harvest of antibody polypeptides from the periplasm, and Better et al. (1988, Science 240: 1041) describes harvest from the culture supernatant. Purification can also be achieved by binding to generic ligands, such as protein A or Protein L. Alternatively, the variable domains can be expressed with a peptide tag, e.g., the Myc, HA or 6X-His tags, which facilitates purification by affinity chromatography.

Polypeptides are concentrated by several methods well known in the art, including, for example, ultrafiltration, diafiltration and tangential flow filtration. The process of ultrafiltration uses semi-permeable membranes and pressure to separate molecular species on the basis of size and shape. The pressure is provided by gas pressure or by centrifugation. Commercial ultrafiltration products are widely available, e.g., from Millipore (Bedford, MA; examples include the Centricon^{™} and Microcon^{™} concentrators) and Vivascience (Hannover, Germany; examples include the Vivaspin^{™} concentrators). By selection of a molecular weight cutoff smaller than the target polypeptide (usually 1/3 to 1/6 the molecular weight of the target polypeptide, although differences of as little as 10 kD can be used successfully), the polypeptide is retained when solvent and smaller solutes pass through the membrane. Thus, a molecular weight cutoff of about 5 kD is useful for concentration of dAb polypeptides described herein.

Diafiltration, which uses ultrafiltration membranes with a "washing" process, is used where it is desired to remove or exchange the salt or buffer in a polypeptide preparation. The polypeptide is concentrated by the passage of solvent and small solutes through the membrane, and remaining salts or buffer are removed by dilution of the retained polypeptide with a new buffer or salt solution or water, as desired, accompanied by continued ultrafiltration. In continuous diafiltration, new buffer is added at the same rate that filtrate passes through the membrane. A diafiltration volume is the volume of polypeptide solution prior to the start of diafiltration - using continuous diafiltration, greater than 99.5% of a fully permeable solute can be removed by washing through six diafiltration volumes with the new buffer. Alternatively, the process can be performed in a discontinuous manner, wherein the sample is repeatedly diluted and then filtered back to its original volume to remove or exchange salt or buffer and ultimately concentrate the polypeptide. Equipment for diafiltration and detailed methodologies for its use are available, for example, from Pall Life Sciences (Ann Arbor, MI) and Sartorius AGNivascience (Hannover, Germany).

Tangential flow filtration (TFF), also known as "cross-flow filtration," also uses ultrafiltration membrane. Fluid containing the target polypeptide is pumped tangentially along the surface of the membrane. The pressure causes a portion of the fluid to pass through the membrane while the target polypeptide is retained above the filter. In contrast to standard ultrafiltration, however, the retained molecules do not accumulate on the surface of the membrane, but are carried along by the tangential flow. The solution that does not pass through the filter (containing the target polypeptide) can be repeatedly circulated across the membrane to achieve the desired degree of concentration. Equipment for TFF and detailed methodologies for its use are available, for example, from Millipore (e.g., the ProFlux M12™ Benchtop TFF system and the Pellicon^{™} systems), Pall Life Sciences (e.g., the Minim^{™} Tangential Flow Filtration system).

Protein concentration is measured in a number of ways that are well known in the art. These include, for example, amino acid analysis, absorbance at 280 nm, the "Bradford" and "Lowry" methods, and SDS-PAGE. The most accurate method is total hydrolysis followed by amino acid analysis by HPLC, concentration is then determined through comparison with the known sequence of the dAb polypeptide. While this method is the most accurate, it is expensive and time-consuming. Protein determination by measurement of UV absorbance at 280 nm is faster and much less expensive, yet relatively accurate and is preferred as a compromise over amino acid analysis. Absorbance at 280 nm was used to determine protein concentrations reported in the Examples described herein.

"Bradford" and "Lowry" protein assays (Bradford, 1976, Anal. Biochem. 72: 248-254; Lowry et al., 1951, J. Biol. Chem. 193: 265-275) compare sample protein concentration to a standard curve most often based on bovine serum albumin (BSA). These methods are less accurate, tending to undersetimate the concentration of single immunoglobulin variable domains. Their accuracy could be improved, however, by using a V_{H} or V_{κ} single domain polypeptide as a standard.

An additional protein assay method is the bicinchoninic acid assay described in U.S. Patent No. 4,839,295 (incorporated herein by reference) and marketed by Pierce Biotechnology (Rockford, IL) as the "BCA Protein Assay" (e.g., Pierce Catalog No. 23227).

The SDS-PAGE method uses gel electrophoresis and Coomassie Blue staining in comparison to known concentration standards, e.g., known amounts of a single immunoglobulin variable domain polypeptide. Quantitation can be done by eye or by densitometry.

In a third aspect, the invention provides a method for producing a ligand comprising a first immunoglobulin single variable domain having a first binding specificity and a second single immunoglobulin single variable domain having a second (different) binding specificity, one or both of the binding specificities being specific for an antigen which increases the half-life of the ligand in vivo, the method comprising the steps of: (a) selecting a first variable domain by its ability to bind to a first epitope, (b) selecting a second variable region by its ability to bind to a second epitope, (c) combining the variable domains; and (d) selecting the ligand by its ability to bind to said first epitope and to said second epitope.

The ligand can bind to the first and second epitopes either simultaneously or, where there is competition between the binding domains for epitope binding, the binding of one domain may preclude the binding of another domain to its cognate epitope. In one embodiment, therefore, step (d) above requires simultaneous binding to both first and second (and possibly further) epitopes; in another embodiment, the binding to the first and second epitoes is not simultaneous.

The epitopes are preferably on separate antigens.

Ligands advantageously comprise VHNL combinations, or VHNH or VLNL combinations of immunoglobulin variable domains, as described above. The ligands may moreover comprise camelid VHH domains, provided that the VHH domain which is specific for an antigen which increases the half-life of the ligand in vivo does not bind Hen egg white lysozyme (HEL), porcine pancreatic alpha-amylase or NmC-A; hog, BSA-linked RR6 ado 5 dye or S. mutates HG982 cells, as described in Conrath et al., (2001) JBC 276:7346- 7350 and WO99/23221, neither of which describe the use of a specificity for an antigen which increases half-life to increase the half life of the ligand in vivo.

In one embodiment, said first variable domain is selected for binding to said first epitope in absence of a complementary variable domain (i.e., it is selected as a dAb as described herein aboye). In a further embodiment, said first variable domain is selected for binding to said first epitope/antigen in the presence of a third variable domain in which said third variable domain is different from said second variable domain and is complementary to the first domain. Similarly, the second domain may be selected in the absence or presence of a complementary variable domain.

The antigens or epitopes targeted by the ligands of the invention, in addition to the half life enhancing protein, may be any antigen or epitope but advantageously is an antigen or epitope that is targeted with therapeutic benefit. The invention provides ligands, including open conformation, closed conformation and isolated dAb monomer ligands, specific for any such target, particularly those targets further identified herein. Such targets may be, or be part of, polypeptides, proteins or nucleic acids, which may be naturally occurring or synthetic. In this respect, the ligand of the invention may bind the epiotpe or antigen and act as an antagonist or agonist (eg, EPO receptor agonist). One skilled in the art will appreciate that the choice is large and varied.

They may be for instance human or animal proteins, cytokines, cytokine receptors, enzymes co-factors for enzymes or DNA binding proteins. Suitable cytokines and growth factors that can be targeted by mono- or dual-specific binding polypeptides as described herein include but are not limited to: ApoE, Apo-SAA, BDNF, BLyS, Cardiotrophin-1, EGF, EGF receptor, ENA-78, Eotaxin, Eotaxin-2, Exodus-2, EpoR, FGF-acidic, FGF-basic, fibroblast growth factor- 10, FLT3 ligand, Fractalkine (CX3C), GDNF, G-CSF, GM-CSF, GF-01, insulin, IFN-y, IGF-I, IGF-II, IL-, IL-1p, 20 IL-2, IL-3, IL-4, IL-5, IL-6, IL-7, IL-8 (72 a.a.), IL-8 (77 a.a.), IL-9, IL-10, IL-11, IL-12, IL-13, IL-15, IL-16, IL-17, IL-18 (IGIF), Inhibin α, Inhibin B IP-10, keratinocyte growth factor-2 (KGF-2), KGF, Leptin, LIF, Lymphotactin, Mullerian inhibitory substance, monocyte colony inhibitory factor, monocyte attractant protein, M-CSF, MDC (67 a.a.), MDC (69 a.a.), MCP-1 (MCAF), MCP-2, MCP-3, MCP-4, MIG, MIP1α, MIP1β, MIP3α, MIP3β, MIP-4, myeloid progenitor inhibitor factor-1 (MPIF-1), NAP-2, Neurturin, Nerve growth factor, β-NGF, NT-3, NT-4, Oncostatin M, PDGF-AA, PDGF-AB, PDGF-BB, PF-4, RANTES, SDF12, SDF1β, SCF, SCGF, stem cell factor (SCF), TARC, TGF-α, TGF-β, TGF-β2, TGF-β3, tumour necrosis factor (TNF), TNF-α, TNF-β, TNF receptor I, TNF receptor II, TNIL-1, TPO, VEGF, VEGF receptor 1, VEGF receptor 2, VEGF receptor 3, GCP-2, GRO/MGSA, GRO-β, GRO-8, HCC1, 1-309, HER 1, HER 2, HER 3, HER 4, TACE recognition site, TNF BP-I and TNF BP-II, as well as any target disclosed in Annex 2 or Annex 3 hereto, whether in combination as set forth in the Annexes, in a different combination, or individually.

As noted, preferred ligands include TNF-α and VEGF, alone, together, and/or with anti-HSA binding activity.

Cytokine receptors include receptors for the foregoing cytokines. It will be appreciated that this list is by no means exhaustive.

In one embodiment of the invention, the variable domains are derived from a respective antibody directed against the antigen or epitope. In a preferred embodiment the variable domains are derived from a repertoire of single variable antibody domains.

In a further aspect, the present invention provides one or more nucleic acid molecules encoding at least a dual-specific ligand as herein defined.

The dual specific ligand may be encoded on a single nucleic acid molecule; alternatively, each domain may be encoded by a separate nucleic acid molecule. Where the ligand is encoded by a single nucleic acid molecule, the domains may be expressed as a fusion polypeptide, in the manner of a scFv molecule, or may be separately expressed and subsequently linked together, for example using chemical linking agents. Ligands expressed from separate nucleic acids will be linked together by appropriate means.

The nucleic acid may further encode a signal sequence for export of the polypeptides from a host cell upon expression and may be fused with a surface component of a filamentous bacteriophage particle (or other component of a selection display system) upon expression.

In a further aspect the present invention provides a vector comprising nucleic acid encoding a dual specific ligand according to the present invention.

In a yet further aspect, the present invention provides a host cell transfected with a vector encoding a dual specific ligand according to the present invention.

Expression from such a vector may be configured to produce, for example on the surface of a bacteriophage particle, variable domains for selection. This allows selection of displayed variable regions and thus selection of 'dual-specific ligands' using the method of the present invention.

The present invention further provides a kit comprising at least a dual- specific ligand according to the present invention.

Dual-Specific ligands according to the present invention preferably comprise combinations of heavy and light chain domains. For example, the dual specific ligand may comprise a VH domain and a VL domain, which may be linked together in the form of an scFv. In addition, the ligands may comprise one or more CH or CL domains. For example, the ligands may comprise a CH1 domain, CH2 or CH3 domain, and/or a C_{L} domain, Cµ, Cµ2, Cµ3 or Cµ4 domains, or any combination thereof. A hinge region domain may also be included. Such combinations of domains may, for example, mimic natural antibodies, such as IgG or IgM, or fragments thereof, such as Fv, scFv, Fab or F(ab')2 molecules. Other structures, such as a single arm of an IgG molecule comprising VH, VL, CH1 and C_{L} domains, are envisaged.

In a preferred embodiment of the invention, the variable regions are selected from single domain V gene repertoires. Generally the repertoire of single antibody domains is displayed on the surface of filamentous bacteriophage. In a preferred embodiment each single antibody domain is selected by binding of a phage repertoire to antigen.

In a preferred embodiment of the invention each single variable domain may be selected for binding to its target antigen or epitope in the absence of a complementary variable region. In an alternative embodiment, the single variable domains may be selected for binding to its target antigen or epitope in the presence of a complementary variable region. Thus the first single variable domain may be selected in the presence of a third complementary variable domain, and the second variable domain may be selected in the presence of a fourth complementary variable domain. The complementary third or fourth variable domain may be the natural cognate variable domain having the same specificity as the single domain being tested, or a non-cognate complementary domain - such as a "dummy" variable domain.

Preferably, the dual specific ligand of the invention comprises only two variable domains although several such ligands may be incorporated together into the same protein, for example two such ligands can be incorporated into an IgG or a multimeric immunoglobulin, such as IgM. Alternatively, in another embodiment a plurality of dual specific ligands are combined to form a multimer. For example, two different dual specific ligands are combined to create a tetra-specific molecule.

It will be appreciated by one skilled in the art that the light and heavy variable regions of a dual-specific ligand produced according to the method of the present invention may be on the same polypeptide chain, or alternatively, on different polypeptide chains. In the case that the variable regions are on different polypeptide chains, then they may be linked via a linker, generally a flexible linker (such as a polypeptide chain), a chemical linking group, or any other method known in the art.

In a further aspect, the present invention provides a composition comprising a dual specific ligand, obtainable by a method of the present invention, and a pharmaceutically acceptable carrier, diluent or excipient.

Moreover, the present invention provides a method for the treatment and/or prevention of disease using a 'dual-specific ligand' or a composition according to the present invention.

In a second configuration, the present invention provides multispecific ligands which comprise at least two non-complementary variable domains. For example, the ligands may comprise a pair of VH domains or a pair of VL domains. Advantageously, the domains are of non-camelid origin; preferably they are human domains or comprise human framework regions (FWs) and one or more heterologous CDRs. CDRs and framework regions are those regions of an immunoglobulin variable domain as deemed in the Kabat database of Sequences of Proteins of Immunological Interest.

Preferred human framework regions are those encoded by germline gene segments DP47 and DPK9. Advantageously, FW1, FW2 and FW3 of a VH or VL domain have the sequence of FW1, FW2 or FW3 from DP47 or DPK9. The human frameworks may optionally contain mutations, for example up to about 5 amino acid changes or up to about 10 amino acid changes collectively in the human frameworks used in the ligands of the invention.

The variable domains in the multispecific ligands according to the second configuration of the invention may be arranged in an open or a closed conformation; that is, they may be arranged such that the variable domains can bind their cognate ligands independently and simultaneously, or such that only one of the variable domains may bind its cognate ligand at any one time.

The inventors have realised that under certain structural conditions, non-complementary variable domains (for example two light chain variable domains or two heavy chain variable domains) may be present in a ligand such that binding of a frst epitope to a first variable domain inhibits the binding of a second epitope to a second variable domain, even though such non-complementary domains do not operate together as a cognate pair.

Advantageously, the ligand comprises two or more pairs of variable domains; that is, it comprises at least four variable domains. Advantageously, the four variable domains comprise frameworks of human origin.

In a preferred embodiment, the human frameworks are identical to those of human germline sequences.

The present inventors consider that such antibodies will be of particular use in ligand binding assays for therapeutic and other uses.

Thus, in a first aspect of the second configuration, the present invention provides a method for producing a multispecific ligand comprising the steps of: a) selecting a first epitope binding domain by its ability to bind to a first epitope, b) selecting a second epitope binding domain by its ability to bind to a second epitope, c) combining the epitope binding domains; and d) selecting the closed conformation multispecific ligand by its ability to bind to said first second epitope and said second epitope.

In a further aspect of the second configuration, the invention provides method for preparing a closed conformation multi-specific ligand comprising a first epitope binding domain having a first epitope binding specificity and a non-complementary second epitope binding domain having a second epitope binding specificity, wherein the first and second binding specificities compete for epitope binding such that the closed conformation multi-specific ligand may not bind both epitopes simultaneously, said method comprising the steps of: a) selecting a first epitope binding domain by its ability to bind to a first epitope, b) selecting a second epitope binding domain by its ability to bind to a second epitope, c) combining the epitope binding domains such that the domains are in a closed conformation; and
d) selecting the closed conformation multispecific ligand by its ability to bind to said first second epitope and said second epitope, but not to both said first and second epitopes simultaneously.

Moreover, the invention provides a closed conformation multi-specific ligand comprising a first epitope binding domain having a first epitope binding specificity and a non-complementary second epitope binding domain having a second epitope binding specificity, wherein the first and second binding specificities compete for epitope binding such that the closed conformation multi- specific ligand may not bind both epitopes simultaneously.

An alternative embodiment of the above aspect of the of the second configuration of the invention optionally comprises a further step (bl) comprising selecting a third or further epitope binding domain. In this way the multi-specific ligand produced, whether of open or closed conformation, comprises more than two epitope binding specificities. In a preferred aspect of the second configuration of the invention, where the multi-specific ligand comprises more than two epitope binding domains, at least two of said domains are in a closed conformation and compete for binding; other domains may compete for binding or may be free to associate independently with their cognate epitope(s).

According to the present invention the term 'multi-specific ligand' refers to a ligand which possesses more than one epitope binding specificity as herein defined.

As herein defined the term 'closed conformation' (multi-specific ligand) means that the epitope binding domains of the ligand are attached to or associated with each other, optionally by means of a protein skeleton, such that epitope binding by one epitope binding domain competes with epitope binding by another epitope binding domain. That is, cognate epitopes may be bound by each epitope binding domain individually but not simultaneosuly. The closed conformation of the ligand can be achieved using methods herein described.

"Open conformation" means that the epitope binding domains of the ligand are attached to or associated with each other, optionally by means of a protein skeleton, such that epitope binding by one epitope binding domain does not compete with epitope binding by another epitope binding domain.

As referred to herein, the term 'competes' means that the binding of a first epitope to its cognate epitope binding domain is inhibited when a second epitope is bound to its cognate epitope binding domain. For example, binding may be inhibited sterically, for example by physical blocking of a binding domain or by alteration of the structure or environment of a binding domain such that its affinity or avidity for an epitope is reduced.

In a further embodiment of the second configuration of the invention, the epitopes may displace each other on binding. For example, a first epitope may be present on an antigen which, on binding to its cognate first binding domain, causes steric hindrance of a second binding domain, or a coformational change therein, which displaces the epitope bound to the second binding domain.

Advantageously, binding is reduced by 25% or more, advantageously 40%, 50%, 60%, 70%, 80%, 90% or more, and preferably up to 100% or nearly so, such that binding is completely inhibited. Binding of epitopes can be measured by conventional antigen binding assays, such as ELISA, by fluorescence based techniques, including FRET, or by techniques such as suface plasmon resonance which measure the mass of molecules.

According to the method of the present invention, advantageously, each epitope binding domain is of a different epitope binding specificity.

In the context of the present invention, first and second "epitopes" are understood to be epitopes which are not the same and are not bound by a single monospecific ligand. They may be on different antigens or on the same antigen, but separated by a sufficient distance that they do not form a single entity that could be bound by a single monospecific VHNL binding pair of a conventional antibody. Experimentally, if both of the individual variable domains in single chain antibody form (domain antibodies or dAbs) are separately competed by a monospecific VH/VL ligand against two epitopes then those two epitopes are not sufficiently far apart to be considered separate epitopes according to the present invention.

The closed conformation multispecific ligands of the invention do not include ligands as described in WO 02/02773. Thus, the ligands of the present invention do not comprise complementary VH/VL pairs which bind any one or more antigens or epitopes co-operatively. Instead, the ligands according to the invention preferably comprise non-complementary VH or VL pairs. Advantageously, each VH or VL domain in each VH or VL pair has a different epitope binding specificity, and the epitope binding sites are so arranged that the binding of an epitope at one site competes with the binding of an epitope at another site.

According to the present invention, advantageously, each epitope binding domain comprises an immunoglobulin variable domain. More advantageously, each immunoglobulin variable domain will be either a variable light chain domain (VL) or a variable heavy chain domain VH. In the second configuration of the present invention, the immunoglobulin domains when present on a ligand according to the present invention are non- complementary, that is they do not associate to form a VHNL antigen binding site.

Thus, multi-specific ligands as deemed in the second configuration of the invention comprise immunoglobulin domains of the same sub-type, that is either variable light chain domains (VL) or variable heavy chain domains (VH). Moreover, where the ligand according to the invention is in the closed conformation, the immunoglobulin domains may be of the camelid VHH type.

In an alternative embodiment, the ligand(s) according to the invention do not comprise a camelid VHH domain. More particularly, the ligand(s) of the invention do not comprise one or more amino acid residues that are specifc to camelid VHH domains as compared to human VH domains.

Advantageously, the single variable domains are derived from antibodies selected for binding activity against different antigens or epitopes. For example, the variable domains may be isolated at least in part by human immunisation. Alternative methods are known in the art, including isolation from human antibody libraries and synthesis of artificial antibody genes.

The variable domains advantageously bind superantigens, such as protein A or protein L. Binding to superantigens is a property of correctly folded antibody variable domains, and allows such domains to be isolated from, for example, libraries of recombinant or mutant domains. Epitope binding domains according to the present invention comprise a protein scaffold and epitope interaction sites (which are advantageously on the surface of the protein scaffold). Epitope binding domains may also be based on protein scaffolds or skeletons other than immunoglobulin domains. For example natural bacterial receptors such as SpA have been used as scaffolds for the grafting of CDRs to generate ligands which bind specifically to one or more epitopes. Details of this procedure are described in US 5,831,012. Other suitable scaffolds include those based on fibronectin and affibodies. Details of suitable procedures are described in WO 98/58965. Other suitable scaffolds include lipocallin and CTLA4, as described in van den Beuken et al., J. Mol. Biol. (2001) 310, 591-601, and scaffolds such as those described in W00069907 (Medical Research Council), which are based for example on the ring structure of bacterial GroEL or other chaperone polypeptides. Protein scaffolds may be combined; for example, CDRs may be grafted on to a CTLA4 scaffold and used together with immunoglobulin VH or VL domains to form a multivalent ligand. Likewise, fibronectin, lipocallin and other scaffolds may be combined.

It will be appreciated by one skilled in the art that the epitope binding domains of a closed conformation multispecific ligand produced according to the method of the present invention may be on the same polypeptide chain, or alternatively, on different polypeptide chains. In the case that the vaiable regions are on different polypeptide chains, then they may be linked via a linker, advantageously a flexible linker (such as a polypeptide chain), a chemical linking group, or any other method known in the art.

The first and the second epitope binding domains may be associated either covalently or non-covalently. In the case that the domains are covalently associated, then the association may be mediated for example by disulphide bonds.

In the second configuration of the invention, the first and the second epitopes are preferably different. They may be, or be part of, polypeptides, proteins or nucleic acids, which may be naturally occurring or synthetic. In this respect, the ligand of the invention may bind an epitope or antigen and act as an antagonist or agonist (eg, EPO receptor agonist). The epitope binding domains of the ligand in one embodiment have the same epitope specificity, and may for example simultaneously bind their epitope when multiple copies of the epitope are present on the same antigen. In another embodiment, these epitopes are provided on different antigens such that the ligand can bind the epitopes and bridge the antigens. One skilled in the art will appreciate that the choice of epitopes and antigens is large and varied. They may be for instance human or animal proteins, cytokines, cytokine receptors, enzymes co-factors for enzymes or DNA binding proteins.

Suitable cytokines and growth factors that can be targeted by mono- or dual-specific binding polypeptides as described herein include but are not limited to: ApoE, Apo-SAA, BDNF, BLyS, Cardiotrophin-1, EGF, EGF receptor, ENA-78, Eotaxin, Eotaxin-2, Exodus-2, EpoR, FGF-acidic, FGF-basic, fibroblast growth factor- 10, FLT3 ligand, Fractalkine (CX3C), GDNF, G-CSF, GM-CSF, GF-01, insulin, IFN-y, IGF-I, IGF-II, IL-, IL-1p, 20 IL-2, IL-3, IL-4, IL-5, IL-6, IL-7, IL-8 (72 a.a.), IL-8 (77 a.a.), IL-9, IL-10, IL-11, IL-12, IL-13, IL-15, IL-16, IL-17, IL-18 (IGIF), Inhibin α, Inhibin B IP-10, keratinocyte growth factor-2 (KGF-2), KGF, Leptin, LIF, Lymphotactin, Mullerian inhibitory substance, monocyte colony inhibitory factor, monocyte attractant protein, M-CSF, MDC (67 a.a.), MDC (69 a.a.), MCP-1 (MCAF), MCP-2, MCP-3, MCP-4, MIG, MIP1α, MIP1β, MIP3α, MIP3β, MIP-4, myeloid progenitor inhibitor factor-1 (MPIF-1), NAP-2, Neurturin, Nerve growth factor, β-NGF, NT-3, NT-4, Oncostatin M, PDGF-AA, PDGF-AB, PDGF-BB, PF-4, RANTES, SDF 12, SDF1β, SCF, SCGF, stem cell factor (SCF), TARC, TGF-α, TGF-β, TGF-β2, TGF-β3, tumour necrosis factor (TNF), TNF-α, TNF-β, TNF receptor I, TNF receptor II, TNIL-1, TPO, VEGF, VEGF receptor 1, VEGF receptor 2, VEGF receptor 3, GCP-2, GRO/MGSA, GRO-β, GRO-8, HCC1, 1-309, HER 1, HER 2, HER 3, HER 4, TACE recognition site, TNF BP-I and TNF BP-II, as well as any target disclosed in Annex 2 OR Annex 3 hereto, whether in combination as set forth in the Annexes, in a different combination, or individually.

Cytokine receptors include receptors for the foregoing cytokines, e.g. IL- 1 R1; IL-GR; IL-10R; IL-18R, as well as receptors for cytokines set forth in Annex 2 or Annex 3 and also receptors disclosed in Annex 2 and 3.

It will be appreciated that this list is by no means exhaustive. Where the multispecific ligand binds to two epitopes (on the same or different antigens), the antigen(s) may be selected from this list.

Advantageously, dual specific ligands may be used to target cytokines and other molecules which cooperate synergistically in therapeutic situations in the body of an organism. The invention therefore provides a method for synergising the activity of two or more cytokines, comprising administering a dual specific ligand capable of binding to said two or more cytokines. In this aspect of the invention, the dual specific ligand may be any dual specific ligand, including a ligand composed of complementary and/or non-complementary domains, a ligand in an open conformation, and a ligand in a closed conformation. For example, this aspect of the invention relates to combinations of VH domains and VL domains, VH domains only and VL domains only.

Synergy in a therapeutic context may be achieved in a number of ways. For example, target combinations may be therapeutically active only if both targets are targeted by the ligand, whereas targeting one target alone is not therapeutically effective. In another embodiment, one target alone may provide some low or minimal therapeutic effect, but together with a second target the combination provides a synergistic increase in therapeutic effect.

Preferably, the cytokines bound by the dual specific ligands of this aspect of the invention are selected from the list shown in Annex 2.

Moreover, dual specific ligands may be used in oncology applications, where one specificity targets CD89, which is expressed by cytotoxic cells, and the other is tumor specific. Examples of tumor antigens which may be targetted are given in Annex 3.

In one embodiment of the second configuration of the invention, the variable domains are derived from an antibody directed against the first and/or second antigen or epitope. In a preferred embodiment the variable domains are derived from a repertoire of single variable antibody domains. In one example, the repertoire is a repertoire that is not created in an animal or a synthetic repertoire. In another example, the single variable domains are not isolated (at least in part) by animal immunization. Thus, the single domains can be isolated from a nerve library.

The second configuration of the invention, in another aspect, provides a multi-specific ligand comprising a first epitope binding domain having a first epitope binding specificity and a non-complementary second epitope binding domain having a second epitope binding specificity. The first and second binding specificities may be the same or different.

In a further aspect, the present invention provides a closed conformation multi-specific ligand comprising a first epitope binding domain having a first epitope binding specificity and a non-complementary second epitope binding domain having a second epitope binding specificity wherein the first and second binding specificities are capable of competing for epitope binding such that the closed conformation multi-specific ligand cannot bind both epitopes simultaneously.

In a still further aspect, the invention provides open conformation ligands comprising non-complementary binding domains, wherein the deomains are specific for a different epitope on the same target. Such ligands bind to targets with increased avidity.

Similarly, the invention provides multivalent ligands comprising non- complementary binding domains specific for the same epitope and directed to targets which comprise multiple copies of said epitope, such as IL-5, PDGF-AA, PDGF-BB, TGF β, TGF β2, TGF β3 and TNFα, for example human TNF Receptor 1 and human TNFα.

In a similar aspect, ligands according to the invention can be configured to bind individual epitopes with low affinity, such that binding to individual epitopes is not therapeutically significant; but the increased avidity resulting from binding to two epitopes provides a theapeutic benefit. In a particular example, epitopes may be targetted which are present individually on normal cell types, but present together only on abnormal or diseased cells, such as tumor cells. In such a situaton, only the abnormal or tumor diseased cells are effectively targetted by the bispecifc ligands according to the invention. Ligand specific for multiple copies of the same epitope, or adjacent epitopes, on the same target (known as chelating dAbs) may also be trimeric or polymeric (tertrameric or more) ligands comprising three, four or more non-complementary binding domains. For example, ligands may be constructed comprising three or four VH domains or VL domains.

Moreover, ligands are provided which bind to multisubunit targets, wherein each binding domain is specific for a subunit of said target. The ligand may be dimeric, trimeric or polymeric. Preferably, the multi-specific ligands according to the above aspects of the invention are obtainable by the method of the first aspect of the invention.

According to the above aspect of the second configuration of the invention, advantageously the first epitope binding domain and the second epitope binding domains are non-complementary immunoglobulin variable domains, as herein defined. That is either VH-VH or VL-VL variable domains.

Chelating dAbs in particular may be prepared according to a preferred aspect of the invention, namely the use of anchor dAbs, in which a library of dimeric, trimeric or multimeric dAbs is constructed using a vector which comprises a constant dAb upstream or downstream of a linker sequence, with a repertoire of second, third and further dAbs being inserted on the other side of the linker. For example, the anchor or guiding dAb may be TAR1-5 (VK), TAR1-27(V), TAR2h-5(VH) or TAR2h-6(VK).

In alternative methodologies, the use of linkers may be avoided, for example by the use of non-covalent bonding or natural affinity between binding domains such as VH and VL. The invention accordingly provides a method for preparing a chelating multimeric ligand comprising the steps of:
(a) providing a vector comprising a nucleic acid sequence encoding a single binding domain specific for a first epitope on a target;
(b) providing a vector encoding a repertoire comprising second binding domains specific for a second epitope on said target, which epitope can be the same or different to the first epitope, said second epitope being adjacent to said first epitope; and
(c) expressing said first and second binding domains; and
(d) isolating those combinations of first and second binding domains which combine together to produce a target-binding dimer.

The first and second epitopes are adjacent such that a multimeric ligand is capable of binding to both epitopes simultaneously. This provides the ligand with the advantages of increased avidity of binding. Where the epitopes are the same, the increased avidity is obtained by the presence of multiple copies of the epitope on the target, allowing at least two copies to be simultaneously bound in order to obtain the increased avidity effect.

The binding domains may be associated by several methods, as well as the use of linkers.

For example, the binding domains may comprise cys residues, avidin and streptavidin groups or other means for non-covalent attachment post- synthesis; those combinations which bind to the target efficiently will be isolated. Alternatively, a linker may be present between the first and second binding domains, which are expressed as a single polypeptide from a single vector, which comprises the first binding domain, the linker and a repertoire of second binding domains, for instance as described above.

In a preferred aspect, the first and second binding domains associate naturally when bound to antigen; for example, VH and VK domains, when bound to adjacent epitopes, will naturally associate in a three-way interaction to form a stable dimer. Such associated proteins can be isolated in a target binding assay. An advantage of this procedure is that only binding domains which bind to closely adjacent epitopes, in the correct conformation, will associate and thus be isolated as a result of their increased avidity for the target.

In an alternative embodiment of the above aspect of the second configuration of the invention, at least one epitope binding domain comprises a non-immunoglobulin 'protein scaffold' or'protein skeleton' as herein defined. Suitable non- immunoglobulin protein scaffolds include but are not limited to any of those selected from the group consisting of:
SpA, fbronectin, GroEL and other chaperones, lipocallin, CCTLA4 and affibodies, as set forth above.

According to the above aspect of the second configuration of the invention, advantageously, the epitope binding domains are attached to a'protein skeleton'.

Advantageously, a protein skeleton according to the invention is an immunoglobulin skeleton. According to the present invention, the term 'immunoglobulin skeleton' refers to a protein which comprises at least one immunoglobulin fold and which acts as a nucleus for one or more epitope binding domains, as defined herein.

Preferred "immunoglobulin skeletons" as herein defined includes any one or more of those selected from the following: an immunoglobulin molecule comprising at least (i) the CL (kappa or lambda subclass) domain of an antibody; or (ii) the CH1 domain of an antibody heavy chain; an immunoglobulin molecule comprising the CH1 and CH2 domains of an antibody heavy chain; an immunoglobulin molecule comprising the CH1, CH2 and CH3 domains of an antibody heavy chain; or any of the subset (ii) in conjunction with the CL (kappa or lambda subclass) domain of an antibody. A hinge region domain may also be included. Such combinations of domains may, for example, mimic natural antibodies, such as IgG or IgM, or fragments thereof, such as Fv, scFv, Fab or F(ab')2 molecules.

Those skilled in the art will be aware that this list is not intended to be exhaustive.

Linking of the skeleton to the epitope binding domains, as herein defined may be achieved at the polypeptide level, that is after expression of the nucleic acid encoding the skeleton and/or the epitope binding domains. Alternatively, the linking step may be performed at the nucleic acid level. Methods of linking a protein skeleton according to the present invention, to the one or more epitope binding domains include the use of protein chemistry and/or molecular biology techniques which will be familiar to those skilled in the art and are described herein.

Advantageously, the closed conformation multispecific ligand may comprise a first domain capable of binding a target molecule, and a second domain capable of binding a molecule or group which extends the half-life of the ligand. For example, the molecule or group may be a bulky agent, such as HSA or a cell matrix protein. As used herein, the phrase "molecule or group which extends the half-life of a ligand" refers to a molecule or chemical group which, when bound by a dual-specific ligand as described herein increases the in vivo half-life of such dual specific ligand when administered to an animal, relative to a ligand that does not bind that molecule or group. Examples of molecules or groups that extend the half- life of a ligand are described hereinbelow. In a preferred embodiment, the closed conformation multispecific ligand may be capable of binding the target molecule only on displacement of the half-life enhancing molecule or group. Thus, for example, a closed conformation multispecific ligand is maintained in circulation in the bloodstream of a subject by a bulky molecule such as HSA. When a target molecule is encountered, competition between the binding domains of the closed conformation multispecific ligand results in displacement of the HSA and binding of the target.

Ligands according to any aspect of the present invention, as well as dAb monomers useful in constructing such ligands, may advantageously dissociate from their cognate 20 target(s) with a K_{d} of 300nM to 5pM (ie, 3 x 10⁻⁷ to 5 x 10⁻¹²M), preferably 50nM to 20pM, or 5nM to 200pM or InM to 1OOpM, 1 x 10⁻⁷ M or less, 1 x 10⁻⁸ M or less, 1 x 10⁻⁹ M or less, 1 x 10⁻¹⁰ M or less, 1 x 10⁻¹¹ M or less; and/or a Koff rate constant of 5 x 10⁻¹ to 1 x 10⁻⁷ S⁻¹, preferably 1 x 10⁻² to 1 x 10⁻⁶ S⁻¹, or 5 x 10⁻³ to 1 x 10⁻⁵ S⁻¹, or 5 x 10⁻¹ S⁻¹ or less, or 1 x 10⁻² S⁻¹ or less, or 1 x 10⁻³ S⁻¹ or less, or 1 x 10⁻⁴ S⁻¹ or less, or 1 x 10⁻⁵ S⁻¹ or less, or 1 x 10⁻⁶ S⁻¹ or less as determined by surface plasmon resonance. The K_{d} rate constant is defined as K_{off}/Kₒₙ.

In particular the invention provides an anti-TNF-α dAb monomer (or dual specific ligand comprising such a dAb), homodimer, heterodimer or homotrimer ligand, wherein each dAb binds TNF-α. The ligand binds to TNF-α with a K_{d} of 300nM to 5pM (ie, 3 x 10⁻⁷ to 5 x 10⁻¹²M), preferably 50nM to 20pM, more preferably 5nM to 200pM and most preferably 1nM to 100pM; expressed in an alternative manner, the K_{d} is 1 x 10⁻⁷ M or less, preferably 1 x 10⁻⁸ M or less, more preferably 1 x 10⁻⁹ M or less, advantageously 1 x 10⁻¹⁰ M or less and most preferably 1 x 10⁻¹¹ M or less; and/or a K_{off} rate constant of 5 x 10⁻¹ to 1 x 10⁻⁷ S⁻¹, preferably 1 x 10⁻² to 1 x 10⁻⁶ S⁻¹, more preferably 5 x 10⁻³ to 1 x 10⁻⁵ S⁻¹, for example 5 x 10⁻¹S⁻¹ or less, preferably 1 x 10⁻² S⁻¹ or less, more preferably 1 x 10⁻³ S⁻¹ or less, advantageously 1 X 10⁻⁴ S⁻¹ or less, further advantageously 1 x 10⁻⁵ S⁻¹ or less, and most preferably 1 x 10⁻⁶ S⁻¹ or less, as determined by surface plasmon resonance.

Preferably, the ligand neutralises TNF-α in a standard L929 assay with an ND50 of 500nM to 50pM, preferably or 100nM to 50pM, advantageously 10nM to 100pM, more preferably 1nM to 100pM; for example 50nM or less, preferably 5nM or less, advantageously 500pM or less, more preferably 200pM or less and most preferably 100pM or less.

Preferably, the ligand inhibits binding of TNF-α to TNF-α Receptor I (p55 receptor) with an IC50 of 500nM to 50pM, preferably 100nM to 50pM, more preferably 5 10nM to 100pM, advantageously InM to 100pM; for example 50nM or less, preferably 5nM or less, more preferably 500pM or less, advantageously 200pM or less, and most preferably 100pM or less. Preferably, the TNF-α is Human TNF-α.

Furthermore, the invention provides an anti-TNF Receptor I dAb monomer, or dual specific ligand comprising such a dAb, that binds to TNF Receptor I with a K_{d} of 300nM to 5pM (ie, 3 x 10⁻⁷ to 5 x 10⁻¹²M), preferably 50nM to20pM, more preferably 5nM to 200pM and most preferably 1nM to 100pM, for example 1 x 10⁻⁷ M or less, preferably 1 x 10⁻⁸ M or less, more preferably 1 x 10⁻⁹ M or less, advantageously 1 x 10⁻¹⁰ M or less and most preferably 1 x 10⁻¹¹ M or less; and/or a K_{off} rate constant of 5 x 10⁻¹ to 1 x 10⁻⁷ S⁻¹, preferably 1 x 10⁻2 to 1 x 10⁻⁶ S⁻¹, more preferably 5 x 10⁻³ to 1 x 10⁻⁵ S⁻¹, for example 5 x 10⁻¹S⁻¹ or less, preferably 1 x 10⁻² S⁻¹ or less, more preferably 1 x 10⁻³ S⁻¹ or less, advantageously 1 x 10⁻⁴ S⁻¹ or less, further advantageously 1 x 10⁻⁵ S⁻¹ or less, and most preferably 1 x 10⁻⁶ S⁻¹ or less, as determined by surface plasmon resonance.

Preferably, the dAb monomer or ligand neutralises TNF-α in a standard assay (eg, the L929 or HeLa assays described herein) with an ND50 of 500nM to 50pM, preferably 100nM to 50pM, more preferably 10nM to 100pM, advantageously InM to 100pM; for example 50nM or less, preferably 5nM or less, more preferably 500pM or less, advantageously 200pM or less, and most preferably 100pM or less.

Preferably, the dAb monomer or ligand inhibits binding of TNF-α to TNF-α 5 Receptor I (p55 receptor) with an IC50 of 500nM to 50pM, preferably 100nM to 50pM, more preferably 10nM to 100pM, advantageously 1nM to 100pM; for example 50nM or less, preferably 5nM or less, more preferably 500pM or less, advantageously 200pM or less, and most preferably 100pM or less. Preferably, the TNF Receptor I target is Human TNF-α.

Furthermore, the invention provides a dAb monomer(or dual specific ligand comprising such a dAb) that binds to serum albumin (SA) with a K_{d} of 1nM to 500 µM (ie, 1 x 10⁻⁹ to 5 x 10⁻⁴), preferably 100nM to 10,uM. Preferably, for a dual specific ligand comprising a first anti-SA dAb and a second dAb to another target, the affinity (eg Kd and/or Koff as measured by surface plasmon resonance, eg using BiaCore) of the second dAb for its target is from 1 to 100000 times (preferably 100 to 100000, more preferably 1000 to 100000, or 10000 to 100000 times) the affinity of the first dAb for SA. For example, the first dAb binds SA with an affinity of approximately 10 µM, while the second dAb binds its target with an affinity of 100pM. Preferably, the serum albumin is human serum albumin (HSA).

In one embodiment, the first dAb (or a dAb monomer) binds SA (eg, HSA) with a K_{d} of approximately 50, preferably 70, and more preferably 100, 150 or 200 nM.

The invention moreover provides dimers, trimers and polymers of the aforementioned dAb monomers, in accordance with the foregoing aspect of the present invention.

Ligands according to the invention, including dAb monomers, dimers and trimers, can be linked to an antibody Fc region, comprising one or both of CH2 and CH3 domains, and optionally a hinge region. For example, vectors encoding ligands linked as a single nucleotide sequence to an Fc region may be used to prepare such polypeptides.

In a further aspect of the second configuration of the invention, the present invention provides one or more nucleic acid molecules encoding at least a multispecific ligand as herein defined. In one embodiment, the ligand is a closed conformation ligand. In another embodiment, it is an open conformation ligand. The multispecific ligand may be s encoded on a single nucleic acid molecule; alternatively, each epitope binding domain may be encoded by a separate nucleic acid molecule. Where the ligand is encoded by a single nucleic acid molecule, the domains may be expressed as a fusion polypeptide, or may be separately expressed and subsequently linked together, for example using chemical linking agents. Ligands expressed from separate nucleic acids will be linked together by appropriate means.

The nucleic acid may further encode a signal sequence for export of the polypeptides from a host cell upon expression and may be fused with a surface component of a filamentous bacteriophage particle (or other component of a selection display system) upon expression. Leader sequences, which may be used in bacterial expresion and/or phage or phagemid display, include pelB, stII, ompA, phoA, bla and pelA.

In a further aspect of the second configuration of the invention the present invention provides a vector comprising nucleic acid according to the present invention.

In a yet further aspect, the present invention provides a host cell transfected with a vector according to the present invention.

Expression from such a vector may be configured to produce, for example on the surface of a bacteriophage particle, epitope binding domains for selection. This allows selection of displayed domains and thus selection of 'multi specific ligands' using the method of the present invention.

In a preferred embodiment of the second configuration of the invention, the epitope binding domains are immunoglobulin variable regions and are selected from single domain V gene repertoires. Generally the repertoire of single antibody domains is displayed on the surface of filamentous bacteriophage. In a preferred embodiment each single antibody domain is selected by binding of a phage repertoire to antigen.

The present invention further provides a kit comprising at least a multispecific ligand according to the present invention, which may be an open conformation or closed conformation ligand. Kits according to the invention may be, for example, diagnostic kits, therapeutic kits, kits for the detection of chemical or biological species, and the like.

In further aspect still of the second configuration of the invention, the present invention provides a homogeneous immunoassay using a ligand according to the present invention.

In a further aspect still of the second configuration of the invention, the present invention provides a composition comprising a closed conformation multispecific ligand, obtainable by a method of the present invention, and a pharmaceutically acceptable carrier, diluent or excipient. Moreover, the present invention provides a method for the treatment of disease using a closed conformation multispecific ligand' or a composition according to the present invention. In a preferred embodiment of the invention the disease is cancer or an inflammatory disease, e.g. rheumatoid arthritis, asthma or Crohn's disease.

In a further aspect of the second configuration of the invention, the present invention provides a method for the diagnosis, including diagnosis of disease using a closed conformation multispecific ligand, or a composition according to the present invention.

Thus in general the binding of an analyte to a closed conformation multispecific ligand may be exploited to displace an agent, which leads to the generation of a signal on displacement. For example, binding of analyte (second antigen) could displace an enzyme (first antigen) bound to the antibody providing the basis for an immunoassay, especially if the enzyme were held to the antibody through its active site.

Thus in a final aspect of the second configuration, the present invention provides a method for detecting the presence of a target molecule, comprising:
(a) providing a closed conformation multispecifc ligand bound to an agent,
   said ligand being specific for the target molecule and the agent, wherein the agent which is bound by the ligand leads to the generation of a detectable signal on displacement from the ligand; (b) exposing the closed conformation multispecific ligand to the target molecule; and (c) detecting the signal generated as a result of the displacement of the agent.

According to the above aspect of the second configuration of the invention, advantageously, the agent is an enzyme, which is inactive when bound by the closed conformation multi-specific ligand. Alternatively, the agent may be any one or more selected from the group consisting of the following: the substrate for an enzyme, and a fluorescent, luminescent or chromogenic molecule which is inactive or quenched when bound by the ligand.

Sequences similar or homologous (e.g., at least about 70% sequence identity) to the sequences disclosed herein are also part of the invention. In some embodiments, the sequence identity at the amino acid level can be about 80%, 85%, 90%, 91%, 92%, 93%, 94%, 95%, 96%, 97%, 98%, 99% or higher. At the nucleic acid level, the sequence identity can be about 70%, 75%, 80%, 85%, 90%, 91%, 92%, 93%, 94%, 95%, 96%, 97%, 98%, 99% or higher. Alternatively, substantial identity exists when the nucleic acid segments will hybridize under selective hybridization conditions (e.g., very high stringency hybridization conditions), to the complement of the strand. The nucleic acids may be present in whole cells, in a cell lysate, or in a partially purified or substantially pure form.

Calculations of "homology" or "sequence identity" or "similarity" between two sequences (the terms are used interchangeably herein) are performed as follows. The sequences are aligned for optimal comparison purposes (e.g., gaps can be introduced in one or both of a first and a second amino acid or nucleic acid sequence for optimal alignment and non-homologous sequences can be disregarded for comparison purposes).

In a preferred embodiment, the length of a reference sequence aligned for comparison purposes is at least 30%, preferably at least 40%, more preferably at least 50%, even more preferably at least 60%, and even more preferably at least 70%, 80%, 90%, 100% of the length of the reference sequence. The amino acid residues or nucleotides at corresponding amino acid positions or nucleotide positions are then compared. When a position in the first sequence is occupied by the same amino acid residue or nucleotide as the corresponding position in the second sequence, then the molecules are identical at that position (as used herein amino acid or nucleic acid "homology" is equivalent to amino acid or nucleic acid "identity"). The percent identity between the two sequences is a function of the number of identical positions shared by the sequences, taking into account the number of gaps, and the length of each gap, which need to be introduced for optimal alignment of the two sequences.

Advantageously, the BLAST algorithm (version 2.0) is employed for sequence alignment, with parameters set to default values. The BLAST algorithm is described in detail at the world wide web site ("www") of the National Center for Biotechnology Information (".ncbi") of the National Institutes of Health ("nib") of the U.S. government (".gov"), in the "/Blast!" directory, in the "blast_help.html" file. The search parameters are defined as follows, and are advantageously set to the defined default parameters.

BLAST (Basic Local Alignment Search Tool) is the heuristic search algorithm employed by the programs blastp, blastn, blastx, tblastn, and tblastx; these programs ascribe significance to their findings using the statistical methods of Karlin and Altschul, 1990, 20 Proc. Natl. Acad. Sci. USA 87(6):2264-8 (see the "blast_help.html" file, as described above) with a few enhancements. The BLAST programs were tailored for sequence similarity searching, for example to identify homologues to a query sequence. The programs are not generally useful for motif-style searching. For a discussion of basic issues in similarity searching of sequence databases, see Altschul et al. (1994).

The five BLAST programs available at the National Center for Biotechnology Information web site perform the following tasks: "blastp" compares an amino acid query sequence against a protein sequence database; "blastn" compares a nucleotide query sequence against a nucleotide sequence database; "blastx" compares the six-frame conceptual translation products of a nucleotide query sequence (both strands) against a protein sequence database; "tblastn" compares a protein query sequence against a nucleotide sequence database dynamically translated in all six reading frames (both strands). "tblastx" compares the six-frame translations of a nucleotide query sequence against the six-frame translations of a nucleotide sequence database.

BLAST uses the following search parameters:
HISTOGRAM Display a histogram of scores for each search; default is yes. (See s parameter H in the BLAST Manual).
DESCRIPTIONS Restricts the number of short descriptions of matching sequences reported to the number specified; default limit is 100 descriptions. (See parameter V in the manual page). See also EXPECT and CUTOFF.
ALIGNMENTS Restricts database sequences to the number specified for which high scoring segment pairs (HSPs) are reported; the default limit is 50. If more database sequences than this happen to satisfy the statistical significance threshold for reporting (see EXPECT and CUTOFF below), only the matches ascribed the greatest statistical significance are reported. (See parameter B in the BLAST Manual).
EXPECT The statistical significance threshold for reporting matches against database sequences; the default value is 10 , such that 10 matches are expected to be found merely by chance, according to the stochastic model of Karlin and Altschul (1990). If the statistical significance ascribed to a match is greater than the EXPECT threshold, the match will not be reported. Lower EXPECT thresholds are more stringent, leading to fewer chance matches being reported. Fractional values are acceptable. (See parameter E in the BLAST Manual).
CUTOFF Cutoff score for reporting high-scoring segment pairs. The default value is calculated from the EXPECT value (see above). HSPs are reported for a database sequence only if the statistical significance ascribed to them is at least as high as would be ascribed to a lone HSP having a score equal to the CUTOFF value. Higher CUTOFF values are more stringent, leading to fewer chance matches being reported. (See parameter S in the BLAST Manual). Typically, significance thresholds can be more intuitively managed using EXPECT.
MATRIX Specify an alternate scoring matrix for BLASTP, BLASTX, TBLASTN and TBLASTX. The default matrix is BLOSUM62 (Henikoff & Henikoff, 1992, Proc. Natl. 30 Acad. Sci. USA 89(22):10915-9). The valid alternative choices include: PAM40, PAM120, PAM:250 and IDENTITY. No alternate scoring matrices are available for BLASTN; specifying the MATRIX directive in BLASTN requests returns an error response.
STRAND Restrict a TBLASTN search to just the top or bottom strand of the database sequences; or restrict a BLASTN, BLASTX or TBLASTX search to just reading frames on the top or bottom strand of the query sequence.
FILTER Mask off segments of the query sequence that have low compositional complexity, as determined by the SEG program of Wootton & Federhen (1993) Computers and Chemistry 17:149-163, or segments consisting of short-periodicity internal repeats, as determined by the XNU program of Claverie & States, 1993, Computers and Chemistry 17:191-201, or, for BLASTN, by the DUST program of Tatusov and Lipman (see the world wide web site of the NCBI). Filtering can eliminate statistically significant but biologically uninteresting reports from the blast output (e.g., hits against common acidic-, basic- or proline-rich regions), leaving the more biologically interesting regions of the query sequence available for specific matching against database sequences. Low complexity sequence found by a filter program is substituted using the letter "N" in nucleotide sequence (e.g., "N" repeated 13 times) and the letter "X" in protein sequences (e.g., "X" repeated 9 times).
Filtering is only applied to the query sequence (or its translation products), not to database sequences. Default filtering is DUST for BLASTN, SEG for other programs. It is not unusual for nothing at all to be masked by SEG, XNU, or both, when applied to sequences in SWISS-PROT, so filtering should not be expected to always yield an effect. Furthermore, in some cases, sequences are masked in their entirety, indicating that the statistical significance of any matches reported against the unfiltered query sequence should be suspect.
NCBI-gi Causes NCBI gi identifiers to be shown in the output, in addition to the accession and/or locus name.
Most preferably, sequence comparisons are conducted using the simple BLAST search algorithm provided at the NCBI world wide web site described above, in the "/BLAST" directory.

### Preparation of immunoglobulin based multi-specific ligands.

Dual specific ligands according to the invention, whether open or closed in conformation according to the desired configuration of the invention, may be prepared according to previously established techniques, used in the field of antibody engineering, for the preparation of scFv, "phage" antibodies and other engineered antibody molecules. Techniques for the preparation of antibodies, and in particular bispecific antibodies, are for example described in the following reviews and the references cited therein: Winter & Milstein, (1991) Nature 349:293-299; Pluckthun (1992) Immunological Reviews 5 130:151-188; Wright et al., (1992) Crit. Rev. Immunol.12:125- 168; Holliger, P. & Winter, G. (1993) Curr. Op. Biotechn. 4, 446-449; Carter, et al. (1995) J. Hematother. 4, 463-470; Chester, K.A. & Hawkins, R.E. (1995) Trends Biotechn. 13, 294-300; Hoogenboom, H.R. (1997) Nature Biotechnol. 15, 125-126, Fearon, D. (1997) Nature Biotechnol. 15, 618-619; Pluckthun, A. & Pack, P. (1997) Immunotechnology 3, 83-105; Carter, P. & Merchant, A.M. (1997) Curr. Opin. Biotechnol. 8, 449- 454; Holliger, P. & Winter, G. (1997) Cancer Immunol. Immunother. 45, 128-130.

The invention provides for the selection of variable domains against two different antigens or epitopes, and subsequent combination of the variable domains. The techniques employed for selection of the variable domains employ libraries and selection procedures which are known in the art. Natural libraries (Marks et al. (1991) J. Mol. Biol., 222: 581; Vaughan et al. (1996) Nature Biotech., 14: 309) which use rearranged V genes harvested from human B cells are well known to those skilled in the art. Synthetic libraries (Hoogenboom & Winter (1992) J. Mol. Biol., 227: 381; Barbas et al. (1992) Proc. Natl. Acad. Sci. USA, 89: 4457; Nissim et al. (1994) EMBO J., 13: 692; Griffiths et al. (1994) EMBO], 13: 3245; De Kruif et al. (1995) J. Mol. Biol., 248: 97) are prepared by cloning immunoglobulin V genes, usually using PCR. Errors in the PCR process can lead to a high degree of randomization. VH and/or VL libraries may be selected against target antigens or epitopes separately, in which case single domain binding is directly selected for, or together.

A preferred method for making a dual specific ligand according to the present invention comprises using a selection system in which a repertoire of variable domains is selected for binding to a first antigen or epitope and a repertoire of variable domains is selected for binding to a second antigen or epitope. The selected variable first and second variable domains are then combined and the dual-specific ligand selected for binding to both first and second antigen or epitope. Closed conformation ligands are selected for binding both first and second antigen or epitope in isolation but not simultaneously.

### A. Library vector systems.

A variety of selection systems are known in the art which are suitable for use in the present invention. Examples of such systems are described below.

Bacteriophage lambda expression systems may be screened directly as bacteriophage plaques or as colonies of lysogens, both as previously described (Muse et al. (1989) 20 Science, 246: 1275; Caton and Koprowski (1990) Proc. Natl. Acad. Sci. U.S.A., 87; Mullinax et al. (1990) Proc. Natl. Acad. Sci. U.S.A., 87: 8095; Persson et al. (1991) Proc. Natl. Acad. Sci. U.S.A., 88: 2432) and are of use in the invention. Whilst such expression systems can be used to screen up to 10⁶ different members of a library, they are not really suited to screening of larger numbers (greater than 10⁶ members).

Of particular use in the construction of libraries are selection display systems, which enable a nucleic acid to be linked to the polypeptide it expresses. As used herein, a selection display system is a system that permits the selection, by suitable display means, of the individual members of the library by binding the generic and/or target ligands.

Selection protocols for isolating desired members of large libraries are known in the art, as typified by phage display techniques. Such systems, in which diverse peptide sequences are displayed on the surface of filamentous bacteriophage (Scott and Smith (1990) Science, 249: 386), have proven useful for creating libraries of antibody fragments (and the nucleotide sequences that encoding them) for the in vitro selection and amplification of specific antibody fragments that bind a target antigen (McCafferty et al., WO 92/01047). The nucleotide sequences encoding the VH and VL regions are linked to s gene fragments which encode leader signals that direct them to the periplasmic space of E. col; and as a result the resultant antibody fragments are displayed on the surface of the bactenophage, typically as fusions to bacteriophage coat proteins (e.g., pIII or pVIII).

Alternatively, antibody fragments are displayed externally on lambda phage capsids (phagebodies). An advantage of phage-based display systems is that, because they are biological systems, selected library members can be amplified simply by growing the phage containing the selected library member in bacterial cells. Furthermore, since the nucleotide sequence that encode the polypeptide library member is contained on a phage or phagemid vector, sequencing, expression and subsequent genetic manipulation is relatively straightforward.

Methods for the construction of bacteriophage antibody display libraries and lambda phage expression libraries are well known in the art (McCafferty et al. (1990) Nature, 348: 552; Kang et al. (1991) Proc. Natl. Acad. Sci. U.S.A., 88: 4363; Clackson et al. (1991) Nature, 352: 624; Lowman et al. (1991) Biochemistry, 30: 10832; Burton et al. 20 (1991) Proc. Natl. Acad. Sci U.S.A., 88: 10134; Hoogenboom et al. (1991) Nucleic Acids Res., 19: 4133; Chang et al. (1991) J. Immunol., 147: 3610; Breitling et al. (1991) Gene, 104: 147; Marks et al. (1991) supra; Barbas et al. (1992) supra; Hawkins and Winter (1992) J. Immunol., 22: 867; Marks et al., 1992, J. Biol. Chem., 267: 16007; Lerner et al. (1992) Science, 258: 1313, incorporated herein by reference).

One particularly advantageous approach has been the use of scFv phage- libraries (Huston et al., 1988, Proc. Natl. Acad. Sci U.S.A., 85: 5879- 5883; Chaudhary et al. (1990) Proc. Natl. Acad. Sci U.S.A., 87: 1066-1070; McCafferty et al. (1990) supra; Clackson et al. (1991) Nature, 352: 624; Marks et al. (1991) J. Mol. Biol., 222: 581; Chiswell et al. 30 (1992) Trends Biotech., 10: 80; Marks et al. (1992) J. Biol. Chem., 267). Various embodiments of scFv libraries displayed on bacteriophage coat proteins have been described. Refinements of phage display approaches are also known, for example as described in W096/06213 and WO92/01047 (Medical Research Council et al.) and W097/08320 (Morphosys), which are incorporated herein by reference.

Other systems for generating libraries of polypeptides involve the use of cell-free enzymatic machinery for the in vitro synthesis of the library members. In one method, RNA molecules are selected by alternate rounds of selection against a target ligand and PCR amplification (Tuerk and Gold (1990) Science, 249: 505; Ellington and Szostak (1990) Nature, 346:818). A similar technique may be used to identify DNA sequences which bind a predetermined human transcription factor (Thiesen and Bach (1990) Nucleic Acids Res., 18: 3203; Beaudry and Joyce (1992) Science, 257: 635;W092/05258 and WO92/14843). In a similar way, in vitro translation can be used to synthesise polypeptides as a method for generating large libraries. These methods which generally comprise stabilised polysome complexes, are described further in W088/08453, WO90/05785,WO90/07003, WO91/02076,WO91/05058, and W092/02536. Alternative display systems which are not phage-based, such as those disclosed in WO95/22625 and WO95/11922 (Affymax) use the polysomes to display polypeptides for selection.

A still further category of techniques involves the selection of repertoires in artificial compartments, which allow the linkage of a gene with its gene product. For example, a selection system in which nucleic acids encoding desirable gene products may be selected in microcapsules formed by water-in-oil emulsions is described in WO99/02671,

WO00/40712 and Tawfik & Griffiths (1998) Nature Biotechnol 16(7), 652-6. Genetic elements encoding a gene product having a desired activity are compartmentalised into microcapsules and then transcribed and/or translated to produce their respective gene products (RNA or protein) within the microcapsules. Genetic elements which produce gene product having desired activity are subsequently sorted. This approach selects gene products of interest by detecting the desired activity by a variety of means.

### B. Library Construction.

Libraries intended for selection, may be constructed using techniques known in the art, for example as set forth above, or may be purchased from commercial sources. Libraries which are useful in the present invention are described, for example, in WO99/20749. Once a vector system is chosen and one or more nucleic acid sequences encoding polypeptides of interest are cloned into the library vector, one may generate diversity within the cloned molecules by undertaking mutagenesis prior to expression;
alternatively, the encoded proteins may be expressed and selected, as described above, before mutagenesis and additional rounds of selection are performed. Mutagenesis of nucleic acid sequences encoding structurally optimised polypeptides is carried out by standard molecular methods. Of particular use is the polymerase chain reaction, or PCR, (Mullis and Faloona (1987) Methods E'nzymol., 155: 335, herein incorporated by reference). PCR, which uses multiple cycles of DNA replication catalysed by a thermostable, DNA-dependent DNA polymerase to amplify the target sequence of interest, is well known in the art. The construction of various antibody libraries has been discussed in Winter et al. (1994) Ann. Rev. Immunology 12, 433-55, and references cited therein.

PCR is performed using template DNA (at least lfg; more usefully, 1-1000 ng) and at least 25 pmol of oligonucleotide primers; it may be advantageous to use a larger amount of primer when the primer pool is heavily heterogeneous, as each sequence is represented by only a small fraction of the molecules of the pool, and amounts become limiting in the later amplification cycles. A typical reaction mixture includes: 2µl of DNA, 25 pmol of oligonucleotide primer, 2.5 µl of 10X PCR buffer 1 (Perkin-Elmer, Foster City, CA), 0.4 µl of 1.25 µM dNTP, 0.15 µl (or 2.5 units) of Taq DNA polymerase (Perkin Elmer, Foster City, CA) and deionized water to a total volume of 25 µl. Mineral oil is overlaid and the PCR is performed using a programmable thermal cycler. The length and temperature of each step of a PCR cycle, as well as the number of cycles, is adjusted in accordance to the stringency requirements in effect. Annealing temperature and timing are determined both by the efficiency with which a primer is expected to anneal to a template and the degree of mismatch that is to be tolerated; obviously, when nucleic acid molecules are simultaneously amplified and mutagenised, mismatch is required, at least in the first round of synthesis. The ability to optimise the stringency of primer annealing conditions is well within the knowledge of one of moderate skill in the art. An annealing temperature of between 30 °C and 72 °C is used. Initial denaturation of the template molecules normally occurs at between 92°C and 99°C for 4 minutes, followed by 20-40 cycles consisting of denaturation (94-99°C for 15 seconds to 1 minute), annealing (temperature determined as discussed above; 1-2 minutes), and extension (72°C for 1-5 minutes, depending on the length of the amplified product). Final extension is generally for 4 minutes at 72°C, and may be followed by an indefinite (0-24 hour) step at 4°C.

### C. Combining single variable domains.

Domains useful in the invention, once selected, may be combined by a variety of methods known in the art, including covalent and non-covalent methods.

Preferred methods include the use of polypeptide linkers, as described, for example, in connection with scFv molecules (Bird et al., (1988) Science 242:423-426). Discussion of suitable linkers is provided in Bird et al. Science 242, 423-426; Hudson et al, Journal Immunol. Methods 231 (1999) 177-189; Hudson et al, Proc Nat Acad Sci USA 85, 5879 5883. Linkers are preferably flexible, allowing the two single domains to interact. One linker example is a (Gly4 Ser)n linker, where n=1 to 8, eg, 2, 3, 4, 5 or 7. The linkers used in diabodies, which are less flexible, may also be employed (Holliger et al., (1993) PNAS (USA) 90:6444-6448).

In one embodiment, the linker employed is not an immunoglobulin hinge region.

Variable domains may be combined using methods other than linkers. For example, the use of disulphide bridges, provided through naturally- occurring or engineered cysteine residues, may be exploited to stabilise V_{H}-V_{H}, V_{L}-V_{L}, or V_{H}-V_{L} dimers (Reiter et al., (1994) Protein Eng. 7:697- 704) or by remodelling the interface between the variable domains to improve the "fit" and thus the stability of interaction (Ridgeway et al., (1996) Protein Eng. 7:617-621; Zhu et al., (1997) Protein Science 6:781- 788).

Other techniques for joining or stabilising variable domains of immunoglobulins, and in particular antibody VH domains, may be employed as appropriate.

In accordance with the present invention, dual specific ligands can be in "closed" conformations in solution. A "closed" configuration is that in which the two domains (for example VH and VL) are present in associated form, such as that of an associated VL pair which forms an antibody binding site. For example, scFv may be in a closed conformation, depending on the arrangement of the linker used to link the VH and VL domains. If this is sufficiently flexible to allow the domains to associate, or rigidly holds them in the associated position, it is likely that the domains will adopt a closed conformation.

Similarly, VH domain pairs and VL domain pairs may exist in a closed conformation. Generally, this will be a function of close association of the domains, such as by a rigid linker, in the ligand molecule. Ligands in a closed conformation will be unable to bind both the molecule which increases the half-life of the ligand and a second target molecule. Thus, the ligand will typically only bind the second target molecule on dissociation from the molecule which increases the half-life of the ligand.

Moreover, the construction of VHNH, VLNL or VH/VL dimers without linkers provides for competition between the domains.

Ligands according to the invention may moreover be in an open conformation. In such a conformation, the ligands will be able to simultaneously bind both the molecule which increases the half-life of the ligand and the second target molecule. Typically, variable domains in an open configuration are (in the case of VH VL pairs) held far enough apart for the domains not to interact and form an antibody binding site and not to compete for binding to their respective epitopes. In the case of VH/VH or VI/VL dimers, the domains are not forced together by rigid linkers. Naturally, such domain pairings will not compete for antigen binding or form an antibody binding site.

Fab fragments and whole antibodies will exist primarily in the closed conformation, although it will be appreciated that open and closed dual specific ligands are likely to exist in a variety of equilibria under different circumstances. Binding of the ligand to a target is likely to shift the balance of the equilibrium towards the open configuration. Thus, certain ligands according to the invention can exist in two conformations in solution, one of which (the open form) can bind two antigens or epitopes independently, whilst the alternative conformation (the closed form) can only bind one antigen or epitope; antigens or epitopes thus compete for binding to the ligand in this conformation.

Although the open form of the dual specific ligand may thus exist in equilibrium with the closed form in solution, it is envisaged that the equilibrium will favour the closed form; moreover, the open form can be sequestered by target binding into a closed conformation. Preferably, therefore, certain dual specific ligands of the invention are present in an equilibrium between two (open and closed) conformations.

Dual specific ligands according to the invention may be modified in order to favour an open or closed conformation. For example, stabilisation of V_{H}-V_{L} interactions with disulphide bonds stabilises the closed conformation. Moreover, linkers used to join the domains, including VH domain and V_{L} domain pairs, may be constructed such that the open from is favoured; for example, the linkers may sterically hinder the association of the domains, such as by incorporation of large amino acid residues in opportune locations, or the designing of a suitable rigid structure which will keep the domains physically spaced apart.

### D. Characterisation of the dual-specific ligand.

The binding of the dual-specific ligand to its specific antigens or epitopes can be tested by methods which will be familiar to those skilled in the art and include ELISA. In a preferred embodiment of the invention binding is tested using monoclonal phage ELISA.

Phage ELISA may be performed according to any suitable procedure: an exemplary protocol is set forth below.

Populations of phage produced at each round of selection can be screened for binding by ELISA to the selected antigen or epitope, to identify "polyclonal" phage antibodies. Phage from single infected bacterial colonies from these populations can then be screened by ELISA to identify "monoclonal" phage antibodies. It is also desirable to screen soluble antibody fragments for binding to antigen or epitope, and this can also be undertaken by ELISA using reagents, for example, against a C- or N-terminal tag (see for example Winter et al. (1994) Ann. Rev. Immunology 12, 433-55 and references cited therein).

The diversity of the selected phage monoclonal antibodies may also be assessed by gel 5 electrophoresis of PCR products (Marks et al. 1991, supra; Nissim et al. 1994 supra), probing (Tomlinson et al., 1992) J. Mol. Biol. 227, 776) or by sequencing of the vector DNA.

### E. Structure of 'Dual-specific ligands'.

As described above, an antibody is herein defined as an antibody (for example IgG, IgM, IgA, IgA, IgE) or fragment (Fab, Fv, disulphide linked Fv, scFv, diabody) which comprises at least one heavy and a light chain variable domain, at least two heavy chain variable domains or at least two light chain variable domains. (The term antibody also encompasses a dAb). It may be at least partly derived from any species naturally producing an antibody, or created by recombinant DNA technology; whether isolated from serum, B-cells, hybridomas, transfectomas, yeast or bacteria).

In a preferred embodiment of the invention the dual-specific ligand comprises at least one 20 single heavy chain variable domain of an antibody and one single light chain variable domain of an antibody, or two single heavy or light chain variable domains. For example, the ligand may comprise a VH/VL pair, a pair of VH domains or a pair of V_{L} domains.

The first and the second variable domains of such a ligand may be on the same polypeptide chain. Alternatively they may be on separate polypeptide chains. In the case that they are on the same polypeptide chain they may be linked by a linker, which is preferentially a peptide sequence, as described above.

The first and second variable domains may be covalently or non-covalently associated. In the case that they are covalently associated, the covalent bonds may be disulphide bonds.

In the case that the variable domains are selected from V-gene repertoires selected for instance using phage display technology as herein described, then these variable domains comprise a universal framework region, such that is they may be recognised by a specific generic ligand as herein defined. The use of universal frameworks, generic ligands and the like is described in WO99/20749.

Where V-gene repertoires are used variation in polypeptide sequence is preferably located within the structural loops of the variable domains. The polypeptide sequences of either variable domain may be altered by DNA shuffling or by mutation in order to enhance the interaction of each variable domain with its complementary pair. DNA shuffling is known in the art and taught, for example, by Stemmer, 1994, Nature 370: 389-391 and U.S. Patent No. 6,297,053, both of which are incorporated herein by reference. Other methods of mutagenesis are well known to those of skill in the art.

In a preferred embodiment of the invention the'dual-specific ligand' is a single chain Fv fragment. In an alternative embodiment of the invention, the 'dual-specific ligand' consists of a Fab format.

In a further aspect, the present invention provides nucleic acid encoding at least a 'dual specific ligand' as herein defined.

One skilled in the art will appreciate that, depending on the aspect of the invention, both antigens or epitopes may bind simultaneously to the same antibody molecule. Alternatively, they may compete for binding to the same antibody molecule. For example, where both epitopes are bound simultaneously, both variable domains of a dual specific ligand are able to independently bind their target epitopes. Where the domains compete, the one variable domain is capable of binding its target, but not at the same time as the other variable domain binds its cognate target; or the first variable domain is capable of binding its target, but not at the same time as the second variable domain binds its cognate target.

The variable regions may be derived from antibodies directed against target antigens or epitopes. Alternatively they may be derived from a repertoire of single antibody domains such as those expressed on the surface of filamentous bacteriophage. Selection may be performed as described below.

In general, the nucleic acid molecules and vector constructs required for the performance of the present invention may be constructed and manipulated as set forth in standard laboratory manuals, such as Sambrook et al. (1989J Molecular Cloning: A Laboratory Manual, Cold Spring Harbor, USA.

The manipulation of nucleic acids useful in the present invention is typically carried out in recombinant vectors.

Thus in a further aspect, the present invention provides a vector comprising nucleic acid encoding at least a 'dual-specific ligand' as herein defined.

As used herein, vector refers to a discrete element that is used to introduce heterologous DNA into cells for the expression and/or replication thereof. Methods by which to select or construct and, subsequently, use such vectors are well known to one of ordinary skill in the art. Numerous vectors are publicly available, including bacterial plasmids, bacteriophage, artificial chromosomes and episomal vectors. Such vectors may be used for simple cloning and mutagenesis; alternatively gene expression vector is employed. A vector of use according to the invention may be selected to accommodate a polypeptide coding sequence of a desired size, typically from 0.25 kilobase (kb) to 40 kb or more in length A suitable host cell is transformed with the vector after in vitro cloning manipulations. Each vector contains various functional components, which generally include a cloning (or 'polylinker") site, an origin of replication and at least one selectable marker gene. If given vector is an expression vector, it additionally possesses one or more of the following: enhancer element, promoter, transcription termination and signal sequences, each positioned in the vicinity of the cloning site, such that they are operatively linked to the gene encoding a ligand according to the invention.

Both cloning and expression vectors generally contain nucleic acid sequences that enable 30 the vector to replicate in one or more selected host cells. Typically in cloning vectors, this sequence is one that enables the vector to replicate independently of the host chromosomal DNA and includes origins of replication or autonomously replicating sequences. Such sequences are well known for a variety of bacteria, yeast and viruses.

The origin of replication from the plasmid pBR322 is suitable for most Gram-negative bacteria, the 2 micron plasmid origin is suitable for yeast, and various viral origins (e.g. SV 40, adenovirus) are useful for cloning vectors in mammalian cells. Generally, the origin of replication is not needed for mammalian expression vectors unless these are s used in mammalian cells able to replicate high levels of DNA, such as COS cells.

Advantageously, a cloning or expression vector may contain a selection gene also referred to as selectable marker. This gene encodes a protein necessary for the survival or growth of transformed host cells grown in a selective culture medium. Host cells not transformed with the vector containing the selection gene will therefore not survive in the culture medium. Typical selection genes encode proteins that confer resistance to antibiotics and other toxins, e.g. ampicillin, neomycin, methotrexate or tetracycline, complement auxotrophic deficiencies, or supply critical nutrients not available in the growth media. Since the replication of vectors encoding a ligand according to the present invention is most conveniently performed in E. coli, an E. coli- selectable marker, for example, the β-lactamase gene that confers resistance to the antibiotic ampicillin, is of use. These can be obtained from E. coli plasmids, such as pBR322 or a pUC plasmid such as pUC18 or pUC19.

Expression vectors usually contain a promoter that is recognised by the host organism and is operably linked to the coding sequence of interest. Such a promoter may be inducible or constitutive. The tenn "operably linked" refers to a juxtaposition wherein the components described are in a relationship permitting them to function in their intended manner. A control sequence "operably linked" to a coding sequence is ligated in such a way that expression of the coding sequence is achieved under conditions compatible with the control sequences.

Promoters suitable for use with prokaryotic hosts include, for example, the p-lactamase and lactose promoter systems, alkaline phosphatase, the tryptophan (trp) promoter system and hybrid promoters such as the tac promoter. Promoters for use in bacterial systems will also generally contain a Shine-Delgarno sequence operably linked to the coding sequence. The preferred vectors are expression vectors that enables the expression of a nucleotide sequence corresponding to a polypeptide library member. Thus, selection with the first and/or second antigen or epitope can be performed by separate propagation and expression of a single clone expressing the polypeptide library member or by use of any selection display system. As described above, the preferred selection display system is bacteriophage display. Thus, phage or phagemid vectors may be used, eg pIT1 or pIT2. Leader sequences useful in the invention include peIB, stII, ompA, phoA, bla and pelA. One example are phagemid vectors which have an E. coli origin of replication (for double stranded replication) and also a phage origin of replication (for production of single-stranded DNA). The manipulation and expression of such vectors is well known in the art (Hoogenboom and Winter (1992) supra; Nissim et al. (1994) supra). Briefly, the vector contains a β-lactamase gene to confer selectivity on the phagemid and a lac promoter upstream of a expression cassette that consists (N to C terminal) of a pelB leader sequence (which directs the expressed polypeptide to the periplasmic space), a multiple cloning site (for cloning the nucleotide version of the library member), optionally, one or more peptide tag (for detection), optionally, one or more TAG stop codon and the phage protein pIII. Thus, using various suppressor and non- suppressor strains of E. cold and with the addition of glucose, iso- propyl thio-,β-D-galactoside (IPTG) or a helper phage, such as VCS M13, the vector is able to replicate as a plasmid with no expression, produce large quantities of the polypeptide library member only or produce phage, some of which contain at least one copy of the polypeptide-pIII fusion on their surface.

Construction of vectors encoding ligands according to the invention employs conventional ligation techniques. Isolated vectors or DNA fragments are cleaved, tailored, and religated in the form desired to generate the required vector. If desired, analysis to confirm that the correct sequences are present in the constructed vector can be performed in a known fashion. Suitable methods for constructing expression vectors, preparing in vitro transcripts, introducing DNA into host cells, and performing analyses for assessing expression and function are known to those skilled in the art. The presence of a gene sequence in a sample is detected, or its amplification and/or expression quantified by conventional methods, such as Southern or Northern analysis, Western blotting, dot blotting of DNA, RNA or protein, in situ hybridisation, immunocytochemistry or sequence analysis of nucleic acid or protein molecules. Those skilled in the art will readily envisage how these methods may be modified, if desired.

### Structure of closed conformation multispecific ligands

According to one aspect of the second configuration of the invention present invention, to the two or more non-complementary epitope binding domains are linked so that they are in a closed conformation as herein defined. Advantageously, they may be further attached to a skeleton which may, as a alternative, or on addition to a linker described herein, facilitate the formation and/or maintenance of the closed conformation of the epitope binding sites with respect to one another.

### (I) Skeletons

Skeletons may be based on immunoglobulin molecules or may be non-immunoglobulin in origin as set forth above. Preferred immunoglobulin skeletons as herein defined includes any one or more of those selected from the following: an immunoglobulin molecule comprising at least (i) the CL (kappa or lambda subclass) domain of an antibody; or (ii) the CH1 domain of an antibody heavy chain; an immunoglobulin molecule comprising the CH1 and CH2 domains of an antibody heavy chain; an immunoglobulin molecule comprising the CH1, CH2 and CH3 domains of an antibody heavy chain; or any of the subset (ii) in conjunction with the CL (kappa or lambda subclass) domain of an antibody. A hinge region domain may also be included.. Such combinations of domains may, for example, mimic natural antibodies, such as IgG or IgM, or fragments thereof, such as Fv, scFv, Fab or F(ab')2 molecules. Those skilled in the art will be aware that this list is not intended to be exhaustive.

### (II) Protein scaffolds

Each epitope binding domain comprises a protein scaffold and one or more CDRs which are involved in the specific interaction of the domain with one or more epitopes. Advantageously, an epitope binding domain according to the present invention comprises three CDRs. Suitable protein scaffolds include any of those selected from the group consisting of the following: those based on immunoglobulin domains, those based on fibronectin, those based on affibodies, those based on CTLA4, those based on chaperones such as GroEL, those based on lipocallin and those based on the bacterial Fc receptors SpA and SpD. Those skilled in the art will appreciate that this list is not intended to be exhaustive.

### F: Scaffolds for use in Constructing Dual Specific Ligands

### Selection of the main-chain conformation

The members of the immunoglobulin superfamily all share a similar fold for their polypeptide chain. For example, although antibodies are highly diverse in terms of their primary sequence, comparison of sequences and crystallographic structures has revealed that, contrary to expectation, five of the six antigen binding loops of antibodies (H1, H2, L1, L2, L3) adopt a limited number of main-chain conformations, or canonical structures (Chothia and Lesk (1987) J. Mol. Biol., 196: 901; Chothia et al. (1989) Nature, 342: 877). Analysis of loop lengths and key residues has therefore enabled prediction of the main chain conformations of H1, H2, L1, L2 and L3 found in the majority of human antibodies (Chothia et al. (1992) J. Mol. Biol., 227: 799; Tomlinson et al. (1995) EMBO J., 14: 20 4628; Williams et al. (1996) J. Mol. Biol., 264: 220). Although the H3 region is much more diverse in terms of sequence, length and structure (due to the use of D segments), it also forms a limited number of main-chain conformations for short loop lengths which depend on the length and the presence of particular residues, or types of residue, at key positions in the loop and the antibody framework (Martin et al. (1996) J; Mol. Biol., 263: 800; Shirai et al. (1996) FEDS Letters, 399: 1).

The dual specific ligands of the present invention are advantageously assembled from libraries of domains, such as libraries of VH domains and/or libraries of V_{L} domains. Moreover, the dual specific ligands of the invention may themselves be provided in the form of libraries. In one aspect of the present invention, libraries of dual specific ligands and/or domains are designed in which certain loop lengths and key residues have been chosen to ensure that the main-chain conformation of the members is known. Advantageously, these are real conformations of immunoglobulin superfamily molecules found in nature, to minimise the chances that they are non-functional, as discussed above. Germline V gene segments serve as one suitable basic framework for constructing antibody or T-cell receptor libraries; other sequences are also of use. Variations may occur at a low frequency, such that a small number of functional members may possess an altered main- chain conformation, which does not affect its function.

Canonical structure theory is also of use to assess the number of different main-chain conformations encoded by ligands, to predict the main-chain conformation based on ligand sequences and to chose residues for diversification which do not affect the canonical structure. It is known that, in the human VK domain, the L1 loop can adopt one of four canonical structures, the L2 loop has a single canonical structure and that 90% of human VK domains adopt one of four or live canonical structures for the L3 loop (Tomlinson et al. (1995) supra); thus, in the VK domain alone, different canonical structures can combine to create a range of different main-chain conformations. Given that the Vα domain encodes a different range of canonical structures for the L1; L2 and L3 loops and that VK and Vα domains can pair with any VH domain which can encode several canonical structures for the H1 and H2 loops, the number of canonical structure combinations observed for these five loops is very large. This implies that the generation of diversity in the main-chain conformation may be essential for the production of a wide range of binding specificities. However, by constructing an antibody library based on a single known main-chain conformation it has been found, contrary to expectation, that diversity in the main-chain conformation is not required to generate sufficient diversity to target substantially all antigens. Even more surprisingly, the single main-chain conformation need not be a consensus structure - a single naturally occurring conformation can be used as the basis for an entire library. Thus, in a preferred aspect, the dual-specific ligands of the invention possess a single known main-chain conformation.

The single main-chain conformation that is chosen is preferably commonplace among molecules of the immunoglobulin superfamily type in question. A conformation is commonplace when a significant number of naturally occurring molecules are observed to adopt it. Accordingly, in a preferred aspect of the invention, the natural occurrence of the different main-chain conformations for each binding loop of an immunoglobulin domain are considered separately and then a naturally occurring variable domain is chosen which possesses the desired combination of main-chain conformations for the different loops. If none is available, the nearest equivalent may be chosen. It is preferable that the desired combination of main-chain conformations for the different loops is created by selecting germline gene segments which encode the desired main-chain conformations. It is more preferable, that the selected germline gene segments are frequently expressed in nature, and most preferable that they are the most frequently expressed of all natural germline gene segments.

In designing dual specific ligands or libraries thereof the incidence of the different main chain conformations for each of the six antigen binding loops may be considered separately. For H1, H2, L1, L2 and L3, a given conformation that is adopted by between 20% and 100% of the antigen binding loops of naturally occurring molecules is chosen.

Typically, its observed incidence is above 35% (i.e. between 35% and 100%) and, ideally, above 50% or even above 65%. Since the vast majority of H3 loops do not have canonical structures, it is preferable to select a main-chain conformation which is commonplace among those loops which do display canonical structures. For each of the loops, the conformation which is observed most often in the natural repertoire is therefore selected. In human antibodies, the most popular canonical structures (CS) for each loop are as follows: H1 - CS 1 (79% of the expressed repertoire), H2 - CS 3 (46%), L1 - CS 2 of VK (39%), L2 - CS 1 (100%), L3 - CS 1 of VK (36%) (calculation assumes α k:λ ratio of 70:30, Hood et al. (1967) Cold Spring Harbor Symp. Quant. Biol, 48: 133). For H3 loops that have canonical structures, a CDR3 length (Kabat et al. (1991) Sequences of proteins of immunological interest, U.S. Department of Health and Human Services) of seven residues with a salt-bridge from residue 94 to residue 101 appears to be the most common. There are at least 16 human antibody sequences in the EMBL data library with the required H3 length and key residues to form this conformation and at least two crystallographic structures in the protein data bank which can be used as a basis for antibody modelling (2cgr and 1tet). The most frequently expressed germline gene segments that this combination of canonical structures are the VH segment 3-23 (DP-47), the JH segment JH4b, the VK segment 02/012 (DPK9) and the JK segment JK1. VH segments DP45 and DP38 are also suitable. These segments can therefore be used in combination as a basis to construct a library with the desired single main-chain conformation.

Alternatively, instead of choosing the single main-chain conformation based on the natural occurrence of the different main-chain conformations for each of the binding loops in isolation, the natural occurrence of combinations of main-chain conformations is 5 used as the basis for choosing the single main-chain conformation. In the case of antibodies, for example, the natural occurrence of canonical structure combinations for any two, three, four, five or for all six of the antigen binding loops can be determined. Here, it is preferable that the chosen conformation is commonplace in naturally occurring antibodies and most preferable that it observed most frequently in the natural repertoire. Thus, in human antibodies, for example, when natural combinations of the five antigen binding loops, H1, H2, L1, L2 and L3, are considered, the most frequent combination of canonical structures is determined and then combined with the most popular conformation for the H3 loop, as a basis for choosing the single main-chain conformation.

### ii. Diversification of the canonical sequence.

Having selected several known main-chain conformations or, preferably a single known main-chain conformation, dual specific ligands according to the invention or libraries for use in the invention can be constructed by varying the binding site of the molecule in order to generate a repertoire with structural and/or functional diversity. This means that variants are generated such that they possess sufficient diversity in their structure and/or in their function so that they are capable of providing a range of activities.

The desired diversity is typically generated by varying the selected molecule at one or more positions. The positions to be changed can be chosen at random or are preferably selected. The variation can then be achieved either by randomisation, during which the resident amino acid is replaced by any amino acid or analogue thereof, natural or synthetic, producing a very large number of variants or by replacing the resident amino acid with one or more of a defined subset of amino acids, producing a more limited number of variants.

Various methods have been reported for introducing such diversity. Error- prone PCR (Hawkins et al. (1992) J. Mol. Biol., 226: 889), chemical mutagenesis (Deng et al. (1994) J. Biol. Chem., 269: 9533) or bacterial mutator strains (Low et al. (1996) J. Mol. Biol., 260: 359) can be used to introduce random mutations into the genes that encode the molecule. Methods for mutating selected positions are also well known in the art and include the use of mismatched oligonucleotides or degenerate oligonucleotides, with or without the use of PCR. For example, several synthetic antibody libraries have been created by targeting mutations to the antigen binding loops. The H3 region of a human tetanus toxoid-binding Fab has been randomised to create a range of new binding specificities (Barbas et al. (1992) Proc. Natl. Acad. Sci. USA, 89: 4457). Random or semi-random H3 and L3 regions have been appended to germline V gene segments to produce large libraries with unmutated framework regions (Hoogenboom & Winter (1992) J: Mol. Biol., 227: 381; Barbas et al. (1992) Proc. Natl. Acad. Sci. USA, 89: 4457; Nissim et al. (1994) EMBO J., 13: 692; Griffiths et al. (1994) EMBO J., 13: 3245; De Kruif et al. (1995) J. Mol. Biol., 248: 97). Such diversification has been extended to include some or all of the other antigen binding loops (Crameri) et al. (1996) Nature Med., 5 2: 100; Riechmann et al. (1995) Bio/Techrology, 13: 475; Morphosys, W097/08320, supra). Since loop randomisation has the potential to create approximately more than 10¹⁵ structures for H3 alone and a similarly large number of variants for the other five loops, it is not feasible using current transformation technology or even by using cell free systems to produce a library representing all possible combinations. For example, in one of the largest libraries constructed to date, 6 x 10¹⁰ different antibodies, which is only a fraction of the potential diversity for a library of this design, were generated (Griffiths et al. (1994) supra).

In a preferred embodiment, only those residues which are directly involved in creating or modifying the desired function of the molecule are diversified. For many molecules, the function will be to bind a target and therefore diversity should be concentrated in the target binding site, while avoiding changing residues which are crucial to the overall packing of the molecule or to maintaining the chosen main- chain conformation.

### Diversification of the canonical sequence as it applies to antibody domains

In the case of antibody dual-specific ligands, the binding site for the target is most often the antigen binding site. Thus, in a highly preferred aspect, the invention provides libraries of or for the assembly of antibody dual-specific ligands in which only those residues in the antigen binding site are varied. These residues are extremely diverse in the human antibody repertoire and are known to make contacts in high- resolution antibody/antigen complexes. For example, in L2 it is known that positions 50 and 53 are diverse in naturally occurring antibodies and are observed to make contact with the antigen. In contrast, the conventional approach would have been to diversify all the residues in the corresponding Complementarity Determining Region (CDR1) as defined by Kabat et al. (1991, supra), some seven residues compared to the two diversified in the library for use according to the invention. This represents a significant improvement in terms of the functional diversity required to create a range of antigen binding specificities.

In nature, antibody diversity is the result of two processes: somatic recombination of germline V, D and J gene segments to create a naive primary repertoire (so called germline and junctional diversity) and somatic hypermutation of the resulting rearranged V genes. Analysis of human antibody sequences has shown that diversity in the primary repertoire is focused at the centre of the antigen binding site whereas somatic hypermutation spreads diversity to regions at the periphery of the antigen binding site that are highly conserved in the primary repertoire (see Tomlinson et al. (1996) J. Mol. Biol., 256: 813). This complementarily has probably evolved as an efficient strategy for searching sequence space and, although apparently unique to antibodies, it can easily be applied to other polypeptide repertoires. The residues which are varied are a subset of those that form the binding site for the target. Different (including overlapping) subsets of residues in the target binding site are diversified at different stages during selection, if desired.

In the case of an antibody repertoire, an initial 'naive' repertoire is created where some, but not all, of the residues in the antigen binding site are diversified. As used herein in this context, the term "naive" refers to antibody molecules that have no pre-determined target. These molecules resemble those which are encoded by the immunoglobulin genes of an individual who has not undergone immune diversification, as is the case with fetal and newborn individuals, whose immune systems have not yet been challenged by a wide variety of antigenic stimuli. This repertoire is then selected against a range of antigens or epitopes. If required, further diversity can then be introduced outside the region diversified in the initial repertoire. This matured repertoire can be selected for modified function, specificity or affinity.

The invention provides two different naive repertoires of binding domains for the construction of dual specific ligands, or a naive library of dual specific ligands, in which some or all of the residues in the antigen binding site are varied. The "primary" library mimics the natural primary repertoire, with diversity restricted to residues at the centre of the antigen binding site that are diverse in the germline V gene segments (germline diversity) or diversified during the recombination process (junctional diversity). Those residues which are diversified include, but are not limited to, H50, H52, H52a, H53, H55, H56, HS8, H95, H96, H97, H98, L50, L53, L91, L92, L93, L94 and L96. In the "somatic" library, diversity is restricted to residues that are diversified during the recombination process (junctional diversity) or are highly somatically mutated). Those residues which are diversified include, but are not limited to: H31, H33, H35, H95, H96, H97, H98, L30, L31, L32, L34 and L96. All the residues listed above as suitable for diversification in these libraries are known to make contacts in one or more antibody- antigen complexes. Since in both libraries, not all of the residues in the antigen binding site are varied, additional diversity is incorporated during selection by varying the remaining residues, if it is desired to do so. It shall be apparent to one skilled in the art that any subset of any of these residues (or additional residues which comprise the antigen binding site) can be used for the initial andlor subsequent diversification of the antigen binding site.

In the construction of libraries for use in the invention, diversification of chosen positions is typically achieved at the nucleic acid level, by altering the coding sequence which specifies the sequence of the polypeptide such that a number of possible amino acids (all 20 or a subset thereof) can be incorporated at that position. Using the IUPAC nomenclature, the most versatile codon is NNK, which encodes all amino acids as well as the TAG stop codon. The NNK codon is preferably used in order to introduce the required diversity. Other codons which achieve the same ends are also of use, including the NNN codon, which leads to the production of the additional stop codons TGA and TAA.

A feature of side-chain diversity in the antigen binding site of human antibodies is a pronounced bias which favours certain amino acid residues. If the amino acid composition of the ten most diverse positions in each of the VH, Vₖ and Vλ; regions are summed, more than 76% of the side-chain diversity comes from only seven different residues, these being, serine (24%), tyrosine (14%), asparagine (11 %), glycine (9%), alanine (7%), aspartate (6%) and threonine (6%). This bias towards hydrophilic residues and small residues which can provide main- chain flexibility probably reflects the evolution of surfaces which are predisposed to binding a wide range of antigens or epitopes and may help to explain the required promiscuity of antibodies in the primary repertoire.

Since it is preferable to mimic this distribution of amino acids, the distribution of amino acids at the positions to be varied preferably mimics that seen in the antigen binding site of antibodies. Such bias in the substitution of amino acids that permits selection of certain polypeptides (not just antibody polypeptides) against a range of target antigens is easily applied to any polypeptide repertoire. There are various methods for biasing the amino acid distribution at the position to be varied (including the use of tri-nucleotide mutagenesis, see W097/08320), of which the preferred method, due to ease of synthesis, is the use of conventional degenerate codons. By comparing the amino acid profile encoded by all combinations of degenerate codons (with single, double, triple and quadruple degeneracy in equal ratios at each position) with the natural amino acid use it is possible to calculate the most representative codon. The codons (AGT)(AGC)T, (AGT)(AGC)C and (AGT)(AGC) (CT) - that is, DVT, DVC and DVY, respectively using IUPAC nomenclature - are those closest to the desired amino acid profle: they encode 22% serine and 11 % tyrosine, asparagine, glycine, alanine, aspartate, threonine and cysteine. Preferably, therefore, libraries are constructed using either the DVT, DVC or 30 DVY codon at each of the diversifed positions.

### G: Antigens capable of increasing ligand half-life.

The dual specific ligands according to the invention, in one configuration thereof, are capable of binding to one or more molecules which can increase the half- life of the ligand in vivo. Typically, such molecules are polypeptides which occur naturally in vivo and which resist degradation or removal by endogenous mechanisms which remove unwanted material from the organism. For example, the molecule which increases the half-life of the organism may be selected from the following:
Proteins from the extracellular matrix; for example collagen, laminins, integrins and fibronectin. Collagens are the major proteins of the exkacellular matrix. About 15 types of collagen molecules are currently known, found in different parts of the body, eg type I collagen (accounting for 90% of body collagen) found in bone, skin, tendon, ligaments, cornea, internal organs or type II collagen found in cartilage, invertebral disc, notochord, vitreous humour of the eye.
Proteins found in blood, including: Plasma proteins such as fibrin, α-2 macroglobulin, serum albumin, fibrinogen A, fibrinogen B. serum amyloid protein A, heptaglobin, protein, ubiquitin, uteroglobulin and β-2-microglobulin; Enzymes and inhibitors such as plasminogen, lysozyme, cystatin C, alpha-1-antitrypsin and pancreatic kypsin inhibitor. Plasminogen is the inactive precursor of the trypsin-like serine protease plasmin. It is normally found circulating through the blood stream. When plasminogen becomes activated and is converted to plasmin, it unfolds a potent enzymatic domain that dissolves the fibrinogen fibers that entgangle the blood cells in a blood clot. This is called fibrinolysis.
Immune system proteins, such as IgE, IgG, IgM.
Transport proteins such as retinol binding protein, α-1 microglobulin.
Defensins such as beta-defensin 1, Neutrophil defensins 1, 2 and 3.
Proteins found at the blood brain barrier or in neural tissues, such as melanocortin receptor, myelin, ascorbate transporter.
Transferrin receptor specific ligand-neuropharmaceutical agent fusion proteins (see US5977307); brain capillary endothelial cell receptor, transferrin, transferrin receptor, insulin, insulin- like growth factor I (IGF 1) receptor, insulin-like growth factor 2 (IGF 2) receptor, insulin receptor.
Proteins localised to the kidney, such as polycystin, type IV collagen, organic anion transporter K1, Heymann's antigen.
Proteins localised to the liver, for example alcohol dehydrogenase, G250.
Blood coagulation factor X
α-1 antitrypsin
HNF 1α
Proteins localised to the lung, such as secretory component (binds IgA).
Proteins localised to the Heart, for example HSP 27. This is associated with dilated cardiomyopathy.
Proteins localised to the skin, for example keratin.
Bone specifc proteins, such as bone morphogenic proteins (BMPs), which are a subset of the transforming growth factor β superfamily that demonstrate osteogenic activity.

### Examples include BMP-2, -4, -5, -6, -7 (also referred to as osteogenic protein (OP-1) and -8 (OP-2)

Tumour specific proteins, including human trophoblast antigen, herceptin receptor, oestrogen receptor, cathepsins eg cathepsin B (found in liver and spleen).

Disease-specific proteins, such as antigens expressed only on activated T- cells: including LAG-3 (lymphocyte activation gene); osteoprotegerin ligand (OPGL) see Nature 402, 304-309, 1999; OX40 (a member of the TNF receptor family, expressed on activated T cells and the only costimulatory T cell molecule known to be specifically up-regulated in human T cell leukaemia virus type-I (HTLV-I)-producing cells - see J. Immunol. 2000 Jul 1;16561):263-70; Metalloproteases (associated with arthritis/cancers), including CG6512 Drosophila, human paraplegin, human FtsH, human AFG3L2, murine ftsH; angiogenic growth factors, including acidic fibroblast growth factor (FGF-1), basic fibroblast growth factor (FGF-2), Vascular endothelial growth factor/vascular permeability factor (VEGF/VPF), transforming growth factor-α (TGF-α), tumor necrosis factor-alpha (TNF-α), angiogenin, interleukin-3 (IL-3), interleukin-8 (IL-8), platelet derived endothelial growth factor (PD- ECGF), placental growth factor (P1GF), midkine platelet-derived growth factor-BB (PDGF), fractalkine.

### Stress proteins (heat shock proteins)

HSPs are normally found intracellularly. When they are found extracellularly, it is an indicator that a cell has died and spilled out its contents. This unprogrammed cell death (necrosis) only occurs when as a result of trauma, disease or injury and therefore in vivo, extracellular HSPs trigger a response from the immune system that will fight infection and disease. A dual specific which binds to extracellular HSP can be localised to a disease site.

Proteins involved in Fc transport

Brambell receptor (also known as FcRB)

This Fc receptor has two functions, both of which are potentially useful for delivery The functions are (1) The transport of IgG from mother to child across the placenta (2) the protection of IgG from degradation thereby prolonging its serum half life of IgG. It is thought that the receptor recycles IgG from endosome. See Holliger et al, Nat Biotechnol 1997 Jul;15(7):632-6.

Ligands according to the invention may designed to be specific for the above targets without requiring any increase in or increasing half life in vivo. For example, ligands according to the invention can be specific for targets selected from the foregoing which are tissue-specifc, thereby enabling tissue-specific targeting of the dual specific ligand, or a dAb monomer that binds a tissue-specific therapeutically relevant target, irrespective of any increase in half-life, although this may result. Moreover, where the ligand or dAb monomer targets kidney or liver,
this may redirect the ligand or dAb monomer to an alternative clearance pathway in vivo (for example, the ligand may be directed away from liver clearance to kidney clearance).

Other Approaches to Increasing In Vivo Half-Life:

In addition to the design of dual-specific ligands in which one of the specificities is for a target protein that increases the serum half-life of the antibody polypeptide construct, antibody polypeptides as described herein can be further stabilized by linkage to a chemical moiety that increases serum half-life. In order to provide improvement in the pharmacokinetics of antibody molecules, the present invention provides single domain variable region polypeptides that are linked to polymers which provide increased stability and half-life. The attachment of polymer molecules (e.g., polyethylene glycol; PEG) to proteins is well established and has been shown to modulate the pharmacokinetic properties of the modified proteins. For example, PEG modification of proteins has been shown to alter the *in vivo* circulating half-life, antigenicity, solubility, and resistance to proteolysis of the protein (Abuchowski et al., J. Biol. Chem. 1977, 252:3578; Nucci et al., Adv. Drug Delivery Reviews 1991, 6:133; Francis et al., Pharmaceutical Biotechnology Vol. 3 (Borchardt, R. T. ed.); and Stability of Protein Pharmaceuticals: in vivo Pathways of Degradation and Strategies for Ptotein Stabilization 1991 pp235-263, Plenum, NY).

Both site-specific and random PEGylation of protein molecules is known in the art (See, for example, Zalipsky and Lee, Poly(ethylene glycol) Chemistry: Biotechnical and Biomedical Applications 1992, pp 347-370, Plenum, NY; Goodson and Katre, 1990, Bio/Technology, 8:343; Hershfield et al., 1991, PNAS 88:7185). More specifically, random PEGylation of antibody molecules has been described at lysine residues and thiolated derivatives (Ling and Mattiasson, 1983, Immunol. Methods 59: 327; Wilkinson et al., 1987, Immunol. Letters, 15: 17; Kitamura et al., 1991, Cancer Res. 51:4310; Delgado et al., 1996 Br. J. Cancer, 73: 175; Pedley et al., 1994, Br. J. Cancer, 70:1126).

Methods of PEGylatioon are described herein below. Specific examples of PEGylation of antibody polypeptides, and dAbs in particular, are also provided in copending applications PCT/GB2004/002829, filed June 30, 2004, which designated the U.S, and of U.S. provisional application No. 60/535,076, filed January 8, 2004, the entirety of each of which is incorporated herein by reference.

### Affinity/Activity Determination:

Isolated single domain antibody (e.g., dAb) polypeptides as described herein have affinities (dissociation constant, K_{d}, = K*_{off}*/K*ₒₙ*) of at least 300 nM or less, and preferably at least 300 nM-50 pM, 200 nM - 50 pM, and more preferably at least 100 nM - 50 pM, 75 nM - 50 pM, 50 nM - 50 pM, 25 nM - 50 pM, 10 nM - 50 pM, 5 nM - 50 pM, 1 nM - 50 pM, 950 pM - 50 pM, 900 pM - 50 pM, 850 pM - 50 pM, 800 pM - 50 pM, 750 pM - 50 pM, 700 pM - 50 pM, 650 pM - 50 pM, 600 pM - 50 pM, 550 pM - 50 pM, 500 pM - 50 pM, 450 pM - 50 pM, 400 pM - 50 pM, 350 pM - 50 pM, 300 pM - 50 pM, 250 pM - 50 pM, 200 pM - 50 pM, 150 pM - 50 pM, 100 pM - 50 pM, 90 pM - 50 pM, 80 pM - 50 pM, 70 pM - 50 pM, 60 pM - 50 pM, or even as low as 50 pM.

The antigen-binding affinity of a variable domain polypeptide can be conveniently measured by surface plasmon resonance (SPR) using the BIAcore system (Pharmacia Biosensor, Piscataway, N.J.). In this method, antigen is coupled to the BIAcore chip at known concentrations, and variable domain polypeptides are introduced. Specific binding between the variable domain polypeptide and the immobilized antigen results in increased protein concentration on the chip matrix and a change in the SPR signal. Changes in SPR signal are recorded as resonance units (RU) and displayed with respect to time along the Y axis of a sensorgram. Baseline signal is taken with solvent alone (e.g., PBS) passing over the chip. The net difference between baseline signal and signal after completion of variable domain polypeptide injection represents the binding value of a given sample. To determine the off rate (K*_{off}*), on rate (K*ₒₙ*) and dissociation rate (K_{d}) constants, BIAcore kinetic evaluation software (e.g., version 2.1) is used.

High affinity is dependent upon the complementarity between a surface of the antigen and the CDRs of the antibody or antibody fragment. Complementarity is determined by the type and strength of the molecular interactions possible between portions of the target and the CDR, for example, the potential ionic interactions, van der Waals attractions, hydrogen bonding or other interactions that can occur. CDR3 tends to contribute more to antigen binding interactions than CDRs 1 and 2, probably due to its generally larger size, which provides more opportunity for favorable surface interactions. (See, e.g., Padlan et al., 1994, Mol. Immunol. 31: 169-217; Chothia & Lesk, 1987, J. Mol. Biol. 196: 904-917; and Chothia et al., 1985, J. Mol. Biol. 186: 651-663.) High affinity indicates single immunoglobulin variable domain/antigen pairings that have a high degree of complementarity, which is directly related to the structures of the variable domain and the target.

The structures conferring high affinity of a single immunoglobulin variable domain polypeptide for a given antigen can be highlighted using molecular modeling software that permits the docking of an antigen with the polypeptide structure. Generally, a computer model of the structure of a single immunoglobulin variable domain of known affinity can be docked with a computer model of a polypeptide or other target antigen of known structure to determine the interaction surfaces. Given the structure of the interaction surfaces for such a known interaction, one can then predict the impact, positive or negative, of conservative or less-conservative substitutions in the variable domain sequence on the strength of the interaction, thereby permitting the rational design of improved binding molecules.

### Multimeric Forms of Antibody Single Variable Domains:

In one aspect, an antibody polypeptide construct (e.g., a dAb) as described herein is multimerized, as for example, hetero- or homodimers, hetero- or homotrimers, hetero- or homotetramers, or higher order hetero- or homomultimers (e.g., hetero- or homo-pentamer and up to octomers). Multimerization can increase the strength of antigen binding through the avidity effect, wherein the strength of binding is related to the sum of the binding affinities of the multiple binding sites.

Hetero- and Homomultimers are prepared through expression of single domain antibodies fused, for example, through a peptide linker, leading to the configuration dAb-linker-dAb or a higher multiple of that arrangement. The multimers can also be linked to additional moieties, e.g., a polypeptide sequence that increases serum half-life or another effector moiety, e.g., a toxin or targeting moiety; e.g., PEG. Any linker peptide sequence can be used to generate hetero- or homomultimers, e.g., a linker sequence as would be used in the art to generate an scFv. One commonly useful linker comprises repeats of the peptide sequence (Gly₄Ser)ₙ (SEQ ID NO: 7), wherein n= 1 to about 10 (e.g., n=1, 2, 3, 4, 5, 6, 7, 8, 9, or 10). For example, the linker can be (Gly₄Ser)₃ (SEQ ID NO: 8), (Gly₄Ser)₅ (SEQ ID NO: 9), (Gly₄Ser)₇ (SEQ ID NO: 10) or another multiple of the (Gly₄Ser) (SEQ ID NO: 7) sequence.

An alternative to the expression of multimers as monomers linked by peptide sequences is linkage of the monomeric immunoglobulin variable domains post-translationally through, for example, disulfide bonding or other chemical linkage. For example, a free cysteine is engineered, e.g., at the C-terminus of the monomeric polypeptide, permits disulfide bonding between monomers. In this aspect or others requiring a free cysteine, the cysteine is introduced by including a cysteine codon (TGT, TGC) into a PCR primer adjacent to the last codon of the dAb sequence (for a C-terminal cysteine, the sequence in the primer will actually be the reverse complement, i.e., ACA or GCA, because it will be incorporated into the downstream PCR primer) and immediately before one or more stop codons. If desired, a linker peptide sequence, e.g., (Gly₄Ser)ₙ (SEQ ID NO: 7) is placed between the dAb sequence and the free cysteine. Expression of the monomers having a free cysteine residue results in a mixture of monomeric and dimeric forms in approximately a 1:1 mixture. Dimers are separated from monomers using gel chromatography, e.g., ion-exchange chromatography with salt gradient elution.

Alternatively, an engineered free cysteine is used to couple monomers through thiol linkages to a multivalent chemical linker, such as a trimeric maleimide molecule (e.g., Tris[2-maleimidoethyl]amine, TMEA) or a bi-maleimide PEG molecule (available from, for example, Nektar (Shearwater).

In one embodiment, a homodimer or heterodimer of the invention includes V_{H} or V_{L} domains which are covalently attached at a C-terminal amino acid to an immunoglobulin C_{H}1 domain or C_{κ} domain, respectively. Thus the hetero- or homodimer may be a Fab-like molecule wherein the antigen binding domain contains associated V_{H} and/or V_{L} domains covalently linked at their C-termini to a C_{H1} and C_{κ} domain respectively. In addition, or alternatively, a dAb multimer of the invention may be modeled on the camelid species which express a large proportion of fully functional, highly specific antibodies that are devoid of light chain sequences. The camelid heavy chain antibodies are found as homodimers of a single heavy chain, dimerized via their constant regions. The variable domains of these camelid heavy chain antibodies are referred to as V_{H}H domains and retain the ability, when isolated as fragments of the V_{H} chain, to bind antigen with high specificity ((Hamers-Casterman et al., 1993, Nature 363: 446-448; Gahroudi et al., 1997, FEBS Lett. 414: 521-526). Thus, an antibody single variable domain multimer of the invention may be constructed, using methods known in the art, and described above, to possess the V_{H}H conformation of the camelid species heavy chain antibodies.

### PEGylation of Antibody Polypeptide

The present invention provides PEGylated antibody polypeptide (e.g., dAb) monomers and multimers which provide increased half-life and resistance to degredation without a loss in activity (e.g., binding affinity) relative to non-PEGylated antibody polypeptides.

Antibody polypeptide molecules as described herein can be coupled, using methods known in the art, to polymer molecules (preferably PEG) useful for achieving the increased half-life and degradation resistance properties. Polymer moieties which can be utilized in the invention can be synthetic or naturally occurring and include, but are not limited to straight or branched chain polyalkylene, polyalkenylene or polyoxyalkylene polymers, or a branched or unbranched polysaccharide such as a homo- or heteropolysaccharide. Preferred examples of synthetic polymers which can be used in the invention include straight or branched chain poly(ethylene glycol) (PEG), poly(propylene glycol), or poly(vinyl alcohol) and derivatives or substituted forms thereof. Particularly preferred substituted polymers for linkage to antibody polypeptides as described herein include substituted PEG, including methoxy(polyethylene glycol). Naturally occurring polymer moieties which can be used in addition to or in place of PEG include lactose, amylose, dextran, or glycogen, as well as derivatives thereof which would be recognized by one of skill in the art. Derivatized forms of polymer molecules include, for example, derivatives which have additional moieties or reactive groups present therein to permit interaction with amino acid residues of the antibody polypeptides described herein. Such derivatives include N-hydroxylsuccinimide (NHS) active esters, succinimidyl propionate polymers, and sulfhydryl-selective reactive agents such as maleimide, vinyl sulfone, and thiol. Particularly preferred derivatized polymers include, but are not limited to PEG polymers having the formulae: PEG-O-CH₂CH₂CH₂-CO₂-NHS; PEG-O-CH₂-NHS; PEG-O-CH₂CH₂-CO₂-NHS; PEG-S-CH₂CH₂-CO-NHS; PEG-O₂CNH-CH(R)-CO₂-NHS; PEG-NHCO-CH₂CH₂-CO-NHS; and PEG-O-CH₂-CO₂-NHS; where R is (CH₂)₄)NHCO₂(mPEG). PEG polymers can be linear molecules, or can be branched
wherein multiple PEG moieties are present in a single polymer. Some particularly preferred PEG derivatives which are useful in the invention include, but are not limited to the following: 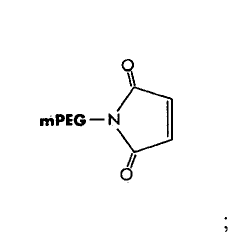 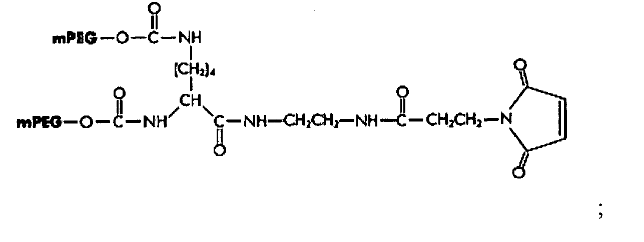 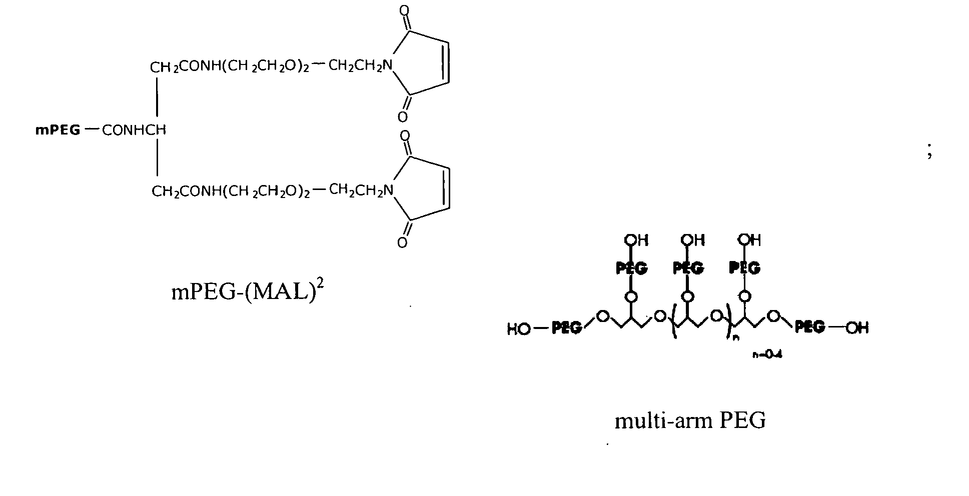 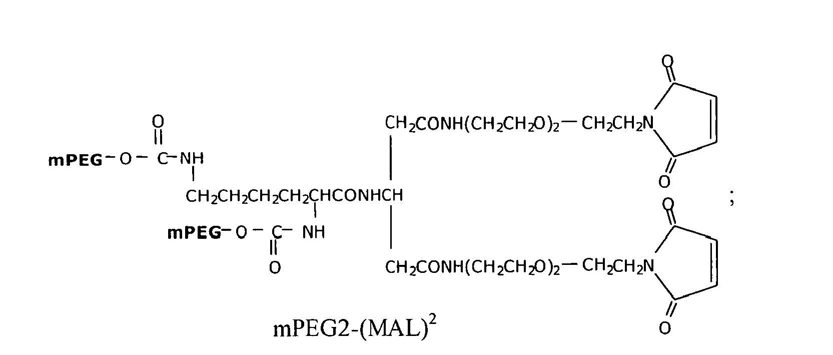 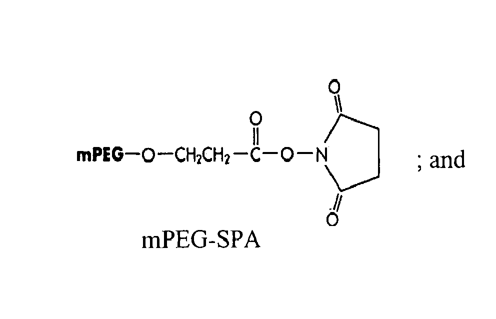 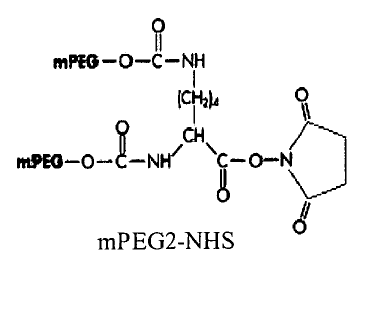 The reactive group (e.g., MAL, NHS, SPA, VS, or Thiol) may be attached directly to the PEG polymer or may be attached to PEG via a linker molecule.

The size of polymers useful in the invention can be in the range of between 500 Da to 60 kDa, for example, between 1000 Da and 60 kDa, 10 kDa and 60 kDa, 20 kDa and 60 kDa, 30 kDa and 60 kDa, 40 kDa and 60 kDa, and up to between 50 kDa and 60 kDa. The polymers used in the invention, particularly PEG, can be straight chain polymers or may possess a branched conformation. Depending on the combination of molecular weight and conformation, the polymer molecules, when attached to an antibody construct (e.g., dAb) monomer or multimer, will yield a molecule having an average hydrodynamic size of between 24 and 500 kDa. The hydrodynamic size of a polymer molecule used herein refers to the apparent size of a molecule (e.g., a protein molecule) based on the diffusion of the molecule through an aqueous solution. The diffusion, or motion of a protein through solution can be processed to derive an apparent size of the protein, where the size is given by the Stokes radius or hydrodynamic radius of the protein particle. The "hydrodynamic size" of a protein depends on both mass and shape (conformation), such that two proteins having the same molecular mass may have differing hydrodynamic sizes based on the overall conformation of the protein. The hydrodynamic size of a PEG-linked antibody single variable domain (including single domain antibody multimers as described herein) can be in the range of 24 kDa to 500 kDa; 30 to 500 kDa; 40 to 500 kDa; 50 to 500 kDa; 100 to 500 kDa; 150 to 500 kDa; 200 to 500 kDa; 250 to 500 kDa; 300 to 500 kDa; 350 to 500 kDa; 400 to 500 kDa and 450 to 500 kDa. Preferably the hydrodynamic size of a PEGylated dAb is 30 to 40 kDa; 70 to 80 kDa or 200 to 300 kDa. The size of a polymer molecule attached to an antibody polypeptide, such as a dAb or dAb multimer, can be thus varied depending on the desired application. For example, where the PEGylated dAb is intended to leave the circulation and enter into peripheral tissues, it is desirable to keep the size of the attached polymer low to facilitate extravazation from the blood stream. Alternatively, where it is desired to have the PEGylated dAb remain in the circulation for a longer period of time, a higher molecular weight polymer can be used (e.g., a 30 to 60 kDa polymer).

The polymer (PEG) molecules useful in the invention can be attached to antibody polypeptide constructs using methods which are well known in the art. The first step in the attachment of PEG or other polymer moieties to an antibody polypeptide monomer or multimer of the invention is the substitution of the hydroxyl end-groups of the PEG polymer by electrophile-containing functional groups. Particularly, PEG polymers are attached to either cysteine or lysine residues present in the antibody polypeptide monomers or multimers. The cysteine and lysine residues can be naturally occurring, or can be engineered into the antibody polypeptide molecule. For example, cysteine residues can be recombinantly engineered at the C-terminus of a dAb polypeptide, or residues at specific solvent accessible locations in a dAb or other antibody polypeptide can be substituted with cysteine or lysine. In a preferred embodiment, a PEG moiety is attached to a cysteine residue which is present in the hinge region at the C-terminus of a dAb monomer or multimer as described herein.

In one embodiment, the PEG polymer(s) is attached to one or more cysteine or lysine residues present in a framework region (FWs) and one or more heterologous CDRs of a dAb. CDRs and framework regions are those regions of an immunoglobulin variable domain as defined in the Kabat database of Sequences of Proteins of Immunological Interest (Kabat et al. (1991) Sequences of proteins of immunological interest, U.S. Department of Health and Human Services). In a preferred embodiment, a PEG polymer is linked to a cystine or lysine residue in the V_{H} framework segment DP47, or the V_{κ} framework segment DPK9. Cysteine and/or lysine residues of DP47 which can be linked to PEG include the cysteine at positions 22, or 96 and the lysine at positions 43, 65, 76, or 98 of SEQ ID NO: 1 (**Figure 21**). Cysteine and/or lysine residues of DPK9 which can be linked to PEG according to the invention include the cysteine residues at positions 23, or 88 and the lysine residues at positions 39, 42, 45, 103, or 107 of SEQ ID NO: 2 (**Figure 22**). In addition, specific cysteine or lysine residues can be linked to PEG in the V_{H} canonical framework region DP38, or DP45.

In addition, specific solvent accessible sites in a dAb molecule which are not naturally occuring cysteine or lysine residues can be mutated to a cysteine or lysine for attachment of a PEG polymer. Solvent accessible residues in any given dAb monomer or multimer can be determined using methods known in the art such as analysis of the crystal structure of a given dAb. For example, using the solved crystal structure of the V_{H} dAb HEL4 (which binds hen egg lysozyme; see below),

Primary amino acid sequence of HEL4 (SEQ ID NO: 5).
1 EVQLLESGGG LVQPGGSLRL SCAASGFRIS DEDMGWVRQA PGKGLEWVSS
51 IYGPSGSTYY ADSVKGRFTI SRDNSKNTLY LQMNSLRAED TAVYYCASAL
101 EPLSEPLGFW GQGTLVTVSS

Primary amino acid sequence of Vₖ dummy (SEQ ID NO: 6).
1 DIQMTQSPSS LSASVGDRVT ITCRASQSIS SYLNWYQQKP GKAPKLLIYA
51 ASSLQSGVPS RFSGSGSGTD FTLTISSLQP EDFATYYCQQ SYSTPNTFGQ
101 GTKVEIKR
the residues Gln-12, Pro-41, Asp-62, Glu-89, Gln-112, Leu-115, Thr-117, Ser-119, and Ser-120 have been identified as being solvent accessible, and would be attractive candidates for mutation to cysteine or lysine residues for the attachment of a PEG polymer. In addition, using the solved crystal structure of the V_{κ} dummy dAb (see above), the residues Val-15, Pro-40, Gly-41, Ser-56, Gly-57, Ser-60, Pro-80, Gly-71, Gln-100, Lys-107, and Arg-108 have been identified as being solvent accessible, and would be attractive candidates for mutation to cysteine or lysine residues for the attachment of a PEG polymer. In one embodiment, a PEG polymer is linked to multiple solvent accessible cysteine or lysine residues, or to solvent accessible residues which have been mutated to a cysteine or lysine residue. Alternatively, only one solvent accessible residue is linked to PEG, either where the particular antibody polypeptide construct only possesses one solvent accessible cysteine or lysine (or residue modified to a cysteine or lysine) or where a particular solvent accessible residue is selected from among several such residues for PEGylation.

Several attachment schemes which are useful in the invention are provided by the company Nektar (SanCarlos, CA). For example, where attachment of PEG or other polymer to a lysine residue is desired, active esters of PEG polymers which have been derivatized with N-hydroxylsuccinimide, such as succinimidyl propionate may be used. Where attachment to a cysteine residue is intended, PEG polymers which have been derivatized with sulfhydryl-selective reagents such as maleimide, vinyl sulfone, or thiols may be used. Other examples of specific embodiments of PEG derivatives which may be used according to the invention to generate PEGylated dAbs may be found in the Nektar Catalog (available on the world wide web at nektar.com). In addition, several derivitized forms of PEG may be used according to the invention to facilitate attachment of the PEG polymer to a dAb monomer or multimer of the invention. PEG derivatives useful in the invention include, but are not limited to PEG-succinimidyl succinate, urethane linked PEG, PEG phenylcarbonate, PEG succinimidyl carbonate, PEG-carboxymethyl azide, dimethylmaleic anhydride PEG, PEG dithiocarbonate derivatives, PEG-tresylates (2,2,2-trifluoroethanesolfonates), mPEG imidoesters, and other as described in Zalipsky and Lee, (1992) ("Use of functionalized poly(ethylene glycol)s for modification of peptides" in Poly(Ethylene Glycol) Chemistry: Biotechnical and Biomedical Applications, J. Milton Harris, Ed., Plenum Press, NY).

In each of the above embodiments, the PEG polymers can be attached to any amenable residue present in the antibody polypeptide construct peptides, or, preferably, one or more residues of the construct can be modified or mutated to a cysteine or lysine residue which may then be used as an attachment point for a PEG polymer. Preferably, a residue to be modified in this manner is a solvent accessible residue; that is, a residue, which when the antibody polypeptide construct is in its natural folded configuration is accessible to an aqueous environment and to a derivatized PEG polymer. Once one or more of these residues is mutated to a cysteine residue according to the invention, it is available for PEG attachment using a linear or branched MAL derivatized PEG (MAL-PEG).

In one embodiment, there is provided an antibody construct comprising an antibody single variable domain and PEG polymer wherein the ratio of PEG polymer to antibody single variable domain is a molar ratio of at least 0.25:1. In a further embodiment, the molar ratio of PEG polymer to antibody single variable domain is 0.33:1 or greater. In a still further embodiment the molar ratio of PEG polymer to antibody single variable domain is 0.5:1 or greater.

### H: Use of ligands as described herein.

### 1) Use of multispecific ligands according to the second configuration of the invention.

Multispecific ligands according to the method of the second configuration of the present invention may be employed in in vivo therapeutic and prophylactic applications, in vitro and in vivo diagnostic applications, in vitro assay and reagent applications, and the like. For example antibody molecules may be used in antibody based assay techniques, such as µ ELISA techniques, according to methods known to those skilled in the art.

As alluded to above, the multispecific ligands according to the invention are of use in diagnostic, prophylactic and therapeutic procedures. Multispecific antibodies according to the invention are of use diagnostically in Western analysis and in situ protein detection by standard immunohistochemical procedures; for use in these applications, the ligands may be labelled in accordance with techniques known to the art. In addition, such antibody polypeptides may be used preparatively in affinity chromatography procedures, when complexed to a chromatographic support, such as a resin. All such techniques are well known to one of skill in the art.

Diagnostic uses of the closed conformation multispecific ligands according to the invention include homogenous assays for analyses which exploit the ability of closed conformation multispecific ligands to bind two targets in competition, such that two targets cannot bind simultaneously (a closed conformation), or alternatively their ability to bind two targets simultaneously (an open conformation).

A true homogenous immunoassay format has been avidly sought by manufacturers of diagnostics and research assay systems used in drug discovery and development. The main diagnostics markets include human testing in hospitals, doctor's offices and clinics, commercial reference laboratories, blood banks, and the home, non-human diagnostics (for example food testing, water testing, environmental testing, bio-defence, and veterinary testing), and finally research (including drug development; basic research and academic research).

At present all these markets utilise immunoassay systems that are built around chemiluminescent, ELISA, fluorescence or in rare cases radio- immunoassay technologies. Each of these assay formats requires a separation step (separating bound from un-bound reagents). In some cases, several separation steps are required. Adding these additional steps adds reagents and automation, takes time, and affects the ultimate outcome of the assays. In human diagnostics, the separation step may be automated, which masks the problem, but does not remove it. The robotics, additional reagents, additional incubation times, and the like add considerable cost and complexity. In drug development, such as high throughput screening, where literally millions of samples are tested at once, with very low levels of test molecule, adding additional separation steps can eliminate the ability to perform a screen. However, avoiding the separation creates too much noise in the read out. Thus, there is a need for a true homogenous format that provides sensitivities at the range obtainable from present assay formats. Advantageously, an assay possesses fully quantitative read-outs with high sensitivity and a large dynamic range. Sensitivity is an important requirement, as is reducing the amount of sample required. Both of these features are features that a homogenous system offers. This is very important in point of care testing, and in drug development where samples are precious. Heterogenous systems, as currently available in the art, require large quantities of sample and expensive reagents.

Applications for homogenous assays include cancer testing, where the biggest assay is that for Prostate Specific Antigen, used in screening men for prostate cancer. Other applications include fertility testing, which provides a series of tests for women attempting to conceive including beta-hcg for pregnancy. Tests for infectious diseases, including hepatitis, HIV, rubella, and other viruses and microorganisms and sexually transmitted diseases. Tests are used by blood banks, especially tests for HIV, hepatitis A, B, C, non A non B. Therapeutic drug monitoring tests include monitoring levels of prescribed drugs in patients for efficacy and to avoid toxicity, for example digoxin for arrhythmia, and phenobarbital levels in psychotic cases; theophylline for asthma. Diagnostic tests are moreover useful in abused drug testing, such as testing for cocaine, marijuana and the like. Metabolic tests are used for measuring thyroid function, anaemia and other physiological disorders and functions.

The homogenous immunoassay format is moreover useful in the manufacture of standard clinical chemistry assays. The inclusion of immunoassays and chemistry assays on the same instrument is highly advantageous in diagnostic testing. Suitable chemical assays include tests for glucose, cholesterol, potassium, and the like.

A further major application for homogenous immunoassays is drug discovery and development: high throughput screening includes testing combinatorial chemistry libraries versus targets in ultra high volume. Signal is detected, and positive groups then split into smaller groups, and eventually tested in cells and then animals. Homogenous assays may be used in all these types of test. In drug development, especially animal studies and clinical trials heavy use of immunoassays is made. Homogenous assays greatly accelerate and simplify these procedures. Other Applications include food and beverage testing: testing meat and other foods for E. colt, salmonella, etc; water testing, including testing at water plants for all types of contaminants including E. coli; and veterinary testing.

In a broad embodiment, the invention provides a binding assay comprising a detectable agent which is bound to a closed conformation multispecifc ligand according to the invention, and whose detectable properties are altered by the binding of an analyte to said closed conformation multispecific ligand. Such an assay may be configured in several different ways, each exploiting the above properties of closed conformation multispecific ligands.

The assay relies on the direct or indirect displacement of an agent by the analyte, resulting in a change in the detectable properties of the agent. For example, where the agent is an enzyme which is capable of catalysing a reaction which has a detectable end-point, said enzyme can be bound by the ligand such as to obstruct its active site, thereby inactivating the enzyme. The analyte, which is also bound by the closed conformation multispecific ligand, displaces the enzyme, rendering it active through freeing of the active site. The enzyme is then able to react with a substrate, to give rise to a detectable event. In an alternative embodiment, the ligand may bind the enzyme outside of the active site, influencing the conformation of the enzyme and thus altering its activity. For example, the structure of the active site may be constrained by the binding of the ligand, or the binding of cofactors necessary for activity may be prevented.

The physical implementation of the assay may take any form known in the art. For example, the closed conformation multispecific ligandlenzyme complex may be provided on a test strip; the substrate may be provided in a different region of the test strip, and a solvent containing the analyte allowed to migrate through the ligand/enzyme complex, displacing the enzyme, and carrying it to the substrate region to produce a signal. Alternatively, the ligandlenzyme complex may be provided on a test stick or other solid phase, and dipped into an analyte/substrate solution, releasing enzyme into the solution in response to the presence of analyte.

Since each molecule of analyte potentially releases one enzyme molecule, the assay is quantitative, with the strength of the signal generated in a given time being dependent on the concentration of analyte in the solution.

Further configurations using the analyte in a closed conformation are possible. For example, the closed conformation multispecific ligand may be configured to bind an enzyme in an allosteric site, thereby activating the enzyme. In such an embodiment, the enzyme is active in the absence of analyte. Addition of the analyte displaces the enzyme and removes allosteric activation, thus inactivating the enzyme.

In the context of the above embodiments which employ enzyme activity as a measure of the analyte concentration, activation or inactivation of the enzyme refers to an increase or decrease in the activity of the enzyme, measured as the ability of the enzyme to catalyse a signal-generating reaction. For example, the enzyme may catalyse the conversion of an undetectable substrate to a detectable form thereof. For example, horseradish peroxidase is widely used in the art together with chromogenic or chemiluminescent substrates, which are available commercially. The level of increase or decrease of the activity of the enzyme may between 10% and 100%, such as 20%, 30%, 40%, 50%, 60%, 70%, 80% or 90%; in the case of an increase in activity, the increase may be more than 100%, i.e. 200%, 300%, 500% or more, or may not be measurable as a percentage if the baseline activity of the inhibited enzyme is undetectable.

In a further configuration, the closed conformation multispecific ligand may bind the substrate of an enzyme/substrate pair, rather than the enzyme. The substrate is therefore unavailable to the enzyme until released from the closed conformation multispecific ligand through binding of the analyte. The implementations for this configuration are as for the configurations which bind enzyme.

Moreover, the assay may be configured to bind a fluorescent molecule, such as a fluorescein or another fluorophore, in a conformation such that the fluorescence is quenched on binding to the ligand. In this case, binding of the analyte to the ligand will displace the fluorescent molecule, thus producing a signal. Alternatives to fluorescent molecules which are useful in the present invention include luminescent agents, such as luciferin/luciferase, and chromogenic agents, including agents commonly used in immunoassays such as HRP.

Therapeutic and prophylactic uses of multispecific ligands prepared according to the invention involve the administration of ligands according to the invention to a recipient mammal, such as a human. Multi- specificity can allow antibodies to bind to multimeric antigen with great avidity. Multispecific ligands can allow the cross- linking of two antigens, for example in recruiting cytotoxic T-cells to mediate the killing of tumour cell lines.

Substantially pure ligands or binding proteins thereof, for example dAb monomers, of at least 90 to 95% homogeneity are preferred for administration to a mammal, and 98 to 99% or more homogeneity is most preferred for pharmaceutical uses, especially when the mammal is a human. Once purified, partially or to homogeneity as desired, the ligands may be used diagnostically or therapeutically (including extracorporeally) or in developing and performing assay procedures, immunofluorescent stainings and the like (Lefkovite and Pernis, (1979 and 1981) Immunological Methods, Volumes I and II, Academic Press, NY).

The ligands or binding proteins thereof, for example dAb monomers, of the present invention will typically find use in preventing, suppressing or treating inflammatory states, allergic hypersensitivity, cancer, bacterial or viral infection, and autoimmune disorders (which include, but are not limited to, Type I diabetes, asthma, multiple sclerosis, rheumatoid arthritis, systemic lupus erythematosus, Crohn's disease and myasthenia gravis).

In addition to rheumatoid arthritis, anti-TNF-alpha polypeptides as described herein are applicable to the treatment of autoimmune diseases, such as (parentheticals indicate affected organ), but not limited to: Addison's disease (adrenal); autoimmune diseases of the ear (ear); autoimmune diseases of the eye (eye); autoimmune hepatitis (liver); autoimmune parotitis (parotid glands); Crohn's disease and inflammatory bowel disease (intestine); Diabetes Type I (pancreas); epididymitis (epididymis), glomerulonephritis (kidneys); Graves' disease (thyroid); Guillain-Barre syndrome (nerve cells); Hashimoto's disease (thyroid); hemolytic anemia (red blood cells); systemic lupus erythematosus (multiple tissues); male infertility (sperm); multiple sclerosis (nerve cells); myasthenia gravis (neuromuscular junction); pemphigus (primarily skin); psoriasis (skin); rheumatic fever (heart and joints); sarcoidosis (multiple tissues and organs); scleroderma (skin and connective tissues); Sjogren's syndrome (exocrine glands, and other tissues); spondyloarthropathies (axial skeleton, and other tissues); thyroiditis (thyroid); ulcerative colitis (intestine); and vasculitis (blood vessels).
In addition to rheumatoid arthritis and other chronic inflammatory disorders (e.g., Crohn's disease, psoriasis, etc.), anti-VEGF polypeptides as described herein can be used to treat diabetes, acute myeloid leukemia, leukemia and ophthalmic disorders, including macular degeneration and diabetic retinopathy.

In the instant application, the term 'prevention" involves administration of the protective composition prior to the induction of the disease. "Suppression" refers to administration of the composition after an inductive event, but prior to the clinical appearance of the disease.

Animal model systems which can be used to screen the effectiveness of the antibodies or binding proteins thereof in protecting against or treating the disease are available. Methods for the testing of systemic lupus erythematosus (SLE) in susceptible mice are known in the art (Knight et al. (1978) J. Exp. Med., 147: 1653; Reinersten et al. (1978) New Eng. J. Med., 299: 515). Myasthenia Gravis (MG) is tested in SJL/J female mice by inducing the disease with soluble AchR protein from another species (Lindstrom et al. (1988) Adv. Immurzol., 42: 233). Arthritis is induced in a susceptible strain of mice by 30 injection of Type II collagen (Stuart et al. (1984) Ann. Rev. Immunol., 42: 233). A model by which adjuvant arthritis is induced in susceptible rats by injection of mycobacterial heat shock protein has been described (Van Eden et al. (1988) Nature, 331: 171). Thyroiditis is induced in mice by administration of thyroglobulin as described (Maron et al. (1980) J. Exp. Med., 152: 1115). Insulin dependent diabetes mellitus (IDDM) occurs naturally or can be induced in certain strains of mice such as those described by Kanasawa et al. (1984) Diabetologia, 27: 113. EAE in mouse and rat serves as a model for MS in human. In this model, the demyelinating disease is induced by administration of myelin basic protein (see Paterson (1986) Textbook of Immuopathology, Mischer et al., eds., Grune and Stratton, New York, pp. 179-213; McFarlin et al. (1973) Science, 179: 478: and Satoh et al. (1987) J. Immunol., 138: 179).

Generally, the present ligands will be utilised in purified form together with pharmacologically appropriate carriers. Typically, these carriers include aqueous or alcoholic/aqueous solutions, emulsions or suspensions, any including saline and/or buffered media. Parenteral vehicles include sodium chloride solution, Ringer's dextrose, dextrose and sodium chloride and lactated Ringer's. Suitable physiologically-acceptable adjuvants, if necessary to keep a polypeptide complex in suspension, may be chosen from thickeners such as carboxymethylcellulose, polyvinylpyrrolidone, gelatin and alginates.

Intravenous vehicles include fluid and nutrient replenishers and electrolyte replenishers, such as those based on Ringer's dextrose. Preservatives and other additives, such as antimicrobials, antioxidants, chelating agents and inert gases, may also be present (Mack (1982) Remington's Pharmaceutical Sciences, 16th Edition).

The ligands of the present invention may be used as separately administered compositions or in conjunction with other agents. These can include various immunotherapeutic drugs, such as cylcosporine, methotrexate, adriamycin or cisplatinum, and immunotoxins. Pharmaceutical compositions can include "cocktails" of various cytotoxic or other agents in conjunction with the ligands of the present invention, or even combinations of lignds according to the present invention having different specificities, such as ligands selected using different target antigens or epitopes, whether or not they are pooled prior to administration.

The route of administration of pharmaceutical compositions according to the invention may be any of those commonly known to those of ordinary skill in the art. For therapy, including without limitation immunotherapy, the selected ligands thereof of the invention can be administered to any patient in accordance with standard techniques. The administration can be by any appropriate mode, including parenterally, intravenously, intramuscularly, intraperitoneally, transdermally, via the pulmonary route, or also, appropriately, by direct infusion with a catheter. The dosage and frequency of administration will depend on the age, sex and condition of the patient, concurrent administration of other drugs, counterindications and other parameters to be taken into account by the clinician.

As will be appreciated by the skilled artisan, the route and/or mode of administration will vary depending upon the desired results. In certain embodiments, the active compound can be prepared with a carrier that will protect the compound against rapid release, such as a controlled release formulation, including implants, transdermal patches, and microencapsulated delivery systems. Single domain antibody constructs are well suited for formulation as extended release preparations due, in part, to their small size - the number of moles per dose can be significantly higher than the dosage of, for example, full sized antibodies. BiodegradAble, biocompatible polymers can be used, such as ethylene vinyl acetate, polyanhydrides, polyglycolic acid, collagen, polyorthoesters, and polylactic acid. Prolonged absorption of injectable compositions can be brought about by including in the composition an agent that delays absorption, for example, monostearate salts and gelatin. Many methods for the preparation of such formulations are patented or generally known to those skilled in the art. See, e.g., Sustained and Controlled Release Drug Delivery Systems, J. R. Robinson, ed., Marcel Dekker, Inc., New York, 1978. Additional methods applicable to the controlled or extended release of polypeptide agents such as the single immunoglobulin variable domain polypeptides disclosed herein are described, for example, in U.S. Patent Nos. 6,306,406 and 6,346,274, as well as, for example, in U.S. Patent Application Nos. US20020182254 and US20020051808, all of which are incorporated herein by reference.

The ligands as described herein can be lyophilised for storage and reconstituted in a suitable carrier prior to use. This technique has been shown to be effective with conventional immunoglobulins and art- known lyophilisation and reconstitution techniques can be employed. It will be appreciated by those skilled in the art that lyophilisation and reconstitution can lead to varying degrees of antibody activity loss (e.g. with conventional immunoglobulins, IgM antibodies tend to have greater activity loss than IgG antibodies) and that use levels may have to be adjusted upward to compensate.

The compositions containing the present ligands or a cocktail thereof can be administered for prophylactic andlor therapeutic treatments. In certain therapeutic applications, an adequate amount to accomplish at least partial inhibition, suppression, modulation, killing, or some other measurable parameter, of a population of selected cells is defined as a "therapeutically-effective dose". Amounts needed to achieve this dosage will depend upon the severity of the disease and the general state of the patient's own immune system, but generally range from 0.005 to 5.0 mg of ligand, e.g. antibody, receptor (e.g. a T-cell receptor) or binding protein thereof per kilogram of body weight, with doses of 0.05 to 2.0 mg/kg/dose being more commonly used. For prophylactic applications, compositions containing the present ligands or cocktails thereof may also be administered in similar or slightly lower dosages.

Treatment performed using the compositions described herein is considered "effective" if one or more symptoms is reduced (e.g., by at least 10% or at least one point on a clinical assessment scale), relative to such symptoms present before treatment, or relative to such symptoms in an individual (human or model animal) not treated with such composition. Symptoms will obviously vary depending upon the disease or disorder targeted, but can be measured by an ordinarily skilled clinician or technician. Such symptoms can be measured, for example, by monitoring the level of one or more biochemical indicators of the disease or disorder (e.g., levels of an enzyme or metabolite correlated with the disease, affected cell numbers, etc.), by monitoring physical manifestations (e.g., inflammation, tumor size, etc.), or by an accepted clinical assessment scale, for example, the Expanded Disability Status Scale (for multiple sclerosis), the Irvine Inflammatory Bowel Disease Questionnaire (32 point assessment evaluates quality of life with respect to bowel function, systemic symptoms, social function and emotional status - score ranges from 32 to 224, with higher scores indicating a better quality of life), the Quality of Life Rheumatoid Arthritis Scale, or other accepted clinical assessment scale as known in the field. A sustained (e.g., one day or more, preferably longer) reduction in disease or disorder symptoms by at least 10% or by one or more points on a given clinical scale is indicative of'effective" treatment. Similarly, prophylaxis performed using a composition as described herein is "effective" if the onset or severity of one or more symptoms is delayed, reduced or abolished relative to such symptoms in a similar individual (human or animal model) not treated with the composition.

A composition containing a ligand or cocktail thereof according to the present invention may be utilised in prophylactic and therapeutic settings to aid in the alteration, inactivation, killing or removal of a select target cell population in a mammal. In addition, the selected repertoires of polypeptides described herein may be used exhacorporeally or in vitro selectively to kill, deplete or otherwise effectively remove a target cell population from a heterogeneous collection of cells. Blood from a mammal may be combined extracorporeally with the ligands, e.g. antibodies, cell-surface receptors or binding proteins thereof whereby the undesired cells are killed or otherwise removed from the blood for return to the mammal in accordance with standard techniques.

### 2: Use of half-life enhanced dual-specific ligands according to the invention.

Dual-specific ligands according to the method of the present invention may be employed in in vivo therapeutic and prophylactic applications, in vivo diagnostic applications and the like.

Therapeutic and prophylactic uses of dual-specific ligands prepared according to the invention involve the administration of ligands according to the invention to a recipient mammal, such as a human. Dual specific antibodies according to the invention comprise at least one specificity for a half-life enhancing molecule; one or more further specificities may be directed against target molecules. For example, a dual-specific IgG may be specific for four epitopes, one of which is on a half-life enhancing molecule. Dual-specificity can allow antibodies to bind to multimeric antigen with great avidity. Dual-specific antibodies can allow the cross-linking of two antigens, for example in recruiting cytotoxic T-cells to mediate the killing of tumour cell lines.

Substantially pure ligands or binding proteins thereof, such as dAb monomers, of at least 90 to 95% homogeneity are preferred for administration to a mammal, and 98 to 99% or more homogeneity is most preferred for pharmaceutical uses, especially when the mammal is a human. Once purified, partially or to homogeneity as desired, the ligands may be used diagnostically or therapeutically (including extracorporeally) or in developing and performing assay procedures, immunofluorescent stainings and the like (Lefkovite and Pernis, (1979 and 1981) Immunological Methods, Volumes I and II, Academic Press, NY).

The ligands of the present invention will typically find use in preventing, suppressing or treating inflammatory states, allergic hypersensitivity, cancer, bacterial or viral infection, and autoimmune disorders (which include, but are not limited to, Type I diabetes, multiple sclerosis, rheumatoid arthritis, systemic lupus erythematosus, Crohn's disease and myasthenia gravis).

Animal model systems which can be used to screen the effectiveness of the dual specific ligands in protecting against or treating the disease are available. Methods for the testing of systemic lupus erythematosus (SLE) in susceptible mice are known in the art (Knight et al. (1978) J. Exp. Med., 147: 1653; Reinersten et al. (1978) New Eng J. Med., 299: s 515). Myasthenia Gravis (MG) is tested in SJL/J female mice by inducing the disease with soluble AchR protein from another species (Lindstrom et al. (1988) Adv. Immunol., 42: 233). Arthritis is induced in a susceptible strain of mice by injection of Type II collagen (Stuart et al. (1984) Ann. Rev. Immunol., 42: 233). A model by which adjuvant arthritis is induced in susceptible rats by injection of mycobacterial heat shock protein has been described (Van Eden et al. (1988) Nature, 331: 171). Thyroiditis is induced in mice by administration of thyroglobulin as described (Maron et al. (1980) J. Exp. Med., 152: 1115). Insulin dependent diabetes mellitus (IDDM) occurs naturally or can be induced in certain strains of mice such as those described by Kanasawa et al. (1984) Diabetologia, 27: 113. EAR in mouse and rat serves as a model for MS in human. In this model, the 5 demyelinating disease is induced by administration of myelin basic protein (see Paterson (1986) Textbook of Immunopathology, Mischer et al., eds., Grune and Stratton, New York, pp. 179-213; McFarlin et al. (1973) Science, 179: 478: and Satoh et al. (1987) J; Immunol., 138: 179).

Dual specific ligands according to the invention and dAb monomers able to bind to extracellular targets involved in endocytosis (e.g. Clathrin) enable dual specifc ligands to be endocytosed, enabling another specificity able to bind to an intracellular target to be delivered to an intracellular environment. This strategy requires a dual specific ligand with physical properties that enable it to remain functional inside the cell. Alternatively, if the final destination intracellular compartnent is oxidising, a well folding ligand may not need to be disulphide free.

Generally, the present dual specific ligands will be utilised in purified form together with pharmacologically appropriate carriers. Typically, these carriers include aqueous or alcoholic/aqueous solutions, emulsions or suspensions, any including saline and/or buffered media. Parenteral vehicles include sodium chloride solution, Ringer's dextrose, dextrose and sodium chloride and lactated Ringer's. Suitable physiologically-acceptable adjuvants, if necessary to keep a polypeptide complex in suspension, may be chosen from thickeners such as carboxymethylcellulose, polyvinylpyrrolidone, gelatin and alginates. Intravenous vehicles include fluid and nutrient replenishers and electrolyte replenishers, such as those based on Ringer's dextrose. Preservatives and other additives, such as antimicrobials, antioxidants, chelating agents and inert gases, may also be present (Mack (1982) Remington's Pharmaceutical Sciences, 16th Edition).

The ligands of the present invention may be used as separately administered compositions or in conjunction with other agents. These can include various immunotherapeutic drugs, such as cylcosporine, methotrexate, adriamycin or cisplatinum, and immunotoxins. Pharmaceutical compositions can include "cocktails" of various cytotoxic or other agents in conjunction with the ligands of the present invention.

The route of administration of pharmaceutical compositions according to the invention may be any of those commonly known to those of ordinary skill in the art. For therapy, including without limitation immunotherapy, the ligands of the invention can be administered to any patient in accordance with standard techniques. The administration can be by any appropriate mode, including parenterally, intravenously, intramuscularly, intraperitoneally, kansdermally, via the pulmonary route, or also, appropriately, by direct infusion with a catheter. The dosage and frequency of administration will depend on the age, sex and condition of the patient, concurrent administration of other drugs, counterindications and other parameters to be taken into account by the clinician.

The ligands of the invention can be lyophilised for storage and reconstituted in a suitable carrier prior to use. This technique has been shown to be effective with conventional immunoglobulins and art-known lyophilisation and reconstitution techniques can be employed. It will be appreciated by those skilled in the art that lyophilisation and 30 reconstitution can lead to varying degrees of antibody activity loss (e.g. with conventional immunoglobulins, IgM antibodies tend to have greater activity loss than IgG antibodies) and that use levels may have to be adjusted upward to compensate.

The compositions containing the present ligands or a cocktail thereof can be administered for prophylactic and/or therapeutic treatments. In certain therapeutic applications, an adequate amount to accomplish at least partial inhibition, suppression, modulation, killing, or some other measurable parameter, of a population of selected cells is defined as a "therapeutically-effective dose". Amounts needed to achieve this dosage will depend upon the severity of the disease and the general state of the patient's own immune system, but generally range from 0.005 to 5.0 mg of ligand per kilogram of body weight, with doses of 0.05 to 2.0 mg/kgldose being more commonly used. For prophylactic applications, compositions containing the present ligands or cocktails thereof may also be administered in similar or slightly lower dosages.

A composition containing a ligand according to the present invention may be utilised in prophylactic and therapeutic settings to aid in the alteration, inactivation, killing or removal of a select target cell population in a mammal.

In addition, the selected repertoires of polypeptides described herein may be used extracorporeally or in vitro selectively to kill, deplete or otherwise effectively remove a target cell population from a heterogeneous collection of cells. Blood from a mammal may be combined extracorporeally with the ligands, e.g. antibodies, cell- surface receptors or binding proteins thereof whereby the undesired cells are killed or otherwise removed from the blood for return to the mammal in accordance with standard techniques. The invention is further described, for the purposes of illustration only, in the following examples. As used herein, for the purposes of dAb nomenclature, human TNF-α is referred to as TAR1 and human TNFa receptor 1 (p55 receptor) is referred to as TAR2.

### 3. Treatment of Rheumatoid Arthritis

In a preferred embodiment, ligands as described herein can be used to treat rheumatoid arthritis.

In one aspect, the invention provides methods of treating rheumatoid arthritis, comprising the use of one or more single domain antibody polypeptide constructs,
wherein one or more of the constructs antagonizes human TNFα's binding to a receptor. The present invention encompasses compositions comprising one or more single domain antibody polypeptide constructs that antagonize human TNFα's binding to a receptor, and dual specific ligands in which one specificity of the ligand is a single domain antibody directed toward TNFα and a second specificity is a single domain antibody directed to VEGF or HSA. The present invention further encompasses dual specific ligands in which one specificity of the ligand is directed toward VEGF and a second specificty is directed to HSA.

In one embodiment the invention provides methods of treatment of rheumatoid arthritis comprising administering a composition comprising one or more single domain antibody polypeptide constructs, wherein one or more of the constructs antagonizes human TNFα's binding to a receptor, and/or prevents an increase in arthritic score when administered to a mouse of the Tg197 transgenic mouse model of arthritis, and/or neutralizes TNF-α in the L929 cytotoxicity assay. In particular, methods of treatment of arthritis comprise the administration of a composition comprising one or more single domain antibody polypeptide constructs, wherein one or more of the constructs antagonizes human TNFα's binding to a receptor, and wherein the administration of the composition to a Tg197 transgenic mouse prevents an increase in arthritic score.

### a) Receptor Binding Assays

Ligands for the treatment of rheumatoid arthritis can interfere with the binding of TNF-α to a TNF-α receptor. The receptor can be an isolated (usually membrane-bound) receptor, or it can be a receptor present on a cell, either in vitro or in vivo.

Assays for the measurement of TNF-α receptor binding and interference with such binding by ligands as described herein are described below in Example 6. These include ELISAs (Example 6, section 1.3.1), BIAcore analyses (Example 6, section 1.3.2) and biochemical receptor binding assays using both isolated (or membrane-associated) receptors (Example 6, section 1.3.3) and receptors expressed on the surface of cultured cells (Example 6, section 1.3.3).

As used herein, the term "antagonizes binding" of the receptor refers to the ability or effect of a given antibody polypeptide construct to interfere with the binding of TNF-α (or VEGF or other factor) to a cognate receptor. Antagonism is measured using one or more of the in vitro, cell-based or in vivo assays as described herein. Thus, the receptor can be isolated, membrane bound, or present on the cell surface. A construct interferes with or antagonizes binding to a cognate receptor (e.g., TNFR1, TNFR2, VEGFR1, VEGFR2) if there is a statistically significant decrease in binding detected in the presence of the construct relative to the absence of the construct. Alternatively, a construct interferes with binding if there is at least a 10% decrease in measured binding in the presence of the construct, relative to its absence.

### b) L929 cytotoxicity assay

Ligands for the treatment of rheumatoid arthritis can interfere with the cytotoxic effects of TNF-α in the L929 cytotoxicity assay. This assay, based on the assay described by Evans et al., 2000, Molecular Biotechnology 15: 243-248, is described in Example 6, section 1.3.3. Anti-TNF-α ligands useful for the treatment of rheumatoid arthritis can neutralize the activity of TNF-α in this cell assay.

As used herein, the term "neutralizing," when used in reference to an antibody or dAb polypeptide as described herein, means that the polypeptide interferes with a measurable activity or function of the target antigen. A polypeptide is a "neutralizing" polypeptide if it reduces a measurable activity or function of the target antigen by at least 50%, and preferably at least 60%, 70%, 80%, 90%, 95% or more, up to and including 100% inhibition (i.e., no detectable effect or function of the target antigen). Thus, where the target is TNF-α, neutralizing activity can be assessed using the standard L929 cell killing assay described herein or by measuring the ability of an anti-TNF-α polypeptide construct to inhibit TNF-α-induced expression of ELAM-1 on HUVEC, which measures TNF-α-induced cellular activation.

Additional assays for antibody polypeptide interference with the receptor biding activity of TNF-α include the HeLa IL-8 assay also described in Example 6, section 1.3.3.

### c) In vivo assays.

The efficacy of anti-TNF-α ligands as described herein can be assessed using the Tg197 transgenic mouse arthritis model. Tg197 mice are transgenic for the human TNF-globin hybrid gene and heterozygotes at 4-7 weeks of age develop a chronic, progressive polyarthritis with histological features in common with rheumatoid arthritis (Keffer et al., 1991, EMBO J. 10: 4025-4031). The arthritic phenotype can be scored by assessing joint mobility and joint swelling. The arthritic phenotype of the joints can be scored by X-ray imaging of the joints and by histolopathological analysis of fixed sections of the knee and ankle/paw joints.

Experimental treatment to assess the efficacy of a given antibody polypeptide construct is performed as follows.
1) To test the prevention of arthritis with an antibody polypeptide construct, animals are treated as follows:
   a) heterozygous Tg197 mice are divided into groups of 10 animals with equal numbers of males and females. Treatment commences at 3 weeks of age, with weekly intraperitoneal administration of the antibody polypeptide in PBS, or PBS alone in the control animals;
   b) weigh the mice weekly;
   c) score the mice for macrophenotypic signs of arthritis according to the following system: 0 = no arthritis (normal appearance and flexion), 1 = mild arthritis (joint distortion), 2 = moderate arthritis (swelling, joint deformation), 3 = heavy arthritis (severely impaired movement).

The studies should best be performed such that the individual scoring is blinded to the test groupings. The preferred mechanism of antibody delivery for this assay is IP injection. However, the assay can be adapted to use subcutaneous injection, IV injection (e.g., via tail vein), intramuscular injection, or oral, inhalation or topical administration.

A treatment is effective in the Tg197 model system if the average arthritic score in the treatment group is lower (by a stistically significant amount) than that of the vehicle-only control group. Treatment is also considered effective if the average arthritic score is lower by at least 0.5 units, at least 1.0 units, at least 1.5 units or by at least 2 units relative to the vehicle-only control animals. Alternatively, the treatment is effective is the average arthritic score remains at or is lowered to 0 to 0.25 throughout the course of the therapeutic regimen.

A treatment is effective in the Tg197 model system if the average arthritic score in the treatment group increases during the course of the experiment but the start of this increase is delayed when compared with the vehicle only control. Treatment is also considered effective if the start of the increase in the average arthritic score of the treatment group when compared to the vehicle only control is delayed by 0.5 weeks, at least 1 week, at least 1.5 weeks, at least 2 weeks or by greater than 3 weeks.

As an alternative to the macrophenotypic scoring, at various intervals durint treatment, ankle/paw and knee joints can be fixed and analyzed histopathologically using the following system: 0 = no detectable pathology; 1 = hyperplasia of the synovial membrane and presence of polymorphonuclear infiltrates; 2 = pannus and fibrous tissue formation and focal subchondral bone erosion; 4 = extensive articular cartilage destruction and bone erosion. Treatment is considered effective if the average histopathological score is lower (by a statistically significant amount) than that of the vehicle control group. Treatment is also considered effective if the average histopathological score is lower by at least 0.5 units, at least 1.0 unit, at least 1.5 units, at least 2.0 units, at least 2.5 units, at least 3.0 units, or by at least 3.5 units relative to the vehicle-only control group. Alternatively, the treatment is effective is the average histopatholigical score remains at or is lowered to 0 to 0.5 throughout the course of the therapeutic regimen.
2) To test the effect of an antibody polypeptide construct (anti-TNF-α, anti-VEGF, etc.) on established arthritis, the assay can be performed on Tg197 animals as described above, only beginning treatment at 6 weeks of age, a time at which the animals have significant arthritic phenotypes. Scoring and efficacy analyses are also as described above. Anti-TNF-α dAb constructs as described herein can halt or reverse the progression of established arthritis in one or more of the model systems described.

In either format, treatment approaches include anti-TNF-α (e.g., anti-TNF-α dAb as described herein) in monomeric, dimeric or other multimeric forms, anti-VEGF (e.g., anti-VEGF dAb as described herein, including also camelid anti-VEGF dAbs) in monomeric, dimeric or other multimeric forms, a dual specific format of anti-TNF/anti-VEGF, and individual or dual specific constructs bearing anti-HSA, PEG or other half-life modifying moiet(ies). Additionally, anti-VEGF compositions described herein can be administered in combination with other anti-TNF compositions, such as etanercept (Enbrel), D2E7 (Humira) and infliximab (Remicade). The effectiveness of such combination therapy can be assessed using, for example, the cell culture and in vivo model systems described herein.

Additional accepted animal models of arthrits include collagen induced arthritis (CIA), described, for example, by Horsfall et al., 1997, J. of Immunol. 159:5687), and pristane-induced arthritis, described, for example, by Stasluk et al., 1997, Immunol. 90:81.

Assays for anti-VEGF polypeptide construct effectiveness:

### a) VEGF Receptor 2 binding assay

This method describes a VEGF receptor binding assay for measuring the ability of soluble domain antibodies (dAbs) to prevent VEGF₁₆₅ binding to VEGF Receptor 2.

VEGF is a specific mitogen for endothelial cells *in vitro* and a potent angiogenic factor *in vivo,* with high levels of the protein being expressed in various types of tumours. It is a 45kDa glycoprotein that is active as a homodimer. So far five different isoforms have been described which occur through alternative mRNA splicing. Of these isoforms VEGF₁₂₁ and VEGF₁₆₅ are the most abundant.

The specific action of VEGF on endothelial cells is mainly regulated by two types of receptor tyrosine kinases (RTK), VEGF R1 (Flt-1), and VEGF R2 (KDR/Flk-1). However, it appears that the VEGF activities such as mitogenicity, chemotaxis, and induction of morphological changes are mediated by VEGF R2, even though both receptors undergo phosphorylation upon binding of VEGF.

A recombinant human VEGF R2/Fc chimera is used in this assay, comprising the extracellular domain of human VEGF R2 fused to the Fc region of human IgG₁. Briefly, the receptor is captured on an ELISA plate, then the plate is blocked to prevent non specific binding. A mixture of VEGF₁₆₅ and dAb protein is then added, the plate is washed and receptor bound VEGF₁₆₅ detected using a biotinylated anti-VEGF antibody and an HRP conjugated anti-biotin antibody. The plate is developed using a colorimetric substrate and the OD read at 450nm. If the dAb blocks VEGF binding to the receptor then no colour is detected.

The assay is performed as follows. A 96 well Nunc Maxisorp assay plate is coated overnight at 4C with 100µl per well of recombinant human VEGF R2/Fc (R&D Systems, Cat. No: 357-KD-050) @ 0.5 µg/ml in carbonate buffer. Wells are washed 3 times with 0.05%tween/PBS and 3 times with PBS. 200µl per well of 2% BSA in PBS is added to block the plate and the plate is incubated for a minimum of 1h at room temperature.

Wells are washed (as above), then 50µl per well of purified dAb protein is added to each well. 50µl of VEGF, @ 6ng/ml in diluent (for a final concentration of 3ng/ml), is then added to each well and the plate incubated for 2hr at room temperature (for assay of supernatants; add 80µl of supernatant to each well then 20µl of VEGF @ 15ng/ml).

The following controls should be included: 0ng/ml VEGF (diluent only); 3ng/ml VEGF (R&D Systems, Cat No: 293-VE-050); 3ng/ml VEGF with 0.1µg/ml anti-VEGF neutralizing antibody (R&D Systems cat#MAB293).

The plate is washed (as above) and then 100µl biotinylated anti-VEGF antibody (R&D Systems, Cat No: BAF293), 0.5µg/ml in diluent, is added and incubated for 2hr at room temperature.

Wells are washed (as above) then add 100µl HRP conjugated anti-biotin antibody (1:5000 dilution in diluent; Stratech, Cat No: 200-032-096). The plate is then incubated for 1hr at room temperature.

The plate is washed (as above) ensuring any traces of Tween-20 have been removed to limit background in the subsequent peroxidase assay and to help the prevention of bubbles in the assay plate wells that will give inaccurate OD readings.

100µl of SureBlue 1-Component TMB MicroWell Peroxidase solution is added to each well, and the plate is left at room temperature for up to 20min. A deep blue soluble product will develop as bound HRP labelled conjugate reacts with the substrate. The reaction is stopped by the addition of 100µl 1M hydrochloric acid (the blue colour will turn yellow). The OD, at 450nm, of the plate should be read in a 96-well plate reader within 30min of acid addition. The OD450nm is proportional to the amount of bound streptavidin-HRP conjugate.

Expected result from the controls are as follows: 0ng/ml VEGF should give a low signal of <0.15 OD; 3ng/ml VEGF should give a signal of >0.5 OD; and 3ng/ml VEGF pre-incubated with 0.1 µg/ml neutralising antibody should give a signal <0.2 OD.

### b) VEGF Receptor 1 binding assay

This assay measures the binding of VEGF₁₆₅ to VEGF R1 and the ability of dAbs to block this interaction.

A recombinant human VEGF R1/Fc chimera is used here, comprising the extracellular domain of human VEGF R1 fused to the Fc region of human IgG₁. The receptor is captured on an ELISA plate then the plate is blocked to prevent non specific binding. A mixture of VEGF₁₆₅ and dAb protein is then added, the plate is washed and receptor bound VEGF₁₆₅ detected using a biotinylated anti-VEGF antibody and an HRP conjugated anti-biotin antibody. The plate is developed using a colorimetric substrate and the OD read at 450nm. If the dAb blocks VEGF binding to the receptor then no coiour will show.

The assay is performed as follows. A 96 well Nunc Maxisorp assay plate is coated overnight at 4C with 100µl per well of recombinant human VEGF R1/Fc (R&D Systems, Cat No: 321-FL-050) @ 0.1 µg/ml in carbonate buffer. Wells are washed 3 times with 0.05%tween/PBS and 3 times with PBS.

200µl per well of 2% BSA in PBS is added to block the plate and the plate is incubated for a minimum of 1h at room temperature.

Wells are washed (as above), then 50µl per well of purified dAb protein is added to each well. 50µl of VEGF, @ 1ng/ml in diluent (for a final concentration of 500pg/ml), is then added to each well and the plate incubated for 1hr at room temperature (assay of supernatants; add 80µl of supernatant to each well then 20µl of VEGF @ 2.5ng/ml).

The following controls should be included: 0ng/ml VEGF (diluent only); 500pg/ml VEGF; and 500pg/ml VEGF with 1µg/ml anti-VEGF antibody (R&D Systems cat#MAB293).

The plate is washed (as above) and then 100µl biotinylated anti-VEGF antibody, 50ng/ml in diluent, is added and incubated for 1hr at room temperature.

Wells are washed (as above) then add 100µl HRP conjugated anti-biotin antibody (1:5000 dilution in diluent). The plate is then incubated for 1hr at room temperature.

The plate is washed (as above), ensuring any traces of Tween-20 have been removed to limit background in the subsequent peroxidase assay and to help the prevention of bubbles in the assay plate wells that will give inaccurate OD readings.

100µl of SureBlue 1-Component TMB Micro Well Peroxidase solution is added to each well, and the plate is left at room temperature for up to 20min. A deep blue soluble product will develop as bound HRP labelled conjugate reacts with the substrate. The reaction is stopped by the addition of 100µl 1 M hydrochloric acid (the blue colour will turn yellow). The OD, at 450nm, of the plate should be read in a 96-well plate reader within 30 min of acid addition. The OD450 nm is proportional to the amount of bound streptavidin-HRP conjugate.

Expected result from the controls: 0ng/ml VEGF should give a low signal of <0.15 OD; 500pg/ml VEGF should give a signal of >0.8 OD; and 500pg/ml VEGF pre-incubated with 1µg/ml neutralising antibody should give a signal <0.3 OD

### c) Cell-based assay for VEGF activity:

This bioassay measures the ability of antibody polypeptides (e.g., dAbs) and other inhibitors to neutralise the VEGF induced proliferation of HUVE cells. HUVE cells plated in 96 well plates are incubated for 72 hours with pre-equilibrated VEGF and dAb protein. Cell number is then measured using a cell viability dye.

The assay is performed as follows. HUVE cells are trypsinized from a sub-confluent 175cm² flask. Medium is aspirated off, the cells are washed with 5ml trypsin and then incubated with 2ml trypsin at room temperature for 5min. The cells are gently dislodged from the base of the flask by knocking against your hand. 8ml of induction medium are then added to the flask, pipetting the cells to disperse any clumps. Viable cells are counted using trypan blue stain.

Cells are spun down and washed 2X in induction medium, spinning cells down and aspirating the medium after each wash. After the final aspiration the cells are diluted to 10⁵ cells/ml (in induction medium) and plated at 100 µl per well into a 96 well plate (10,000 cells/well). The plate is incubated for >2h @ 37C to allow attachment of cells.

60µl dAb protein and 60µl induction media containing 40ng/ml VEGF₁₆₅ (for a final concentration of 10ng/ml) is added to a v bottom 96 well plate and sealed with film. The dAbNEGF mixture is then incubated at 37C for 0.5-1 hour.

The dAbNEGF plate is removed from the incubator and 100µl of solution added to each well of the HUVEC containing plate (final volume of 200µl). This plate is then returned to the 37C incubator for a period of at least 72 hours.

Control wells include the following: wells containing cells, but no VEGF; wells containing cells, a positive control neutralising anti-VEGF antibody and VEGF; and control wells containing cells and VEGF only.

Cell viability is assessed by adding 20µl per well Celltiter96 reagent, and the plate incubated at 37C for 2-4h until a brown colour develops. The reaction is stopped by the addition of 20µl per well of 10% (w/v) SDS. The absorbance is then read at 490nm using a Wallac microplate reader.

The absorbance of the no VEGF control wells is subtracted from all other values. Absorbance is proportional to cell number. The control wells containing control anti-VEGF antibodies should also exhibit minimum cell proliferation. The wells containing VEGF only should exhibit maximum cell proliferation.

### d) In vivo assay for VEGF activity:

The efficacy of anti-VEGF polypeptide constructs (monomers, multimers or dual- or multi-specific) can also be tested in the Tg197 transgenic mouse model of arthritic disease. Dosing regimens and scoring are essentially as described for anti-TNF-α polypeptide constructs.

### 4. Treatment of Crohn's Disease

Anti-TNF-α polypeptides as described herein can be used to treat Crohn's disease in humans. In one embodiment the invention provides methods of treatment of Crohn's disease or other inflammatory bowel disease (IBD) in which TNF-α is involved. The methods comprise administering a composition comprising one or more single domain antibody polypeptide constructs, wherein one or more of the constructs antagonizes human TNFα's binding to a receptor, and/or prevents an increase in acute or chronic inflammatory bowel score when administered to a mouse of the Tnf^{ΔARE} transgenic mouse model of IBD, and/or neutralizes TNF-α in the L929 cytotoxicity assay. In particular, methods of treatment of Crohn's or other inflammatory bowel disorders comprise the administration of a composition comprising one or more single domain antibody polypeptide constructs, wherein one or more of the constructs antagonizes human TNFα's binding to a receptor, and wherein the administration of the composition to a Tnf^{ΔARE} transgenic mouse prevents an increase or effects a decrease in acute or chronic inflammatory bowel score.

The Tnf^{ΔARE} transgenic mouse model of Crohn's disease was originally described by Kontoyiannis et al., 1999, Immunity 10: 387-398; see also Kontoyiannis et al., 2002, J. Exp. Med. 196: 1563-1574. These mice bear a targeted deletion mutation in the 3' AU-rich elements (AREs) of TNF-α mRNA. AU-rich elements are involved in maintaining low mRNA stability, and their disruption leads to overexpression of murine TNF-α in these animals. The animals develop an IBD phenotype with remarkable similarity to Crohn's disease starting between 4 and 8 weeks of age. The basic histopathological characteristics include villus blunting and submucosal inflammation with prevailing PMN/macrophage and lymphocytic exudates, proceeding to patchy transmural inflammation and the appearance of lymphoid aggregates and rudimentary granulomata (Kontoyiannis et al., 2002, supra.). These animals also develop an arthritic phenotype and can thus also be used to separately evaluate the efficacy of anti-TNF-α treatments in RA.

Where treatment is to be evaluated for its effect in preventing IBD, treatment is initiated at, for example, 3 weeks of age, with initial weekly IP doses of a given antibody polypeptide construct. More or less frequent dosing intervals can be selected by one of skill in the art, depending upon the outcome of initial studies. Animals can then be monitored for bowel disease according to a standard scale as described in Kontoyiannis et al., 2002, supra. Paraffin-embedded intestinal tissue sections of ileum are histologically evaluated in a blinded fashion according to the following scale: Acute and chronic inflammation are assessed separately in a minimum of 8 high power fields (hpf) as follows - acute inflammatory score 0 = 0-1 polymorphonuclear (PMN) cells per hpf (PMN/hpf); 1 = 2-10 PMN/hpf within mucosa; 2 = 11-20 PMN/hpf within mucosa; 3 = 21-30 PMN/hpf within mucosa or 11-20 PMN/hpf with extension below muscularis mucosae; and 4 = >30 PMN/hpf within mucosa or >20 PMN/hpf with extension below muscularis mucosae. Chronic inflammatory score 0 = 0-10 mononuclear leukocytes (ML) per hpf (ML/hpf) within mucosa; 1 = 11-20 ML/hpf within mucosa; 2 = 21-30 ML/hpf within mucosa or 11-20 ML/hpf with extension below muscularis mucosae; 3 = 31-40 ML/hpf within mucosa or 21-30 ML/hpf with extension below muscularis mucosae or follicular hyperplasia; and 4 = >40 ML/hpf within mucosa or >30 ML/hpf with extension below muscularis mucosae or follicular hyperplasia. Total disease score per mouse is calculated by summation of the acute inflammatory or chronic inflammatory scores for each mouse.

To evaluate the effect of treatment on established disease, treatment can be begun at 6-8 weeks of age, with scoring performed in the same manner.

Treatment is considered effective if the average histopathological disease score is lower in treated animals (by a statistically significant amount) than that of the vehicle control group. Treatment is also considered effective if the average histopathological score is lower by at least 0.5 units, at least 1.0 units, at least 1.5 units, at least 2.0 units, at least 2.5 units, at least 3.0 units, or by at least 3.5 units relative to the vehicle-only control group. Alternatively, the treatment is effective if the average histopatholigical score remains at or is lowered to 0 to 0.5 throughout the course of the therapeutic regimen.

Other models of IBD include, for example, the DSS (dextran sodium sulfate) model of chronic colitis in BALB/c mice. The DSS model was originally described by Okayasu et al., 1990, Gastroenterology 98: 694-702 and was modified by Kojouharoff et al., 1997, Clin Exp. Immunol. 107: 353-358 (see also WO 2004/041862, which designates the U.S., incorporated herein by reference). BALB/c mice weighing 21-22 g are treated to induce chronic colitis by the administration of DSS in their drinking water at 5% w/v in cycles of 7 days of treatment and 12 days recovery interval without DSS. The 4^{th} recovery period can be extended from 12 to 21 days to represent a chronic inflammation status, rather than the acute status modeled by shorter recovery. After the last recovery period, treatment with antibody polypeptide, e.g., anti-TNF-α polypeptide as described herein is administered. Weekly administration is recommended initially, but can be adjusted by one of skill in the art as necessary (especially, e.g., to evaluate dosage forms with different half-life modifying moieties). At intervals during treatment, animals are killed, intestine is dissected and histopathological scores are assessed as described herein or as described in Kojouharoff et al., 1997, supra.

Other animal models of inflammatory bowel disease include the chronic intestinal inflammation induced by rectal instillation of 2,4,6-Trinitrobenzene sulfonic acid (TNBS; method described by Neurath et al., 1995, J. Exp. Med. 182: 1281; see also U.S. 6,764,838, incorporated herein by reference). Histopathological scoring can be performed using the same standard described above.

Comparison with other anti-TNF-α agents:

Disclosed herein are anti-TNF-α dAb constructs effective for the treatment of RA, Crohn's disease and other TNF-α mediated disorders. In one aspect, the effectiveness of the anti-TNF-α dAb constructs is greater than or equal to that of an agent selected from the group consisting of etanercept (ENBREL), infliximab (REMICADE) and D2E7 (HUMIRA; see U.S. patent No. 6,090,382, incorporated herein by reference).

Clinical trials of a recombinant version of the soluble human TNFR (p75) linked to the Fc portion of human IgG1 (sTNFR(p75):Fc, ENBREL, Immunex) have shown that its administration resulted in significant and rapid reductions in RA disease activity (Moreland et al., 1997, N. Eng. J. Med., 337:141-147). In addition, preliminary safety data from a pediatric clinical trial for sTNFR(p75):Fc indicates that this drug is generally well-tolerated by patients with juvenile rheumatoid arthritis (JRA) (Garrison et al, 1998, Am. College of Rheumatology meeting, Nov. 9, 1998, abstract 584).

As noted above, ENBREL is a dimeric fusion protein consisting of the extracellular ligand-binding portion of the human 75 kilodalton (p75) TNFR (GenBank Accession No. P20333) linked to the Fc portion of human IgG1. The Fc component of ENBREL contains the CH2 domain, the CH3 domain and hinge region, but not the CH 1 domain of IgG1. ENBREL is produced in a Chinese hamster ovary (CHO) mammalian cell expression system. It consists of 934 amino acids and has an apparent molecular weight of approximately 150 kilodaltons (Smith et al., 1990, Science 248:1019-1023; Mohler et al., 1993, J. Immunol. 151:1548-1561; U.S. Pat. No. 5,395,760 (Immunex Corporation, Seattle, Wash.; incorporated herein by reference); U.S. Pat. No. 5,605,690 (Immunex Corporation, Seattle, Wash.; incorporated herein by reference).

A monoclonal antibody directed against TNF-α. (infliximab, REMICADE, Centocor), administered with and without methotrexate, has demonstrated clinical efficacy in the treatment of RA (Elliott et al., 1993, Arthritis Rheum. 36:1681-1690; Elliott et al., 1994, Lancet 344:1105-1110). These data demonstrate significant reductions in Paulus 20% and 50% criteria at 4, 12 and 26 weeks. This treatment is administered intravenously and the anti-TNF monoclonal antibody disappears from circulation over a period of two months. The duration of efficacy appears to decrease with repeated doses. The patient can generate antibodies against the anti-TNF antibodies which limit the effectiveness and duration of this therapy (Kavanaugh et al., 1998, Rheum. Dis. Clin. North Am. 24:593-614). Administration of methotrexate in combination with infliximab helps prevent the development of anti-infliximab antibodies (Maini et al., 1998, Arthritis Rheum. 41:1552-1563). Infliximab has also demonstrated clinical efficacy in the treatment of the inflammatory bowel disorder Crohn's disease (Baert et al., 1999, Gastroenterology 116:22-28).

As discussed in the background section, infliximab is a chimeric monoclonal IgG antibody bearing human IgG4 constant and mouse variable regions. The infliximab polypeptide is described in U.S. patent Nos. 5,698,195 and 5,656,272, which are incorporated herein by reference. '

To compare efficacy with these or other anti-TNF-α compositions, one need only perform one or more of the receptor binding, cell-based or in vivo assays as described herein above using the anti-TNF-α dAb construct in parallel with the existing composition. This approach thus identifies those anti-TNF-α dAb constructs that show an effectiveness at inhibiting the effects of TNF-α in one or more of the assays that is equal to or greater than (in a statistically significant manner) the effectiveness of the comparison composition. Examples of such constructs and the analyses demonstrating equal or superior effectiveness are provided in the Examples.

### EXAMPLES

### Example 1. Selection of a dual specific scFv antibody (K8) directed against human serum albumin (HSA) and β-galactosidase (β-gal).

This example explains a method for making a dual specific antibody directed against β-gal and HSA in which a repertoire of VK variable domains linked to a gernline (dummy) VH domain is selected for binding to p-gal and a repertoire of VH variable domains linked to a germline (dummy) VK domain is selected for binding to HSA. The selected variable VH HSA and V_{K}B-gal domains are then combined and the antibodies selected for binding to β-gal and HSA. HSA is a half-life increasing protein found in human blood.

s Four human phage antibody libraries were used in this experiment.

| | | |
|---|---|---|
| Library 1 | Germline V_{κ}/DVT V_{H} | 8.46 x 10⁷ |
| | | |
| Library 2 | Germline V_{κ}/NNK V_{H} | 9.64 x 10⁷ |
| | | |
| Library3 | Germline VH/DVT V_{κ} | 1.47x 10⁸ |
| | | |
| Library 4 | Germline V_{H}/NNK V_{κ} | 1.45 x 10⁸ |

All libraries are based on a single human framework for V_{H} (V3- 23/DP47 and J_{H}4b) and V_{κ} (O12/O2/DPK9 and J_{κ}1) with side chain diversity incorporated in complementarity determining regions (CDR2 and CDR3).

Library 1 and Library 2 contain a dummy V_{κ} sequence, whereas the sequence of V_{H} is diversified at positions H50, H52, H52a, H53, H55, H56, H58, H95, H96, H97 and H98 (DVT or NNK encoded, respectively) (**Figure 1**). Library 3 and Library 4 contain a dummy V_{H} sequence, whereas the sequence of V_{κ} is diversified at positions L50, L53, L91, L92, L93, L94 and L96 (DVT or NNK encoded, respectively) (**Figure 1**). The libraries are in phagemid pIT2/ScFv format (**Figure 2)** and have been preselected for binding to generic ligands, Protein A and Protein L, so that the majority of clones in the unselected libraries are functional. The sizes of the libraries shown above correspond to the sizes after preselection. Library 1 and Library 2 were mixed prior to selections on antigen to yield a single V_{H}/dummy V_{κ} library and Library 3 and Library 4 were mixed to form a single V_{κ}/dummy V_{H} library.

Three rounds of selections were performed on β-gal using V_{κ}/dummy V_{H} library and three rounds of selections were performed on HSA using V_{H}/dummy V_{κ} library. In the case of β-gal the phage titres went up from 1.1 x 10⁶ in the first round to 2.0 x 10⁸ in the third round. In the case of HSA the phage titres went up from 2 x 10⁴ in the first round to 1.4 x 10⁹ in the third round. The selections were performed as described by Griffith et al., (1993), except that KM13 helper phage (which contains a pIII protein with a protease cleavage site between the D2 and D3 domains) was used and phage were eluted with 1 mg/ml trypsin in PBS. The addition of trypsin cleaves the pIII proteins derived from the helper phage (but not those from the phagemid) and elutes bound scFv-phage fusions by cleavage in the c-myc tag (Figure 2), thereby providing a further enrichment for phages expressing functional scFvs and a corresponding reduction in background (Kristensen & Winter, Folding & Design 3: 321-328, Jul 9, 1998). Selections were performed using immunotubes coated with either HSA or β-gal at 100 µg/ml concentration.

To check for binding, 24 colonies from the third round of each selection were screened by monoclonal phage ELISA. Phage particles were produced as described by Harrison et al., Methods Enzymol. 1996;267:83-109. 96-well ELISA plates were coated with 100 µl of HSA or β-gal at 10 µg/ml concentration in PBS overnight at 4°C. A standard ELISA protocol was followed (Hoogenboom et al., 1991) using detection of bound phage with anti-M13-HRP conjugate. A selection of clones gave ELISA signals of greater than 1.0 with 50µl supernatant.

Next, DNA preps were made from V_{H}/dummy V_{κ} library selected on HSA and from V_{κ}/dummy V_{H} library selected on β-gal using the QIAprep Spin Miniprep kit (Qiagen).

To access most of the diversity, DNA preps were made from each of the three rounds of selections and then pulled together for each of the antigens. DNA preps were then digested with SaII/NotI overnight at 37°C. Following gel purification of the fragments, V_{κ} chains from the V_{κ}/dummy V_{H} library selected on β-gal were ligated in place of a dummy V_{κ} chain of the V_{H}/dummy V_{κ} library selected on HSA creating a library of 3. 3 x 10⁹ clones.

This library was then either selected on HSA (first round) and β-gal (second round), HSA/β-gal selection, or on β-gal (first round) and HSA (second round), β-gal/HSA selection. Selections were performed as described above. In each case after the second round 48 clones were tested for binding to HSA and β-gal by the monoclonal phage ELISA (as described above) and by ELISA of the soluble scFv fragments. Soluble antibody fragments were produced as described by Harrison et al., (1996), and standard ELISA protocol was followed (Hoogenboom et al. (1991) Nucleic Acids Res., 19: 4133), except that 2% Tween/PBS was used as a blocking buffer and bound scFvs were detected with Protein L-HRP. Three clones (E4, E5 and E8) from the HSA/β- gal selection and two clones (K8 and K10) from the β-gal/HSA selection were able to bind both antigens. scFvs from these clones were PCR amplified and sequenced as described by Ignatovich et al., (1999) J. Mol. Biol .1999 Nov. 26;294(2):457-65, using the primers LMB3 and pHENseq. Sequence analysis revealed that all clones were identical. Therefore, only one clone encoding a dual specific antibody (K8) was chosen for further work (**Figure 3**).

### Example 2. Characterisation of the binding properties of the K8 antibody.

Firstly, the binding properties of the K8 antibody were characterized by the monoclonal phage ELISA. A 96-well plate was coated with 100 µl of HSA and β-gal alongside with alkaline phosphatase (APS), bovine serum albumin (BSA), peanut agglutinin, lysozyme and cytochrome c (to check for cross-reactivity) at 10 µg/ml concentration in PBS overnight at 4°C. The phagemid from K8 clone was rescued with KM13 as described by Harrison et al., (1996) and the supernatant (50 µl) containing phage assayed directly. A standard ELISA protocol was followed (Hoogenboom et al., 1991) using detection of bound phage with anti-M13-HRP conjugate. The dual specific K8 antibody was found to bind to HSA and β-gal when displayed on the surface of the phage with absorbance signals greater than 1.0 (**Figure 4**). Strong binding to BSA was also observed (**Figure 4**).

Since HSA and BSA are 76% homologous on the amino acid level, it is not surprising that K8 antibody recognised both of these structurally related proteins. No cross-reactivity with other proteins was detected (**Figure 4**).

Secondly, the binding properties of the K8 antibody were tested in a soluble scFv ELISA.

Production of the soluble scFv fragment was induced by IPTG as described by Harrison et al., (1996). To determine the expression levels of K8 scFv, the soluble antibody fragments were purified from the supernatant of 50 ml inductions using Protein A Sepharose columns as described by Harlow and Lane, Antibodies: a Laboratory Manual, (1988) Cold Spring Harbor. OD₂₈₀ was then measured and the protein concentration calculated as described by Sambrook et al., (1989). K8 scFv was produced in supernatant at 19mg/l.

A soluble scFv ELISA was then performed using known concentrations of the K8 antibody fragment. A 96-well plate was coated with 100µl of HSA, BSA and β-gal at 10 µg/ml and 100 µl of Protein A at 1 µg/ml concentration. 50 µl of the serial dilutions of the K8 scFv was applied and the bound antibody fragments were detected with Protein L-HRP. ELISA results confirmed the dual specific nature of the K8 antibody (**Figure 5**).

To confirm that binding to β-gal is determined by the V_{κ} domain and binding to HAS/BSA by the V_{H} domain of the K8 scFv antibody, the V_{κ} domain was cut out from K8 scFv DNA by SalI/NotI digestion and ligated into a SalI/NotI digested pIT2 vector containing dummy V_{H} chain **(Figures 1 and 2).** Binding characteristics of the resulting clone K8V_{κ}/dummy V_{H} were analysed by soluble scFv ELISA. Production of the soluble scFv fragments was induced by IPTG as described by Harrison et al., (1996) and the supernatant (50 µl) containing scFvs assayed directly. Soluble scFv ELISA was performed as described in Example 1 and the bound scFvs were detected with Protein L-HRP. The ELISA results revealed that this clone was still able to bind β-gal, whereas binding to BSA was abolished (**Figure 6**).

### Example 3. Selection of single V_{H} domain antibodies antigens A and B and single V_{κ} domain antibodies directed against antigens C and D.

This example describes a method for making single V_{H} domain antibodies directed against antigens A and B and single V_{κ} domain antibodies directed against antigens C and D by selecting repertoires of virgin single antibody variable domains for binding to these antigens in the absence of the complementary variable domains.

Selections and characterization of the binding clones is performed as described previously (see Example 5, PCT/GB 02/003014). Four clones are chosen for further work:
VH1 - Anti A V_{H}
VH2 - Anti B V_{H}
VK1 - Anti C V_{κ}
VK2 - Anti D V_{κ}

The procedures described above in Examples 1-3 may be used, in a similar manner as that described, to produce dimer molecules comprising combinations of V_{H} domains (i.e., V_{H} ligands) and cominations of V_{L} domains (V_{L}-V_{L} ligands).

### Example 4. Creation and characterization of the dual specific ScFv antibodies (VH1/VH2 directed against antigens A and B and VK1/VK2 directed against antigens C and D).

This example demonstrates that dual specific ScFv antibodies (VH1/VH2 directed against antigens A and B and VK1/VK2 directed against antigens C and D) could be created by combining V_{κ} and V_{H} single domains selected against respective antigens in a ScFv vector. To create dual specific antibody VH1/VH2, VH1 single domain is excised from variable domain vector 1 (**Figure 7**) by NcoI/XhoI digestion and ligated into NcoI/XhoI digested variable domain vector 2 (**Figure 7**) to create VH 1/ variable domain vector 2. VH2 single domain is PCR amplified from variable domain vector 1 using primers to introduce a SalI restriction site to the 5' end and a NotI restriction site to the 3' end. The PCR product is then digested with SalI/NotI and ligated into SalI/NotI digested VH1/variable domain vector 2 to create VH 1/VH2/ variable domain vector 2.

VK1/VK2/ variable domain vector 2 is created in a similar way. The dual specific nature of the produced VH1/VH2 ScFv and VK1/VK2 ScFv is tested in a soluble ScFv ELISA as described previously (see Example 6, PCT/GB02/003014). Competition ELISA is performed as described previously (see Example 8,PCT/GB02/003014).

Possible outcomes:
- VH1/VH2 ScFv is able to bind antigens A and B simultaneously
- VK1/VK2 ScFv is able to bind antigens C and D simultaneously
- VH1/VH2 ScFv binding is competitive (when bound to antigen A, VH1/VH2 ScFv cannot bind to antigen B)
- VK1/VK2 ScFv binding is competitive (when bound to antigen C, VK1/VK2 ScFv cannot bind to antigen D)

### Example 5. Construction of dual specific VH1/VH2 Fab and VK1/VK2 Fab and analysis of their binding properties.

To create VH1/VH2 Fab, VH1 single domain is ligated into NcoI/XhoI digested CH vector (**Figure 8**) to create VH 1/CH and VH2 single domain is ligated into SalI/NotI digested CK vector (**Figure 9**) to create VH2/CK. Plasmid DNA from VH1/CH and VH2/CKis used to co-transform competent E. coli cells as described previously (see Example 8, PCT/GB02/003014).

The clone containing VH1/CH and VH2/CK plasmids is then induced by IPTG to produce soluble VH1/VH2 Fab as described previously (see Example 8, PCT/GB 02/003014).

VK1/VK2 Fab is produced in a similar way.

Binding properties of the produced Fabs are tested by competition ELISA as described previously (see Example 8, PCT/GB 02/003014).

Possible outcomes
- VH1/VH2 Fab is able to bind antigens A and B simultaneously
- VK1/VK2 Fab is able to bind antigens C and D simultaneously
- VH1/VH2 Fab binding is competitive (when bound to antigen A, VH1/VH2 Fab cannot bind to antigen B)
- VK1/VK2 Fab binding is competitive (when bound to antigen C, VK1/VK2 Fab cannot bind to antigen D)

### Example 6. Chelating dAb Dimers

### Summary

VH and VK homo-dimers are created in a dAb-linker-dAb format using flexible polypeptide linkers. Vectors were created in the dAb linker-dAb format containing glycine-serine linkers of different lengths 3U:(Gly₄Ser)₃, 5U:(Gly₄Ser)₅, 7U:(Gly₄Ser)₇. Dimer libraries were created using guiding dAbs upstream of the linker: TAR1-5 (VK), 5 TAR1-27(VK), TAR2- 5(VH) or TAR2-6(VK) and a library of corresponding second dAbs after the linker. Using this method, novel dimeric dAbs were selected. The effect of dimerisation on antigen binding was determined by ELISA and BIAcore studies and in cell neutralization and receptor binding assays. Dimerisation of both TAR1-5 and TAR1-27 resulted in significant improvement in binding affinity and neutralization levels.

### 1.0 Methods

### 1.1 Library generation

### 1.1.1 Vectors

pEDA3U, pEDA5U and pEDA7U vectors were designed to introduce different linker lengths compatible with the dAb-linker-dAb format. For pEDA3U, sense and anti- sense 73-base pair oligo linkers were annealed using a slow annealing program (95°C-5mins, 80°C-10mins, 70°C-15mins, 56°C-15mins, 42°C until use) in buffer containing 0.1M NaCl, 10mM Tris-HCl pH7.4 and cloned using the XhoI and NotI restriction sites.

The linkers encompassed 3 (Gly₄Ser) (SEQ ID NO: 7) units and a stuffer region housed between SalI and NotI cloning sites (scheme 1). In order to reduce the possibility of monomeric dAbs being selected for by phage display, the stuffer region was designed to include 3 stop codons, a SacI restriction site and a frame shift mutation to put the region out of frame when no second dAb was present. For pEDA5U and 7U, due to the length of the linkers required, overlapping oligo-linkers were designed for each vector, annealed and elongated using Klenow. The fragment was then purified and digested using the appropriate enzymes before cloning using the XhoI and NotI restriction sites.

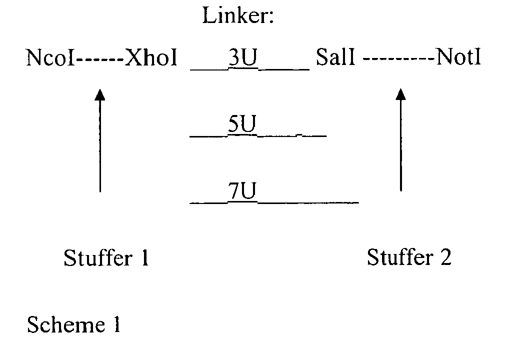

### 1.1.2 Library preparation

The N-terminal V gene corresponding to the guiding dAb was cloned upstream of the linker using NcoI and XhoI restriction sites. VH genes have existing compatible sites, however cloning VK genes required the introduction of suitable restriction sites. This was achieved by using modifying PCR primers (VK-DLIBF: 5' cggccatggcgtcaacggacat-3'; VKXhoIR: 5' atgtgcgctcgagcgtttgattt- 3') in 30 cycles of PCR amplification using a 2:1 mixture of SuperTaq (HTBiotechnology Ltd) and *pfu* turbo (Stratagene). This maintained 5 the NcoI site at the 5' end while destroying the adjacent SalI site and introduced the XhoI site at the 3' end. 5 guiding dAbs were cloned into each of the 3 dimer vectors:
TAR1-5 (VK), TAR1-27(VK), TAR2-5(VH), TAR2- 6(VK) and TAR2-7(VK). All constructs were verified by sequence analysis.

Having cloned the guiding dAbs upstream of the linker in each of the vectors (pEDA3U, 5U and 7U): TAR1-5 (VK), TAR1-27(VK), TAR2-5(VH) or TAR2-6(VK) a library of corresponding second dAbs were cloned after the linker. To achieve this, the complimentary dAb libraries were PCR amplified from phage recovered from round 1 selections of either a VK library against Human TNF-α (at approximately 1 x 10⁶ diversity after round 1) when TAR1-5 or TAR1-27 are the guiding dAbs, or a VH or VK library against human p55 TNF receptor (both at approximately 1 x 10⁵ diversity after round 1) when TAR2-5 or TAR2-6 respectively are the guiding dAbs. For VK libraries PCR amplification was conducted using primers in 30 cycles of PCR amplification using a 2:1 mixture of SuperTaq and *pfu* turbo. VH libraries were PCR amplified using primers in order to introduce a SalI restriction site at the 5' end of the gene. The dAb library PCRs were digested with the appropriate restriction enzymes, ligated into the corresponding vectors down stream of the linker, using SalI/NotI restriction sites and electroporated into freshly prepared competent TG 1 cells.

The titres achieved for each library are as follows:
TAR1-5: pEDA3U = 4x10⁸, pEDA5U = 8x10⁷, pEDA7U = 1x10⁸
TAR1-27: pEDA3U = 6.2x10⁸, pEDA5U = 1x10⁸, pEDA7U = 1x10⁹
TAR2h-5: pEDA3U = 4x10⁷, pEDA5U = 2 x 10⁸, pEDA7U = 8x10⁷
TAR2h-6: pEDA3U = 7.4x10⁸, pEDA5U = 1.2 x 10⁸, pEDA7U = 2.2x10⁸

### 1. 2 Selections

### 1.2.1 TNF-α

Selections were conducted using human TNFa passively coated on immunotubes. Briefly, Immunotubes were coated overnight with 1-4mls of the required antigen. The immunotubes were then washed 3 times with PBS and blocked with 2% milk powder in PBS for 1-2hrs and washed a further 3 times with PBS. The phage solution is diluted in 2%milk powder in PBS and incubated at room temperature for 2hrs. The tubes are then washed with PBS and the phage eluted with 1mg/ml trypsin-PBS. Three selection strategies were investigated for the TAR1-5 dimer libraries. The first round selections were carried out in immunotubes using human TNFa coated at 1 µg/ml or 20 µg/ml with 20 washes in PBS 0.1%Tween. TG1 cells are infected with the eluted phage and the titres are determined (eg, Marks et al. J Mol Biol. 1991 Dec 5;222(3):581-97, Richmann et al Biochemistry. 1993 Aug 31;32(34):8848-55).

The titres recovered were:
pEDA3U = 2.8x10⁷ (1 µg/ml TNF) 1.5x10⁸ (20 µg/ml TNF),
pEDA5U = 1.8x10⁷ (1 µg/ml TNF), 1.6x10⁸ (20 µg/ml TNF)
pEDA7U = 8X10⁶ (1 µg/ml TNF), 7x10⁷ (20 µg/ml TNF).

The second round selections were carried out using 3 different methods.
1. In immunotubes, 20 washes with overnight incubation followed by a further 10 washes.
2. In immunotubes, 20 washes followed by 1hr incubation at RT in wash buffer with (1 µg/ml TNF-α) and 10 further washes.
3. Selection on streptavidin beads using 33 pmoles biotinylated human TNF-α (Henderikx et al., 2002, Selection of antibodies against biotinylated antigens. Antibody Phage Display: Methods and protocols, Ed. O'Brien and Atkin, Humana Press). Single clones from round 2 selections were picked into 96 well plates and crude supernatant preps were made in 2ml 96 well plate format.

| | Round 1 Human TNFαimmunotube coating concentration | Round 2 selection method 1 | Round 2 selection method 2 | Round 2 selection method 3 |
|---|---|---|---|---|
| pEDA3U | 1µg/ml | 1 x 10⁹ | 1.8 x 10⁹ | 2.4 x 10¹⁰ |
| pEDA3U | 20µg/ml | 6 x 10⁹ | 1.8 x 10¹⁰ | 8.5 x 10¹⁰ |
| pEDA5U | 1µg/ml | 9 x 10⁸ | 1.4 x 10⁹ | 2.8 x 10¹⁰ |
| pEDA5U | 20µg/ml | 9.5 x 10⁹ | 8.5 x 10⁹ | 2.8 x 10¹⁰ |
| pEDA7U | 1µg/ml | 7.8 x 10⁸ | 1.6 x 10⁸ | 4 x 10¹⁰ |
| pEDA7U | 20µg/ml | 1 x 10¹⁰ | 8 x 10⁹ | 1.5 x 10¹⁰ |

For TAR1-27, selections were carried out as described previously with the following modifications. The first round selections were carried out in immunotubes using human TNF-α coated at 1 µg/ml or 20 µg/ml with 20 washes in PBS 0.1 %Tween. The second round selections were carried out in immunotubes using 20 washes with overnight incubation followed by a further 20 washes. Single clones from round 2 selections were picked into 96 well plates and crude supernatant preps were made in 2 ml 96 well plate format.

TAR1-27 titres are as follows:

| | Human TNFαimmunotube coating conc | Round 1 | Round 2 |
|---|---|---|---|
| pEDA3U | 1µg/ml | 4 x 10⁹ | 6 x 10⁹ |
| pEDA3U | 20µg/ml | 5 x 10⁹ | 4.4 x 10¹⁰ |
| pEDA3U | 1µg/ml | 1.5 x 10¹⁰ | 1.9 x 10¹⁰ |
| pEDA5U | 20µg/ml | 3.4x10⁹ | 3.5x10¹⁰ |
| pEDA7U | 1µg/ml | 2.6 x 10⁹ | 5 x 10⁹ |
| pEDA7U | 20µg/ml | 7 x 10⁹ | 1.4 x 10¹⁰ |

### 1.2.2 TNF RECEPTOR 1 (p55 RECEPTOR; TAR2)

Selections were conducted as described previously for the TAR2h-5 libraries only. 3 rounds of selections were carried out in immunotubes using either 1 µg/ml human p55 TNF receptor or 10 µg/ml human p55 TNF receptor with 20 washes in PBS 0. 1%Tween with overnight incubation followed by a farther 20 washes. Single clones from round 2 and 3 selections were picked into 96 well plates and crude supernatant preps were made in 2ml 96 well plate format.

0 TAR2h-5 titres are as follows:

| | Round 1 human | Round 1 | Round 2 | Round 3 |
|---|---|---|---|---|
| | p55 TNF receptor immunotube coating concentration | | | |
| pEDA3U | 1µg/ml | 2.4 x 10⁶ | 1.2 x 10⁷ | 1.9 x 10⁹ |
| pEDA3U | 10µg/ml | 3.1 x 10⁷ | 7 x 10⁷ | 1 x 10⁹ |
| pEDA5U | 1µg/ml | 2.5 x 10⁶ | 1.1 x 10⁷ | 5.7 x 10⁸ |
| pEDA5U | 10µg/ml | 3.7 x 10⁷ | 2.3 x 10⁸ | 2.9 x 10⁹ |
| pEDA7U | 1µg/ml | 1.3 x 10⁶ | 1.3 x 10⁷ | 1.4 x 10⁹ |
| pEDA7U | 10µg/ml | 1.6 x 10⁷ | 1.9 x 10⁷ | 3 x 10¹⁰ |

### 1.3 Screening

Single clones from round 2 or 3 selections were picked from each of the 3U, 5U and 7U libraries from the different selections methods, where appropriate. Clones were grown in 2xTY with 100 µg/ml ampicillin and 1% glucose overnight at 37°C. A 1/100 dilution of this culture was inoculated into 2mls of 2xTY with 100 µg/ml ampicillin and 0.1% glucose in 2ml, 96 well plate format and grown at 37°C shaking until OD600 was approximately 0.9. The culture was then induced with 1mM IPTG overnight at 30°C.

The supernatants were clarified by centrifugation at 4000 rpm for 15 mins in a Sorval plate centrifuge. The supernatant preps were used for initial screening.

### 1.3.1 ELISA

Binding activity of dimeric recombinant proteins was compared to monomer by Protein A/L ELISA or by antigen ELISA. Briefly, a 96 well plate is coated with antigen or Protein A/L overnight at 4°C. The plate is washed with 0.05% Tween-PBS, blocked for 2hrs with 2% Tween-PBS. The sample is added to the plate, and incubated for 1 hr at room temperature. The plate is washed and incubated with the secondary reagent for 1 hr at room temperature. The plate is washed and developed with TMB substrate. Protein A/L HRP or India-HRP was used as a secondary reagent. For antigen ELISAs, the antigen concentrations used were 1 µg/ml in PBS for Human TNFa and human TNF receptor 1. Due to the presence of the guiding dAb in most cases dimers gave a positive ELISA signal - therefore off rate determination was examined by BIAcore.

### 1.3.2 BIAcore

BIAcore analysis was conducted for TAR1-5 and TAR2h-5 clones. For screening, Human TNF-α was coupled to a CM5 chip at high density (approximately 10,000 RUs).

50 µl of Human TNFa (50 µg/ml) was coupled to the chip at 5 µl/min in acetate buffer pH5.5. Regeneration of the chip following analysis using the standard methods is not possible due to the instability of Human TNF-α. Ttherefore after each sample was analysed, the chip was washed for 10 mins with buffer.

For TAR1-5, clone supernatants from the round 2 selection were screened by BIAcore. 48 clones were screened from each of the 3U, 5U and 7U libraries obtained using the following selection methods:
R1: 1 µg/ml human TNF-α immunotube, R2 1 µg/ml human TNF-α immunotube, overnight wash.
R1: 20 µg/ml human TNF-α immunotube, R2 20 µg/ml human TNF-α immunotube, overnight wash.
R1: 1 µg/ml human TNF-α immunotube, R2 33 pmoles biotinylated human TNF-α on beads.
R1: 20 µg/ml human TNF-α immunotube, R2 33 pmoles biotinylated human TNF-α beads.

For screening, human p55 TNF receptor was coupled to a CM5 chip at high density (approximately 4,000 RUs). 100 µl of human p55 TNF receptor (10 10 µg/ml) was coupled to the chip at 5 µl/min in acetate buffer - pH5.5. Standard regeneration conditions were examined (glycine pH2 or pH3) but in each case antigen was removed from the surface of the chip - therefore as with TNF-α, after each sample was analysed, the chip was washed for 10 mins with buffer.

For TAR2-5, clones supernatants from the round selection were screened.

48 clones were screened from each of the 3U, 5U and 7U libraries, using the following selection methods:
R1: 1 µg/ml human p55 TNF receptor immunotube, R2 1 µg/ml human p55 TNF receptor immunotube, overnight wash.
R1: 10 µg/ml human p55 TNF receptor immunotube, R2 10 µg/ml human p55 TNF receptor immunotube, overnight wash.

### 1.3.3 Receptor and Cell Assays

The ability of the dimers to neutralise in the receptor assay was conducted as follows:

### Receptor binding

Anti-TNF dAbs were tested for the ability to inhibit the binding of TNF to recombinant TNF receptor 1 (p55). Briefly, Maxisorp plates were incubated overnight with 30mg/ml anti-human Fc mouse monoclonal antibody (Zymed, San Francisco, USA). The wells were washed with phosphate buffered saline (PBS) containing 0.05% Tween-20 and then blocked with 1% BSA in PBS before being incubated with 100 ng/ml TNF receptor 1 Fc fusion protein (R&D Systems, Minneapolis, USA). Anti-TNF dAb was mixed with TNF which was added to the washed wells at a final concentration of 10 ng/ml. TNF binding was detected with 0.2mg/ml biotinylated anti-TNF antibody (HyCult biotechnology, Uben, Netherlands) followed by 1 in 500 dilution of horse radish peroxidase labelled streptavidin (Amersham Biosciences, UK) and then incubation with TMB substrate (KPL, Gaithersburg, USA). The reaction was stopped by the addition of HCl and the absorbance was read at 450nm. Anti-TNF dAb activity lead to a decrease in TNF binding and therefore a decrease in absorbance compared with the TNF only control.

L929 Cytotoxicity Assay Anti-TNF dAbs were also tested for the ability to neutralise the cytotoxic activity of TNF on mouse L929 fbroblasts (Evans, T. (2000) Molecular Biotechnology 15, 243-248). Briefly, L929 cells plated in microtitre plates were incubated overnight with anti-TNF dAb, 100 pg/ml TNF and 1 mg/ml actinomycin D (Sigma, Poole, UK). Cell viability was measured by reading absorbance at 490nm following an incubation with [3-(4,5-dimethylthiazol-2-yl)-5-(3 - carbboxymethoxyphenyl)-2-(4-sulfophenyl)-2H- tetrazolium (Promega, Madison, USA). Anti-TNF dAb activity lead to a decrease in TNF cytotoxicity and therefore an increase in absorbance compared with the TNF only control.

In the initial screen, supernatants prepared for BLAcore analysis, described above, were also used in the receptor assay. Further analysis of selected dimers was also conducted in the receptor and cell assays using purified proteins.

### HeLa IL-8 assay

Anti-TNFR1 or anti-TNF alpha dAbs were tested for the ability to neutralise the induction of IL-8 secretion by TNF in HeLa cells (method adapted from that of Akeson, L. et al. (1996) Journal of Biological Chemistry 271, 30517-30523, describing the induction of IL-8 by IL-1 in HUVEC; here we look at induction by human TNF alpha and we use HeLa cells instead of the HUVEC cell line). Briefly, HeLa cells plated in microtitre plates were incubated overnight with dAb and 300pg/ml TNF. Post incubation the supernatant was aspirated off the cells and IL-8 concentration measured via a sandwich ELISA (R&D Systems). Anti-TNFR1 dAb activity lead to a decrease in IL-8 secretion into the supernatant compared with the TNF only control.

The L929 assay is used throughout the following experiments; however, the use of the HeLa IL-8 assay is preferred to measure anti-TNF receptor 1 (p55) ligands; the presence of mouse p55 in the L929 assay poses certain limitations in its use.

### 1.4 Sequence analysis

Dimers that proved to have interesting properties in the BIAcore and the receptor assay screens were sequenced. Sequences are detailed in the sequence listing, Figures and Examples herein below.

### 1.5 Formatting

### 1.5.1 TAR1-5-19 dimers

The TAR1-5 dimers that were shown to have good neutralization properties were reformatted and analysed in the cell and receptor assays. The TAR1-5 guiding dAb was substituted with the affinity matured clone TAR1-5-19. To achieve this TAR1-5 was cloned out of the individual dimer pair and substituted with TAR1-5-19 that had been amplified by PCR. In addition TAR1-5-19 homodimers were also constructed in the 3U, 5U and 7U vectors. The N terminal copy of the gene was amplified by PCR and cloned as described above and the C-terminal gene fragment was cloned using existing SalI and NotI restriction sites.

### 1.5.2 Mutagenesis

The amber stop codon present in dAb2, one of the C- terminal dAbs in the TAR1-5 dimer pairs was mutated to a glutamine by site-directed mutagenesis.

### 1.5.3 Fabs

The dimers containing TAR1-5 or TAR1-5-19 were re-forrnatted into Fab expression vectors. dAbs were cloned into expression vectors containing either the CK or CH genes using SfiI and NotI restriction sites and verified by sequence analysis. The CK vector is derived from a pUC based ampicillin resistant vector and the CH vector is derived from a pACYC chloramphenicol resistant vector. For Fab expression the dAb-CH and dAb-CK constructs were co-transformed into HB2151 cells and grown in 2xTY containing 0.1% glucose, 100 µg/ml anpicillin and 10 µg/ml chloramphenicol.

### 1.5.3 Hinge dimerisation

Dimerisation of dAbs via cystine bond formation was examined. A short sequence of amino acids EPKSGDKTHTCPPCP (SEQ ID NO: 11) a modified form of the human IgGC1 hinge was engineered at the C terminal region on the dAb. An oligo linker encoding for this sequence was synthesised and annealed, as described previously. The linker was cloned into the pEDA vector containing TAR1-5-19 using XhoI and NotI restriction sites. Dimerisation occurs in situ in the periplasm.

### 1.6 Expression and purification

### 1.6.1 Expression

Supernatants were prepared in the 2ml, 96-well plate format for the initial screening as described previously. Following the initial screening process selected dimers were analysed further. Dimer constructs were expressed in TOP10F' or HB2151 cells as supernatants. Briefly, an individual colony from a freshly streaked plate was grown overnight at 37°C in 2xTY with 100 µg/ml ampicillin and 1% glucose. A 1/100 dilution of this culture was inoculated into 2xTY with 100 µg/ml ampicillin and 0.1% glucose and grown at 37°C shaking until OD600 was approximately 0.9. The culture was then induced with 1mM IPTG overnight at 30°C. The cells were removed by centrifugation and the supernatant purified with protein A or L agarose.

Fab and cysteine hinge dimers were expressed as periplasmic proteins in HB2152 cells.

A 1/100 dilution of an overnight culture was inoculated into 2xTY with 0. 1% glucose and the appropriate antibiotics and grown at 37°C with shaking until OD600 was approximately 0.9. The culture was then induced with 1mM IPTG for 3-4 hours at 30°C. The cells were harvested by centrifugation and the pellet resuspended in periplasmic preparation buffer (30mM Tris-HCl pH8.0, 1mM EDTA, 20% sucrose). Following centrifugation the supernatant was retained and the pellet resuspended in 5mM MgSO4. The supernatant was harvested again by centrifugation, pooled and purified.

### 1.6.2 Protein A/L purification

Optimisation of the purification of dimer proteins from Protein L agarose (Affitech, Norway) or Protein A agarose (Sigma, UK) was examined. Protein was eluted by batch or by column elusion using a peristaltic pump. Three buffers were examined: 0.1M Phosphate-citrate buffer pH2.6; 0.2M Glycine pH2.5; and 0.1M Glycine pH2.5.
The optimal condition was determined to be under peristaltic pump conditions using 0.1M Glycine pH2.5 over 10 column volumes. Purification from protein A was conducted peristaltic pump conditions using 0.1M Glycine pH2.5.

### 1.6.3 FPLC purification

Further purification was carried out by FPLC analysis on the AKTA Explorer 100 system (Amersham Biosciences Ltd). TAR1-5 and TAR1-5-19 dimers were fractionated by cation exchange chromatography (lml Resource S - Amersham Biosciences Ltd) eluted with a 0-1M NaCl gradient in 50mM acetate buffer pH4. Hinge dimers were purified by ion exchange (1ml Resource Q Amersham Biosciences Ltd) eluted with a 0- 1M NaCl gradient in 25mMTris HCl pH 8.0. Fabs were purified by size exclusion chromatography using a superose 12 (Amersham Biosciences Ltd) column run at a flow rate of 0.5ml/min in PBS with 0.05% tween. Following purification, samples were concentrated using Vivaspin 5K cut off concentrators (Vivascience Ltd).

### 2.0 Results

### 2.1 TAR1-5 dimers

6 x 96 clones were picked from the round 2 selection encompassing all the libraries and selection conditions. Supernatant preps were made and assayed by antigen and Protein L ELISA, BIAcore and in the receptor assays. In ELISAs, positive binding clones were identified from each selection method and were distributed between 3U, 5U and 7U libraries. However, as the guiding dAb is always present it was not possible to discriminate between high and low affinity binders by this method - therefore BIAcore analysis was conducted.

BIAcore analysis was conducted using the 2ml supernatants. BIAcore analysis revealed that the dimer Koff rates were vastly improved compared to monomeric TAR1-5. Monomer Koff rate was in the range of 10⁻¹M compared with dimer Koff rates which were in the range of 10⁻³ - 10⁻⁴M. 16 clones that appeared to have very slow off rates were selected, these came from the 3U, 5U and 7U libraries and were sequenced. In addition the supernatants were analysed for the ability to neutralize human TNF-α in the receptor assay.

6 lead clones (d1-d6 below) that neutralized in these assays and have been sequenced. The results show that out of the 6 clones obtained there are only 3 different second dAbs (dAb1, dAb2 and dAb3) however where the second dAb is found more than once they are linked with different length linkers.
**TAR1-5d1:** 3U linker 2nd dAb=dAb1 - 1 µg/ml Ag immunotube overnight wash
**TAR1-5d2:** 3U linker 2nd dAb=dAb2 - 1 µg/ml Ag immunotube overnight wash
**TAR1-5d3:** 5U linker 2nd dAb=dAb2 - 1 µg/ml Ag immunotube overnight wash
**TAR1-5d4:** 5U linker 2nd dAb=dAb3 - 20 µg/ml Ag immunotube overnight wash
**TAR1-5d5:** 5U linker 2nd dAb=dAb1 - 20µg/ml Ag immunotube overnight wash
**TAR1-5d6:** 7U linker 2nd dAb=dAbl- R1:1 µg/ml Ag immunotube overnight wash, R2:beads

The 6 lead clones were examined further. Protein was produced from the periplasm and supernatant, purified with protein L agarose and examined in the cell and receptor assays. The levels of neutralization were variable (Table 1). The optimal conditions for protein preparation were determined. Protein produced from HB2151 cells as supernatants gave the highest yield (approximately 10mgs/L of culture). The supernatants were incubated with protein L agarose for 2hrs at room temperature or overnight at 4°C. The beads were washed with PBS/NaCl and packed onto an FPLC column using a peristaltic pump. The beads were washed with 10 column volumes of PBS/NaCl and eluted with 0.1M glycine pH2.5. In general, dimeric protein is eluted after the monomer.

TARI-5d1-6 dimers were purified by FPLC. Three species were obtained, by FPLC purification and were identified by SDS PAGE. One species corresponds to monomer and the other two species corresponds to dimers of different sizes. The larger of the two species is possibly due to the presence of C terminal tags. These proteins were examined in the receptor assay. The data presented in Table 1 represent, the optimum results obtained from the two dimeric species (**Figure 11**) The three second dAbs from the dimer pairs (i.e., dAb1, dAb2 and dAb3) were cloned as monomers and examined by ELISA and in the cell and receptor assay. All three dAbs bind specifically to TNF by antigen ELISA and do not cross react with plastic or BSA. As monomers, none of the dAbs neutralise in the cell or receptor assays.

### 2.1.2 TAR1-5-19 dimers

TAR1-5-19 was substituted for TAR1-5 in the 6 lead clones. Analysis of all TAR1-5-19 dimers in the cell and receptor assays was conducted using total protein (protein L purified only) unless otherwise stated (Table 2). TAR1-5-19d4 and TAR1-5-19d3 have the best ND₅₀ (~5 nM) in the cell assay, this is consistent with the receptor assay results and is an improvement over TAR 1-5-19 monomer (ND₅₀ ~30nM). Although purified TAR1-5 dimers give variable results in the receptor and cell assays, TAR1-5-19 dimers were more consistent. Variability was shown when using different elution buffers during the protein purification. Elution using 0.1 M Phosphate- citrate buffer pH2.6 or 0.2M Glycine pH2.5 although removing all protein from the protein L agarose in most cases rendered it less functional.

TAR1-5-19d4 was expressed in the fermenter and purified on cation exchange FPLC to yield a completely pure dimer. As with TAR 1-5d4 three species were obtained by FPLC purification, corresponding to monomer and two dimer species. This dimer was amino acid sequenced. TAR1-5-19 monomer and TAR1-5-19d4 were then examined in the receptor assay and the resulting IC₅₀ for monomer was 30nM and for dimer was 5nM.

The results of the receptor assay comparing TAR1-5-19 monomer, TAR1-5- 19d4 and TAR1-5d4 are shown in **Figure 10.**

TAR1-5-19 homodimers were made in the 3U, 5U and 7U vectors, expressed and purified on Protein L. The proteins were examined in the cell and receptor assays and the resulting IC50s (for receptor assay) and ND₅₀s (for cell assay) were determined (Table 3, **Figure 12**).

### 2.2 Fabs

TAR1-5 and TAR1-5-19 dimers were also cloned into Fab format, expressed and purified on protein L agarose. Fabs were assessed in the receptor assays (Table 4). The results showed that for both TAR1-5-19 and TAR1-5 dimers the neutralization levels were similar to the original Gly₄Ser linker dimers from which they were derived. A TAR1-5-19 Fab where TAR1-5-19 was displayed on both CH and CK was expressed, protein L purified and assessed in the receptor assay. The resulting IC₅₀ was approximately 1 nM.

### 2.3 TAR1-27 dimers

3 x 96 clones were picked from the round 2 selection encompassing all the libraries and selection conditions. 2ml supernatant preps were made for analysis in ELISA and bioassays. Antigen ELISA gave 71 positive clones. The receptor assay of crude supernatants yielded 42 clones with inhibitory properties (TNF binding 0- 60%). In the majority of cases inhibitory properties correlated with a strong ELISA signal. 42 clones were sequenced, 39 of these have unique second dAb sequences. The 12 dimers that gave the best inhibitory properties were analysed further.

The 12 neutralizing clones were expressed as 200ml supernatant preps and purified on protein L. These were assessed by protein L and antigen ELISA, BIAcore and in the receptor assay. Strong positive ELISA signals were obtained in all cases. BIAcore analysis revealed all clones to have fast on and off rates. The off rates were improved compared to monomeric TAR1-27, however the off rate of TAR1-27 dimers was faster (Koff is approximately in the range of 10⁻¹ and 10⁻²M) than the TAR1-5 dimers examined previously (Koff is approximately in the range of 10⁻³ - 10⁻⁴M). The stability of the purified dimers was questioned and therefore in order to improve stability, the addition on 5%glycerol, 0.5% Triton X100 or 0.5% NP40 (Sigma) was included in the purification of 2 TAR1-27 dimers (d2 and d16). Addition of NP40 or Triton X100 improved the yield of purified product approximately 2 fold. Both dimers were assessed in the receptor assay. TAR1-27d2 gave IC₅₀ of~30nM under all purification conditions. TARI-27d16 showed no neutralization effect when purified without the use of stabilising agents but gave an IC₅₀ of~50 nM when purified under stabilising conditions. No further analysis was conducted.

### 2.4 TAR1-5 dimers

3 x 96 clones were picked from the second round selections encompassing all the libraries and selection conditions. 2ml supernatant preps were made for analysis. Protein A and antigen ELISAs were conducted for each plate. 30 interesting clones were identified as having good off-rates by BIAcore (Koff ranges between 10⁻² - 10⁻³M). The clones were sequenced and 13 unique dimers were identified by sequence analysis.

**Table 1: TAR1-5 Dimers**

| **Dimer** | **Cell type** | **Purification** | **Protein Fraction** | **Elution conditions** | **Receptor/ Cell assay** |
|---|---|---|---|---|---|
| TAR1-5d1 | HB2151 | Protein L + FPLC | small dimeric species | 0.1M glycine | RA~30nM |
| | | | | pH2.5 | |
| TAR1-5d2 | HB2151 | Protein L + FPLC | small dimeric species | 0.1M glycine | RA~50nM |
| | | | | pH2.5 | |
| | | FPLC | species | pH2.5 | M |
| TAR1-5d3 | HB2151 | Protein L + FPLC | large dimeric species | 0.1M glycine | RA~300nM |
| | | | | pH2.5 | |
| TAR1-5d4 | HB2151 | Protein L + FPLC | small dimeric species | 0.1M glycine | RA~3nM |
| | | | | pH2.5 | |
| TAR1-5d5 | HB2151 | Protein L + FPLC | large dimeric species | 0.1M glycine | RA~200nM |
| | | | | pH2.5 | |
| TAR1-5d6 | HB2151 | Protein L +FPLC | Large dimeric species | 0.1M glycine | RA~100nM |
| | | | | pH2.5 | |

| | | | | | |
|---|---|---|---|---|---|
| *note dimer 2 and dimer 3 have the same second dAb (called dAb2), however have different linker lengths (d2 = (Gly₄Ser)₃, d3 = (Gly₄Ser)₃). dAb1 is the partner dAb to dimers 1, 5 and 6. dAb3 is the partner dAb to dimer 4. None of the partner dAbs neutralise alone. FPLC purification is by cation exchange unless otherwise stated. The optimal dimeric species for each dimer obtained by FPLC was determined in these assays. | | | | | |

**Table 2: TAR1-5-19 Dimers**

| **Dimer** | **Cell type** | **Purification** | **Protein Fraction** | **Elution conditions** | **Receptor/ Cell assay** |
|---|---|---|---|---|---|
| TAR1-5-19d1 | TOP10F' | Protein L | Total protein | 0.1M glycine | RA~15nM |
| | | | | pH2.0 | |
| TAR1-519 d2 (no stop codon) | TOP10F' | Protein L | Total protein | 0.1M glycine | RA~2nM |
| | | | | pH2.0 + 0.05%NP40 | |
| TAR1-5-19d3 (no stop codon) | TOP10F' | Protein L | Total protein | 0.1M glycine | RA~8nM |
| | | | | pH 2.5 + 0.05%NP40 | |
| TAR1-5-19d4 | TOP10F' | Protein L + FPLC | FPLC purified fraction | 0.1M glycine | RA~2nM 5nM |
| | | | | pH2.0 | CA~12nM |
| TAR1-5-19d5 | TOP10F' | Protein L | Total protein | 0.1M glycine | RA~8nM |
| | | | | pH2.0 + NP40 | CA~10nM |
| TAR1-5-19d6 | TOP10F' | Protein L | Total protein | 0.1M glycine | RA~10nM |
| | | | | pH 2.0 | |

**Table 3: TAR1-5-19 homodimers**

| | | | | | |
|---|---|---|---|---|---|
| **Dimer** | **Cell type** | **Purification** | **Protein Fraction** | **Elution conditions** | **Receptor/ Cell assay** |
| homodimer | | | | | nM CA~30nM |
| TAR1-5-19 5U homodimer | HB2151 | Protein L | Total protein | 01M glycine | RA~2nM |
| | | | | pH2.5 | CA~3nM |
| TAR1-5-19 7U homodimer | HB2151 | Protein L + FPLC | FPLC purified dimer fraction | 01M glycine | RA~10nM |
| | | | | pH2.5 | CA~15nM |
| TAR1-5-19 cys hinge | HB2151 | Protein | Total protein | 01M glycine | RA~2nM |
| | | | | pH2.5 | |
| TAR1-5-19CH/ TAR1-5-19CK | HB2151 | Protein | Total protein | 01M glycine | RA~1nM |
| | | | | pH2.5 | |

**Table 4: TAR1-5/TAR1-5-19 Fabs**

| **Dimer** | **Cell type** | **Purification** | **Protein Fraction** | **Elution conditions** | **Receptor/ Cell assay** |
|---|---|---|---|---|---|
| TAR1-5CH/ dAb1CK | HB2151 | Protein L | Total protein | 0.1M citrate | RA~90nM |
| | | | | pH2.6 | |
| TAR1-5CH/ dAb2 CK | HB2151 | Protein L | Total protein | 0.1M citrate | RA~30nM |
| | | | | pH2.5 | CA~60nM |
| dAb3CH/ TAR1-5CK | HB2151 | Protein L | Total protein | 0.1M citrate | RA~10nM |
| | | | | pH2.6 | |
| TAR1-5-19CH/ dAb1 CK | HB2151 | Protein L | Total protein | 0.1M citrate | RA~6nM |
| | | | | pH2.0 | |
| dAb1 CH/ TAR1-5-19CK | HB2151 | Protein L | 0.1M glycine | Myc/flag | RA~6nM |
| | | | pH2.0 | | |
| TAR1-5-19CH/ dAb2 CK | HB2151 | Protein L | Total protein | 0.1M glycine | RA~8nM |
| | | | | pH2.0 | CA~12nM |
| TAR1-5-19CH/ dAb3 CK | HB2151 | Protein L | Total protein | 0.1M glycine | RA~3nM |
| | | | | pH2.0 | |

### PCR construction of TAR1-5-19CYS dimer

See example 8 describing the dAb trimer. The trimer protocol gives rise to a mixture of monomer, dimer and trimer.

### Expression and purification of TAR1-5-19CYS dimer

The dimer was purified from the supernatant of the culture by capture on Protein L agarose as outlined in the example 8.

### Separation of TAR1-5-19CYS monomer from the TAR1-5-19CYS dimer

Prior to cation exchange separation, the mixed monomer/dimer sample was buffer exchanged into 50 mM sodium acetate buffer pH 4.0 using a PD-10 column (Amersham Pharmacia), following the manufacturer's guidelines. The sample was then applied to a ImL Resource S cation exchange column (Amersham Pharmacia), which had been pre-equilibrated with 50 mM sodium acetate pH 4.0. The monomer and dimer were separated is using the following salt gradient in 50 mM sodium acetate pH 4.0:
150 to 200 mM sodium chloride over 15 column volumes
200 to 450 mM sodium chloride over 10 column volumes
450 to 1000 mM sodium chloride over 15 column volumes.

Fractions containing dimer only were identified using SDS-PAGE and then pooled and the pH increased to 8 by the addition of 1/5 volume of 1M Tris pH 8.0.

### In vitro functional binding assay: TNF receptor assay and cell assay

The affinity of the dimer for human TNFcz was determined using the TNF receptor and cell assay. IC50 in the receptor assay was approximately 0.3- 0.8 nM; ND50 in the cell assay was approximately 3-8 nM.

### Other possible TAR1-5-19CYS dimer formats

### PEG dimers and custom synthetic maleimide dimers

Nektar (Shearwater) offer a range of bi-maleimide PEGs [mPEG2-(MAL)2 or mPEG-(MAL)2] which would allow the monomer to be formatted as a dimer, with a small linker separating the dAbs and both being linked to a PEG ranging in size from 5 to 40 kDa. It has been shown that the 5kDa mPEG-(MAL)2 (i.e., [TARI-5-19]- Cys-maleimide-PEG x 2, wherein the maleimides are linked together in the dimer) has an affinity in the TNF receptor assay off~1-3 nM. Also the dimer can also be produced using TMEA (Tris[2 5 maleimidoethyl]amine) (Pierce Biotechnology) or other bi-functional linkers.

It is also possible to produce the disulphide dimer using a chemical coupling procedure using 2,2'-dithiodipyridine (Sigma Aldrich) and the reduced monomer.

### Addition of a polypeptide linker or hinge to the C-terminuls of the dAb.

A small linker, either (Gly₄Ser)ₙ where n=1 to 10, eg, 1, 2, 3, 4, 5, 6 or 7, an immunoglobulin (e.g., IgG hinge region or random peptide sequence (e.g., selected from a library of random peptide sequences) can be engineered between the dAb and the terminal cysteine residue. This can then be used to make dimers as outlined above.

### Example 8. dAb Trimerisation

### Summary

For dAb trimerisation, a free cysteine is required at the C-terminus of the protein. The cysteine residue, once reduced to give the free thiol, can then be used to specifically couple the protein to a trimeric maleimide molecule, for example TMEA (Tris[2 maleimidoethyl]amine).

### PCR construction of TAR1-5-19CYS

The following oligonucleotides were used to specifically PCR TAR1-5-19 with a SalI and BamHI sites for cloning and also to introduce a C-terminal cysteine residue: 
Sal I

Trp Ser Ala Ser Thr Asp* Ile Gln Met Thr Gln Ser Pro Ser Ser Leu Ser Ala Ser Val
 
TGG AGC GCG TCG ACG GAC ATC CAG ATG ACC CAG TCT CCA TCC TCT CTG TCT GCA TCT GTA

ACC TCG CGC AGC TGC CTG TAG GTC TAC TGG GTC AGA GGT AGG AGA GAC AGA CGT AGA CAT

Gly Asp Arg Val Thr Ile Thr Cys Arg Ala Ser Gln Ser Ile Asp Ser Tyr Leu His Trp

GGA GAC CGT GTC ACC ATC ACT TGC CGG GCA AGT CAG AGC ATT GAT AGT TAT TTA CAT TGG

CCT CTG GCA CAG TGG TAG TGA ACG GCC CGT TCA GTC TCG TAA CTA TCA ATA AAT GTA ACC

Tyr Gln Gln Lys Pro Gly Lys Ala Pro Lys Leu Leu Ile Tyr Ser Ala Ser Glu Leu Gln

TAC CAG CAG AAA CCA GGG AAA GCC CCT AAG CTC CTG ATC TAT AGT GCA TCC GAG TTG CAA

ATG GTC GTC TTT GGT CCC TTT CGG GGA TTC GAG GAC TAG ATA TCA CGT AGG CTC AAC GTT

Ser Gly Val Pro Ser Arg Phe Ser Gly Ser Gly Ser Gly Thr Asp Phe Thr Leu Thr Ile

AGT GGG GTC CCA TCA CGT TTC AGT GGC AGT GGA TCT GGG ACA GAT TTC ACT CTC ACC ATC

TCA CCC CAG GGT AGT GCA AAG TCA CCG TCA CCT AGA CCC TGT CTA AAG TGA GAG TGG TAG

Ser Ser Leu Gln Pro Glu Asp Phe Ala Thr Tyr Tyr Cys Gln Gln Val Val Trp Arg Pro

AGC AGT CTG CAA CCT GAA GAT TTT GCT ACG TAC TAC TGT CAA CAG GTT GTG TGG CGT CCT

TCG TCA GAC GTT GGA CTT CTA AAA CGA TGC ATG ATG ACA GTT GTC CAA CAC ACC GCA GGA

BamHI

Phe Thr Phe Gly Gln Gly Thr Lys Val Glu Ile Lys Arg Cys *** *** Gly Ser Gly

TTT ACG TTC GGC CAA GGG ACC AAG GTG GAA ATC AAA CGG TGC TAA TAA GGA TCC GGC

AAA TGC AAG CCG GTT CCC TGG TTC CAC CTT TAG TTT GCC ACG ATT ATT CCT AGG CCG
 
(* start of TAR1-5-19CYS sequence; Amino Acid sequence = SEQ ID NO: 12;
Nucleotide Sequence = SEQ ID NO: 13)
 Forward primer 5'- TGGAGCGCGTCGACGGACATCCAGATGACCCAGTCTCCA-3' (SEQ ID NO: 14)
Reverse primer 5'-TTAGCAGCCGGATCCTTATTAGCACCGTTTGATTTCCAC-3' (SEQ ID NO: 15)

The PCR reaction (50µL volume) was set up as follows: 200 µM dNTPs, 0. 4 µM of each primer, 5 µL of 10x PfuTurbo buffer (Stratagene), 100 ng of template plasmid (encoding TAR1-5-19), 1µL of PfuTurbo enzyme (Stratagene) and the volume adjusted to 50 µL using sterile water. The following PCR conditions were used: initial denaturing step 94 °C for 2 mins, then cycles of 94 °C for 30 sees, 64 °C for 30 sec and 72 °C for 30 sec. A final extension step was also included of 72 °C for 5 mins. The PCR product was purified and digested with SalI and BamHI and ligated into the vector which had also been cut with the seine reskiction enzymes. Correct clones were verified by DNA sequencing.

### Expression and purification of TAR1-5-19CYS

TAR1-5-19CYS vector was transformed into BL21 (DE3) pLysS chemically competent cells (Novagen) following the manufacturer's protocol. Cells carrying the dAb plasmid were selected for using 100 µg/mL carbenicillin and 37 µg/mL chloramphenicol. Cultures were set up in 2L baffled flasks containing 500 mL of terrific broth (Sigma-Aldrich), 100 µg/mL carbenicillin and 37 µg/mL chloramphenicol. The cultures were grown at 30 °C at 200rpm to an O. D.600 of 1-1.5 and then induced with 1mM IPTG (isopropyl-beta-D-thiogalactopyranoside, from Melford Laboratories). The expression of the dAb was allowed to continue for 12-16 hrs at 30 °C. It was found that most of the dAb was present in the culture medium. Therefore, the cells were separated from the medium by centrifugation (8,000xg for 30 min), and the supernatant used to purify the dAb. Per litre of supernatant, 30 mL of Protein L agarose (Afftech) was added and the dAb allowed to batch bind with stirring for 2 hours. The resin was then allowed to settle under gravity for a further hour before the supernatant was siphoned off. The agarose was then packed into a XK 50 column (Amersham Phamacia) and was washed with 10 column volumes of PBS. The bound dAb was eluted with 100 mM glycine pH 2.0 and protein containing fractions were then neutralized by the addition of 1/5 volume of 1 M Tris pH 8.0. Per litre of culture supernatant 20 mg of pure protein was isolated, which contained a 50:50 ratio of monomer to dimer.

### Trimerisation of TAR1-5-19CYS

2.5 ml of 100 M TAR1-5-19CYS was reduced with 5 mM dithiothreitol and left at room temperature for 20 minutes. The sample was then buffer exchanged using a PD-10 column (Amersham Pharmacia). The column had been pre-equilibrated with 5 mM EDTA, 50 mM sodium phosphate pH 6.5, and the sample applied and eluted following the manufacturer's guidelines. The sample was placed on ice until required. TMEA (Tris[2-maleimidoethyl]amine) was purchased from Pierce Biotechnology. A 20 mM stock solution of TMEA was made in 100% DMSO (dimethyl sulphoxide). It was found that a concentration of TMEA greater than 3:1 (molar ratio of dAb:TMEA) caused the rapid precipitation and cross-linking of the protein. Also the rate of precipitation and cross-linking was greater as the pH increased. Therefore using 100 µM reduced TAR 1-5-19CYS, 25 µM TMEA was added to trimerise the protein and the reaction allowed to proceed at room temperature for two hours. It was found that the addition of additives such as glycerol or ethylene glycol to 20% (v/v), significantly reduced the precipitation of the trimer as the coupling reaction proceeded. After coupling, SDS-PAGE analysis showed the presence of monomer, dimer and trimer in solution.

### Purification of the trimeric TAR1-5-19CYS

40 µL of 40% glacial acetic acid was added per mL of the TMEA-TAR1-5- 1 9cys reaction to reduce the pH to 4. The sample was then applied to a 1mL Resource S cation exchange column (Amersham Pharmacia), which had been pre-equilibrated with 50 mM sodium acetate pH 4.0. The dimer and trimer were partially separated using a salt gradient of 340 to 450 mM Sodium chloride, 50 mM sodium acetate pH 4.0 over 30 column volumes. Fractions containing trimer only were identified using SDS-PAGE and then pooled and the pH increased to 8 by the addition of 1/5 volume of 1M Tris pH 8.0. To prevent precipitation of the trimer during concentration steps (using 5K cut off Viva spin concentrators; Vivascience), 10% glycerol was added to the sample.

### In vitro functional binding assay: TNF receptor assay and cell assay

The affinity of the trimer for human TNF-α was determined using the TNF receptor and cell assay. IC50 in the receptor assay was 0.3nM; ND50 in the cell assay was in the range of 3 to 10nM (e.g., 3nM).

### Other possible TAR1-5-19CYS trimer formats

TAR1-5-19CYS may also be formatted into a trimer using the following reagents:

### PEG trimers and custom synthetic maleimide trimers

Nektar (Shearwater) offers a range of multi arm PEGs, which can be chemically modified at the terminal end of the PEG. Therefore using a PEG trimer with a maleimide functional group at the end of each arm would allow the trimerisation of the dAb in a manner similar to that outlined above using THEA. The PEG may also have the advantage in increasing the solubility of the trimer thus preventing the problem of aggregation. Thus, one could produce a dAb trimer in which each dAb has a C-terminal cysteine that is linked to a maleimide functional group, the maleimide functional groups being linked to a PEG trimer.

### Addition of a polypeptide linker or hinge to the C-terminus of the dAb

A small linker, either (Gly₄Ser)ₙ where n= 1 to 10, eg, 1, 2, 3, 4, 5, 6 or 7, an inmunoglobulin (eg, IgG hinge region) or random peptide sequence (eg, selected from a library of random peptide sequences) could be engineered between the dAb and the terminal cysteine residue. When used to make multimers (eg, dimers or trimers), this again would introduce a greater degree of flexibility and distance between the individual monomers, which may improve the binding characteristics to the target, eg a multisubunit target such as human TNF-α.

### Example 9.

### Selection of a collection of single domain antibodies (dAbs) directed against human serum albumin (HSA) and mouse serum albumin MSA).

This example explains a method for making a single domain antibody (dAb) directed against serum albumin. Selection of dAbs against both mouse serum albumin (MSA) and human serum albumin (HSA) is described. Three human phage display antibody libraries were used in this experiment, each based on a single human framework for VH (see **Figure 13:** sequence of dummy VH based on V3-23/DP47 and JH4b) or V_{κ} (see **Figure 15:** sequence of dummy V_{κ} based on 012/02/DPK9 and Jkl) with side chain diversity encoded by NNK codons incorporated in complementarity determining regions (CDR1, CDR2 and CDR3).

Library 1 (V_{H}):
Diversity at positions: H30, H31, H33, H35, H50, H52, H52a, H53, H55, H56, H58, H95, H97, H98.
Library size: 6.2 x 10⁹

Library 2 (V_{H}):
Diversity at positions: H30, H31, H33, H35, H50, H52, H52a, H53, H55, H56, H58, H95, H97, H98, H99, H100, H100a, H100b.
Library size: 4.3 x 10⁹

Library 3 (V_{κ}):
Diversity at positions: L30, L31, L32, L34, L50, L53, L91, L92, L93, L94, L96
Library size: 2 x 10⁹

The V_{H} and V_{κ} libraries have been preselected for binding to generic ligands protein A and protein L respectively so that the majority of clones in the unselected libraries are functional. The sizes of the libraries shown above correspond to the sizes after preselection. Two rounds of selection were performed on serum albumin using each of the libraries separately. For each selection, antigen was coated on immunotube (nunc) in 4ml of PBS at a concentration of 100 µg/ml. In the first round of selection, each of the three libraries was panned separately against HSA (Sigma) and MSA (Sigma). In the second round of selection, phage from each of the six first round selections was panned against (i) the same antigen again (eg 1st round MSA, 2nd round MSA) and (ii) against the reciprocal antigen (eg 1 st round MSA, 2nd round HSA) resulting in a total of twelve 2nd round selections. In each case, after the second round of selection 48 clones were tested for binding to HSA and MSA. Soluble dAb fragments were produced as described for scFv fragments by Harrison et al., Methods Enzymol. 1996;267:83-109 and standard ELISA protocol was followed (Hoogenboom et al. (1991) Nucleic Acids Res., 19: 4133) except that 2% tween PBS was used as a blocking buffer and bound dAbs were detected with either protein L-HRP (Sigma) (for the V_{κ}s) and protein A-HRP (Amersham Pharmacia Biotech) (for the V_{H}s).

dAbs that gave a signal above background indicating binding to MSA, HSA or both were tested in ELISA insoluble form for binding to plastic alone but all were specific for serum albumin. Clones were then sequenced (see table below) revealing that 21 unique dAb sequences had been identified. The minimum similarity (at the amino acid level) between the VK dAb clones selected was 86.25% ((69/80)x100; the result when all the diversified residues are different, eg clones 24 and 34). The minimum similarity between the V_{H} dAb clones selected was 94 % ((127/136)x100).

Next, the serum albumin binding dAbs were tested for their ability to capture biotinylated antigen from solution. ELISA protocol (as above) was followed except that ELISA plate was coated with 1 µg/ml protein L (for the V_{κ} clones) and 1 µg/ml protein A (for the V_{H} clones). Soluble dAb was captured from solution as in the protocol and detection was with biotinylated MSA or HSA and streptavidin HRP. The biotinylated MSA and HSA had been prepared according to the manufacturer's instructions, with the aim of achieving an average of 2 biotins per serum albumin molecule. Twenty four clones were identifed that captured biotinylated MSA from solution in the ELISA. Two of these (clones 2 and 38 below) also captured biotinylated HSA. Next, the dAbs were tested for their ability to bind MSA coated on a CM5 biacore chip. Eight clones were found that bound MSA on the biacore.

| **dAb (all capture biotinylated MSA)** | **H or κ** | **CDR1** | **CDR2** | **CDR3** | **Binds MSA in biacore?** | **Captures biotinylated HSA?** |
|---|---|---|---|---|---|---|
| V_{κ} library 3 template (dummy) | | | | | | |
| | κ | XXXLX; | XASXLQS; | QQXXXXPXT; | | |
| | | SEQ ID | SEQ ID NO: | SEQ ID NO: 18 | | |
| | | NO: 16 | 17 | | | |
| 2,4,7,41, | κ | SSYLN; | RASPLQS; | QQTYSVPPT; | | 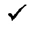 all 4 bind |
| | | SEQ ID | SEQ ID NO: | SEQ ID NO: 21 | | |
| | | NO: 19 | 20 | | | |
| 38,54 | κ | SSYLN; | RASPLQS; | QQTYRIPPT; | | 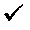 both bind |
| | | SEQ ID | SEQ ID NO: | SEQ ID NO: 24 | | |
| | | NO: 22 | 23 | | | |
| 46,47,52,56 | κ | FKSLK; | NASYLQS; | QQVVYWPVT; | | |
| | | SEQ ID | SEQ ID NO: | SEQ ID NO: 27 | | |
| | | NO: 25 | 26 | | | |
| 13, 15 | κ | YYHLK; | KASTLQS; | QQVRKVPRT; | | |
| | | SEQ ID | SEQ ID NO: | SEQ ID NO: 30 | | |
| | | NO: 28 | 29 | | | |
| 30,35 | κ | RRYLK; | QASVLQS; | QQGLYPPIT; | | |
| | | SEQ ID | SEQ ID NO: | SEQ ID NO: 33 | | |
| | | NO: 31 | 32 | | | |
| 19, | κ | YNWLK | RASSLQS; | QQNVVIPRT; | | |
| | | ; SEQ ID | SEQ ID NO: | SEQ ID NO: 36 | | |
| | | NO: 34 | 35 | | | |
| 22, | κ | LWHLR; | HASLLQS; | QQSAVYPKT; | | |
| | | SEQ ID | SEQ ID NO: | SEQ ID NO: 39 | | |
| | | NO: 37 | 38 | | | |
| 23, | κ | FRYLA; | HASHLQS; | QQRLLYPKT; | | |
| | | SEQ ID | SEQ ID NO: | SEQ ID NO: 42 | | |
| | | NO: 40 | 41 | | | |
| 24, | κ | FYHLA; | PASKLQS; | QQRARWPRT; | | |
| | | SEQ ID | SEQ ID NO: | SEQ ID NO: 45 | | |
| | | NO: 43 | 44 | | | |
| 31, | κ | IWHLN; | RASRLQS; | QQVARVPRT; | | |
| | | SEQ ID | SEQ ID NO: | SEQ ID NO: 48 | | |
| | | NO: 46 | 47 | | | |
| 33, | κ | YRYLR; | KASSLQS | QQYVGYPRT | | |
| | | SEQ ID | SEQ ID NO: | SEQ ID NO: 51 | | |
| | | NO: 49 | 50 | | | |
| 34, | κ | LKYLK; | NASHLQS; | QQTTYYPIT; | | |
| | | SEQ ID | SEQ ID NO: | SEQ ID NO: 54 | | |
| | | NO: 52 | 53 | | | |
| 53, | κ | LRYLR; | KASWLQS | QQVLYYPQT; | | |
| | | SEQ ID | ; SEQ ID NO: | SEQ ID NO: 57 | | |
| | | NO: 55 | 56 | | | |
| 11, | κ | LRSLK; | AASRLQS; | QQVVYWPAT; | 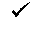 | |
| | | SEQ ID | SEQ ID NO: | SEQ ID NO: 60 | | |
| | | NO: 58 | 59 | | | |
| 12, | κ | FRHLK; | AASRLQS; | QQVALYPKT; | 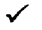 | |
| | | SEQ ID | SEQ ID NO: | SEQ ID NO: 63 | | |
| | | NO: 61 | 62 | | | |
| 17, | κ | RKYLR; | TASSLQS; | QQNLFWPRT; | 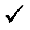 | |
| | | SEQ ID | SEQ ID NO: | SEQ ID NO: 66 | | |
| | | NO: 64 | 65 | | | |
| 18, | κ | RRYLN; | AASSLQS; | QQMLFYPKT; | 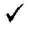 | |
| | | SEQ ID | SEQ ID NO: | SEQ ID NO: 69 | | |
| | | NO: 67 | 68 | | | |
| 16, 21 | κ | IKHLK; | GASRLQS; | QQGARWPQT; | 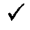 | |
| | | SEQ ID | SEQ ID NO: | SEQ ID NO: 72 | | |
| | | NO: 70 | 71 | | | |
| 25, 26 | κ | YYHLK; | KASTLQS; | QQVRKVPRT; | 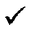 | |
| | | SEQ ID | SEQ ID NO: | SEQ ID NO: 75 | | |
| | | NO: 73 | 74 | | | |
| 27, | κ | YKHLK; | NASHLQS; | QQVGRYPKT; | 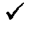 | |
| | | SEQ ID | SEQ ID NO: | SEQ ID NO: 78 | | |
| | | NO: 76 | 77 | | | |
| 55, | κ | FKSLX; | NASYLQS; | QQVVYWPVT; | 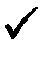 | |
| | | SEQ ID | SEQ ID NO: | SEQ ID NO: 81 | | |
| | | NO: 79 | 80 | | | |
| V_{H} library 1 (and 2) template (dummy) | | | | | | |
| | H | XXYXXX; | XIXXXGXXTXYADSVKG; | | XXXX(XXXX)FDY; | |
| | | SEQ ID NO: | SEQ ID NO: 83 | | SEQ ID NO: 84 | |
| | | 82 | | | | |
| 8, 10 | H | WVYQMD; | SISAFGAKTLYADSVKG; | | LSGKFDY; SEQ ID NO: | |
| | | SEQ ID NO: | SEQ ID NO: 86 | | 87 | |
| | | 85 | | | | |
| 36, | H | WSYQMT; | SISSFGSSTLYADSVKG; | | GRDHNYSLFDY; SEQ | |
| | | SEQ ID NO: | SEQ ID NO: 89 | | ID NO: 90 | |
| | | 88 | | | | |

In all cases the frameworks were identical to the frameworks in the corresponding dummy sequence, with diversity in the CDRs as indicated in the table above.

Of the eight clones that bound MSA on the BIAcore, two clones that are highly expressed in E. coli (clones MSA16 and MSA26) were chosen for further study (see Example 10).

Full nucleotide and amino acid sequences for MSA16 and 26 are given in **Figure 16**.

### Example 10.

### Determination of affinity and serum half-life in mouse of MSA binding dAbs MSA16 and MSA26.

dabs MSA16 and MSA26 were expressed in the periplasm of E. coli and purified using batch absorption to protein L-agarose affinity resin (Affitech, Norway) followed by elution with glycine at pH 2.2. The purified dAbs were then analysed by inhibition biacore to determine K_{d}. Briefly, purified MSA16 and MSA26 were tested to determine the concentration of dAb required to achieve 200RUs of response on a biacore CM5 chip coated with a high density of MSA. Once the required concentrations of dAb had been determined, MSA antigen at a range of concentrations around the expected K_{d} was premixed with the dAb and incubated overnight. Binding to the MSA coated biacore chip of dAb in each of the premixes was then measured at a high flow-rate of 30 µl/minute. The resulting curves were used to create Klotz plots, which gave an estimated K_{d} of 200nM for MSA16 and 70nM for MSA 26 (**Figure 17 A & B**).

Next, clones MSA16 and MSA26 were cloned into an expression vector with the HA tag (nucleic acid sequence: TATCCTTATGATGTTCCTGATTATGCA (SEQ ID NO: 91) and amino acid sequence: YPYDVPDYA (SEQ ID NO: 92) and 2-10 mg quantities were expressed in E. coli and purified from the supernatant with protein L-agarose affinity resin (Affitech, Norway) and eluted with glycine at pH2.2. Serum half life of the dAbs was determined in mouse. MSA26 30 and MSA16 were dosed as single i.v. injections at approx 1.5 mg/kg into CD1 mice.

Analysis of serum levels was by goat anti-HA (Abcam, UK) capture and protein L-HRP (Invitrogen) detection ELISA which was blocked with 4% Marvel. Washing was with 0.05% tween PBS. Standard curves of known concentrations of dAb were set up in the presence of 1x mouse serum to ensure comparability with the test samples. Modelling with a 2 compartment model showed MSA-26 had a t1/2α of 0.16hr, a t1/2β of 14.5hr and an area under the curve (AUC) of 465 hr.mg/ml (data not shown) and MSA-16 had a t1/2α of 0.98hr, a t1/2β of 36.5hr and an AUC of 913 hr.mg/ml (**Figure 18)**. Both anti-MSA clones had considerably lengthened half life compared with HEL4 (an anti-hen egg white lysozyme dAb) which had a t1/2α of 0.06hr, and a t1/2β of 0.34hr.

### Example 11.

### Creation of V_{H}-V_{H} and V_{κ}-V_{κ} dual specific Fab like fragments.

This example describes a method for making V_{H} and V_{κ} dual specifics as Fab like fragments. Before constructing each of the Fab like fragments described, dAbs that bind to targets of choice were first selected from dAb libraries similar to those described in Example 9. A V_{H} dAb, HEL4, that binds to hen egg lysozyme (Sigma) was isolated and a second V_{H} dAb (TAR2h-5) that binds to TNF-α receptor (R and D Systems) was also isolated. The sequences of these are given in the sequence listing. A V_{κ} dAb that binds TNF-α (TARI-5-19) was isolated by selection and affinity maturation and the sequence is also set forth in the sequence listing. A second V_{κ} dAb (MSA 26) described in Example 9 whose sequence is in Figure 17B was also used in these experiments.

DNA from expression vectors containing the four dAbs described above was digested with enzymes SalI and NotI to excise the DNA coding for the dAb. A band of the expected size (300-400bp) was purified by running the digest on an agarose gel and excising the band, followed by gel purification using the Qiagen gel purification kit (Qiagen, UK). The DNA coding for the dAbs was then inserted into either the CH or CK vectors (**Figures 8** and **9**) as indicated in the table below.

| dAb | Target antigen | dAb V_{H} or dAb V_{κ} | Inserted into vector | tag (C terminal) | Antibiotic resistance |
|---|---|---|---|---|---|
| HEL4 | Hen egg lysozyme | V_{H} | C_{H} | myc | Chloramphenicol |
| TAR2-5 | TNF receptor | V_{H} | C_{κ} | flag | Ampicillin |
| TAR1-5-19 | TNF α | V_{κ} | C_{H} | myc | Chloramphenicol |
| MSA 26 | Mouse serum albumin | V_{κ} | C_{κ} | flag | Ampicillin |

The VH CH and VH C_{κ} constructs were cotransfonned into HB2151 cells. Separately, the V_{κ} CH and V_{κ} C_{κ} constructs were cotransformed into HB2151 cells. Cultures of each of the cotransformed cell lines were grown overnight (in 2x TY containing 5% glucose, 10 µg/ml chloramphenicol and 100 µg/ml ampicillin to maintain antibiotic selection for both CH and C_{κ} plasmids). The overnight cultures were used to inoculate fresh media (2x TY, 10 µg/ml chloramphenicol and 100 µg/ml ampicillin) and grown to OD 0.7-0.9 before induction by the addition of IPTG to express their CH and Cκ constructs.

Expressed Fab like fragment was then purified from the periplasm by protein A purification (for the contransformed VH CH and VH C_{κ}) and MSA affinity resin purification (for the contransformed V_{κ} CH and V_{κ} C_{κ}).

### V_{H}-V_{H} dual specific

Expression of the VH CH and VH C_{κ} dual specific was tested by running the protein on a gel. The gel was blotted and a band the expected size for the Fab fragment could be detected on the Western blot via both the myc tag and the flag tag, indicating that both the VH CH and VH C_{κ} parts of the Fab like fragment were present. Next, in order to determine whether the two halves of the dual specific were present in the same Fab-like fragment, an ELISA plate was coated overnight at 4°C with 100 µl per well of hen egg lysozyme (HEL) at 3 mg/ml in sodium bicarbonate buffer. The plate was then blocked (as described in Example 1) with 2% tween PBS followed by incubation with the VH CH NH C_{κ} dual specific Fab like fragment. Detection of binding of the dual specific to the HEL was via the non cognate chain using 9e10 (a monoclonal antibody that binds the myc tag, Roche) and anti mouse IgG-HRP (Amersham Pharmacia Biotech). The signal for the VH CH NH C_{κ} dual specific Fab like fragment was 0.154 compared to a background signal of 0.069 for the VH C_{κ} chain expressed alone. This demonstrates that the Fab like fragment has binding specificity for target antigen.

### V_{κ}-V_{κ} dual specific

After purifying the contransformed V_{κ} CH and V_{κ} C_{κ} dual specific Fab like fragment on an MSA affinity resin, the resulting protein was used to probe an ELISA plate coated with 1 µg/ml TNF-α and an ELISA plate coated with 10 µg/ml MSA. As predicted, there was signal above background when detected with protein L-HRP on both ELISA plates (data not shown). This indicated that the fraction of protein able to bind to MSA (and therefore purified on the MSA affinity column) was also able to bind TNF-α in a subsequent ELISA, confirming the dual specificity of the antibody fragment. This fraction of protein was then used for two subsequent experiments. Firstly, an ELISA plate coated with 1 µg/ml TNF-α was probed with dual specific V_{κ} CH and V_{κ} C_{κ} Fab like fragment and also with a control TNF-α binding dAb at a concentration calculated to give a similar signal on the ELISA. Both the dual specific and control dAb were used to probe the ELISA plate in the presence and in the absence of 2 mg/ml MSA. The signal in the dual specific well was reduced by more than 50% but the signal in the dAb well was not reduced at all (see **Figure 19a**). The same protein was also put into the receptor assay with and without MSA and competition by MSA was also shown (see **Figure 19c).** This demonstrates that binding of MSA to the dual specific is competitive with binding to TNF-α.

### Example 12.

### Creation of a V_{κ}-V_{κ} dual specific cys bonded dual specific with specificity for mouse serum albumin and TNFa

This example describes a method for making a dual specific antibody fragment specific for both mouse serum albumin and TNF-α by chemical coupling via a disulphide bond. Both MSA16 (from Example 1) and TAR1-5-19 dAbs were recloned into a pET based vector with a C terminal cysteine and no tags. The two dAbs were expressed at 4-10 mg levels and purified from the supernatant using protein L-agarose affinity resin (Affitiech, Norway). The cysteine tagged dAbs were then reduced with dithiothreitol. The TAR1-5-19 dAb was then coupled with dithiodipyridine to block reformation of disulphide bonds resulting in the formation of PEP 1-5-19 homodimers. The two different dAbs were then mixed at pH 6.5 to promote disulphide bond formation and the generation of TAR1-5-19, MSA16 cys bonded heterodimers. This method for producing conjugates of two unlike proteins was originally described by King et al. (King TP, Li Kochoumian L Biochemistry. 1978 vol 17: 1499-506 Preparation of protein conjugates via intermolecular disulfide bond formation.) Heterodimers were separated from monomeric species by cation exchange. Separation was confirmed by the presence of a band of the expected size on a SDS gel. The resulting heterodimeric species was tested in the TNF receptor assay and found to have an IC50 for neutralising TNF of approximately 18 nM. Next, the receptor assay was repeated with a constant concentration of heterodimer (18 nM) and a dilution series of MSA and HSA. The presence of HSA at a range of concentrations (up to 2 mg/ml) did not cause a reduction in the ability of the dimer to inhibit TNF-α.

However, the addition of MSA caused a dose dependent reduction in the ability of the dimer to inhibit TNF-α: **(Figure 20).**This demonstrates that MSA and TNF-α compete for binding to the cys bonded TARI--19, MSA 16 dimer.

### Data Summary

A summary of data obtained in the experiments set forth in the foregoing examples is set forth in **Annex 4**.

### Example 13: Summary of Nucleic Acid and Polypeptide Sequences for anti-TNF-α dAbs.

Throughout the course of studies regarding the anti-TNF-α dAbs described herein, a number of different dAbs have been identified that bind human and/or mouse TNF-α. Sequences and further information are provided herein below.

### Clones that bind Mouse TNF-α:

The nucleotide and amino acid sequences for four anti-mouse TNF-α dAbs are provided below. Two of these (TAR1-2m-9 and TAR1-2m-30) inhibit the activity of mouse TNF-α, and two bind but do not inhibit (TAR1-2m-1 and TAR1-2m-2).

### TAR1-2m-9:

TAR-2m-9 is a Vk clone, with an IC50 of 6 µM and an ND50 of 5 µM. The IC50 and ND50 are not improved upon Protein L cross-linking. This clone has no effect against human TNF-α (species cross-reactivity has been assessed in cell assays at two concentrations), but has similar neutralizing activity against rat TNF-α.

Amino acid sequence (CDR3 is in BOLD) (SEQ ID NO: 93):
DIQMTQSPSSLSASVGDRVTITCRASQPIGSFLWWYQQKPGKAPKLLIYYSSYLQSGVP
SRFSGSGSGTDFTLTISSLQPEDFATYYCQQYRWHPNTFGQGTKVEIKR
Nucleotide sequence (SEQ ID NO: 94):
1
gacatccagatgacccagtctccatcctccctgtctgcatctgtaggagaccgtgtcacc
61

atcacttgccgggcaagtcagcctattgggagtttttatggtggtaccagcagaaacca
121
gggaaagcccctaaactcctgatctattatagttcctatttgcaaagtggggtcccatca
181
cgtttcagtggcagtggatctgggacagatttcactctcaccatcagcagtctgcaacct
241
gaagattttgctacgtactactgtcaacagtatcgttggcatcctaataccttcggccaa
301 gggaccaaggtggaaatcaaacgg

### TAR1-2m-30:

TAR1-2m-30 is a Vk clone, with an ND50 of 10 µM. ND50 is not improved upon Protein L cross-linking. This clone has no effect against human TNF-α (species cross-reactivity has been assessed in cell assays at two concentrations), and is slightly less effective against rat TNF when compared to mouse.

Amino acid sequence (CDR3 is in BOLD) (SEQ ID NO: 95):
DIQMTQSPSSLSASVGDRVTITCRASQSIYSWLNWYQQKPGKAPKLLIYRASHLQSGV
PSRFSGSGSGTDFTLTISSLQPEDFATYYCQQIWNMPFTFGQGTKVEIKR
Nucleotide sequence (SEQ ID NO: 96):
1
gacatccagatgacccagtctccatcctccctgtctgcatctgtaggagaccgtgtcacc
61
atcacttgccgggcaagtcagtcgatttatagttggttaaattggtaccagcagaaacca
121
gggaaagcccctaagctcctgatctatagggcgtcccatttgcaaagtggggtcccatca
181
cgtttcagtggcagtggatctgggacagatttcactctcaccatcagcagtctgcaacct
241
gaagattttgctacgtactactgtcaacagatttggaatatgccttttacgttcggccaa
301 gggaccaaggtggaaatcaaacgg

### TAR1-2m-1:

This clone binds mouse TNF-α but does not inhibit receptor binding activity.

Amino acid sequence (SEQ ID NO: 97):
DIQMTQSPSSLSASVGDRVTITCRASQPIGYDLFWYQQKPGKAPKLLIYRGSVLQSGVP
SRFSGSGSGTDFTLTISSLQPEDFATYYCQQRWRWPFTFGQGTKVEIKR
Nucleotide sequence (SEQ ID NO: 98):
1
gacatccagatgacccagtctccatcctccctgtctgcatctgtaggagaccgtgtcacc
61
atcacttgccgggcaagtcagcctattggttatgatttattttggtaccagcagaaacca

121
gggaaagcccctaagctcctgatctatcggggttccgtgttgcaaagtggggtcccatca
181
cgtttcagtggcagtggatctgggacagatttcactctcaccatcagcagtctgcaacct
241
gaagattttgctacgtactactgtcaacagcggtggcgttggccttttacgttcggccaa
301 ggcaccaaggtggaaatcaaacgg

### TAR1-2m-2:

This clone binds mouse TNF-α but does not inhibit receptor binding activity.

Amino acid sequence (SEQ ID NO: 99):
DIQMTQSPSSLSASVGDRVTITCRASLPIGRDLWWYQQKPGKAPKLLIYRGSFLQSGVP
SRFSGSGSGTDFTLTISSLQPEDFATYYCQQRWYYPHTFGQGTKVEIKR
Nucleotide sequence (SEQ ID NO: 100):
1
gacatccagatgacccagtctccatcctccctgtctgcatctgtaggagaccgtgtcacc
61
atcacttgccgggcaagtctgcctattggtcgtgatttatggtggtatcagcagaaacca
121
gggaaagcccctaagctcctgatctatcggggtcctttttgcaaagtggggtcccatca
181
cgtttcagtggcagtggatctgggacagatttcactctcaccatcagcagtctgcaacct
241
gaagattttgctacgtactactgtcaacagaggtggtattatcctcatacgttcggccaa
301 gggaccaaggtggaaatcaaacgg

### dAb clones that bind human TNF-α

The following is a listing of the nucleotide sequences of dAbs identified for binding human TNF-α. Corresponding amino acid sequences are provided in **Figure 23**.

TAR1-5 (SEQ ID NO: 101)
GACATCCAGATGACCCAGTCTCCATCCTCCCTGTCTGCATCTGTAGGAGA
CCGTGTCACCATCACTTGCCGGGCAAGTCAGAGCATTTTTATGAATTTAT
TGTGGTACCAGCAGAAACCAGGGAAAGCCCCTAAGCTCCTGATCTATAAT
GCATCCGTGTTGCAAAGTGGGGTCCCATCACGTTTCAGTGGCAGTGGATC
TGGGACAGATTTCACTCTCACCATCAGCAGTCTGCAACCTGAAGATTTTG
CTACGTACTACTGTCAACAGGTTGTGTGGCGTCCTTTTACGTTCGGCCAA
GGGACCAAGGTGGAAATCAAACGC

TAR1-27 (SEQ ID NO: 102)
GACATCCAGATGACCCAGTCTCCATCCTCCCTGTCTGCATCTGTAGGAGA
CCGTGTCACCATCACTTGCCGGGCAAGTCAGAGCATTTGGACGAAGTTAC
ATTGGTACCAGCAGAAACCAGGGAAAGCCCCTAAGCTCCTGATCTATATG
GCATCCAGTTTGCAAAGTGGGGTCCCATCACGTTTCAGTGGCAGTGGATC
TGGGACAGATTTCACTCTCACCATCAGCAGTCTGCAACCTGAAGATTTTG
CTACGTACTACTGTCAACAGTGGTTTAGTAATCCTAGTACGTTCGGCCAA
GGGACCAAGGTGGAAATCAAACG

TAR1-261 (SEQ ID NO: 103)
GACATCCAGATGACCCAGTCTCCATCCTCCCTGTCTGCATCTGTAGGAGA
CCGTGTCACCATCACTTGCCGGGCAAGTCAGAGCATTGAGCATTATTTAT
GGTGGTACCAGCAGAAACCAGGGAAAGCCCCTAAGCTCCTGATCTATGCT
GCATCCTATTTGCAAAGTGGGGTCCCATCACGTTTCAGTGGCAGTGGATC
TGGGACAGATTTCACTCTCACCATCAGCAGTCTGCAACCTGAAGATTTTG
CTACGTACTACTGTCAACAGAGTTTGGCGTGTCCTCCTACGTTCGGCCAA
GGGACCAAGGTGGAAATCAAACGG

TAR1-398 (SEQ ID NO: 104)
GACATCCAGATGACCCAGTCTCCATCCTCCCTGTCTGCATCTGTAGGAGA
CCGTGTCACCATCACTTGCCGGGCAAGTCAGAGCATTTATGGTCATTTAT
TGTGGTACCAGCAGAAACCAGGGAAAGCCCCTAAGCTCCTGATCTATGCT
GCATCCAGTTTGCAAAGTGGGGTCCCATCACGTTTCAGTGGCAGTGGATC
TGGGACAGATTTCACTCTCACCATCAGCAGTCTGCAACCTGAAGATTTTG
CTACGTACTACTGTCAACAGCCTTTGGTGCGGCCTTTTACGTTCGGCCAA
GGGACCAAGGTGGAAATCAAACGG

TAR1-701 (SEQ ID NO: 105)
GACATCCAGATGACCCAGTCTCCATCCTCCCTGTCTGCATCTGTAGGAGA
CCGTGTCACCATCACTTGCCGGGCAAGTCAGAGCATTGCTAAGTTGTTAT
ATTGGTACCAGCAGAAACCAGGGAAAGCCCCTAAGCTCCTGATCTATGAT
GCATCCTCTTTGCAAAGTGGGGTCCCATCACGTTTCAGTGGCAGTGGATC
TGGGACAGATTTCACTCTCACCATCAGCAGTCTGCAACCTGAAGATTTTG
CTACGTACTACTGTCAACAGTGGTGGGGTATCCTGGTACGTTCGGCCAA
GGGACCAAGGTGGAAATCAAACGG

TAR1-5-2 (SEQ ID NO: 106)
GACATCCAGATGACCCAGTCTCCATCCTCCCTGTCTGCATCTGTAGGAGA
CCGTGTCACCATCACTTGCCGGGCAAGTCAGAGCATTTTTCCTGCTTTAC
TTTGGTACCAGCAGAAACCAGGGAAAGCCCCTAAGCTCCTGATCTATCAT
GCATCCAGTTTGCAAAGTGGGGTCCCATCACGTTTCAGTGGCAGTGGATC
TGGGACAGATTTCACTCTCACCATCAGCAGTCTGCAACCTGAAGATATTG
CTACGTACTACTGTCAACAGGTTGTGTGGCGTCCTTTTACGTTCGGCCAA
GGGACCAAGGTGGAAATCAAACGG

TAR1-5-3 (SEQ ID NO: 107)
GACATCCAGATGACCCAGTCTCCATCCTCCCTGTCTGCATCTGTAGGAGA
CCGTGTCACCATCACTTGCCGGGCAAGTCAGAGCATTGATAATGCGTTAC
ATTGGTACCAGCAGAAACCAGGGAAAGCCCCTAAGCTCCTGATCTATCAG
GCATCCATTTTGCAAAGTGGGGTCCCATCACGTTTCAGTGGCAGTGGATC
TGGGACAGATTTCACTCTCACCATCAGCAGTCTGCAACCTGAAGATTTTG
CTACGTACTACTGTCAACAGGTTGTGTGGCGTCCTTTTACGTTCGGCCAA
GGGACCAAGGTGGAAATCAAACGG

TAR1-5-4 (SEQ ID NO: 108)
GACATCCAGATGACCCAGTCTCCATCCTCCCTGTCTGCATCTGTAGGAGA
CCGTGTCACCATCACTTGCCGGGCAAGTCAGAGCATTTTTATGAATTTAT
TGTGGTACCAGCAGAAACCAGGGAAAGCCCCTAAGCTCCTGATCTATAAT
GCATCCGTGTTGCAAAGTGGGGTCCCATCACGTTTCAGTGGCAGTGGATC
TGGGACAGGTTTCACTCTCACCATCAGCAGTCTGCAACCTGAAGATTTTG
CTACGTACTACTGTCAACAGGTTGTGTGGCGTCCTTTTACGTTCGGCCAA
GGGACCAAGGTGGAAATCAAACGG

TAR1-5-7 (SEQ ID NO: 109)
GACATCCAGATGACCCAGTCTCCATCCTCCCTGTCTGCATCTGTAGGAGA
CCGTGTCACCATCACTTGCCGGGCAAGTCAGAGCATTTTGAATTCTTTAC

ATTGGTACCAGCAGAAACCAGGGAAAGCCCCTAAGCTCCTGATCTATCAT
GCATCCACTTTGCAAAGTGGGGTCCCATCACGTTTCAGTGGCAGTGGATC
TGGGACAGATTTCACTCTCACCATCAGCAGTCTGCAACCTGAAGATTTTG
CTACGTACTACTGTCAACAGGTTGTGTGGCGTCCTTTTACGTTCGGCCAA
GGGACCAAGGTGGAAATCAAACGG

TAR1-5-8 (SEQ ID NO: 110)
GACATCCAGATGACCCAGTCTCCATCCTCCCTGTCTGCATCTGTAGGAGA
CCGTGTCACCATCACTTGCCGGGCAAGTCAGAGCATTTTGAATTCTTTAC
ATTGGTACCAGCAGAAACCAGGGAAAGCCCCTAAGCTCCTGATCTATCAT
GCATCCACTTTGCAAAGTGGGGTCCCATCACGTTTCAGTGGCAGTGGATC
TGGGACAGATTTCACTCTCACCATCAGCAGTCTGCAACCTGAAGATTTTG
CTACGTACTACTGTCAACAGGTTGTGTGGCGTCCTTTTACGTTCGGCCAA
GGGACCAAGGTGGAAATCAAACGG

TAR1-5-10 (SEQ ID NO: 111)
GACATCCAGATGACCCAGTCTCCATCCTCCCTGTCTGCATCTGTAGGAGA
CCGTGTCACCATCACTTGCCGGGCAAGTCAGAGCATTGATAATTATTTAC
ATTGGTACCAGCAGAAACCAGGGAAAGCCCCTAAGCTCCTGATCTATTCT
GCATCCCATTTGCAAAGTGGGGTCCCATCACGTTTCAGTGGCAGTGGATC
TGGGACAGATTTCACTCTCACCATCAGCAGTCTGCAACCTGAAGATTTTG
CTACGTACTACTGTCAACAGGTTGTGTGGCGTCCTTTTACGTTCGGCCAA
GGGACCAAGGTGGAAATCAAACGG

TAR1-5-11 (SEQ ID NO: 112)
GACATCCAGATGACCCAGTCTCCATCCTCCCTGTCTGCATCTGTAGGAGA
CCGTGTCACCATCACTTGCCGGGCAAGTCAGAGCATTAATGAGTATTTAC
ATTGGTACCAGCAGAAACCAGGGAAAGCCCCTAAGCTCCTGATCTATTCT
GCATCCGTGTTGCAAAGTGGGGTCCCATCACGTTTCAGTGGCAGTGGATC
TGGGACAGATTTCACTCTCACCATCAGCAGTCTGCAACCTGAAGATTTTG
CTACGTACTACTGTCAACAGGTTGTGTGGCGTCCTTTTACGTTCGGCCAA
GGGACCAAGGTGGAAATCAAACGG

TAR1-5-12 (SEQ ID NO: 113)
GACATCCAGATGACCCAGTCTCCATCCTCCCTGTCTGCATCTGTAGGAGA
CCGTGTCACCATCACTTGCCGGGCAAGTCAGAGCATTAATTATGCTTTAC
ATTGGTACCAGCAGAAACCAGGGAAAGCCCCTAAGCTCCTGATCTATCAG
GCATCCATTTTGCAAAGTGGGGTCCCATCACGTTTCAGTGGCAGTGGATC
TGGGACAGATTTCACTCTCACCATCAGCAGTCTGCAACCTGAAGATTTTG
CTACGTACTACTGTCAACAGGTTGTGTGGCGTCCTTTTACGTTCGGCCAA
GGGACCAAGGTGGAAATCAAACGG

TAR1-5-13 (SEQ ID NO: 114)
GACATCCAGATGACCCAGTCTCCATCCTCCCTGTCTGCATCTGTAGGAGA
CCGTGTCACCATCACTTGCCGGGCAAGTCAGAGCATTGATAGTTTTTTAC
ATTGGTACCAGCAGAAACCAGGGAAAGCCCCTAAGCTCCTGATCTATAGT
GCATCCGAGTTGCAAAGTGGGGTCCCATCACGTTTCAGTGGCAGTGGATC
TGGGACAGATTTCACTCTCACCATCAGCAGTCTGCATCCTGAAGATTTTG
CTACGTACTACTGTCAACAGGTTGTGTGGCGTCCTTTTACGTTCGGCCAA
GGGACCAAGGTGGAAATCAAACGG

TAR1-5-19 (SEQ ID NO: 115)
GACATCCAGATGACCCAGTCTCCATCCTCTCTGTCTGCATCTGTAGGAGA
CCGTGTCACCATCACTTGCCGGGCAAGTCAGAGCATTGATAGTTATTTAC
ATTGGTACCAGCAGAAACCAGGGAAAGCCCCTAAGCTCCTGATCTATAGT
GCATCCGAGTTGCAAAGTGGGGTCCCATCACGTTTCAGTGGCAGTGGATC
TGGGACAGATTTCACTCTCACCATCAGCAGTCTGCAACCTGAAGATTTTG
CTACGTACTACTGTCAACAGGTTGTGTGGCGTCCTTTTACGTTCGGCCAA

GGGACCAAGGTGGAAATCAAACGG

TAR1-5-20 (SEQ ID NO: 116)
GACATCCAGATGACCCAGTCTCCATCCTCCCTGTCTGCATCTGTAGGAGA
CCGTGTCACCATCACTTGCCGGGCAAGTCAGAGCATTGATCAGTATTTAC
ATTGGTACCAGCAGAAACCAGGGAAAGCCCCTAAGCTCCTGATCTATGGT
GCATCCAATTTGCAAAGTGAGGTCCCATCACGTTTCAGTGGCAGTGGATC
TGGGACAGATTTCACTCTCACCATCAGCAGTCTGCAACCTGAAGATTTTG
CTACGTACTACTGTCAACAGGTTGTGTGGCGTCCTTTTACGTTCGGCCAA
GGGACCAAGGTGGAAATCAAACGG

TAR1-5-21 (SEQ ID NO: 117)
GACATCCAGATGACCCAGTCTCCATCCTCCCTGTCTGCATCTGTAGGAGA
CCGTGTCACCATCACTTGCCGGGCAAGTCAGAGCATTGATAGTTTTTTAC
ATTGGTACCAGCAGAAACCAGGGAAAGCCCCTAAGCTCCTGATCTATAGT
GCATCCGAGTTGCAAAGTGGGGTCCCATCACGTTTCAGTGGCAGTGGATC
TGGGACAGATTTCACTCTCACCATCAGCAGTCTGCATCCTGAAGATTTTG
CTACGTACTACTGTCAACAGGTTGTGTGGCGTCCTTTTACGTTCGGCCAA
GGGACCAAGGTGGAAATCAAACGG

TAR1-5-22 (SEQ ID NO: 118)
GACATCCAGATGACCCAGTCTCCATCCTCCCTGTCTGCATCTGTAGGAGA
CCGTGTCACCATCACTTGCCGGGCAAGTCAGAGCATTGATTCTTATTTAC
ATTGGTACCAGCAGAAACCAGGGAAAGCCCCTAAGCTCCTGATCTATAGT
GCATCCCTGTTGCAAAGTGGGGTCCCATCACGTTTCAGTGGCAGTGGATC
TGGGACAGATTTCACTCTCACCATCAGCAGTCTGCAACCTGAAGATTTTG
CTACGTACTACTGTCAACAGGTTGTGTGGCGTCCTTTTACGTTCGGCCAA
GGGACCAAGGTGGAAATCAAACGG

TAR1-5-23 (SEQ ID NO: 119)
GACATCCAGATGACCCAGTCTCCATCCTCCCTGTCTGCATCTGTAGGAGA
CCGTGTCACCATCACTTGCCGGGCAAGTCAGAGCATTGATCAGTATTTAC
ATTGGTACCAGCAGAAACCAGGGAAAGCCCCTAAGCTCCTGATCTATTCT
GCATCCCTTTTGCAAAGTGGGGTCCCATCACGTTTCAGTGGCAGTGGATC
TGGGACAGATTTCACTCTCACCATCAGCAGTCTGCAACCTGAAGATTTTG
CTACATACTACTGTCAACAGGTTGTGTGGCGTCCTTTTACGTTCGGCCAA
GGGACCAAGGTGGAAATCAAACGG

TAR1-5-24 (SEQ ID NO: 120)
GACATCCAGATGACCCAGTCTCCATCCTCCCTGTCTGCATCTGTAGGAGA
CCGTGTCACCATCACTTGCCGGGCAAGTCAAAGCATTGATGAGTTTTTAC
ATTGGTACCAGCAGAAACCAGGGAAAGCCCCTAAGCTCCTGATCTATTGT
GCATCCCAGTTGCAAAGTGGGGTCCCATCACGTTTCAGTGGCAGTGGATC
TGGGACAGATTTCACTCTCACCATCAGCAGTCTACATCCTGAAGATTTTG
CTACGTACTACTGTCAACAGGTTGTGTGGCGTCCTTTTACGTTCGGCCAA
GGGACCAAGGTGGAAATCAAACGG

TAR1-5-25 (SEQ ID NO: 121)
GACATCCAGATGACCCAGTCTCCATCCTCCCTGTCTGCATCTGTAGGAGA
CCGTGTCACCATCACTTGCCGGGCAAGTCAGAGCATTGATGCGTATTTAC
ATTGGTACCAGCAGAAACCAGGGAAAGCCCCTAAGCTCCTGATCTATTCT
GCATCCCTGTTGCAAAGTGGGGTCCCATCACGTTTCAGTGGCAGTGGATC
TGGGACAGATTTCACTCTCACCATCAGCAGTCTGCAACCTGAAGATTTTG
CTACGTACTACTGTCAACAGGTTGTGTGGCGTCCTTTTACGTTCGGCCAA
GGGACCAAGGTGGAAATCAAACGG

TAR1-5-26 (SEQ ID NO: 122)
GACATCCAGATGACCCAGTCTCCATCCTCCCTGTCTGCATCTGTAGGAGA

CCGTGTCACCATCACTTGCCGGGCAAGTCAGAGCATTGATAGGTATTTAC
ATTGGTACCAGCAGAAACCAGGGAAAGCCCCTAAGCTCCTGATCTATAGT
GCATCCGTGTTGCAAAGTGGGGTCCCATCACGTTTCAGTGGCAGTGGATC
TGGGACAGATTTCACCCTCACCATCAGCAGTCTGCAGCCTGAAGATTTTG
CTACGTACTACTGTCAACAGGTTGTGTGGCGTCCTTTTACGTTCGGCCAA
GGGACCAAGGTGGAAATCAAACGG

TAR1-5-27 (SEQ ID NO: 123)
GACATCCAGATGACCCAGTCTCCATCCTCCCTGTCTGCATCTGTAGGAGA
CCGTGTCACCATCACTTGCCGGGCAAGTCAGAGCATTGATAAGTATTTAC
ATTGGTACCAGCAGAAACCAGGGAAAGCCCCTAAGCTCCTGATCTATAGT
GCATCCTCGTTGCAAAGTGGGGTCCCATCACGTTTCAGTGGCAGTGGATC
TGGGACAGATTTCACTCTCACCATCAGCAGTCTGCAACCTGAAGATTTTG
CTACGTACTACTGTCAACAGGTTGTGTGGCGTCCTTTTACGTTCGGCCAA
GGGACCAAGGTGGAAATCAAACGG

TAR1-5-28 (SEQ ID NO: 124)
GACATCCAGATGACCCAGTCTCCATCCTCCCTGTCTGCATCTGTAGGAGA
CCGTGTCACCATCACTTGCCGGGCAAGTCAGAGCATTGATCATTATTTAC
ATTGGTACCAGCAGAAACCAGGGAAAGCCCCTAAGCTCCTGATCTATAGT
GCATCCGTTTTGCAAAGTGGGGTCCCATCACGTTTCAGTGGCAGTGGATC
TGGGACAGATTTCACTCTCACCATCAGCAGTCTGCAACCTGAAGATTTTG
CAACGTACTACTGTCAACAGGTTGTGTGGCGTCCTTTTACGTTCGGCCAA
GGGACCAAGGTGGAAATCAAACGG

TAR1-5-29 (SEQ ID NO: 125)
GACATCCAGATGACCCAGTCTCCATCCTCCCTGTCTGCATCTGTAGGAGA
CCGTGTCACCATCACTTGCCGGGCAAGTCAGAGCATTGATGAGTTTTTAC
ATTGGTACCAGCAGAAACCAGGGAAAGCCCCTAAGCTCCTGATCTATAGT
GCATCCATTTTGCAAAGTGGGGTCCCATCACGTTTCAGTGGCAGTGGATC
TGGGACAGATTTCACTCTCACCATCAGCAGTCTGCAACCTGAAGATTTTG
CTACGTACTACTGTCAACAGGTTGTGTGGCGTCCTTTTACGTTCGGCCAA
GGGACCAAGGTGGAAATCAAACGG

TAR1-5-34 (SEQ ID NO: 126)
GACATCCAGATGACCCAGTCTCCATCCTCCCTGTCTGCATCTGTAGGAGA
CCGTGTCACCATCACTTGCCGGGCAAGTCAGAGCATTCAGACTGCGTTAC
TGTGGTACCAGCAGAAACCAGGGAAAGCCCCTAAGCTCCTGATCTATAAT
GCATCCAGTTTGCAAAGTGGGGTCCCATCACGTTTCAGTGGCAGTGGATC
TGGGACAGATTTCACTCTCACCATCAGCAGTCTGCAACCTGAAGATTTTG
CTACATACTACTGTCAACAGGTTGTGTGGCGTCCTTTTACGTTCGGCCAA
GGGACCAAGGTGGAAATCAAACGG

TAR1-5-35 (SEQ ID NO: 127)
GACATCCAGATGACCCAGTCTCCATCCTCCCTGTCTGCATCTGTAGGAGA
CCGTGTCACCATCACTTGCCGGGCAAGTCAGAGCATTGATCAGTATTTAC
ATTGGTACCAGCAGAAACCAGGGAAAGCCCCTAAGCTCCTGATCTATGGT
GCATCCAATTTGCAAAGTGGGGTCCCATCACGTTTCAGTGGCAGTGGATC
TGGGACAGATTTCACTCTCACCATCAGCAGTCTGCAACCTGAAGATTTTG
CTACGTACTACTGTCAACAGGTTGTGTGGCGTCCTTTTACGTTCGGCCAA
GGGACCAAGGTGGAAATCAAACGG

TAR1-5-36 (SEQ ID NO: 128)
GACATCCAGATGACCCAGTCTCCATCCTCCCTGTCTGCATCTGTAGGAGA
CCGTGTCACCATCACTTGCCGGGCAAGTCAGAGCATTGATAATTATTTAC
ATTGGTACCAGCAGAAACCAGGGAAAGCCCCTAAGCTCCTGATCTATAGT
GCATCCCAGTTGCAAAGTGGGGTCCCATCACGTTTCAGTGGCAGTGGATC
TGGGACAGATTTCACTCTCACCATCAGCAGTCTGCAACCTGAAGATTTTG

CTACGTACTACTGTCAACAGGTTGTGTGGCGTCCTTTTACGTTCGGCCAA
GGGACCAAGGTGGAAATCAAACGG

TAR1-5-464 (SEQ ID NO: 129)
GACATCCAGATGACCCAGTCTCCATCCTCCCTGTCTGCATCTGTAGGAGA
CCGTGTCACCATCACTTGCCGGGCAAGTCAGAGCATTGATAATTTTTTAC
ATTGGTACCAGCAGAAACCAGGGAAAGCCCCTAAGCTCCTGATCTATAGT
GCATCCGAGTTGCAAAGTGGGGTCCCATCACGTTTCAGTGGCAGTGGATC
TGGGACAGATTTCACTCTCACCATCAGCAGTCTGCAACCTGAAGATTTTG
CTACGTACTACTGTCAACAGGTTGTGTGGCGTCCTTTTACGTTCGGCCAA
GGGACCAAGGTGGAAATCAAACGG

TAR1-5-463 (SEQ ID NO: 130)
GACATCCAGATGACCCAGTCTCCATCCTCCCTGTCTGCATCTGTAGGAGA
CCGTGTCACCATCACTTGCCGGGCAAGTCAGAGCATTGATGAGTATTTAC
ATTGGTACCAGCAGAAACCAGGGAAACCCCCTAAGCTCCTGATCTATTCT
GCATCCAGTTTGCAAAGTGGGGTCCCATCACGTTTCAGTGGCAGTGGATC
TGGGACAGATTTCACTCTCACCATCAGCAGTCTGCAACCTGAAGATTTTG
CTACGTACTACTGTCAACAGGTTGTGTGGCGTCCTTTTACGTTCGGCCAA
GGGACCAAGGTGGAAATCAAACGG

TAR1-5-460 (SEQ ID NO: 131)
GACATCCAGATGACCCAGTCTCCATCCTCCCTGTCTGCATCTGTAGGAGA
CCGTGTCACCATCACTTGCCGGGCAAGTCAGAGCATTGATCATTTTTTAC
ATTGGTACCAGCAGAAACCAGGGAAAGCCCCTAAGCTCCTGATCTATAGT
GCATCCGAGTTGCAAAGTGGGGTCCCATCACGTTTCAGTGGCAGTGGATC
TGGGACAGATTTCACTCTCACCATCAGCAGTCTGCAACCTGAAGATTTTG
CTACGTACTACTGTCAACAGGTTGTGTGGCGTCCTTTTACGTTCGGCCAA
GGGACCAAGGTGGAAATCAAACGG

TAR1-5-461 (SEQ ID NO: 132)
GACATCCAGATGACCCAGTCTCCATCCTCCCTGTCTGCATCTGTAGGAGA
CCGTGTCACCATCACTTGCCGGGCAAGTCAGAGCATTGATAATTATTTAC
ATTGGTACCAGCAGAAACCAGGGAAAGCCCCTAAGCTCCTGATCTATTCG
GCATCCATGTTGCAAAGTGGGGTCCCATCACGTTTCAGTGGCAGTGGATC
TGGGACAGATTTCACTCTCACCATCAGCAGTCTGCAACCTGAAGATTTTG
CTACGTACTACTGTCAACAGGTTGTGTGGCGTCCTTTTACGTTCGGCCAA
GGGACCAAGGTGGAAATCAAACGG

TAR1-5-479 (SEQ ID NO: 133)
GACATCCAGATGACCCAGTCTCCATCCTCCCTGTCTGCATCTGTAGGAGA
CCGTGTCACCATCACTTGCCGGGCAAGTCAGAGCATTGATGAGTATTTAC
ATTGGTACCAGCAGAAACCAGGGAAAGCCCCCAAGCTCCTGATCTATTCT
GCATCCATTTTGCAAAGTGGGGTCCCATCACGTTTCAGTGGCAGTGGATC
TGGGACAGATTTCACTCTCACCATCAGCAGTCTGCAACCTGAAGATTTTG
CTACGTACTACTGTCAACAGGTTGTGTGGCGTCCTTTTACGTTCGGCCAA
GGGACCAAGGTGGAAATCAAACGG

TAR1-5-477 (SEQ ID NO: 134)
GACATCCAGATGACCCAGTCTCCATCCTCCCTGTCTGCATCTGTAGGAGA
CCGTGTCACCATCACTTGCCGGGCAAGTCAGAGCATTGATGAGTTTTTAC
ATTGGTACCAGCAGAAACCAGGGAAAGCCCCTAAGCTCCTGATCTATTCG
GCATCCGCTTTGCAAAGTGGGGTCCCATCACGTTTCAGTGGCAGTGGATC
TGGGACAGATTTCACTCTCACCATCAGCAGTCTGCAACCTGAAGATTTTG
CTACGTACTACTGTCAACAGGTTGTGTGGCGTCCTTTTACGTTCGGCCAA
GGGACCAAGGTGGAAATCAAACGG

TAR1-5-478 (SEQ ID NO: 135)

GACATCCAGATGACCCAGTCTCCATCCTCCCTGTCTGCATCTGTAGGAGA
CCGTGTCACCATCACTTGCCGGGCAAGTCAGAGCATTGATGAGTATTTAC
ATTGGTACCAGCAGAAACCAGGGAAAGCCCCTAAGCTCCTGATCTATTCT
GCATCCATTTTGCAAAGTGGGGTCCCATCACGTTTCAGTGGCAGTGGATC
TGGGACAGATTTCACTCTCACCATCAGCAGTCTGCACCCTGAAGATTTTG
CTACGTACTACTGTCAACAGGTTGTGTGGCGTCCTTTTACGTTCGGCCAA
GGGACCAAGGTGGAAATCAAACGG

TAR1-5-476 (SEQ ID NO: 136)
GACATCCAGATGACCCAGTCTCCATCCTCCCTGTCTGCATCTGTAGGAGA
CCGTGTCACCATCACTTGCCGGGCAAGTCAGAGCATTGATAATTATTTAC
ATTGGTACCAGCAGAAACCAGGGAAAGCCCCTAAGCTCCTGATCTATGCT
GCATCCAGTTTGCAAAGTGGGGTCCCATCACGTTTCAGTGGCAGTGGATC
TGGGACAGATTTCACTCTCACCATCAGCAGTCTGCAACCTGATGATTTTG
CTACGTACTACTGTCAACAGGTTGTGTGGCGTCCTTTTGCGTTCGGCCAA
GGGACCAAGGTGGAAATCAAACGG

TAR1-5-490 (SEQ ID NO: 137)
GACATCCAGATGACCCAGTCTCCATCCTCCCTGTCTGCATCTGTAGGAGA
CCGTGTCACCATCACTTGCCGGGCAAGTCAGAGCATTGATAGTTATTTAC
ATTGGTACCAGCAGAAACCAGGGAAAGCCCCTAAGCTCCTGATCTATAGT
GCATCAAATTTAGAAACAGGGGTCCCATCACGTTTCAGTGGCAGTGGATC
TGGGACAGATTTCACTCTCACCATCAGCAGTCTGCAACCTGAAGATTTTG
CTACGTACTACTGTCAACAGGTTGTGTGGCGTCCTTTTACGTTCGGCCAA
GGGACCAAGGTGGAAATCAAACGG

TAR1h-1 (SEQ ID NO: 138)
GACATCC AGATGACCCAGTCTCCATCCTCCCTGTCTGCATCTGTAGGAGA
CCGTGTCACCATCACTTGCCGGGCAAGTCAGGTGATTTGGGATGCGTTAG
ATTGGTACCAGCAGAAACCAGGGAAAGCCCCTAAGCTCCTGATCTATAGT
GCGTCCCGTTTGCAAAGTGGGGTCCCATCACGTTTCAGTGGCAGTGGATC
TGGGACAGATTTCACTCTCACCATCAGCAGTCTGCATCCTGAAGATTTTG
CTACGTACTACTGTCAACAGTATGCTGTGTTTCCTGTGACGTTCGGCCAA
GGGACCAAGGTGGAAATCAAACGG

TAR1h-2 (SEQ ID NO: 139)
GACATCCAGATGACCCAGTCTCCATCCTCCCTGTCTGCATCTGTAGGAGA
CCGTGTCACCATCACTTGCCGGGCAAGTCAGACTATTTATGATGCGTTAA
GTTGGTACCAGCAGAAACCAGGGAAAGCCCCTAAGCTCCTGATCTATGGT
GGTTCCAGGTTGCAAAGTGGGGTCCCATCACGTTTCAGTGGCGGTGGATC
TGGGACAGATTTCACTCTCACCATCAGCAGTCTGCAACCTGAAGATTTTG
CTACGTACTACTGTCAACAGTATAAGACTAAGCCTTTGACGTTCGGCCAA
GGGACCAAGGTGGAAATCAAACGG

TAR1h-3 (SEQ ID NO: 140)
GACATCCAGATGACCCAGTCCCCATCCTCCCTGTCTGCATCTGTAGGAGA
CCGTGTCACCATCACTTGCCGGGCAAGTCAGACTATTTATGATGCGTTAA
GTTGGTACCAGCAGAAACCAGGGAAAGCCCCTAAGCTCCTGATCTATGGT
GGTTCCAGGTTGCAAAGTGGGGTCCCATCACGTTTCAGTGGTAGTGGATC
TGGGACAGATTTCACTCTCACCATCAGCAGTCTGCAACCCGAAGATTTTG
CTACGTACTACTGTCAACAGTATGCTCGTTATCCTCTTACGTTCGGCCAA
GGGACCAAGGTGGAAATCAAACGG

Additional anti-human TNF-α dAb clones include the following:

Several clones have been subjected to affinity maturation. Clone TAR1-100-47 is an affinity-matured clone with an ND50 of 30-50 nM in the L929 cell assay, and 3-5 nM when cross-linked with protein L. TAR 1-100-47 cross-reacts with rhesus TNF. Its amino acid sequence and those of a number of other clones are as provided bleow. TAR1-2-100 and TAR1-2-109 are parent clones used for construction of the library. The good TAR1 clones in this group have the following consensus sequence:

D/E30, W32, R94 and F96, as indicated in bold in TAR1-100-47

TAR1-100-29 (SEQ ID NO: 141),
DIQMTQSPSSLSASVGDRVTITCRASQDIEEWLMWYQQKPGKAPKLLIYNSSTLQ
SGVPSRFSGSGSGTDFTLTISSLQPEDYATYYCQQPLSRPFTFGQGTKVEIKR

TAR1-100-35 (SEQ ID NO: 142)
DIQMTQSPSSLSASVGDRVTITCRASQHIDDWLFWYQQKPGKAPKLLIYRASFLQ
SGVPSRFSGSGSGTDFTLTISSLQPEDFATYYCQQPLSRPFTFGQGTKVEIKR

TAR1-100-43 (SEQ ID NO: 143)
DIQMTQSPSSLSASVGDRVTITCRASQFIEDWLFWYQQKPGKAPKLLIYQASKLQ
SGVPSRFSGSGSGTDFTLTISSLQPEDFATYYCQQPLSRPFTFGQGTKVEIKR

TAR1-100-47 (SEQ ID NO: 144)
DIQMTQSPSSLSASVGDRVTITCRASQPIDSWLMWYQQKPGKAPKLLIYQASRLQ
SGVPSRFSGSGSGTDFTLTISSLQPEDFATYYCQQPLSRPFTFGQGTKVEIKR

TAR1-100-52 (SEQ ID NO: 145)
DIQMTQSPSSLSASVGDRVTITCRASQHIDDWLFWYQQKPGKAPKLLIYRASFLQ
SGVPPRFSGSGSGTDFTLTISSLQPEDFATYYCQQPLSRPFTFGQGTKVEIKR

TAR1-109 (SEQ ID NO: 146)
DIQMTQSPSSLSASVGDRVTITCRASQNIDDHLMWYQQKPGKAPKLLIYSSSILQS
GVPPRFSGSGSGTDFTLTISSLQPEDFATYYCQQPLSRPFTFGQGTKVEIKR

TAR1-100 (SEQ ID NO: 147)
DIQMTQSPSSLSASVGDRVTITCRASQDIDHALLWYQQKPGKAPRLLIYNGSMLQ
SGVPSRFSGSGSGTDFTLTISSLQPEDFATYYCQQVLRRPFTFGQGTKVEIKR

TAR1-100-34 (SEQ ID NO: 148)
DIQMTQSPSSLSASVGDRVTITCRASQHIGDWLLWYQQKPGKAPMLLIYQSSRLQ
SGVPSRFSGSGSGTDFILTISSLQPEDFATYYCQQPLSRPFTFGQGTKVEIKR

TAR1-100-36 (SEQ ID NO: 149)
DIQMTQSPSSLSASVGDRVTITCRASQHIDSYLMWYQQKPGKAPKLLIYNTSVLQ
SGVPSRFSGSGSGTDFTLTISSLQPEDFATYYCQQPLSRPFTFGQGTKVEIKR

TAR1-100-38 (SEQ ID NO: 150)

DIQMTQSPSSLSASVGDRVTITCRASQWIDDHLFWYQQKPGKAPKLLIYNTSTLQ
SGVPSRFSGSGSGTDFILTISSLQPEDFATYYCQQPLSRPFTFGQGTKVEIKR

TAR1-100-39 (SEQ ID NO: 151)
DIQMTQSPSSLSASVGDRVTITCRASQFIDEHLMWYQQKPGKAPKLLIYRSSELQS
GVPSRFSGSGSGTDFTLTISSLQPEDFATYYCQQPLSRPFTFGQGTKVEIKR

TAR1-100-40 (SEQ ID NO: 152)
DIQMTQSPSSLSASVGDRVTITCRASQWINNWLLWYQQKPGKAPKLLIYESSNLQ
SGVPSRFSGSGSGTDFTLTISSLQPEDFATYYCQQPLSRPFTFGQGTKVEIKR

TAR1-100-41 (SEQ ID NO: 153)
DIQMTQSPSSLSASVGDRVTITCRASQLIDDHFWYQQKPGKAPTLLIYNSSVLQSG
VPSRFSGSGSGTDFTLTISSLQPEDFATYYCQQPLSRPFTFGQGTKVEIKR

TAR1-100-45 (SEQ ID NO: 154)
DIQMTQSPSSLSASVGDRVTITCRASQDIDQWLMWYQQKPGKAPKLLIYQSSML
QSGVPSRFSGSGSGTDFTLTISSLQPEDFATYYCQQPLSRPFTFGQGTKVEIKR

TAR1-100-60 (SEQ ID NO: 155)
DIQMTQSPSSLSASVGDRVTITCQASQDIDNWLLWYQQKPGKAPKLLIYQASNLQ
SGVPSRFSGSGSGTDFTLTISSLQPEDFATYYCQQPLSRPFTFGQGTKVEIKR

TAR1-100-62 (SEQ ID NO: 156)
DIQMTQSPSSLSASVGDRVTITCRASQPIDSWLMWYQQKPGKAPKLLIYQASRLQ
SGVPSRFSGSGSGTDFTLTISSLQPEDFATYYCQQPLSGPFTFGQGTKVEIKR

TAR1-100-64 (SEQ ID NO: 157)
DIQMTQSPSSLSASVGDRVTITCRASQYIDYGLMWYQQKPGKAPKLLIYRTSELQ
SGVPSRFSGSGSGTDFTLTISSLQPEDFATYYCQQPLSRPFTFGQGTKVEIKR

TAR1-100-65 (SEQ ID NO: 158)
DIQMTQSPSSLSASVGDRVTITCRASQWIDSFLMWYQQKPGKAPKLLIYNGSVLQ
SGVPSRFSGSGSGTDFTLTISSLQPEDFATYYCQQPLSRPFTFGQGTKVEIKR

TAR1-100-75 (SEQ ID NO: 159)
DIQMTQSPSSLSASVGDRVTITCRASQDIGPWLMWYQQKPGKAPKLLIYQGSRLQ
SGVPLRFSGSGSGTDFTLTISSLQPEDFATYYCQQPLSRPFTFGQGTKVEIRR

TAR1-100-76 (SEQ ID NO: 160)
DIQMTQSPSSLSASVGDRVTITCRASQHIDSWLLWYQQKPGKAPKLLIYNGSVLQ
SGVPSRFSGSGSGTDFTLTISSLQPEDFATYYCQQPLSGPFTFGQGTKVEIKR

TAR1-100-77 (SEQ ID NO: 161)
DIQMTQSPSSLSASVGDRVTITCRASQHIDTHLFWYQQKPGKAPKLLIYNTSTLQS
GVPSRFSGSGSGTDFTLTISSLQPEDFATYYCQQPLSRPFTFGQGTKVEIKR

TAR1-100-78 (SEQ ID NO: 162)

DIQMTQSPSSLSASVGDRVTITCRASQFIDTHLMWYQQKPGKAPRLLIYNTSTLQS
GVPSRFSGSGSGTDFTLTISSLQPEDFATYYCQQPLSRPFTFGQGTKVEIKR

TAR1-100-80 (SEQ ID NO: 163)
DIQMTQSPSSLSASVGDRVTITCRASQDIDDWLLWYQQKPGKAPKLLIYQGSRLQ
SGVPSRFSGSGSGTDFTLTISSLQPEDFATYYCQQPLSRPFTFGQGTKVEIKR

TAR1-100-82 (SEQ ID NO: 164)
DIQMTQSPSSLSASVGDRVTITCRASQWIDDTLMWYQQKPGKAPKLLIYRSSMLQ
SGVPSRFSGSGSGTDFTLTISSLQPEDFATYYCQQPLSRPFTFGQGTKVEIKR

TAR1-100-83 (SEQ ID NO: 165)
DIQMTQSPSSLSASVGDRVTITCRASQYIDSHLMWYQQKPGKAPKLLIYDTSRLQ
SGVPSRFSGSGSGTDFTLTISSLQPEDFATYYCQQPLSRPFTFGQGTKVEIKR

TAR1-100-84 (SEQ ID NO: 166)
DIQMTQSPSSLSASVGDRVTITCRASQHIDQHLFWYQQKPGKAPKLLIYNSSSLQS
GVPSRFSGSGSGTDFTLTISSLQPEDFATYYCQQPLSRPFTFGQGTKVEIKR

TAR1-100-89 (SEQ ID NO: 167)
DIQMTQSPSSLSASVGDRVTITCRASQHIERWLLWYQQKPGKAPKLLIYNSSKLQ
SGVPSRFSGSGSGTDFTLTISSLQPEDFATYYCQQPLSRPFTFGQGTKVEIKR

TAR1-100-90 (SEQ ID NO: 168)
DIQMTQSPSSLSASVGDRVTISCRASQHIERWLLWYQQKPGKAPKLLIYNSSKLQS
GVPSRFSGSGSGTDFTLTISSLQPEDFATYYCQQPLSRPFTFGQGTKVEIKR

TAR1-100-91 (SEQ ID NO: 169)
DIQMTQSPSSLSASVGDRVTITCRASQDIGSWLMWYQQKSGKAPKLLIYNGSALQ
SGVPSRFSGSGSGTDFTLTISSLQPEDFATYYCQQPLSRPFTFGQGTKVEIKR

TAR1-100-92 (SEQ ID NO: 170)
DIQMTQSPSSLSASVGDRVTITCRASQHIDKWLMWYQQKPGKAPKLLIYQASKL
QSGVPSRFSGSGSGTDFTLTISSLQPEDFATYYCQQPLSRPFTFGQGTKVEIKR

TAR1-100-93 (SEQ ID NO: 171)
DIQMTQSPSSLSASVGDRVTITCRASQDIEEWLMWYQQKPGKAPKLLIYNSSTLQ
SGVPSRFSGSGSGTDFTLTISSLQPEDFATYYCQQPLSRPFTFGQGTKVEIKR

TAR1-100-94 (SEQ ID NO: 172)
DIQMTQSPSSLSASVGDRVTITCRASQYIDYGLMWYQQKPGKAPKLLIYRTSELQ
SGVPSRFSGSGSGTDFTLTISSLQPEDFATYYCQQPLSRPFTFGQ

TAR1-100-95 (SEQ ID NO: 173)
DIQMTQSPSSLSASVGDRVTITCRASQNIDIHLMWYQQKPGKAPKLLIYQSSNLQS
GVPSPFSGSGSGTDFTLTISSLQPEDFATYYCQQPLSRPFTFGQGTKVEIKR

TAR1-100-96 (SEQ ID NO: 174)
DIQMTQSPSSLSASVGDRVTITCRASQDIGPWLLWYQQKPGKAPKLLIYQSSELQS
GVPSRFSGSGSGTDFTLTISSLQPEDLATYYCQQPLSRPFTFGQGTKVEIKR

TAR1-100-97 (SEQ ID NO: 175)
DIQMTQSPSSLSASVGDRVTITCRASQEIGVWLMWYQQKPGKAPKLLIYEGSRLQ
SGVPSRFSGSGSGTDFTLTISSLQPEDFATYYCQQPLSRPFVFGQGTKVEIKR

TAR1-100-98 (SEQ ID NO: 176)
DIQMTQSPSSLSASVGDRVTITCRASQSIGKWLMWYQQKPGKAPKLLIYQSSLLQ
SGVPSRFSGSGSGTDFTLTISSLQPEDFATYYCQQPLSRPFTFGQGTKVEIKR

TAR1-100-99 (SEQ ID NO: 177)
DIQMTQSPSSLSASVGDRVTITCRASQDIDTWLFWYQQKPGKAPKLLIYNGSRLQ
SGVPSRFSGSGSGTDFTLTISGLQPEDFATYYCQQPLSRPFTFGQGTKVEIKR

TAR1-100-100 (SEQ ID NO: 178)
DIQMTQSPSSLSASVGDRVTITCRASQPIDSWLMWYQQKPGKAPKLLIYQASRLQ
SGVPSRFSGSGSGTDFTLTISSLQPEDFATYYCQQPLSRPFTFGQGTKVEIKR

TAR1-100-101 (SEQ ID NO: 179)
DIQMTQSPSSLSASVGDRVTITCRASQDIEGWLLWYQQKPGKAPKLLIYNSSTLQS
GVPSRFSGSGSGTDFTLTISSLQPEDFATYYCQQPLSRPFTFGQGTKVEIKR

TAR1-100-102 (SEQ ID NO: 180)
DIQMTQSPSSLSASVGDRVTITCRASQHIDDWLFWYQQKPGKAPKLLIYRASFLQ
SGVPSRFSGSGSGTDFTLTISSLQPEDFATYYCQQPLSRPFTFGQGTKVEIKR

TAR1-100-103 (SEQ ID NO: 181)
DIQMTQSPSSLSASVGDRVTITCRASQDIDTWLFWYQQKPGKAPKLLIYNGSRLQ
SGVPSRFSGSGSGTDFTLTISGLQPEDFATYYCQQPLSRPFTFGQGTKVEIKR

TAR1-100-105 (SEQ ID NO: 182)
DIQMTQSPSSLSASVGDRVTITCRASQPIEEWLLWYQQKPGKAPKLLIYNGSHLQS
GVPSRFSGSGSGTDFTLTISSLQPEDFATYYCQQPLSRPFTFGQGTKVEIKR

TAR1-100-106 (SEQ ID NO: 183)
DIQMTQSPSSLSASVGDRVTITCRASQHIDKWLMWYQQKPGKAPKLLIYQASKL
QSGVPSRFSGSGSGTDFTLTISSLQPEDFATYYCQQPLSRPFTFGQGTKVEIKR

TAR1-100-107 (SEQ ID NO: 184)
DIQMTQSPSSLSASVGDRVTITCRASQDIEEWLMWYQQKPGKAPKLLIYNSSTLQ
SGVPSRFSGSGSGTDFTLTISSLQPEDYATYYCQQPLSRPFTFGQGTKVEIKR

TAR1-100-108 (SEQ ID NO: 185)
DIQMTQSPSSLSASVGDRVTITCRASQPIDYGLMWYQQKPGKAPKLLIYRSSQLQ
SGVPSRFSGSGSGTDFTLTISSLQPEDFATYYCQQPLSRPFTFGQGTKVEIKR

TAR1-100-109 (SEQ ID NO: 186)
DIQMTQSPSSLSASVGDRVTITCRASQEIGSWLMWYQQKPGKAPKLLIYQSSKLQ
SGVPSRFSGSGSGTDFTLTISSLQPEDFATYYCQQPLSRPFTFGQGTKVEIKR

TAR1-100-110 (SEQ ID NO: 187)
DIQMTQSPSSLSASVGDRVTITCRASQPIDSWLLWYQQKPGKAPKLLIYNASSLQS
GVPSRFSGSGSGTDFTLTISSLQPEDFATYYCQQPLSRPFTFGQGTKVEIKR

TAR1-100-111 (SEQ ID NO: 188)
DIQMTQSPSSLSASVGDRVTITCRASQDIGPWLMWYQQKPGKAPKLLIYQASALQ
SGVPSRFSGSGSGTDFTLTISSLQPEDFATYYCQQPLSRPFTFGQGTKVEIKR

TAR1-100-112 (SEQ ID NO: 189)
DIQMTQSPSSLSASVGDRVTITCRASQNIHEWLMWYQQKPGKAPKLLIYQGSRLQ
SGVPSRFSGSGSGTDFTLTISSLQPEDFATYYCQQPLSRPFTFGQGTKVEIKR

TAR1-100-113 (SEQ ID NO: 190)
DIQMTQSPSSLSASVGDRVTITCRASQDIGPWLMWYQQKPGKAPKLLIYQASALQ
SGVPSRFSGSGSGTDFTLTISSLQPEDSATYYCQQPLSRPFTFGQGTKVEIKR

The sequence of the TAR1-5-19 anti-human TNF-α dAb adapted to various formats in these examples is as follows:

### TAR1-5-19

Amino acid (SEQ ID NO: 191)

DIQMTQSPSSLSASVGDRVTITCRASQSVKEFLWWYQQKPGKAPKLLIYMASNLQSGVPS
RFSGSGSGTDFTLTISSLQPEDFATYYCQQKFKLPRTFGQGTKVEIKR

Nucleotide (SEQ ID NO: 115)

gacatccagatgacccagtctccatcctccctgtctgcatctgtaggagaccgtgtcacc
atcacttgccgggcaagtcagagcgttaaggagtttttatggtggtaccagcagaaacca
gggaaagcccctaagctcctgatctatatggcatccaatttgcaaagtggggtcccatca
cgtttcagtggcagtggatctgggacagatttcactctcaccatcagcagtctgcaacct
gaagattttgctacgtactactgtcaacagaagtttaagctgcctcgtacgttcggccaa
gggaccaaggtggaaatcaaacgg

### Example 14: Efficacy study of PEGylated TAR1-5-19 in a prophylactic model of arthritis.

Tg197 mice are transgenic for the human TNF-globin hybrid gene and heterozygotes at 4-7 weeks of age develop a chronic, progressive polyarthritis with histological features in common with rheumatoid arthritis [Keffer, J., Probert, L.,Cazlaris, H., Georgopoulos, S.,Kaslaris, E., Kioussis, D., Kollias, G. (1991). Transgenic mice expressing human tumor necrosis factor: a predictive genetic model of arthritis. EMBO J., Vol. 10, pp. 4025-4031.]

To test the efficacy of a PEGylated dAb (PEG format being 2x20k branched with 2 sites for attachment of the dAb [i.e. 40K mPEG2 MAL2], the dAb being TAR1-5-19cys) in the prevention of arthritis in the Tg197 model, heterozygous transgenic mice were divided into groups of 10 animals with equal numbers of male and females. Treatment commenced at 3 weeks of age with weekly intraperitoneal injections of test items. The expression and PEGylation of TAR1-5-19cys monomer is outlined in Section 1.3.3, example 1. All protein preparations were in phosphate buffered saline and were tested for acceptable levels of endotoxins.

The study was performed blind. Each week the animals were weighed and the macrophenotypic signs of arthritis scored according to the following system: 0 = no arthritis (normal appearance and flexion), 1 = mild arthritis (joint distortion), 2 = moderate arthritis (swelling, joint deformation), 3 = heavy arthritis (severely impaired movement).

The outcome of the study clearly demonstrated that 10mg/kg PEGylated TAR 1-5-19 inhibited the development of arthritis with a significant difference between the arthritic scoring of the saline control and treated group. The 1mg/kg dose of PEGylated TAR1-5-19 also produced a statistically significantly lower median arthritic score than saline control group (P<0.05% using normal approximation to the Wilcoxon Test).

### Example 15: Efficacy study of PEGylated TAR1-5-19 in a therapeutic model of arthritis.

To test the efficacy of a PEGylated dAb in the therapeutic model of arthritis in the Tg197 model, heterozygous transgenic mice were divided into groups of 10 animals with equal numbers of male and females. Treatment commenced at 6 weeks of age when the animals had significant arthritic phenotypes. Treatment was twice weekly with 4.6mg/kg intraperitoneal injections of test items. The sample preparation and disease scoring are as described above in Example 14.

The arthritic scoring clearly demonstrated that PEGylated TAR1-5-19 inhibited the progression of arthritis in a therapeutic model. The 4.6mg/kg dose of PEGylated TAR1-5-19 produced a statistically significantly lower median arthritic score than saline control group at week 9 (P<0.01% using normal approximation to the Wilcoxon Test).

### Example 16: dAb Efficacy in a Slow Release Format

To test the efficacy of a dAb from a slow release format, a dAb with a small PEG molecule (where the PEG is 4x5k with four sites for attachment of a dAb with a C-terminal cys residue, the dAb being TAR1-5-19 [i.e. 20K PEG 4 arm MAL]) was loaded into a 0.2 ml osmotic pump. The pump had a release rate of 0.2 ml over a 4 week period was implanted subcutaneously into mice at week 6 in the therapeutic Tg197 model as described above. The arthritic scores of these animals increased at a clearly slower rate when compared to animals implanted with pumps loaded with saline. This demonstrates that dAbs are efficacious when delivered from a slow release format.

### Example 17: Half-life stabilized anti-human TNF-α dAb prevents the onset of RA in the Tg197 mouse model.

The dAb monomer TAR1-5-19 described herein is an affinity matured dAb monomer derived from a dAb initially selected using passively coated TNF-α. The initial clone had a ND50 in the L929 TNF-cytotoxicity neutralization assay greater than 5 µM. TAR1-5-19 has an ND50 of less than 30 nM. When formatted as an Fc Fusion as described herein, the TAR1-5-19 clone has an ND50 of less than 5 nM in the L929 assay.

The serum half-life of TAR1-5-19 dAb Fc-fusion was examined following injection into mice. Results are shown in **Figure 24.** Where the TAR1-5-19 dAb monomer had a t1/2β of approximately 20 minutes, the Fc-fusion formatted version of the same dAb had a t1/2β of greater than 24 hours, representing a greater than 70-fold increase in serum half-life.

The TAR1-5-19 dAb Fc fusion construct was tested in the Tg197 mouse model of RA described herein above. Mice were divided into five groups of 10, with equal numbers of male and female mice per group. Treatment with twice weekly IP injections of TAR1-5-19 dAb Fc fusion, ENBREL or saline was begun at 3 weeks of age, a time at which RA symptoms have not yet manifested. The study was conducted for 7 weeks. As shown in **Figure 25,** two dosages of the TAR1-5-19 dAb Fc fusion, 1 mg/kg and 10 mg/kg, were administered. Negative control animals received a negative control anti-β-gal Fc fusion twice weekly at 10 mg/kg, and one group was treated twice weekly with saline injection. For comparison, one group received 10 mg/kg of ENBREL twice weekly.

Animals were assessed for arthritic scores as described herein, in a blinded manner. At the end of the 7 week course of treatment, animals receiving the twice weekly dosage of 10 mg/kg of the TAR1-5-19 dAb Fc fusion had lower arthritic scores than the animals receiving ENBREL at 10 mg/kg, and had experienced essentially complete prevention of arthritic disease relative to non-treated animals or animals receiving the negative control dAb Fc fusion.

TNF-α is associated with cachexia. Animals were weighed throughout the course of anti-TNF-α dAb treatment. The weights of the animals receiving the TAR1-5-19 dAb Fc fusion were significantly greater than those receiving negative control dAb Fc fusion and no treatment and similar to the weights of the animals receiving ENBREL injections.

In summary, 10 mg/kg TAR1-5-19 completely prevented the onset of arthritis in the Tg197 model. This response was dose-dependent, with a partial effect resulting from a 1 mg/kg dose, and the response was superior to that observed with a similar dose of the existing anti-TNF-α drug ENBREL. This study demonstrates the efficacy of dAbs as therapeutics in a clinically accepted model of human disease.

Histopathological analyses of fixed sections from the joints of the animals are in agreement with these data (not shown).

### Example 18: In vivo studies on differing extended half-life formats.

In one series of studies, three different extended half-life anti-TNF-α dAb formats were examined for their effect on arthritic score. These formats were an anti-TNF-α dAb Fc fusion (two anti-human TNF-α dAbs homodimerized by fusion to human IgG C_{H}2/C_{H}3 region), two different PEG-linked anti-TNF-α dAb constructs (a homodimer formed by the cys-maleimide linkage of two identical dAbs to a 2x20K branched PEG and a homotetramer formed by the cys-maleimide linkage of four identical dAbs to a 4x10K branched PEG) and a dual-specific anti-TNF-α/Anti SA dAb comprising two identical anti-TNF-α dAbs followed by an anti-mouse serum albumin dAb.

In separate studies, drug compositions were administered either weekly at 10 mg/kg or 1 mg/kg as shown in **Figure 26** or twice weekly at varying doses, commencing at 3 weeks of age and continuing for 7 weeks.

The PEGylated anti-TNF dAb homodimer was effective at 10 mg/kg in the weekly injection protocol for the complete prevention of arthritis based on arthritic score.
Current anti-TNF-α drug used for comparison had a reduced arthritic score relative to untreated animals, but the score was higher in a statistically significant manner than the score achieved with the PEGylated dAb construct. The anti-TNF-α/anti-SA dual specific and the Fc fusion showed effect relative to no treatment.

In the 1 mg/kg weekly injection regimen, while none of the treatments was 100% effective at preventing the onset of disease, the PEGylated anti-TNF-α dAb construct was still highly effective in preventing the progression of disease symptoms relative to no treatment and current anti-TNF-α drug. In this dosing regimen, the anti-TNF-α dAb Fc fusion and the dual-specific construct were also more effective than the current drug.

In summary, the weekly dosing regimen studies with three different formats of half-life-extended dAbs further validates the efficacy of treatment in a clinically accepted model of human disease.

### Example 19: Efficacy of anti-human TNF-α dAbs in the Tg197 mouse RA model relative to existing anti-TNF-α therapeutics against established disease.

In this study, the efficacy of various formats and dosage regimens of anti-TNF-α dAb constructs against established disease was compared to that of equal molar doses of the current anti-TNF-α therapeutics ENBREL, HUMIRA and REMICADE in the Tg197 RA model. Animals were administered the therapeutics starting at 6 weeks, instead of at 3 weeks, such that arthritic symptoms had already manifested. Symptoms were monitored by histology (at 9 weeks) and arthritic scoring (weekly) in a blinded manner.

The various formats and dosages for twice-weekly administration are shown in **Figure 27.** Formats included the Fc fusion (two copies of the TAR1-5-19 dAb homodimerized by fusion to human IgG1 C_{H}2/C_{H}3 region), the TAR1-5-19 dAb PEG dimer (a homodimer formed by the cys-maleimide linkage of two identical dAbs to a 2x20K branched PEG), the TAR1-5-19 dAb PEG tetramer (a homotetramer formed by the cys-maleimide linkage of four identical dAbs to a 4x 10K branched PEG), the TAR1-5-19 dAb/anti mouse SA dual-specific (linear fusion of two identical anti-TNF-α dAbs followed by an anti-mouse serum albumin dAb). The dosing regimen is shown schematically in **Figure 28.** Continuous administration of a 4x5k PEGylated TAR1-5-19 construct via an implanted osmotic pump was also evaluated.

The results of the study showed not one of the current biologics appreciably reversed the arthritic score by 9 weeks. The TAR formats all to a greater or lesser degree stabilized the arthritic score when compared with the saline control, and this was statistically significant. Moreover when compared with the week 6 score there were signs of disease reversal.

The arthritic joints at week 9 when examined for histopathological disease status also showed a reduction in disease severity following treatment with the TAR formats when compared with the joints at week 6. This confirms that the TAR formats can elicit a reversal of the arthritic phenotype of the established disease.

These studies demonstrate the effectiveness of the tested anti-TNF-α dAb constructs against established arthritic disease, including the ability of a TNF-α dAb to at least partially reverse the course of disease.

### Example 20: Efficacy of an anti-TNF dAb as a fusion with an anti-serum albumin dAb A Efficacy study of TAR1-5-19/anti-serum albumin dAb fusion in a prophylactic model of arthritis.

Tg197 mice are transgenic for the human TNF-globin hybrid gene and heterozygotes at 4-7 weeks of age develop a chronic, progressive polyarthritis with histological features in common with rheumatoid arthritis [Keffer, J., Probert, L.,Cazlaris, H., Georgopoulos, S., Kaslaris, E., Kioussis, D., Kollias, G. (1991). Transgenic mice expressing human tumour necrosis factor: a predictive genetic model of arthritis. EMBO J., Vol. 10, pp. 4025-4031.]

To test the efficacy of a TAR1-5-19/anti-serum albumin dAb fusion (an inline trimer of 3 dAbs, being TAR1-5-19, TAR1-5-19 and an anti-mouse serum albumin dAb) in the prevention of arthritis in the Tg197 model, heterozygous transgenic mice were divided into groups of 10 animals with equal numbers of male and females. Treatment commenced at 3 weeks of age with weekly intraperitoneal injections of test items. TAR1-5-19/anti-serum albumin dAb fusion was expressed in E. coli with a C-terminal hexa histidine tag and purified by Ni affinity chromatography, IEX and gel filtration. All protein preparations were in phosphate buffered saline and were tested for acceptable levels of endotoxins.
The study was performed blind. Each week the animals were weighed and the macrophenotypic signs of arthritis scored according to the following system: 0 = no arthritis (normal appearance and flexion), 1 = mild arthritis (joint distortion), 2 = moderate arthritis (swelling, joint deformation), 3 = heavy arthritis (severely impaired movement).
The outcome of the study clearly demonstrated that 10mg/kg TAR 1-5-19/anti-serum albumin dAb fusion inhibited the development of arthritis with a significant difference between the arthritic scoring of the saline control and treated group. The 1mg/kg dose of TAR1-5-19/anti-serum albumin dAb fusion also produced a statistically significantly lower median arthritic score than saline control group (P<2% using normal approximation to the Wilcoxon Test).

### B Efficacy study of TAR1-5-19/anti-serum albumin dab fusion in a therapeutic model of arthritis.

To test the efficacy of a TAR1-5-19/anti-serum albumin dAb fusion in the therapeutic model of arthritis in the Tg197 model, heterozygous transgenic mice were divided into groups of 10 animals with equal numbers of male and females. Treatment commenced at 6 weeks of age when the animals had significant arthritic phenotypes.
Treatment was twice weekly with 2.7mg/kg intraperitoneal injections of test items. The sample preparation and disease scoring are as described above.

The arthritic scoring clearly demonstrated that TAR1-5-19/anti-serum albumin dAb fusion inhibited the progression of arthritis in a therapeutic model. The 2.7mg/kg dose of TAR1-5-19/anti-serum albumin dAb fusion produced a statistically significantly lower median arthritic score than saline control group at week 9 (P<0.05% using normal approximation to the Wilcoxon Test).

This clearly demonstrates that anti-TNF dAbs can be effective in a format with anti-SA dAbs and that the anti-SA dAb has extended the serum half life of the anti-TNF dAb from that which would be expected for an anti-TNF dAb alone.

### Example 21: Examination of the effects of anti-TNF-α dAbs as disclosed herein on arthritic and histopathological scores in the Tg197 mouse model of RA.

Two additional studies were carried out examining the effects of anti-TNF-α dAbs on arthritic and histopathologic scores in the Tg197 model of RA.

In the first study, a TAR1-5-19 dAb Fc fusion as described above was administered at 10 mg/kg, twice weekly commencing at 3 weeks of age - before the onset of RA symptoms. Results were judged in comparison with saline, ENBREL and control Fc fusion dAb injection on the same schedule.

The TAR1-5-19 dAb Fc fusion was more effective than ENBREL in preventing the onset of RA symptoms in the mice as judged by arthritic score and from analysis of the histology slides.

In the second study, the effects of weekly injections of anti-TNF-α dAb Fc fusion, PEG dimer and dual-specific anti-TNF/antiSA at 10 or 1 mg/kg, commencing at 3 weeks of age. Comparison is to ENBREL and HUMIRA.

The arthritic scores for all the TAR formats, given as either 1 mg/kg or 10mg/kg doses, were reduced when compared with the saline control. Moreover there was evidence of a delay in the onset of the disease. The PEGylated and anti-SA dual specific formats were more effective at reducing the severity of the arthritis when compared with Humira and Enbrel. In addition analysis of the histology of the joints at week 10 also showed that the TAR formats had been efficacious and reduced the disease severity when compared with the saline control.

In summary, the TAR1-5-19 anti-TNF-dAb in the Fc fusion, PEGylated and anti-SA dual specific formats are all effective against RA symptoms in the Tg197 model system, whether administered before or after the onset of arthritic symptoms. The most effective anti-TNF dAb formats are either equivalent to or more effective than HUMIRA, and the most effective anti-TNF dAb formats are significantly more effective than ENBREL in all studies.

### Example 22: Anti-human VEGF dAbs

### TAR15 (anti-human VEGF)

VK dAbs that bind human VEGF are described below. RBA refers to the VEGF receptor 2 binding assay described herein.

| Lead | dAb | RBA (R2) IC50 - protein L (nM) | RBA (R2) IC50 + protein L (nM) | Cross-reactivity with mouse VEGF in ELISA |
|---|---|---|---|---|
| TAR15-1 | VK | 171 | 7.4 | + |
| TAR15-10 | VK | 12.2 | 0.3 | + |
| TAR15-16 | VK | 31 | 1.7 | +/- |
| TAR15-17 | VK | 38 | 0.5 | +/- |
| TAR15-18 | VK | 174 | 0.4 | + |
| TAR15-20 | VK | 28 | 0.3 | - |

The TAR15-1 clone has a Kd of 50-80 nM when tested at various concentrations on a low density BIAcore chip. Other VK clones were passed over the low density chip at one concentration (50nM). Different clones show different kinetic profiles.

### Amino acid sequences:

Consensus sequence: W28, G30, E32, S34, H50 and Y93.

Additional TAR15 anti-human VEGF dAb clones have a consensus sequence:

W28, G30, E32, S34, H50 and Y93, as shown in TAR15-10 below. 
TAR15-1 (SEQ ID NO: 192)
DIQMTQSPSSLSASVGDRVTITCRASQWIGPELSWYQQKPGKAPKLLIYHGSILQS
GV
PSRFSGSGSGTDFTLTISSLQPEDFATYYCQQRMYRPATFGQGTKVEIKR

TAR15-3 (SEQ ID NO: 193)
DIQMTQSPSSLSASVGDRVTITCRASQWIGRELKWYQQKPGKAPRLLIYHGSVL
QSG
VPSRFSGSGSGTDFTLTISSLQPEDFATYYCQQDFFVPDTFGQGTKVEIKR

TAR15-4 (SEQ ID NO: 194)
DIQMTQSPSSLSASVGDRVTITCRASQDIANDLMWYQQKPGKAPKLLIYRNSRLQ
GG
VPSRFSGSGSGTDFTLTISSLQPEDFATYYCQQLVHRPYTIGQGTKVEIKR

TAR15-9 (SEQ ID NO: 195)
DIQMTQSPSSLSASVGDRVTITCRASQFIGPHLTWYQQKPGKAPKLLIYHSSLLQS
GV
PSRFSGSGSGTDFTLTISSLQPEDFATYYCQQYMYYPSTFGQGTKVKIKR

TAR15-10 (SEQ ID NO: 196)
DIQMTQSPSSLSASVGDRVTITCRASQWIGPELSWYQQKPGKAPKLLIYHTSILQS
GVP
SRFSGSGSGTDFTLTISSLQPEDFATYYCQQYMFQPRTFGQGTKVEIRR

TAR15-11 (SEQ ID NO: 197)
DIQMIQSPSSLSASVGDRVTITCRASQFIGNELSWYQQKPGKAPKLLIYHASSLQS
GV
PSRFSGSGSGTDFTLTISSLQPEDFATYYCQQVLGYPYTFGQGTKVEIKR

TAR15-12 (SEQ ID NO: 198)
DIQMTQSPSSLSASVGDRVTITCRASQWIGPELSWYQQKPGKAPKLLIYHGSILQ
SG
VPSRFSGSGSGTDFTLTISSLQPEDFATYYCQQVLYSPLTFGQGTKVEIKR

TAR15-13 (SEQ ID NO: 199)
DIQMTQSPSSLSASVGDRVTITCRASQWIGNELKWYQQKPGKAPKLLIYMSSLL
QSG
VPSRFSGSGSGTDFTLTISSLQPEDLATYYCQQTLLLPFTFGQGTKVEIKR

TAR15-14 (SEQ ID NO: 200)
DIQMTQSPSSLSASVGDRVTITCRASQWIGPELSWYQQKPGKAPKLLIYHGSILQ
SG
VPSRFSGSGSGTDFTLTISSLQPEDFATYYCQQRLYYPGTFGQGTKVEIKR
 
TAR15-15 (SEQ ID NO: 201)
DIQMTQSPSSLSASVGDRVTITCRASQSIGRELSWYQQKPGKAPMLLIYHSSNLQ
SG
VPSRFSGSGSGTDFTLTISSLQPEDFATYYCQQGMYWPYTFGQGTKVEIKR

TAR15-16 (SEQ ID NO: 202)
DIQMTQSPSSLSASVGDRVTITCRASQWIKPALHWYQQKPGKAPKLLIYHGSILQS
GV
PSRFSGSGSGTDFTLTISSLQPEDFATYYCQQTLFMPYTFGQGTKVEIKR

TAR15-17 (SEQ ID NO: 203)
DIQMTQSPSSLSASVGDRVTITCRASQSISTALLWYQQKPGKAPKLLIYNGSMLPN
GVP
SRFSGSGSGTDFTLTISSLQPEDFATYYCQQTWDTPMTFGQGTKVEIKR

TAR15-18 (SEQ ID NO: 204)
DIQMTQSPSSLSASVGDRVTITCRASQWIGHDLSWYQQKPGKAPKLLIYHSSSLQS
GV
PSRFSGSGSGTDFTLTISSLQPEDVATYYCQQLMGYPFTFGQGTKVEIKR

TAR15-19 (SEQ ID NO: 205)
DIQMTQSPSSLSASVGDRVTITCRASQDIGGLLVWYQQKPGKAPKLLIYRSSYLQ
SGV
PSRFSGSGSGTDFTLTISSLQPEDFATYYCQQTWGIPHTFGQGTKVEIKR

TAR15-20 (SEQ ID NO: 206)
DIQMTQSPSSLSASVGDRVTITCRASQKIFNGLSWYQQKPGKAPKLLIYHSSTLQS
GVP
SRFSGSGSGTDFTLTISSLQPEDFATYYCQQVLLYPYTFGQGTKVEIKR

TAR 15-22 (SEQ ID NO: 207)
DIQMTQSPSSLSASVGDRVTITCRASQSIGTNLSWYQQKPGKAPRLLIYRTSMLQ
SG
VPSRFSGSGSGTDFTLTISSLQPEDFATYYCQQQFFWPHTFGQGTKVEIKR
 VH dAbs that bind human VEGF are described below. These clones give a reduction (more than 50%) in the supernatant RBA (R2):

| Lead | dAb | More than 50% reduction in supernatant RBA (R2) | Cross-reactivity with mouse VEGF in ELISA |
|---|---|---|---|
| TAR15-5 | VH | + | + |
| TAR15-6 | VH | + | +/- |
| TAR15-7 | VH | + | +/- |
| TAR15-8 | VH | + | + |
| TAR15-23 | VH | + | - |
| TAR15-24 | VH | + | - |
| TAR15-25 | VH | + | - |
| TAR15-26 | VH | + | +/- |
| TAR15-27 | VH | + | +/- |
| TAR15-29 | VH | + | - |
| TAR15-30 | VH | + | - |

VH clones were passed over the low density VEGF chip on a BIAcore at one concentration (50nM). Different clones give different kinetic profiles.

Amino acid sequences:

TAR15-5 (SEQ ID NO: 208)
EVQLLESGGGLVQPGGSLRLSCAASGFTFRLYDMVWVRQAPGKGLEWVSYISSGGSGTYYADS
VKGRFTISRDNSKNTLYLQMNSLRAEDTAVYYCAKAGGRASFDYWGQGTLVTVSS

TAR15-6_(SEQ ID NO: 209)
EVQLLESGGGLVQPGGSLRLSCAASGFTFHLYDMMWVRQAPGKGLEWVSFIGGDGLNTYYAD
SVKGRFTISRDNSKNTLYLQMNSLRAEDTAVYYCAKAGTQFDYWGQGTLVTVSS

TAR15-7 (SEQ ID NO: 210)
EVQLLESGGGLVQPGGSLRLSCAASGFTFNKYPMMWVRQAPGKGLEWVSEISPSGQDTYYAD
SVKGRFTISRDNSKNTLYLQMNSLRAEDTAVYYCAKNPQILSNFDYWGQGTLVTVSS

TAR15-8 (SEQ ID NO: 211)
EVQLLESGGGLVQPGGSLRLSCAASGFTFQWYPMWWVRQAPGKGLEWVSLIEGQGDRTYYAD
SVKGRFTISRDNSKNTLYLQMNSLRAEDTAVYYCAKAGDRTAGSRGNSFDYWGQGTLVTVSS

TAR15-23 (SEQ ID NO: 212)
EVQLLESGGGLVQPGGSLRLSCAASGFTFKAYEMGWVRQAPGKGLEWVSGISPNGGWTYYAD
SVKGRFTISRDNSKNTLYLQMNSLRAEDTAVYYCAKESISPTPLGFDYWGQGTLVTVSS

TAR15-24 (SEQ ID NO: 213)
EVQLLESGGGLVQPGGSLRLSCAASGFTFTGYEMGWVRQAPGKGLEWVSYISRGGRWTYYAD
SVKGRFTISRDNSKNTLYLQMNSLRAEDTAVYYCAKSDTMFDYWGQGTLVTVSS

TAR15-25 (SEQ ID NO: 214)
EVQLLESGGGLVQPGGSLRLSCAASGFTFSAYEMGWVRQAPGKGLEWVSFISGGGRWTYYAD
SVKGRFTISRDNSKNTLYLQMNSLRAEDTAVYYCAKYSEDFDYWGQGTLVTVSS

TAR15-26 (SEQ ID NO: 215)
EVQLLESGGGLVQPGGSLRLSCAASGFTFGAYPMMWVRQAPGKGLEWVSEISPSGSYTYYADS
VKGRFTISRDNSKNTLYLQMNSLRAEDTAVYYCAKDPRKFDYWGQGTLVTVSS

TAR15-27 (SEQ ID NO: 216)
EVQLLESGGGLVQPGGSLRLSCAASGFTFQFYKMGWVRQAPGKGLEWVSSISSVGDATYYADS
VKGRFTISRDNSKNTLYLQMNSLRAEDTAVYYCAKMGGGPPTYVVYFDYWGQGTLVTVSS

TAR15-29 (SEQ ID NO: 217)
EVQLLESGGGLVQPGGSLRLSCAASGFTFGEYGMYWVRQAPGKGLEWVSSISERGRLTYYADS
VKGRFTISRDNSKNTLYLQMNNLRAEDTAVYYCAKSALSSEGFSRSFDYWGQGTLVTVSS

TAR15-30 (SEQ ID NO: 218)
EVQLLESGGGLVQPGGSLRLSCAASGFTFSDYAMYWVRQAPGKGLEWVSSITARGFITYYADS
VKGRFTISRDNSKNTLYLQMNSLRAEDTAVYYCAKSGFPHKSGSNYFDYWGQGTLVTVSS

### Example 23: Additional studies with anti-VEGF dAbs

Anti VEGF dAbs as described herein can be tested for efficacy in various formats as described above for the anti-TNF-α dAbs, including, for example, Fc fusion, Fabs, PEGylated forms, dimers, tetramers, and anti-SA dual specific forms. The anti-VEGF dAbs can also be evaluated not only with anti-TNF-α dAbs as described herein, but also with other anti-TNF-α preparations, such as HUMIRA, ENBREL, and/or REMICADE.

The additional studies can be carried out to examine the effects of anti-VEGF dAbs on, for example, arthritic and histopathologic scores in the Tg197 model of RA.

For example, a TAR15 dAb Fc fusion similar to the TAR1-5-19 Fc fusions described herein above is administered IP at 1 mg/kg or 10 mg/kg, weekly or twice weekly commencing at 3 weeks of age (before the onset of RA symptoms), or at 6 weeks of age (after the onset of symptoms) and continuing for up to 7 weeks or more. Results are judged in comparison with saline, control Fc fusion (anti-β-gal), TAR1-5-19 alone, ENBREL, REMICADE and/or HUMIRA, preferably in equal molar amounts.

Animals are scored for macrophenotypic indicia (e.g., arthritic score) and histopathological scores as described above. Efficacy is demonstrated by any of
i) a failure to develop disease symptoms (as evidenced by arthritic or histopathological scores) when administered to animals beginning at 3 weeks of age,
ii) lessened severity of disease symptoms appearing when administered starting at 3 weeks of age, relative to control animals,
iii) failure to progress to more severe disease or progression at a lower rate relative to control animals when administered beginning at 6 weeks of age,
iv) reversal of symptoms (again, by arthritic score or hostopathological score) at any of 7, 8, 9, 10, 11, 12, or 14 weeks when administered to an animal beginning at 6 weeks of age.

Similar studies can be carried out with each of the different formats described above, e.g., Fabs, PEGylated forms, dimers, tetramers, and anti-SA dual specific forms.

Anti VEGF dAbs such as TAR15 dAb can also be administered to the Tg197 mouse model in combination with HUMIRA, ENBREL, and/or REMICADE. Such studies are performed in the same manner as described above for the testing of VEGF dAbs alone, and efficacy is also determined in the same manner.

### Example 24: Evaluation of anti-TNF-α dAbs in a Crohn's disease model

To evaluate the effectiveness of anti-TNF-α dAbs (and/or anti-VEGF dAbs) in Crohn's disease, the Tnf^{ΔARE} transgenic mouse model of Crohn's disease originally described by Kontoyiannis et al., 1999, Immunity 10: 387-398 is used (the DSS model can also be used in a similar fashion). The animals develop an IBD phenotype with similarity to Crohn's disease starting between 4 and 8 weeks of age. Therefore, anti-TNF-a dAb, e.g., TAR1-5-19 in various formats (Fc fusion, Fab, PEGylated (dimeric, tetrameric, etc.), dual specific with VEGF, dual specific with anti-SA, etc.) is administered at either 3 weeks of age (to test prevention of disease) or 6 weeks of age (to test stabilization, prevention of progression or reversal of disease symptoms), and animals are scored by weight and histologically as described herein. IP dosages of 1 mg/kg and 10 mg/kg are used for initial studies, with adjustments made in accord to the results of these initial studies. Test compositions are administered either weekly or twice weekly, or can be administered continuously, for example, using an osmotic pump. Alternatively, oral delivery formulations, e.g., by oral gavage with Zantac or by enteric coated formulations can also be applied. The studies are continued for up to 7 weeks or more once initiated.

Efficacy in the TNF^{ΔARE} model of Crohn's disease is shown by any of:
i) a failure to develop disease symptoms when administered to animals beginning at 3 weeks of age,
ii) lessened severity of disease symptoms appearing when administered starting at 3 weeks of age, relative to control animals,
iii) failure to progress to more severe disease or progression at a lower rate relative to control animals when administered beginning at 6 weeks of age,
iv) reversal of symptoms at any of 7, 8, 9, 10, 11, 12, or 14 weeks when administered to an animal beginning at 6 weeks of age.

In particular, treatment is considered effective if the average histopathological disease score is lower in treated animals (by a statistically significant amount) than that of a vehicle control group. Treatment is also considered effective if the average histopathological score is lower by at least 0.5 units, at least 1.0 unit, at least 1.5 units, at least 2.0 units, at least 2.5 units, at least 3.0 units, or by at least 3.5 units relative to the vehicle-only control group. Alternatively, the treatment is effective if the average histopatholigical score remains at or is lowered to 0 to 0.5 throughout the course of the therapeutic regimen.

As with the RA model, the effect of combination therapies with dAbs specific for VEGF or with other anti-TNF-a compositions (e.g., ENBREL, REMICADE and/or HUMIRA) is also evaluated in this model.

### Example 25: Dual-specific IgG directed against human TNF-alpha and human VEGF

In the engineered IgG-like dual-specific format described below, dAbs of two different specificities are fused to heavy and light chain constant domains, respectively. Upon co-expression in a cell, a two-armed IgG-like molecule is generated in which two variable domains capable of binding to two therapeutic targets (e.g., one specific for TNF-α and one specific for VEGF) are present on each ann of the dual targeting IgG.

**DNA constructs.** Mammalian expression vectors used were based on the Invitrogen pcDNA3.1 backbone, which facilitates gene expression in mammalian cells via the CMV immediate early promoter. For heavy chain expression, a cassette consisting of a human CD33 signal peptide and the human IgG1 heavy chain constant domain was inserted into the NheI and XbaI restriction sites of the vector pcDNA3.1(+), and variable domains specific for VEGF and expressed as part of the heavy chain polypeptide were cloned into this cassette between the CD33 signal peptide and the IgG1 heavy chain constant domain, using HindIII and NotI restriction sites. For light chain expression, a cassette consisting of a CD33 signal peptide and the human C kappa constant domain was inserted into the NheI and XhoI restriction sites of the vector pcDNA3. zeo(+), and variable domains specific for TNF-alpha and expressed as part of the light chain polypeptide were cloned into this cassette between the CD33 signal peptide and the C kappa constant domain, using HindIII and NotI restriction sites.

**Protein expression and purification.** DNA of the heavy and light chain expression vectors was prepared using the Qiagen EndoFree plasmid Mega kit according to manufacturer's instructions, and used to transfect HEK293 (obtained from the European Collection of Cell Cultures) or Cos-7 cells (obtained from the American Type Culture Collection) with the Roche transfection reagent Fugene6, according to manufacturer's instructions. After 5 days, culture supernatants were harvested by centrifugation and secreted dual-specific antibodies were purified using two-step affinity purification. First, culture supernatants were supplemented with phosphate-buffered saline (PBS) to a final concentration of 1.5x PBS, and antibodies were captured on Amersham Streamline protein A resin. Resins were washed with 2x PBS, followed by 10mM Tris pH8, and bound antibodies were eluted using 0.1M glycine pH2. Eluates were neutralised by adding a 25% volume of 1M Tris pH8, and recombinant antibodies were captured on Affitech protein L agarose resin. Resins were again washed using 2x PBS, followed by 10mM Tris pH8, and bound recombinant antibodies were eluted using 0.1M glycine pH2 and eluates neutralised by adding a 25% volume of 1M Tris pH8.

**Analysis of recombinant antibodies**. The purified recombinant antibodies were quantified on a spectrophotometer using absorbance reading at 280nm and analysed by SDS-PAGE, using Invitrogen NuPAGE 4-12% Bis-Tris gels and SilverQuest staining, according to manufacturer's instructions. Figure 29 shows the SDS-PAGE analysis of a dual-specific antibody that comprises a kappa variable domain specific for human VEGF fused to the human IgG1 heavy chain constant domain and a kappa variable domain specific for human TNF-alpha fused to the human C kappa constant domain. Lane 1 was loaded with the Invitrogen MultiMark molecular weight marker, lane 2 was loaded with the dual-specific antibody in 1 x Invitrogen NuPAGE LDS sample buffer and lane 3 with the dual-specific antibody in 1 x Invitrogen NuPAGE LDS sample buffer supplemented with 10mM betamercaptoethanol. In lane 3, the heavy chain is seen as a 50kDa band and the light chain is seen as a 25kDa band.

**Testing of dual specificity**. The dual-specific nature of the expressed antibodies was demonstrated by measuring the potency of each purified batch of antibody both in a human TNF-cell assay and in a human VEGF receptor binding assay.

The human TNF cell-based assay used was the L929 cytotoxicity assay described by Evans (2000, Molecular Biotechnology 15, 243-248). Briefly, L929 cells plated in microtitre plates were incubated overnight with dual-specific antibody, 100 pg/ml TNF and 1 mg/ml actinomycin D (Sigma, Poole, UK). Cell viability was measured by reading absorbance at 490nm following an incubation with [3-(4,5-dimethylthiazol-2-yl)-5-(3 - carbboxymethoxyphenyl)-2-(4-sulfophenyl)-2H- tetrazolium (Promega, Madison, USA). Anti-TNF activity led to a decrease in TNF cytotoxicity and therefore an increase in absorbance compared with the TNF only control.

VEGF activity was measured using the VEGFR2 binding assay essentially as described above in the section titled "Preparation of immunoglogulin based multi-specific ligands." Briefly, a 96 well Nunc Maxisorp assay plate was coated overnight with recombinant human VEGF R2/Fc (R&D Systems, Cat. No: 357-KD-050) at 0.5 µg/ml in carbonate buffer. Wells were washed repeatedly with 0.05% tween/PBS and then PBS. 2% BSA in PBS was added to block the plate. Wells were washed (as above), then purified dual-specific antibody was added to each well. VEGF, at 6ng/ml in diluent (for a final concentration of 3ng/ml), was then added to each well and the plate incubated for 2hr at room temperature. Wells were washed as above, and then biotinylated anti-VEGF antibody (R&D Systems, Cat No: BAF293) at 0.5µg/ml in diluent was added and incubated for 2hr at room temperature. Wells were washed as above, followed by the addition of HRP conjugated anti-biotin antibody (1:5000 dilution in diluent; Stratech, Cat No: 200-032-096). The plate was then incubated for 1hr at room temperature. The plate was washed as above, ensuring any traces of Tween-20 have been removed. For detection, 100µl of SureBlue 1-Component TMB Micro Well Peroxidase solution was added to each well. The reaction is stopped by the addition of 1 M hydrochloric acid, followed by reading OD₄₅₀ using a plate reader.

Figure 30 shows the results for a dual-specific antibody that comprises a kappa variable domain specific for human VEGF fused to the human IgG 1 heavy chain constant domain and a kappa variable domain specific for human TNF-alpha fused to the human C kappa constant domain. The dual-specific antibody (denoted anti-TNF-alpha x anti-VEGF) bound both human TNF-alpha and human VEGF. The antibody is bivalent for both targets: The ND50 for TNF-alpha is significantly lower (24nM) than for the anti-TNF-alpha monomer (200nM) that is fused as a variable domain to C kappa in the dual-specific molecule. The EC50 for VEGF is much lower (75pM) than for the anti-VEGF monomer (12nM, not shown) that is fused as a variable domain to the heavy chain constant domain in the dual-specific molecule, and also lower than for the anti-VEGF monomer oligomerised by protein L cross-linking (line with data points shown as squares, 990pM).

The constructs of this embodiment are tetravalent, dual-specific antigen-binding polypeptide constructs comprising two copies of a V_{H} or V_{L} single domain antibody that binds a first epitope; and two copies of a V_{H} or V_{L} single domain antibody that binds a second epitope. Each of the two copies of the single domain antibody that binds the first epitope is fused to an IgG heavy chain constant domain, and each of the two copies of the single domain antibody that binds the second epitope is fused to a light chain constant domain.

Additional dual-specific, tetravalent polypeptide constructs similar to those described in this Example can be generated by one of skill in the art using, for example, other anti-TNF-α and anti-VEGF antibody sequences, e.g., any of those described herein. In other embodiments, C_{κ} or C_{λ} light chain constant domains can be used, and IgG heavy chain constant domains other than IgG1 can also be used. Of particular interest for use in the development into constructs of this sort are single domain anti-TNF-α antibody clones that prevent an increase in arthritic score when administered to a mouse of the Tg197 transgenic mouse model of arthritis as a dAb monomer, and single domain anti-VEGF antibody clones that prevent an increase in arthritic score when administered to a mouse of a collagen-induced arthritis mouse model as a dAb monomer. It is also preferred that the monomer of the single domain anti-TNF-α antibody clone neutralizes human TNF-α in the L929 cell cytotoxicity assay described herein, and that the monomer of the single domain anti-VEGF antibody clone antagonizes VEGF receptor binding in an assay of VEGF Receptor 2 binding as described herein. It is preferred that the single domain antibody clones used bind their respective epitopes with a K_{d} of < 100 nM. It is also preferred that such dual-specific, tetravalent constructs bind the respective epitopes with a K_{d} of <100 nM and prevent an increase in arthritic score in either or both of the Tg 197 and CIA models of arthritis described herein.

Such constructs can be used for the treatment of rheumatoid arthritis in a manner similar to the other constructs described herein, in terms of administration, dosage and monitoring of efficacy. The half-life of the construct can be modified as described herein above, e.g., by addition of a PEG moiety, and/or by further fusion of a binding moiety (e.g., a further single domain antibody) specific for a protein that increases circulating half-life, e.g., a serum protein such as HSA.

All publications, patents and published patent applications mentioned in the present specification, and references cited in said publications, are herein incorporated by reference. Various modifications and variations of the described methods and system of the invention will be apparent to those skilled in the art without departing from the scope and spirit of the invention. Although the invention has been described in connection with specific preferred embodiments, it should be understood that the invention as claimed should not be unduly limited to such specific embodiments. Indeed, various modifications of the described modes for carrying out the invention which are obvious to those skilled in molecular biology or related fields are intended to be within the scope of the following claims.

### Annex 1: polypeptides which enhance half-life in vivo.

Alpha-1 Glycoprotein (Orosomucoid) (AAG)
Alpha-1 Antichyromotrypsin (ACT)
Alpha-1 Antitrypsin (AAT)
Alpha-1 Microglobulin (Protein HC) (AIM)
Alpha-2 Macro globulin (A2M)
Antithrombin III (AT III)
Apolipoprotein A-1 (Apo A-1)
Apoliprotein B (Apo B)
Beta-2-microglobulin (β2M)
Ceruloplasmin (Cp)
Complement Component (C3)
Complement Component (C4)
C1 Esterase Inhibitor (C1 INH)
C-Reactive Protein (CRP)
Cystatin C (Cys C)
Ferritin (FER)
Fibrinogen (FIB)
Fibronectin (FN)
Haptoglobin (Hp)
Hemopexin (HPX)
Immunoglobulin A (IgA)
Immunoglobulin D (IgD)
Immunoglobulin E (IgE)
Immunoglobulin G (IgG)
Immunoglobulin M (IgM)
Immunoglobulin Light Chains (kapa/lambda)
Lipoprotein(a) [Lp(a)]
Mannose-bindign protein (MBP)
Myoglobin (Myo)
Plasminogen (PSM)
Prealbumin (Transthyretin) (PAL)
Retinol-binding protein (RBP)
Rheomatoid Factor (RF)
Serum Amyloid A (SAA)
Soluble Tranferrin Receptor (sTfR)
Transferrin (Tf)

### Annex 2

| **Pairing** | **Therapeutic relevant references.** |
|---|---|
| TNF ALPHA/TGF-β | • TGF-b and TNF when injected into the ankle joint of collagen induced arthritis model significantly enhanced joint inflammation. In non-collagen challenged mice there was no effect. |
| TNF ALPHA/IL-1 | • TNF and IL-1 synergize in the pathology of uveitis. |
| | • TNF and IL-1 synergize in the pathology of malaria (hypoglycaemia, NO). |
| | • TNF and IL-1 synergize in the induction of polymorphonuclear (PMN) cells migration in inflammation. |
| | • IL-1 and TNF synergize to induce PMN infiltration into the peritoneum. |
| | • IL-1 and TNF synergize to induce the secretion of IL-1 by endothelial cells. Important in inflammation. |
| | • IL-1 or TNF alone induced some cellular infiltration into knee synovium. |
| | • IL-1 induced PMNs, TNF - monocytes. Together they induced a more severe infiltration due to increased PMNs. |
| | • Circulating myocardial depressant substance (present in sepsis) is low levels of IL-1 and TNF acting synergistically. |
| TNF ALPHA/IL-2 | • Most relating to synergistic activation of killer T-cells. |
| TNF ALPHA/IL-3 | • Synergy of interleukin 3 and tumor necrosis factor alpha in stimulating clonal growth of acute myelogenous leukemia blasts is the result of induction of secondary hematopoietic cytokines by tumor necrosis factor alpha. Cancer Res. 1992 Apr 15;52(8):2197-201. |
| TNF ALPHA/IL-4 | • IL-4 and TNF synergize to induce VCAM expression on endothelial cells. Implied to have a role in asthma. Same for synovium - implicated in RA. |
| | • TNF and IL-4 synergize to induce IL-6 expression, in keratinocytes. |
| | • Sustained elevated levels of VCAM-1 in cultured fibroblast-like synoviocytes can be achieved by TNF-αlpha in combination with either IL-4 or IL-13 through increased mRNA stability. Am J Pathol. 1999 Apr;154(4):1149-58. |
| TNF ALPHA/IL-5 | • Relationship between the tumor necrosis factor system and the serum interleukin-4, interleukin-5, interleukin-8, eosinophil cationic protein, and immunoglobulin B levels in the bronchial hyperreactivity of adults and their children. Allergy Asthma Proc. 2003 Mar-Apr;24(2):111-8. |
| TNF ALPHA/IL-6 | • TNF and IL-6 are potent growth factors for OH-2, a novel human myeloma cell line. Eur J Haematol. 1994 Jul;53(1):31-7. |
| TNF ALPHA/IL-8 | • TNF and IL-8 synergized with PMNs to activate platelets. Implicated in Acute Respiratory Distress Syndrome. |
| | • See IL-5/TNF (asthma). Synergism between interleukin-8 and tumor necrosis factor-alpha for neutrophil-mediated platelet activation. Eur Cytokine Netw. 1994 Sep-Oct;5(5):455-60. (Adult respiratory distress syndrome (ARDS)) |
| TNF ALPHA/IL-9 | |
| TNF ALPHA/IL-10 | • IL-10 induces and synergizes with TNF in the induction of HIV expression in chronically infected T-cells. |
| TNF ALPHA/IL-11 | • Cytokines synergistically induce osteoclast differentiation: support by immortalized or normal calvarial cells. Am J Physiol Cell Physiol. 2002 Sep;283(3):C679-87. (Bone loss) |
| TNF ALPHA/IL-12 | |
| TNF ALPHA/IL-13 | • Sustained elevated levels of VCAM-1 in cultured fibroblast-like synoviocytes can be achieved by TNF-αlpha in combination with either IL-4 or IL-13 through increased mRNA stability. Am J Pathol. 1999 Apr;154(4):1149-58. |
| | • Interleukin-13 and tumour necrosis factor-alpha synergistically induce eotaxin production in human nasal fibroblasts. Clin Exp Allergy. 2000 Mar;30(3):348-55. |
| | • Interleukin-13 and tumour necrosis factor-alpha synergistically induce eotaxin production in human nasal fibroblasts. Clin Exp Allergy. 2000 Mar;30(3):348-55. (Allergic inflammation) |
| | • Implications of serum TNF-beta and IL-13 in the treatment response of childhood nephrotic syndrome. Cytokine. 2003 Feb 7;21(3):155-9. |
| TNF ALPHA/IL-14 | • Effects of inhaled tumour necrosis factor alpha in subjects with mild asthma. Thorax. 2002 Sep;57(9):774-8. |
| TNF ALPHA/IL-15 | • Effects of inhaled tumour necrosis factor alpha in subjects with mild asthma. Thorax. 2002 Sep;57(9):774-8. |
| TNF ALPHA/IL-16 | • Tumor necrosis factor-alpha-induced synthesis of interleukin-16 in airway epithelial cells: priming for serotonin stimulation. Am J Respir Cell Mol Biol. 2003 Mar;28(3):354-62. (Airway inflammation) |
| | • Correlation of circulating interleukin-16 with proinflammatory cytokines in patients with rheumatoid arthritis. Rheumatology (Oxford). 2001 Apr;40(4):474-5. No abstract available. |
| | • Interleukin 16 is up-regulated in Crohn's disease and participates in TNBS colitis in mice. Gastroenterology. 2000 Oct;119(4):972-82. |
| TNF ALPHA/IL-17 | • Inhibition of interleukin-17 prevents the development of arthritis in vaccinated mice challenged with Borrelia burgdorferi. Infect Immun. 2003 Jun;71(6):3437-42. |
| | • Interleukin-17 synergises with tumour necrosis factor alpha to induce cartilage destruction *in vitro.* Ann Rheum Dis. 2002 Oct;61(10):870-6. |
| | • A role of GM-CSF in the accumulation of neutrophils in the airways caused by IL-17 and TNF-αlpha. Eur Respir J. 2003 Mar;21(3):387-93. (Airway inflammation) |
| | • Abstract Interleukin-1, tumor necrosis factor alpha, and interleukin-17 synergistically up-regulate nitric oxide and prostaglandin E2 production in explants of human osteoarthritic knee menisci. Arthritis Rheum. 2001 Sep;44(9):2078-83. |
| TNP ALPHA/IL-18 | • Association of interleukin-18 expression with enhanced levels of both interleukin-1beta and tumor necrosis factor alpha in knee synovial tissue of patients with rheumatoid arthritis. Arthritis Rheum. 2003 Feb;48(2):339-47. |
| | • Abstract Elevated levels of interleukin-18 and tumor necrosis factor-alpha in serum of patients with type 2 diabetes mellitus: relationship with diabetic nephropathy. Metabolism. 2003 May;52(5):605-8. |
| TNF ALPHA/IL-19 | • Abstract IL-19 induces production of IL-6 and TNF-αlpha and results in cell apoptosis through TNF-αlpha. J Immunol. 2002 Oct 15;169(8):4288-97. |
| TNF ALPHA/IL-20 | • Abstract Cytokines: IL-20 - a new effector in skin inflammation. Curr Biol. 2001 Jul 10;11(13):R531-4. |
| TNF ALPHA/Complement | • Inflammation and coagulation: implications for the septic patient. Clin Infect Dis. 2003 May 15;36(10):1259-65. Epub 2003 May 08. Review. |
| TNF ALPHA/IFN-γ | • MNC induction in the brain. |
| | • Synergize in anti-viral response/IFN-β induction. |
| | • Neutrophil activation/respiratory burst. |
| | • Endothelial cell activation. |
| | • Toxicities noted when patients treated with TNF/IFN-γ as anti-viral therapy. |
| | • Fractalkine expression by human astrocytes. |
| | • Many papers on inflammatory responses - i.e. LIP, also macrophage activation. |
| | • Anti-TNF and anti-IFN-γ synergize to protect mice from lethal endotoxemia. |
| TGF-β/IL-1 | • Prostaglandin synthesis by osteoblasts. |
| | • IL-6 production by intestinal epithelial cells (inflammation model). |
| | • Stimulates IL-11 and IL-6 in lung fibroblasts (inflammation model). |
| | • IL-6 and IL-8 production in the retina. |
| TGF-β/IL-6 | • Chondrocarcoma proliferation. |
| IL-1/IL-2 | • B-cell activation. |
| | • LAK cell activation. |
| | • T-cell activation. |
| | • IL-1 synergy with IL-2 in the generation of lymphokine activated killer cells is mediated by TNF-αlpha and beta (lymphotoxin). Cytokine. 1992 Nov;4(6):479-87. |
| IL-1/IL-3 | |
| IL-1/IL-4 | • B-cell activation. |
| | • IL-4 induces IL-1 expression in endothelial cell activation. |
| IL-1/IL-5 | |
| IL-1/IL-6 | • B-cell activation. |
| | • T-cell activation (can replace accessory cells). |
| | • IL-1 induces IL-6 expression |
| | • C3 and serum amyloid expression (acute phase response). |
| | • HIV expression. |
| | • Cartilage collagen breakdown. |
| IL-1/IL-7 | • IL-7 is requisite for IL-1-induced thymocyte proliferation. Involvement of IL-7 in the synergistic effects of granulocyte-macrophage colony-stimulating factor or tumor necrosis factor with IL-1. J Immunol. 1992 Jan 1;148(1):99-105. |
| IL-1/IL-8 | |
| IL-1/IL-10 | |
| IL-1/IL-11 | • Cytokines synergistically induce osteoclast differentiation: support by immortalized or normal calvanal cells. Am J Physiol Cell Physiol. 2002 Sep;283(3):C679-87. (Bone loss) |
| IL-1/IL-16 | • Correlation of circulating interleukin 16 with proinflammatory cytokines in patients with rheumatoid arthritis. Rheumatology (Oxford). 2001 Apr;40(4):474-5. No abstract available. |
| IL-1/IL-17 | • Inhibition of interleukin-17 prevents the development of arthritis in vaccinated mice challenged with Borrelia burgdorferi. Infect Immun. 2003 Jun;71(6):3437-42. |
| | • Contribution of interleukin 17 to human cartilage degradation and synovial inflammation in osteoarthritis. Osteoarthritis Cartilage. 2002 Oct;10(10):799-807. |
| | • Abstract Interleukin-1, tumor necrosis factor alpha, and interleukin-17 synergistically up-regulate nitric oxide and prostaglandin E2 production in explants of human osteoarthritic knee menisci. Arthritis Rheum. 2001 Sep;44(9):2078-83. |
| IL-1/IL-18 | • Association of interleukin-18 expression with enhanced levels of both interleukin-lbeta and tumor necrosis factor alpha in knee synovial tissue of patients with rheumatoid arthritis. Arthritis Rheum. 2003 Feb;48(2):339-47. |
| IL-1/IFN-g | |
| IL-2/IL-3 | • T-cell proliferation. |
| | • B cell proliferation. |
| IL-2/IL-4 | • B-cell proliferation. |
| | • T-cell proliferation. |
| | • (Selectively inducing activation of CD8 and NK lymphocytes) IL-2R beta agonist P1-30 acts in synergy with IL-2, IL-4, IL-9, and IL-15: biological and molecular effects. J lmmunol. 2000 Oct 15;165(8):4312-8. |
| IL-2/IL-5 | • B-cell proliferation/ Ig secretion. |
| | • IL-5 induces IL-2 receptors on B-cells. |
| IL-2/IL-6 | • Development of cytotoxic T-cells. |
| IL-2/IL-7 | |
| IL-2/IL-9 | • See IL-2/IL-4 (NK-cells). |
| IL-2/IL-10 | • B-cell activation. |
| IL-2/IL-12 | • IL-12 synergizes with IL-2 to induce lymphokine-activated cytotoxicity and perform and granzyme gene expression in fresh human NK cells. Cell Immunol. 1995 Oct 1;165(1):33-43. (T-cell activation) |
| IL-2/IL-15 | • See IL-2/IL-4 (NK cells). |
| | • (T cell activation and proliferation) IL-15 and IL-2: a matter of life and death for T cells *in vivo.* Nat Med. 2001 Jan;7(1):114-8. |
| IL-2/IL-16 | • Synergistic activation of CD4+ T cells by IL-16 and IL-2. J Immunol. 1998 Mar 1;160(5):2115-20. |
| IL-2/IL-17 | • Evidence for the early involvement of interleukin 17 in human and experimental renal allograft rejection. J Pathol. 2002 Jul;197(3):322-32. |
| IL-2/IL-18 | • Interleukin 18 (IL-18) in synergy with IL-2 induces lethal lung injury in mice: a potential role for cytokines, chemokines, and natural killer cells in the pathogenesis of interstitial pneumonia. Blood. 2002 Feb 15;99(4):1289-98. |
| IL-2/TGF-β | • Control of CD4 effector fate: transforming growth factor beta 1 and interleukin 2 synergize to prevent apoptosis and promote effector expansion. J Exp Med. 1995 Sep 1; 182(3):699-709. |
| IL-2/IFN-γ | • Ig secretion by B-cells. |
| | • IL-2 induces IFN-γ expression by T-cells. |
| IL-2/IFN-α/β | • None. |
| IL-3/IL-4 | • Synergize in mast cell growth. |
| | • Synergistic effects of IL-4 and either GM-CSF or IL-3 on the induction of CD23 expression by human monocytes: regulatory effects of IFN-alpha and IFN-gamma. Cytokine. 1994 Jul;6(4):407-13. |
| IL-3/IL-5 | |
| IL-3/IL-6 | |
| IL-3/IFN-γ | • IL-4 and IFN-gamma synergistically increase total polymeric IgA receptor levels in human intestinal epithelial cells. Role of protein tyrosine kinases. J Immunol. 1996 Jun 15;156(12):4807-14. |
| IL-3/GM-CSF | • Differential regulation of human eosinophil IL-3, IL-5, and GM-CSF receptor alpha-chain expression by cytokines: IL-3, IL-5, and GM-CSF down-regulate IL-5 receptor alpha expression with loss of IL-5 responsiveness, but up-regulate IL-3 receptor alpha expression. J Immunol. 2003 Jun 1; 170(11):5359-66. (Allergic inflammation) |
| IL-4/IL-2 | • IL-4 synergistically enhances both IL-2- and IL-12-induced IFN-{gamma} expression in murine NK cells. *Blood.* 2003 Mar 13. [Epub ahead of print] |
| IL-4/IL-5 | • Enhanced mast cell histamine etc. secretion in response to IgE. |
| | • A Th2-like cytokine response is involved in bullous pemphigoid. The role of IL-4 and IL-S in the pathogenesis of the disease. Int J Immunopathol Pharmacol. 1999 May-Aug;12(2):55-61. |
| IL-4/IL-6 | |
| IL-4/IL-10 | |
| IL-4/IL-11 | • Synergistic interactions between interleukin-11 and interleukin-4 in support of proliferation of primitive hematopoietic progenitors of mice. Blood. 1991 Sep 15; 78(6):1448-51. |
| IL-4/IL-12 | • Synergistic effects of IL-4 and IL-18 on IL-12-dependent IFN-gamma production by dendritic cells. J Immunol. 2000 Jan 1;164(1):64-71. (Increase Th1/Th2 differentiation) |
| | • IL-4 synergistically enhances both IL-2- and IL-12-induced IFN-{gamma} expression in murine NK cells. *Blood.* 2003 Mar 13. [Epub ahead of print] |
| IL-4/IL-13 | • Abstract Interleukin-4 and interleukin-13 signaling connections maps. Science. 2003 Jun 6;300(5625):1527-8. (Allergy, asthma) |
| | • Inhibition of the IL-4/IL-13 receptor system prevents allergic sensitization without affecting established allergy in a mouse model for allergic asthma. J Allergy Clin Immunol. 2003 Jun;111(6):1361-1369. |
| IL-4/IL-16 | • (Asthma) Interleukin (IL)-4/IL-9 and exogenous IL-16 induce IL-16 production by BEAS-2B cells, a bronchial epithelial cell line. Cell Immunol. 2001 Feb 1;207(2):75-80. |
| IL-4/IL-17 | • Interleukin (IL)-4 and IL-17 synergistically stimulate IL-6 secretion in human colonic myofibroblasts. Int J Mol Med. 2002 Nov;10(5):631-4. (Gut inflammation) |
| IL-4/IL-24 | • IL-24 is expressed by rat and human macrophages. Immunobiology. 2002 Jul;205(3):321-34. |
| IL-4/IL-25 | • Abstract New IL-17 family members promote Th1 or Th2 responses in the lung: in vivo function of the novel cytokine IL-25. J Immunol. 2002 Jul 1;169(1):443-53. (Allergic inflammation) |
| | • Abstract Mast cells produce interleukin-25 upon Fcepsilon RI-mediated activation. Blood. 2003 May 1;101(9):3594-6. Epub 2003 Jan 02. (Allergic inflammation) |
| IL-4/IFN-γ | • Abstract Interleukin 4 induces interleukin 6 production by endothelial cells: synergy with interferon-gamma. Eur J Immunol. 1991 Jan;21(1):97-101. |
| IL-4/SCF | • Regulation of human intestinal mast cells by stem cell factor and IL-4. Immunol Rev. 2001 Feb;179:57-60. Review. |
| IL-5/IL-3 | • Differential regulation of human eosinophil IL-3, IL-5, and GM-CSF receptor alpha-chain expression by cytokines: IL-3, IL-5, and GM-CSF down-regulate IL-5 receptor alpha expression with loss of IL-5 responsiveness, but up-regulate IL-3 receptor alpha expression. J Immunol. 2003 Jun 1; 170(11):5359-66. (Allergic inflammation see abstract) |
| IL-5/IL-6 | |
| IL-5/IL-13 | • Inhibition of allergic airways inflammation and airway hyperresponsiveness in mice by dexamethasone: role of eosinophils, IL-5, eotaxin, and IL-13. J Allergy Clin Immunol. 2003 May;111(5):1049-61. |
| IL-5/IL-17 | • Interleukin-17 orchestrates the granulocyte influx into airways after allergen inhalation in a mouse model of allergic asthma. Am J Respir Cell Mol Biol. 2003 Jan; 28(1):42-50. |
| IL-5/IL-25 | • Abstract New IL-17 family members promote Th1 or Th2 responses in the lung: in vivo function of the novel cytokine IL-25. J Immunol. 2002 Jul 1;169(1):443-53. (Allergic inflammation) |
| | • Abstract Mast cells produce interleukin-25 upon Fcepsilon RI-mediated activation. Blood. 2003 May 1;101(9):3594-6. Epub 2003 Jan 02. (Allergic inflammation) |
| IL-5/IFN-γ | |
| IL-5/GM-CSF | • Differential regulation of human eosinophil IL-3, IL-5, and GM-CSF receptor alpha-chain expression by cytokines: IL-3, IL-5, and GM-CSF down-regulate IL-5 receptor alpha expression with loss of IL-5 responsiveness, but up-regulate IL-3 receptor alpha expression. J Immunol. 2003 Jun 1; 170(11):5359-66. (Allergic inflammation) |
| IL-6/IL-10 | |
| IL-6/IL-11 | |
| IL-6/IL-16 | • Interleukin-16 stimulates the expression and production of pro-inflammatory cytokines by human monocytes. Immunology. 2000 May;100(1):63-9. |
| IL-6/IL-17 | • Stimulation of airway mucin gene expression by interleukin (IL)-17 through IL-6 paracrine/autocrine loop. J Biol Chem. 2003 May 9;278(19):17036-43. Epub 2003 Mar 06. (Airway inflammation, asthma) |
| IL-6/IL-19 | • Abstract IL-19 induces production of IL-6 and TNF-αlpha and results in cell apoptosis through TNF-αlpha. J Immunol. 2002 Oct 15;169(8):4288-97. |
| IL-6/IFN-g | |
| IL-7/IL-2 | • Interleukin 7 worsens graft-versus-host disease. Blood. 2002 Oct 1;100(7):2642-9. |
| IL-7/IL-12 | • Synergistic effects of IL-7 and IL-12 on human T cell activation. J Immunol. 1995 May 15;154(10):5093-102. |
| IL-7/IL-15 | • Interleukin-7 and interleukin-15 regulate the expression of the bc1-2 and c-myb genes in cutaneous T-cell lymphoma cells. Blood. 2001 Nov 1;98(9):2778-83. (Growth factor) |
| IL-8/IL-11 | • Abnormal production of interleukin (IL)-11 and IL-8 in polycythaemia vera. Cytokine. 2002 Nov 21;20(4):178-83. |
| IL-8/IL-17 | • The Role of IL-17 in Joint Destruction. Drug News Perspect. 2002 Jan;15(1):17-23. (Arthritis) |
| | • Abstract Interleukin-17 stimulates the expression of interleukin-8, growth-related oncogene-alpha, and granulocyte-colony-stimulating factor by human airway epithelial cells. Am J Respir Cell Mol Biol. 2002 Jun; 26(6):748-53. (Airway inflammation) |
| IL-8/GSF | • Interleukin-8: an autocrine/paracrine growth factor for human hematopoietic progenitors acting in synergy with colony stimulating factor 1 to promote monocyte-macrophage growth and differentiation. Exp Hematol. 1999 Jan;27(1):28-36. |
| IL-8/VGEF | • Intracavitary VEOF, bFGF, IL-8, IL-12 levels in primary and recurrent malignant glioma. J Neurooncol. 2003 May; 62(3):297-303. |
| IL-9/IL-4 | • Anti-interleukin-9 antibody treatment inhibits airway inflammation and hyperreactivity in mouse asthma model. Am J Respir Crit Care Med. 2002 Aug 1;166(3):409-16. |
| IL-9/IL-5 | • Pulmonary overexpression of IL-9 induces Th2 cytokine expression, leading to immune pathology. J Clin Invest. 2002 Jan;109(1):29-39. |
| | • Th2 cytokines and asthma. Interleukin-9 as a therapeutic target for asthma. Respir Res. 2001;2(2):80-4. Epub 2001 Feb 15. Review. |
| | • Abstract Interleukin-9 enhances interleukin-5 receptor expression, differentiation, and survival of human eosinophils. Blood. 2000 Sep 15;96(6):2163-71. (Asthma) |
| IL-9/IL-13 | • Anti-interleukin-9 antibody treatment inhibits airway inflammation and hyperreactivity in mouse asthma model. Am J Respir Crit Care Med. 2002 Aug 1;166(3):409-16. |
| | • Direct effects of interleukin-13 on epithelial cells cause airway hyperreactivity and mucus overproduction in asthma. Nat Med. 2002 Aug;8(8):885-9. |
| IL-9/IL-16 | • See IL-4/IL-16. |
| IL-10/IL-2 | • The interplay of interleukin-10 (IL-10) and interleukin-2 (IL-2) in humoral immune responses: IL-b synergizes with IL-2 to enhance responses of human B lymphocytes in a mechanism which is different from upregulation of CD25 expression. Cell Immunol. 1994 Sep;157(2):478-88. |
| IL-10/IL-12 | |
| IL-10/TGF-β | • IL-10 and TGF-beta cooperate in the regulatory T cell response to mucosal allergens in normal immunity and specific immunotherapy. Eur J Immunol. 2003 May; 33(5):1205-14. |
| IL-10/INF-γ | |
| IL-11/IL-6 | • Interleukin-6 and interleukin-11 support human osteoclast formation by a RANKL-independent mechanism. Bone. 2003 Jan;32(1):1-7. (Bone resorption in inflammation) |
| IL-11/IL-17 | • Polarized in vivo expression of IL-11 and IL-17 between acute and chronic skin lesions. J Allergy Clin Immunol. 2003 Apr;111(4):875-81. (Allergic dermatitis) |
| | • IL-17 promotes bone erosion in murine collagen-induced arthritis through loss of the receptor activator of NP-kappa B ligand/osteoprotegerin balance. J Immunol. 2003 Mar 1; 170(5):2655-62. |
| IL-11/TGF-β | • Polarized in vivo expression of IL-11 and IL-17 between acute and chronic skin lesions. J Allergy Clin Immunol. 2003 Apr;111(4):875-81. (Allergic dennatitis) |
| IL-12/IL-13 | • Relationship of Interleukin-12 and Interleukin-13 imbalance with class-specific rheumatoid factors and anticardiolipin antibodies in systemic lupus erythematosus. Clin Rheumatol. 2003 May;22(2):107-11. |
| IL-12/IL-17 | • Upregulation of interleukin-12 and -17 in active inflammatory bowel disease. Scand J Gastroenterol. 2003 Feb;38(2):180-5. |
| IL-12/IL-18 | • Synergistic proliferation and activation of natural killer cells by interleukin 12 and interleukin 18. Cytokine. 1999 Nov; 11(11):822-30. |
| | • Inflammatory Liver Steatosis Caused by IL-12 and IL-18*.* J Interferon Cytokine Res. 2003 Mar;23(3):155-62. |
| IL-12/IL-23 | • Interleukin-23 rather than interleukin-12 is the critical cytokine for autoimmune inflammation of the brain. Nature. 2003 Feb 13;421(6924):744-8. |
| | • Abstract A unique role for IL-23 in promoting cellular immunity. J Leukoc Biol. 2003 Jan;73(1):49-56. Review. |
| IL-12/IL-27 | • Abstract IL-27, a heterodimetic cytokine composed of EBI3 and p28 protein, induces proliferation of naive CD4(+) T cells. Immunity. 2002 Jun;16(6):779-90. |
| IL-12/IFN-γ | • IL-12 induces IFN-γ expression by B and T-cells as part of immune stimulation. |
| IL-13/IL-5 | • See IL-5/IL-13. |
| IL-13/IL-25 | • Abstract New IL-17 family members promote Th1 or Th2 responses in the lung: in vivo function of the novel cytokine IL-25. J Immunol. 2002 Jul 1;169(1):443-53. (Allergic inflammation) |
| | • Abstract Mast cells produce interleukin-25 upon Fcepsilon RI-mediated activation. Blood. 2003 May 1;101(9):3594-6. Epub 2003 Jan 02. (Allergic inflammation) |
| IL-15/IL-13 | • Differential expression of interleukins (IL)-13 and IL-15 in ectopic and eutopic endometrium of women with endometriosis and normal fertile women. Am J Reprod Immunol. 2003 Feb;49(2):75-83. |
| IL-15/IL-16 | • IL-15 and IL-16 overexpression in cutaneous T-cell lymphomas: stage-dependent increase in mycosis fungoides progression. Exp Dermatol. 2000 Aug;9(4):248-51. |
| IL-15/IL-17 | • Abstract IL-17, produced by lymphocytes and neutrophils, is necessary for lipopolysaccharide-induced airway neutrophilia: IL-15 as a possible trigger. J lmmunol. 2003 Feb 15;170(4):2106-12. (Airway inflammation) |
| IL-15/IL-21 | • IL-21 in Synergy with IL-15 or IL-18 Enhances IFN-gamma Production in Human NK and T Cells. J Immunol. 2003 Jun 1;170(11):5464-9. |
| IL-17/IL-23 | • Interleukin-23 promotes a distinct CD4 T cell activation state characterized by the production of interleukin-17. J Biol Chem. 2003 Jan 17;278(3):1910-4. Epub 2002 Nov 03. |
| IL-17/TGF-β | • Polarized in vivo expression of IL-11 and IL-17 between acute and chronic skin lesions. J Allergy Clin Immunol. 2003 Apr;111(4):875-81. (Allergic dermatitis) |
| IL-18/IL-12 | • Synergistic proliferation and activation of natural killer cells by interleukin 12 and interleukin 18. Cytokine. 1999 Nov; 11(11):822-30. |
| | • Abstract Inhibition of in vitro immunoglobulin production by 11-12 in murine chronic graft-vs.-host disease: synergism with IL-18. Eur J Immunol. 1998Jun;28(6):2017-24. |
| IL-18/IL-21 | • IL-21 in Synergy with IL-15 or IL-18 Enhances IFN-gamma Production in Human NK and T Cells. J Immunol. 2003 Jun 1;170(11):5464-9. |
| IL-18/TGF-β | • Interleukin 18 and transforming growth factor beta 1 in the serum of patients with Graves' ophthalmopathy treated with corticosteroids. Int Immunopharmacol. 2003 Apr;3(4):549-52. |
| IL-18/IFN-γ | |
| Anti-TNF ALPHA/anti-CD4 | • Synergistic therapeutic effect in DBA/1 arthritic mice. |

### Annex 3: Oncology combinations

| **Target** | **Disease** | **Pair with** |
|---|---|---|
| CD89* | Use as cytotoxic cell recruiter | all |
| | | |
| CD19 | B cell lymphomas | HLA-DR |
| | | CD5 |
| HLA-DR | B cell lymphomas | CD89 |
| | | CD19 |
| | | CD5 |
| CD38 | Multiple myeloma | CD138 |
| | | CD56 |
| | | HLA-DR |
| CD138 | Multiple myeloma | CD38 |
| | | CD56 |
| | | HLA-DR |
| CD138 | Lung cancer | CD56 |
| | | CEA |
| CD33 | Acute myelod lymphoma | CD34 |
| | | HLA-DR |
| CD56 | Lung cancer | CD138 |
| | | CEA |
| CEA | Pan carcinoma | MET receptor |
| VEGF | Pan carcinoma | MET receptor |
| VEGF receptor | Pan carcinoma | MET receptor |
| IL-13 | Asthma/pulmonary inflammation | IL-4 |
| | | IL-5 |
| | | Eotaxin(s) |
| | | MDC |
| | | TARC |
| | | TNFα |
| | | IL-9 |
| | | EGFR |
| | | CD40L |
| | | IL-25 |
| | | MCP-1 |
| | | TGFβ |
| IL-4 | Asthma | IL-13 |
| | | IL-5 |
| | | Eotaxin(s) |
| | | MDC |
| | | TARC |
| | | TNFα |
| | | IL-9 |
| | | EGFR |
| | | CD40L |
| | | IL-25 |
| | | MCP-1 |
| | | TGFβ |
| Eotaxin | Asthma | IL-5 |
| | | Eotaxin-2 |
| | | Eotaxin-3 |
| EGFR | Cancer | HER2/neu |
| | | HER3 |
| | | HER4 |
| HER2 | Cancer | HER3 |
| | | HER4 |
| TNFR1 | RA/Crohn's disease | IL-1R |
| | | IL-6R |
| | | IL-18R |
| TNFα | RA/Crohn's disease | IL-1α/β |
| | | IL-6 |
| | | IL-18 |
| | | ICAM-1 |
| | | IL-15 |
| | | IL-17 |
| IL-1R | RA/Crohn's disease | IL-6R |
| | | IL-18R |
| IL-18R | RA/Crohn's disease | IL-6R |

### Annex 4:

**Data Summary**

| **TARGET** | **dAb** | **Equilibrium dissociation constant (Kd = Koff/Kon)** | **Koff** | **IC50 for ligand assay** | **ND50 for cell based neutralism assay** |
|---|---|---|---|---|---|
| TAR1 | TAR1 monomers | 300nM to 5pM (ie, 3 x 10⁻⁷ to 5 x 10⁻¹²), preferably 50nM to 20pM | 5 x 10⁻¹ to 1 x 10⁻⁷ | 500nM to 100pM | 500nM to 50pM |
| | TAR 1 dimers | As TAR 1 monomer | As TAR 1 monomer | As TAR 1 monomer | As TAR 1 monomer |
| | TAR1 trimers | As TAR 1 monomer | As TAR 1 monomer | As TAR 1 monomer | As TAR 1 monomer |
| | TAR1-5 | | | | |
| | TAR1-27 | | | | |
| | TAR1-5-19 monomer | 30nM | | | |
| | TAR1-5-19 homodimer | | | With (Gly₄Ser)₃ linker = 20nm | 30nM |
| | | | | With (Gly₄Ser)₅ linker = 2nm | =3nM |
| | | | | With (Gly₄Ser)₇ linker = 10nm | =15nM |
| | | | | In Fab format = 1nM | |
| | TAR1-5-19 heterodimers | | | With (Gly₄Ser)ₙ linker TAR1-5-19 d2 = 2nM | = 12nM |
| | | | | TAR1-5-19 d3 8nM | = 10nM |
| | | | | TAR1-5-19 d4 = 2-5nM | |
| | | | | TAR1-5-19 d5 = 8nM | |
| | | | | In Fab format | = 12nM |
| | | | | TAR1-5-19CH | |
| | | | | d1CK = 6nM | |
| | | | | TAR1-5-19CK | |
| | | | | d1CH = 6nM | |
| | | | | TAR1-5-19CH | |
| | | | | d2CK = 8nM | |
| | | | | TAR1-5-19CH | |
| | | | | d3CK = 3nM | |
| | TAR1-5 heterodimers | | | With (Gly₄Ser)ₙ linker | |
| | | | | TAR1-5d1 = 30nM | |
| | | | | TAR1-5d2 = 50nM | |
| | | | | TAR1-Sd3 = 300nM | |
| | | | | TAR1-5d4 = 3nM | |
| | | | | TAR1-5d5 = 200nM | |
| | | | | TAR1-5d6 = 100nM | = 60nM |
| | | | | In Fab format | |
| | | | | TAR1-5CH | |
| | | | | d2CK = 30nM | |
| | | | | TAR1-5CK | |
| | | | | d3 CH = 100nM | |
| | TAR1-5-19 homotrimer | | | 0.3nM | 3-10nM (eg, 3nM) |
| TAR2 | TAR2 monomers | As TAR1 monomer | As TAR1 monomer | 500nM to 100pM | 500nM to 50pM |
| | TAR2-10 | | | | |
| | TAR2-5 | | | | |
| Serum Albumin | Anti-SA monomers | 1mM to 500µM, preferably 100nM to 10,uM | | 1nM to 500µM, preferably 100nM to 10µM | |
| | | In Dual Specific format, target affinity is 1 to 100,000 x affinity of SA dAb affinity, eg 100pM (target) and 10 µM SA affinity. | | In Dual Specific format, target affinity is 1 to 100,000 x affinity of SA dAb affinity, eg 100pM (target) and 10 µM SA affinity. | |
| | MSA-16 | 200nM | | | |
| | MSA-26 | 70nM | | | |

## Claims

1. Use of a composition comprising a single domain antibody polypeptide construct that antagonizes human TNFα's binding to a TNFα receptor in vitro for the preparation of a medicament for the inhibition of progression of a TNFα-related inflammatory disorder in an individual suffering from such a disorder, wherein the composition provides a single domain antibody polypeptide construct having a tβ half-life of at least 2.5 hours.

2. Use according to claim 1 wherein the TNFα-related inflammatory disorder is selected from inflammation, allergic hypersensitivity, cancer, bacterial or viral infection and an autoimmune disorder.

3. Use according to claim 2 wherein the autoimmune disorder is selected from Type I diabetes, multiple sclerosis, rheumatoid arthritis, systemic lupus erythematosus, Crohn's disease, inflammatory bowel disease and myasthenia gravis.

4. Use according to any preceding claim for inhibition of progression of rheumatoid arthritis.

5. Use according to any one of claims 1 to 3 for inhibition of progression of Crohn's disase or inflammatory bowel disease.

6. Use according to any preceding claim wherein the single domain antibody polypeptide construct has a tβ half-life of at least 12 hours.

7. Use according to any preceding claim wherein the tβ half-life is in the range of up to and including 21 days.

8. Use according to any preceding claim wherein the tβ half-life is in the range of up to and including 15 days.

9. The use of any preceding claim, wherein said composition has an efficacy in the Tg197 transgenic mouse arthritis assay that is greater than or equal to that of an agent selected from the group consisting of Etanercept, Infliximab and D2E7.

10. The use of any preceding claim, wherein said composition prevents an increase in arthritic score when administered to a mouse of the Tg197 transgenic mouse model of arthritis.

11. The use of claim 10, wherein said administering results in a statistically significant change in one or more indicia of RA.

12. The use of claim 11, wherein said one or more indicia of RA comprise one or more of erythrocyte sedimentation rate (ESR), Ritchie articular index and duration of morning stiffness, joint mobility, joint swelling, x ray imaging of one or more joints, and histopathological analysis of fixed sections of one or more joints.

13. The use of claim 11, wherein said one or more indicia of RA comprises a decrease in the macrophenotypic signs of arthritis in a Tg197 transgenic mouse, wherein said composition is administered to a Tg197 transgenic mouse, wherein said Tg197 transgenic mouse is scored for said macrophenotypic signs of arthritis,
and wherein said macrophenotypic signs of arthritis are scored according to the following system: 0 = no arthritis (normal appearance and flexion), 1 = mild arthritis (joint distortion), 2 = moderate arthritis (swelling, joint deformation), 3 = heavy arthritis (severely impaired movement).

14. The use of Claim 11, wherein said one or more indicia of RA comprises a decrease in the histopathological signs of arthritis in a Tg197 transgenic mouse, wherein said composition is administered to a Tg197 transgenic mouse, wherein said Tg197 transgenic mouse is scored for said histopathological signs of arthritis, and
wherein said histopathological signs of arthritis are performed on a joint and scored using the following system: 0= no detectable pathology, 1= hyperplasia of the synovial membrane and presence of polymorphonuclear infiltrates, 2= pannus and fibrous tissue formation and focal subchondral bone erosion, 3= articular cartilage destruction and bone erosion, 4= extensive articular cartilage destruction and bone erosion.

15. The use of claim 10, wherein the administration of said composition to a Tg197 transgenic mouse comprises the following steps:
a) administer weekly intraperitoneal injections of said composition to a heterozygous Tg197 transgenic mouse,
b) weigh the mouse of step a) weekly, and
c) score said mouse weekly for macrophenotypic signs of arthritis according to the following system: 0 = no arthritis (normal appearance and flexion), 1 = mild arthritis (joint distortion), 2 = moderate arthritis (swelling, joint deformation), 3 = heavy arthritis (severely impaired movement).

16. The use of claim 10, wherein said composition is first administered when said mouse is three weeks of age.

17. The use of claim 10, wherein said composition is first administered when said mouse is six weeks of age

18. The use of any preceding claim, wherein said composition has an efficacy in the Tg197 transgenic mouse arthritis assay , such that the treatment results in an arthritic score of 0 to 0.5.

19. The use of any preceding claim, wherein said composition has an efficacy in the Tg197 transgenic mouse arthritis assay , such that the treatment results in an arthritic score of 0 to 1.0.

20. The use of any preceding claim, wherein said composition has an efficacy in the Tg197 transgenic mouse arthritis assay , such that the treatment results in an arthritic score of 0 to 1.5.

21. The use of any preceding claim, wherein said composition has an efficacy in the Tg197 transgenic mouse arthritis assay , such that the treatment results in an arthritic score of 0 to 2.0.

22. The use of any preceding claim, wherein said single domain antibody polypeptide construct comprises a human single domain antibody polypeptide.

23. The use of any preceding claim, wherein said human single domain antibody polypeptide binds TNFα.

24. The use of any preceding claim, wherein said single domain antibody polypeptide construct binds human TNFα with a Kd of < 100 nM.

25. The use of any preceding claim, wherein said single domain antibody polypeptide construct binds human TNFα with a K_{d} in the range of 100 nM to 50 pM.

26. The use of any preceding claim, wherein said single domain antibody polypeptide construct binds human TNFα with a K_{d} of 30 nM to 50 pM.

27. The use of any preceding claim, wherein said single domain antibody polypeptide construct binds human TNFα with a K_{d} of 10 nM to 50 pM.

28. The use of any preceding claim, wherein said single domain antibody polypeptide construct binds human TNFα with a K_{d} in the range of 1 nm to 50 pM.

29. The use of any preceding claim, wherein said single domain antibody polypeptide construct antagonizes human TNFα as measured in a standard L929 cell assay.

30. The use of any preceding claim, wherein said single domain antibody polypeptide construct specifically binds to human TNF-α which is bound to a TNF-α receptor.

31. The use of any preceding claim, wherein said single domain antibody polypeptide construct has an in vivo tα-half life in the range of 15 minutes to 12 hours.

32. The use of any preceding claim, wherein said single domain antibody polypeptide construct has an in vivo tα-half life in the range of 1 to 6 hours.

33. The use of any preceding claim, wherein said single domain antibody polypeptide construct has an in vivo tα-half life in the range of 2 to 5 hours.

34. The use of any preceding claim, wherein said single domain antibody polypeptide construct has an in vivo tα-half life in the range of 3 to 4 hours.

35. The use of any preceding claim, wherein said single domain antibody polypeptide construct has an in vivo AUC half-life value of 15 mg.min/ml to 150 mg.min/ml.

36. The use of any preceding claim, wherein said single domain antibody polypeptide construct has an in vivo AUC half-life value of 15 mg.min/ml to 100 mg.min/ml.

37. The use of any preceding claim, wherein said single domain antibody polypeptide construct has an in vivo AUC half-life value of 15 mg.min/ml to 75 mg.min/ml.

38. The use of any preceding claim, wherein said single domain antibody polypeptide construct has an in vivo AUC half-life value of 15 mg.min/ml to 50 mg.min/ml.

39. The use of any preceding claim, wherein said single domain antibody polypeptide construct is linked to a PEG molecule.

40. The use of claim 39, wherein the PEG-linked single domain antibody polypeptide construct has a hydrodynamic size of at least 24 kDa, and wherein the total PEG size is from 20 to 60 kDa.

41. The use of claim 39, wherein the PEG-linked single domain antibody polypeptide construct has a hydrodynamic size of at least 200 kDa and a total PEG size of from 20 to 60 kDa.

42. The use of claim 39, wherein the PEG-linked proteins are linked, on average, to 1-20 polyethylene glycol molecules.

43. The or use of any preceding claim, wherein said antibody construct comprises two or more single immunoglobulin variable domain polypeptides that bind human TNFα.

44. The use of any preceding claim, wherein said antibody construct comprises a homodimer of a single immunoglobulin variable domain polypeptide that binds human TNFα.

45. The use of any preceding claim, wherein said antibody construct comprises a homotrimer of a single immunoglobulin variable domain polypeptide that binds human TNFα.

46. The use of any preceding claim, wherein said antibody construct comprises a homotetramer of a single immunoglobulin variable domain polypeptide that binds human TNFα.

47. The use of any preceding claim, wherein said antibody construct further comprises an antibody polypeptide specific for an antigen other than TNFα.

48. The use of claim 47, wherein said antibody polypeptide specific for an antigen other than TNFα comprises a single domain antibody polypeptide.

49. The use of claim 47 or 48, wherein the binding of said antigen other than TNFα by said antibody polypeptide specific for an antigen other than TNFα increases the in vivo half-life of said antibody polypeptide construct.

50. The use of claims 47-49, wherein said antigen other than TNFα comprises a serum protein.

51. The use of claim 50, wherein said serum protein is selected from the group consisting of fibrin, α-2 macroglobulin, serum albumin, fibrinogen A, fibrinogen, serum amyloid protein A, heptaglobin, protein, ubiquitin, uteroglobulin and β-2- microglobulin.

52. The use of claim 47, wherein said antigen other than TNFα comprises HSA.

53. The use of any preceding claim, wherein at least one additional therapeutic agent is administered together with the medicament.

54. The use of any preceding claim, wherein said single domain antibody polypeptide construct comprises the amino acid sequence of CDR3 of an antibody polypeptide selected from the group consisting of clones TAR1-2m-9, TAR1-2m-30, TAR1-2m-1, TAR1-2m-2, TAR1-5, TAR1-27, TAR1-261, TAR1-398, TAR1-701, TAR1-5-2, TAR1-5-3, TAR1-5-4, TAR1-5-7, TAR1-5-8, TAR1-5-10, TAR1-5-11, TAR1-5-12, TAR1-5-13, TAR1-5-19, TAR1-5-20, TAR1-5-21, TAR1-5-22, TAR1-5-23, TAR1-5-24, TAR1-5-25, TAR1-5-26, TAR1-5-27, TAR1-5-28, TAR1-5-29, TAR1-5-34, TAR1-5-35, TAR1-5-36, TAR1-5-464, TAR1-5-463, TAR1-5-460, TAR1-5-461, TAR1-5-479, TAR1-5-477, TAR1-5-478, TAR1-5-476, TAR1-5-490, TAR1h-1, TAR1h-2, TAR1h-3, TAR1-100-29, TAR1-100-35, TAR1-100-43, TAR1-100-47, TAR1-100-52, TAR1-109, TAR1-100, TAR1-100-34, TAR1-100-36, TAR1-100-38, TAR1-100-39, TAR1-100-40, TAR1-100-41, TAR1-100-45, TAR1-100-60, TAR1-100-62, TAR1-100-64, TAR1-100-65, TAR1-100-75, TAR1-100-76, TAR1-100-77, TAR1-100-78, TAR1-100-80, TAR1-100-82, TAR1-100-83, TAR1-100-84, TAR1-100-89, TAR1-100-90, TAR1-100-91, TAR1-100-92, TAR1-100-93, TAR1-100-94, TAR1-100-95, TAR1-100-96, TAR1-100-97, TAR1-100-98, TAR1-100-99, TAR1-100-100, TAR1-100-101, TAR1-100-102, TAR1-100-103, TAR1-100-105, TAR1-100-106, TAR1-100-107, TAR1-100-108, TAR1-100-109, TAR1-100-110, TAR1-100-111, TAR1-100-112, TAR1-100-113 and TAR1-5-19.

55. The use of any one of claims 1-53, wherein said single domain antibody polypeptide construct comprises the amino acid sequence of an antibody polypeptide selected from the group consisting of clones TAR1-2m-9, TAR1-2m-30,TAR1-2m-1,TAR1-2m-2, TAR1-5, TAR1-27, TAR1-261, TAR1-398, TAR1-701,TAR1-5-2, TAR1-5-3, TAR1-5-4, TAR1-5-7, TAR1-5-8, TAR1-5-10, TAR1-5-11, TAR1-5-12, TAR1-5-13, TAR1-5-19, TAR1-5-20, TAR1-5-21, TAR1-5-22, TAR1-5-23, TAR1-5-24, TAR1-5-25, TAR1-5-26, TAR1-5-27, TAR1-5-28, TAR1-5-29, TAR1-5-34, TAR1-5-35, TAR1-5-36, TAR1-5-464, TAR1-5-463, TAR1-5-460, TAR1-5-461, TAR1-5-479, TAR1-5-477, TAR1-5-478, TAR1-5-476, TAR1-5-490, TAR1h-1, TAR1h-2, TAR1h-3, TAR1-100-29, TAR1-100-35, TAR1-100-43, TAR1-100-47, TAR1-100-52, TAR1-109, TAR1-100, TAR1-100-34, TAR1-100-36, TAR1-100-38, TAR1-100-39, TAR1-100-40, TAR1-100-41, TAR1-100-45, TAR1-100-60, TAR1-100-62, TAR1-100-64, TAR1-100-65, TAR1-100-75, TAR1-100-76, TAR1-100-77, TAR1-100-78, TAR1-100-80, TAR1-100-82, TAR1-100-83, TAR1-100-84, TAR1-100-89, TAR1-100-90, TAR1-100-91, TAR1-100-92, TAR1-100-93, TAR1-100-94, TAR1-100-95, TAR1-100-96, TAR1-100-97, TAR1-100-98, TAR1-100-99, TAR1-100-100, TAR1-100-101, TAR1-100-102, TAR1-100-103, TAR1-100-105, TAR1-100-106, TAR1-100-107, TAR1-100-108, TAR1-100-109, TAR1-100-110, TAR1-100-111, TAR1-100-112, TAR1-100-113 and TAR1-5-19 or an amino acid sequence at least 85% identical thereto.

56. The use of any one of claims 1-53, wherein said single domain antibody polypeptide construct comprises the amino acid sequence of an antibody polypeptide selected from the group consisting of clones TAR1-2m-9, TAR1-2m-30,TAR1-2m-1,TAR1-2m-2, TAR1-5, TAR1-27, TAR1-261, TAR1-398, TAR1-701,TAR1-5-2, TAR1-5-3, TAR1-5-4, TAR1-5-7, TAR1-5-8, TAR1-5-10, TAR1-5-11, TAR1-5-12, TAR1-5-13, TAR1-5-19, TAR1-5-20, TAR1-5-21, TAR1-5-22, TAR1-5-23, TAR1-5-24, TAR1-5-25, TAR1-5-26, TAR1-5-27, TAR1-5-28, TAR1-5-29, TAR1-5-34, TAR1-5-35, TAR1-5-36, TAR1-5-464, TAR1-5-463, TAR1-5-460, TAR1-5-461, TAR1-5-479, TAR1-5-477, TAR1-5-478, TAR1-5-476, TAR1-5-490, TAR1h-1, TAR1h-2, TAR1h-3, TAR1-100-29, TAR1-100-35, TAR1-100-43, TAR1-100-47, TAR1-100-52, TAR1-109, TAR1-100, TAR1-100-34, TAR1-100-36, TAR1-100-38, TAR1-100-39, TAR1-100-40, TAR1-100-41, TAR1-100-45, TAR1-100-60, TAR1-100-62, TAR1-100-64, TAR1-100-65, TAR1-100-75, TAR1-100-76, TAR1-100-77, TAR1-100-78, TAR1-100-80, TAR1-100-82, TAR1-100-83, TAR1-100-84, TARI-100-89, TAR1-100-90, TAR1-100-91, TAR1-100-92, TAR1-100-93, TAR1-100-94, TAR1-100-95, TAR1-100-96, TAR1-100-97, TAR1-100-98, TAR1-100-99, TAR1-100-100, TAR1-100-101, TAR1-100-102, TAR1-100-103, TAR1-100-105, TAR1-100-106, TAR1-100-107, TAR1-100-108, TAR1-100-109, TAR1-100-110, TAR1-100-111, TAR1-100-112, TAR1-100-113 and TAR1-5-19 or an amino acid sequence at least 90% identical thereto.

57. The use of any one of claims 1-53, wherein said single domain antibody polypeptide construct comprises the amino acid sequence of an antibody polypeptide selected from the group consisting of clones TAR1-2m-9, TAR1-2m-30,TAR1-2m-1,TAR1-2m-2, TAR1-5, TAR1-27, TAR1-261, TAR1-398, TAR1-701,TAR1-5-2, TAR1-5-3, TAR1-5-4, TAR1-5-7, TAR1-5-8, TAR1-5-10, TAR1-5-11, TAR1-5-12, TAR1-5-13, TAR1-5-19, TAR1-5-20, TAR1-5-21, TAR1-5-22, TAR1-5-23, TAR1-5-24, TAR1-5-25, TAR1-5-26, TAR1-5-27, TAR1-5-28, TAR1-5-29, TAR1-5-34, TAR1-5-35, TAR1-5-36, TAR1-5-464, TAR1-5-463, TAR1-5-460, TAR1-5-461, TAR1-5-479, TAR1-5-477, TAR1-5-478, TAR1-5-476, TAR1-5-490, TAR1h-1, TAR1h-2, TAR1h-3, TAR1-100-29, TAR1-100-35, TAR1-100-43, TAR1-100-47, TAR1-100-52, TAR1-109, TAR1-100, TAR1-100-34, TAR1-100-36, TAR1-100-38, TAR1-100-39, TAR1-100-40, TAR1-100-41, TAR1-100-45, TAR1-100-60, TAR1-100-62, TAR1-100-64, TAR1-100-65, TAR1-100-75, TAR1-100-76, TAR1-100-77, TAR1-100-78, TAR1-100-80, TAR1-100-82, TAR1-100-83, TAR1-100-84, TAR1-100-89, TAR1-100-90, TAR1-100-91, TAR1-100-92, TAR1-100-93, TAR1-100-94, TAR1-100-95, TAR1-100-96, TAR1-100-97, TAR1-100-98, TAR1-100-99, TAR1-100-100, TAR1-100-101, TAR1-100-102, TAR1-100-103, TAR1-100-105, TAR1-100-106, TAR1-100-107, TAR1-100-108, TAR1-100-109, TAR1-100-110, TAR1-100-111, TAR1-100-112, TAR1-100-113 and TAR1-5-19 or an amino acid sequence at least 92% identical thereto.

58. The use of any one of claims 1-53, wherein said single domain antibody polypeptide construct comprises the amino acid sequence of an antibody polypeptide selected from the group consisting of clones TAR1-2m-9, TAR1-2m-30,TAR1-2m-1,TAR1-2m-2, TAR1-5, TAR1-27, TAR1-261, TAR1-398, TAR1-701,TAR1-5-2, TAR1-5-3, TAR1-5-4, TAR1-5-7, TAR1-5-8, TAR1-5-10, TAR1-5-11, TAR1-5-12, TAR1-5-13, TAR1-5-19, TAR1-5-20, TAR1-5-21, TAR1-5-22, TAR1-5-23, TAR1-5-24, TAR1-5-25, TAR1-5-26, TAR1-5-27, TAR1-5-28, TAR1-5-29, TAR1-5-34, TAR1-5-35, TAR1-5-36, TAR1-5-464, TAR1-5-463, TAR1-5-460, TAR1-5-461, TAR1-5-479, TAR1-5-477, TAR1-5-478, TAR1-5-476, TAR1-5-490, TAR1h-1, TAR1h-2, TAR1h-3, TAR1-100-29, TAR1-100-35, TAR1-100-43, TAR1-100-47, TAR1-100-52, TAR1-109, TAR1-100, TAR1-100-34, TAR1-100-36, TAR1-100-38, TAR1-100-39, TAR1-100-40, TAR1-100-41, TAR1-100-45, TAR1-100-60, TAR1-100-62, TAR1-100-64, TAR1-100-65, TAR1-100-75, TAR1-100-76, TAR1-100-77, TAR1-100-78, TAR1-100-80, TAR1-100-82, TAR1-100-83, TAR1-100-84, TAR1-100-89, TAR1-100-90, TAR1-100-91, TAR1-100-92, TAR1-100-93, TAR1-100-94, TAR1-100-95, TAR1-100-96, TAR1-100-97, TAR1-100-98, TAR1-100-99, TAR1-100-100, TAR1-100-101, TAR1-100-102, TAR1-100-103, TAR1-100-105, TAR1-100-106, TAR1-100-107, TAR1-100-108, TAR1-100-109, TAR1-100-110, TAR1-100-111, TAR1-100-112, TAR1-100-113 and TAR1-5-19 or an amino acid sequence at least 94% identical thereto.

59. The use of any one of claims 1-53, wherein said single domain antibody polypeptide construct comprises the amino acid sequence of an antibody polypeptide selected from the group consisting of clones TAR1-2m-9, TAR1-2m-30,TAR1-2m-1,TAR1-2m-2, TAR1-5, TAR1-27, TAR1-261, TAR1-398, TAR1-701,TAR1-5-2, TAR1-5-3, TAR1-5-4, TAR1-5-7, TAR1-5-8, TAR1-5-10, TAR1-5-11, TAR1-5-12, TAR1-5-13, TAR1-5-19, TAR1-5-20, TAR1-5-21, TAR1-5-22, TAR1-5-23, TAR1-5-24, TAR1-5-25, TAR1-5-26, TAR1-5-27, TAR1-5-28, TAR1-5-29, TAR1-5-34, TAR1-5-35, TAR1-5-36, TAR1-5-464, TAR1-5-463, TAR1-5-460, TAR1-5-461, TAR1-5-479, TAR1-5-477, TAR1-5-478, TAR1-5-476, TAR1-5-490, TAR1h-1, TAR1h-2, TAR1h-3, TAR1-100-29, TAR1-100-35, TAR1-100-43, TAR1-100-47, TAR1-100-52, TAR1-109, TAR1-100, TAR1-100-34, TAR1-100-36, TAR1-100-38, TAR1-100-39, TAR1-100-40, TAR1-100-41, TAR1-100-45, TAR1-100-60, TAR1-100-62, TAR1-100-64, TAR1-100-65, TAR1-100-75, TAR1-100-76, TAR1-100-77, TAR1-100-78, TAR1-100-80, TAR1-100-82, TAR1-100-83, TAR1-100-84, TAR1-100-89, TAR1-100-90, TAR1-100-91, TAR1-100-92, TAR1-100-93, TAR1-100-94, TAR1-100-95, TAR1-100-96, TAR1-100-97, TAR1-100-98, TAR 1-100-99, TAR1-100-100, TAR1-100-101, TAR 1-100-102, TAR1-100-103, TAR1-100-105, TAR1-100-106, TAR1-100-107, TAR1-100-108, TAR1-100-109, TAR1-100-110, TAR1-100-111, TAR1-100-112, TAR1-100-113 and TAR1-5-19 or an amino acid sequence at least 96% identical thereto.

60. The method or use of any one of claims 1-53, wherein said single domain antibody polypeptide construct comprises the amino acid sequence of an antibody polypeptide selected from the group consisting of clones TAR1-2m-9, TAR1-2m-30,TAR1-2m-1,TAR1-2m-2, TAR1-5, TAR1-27, TAR1-261, TAR1-398, TAR1-701,TAR1-5-2, TAR1-5-3, TAR1-5-4, TAR1-5-7, TAR1-5-8, TAR1-5-10, TAR1-5-11, TAR1-5-12, TAR1-5-13, TAR1-5-19, TAR1-5-20, TAR1-5-21, TAR1-5-22, TAR1-5-23, TAR1-5-24, TAR1-5-25, TAR1-5-26, TAR1-5-27, TAR1-5-28, TAR1-5-29, TAR1-5-34, TAR1-5-35, TAR1-5-36, TAR1-5-464, TAR1-5-463, TAR1-5-460, TAR1-5-461, TAR1-5-479, TAR1-5-477, TAR1-5-478, TAR1-5-476, TAR1-5-490, TAR1h-1, TAR1h-2, TAR1h-3, TAR1-100-29, TAR1-100-35, TAR1-100-43, TAR1-100-47, TAR1-100-52, TAR1-109, TAR1-100, TAR1-100-34, TAR1-100-36, TAR1-100-38, TAR1-100-39, TAR1-100-40, TAR1-100-41, TAR1-100-45, TAR1-100-60, TAR1-100-62, TAR1-100-64, TAR1-100-65, TAR1-100-75, TAR1-100-76, TAR1-100-77, TAR1-100-78, TAR1-100-80, TAR1-100-82, TAR1-100-83, TAR1-100-84, TAR1-100-89, TAR1-100-90, TAR1-100-91, TAR1-100-92, TAR1-100-93, TAR1-100-94, TAR1-100-95, TAR1-100-96, TAR1-100-97, TAR1-100-98, TAR1-100-99, TAR1-100-100, TAR1-100-101, TAR1-100-102, TAR1-100-103, TAR1-100-105, TAR1-100-106, TAR1-100-107, TAR1-100-108, TAR1-100-109, TAR1-100-110, TAR1-100-11 1, TAR1-100-112, TAR1-100-113 and TAR1-5-19 or an amino acid sequence at least 98% identical thereto.

61. The use of any one of claims 1-53, wherein said single domain antibody polypeptide construct comprises the amino acid sequence of an antibody polypeptide selected from the group consisting of clones TAR1-2m-9, TAR1-2m-30,TAR1-2m-1,TAR1-2m-2, TAR1-5, TAR1-27, TAR1-261, TAR1-398, TAR1-701,TAR1-5-2, TAR1-5-3, TAR1-5-4, TAR1-5-7, TAR1-5-8, TAR1-5-10, TAR1-5-11, TAR1-5-12, TAR1-5-13, TAR1-5-19, TAR1-5-20, TAR1-5-21, TAR1-5-22, TAR1-5-23, TAR1-5-24, TAR1-5-25, TAR1-5-26, TAR1-5-27, TAR1-5-28, TAR1-5-29, TAR1-5-34, TAR1-5-35, TAR1-5-36, TAR1-5-464, TAR1-5-463, TAR1-5-460, TAR1-5-461, TAR1-5-479, TAR1-5-477, TAR1-5-478, TAR1-5-476, TAR1-5-490, TAR1h-1, TAR1h-2, TAR1h-3, TAR1-100-29, TAR1-100-35, TAR1-100-43, TAR1-100-47, TAR1-100-52, TAR1-109, TAR1-100, TAR1-100-34, TAR1-100-36, TAR1-100-38, TAR1-100-39, TAR1-100-40, TAR1-100-41, TAR1-100-45, TAR1-100-60, TAR1-100-62, TAR1-100-64, TAR1-100-65, TAR1-100-75, TAR1-100-76, TAR1-100-77, TAR1-100-78, TAR1-100-80, TAR1-100-82, TAR1-100-83, TAR1-100-84, TAR1-100-89, TAR1-100-90, TAR1-100-91, TAR1-100-92, TAR1-100-93, TAR1-100-94, TAR1-100-95, TAR1-100-96, TAR1-100-97, TAR1-100-98, TAR1-100-99, TAR1-100-100, TAR1-100-101, TAR1-100-102, TAR1-100-103, TAR1-100-105, TAR1-100-106, TAR1-100-107, TAR1-100-108, TAR1-100-109, TAR1-100-110, TAR1-100-111, TAR1-100-112, TAR1-100-113 and TAR1-5-19 or an amino acid sequence at least 99% identical thereto.

62. The use of any preceding claim wherein said single domain antibody polypeptide construct comprises a tetravalent, dual-specific antibody polypeptide construct comprising:
a) a first copy of a first fusion protein comprising a single domain antibody polypeptide that binds a first epitope, fused to an IgG heavy chain constant domain;
b) a second copy of said first fusion protein;
c) a first copy of a second fusion protein comprising a single domain antibody polypeptide that binds a second epitope, fused to a light chain constant domain;
d) a second copy of said second fusion protein;
wherein said first and said second copies of said first fusion protein are disulfide bonded to each other via their respective IgG heavy chain constant domains, and
wherein said light chain constant domain of said first copy of said second fusion protein is disulfide bonded to the IgG heavy chain constant domain of said first copy of said first fusion protein, and
wherein said light chain constant domain of said second copy of second fusion protein is disulfide bonded to the IgG heavy chain constant domain of said second copy of said first fusion protein, and
wherein said polypeptide construct binds said first and said second epitopes and wherein said first and second epitopes are TNF-α epitopes.

63. Use of a composition comprising a single domain antibody polypeptide construct that antagonizes human TNF-α's binding to a receptor in vitro for the preparation of a medicament for the treatment, prevention, inhibition of progression or delay in the onset of rheumatoid arthritis, wherein said single domain antibody polypeptide construct comprises a tetravalent, dual-specific antibody polypeptide construct as defined in claim 62.

64. A composition comprising a single domain antibody polypeptide construct including a tetravalent, dual-specific antibody polypeptide construct comprising:
a) a first copy of a first fusion protein comprising a single domain antibody polypeptide that binds a first epitope, fused to an IgG heavy chain constant domain;
b) a second copy of said first fusion protein;
c) a first copy of a second fusion protein comprising a single domain antibody polypeptide that binds a second epitope, fused to a light chain constant domain;
d) a second copy of said second fusion protein;
wherein said first and said second copies of said first fusion protein are disulfide bonded to each other via their respective IgG heavy chain constant domains, and
wherein said light chain constant domain of said first copy of said second fusion protein is disulfide bonded to the IgG heavy chain constant domain of said first copy of said first fusion protein, and
wherein said light chain constant domain of said second copy of second fusion protein is disulfide bonded to the IgG heavy chain constant domain of said second copy of said first fusion protein, and
wherein said polypeptide construct binds said first and said second epitopes and wherein said first and second epitopes are TNF-α epitopes.

65. A composition according to claim 64 comprising a single domain antibody polypeptide construct that antagonizes human TNFα's binding to a receptor, and that prevents an increase in arthritic score when administered to a mouse of the Tg197 transgenic mouse model of arthritis, wherein said single domain antibody polypeptide construct neutralizes human TNFα as measured in a standard L929 cell assay.

66. A composition according to claim 64 comprising a single domain antibody polypeptide construct that antagonizes human TNFα's binding to a receptor, and that prevents an increase in arthritic score when administered to a mouse of the Tg197 transgenic mouse model of arthritis, wherein said single domain antibody polypeptide construct inhibits the progression of rheumatoid arthritis.

67. A composition according to claim 64 comprising a single domain antibody polypeptide construct that antagonizes human TNFα's binding to a receptor, and that prevents an increase in arthritic score when administered to a mouse of the Tg197 transgenic mouse model of arthritis, wherein said single domain antibody polypeptide construct binds human TNFα with a Kd of < 100 nM.

68. A composition according to claim 64 comprising a single domain antibody polypeptide construct that antagonizes human TNFα's binding to a receptor, and that prevents an increase in arthritic score when administered to a mouse of the Tg197 transgenic mouse model of arthritis, wherein said single domain antibody polypeptide construct neutralizes human TNFα as measured in a standard L929 cell assay, wherein said single domain antibody polypeptide construct inhibits the progression of rheumatoid arthritis, and wherein said single domain antibody polypeptide construct binds human TNFα with a Kd of < 100 nM.

69. The composition of any one of claims 64-68 wherein said single domain antibody polypeptide construct comprises the amino acid sequence of CDR3 of an antibody polypeptide selected from the group consisting of clones TAR1-2m-9, TAR1-2m-30, TAR1-2m-1, TAR1-2m-2, TAR1-5, TAR1-27, TAR1-261, TAR1-398, TAR1-701,TAR1-5-2, TAR1-5-3, TAR1-5-4, TAR1-5-7, TAR1-5-8, TAR1-5-10, TAR1-5-11, TAR1-5-12, TAR1-5-13, TAR1-5-19, TAR1-5-20, TAR1-5-21, TAR1-5-22, TAR1-5-23, TAR1-5-24, TAR1-5-25, TAR1-5-26, TAR1-5-27, TAR1-5-28, TAR1-5-29, TAR1-5-34, TAR1-5-35, TAR1-5-36, TAR1-5-464, TAR1-5-463, TAR1-5-460, TAR1-5-461, TAR1-5-479, TAR1-5-477, TAR1-5-478, TAR1-5-476, TAR1-5-490, TAR1h-1, TAR1h-2, TAR1h-3, TAR1-100-29, TAR1-100-35, TAR1-100-43, TAR1-100-47, TAR1-100-52, TAR1-109, TAR1-100, TAR1-100-34, TAR1-100-36, TAR1-100-38, TAR1-100-39, TAR1-100-40, TAR1-100-41, TAR1-100-45, TAR1-100-60, TAR1-100-62, TAR1-100-64, TAR1-100-65, TAR1-100-75, TAR1-100-76, TAR1-100-77, TAR1-100-78, TAR1-100-80, TAR1-100-82, TAR1-100-83, TAR1-100-84, TAR1-100-89, TAR1-100-90, TAR1-100-91, TAR1-100-92, TAR1-100-93, TAR1-100-94, TAR1-100-95, TAR1-100-96, TAR1-100-97, TAR1-100-98, TAR1-100-99, TAR 1-100-100, TAR 1-100-101, TAR1-100-102, TAR1-100-103, TAR1-100-105, TAR1-100-106, TAR1-100-107, TAR1-100-108, TAR1-100-109, TAR1-100-110, TAR1-100-111, TAR1-100-112, TAR1-100-113 and TAR 1-5-19.

70. The composition of any one of claims 64-68 wherein said single domain antibody polypeptide construct comprises the amino acid sequence of an antibody polypeptide selected from the group consisting of clones TAR1-2m-9, TAR1-2m-30,TAR 1-2m-1,TAR1-2m-2, TAR1-5, TAR1-27, TAR1-261, TAR1-398, TAR1-701,TAR1-5-2, TAR1-5-3, TAR1-5-4, TAR1-5-7, TAR1-5-8, TAR1-5-10, TAR1-5-11, TAR1-5-12, TAR1-5-13, TAR1-5-19, TAR1-5-20, TAR1-5-21, TAR1-5-22, TAR1-5-23, TAR1-5-24, TAR1-5-25, TAR1-5-26, TAR1-5-27, TAR1-5-28, TAR1-5-29, TAR1-5-34, TAR1-5-35, TAR1-5-36, TAR1-5-464, TAR1-5-463, TAR1-5-460, TAR1-5-461, TAR1-5-479, TAR1-5-477, TAR1-5-478, TAR1-5-476, TAR1-5-490, TAR1h-1, TAR1h-2, TAR1h-3, TAR1-100-29, TAR1-100-35, TAR1-100-43, TAR1-100-47, TAR1-100-52, TAR1-109, TAR1-100, TAR1-100-34, TAR1-100-36, TAR1-100-38, TAR1-100-39, TAR1-100-40, TAR1-100-41, TAR1-100-45, TAR1-100-60, TAR1-100-62, TAR1-100-64, TAR1-100-65, TAR1-100-75, TAR1-100-76, TAR1-100-77, TAR1-100-78, TAR1-100-80, TAR1-100-82, TAR1-100-83, TAR1-100-84, TAR1-100-89, TAR1-100-90, TAR1-100-91, TAR1-100-92, TAR1-100-93, TAR1-100-94, TAR1-100-95, TAR1-100-96, TAR1-100-97, TAR1-100-98, TAR1-100-99, TAR1-100-100, TAR1-100-101, TAR1-100-102, TAR1-100-103, TAR1-100-105, TAR1-100-106, TAR1-100-107, TAR1-100-108, TAR1-100-109, TAR1-100-110, TAR1-100-111, TAR1-100-112, TAR1-100-113 and TAR1-5-19 or an amino acid sequence at least 85% identical thereto.

71. The composition of any one of claims 64-68, wherein said single domain antibody polypeptide construct comprises the amino acid sequence of an antibody polypeptide selected from the group consisting of clones TAR1-2m-9, TAR1-2m-30,TAR1-2m-1,TAR1-2m-2, TAR1-5, TAR1-27, TAR1-261, TAR1-398, TAR1-701,TAR1-5-2, TAR1-5-3, TAR1-5-4, TAR1-5-7, TAR1-5-8, TAR1-5-10, TAR1-5-11, TAR1-5-12, TAR1-5-13, TAR1-5-19, TAR1-5-20, TAR1-5-21, TAR1-5-22, TAR1-5-23, TAR1-5-24, TAR1-5-25, TAR1-5-26, TAR1-5-27, TAR1-5-28, TAR1-5-29, TAR1-5-34, TAR1-5-35, TAR1-5-36, TAR1-5-464, TAR1-5-463, TAR1-5-460, TAR1-5-461, TAR1-5-479, TAR1-5-477, TAR1-5-478, TAR1-5-476, TAR1-5-490, TAR1h-1, TAR1h-2, TAR1h-3, TAR1-100-29, TAR1-100-35, TAR1-100-43, TAR1-100-47, TAR1-100-52, TAR1-109, TAR1-100, TAR1-100-34, TAR1-100-36, TAR1-100-38, TAR1-100-39, TAR1-100-40, TAR1-100-41, TAR1-100-45, TAR1-100-60, TAR1-100-62, TAR1-100-64, TAR1-100-65, TAR1-100-75, TAR1-100-76, TAR1-100-77, TAR1-100-78, TAR1-100-80, TAR1-100-82, TAR1-100-83, TAR1-100-84, TAR1-100-89, TAR1-100-90, TAR1-100-91, TAR1-100-92, TAR1-100-93, TAR1-100-94, TAR1-100-95, TAR1-100-96, TAR1-100-97, TAR1-100-98, TAR1-100-99, TAR1-100-100, TAR1-100-101, TAR1-100-102, TAR1-100-103, TAR1-100-105, TAR1-100-106, TAR1-100-107, TAR1-100-108, TAR1-100-109, TAR1-100-110, TAR1-100-111, TAR1-100-112, TAR1-100-113 and TAR1-5-19 or an amino acid sequence at least 90% identical thereto.

72. The composition of any one of claims 64-68, wherein said single domain antibody polypeptide construct comprises the amino acid sequence of an antibody polypeptide selected from the group consisting of clones TAR1-2m-9, TAR1-2m-30,TAR1-2m-1,TAR1-2m-2, TAR1-5, TAR1-27, TAR1-261, TAR1-398, TAR1-701,TAR1-5-2, TAR1-5-3, TAR1-5-4, TAR1-5-7, TAR1-5-8, TAR1-5-10, TAR1-5-11, TAR1-5-12, TAR1-5-13, TAR1-5-19, TAR1-5-20, TAR1-5-21, TAR1-5-22, TAR1-5-23, TAR1-5-24, TAR1-5-25, TAR1-5-26, TAR1-5-27, TAR1-5-28, TAR1-5-29, TAR1-5-34, TAR1-5-35, TAR1-5-36, TAR1-5-464, TAR1-5-463, TAR1-5-460, TAR1-5-461, TAR1-5-479, TAR1-5-477, TAR1-5-478, TAR1-5-476, TAR1-5-490, TAR1h-1, TAR1h-2, TAR1h-3, TAR1-100-29, TAR1-100-35, TAR1-100-43, TAR1-100-47, TAR1-100-52, TAR1-109, TAR1-100, TAR1-100-34, TAR1-100-36, TAR1-100-38, TAR1-100-39, TAR1-100-40, TAR1-100-41, TAR1-100-45, TAR1-100-60, TAR1-100-62, TAR1-100-64, TAR1-100-65, TAR1-100-75, TAR1-100-76, TAR1-100-77, TAR1-100-78, TAR1-100-80, TAR1-100-82, TAR1-100-83, TAR1-100-84, TAR1-100-89, TAR1-100-90, TAR1-100-91, TAR1-100-92, TAR1-100-93, TAR1-100-94, TAR1-100-95, TAR1-100-96, TAR1-100-97, TAR1-100-98, TAR1-100-99, TAR1-100-100, TAR1-100-101, TAR1-100-102, TAR1-100-103, TAR1-100-105, TAR1-100-106, TAR1-100-107, TAR1-100-108, TAR1-100-109, TAR1-100-110, TAR1-100-111, TAR1-100-112, TAR1-100-113 and TAR1-5-19 or an amino acid sequence at least 92% identical thereto.

73. The composition of any one of claims 64-68, wherein said single domain antibody polypeptide construct comprises the amino acid sequence of an antibody polypeptide selected from the group consisting of clones TAR1-2m-9, TAR1-2m-30,TAR1-2m-1,TAR1-2m-2, TAR1-5, TAR1-27, TAR1-261, TAR1-398, TAR1-701,TAR1-5-2, TAR1-5-3, TAR1-5-4, TAR1-5-7, TAR1-5-8, TAR1-5-10, TAR1-5-11, TAR1-5-12, TAR1-5-13, TAR1-5-19, TAR1-5-20, TAR1-5-21, TAR1-5-22, TAR1-5-23, TAR1-5-24, TAR1-5-25, TAR1-5-26, TAR1-5-27, TAR1-5-28, TAR1-5-29, TAR1-5-34, TAR1-5-35, TAR1-5-36, TAR1-5-464, TAR1-5-463, TAR1-5-460, TAR1-5-461, TAR1-5-479, TAR1-5-477, TAR1-5-478, TAR1-5-476, TAR1-5-490, TAR1h-1, TAR1h-2, TAR1h-3, TAR1-100-29, TAR1-100-35, TAR1-100-43, TAR1-100-47, TAR1-100-52, TAR1-109, TAR1-100, TAR1-100-34, TAR1-100-36, TAR1-100-38, TAR1-100-39, TAR1-100-40, TAR1-100-41, TAR1-100-45, TAR1-100-60, TAR1-100-62, TAR1-100-64, TAR1-100-65, TAR1-100-75, TAR1-100-76, TAR1-100-77, TAR1-100-78, TAR1-100-80, TAR1-100-82, TAR1-100-83, TAR1-100-84, TAR1-100-89, TAR1-100-90, TAR1-100-91, TAR 1-100-92, TAR1-100-93, TAR1-100-94, TAR1-100-95, TAR1-100-96, TAR1-100-97, TAR1-100-98, TAR1-100-99, TAR1-100-100, TAR1-100-101, TAR1-100-102, TAR1-100-103, TAR1-100-105, TAR1-100-106, TAR1-100-107, TAR1-100-108, TAR1-100-109, TAR1-100-110, TAR1-100-111, TAR1-100-112, TAR1-100-113 and TAR1-5-19 or an amino acid sequence at least 94% identical thereto.

74. The composition of any one of claims 64-68, wherein said single domain antibody polypeptide construct comprises the amino acid sequence of an antibody polypeptide selected from the group consisting of clones TAR1-2m-9, TAR1-2m-30,TAR1-2m-1,TAR1-2m-2, TAR1-5, TAR1-27, TAR1-261, TAR1-398, TAR1-701,TAR1-5-2, TAR1-5-3, TAR1-5-4, TAR1-5-7, TAR1-5-8, TAR1-5-10, TAR1-5-11, TAR1-5-12, TAR1-5-13, TAR1-5-19, TAR1-5-20, TAR1-5-21, TAR1-5-22, TAR1-5-23, TAR1-5-24, TAR 1-5-25, TAR1-5-26, TAR1-S-27, TAR1-5-28, TAR1-5-29, TAR1-5-34, TAR1-5-35, TAR1-5-36, TAR1-5-464, TAR1-5-463, TAR1-5-460, TAR1-5-461, TAR1-5-479, TAR1-5-477, TAR1-5-478, TAR1-5-476, TAR1-5-490, TAR1h-1, TAR1h-2, TAR1h-3, TAR1-100-29, TAR1-100-35, TAR1-100-43, TAR1-100-47, TAR1-100-52, TAR1-109, TAR1-100, TAR1-100-34, TAR1-100-36, TAR1-100-38, TAR1-100-39, TAR1-100-40, TAR1-100-41, TAR1-100-45, TAR1-100-60, TAR1-100-62, TAR1-100-64, TAR1-100-65, TAR1-100-75, TAR1-100-76, TAR1-100-77, TAR1-100-78, TAR1-100-80, TAR1-100-82, TAR1-100-83, TAR1-100-84, TAR1-100-89, TAR 1-100-90, TAR1-100-91, TAR1-100-92, TAR1-100-93, TAR1-100-94, TAR1-100-95, TAR1-100-96, TAR1-100-97, TAR 1-100-98, TAR1-100-99, TAR1-100-100, TAR1-100-101, TAR1-100-102, TAR1-100-103, TAR1-100-105, TAR1-100-106, TAR1-100-107, TAR1-100-108, TAR1-100-109, TAR1-100-110, TAR1-100-111, TAR1-100-112, TAR1-100-113 and TAR1-5-19 or an amino acid sequence at least 96% identical thereto.

75. The composition of any one of claims 64-68, wherein said single domain antibody polypeptide construct comprises the amino acid sequence of an antibody polypeptide selected from the group consisting of clones TAR1-2m-9, TAR1-2m-30,TAR1-2m-1,TAR1-2m-2, TAR1-5, TAR1-27, TAR1-261, TAR1-398, TAR1-701,TAR1-5-2, TAR1-5-3, TAR1-5-4, TAR1-5-7, TAR1-5-8, TAR1-5-10, TAR1-5-11, TAR1-5-12, TAR1-5-13, TAR1-5-19, TAR1-5-20, TAR1-5-21, TAR1-5-22, TAR1-5-23, TAR1-5-24, TAR1-5-25, TAR1-5-26, TAR1-5-27, TAR1-5-28, TAR1-5-29, TAR1-5-34, TAR1-5-35, TAR1-5-36, TAR1-5-464, TAR1-5-463, TAR1-5-460, TAR1-5-461, TAR1-5-479, TAR1-5-477, TAR1-5-478, TAR1-5-476, TAR1-5-490, TAR1h-1, TAR1h-2, TAR1h-3, TAR1-100-29, TAR1-100-35, TAR1-100-43, TAR1-100-47, TAR1-100-52, TAR1-109, TAR1-100, TAR1-100-34, TAR1-100-36, TAR1-100-38, TAR1-100-39, TAR 1-100-40, TAR1-100-41, TAR1-100-45, TAR1-100-60, TAR1-100-62, TAR1-100-64, TAR1-100-65, TAR1-100-75, TAR1-100-76, TAR1-100-77, TAR1-100-78, TAR1-100-80, TAR1-100-82, TAR1-100-83, TAR1-100-84, TAR1-100-89, TAR1-100-90, TAR1-100-91, TAR1-100-92, TAR1-100-93, TAR1-100-94, TAR1-100-95, TAR1-100-96, TAR1-100-97, TAR1-100-98, TAR1-100-99, TAR1-100-100, TAR1-100-101, TAR1-100-102, TAR1-100-103, TAR1-100-105, TAR1-100-106, TAR1-100-107, TAR1-100-108, TAR1-100-109, TAR1-100-110, TAR1-100-111, TAR1-100-112, TAR1-100-113 and TAR1-5-19 or an amino acid sequence at least 98% identical thereto.

76. The composition of any one of claims 64-68, wherein said single domain antibody polypeptide construct comprises the amino acid sequence of an antibody polypeptide selected from the group consisting of clones TAR1-2m-9, TAR1-2m-30,TAR1-2m-1,TAR1-2m-2, TAR1-5, TAR1-27, TAR1-261, TAR1-398, TAR1-701,TAR1-5-2, TAR1-5-3, TAR1-5-4, TAR1-5-7, TAR1-5-8, TAR1-5-10, TAR1-5-11, TAR1-5-12, TAR1-5-13, TAR1-5-19, TAR1-5-20, TAR1-5-21, TAR1-5-22, TAR1-5-23, TAR1-5-24, TAR1-5-25, TAR1-5-26, TAR1-5-27, TAR1-5-28, TAR1-5-29, TAR1-5-34, TAR1-5-35, TAR1-5-36, TAR1-5-464, TAR1-5-463, TAR1-5-460, TAR1-5-461, TAR1-5-479, TAR1-5-477, TAR1-5-478, TAR1-5-476, TAR1-5-490, TAR1h-1, TAR1h-2, TAR1h-3, TAR1-100-29, TAR1-100-35, TAR1-100-43, TAR1-100-47, TAR1-100-52, TAR1-109, TAR1-100, TAR1-100-34, TAR1-100-36, TAR1-100-38, TAR1-100-39, TAR1-100-40, TAR1-100-41, TAR1-100-45, TAR1-100-60, TAR1-100-62, TAR1-100-64, TAR1-100-65, TAR1-100-75, TAR1-100-76, TAR1-100-77, TAR1-100-78, TAR1-100-80, TAR1-100-82, TAR1-100-83, TAR1-100-84, TAR1-100-89, TAR1-100-90, TAR1-100-91, TAR1-100-92, TAR1-100-93, TAR1-100-94, TAR1-100-95, TAR1-100-96, TAR1-100-97, TAR1-100-98, TAR1-100-99, TAR1-100-100, TAR1-100-101, TAR1-100-102, TAR1-100-103, TAR1-100-105, TAR1-100-106, TAR1-100-107, TAR1-100-108, TAR1-100-109, TAR1-100-110, TAR1-100-111, TAR1-100-112, TAR1-100-113 and TAR1-5-19 or an amino acid sequence at least 99% identical thereto.


SEQUENCE LISTING

<110> Domantis Ltd.
Ignatovich, Olga
DeWildt, Rudolph M.T.
Benjamin, Woolven
Grant, Steven
Jones, Philip
Basran, Amrik
Brewis, Neil

<120> Compositions and Methods for Treating Inflammatory Disorders

<130> P59456L-WO; 8039/2108

<140> Not yet assigned
<141> 2005-06-30

<150> PCT/GB2004/002829
<151> 2004-06-30

<150> US 10/925,366
<151> 2004-08-24

<150> US 11/098,758
<151> 2005-04-04

<160> 351

<170> PatentIn version 3.1

<210> 1
<211> 116
<212> PRT
<213> Homo Sapiens

<400> 1

Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Ser Ser Tyr
            20                  25                  30

Ala Met Ser Trp Val Arg Gln Ala Pro Gly Lys Gly Leu Glu Trp Val
        35                  40                  45

Ser Ala Ile Ser Gly Ser Gly Gly Ser Thr Tyr Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Lys Ser Tyr Gly Ala Phe Asp Tyr Trp Gly Gln Gly Thr Leu Val
            100                 105                 110

Thr Val Ser Ser
        115

<210>  2
<211>  348
<212>  DNA
<213>  Homo Sapiens

<400>  2
gaggtgcagc tgttggagtc tgggggaggc ttggtacagc ctggggggtc cctgcgtctc      60

tcctgtgcag cctccggatt cacctttagc agctatgcca tgagctgggt ccgccaggct     120

ccagggaagg gtctagagtg ggtctcagct attagtggta gtggtggtag cacatactac     180

gcagactccg tgaagggccg gttcaccatc tcccgtgaca attccaagaa cacgctgtat     240

ctgcaaatga acagcctgcg tgccgaggac accgcggtat attactgtgc gaaaagttat     300

ggtgcttttg actactgggg ccagggaacc ctggtcaccg tctcgagc                  348

<210>  3
<211>  108
<212>  PRT
<213>  Homo Sapiens

<400>  3

Asp Ile Gln Met Thr Gln Ser Pro Ser Ser Leu Ser Ala Ser Val Gly
1               5                   10                  15

Asp Arg Val Thr Ile Thr Cys Arg Ala Ser Gln Ser Ile Ser Ser Tyr
            20                  25                  30

Leu Asn Trp Tyr Gln Gln Lys Pro Gly Lys Ala Pro Lys Leu Leu Ile
        35                  40                  45

Tyr Ala Ala Ser Ser Leu Gln Ser Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Thr Tyr Tyr Cys Gln Gln Ser Tyr Ser Thr Pro Asn
                85                  90                  95

Thr Phe Gly Gln Gly Thr Lys Val Glu Ile Lys Arg
            100                 105

<210>  4
<211>  324
<212>  DNA
<213>  Homo Sapiens

<400>  4
gacatccaga tgacccagtc tccatcctcc ctgtctgcat ctgtaggaga ccgtgtcacc      60

atcacttgcc gggcaagtca gagcattagc agctatttaa attggtacca gcagaaacca     120

gggaaagccc ctaagctcct gatctatgct gcatccagtt ggcaaagtgg ggtcccatca     180

cgtttcagtg gcagtggatc tgggacagat ttcactctca ccatcagcag tctgcaacct     240

gaagattttg ctacgtacta ctgtcaacag agttacagta cccctaatac gttcggccaa     300

gggaccaagg tggaaatcaa acgg                                            324

<210>  5
<211>  120
<212>  PRT
<213>  Artificial Sequence

<220>
<223>  Artificial antibody domain sequence

<400>  5

Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Arg Ile Ser Asp Glu
            20                  25                  30

Asp Met Gly Trp Val Arg Gln Ala Pro Gly Lys Gly Leu Glu Trp Val
        35                  40                  45

Ser Ser Ile Tyr Gly Pro Ser Gly Ser Thr Tyr Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Ser Ala Leu Glu Pro Leu Ser Glu Pro Leu Gly Phe Trp Gly Gln
            100                 105                 110

Gly Thr Leu Val Thr Val Ser Ser
        115                 120

<210>  6
<211>  108
<212>  PRT
<213>  Artificial sequence

<220>
<223>  Artificial Antibody Domain Sequence

<400>  6

Asp Ile Gln Met Thr Gln Ser Pro Ser Ser Leu Ser Ala Ser Val Gly
1               5                   10                  15

Asp Arg Val Thr Ile Thr Cys Arg Ala Ser Gln Ser Ile Ser Ser Tyr
            20                  25                  30

Leu Asn Trp Tyr Gln Gln Lys Pro Gly Lys Ala Pro Lys Leu Leu Ile
        35                  40                  45

Tyr Ala Ala Ser Ser Leu Gln Ser Gly Val Pro Ser Arg Phe Ser Gly
    50                  55                  60

Ser Gly Ser Gly Thr Asp Phe Thr Leu Thr Ile Ser Ser Leu Gln Pro

65                  70                  75                  80

Glu Asp Phe Ala Thr Tyr Tyr Cys Gln Gln Ser Tyr Ser Thr Pro Asn
                85                  90                  95

Thr Phe Gly Gln Gly Thr Lys Val Glu Ile Lys Arg
            100                 105

<210>  7
<211>  5
<212>  PRT
<213>  Artificial Sequence

<220>
<223>  Synthetic Linker Sequence

<400>  7

Gly Gly Gly Gly Ser
1               5

<210>  8
<211>  15
<212>  PRT
<213>  Artificial Sequence

<220>
<223>  Synthetic Linker Sequence

<400>  8

Gly Gly Gly Gly Ser Gly Gly Gly Gly Ser Gly Gly Gly Gly Ser
1               5                   10                  15

<210>  9
<211>  25
<212>  PRT
<213>  Artificial Sequence

<220>
<223>  Synthetic Linker Sequence

<400>  9

Gly Gly Gly Gly Ser Gly Gly Gly Gly Ser Gly Gly Gly Gly Ser Gly
1               5                   10                  15

Gly Gly Gly Ser Gly Gly Gly Gly Ser
            20                  25

<210>  10
<211>  35
<212>  PRT
<213>  Artificial Sequence

<220>
<223>  Synthetic Linker Sequence

<400>  10

Gly Gly Gly Gly Ser Gly Gly Gly Gly Ser Gly Gly Gly Gly Ser Gly
1               5                   10                  15

Gly Gly Gly Ser Gly Gly Gly Gly Ser Gly Gly Gly Gly Ser Gly Gly
            20                  25                  30

Gly Gly Ser
        35

<210>  11
<211>  15
<212>  PRT
<213>  Artificial Sequence

<220>
<223>  Synthetic Linker Sequence

<400>  11

Glu Pro Lys Ser Gly Asp Lys Thr His Thr Cys Pro Pro Cys Pro
1               5                   10                  15

<210>  12
<211>  119
<212>  PRT
<213>  Artificial Sequence

<220>
<223>  Artificial Antibody Domain Sequence

<220>
<221>  MISC_FEATURE
<222>  (115)..(116)
<223>  X=Stop Codon

<400>  12

Trp Ser Ala Ser Thr Asp Ile Gln Met Thr Gln Ser Pro Ser Ser Leu
1               5                   10                  15

Ser Ala Ser Val Gly Asp Arg Val Thr Ile Thr Cys Arg Ala Ser Gln
            20                  25                  30

Ser Ile Asp Ser Tyr Leu His Trp Tyr Gln Gln Lys Pro Gly Lys Ala
        35                  40                  45

Pro Lys Leu Leu Ile Tyr Ser Ala Ser Glu Leu Gln Ser Gly Val Pro
    50                  55                  60

Ser Arg Phe Ser Gly Ser Gly Ser Gly Thr Asp Phe Thr Leu Thr Ile
65                  70                  75                  80

Ser Ser Leu Gln Pro Glu Asp Phe Ala Thr Tyr Tyr Cys Gln Gln Val
                85                  90                  95

Val Trp Arg Pro Phe Thr Phe Gly Gln Gly Thr Lys Val Glu Ile Lys
            100                 105                 110

Arg Cys Xaa Xaa Gly Ser Gly
        115

<210>  13
<211>  357
<212>  DNA
<213>  Artificial Sequence

<220>
<223>  DNA encoding artificial antibody domain sequence

<400>  13
tggagcgcgt cgacggacat ccagatgacc cagtctccat cctctctgtc tgcatctgta      60

ggagaccgtg tcaccatcac ttgccgggca agtcagagca ttgatagtta tttacattgg     120

taccagcaga aaccagggaa agcccctaag ctcctgatct atagtgcatc cgagttgcaa     180

agtggggtcc catcacgttt cagtggcagt ggatctggga cagatttcac tctcaccatc     240

agcagtctgc aacctgaaga ttttgctacg tactactgtc aacaggttgt gtggcgtcct     300

tttacgttcg gccaagggac caaggtggaa atcaaacggt gctaataagg atccggc 357

<210> 14
<211> 39
<212> DNA
<213> Artificial Sequence

<220>
<223> Synthetic PCR Primer

<400> 14
tggagcgcgt cgacggacat ccagatgacc cagtctcca 39

<210> 15
<211> 39
<212> DNA
<213> Artificial Sequence

<220>
<223> Synthetic PCR Primer

<400> 15
ttagcagccg gatccttatt agcaccgttt gatttccac 39

<210> 16
<211> 5
<212> PRT
<213> Artificial Sequence

<220>
<223> CDR Sequence of Synthetic Antibody Domain

<220>
<221> MISC_FEATURE
<222> (1)..(3)
<223> X=any amino acid

<220>
<221> MISC_FEATURE
<222> (1)..(3)
<223> X=any amino acid

<220>
<221> MISC_FEATURE
<222> (5)..(5)
<223> X=any amino acid

<400>  16

Xaa Xaa Xaa Leu Xaa
1               5


<210>  17
<211>  7
<212>  PRT
<213>  Artificial Sequence

<220>
<223>  Artificial CDR2 Sequence

<220>
<221>  MISC_FEATURE
<222>  (1)..(1)
<223>  X=Any Amino Acid


<220>
<221>  MISC_FEATURE
<222>  (4)..(4)
<223>  X=Any Amino Acid


<400>  17

Xaa Ala Ser Xaa Leu Gln Ser
1               5


<210>  18
<211>  9
<212>  PRT
<213>  Artificial Sequence

<220>
<223>  Artificial CDR3 Sequence

<220>
<221>  MISC_FEATURE
<222>  (3)..(6)
<223>  X=Any Amino Acid


<220>
<221>  MISC_FEATURE
<222>  (8)..(8)
<223>  X=Any Amino Acid

<400>  18

Gln Gln Xaa Xaa Xaa Xaa Pro Xaa Thr
1               5


<210>  19
<211>  5
<212>  PRT
<213>  Artificial Sequence

<220>
<223>  Artificial CDR1 Sequence

<400>  19

Ser Ser Tyr Leu Asn
1               5


<210>  20
<211>  7
<212>  PRT
<213>  Artificial Sequence

<220>
<223>  Artificial CDR2 Sequence

<400>  20

Arg Ala Ser Pro Leu Gln Ser
1               5


<210>  21
<211>  9
<212>  PRT
<213>  Artificial Sequence

<220>
<223>  Artificial CDR3 Sequence

<400>  21

Gln Gln Thr Tyr Ser Val Pro Pro Thr
1               5


<210>  22
<211>  5
<212>  PRT
<213>  Artificial Sequence

<220>
<223>  Artificial CDR1 Sequence

<400>  22

Ser Ser Tyr Leu Asn
1               5


<210>  23
<211>  7
<212>  PRT
<213>  Artificial Sequence

<220>
<223>  Artificial CDR2 Sequence

<400>  23

Arg Ala Ser Pro Leu Gln Ser
1               5


<210>  24
<211>  9
<212>  PRT
<213>  Artificial Sequence

<220>
<223>  Artificial CDR3 Sequence

<400>  24

Gln Gln Thr Tyr Arg Ile Pro Pro Thr
1               5


<210>  25
<211>  5
<212>  PRT
<213>  Artificial Sequence

<220>
<223>  Artificial CDR1 Sequence

<400>  25

Phe Lys Ser Leu Lys
1               5


<210>  26
<211>  7

<212>  PRT
<213>  Artificial Sequence

<220>
<223>  Artificial CDR2 Sequence

<400>  26

Asn Ala Ser Tyr Leu Gln Ser
1               5


<210>  27
<211>  9
<212>  PRT
<213>  Artificial Sequence

<220>
<223>  Artificial CDR3 Sequence

<400>  27

Gln Gln Val Val Tyr Trp Pro Val Thr
1               5


<210>  28
<211>  5
<212>  PRT
<213>  Artificial Sequence

<220>
<223>  Artificial CDR1 Sequence

<400>  28

Tyr Tyr His Leu Lys
1               5


<210>  29
<211>  7
<212>  PRT
<213>  Artificial Sequence

<220>
<223>  Artificial CDR2 Sequence

<400>  29

Lys Ala Ser Thr Leu Gln Ser
1               5

<210>  30
<211>  9
<212>  PRT
<213>  Artificial Sequence

<220>
<223>  Artificial CDR3 Sequence

<400>  30

Gln Gln Val Arg Lys Val Pro Arg Thr
1               5


<210>  31
<211>  5
<212>  PRT
<213>  Artificial Sequence

<220>
<223>  Artificial CDR1 Sequence

<400>  31

Arg Arg Tyr Leu Lys
1               5


<210>  32
<211>  7
<212>  PRT
<213>  Artificial Sequence

<220>
<223>  Artificial CDR2 Sequence

<400>  32

Gln Ala Ser Val Leu Gln Ser
1               5


<210>  33
<211>  9
<212>  PRT
<213>  Artificial Sequence

<220>
<223>  Artificial CDR3 Sequence

<400>  33

Gln Gln Gly Leu Tyr Pro Pro Ile Thr
1               5


<210>  34
<211>  5
<212>  PRT
<213>  Artificial Sequence

<220>
<223>  Artificial CDR1 Sequence

<400>  34

Tyr Asn Trp Leu Lys
1               5


<210>  35
<211>  7
<212>  PRT
<213>  Artificial Sequence

<220>
<223>  Artificial CDR2 Sequence

<400>  35

Arg Ala Ser Ser Leu Gln Ser
1               5


<210>  36
<211>  9
<212>  PRT
<213>  Artificial Sequence

<220>
<223>  Artificial CDR3 Sequence

<400>  36

Gln Gln Asn Val Val Ile Pro Arg Thr
1               5


<210>  37
<211>  5
<212>  PRT
<213>  Artificial Sequence

<220>
<223>  Artificial CDR1 Sequence

<400>  37

Leu Trp His Leu Arg
1               5


<210>  38
<211>  7
<212>  PRT
<213>  Artificial Sequence

<220>
<223>  Artificial CDR2 Sequence

<400>  38

His Ala Ser Leu Leu Gln Ser
1               5


<210>  39
<211>  9
<212>  PRT
<213>  Artificial Sequence

<220>
<223>  Artificial CDR3 Sequence

<400>  39

Gln Gln Ser Ala Val Tyr Pro Lys Thr
1               5


<210>  40
<211>  5
<212>  PRT
<213>  Artificial Sequence

<220>
<223>  Artificial CDR1 Sequence

<400>  40

Phe Arg Tyr Leu Ala
1               5


<210>  41
<211>  7
<212>  PRT
<213>  Artificial Sequence

<220>
<223>  Artificial CDR2 Sequence

<400>  41

His Ala Ser His Leu Gln Ser
1               5


<210>  42
<211>  9
<212>  PRT
<213>  Artificial Sequence

<220>
<223>  Artificial CDR3 Sequence

<400>  42

Gln Gln Arg Leu Leu Tyr Pro Lys Thr
1               5


<210>  43
<211>  5
<212>  PRT
<213>  Artificial Sequence

<220>
<223>  Artificial CDR1 Sequence

<400>  43

Phe Tyr His Leu Ala
1               5


<210>  44
<211>  7
<212>  PRT
<213>  Artificial Sequence

<220>
<223>  Artificial CDR2 Sequence

<400>  44

Pro Ala Ser Lys Leu Gln Ser
1               5


<210>  45

<211>  9
<212>  PRT
<213>  Artificial Sequence

<220>
<223>  Artificial CDR3 Sequence

<400>  45

Gln Gln Arg Ala Arg Trp Pro Arg Thr
1               5


<210>  46
<211>  5
<212>  PRT
<213>  Artificial Sequence

<220>
<223>  Artificial CDR1 Sequence

<400>  46

Ile Trp His Leu Asn
1               5


<210>  47
<211>  7
<212>  PRT
<213>  Artificial Sequence

<220>
<223>  Artificial CDR2 Sequence

<400>  47

Arg Ala Ser Arg Leu Gln Ser
1               5


<210>  48
<211>  9
<212>  PRT
<213>  Artificial Sequence

<220>
<223>  Artificial CDR3 Sequence

<400>  48

Gln Gln Val Ala Arg Val Pro Arg Thr
1               5

<210>  49
<211>  5
<212>  PRT
<213>  Artificial Sequence

<220>
<223>  Artificial CDR1 Sequence

<400>  49

Tyr Arg Tyr Leu Arg
1               5


<210>  50
<211>  7
<212>  PRT
<213>  Artificial Sequence

<220>
<223>  Artificial CDR2 Sequence

<400>  50

Lys Ala Ser Ser Leu Gln Ser
1               5


<210>  51
<211>  9
<212>  PRT
<213>  Artificial Sequence

<220>
<223>  Artificial CDR3 Sequence

<400>  51

Gln Gln Tyr Val Gly Tyr Pro Arg Thr
1               5


<210>  52
<211>  5
<212>  PRT
<213>  Artificial Sequence

<220>
<223>  Artificial CDR1 Sequence

<400>  52

Leu Lys Tyr Leu Lys
1               5

<210>  53
<211>  7
<212>  PRT
<213>  Artificial Sequence

<220>
<223>  Artificial CDR2 Sequence

<400>  53

Asn Ala Ser His Leu Gln Ser
1               5

<210>  54
<211>  9
<212>  PRT
<213>  Artificial Sequence

<220>
<223>  Artificial CDR3 Sequence

<400>  54

Gln Gln Thr Thr Tyr Tyr Pro Ile Thr
1               5

<210>  55
<211>  5
<212>  PRT
<213>  Artificial Sequence

<220>
<223>  Artificial CDR1 Sequence

<400>  55

Leu Arg Tyr Leu Arg
1               5

<210>  56
<211>  7
<212>  PRT
<213>  Artificial Sequence

<220>

<223>  Artificial CDR2 Sequence

<400>  56

Lys Ala Ser Trp Leu Gln Ser
1               5


<210>  57
<211>  9
<212>  PRT
<213>  Artificial Sequence

<220>
<223>  Artificial CDR3 Sequence

<400>  57

Gln Gln Val Leu Tyr Tyr Pro Gln Thr
1               5


<210>  58
<211>  5
<212>  PRT
<213>  Artificial Sequence

<220>
<223>  Artificial CDR1 Sequence

<400>  58

Leu Arg Ser Leu Lys
1               5


<210>  59
<211>  7
<212>  PRT
<213>  Artificial Sequence

<220>
<223>  Artificial CDR2 Sequence

<400>  59

Ala Ala Ser Arg Leu Gln Ser
1               5


<210>  60
<211>  9
<212>  PRT

<213>  Artificial Sequence

<220>
<223>  Artificial CDR3 Sequence

<400>  60

Gln Gln Val Val Tyr Trp Pro Ala Thr
1               5


<210>  61
<211>  5
<212>  PRT
<213>  Artificial Sequence

<220>
<223>  Artificial CDR1 Sequence

<400>  61

Phe Arg His Leu Lys
1               5


<210>  62
<211>  7
<212>  PRT
<213>  Artificial Sequence

<220>
<223>  Artificial CDR2 Sequence

<400>  62

Ala Ala Ser Arg Leu Gln Ser
1               5


<210>  63
<211>  9
<212>  PRT
<213>  Artificial Sequence

<220>
<223>  Artificial CDR3 Sequence

<400>  63

Gln Gln Val Ala Leu Tyr Pro Lys Thr
1               5

<210>  64
<211>  5
<212>  PRT
<213>  Artificial Sequence

<220>
<223>  Artificial CDR1 Sequence

<400>  64

Arg Lys Tyr Leu Arg
1               5


<210>  65
<211>  7
<212>  PRT
<213>  Artificial Sequence

<220>
<223>  Artificial CDR2 Sequence

<400>  65

Thr Ala Ser Ser Leu Gln Ser
1               5


<210>  66
<211>  9
<212>  PRT
<213>  Artificial Sequence

<220>
<223>  Artificial CDR3 Sequence

<400>  66

Gln Gln Asn Leu Phe Trp Pro Arg Thr
1               5


<210>  67
<211>  5
<212>  PRT
<213>  Artificial Sequence

<220>
<223>  Artificial CDR1 Sequence

<400>  67

Arg Arg Tyr Leu Asn

1               5


<210>  68
<211>  7
<212>  PRT
<213>  Artificial Sequence

<220>
<223>  Artificial CDR2 Sequence

<400>  68

Ala Ala Ser Ser Leu Gln Ser
1               5


<210>  69
<211>  9
<212>  PRT
<213>  Artificial Sequence

<220>
<223>  Artificial CDR3 Sequence

<400>  69

Gln Gln Met Leu Phe Tyr Pro Lys Thr
1               5


<210>  70
<211>  5
<212>  PRT
<213>  Artificial Sequence

<220>
<223>  Artificial CDR1 Sequence

<400>  70

Ile Lys His Leu Lys
1               5


<210>  71
<211>  7
<212>  PRT
<213>  Artificial Sequence

<220>
<223>  Artificial CDR2 Sequence

<400>  71

Gly Ala Ser Arg Leu Gln Ser
1               5


<210>  72
<211>  9
<212>  PRT
<213>  Artificial Sequence

<220>
<223>  Artificial CDR3 Sequence

<400>  72

Gln Gln Gly Ala Arg Trp Pro Gln Thr
1               5


<210>  73
<211>  5
<212>  PRT
<213>  Artificial Sequence

<220>
<223>  Artificial CDR1 Sequence

<400>  73

Tyr Tyr His Leu Lys
1               5


<210>  74
<211>  7
<212>  PRT
<213>  Artificial Sequence

<220>
<223>  Artificial CDR2 Sequence

<400>  74

Lys Ala Ser Thr Leu Gln Ser
1               5


<210>  75
<211>  9
<212>  PRT
<213>  Artificial Sequence

<220>
<223>  Artificial CDR3 Sequence

<400>  75

Gln Gln Val Arg Lys Val Pro Arg Thr
1               5


<210>  76
<211>  5
<212>  PRT
<213>  Artificial Sequence

<220>
<223>  Artificial CDR1 Sequence

<400>  76

Tyr Lys His Leu Lys
1               5


<210>  77
<211>  7
<212>  PRT
<213>  Artificial Sequence

<220>
<223>  Artificial CDR2 Sequence

<400>  77

Asn Ala Ser His Leu Gln Ser
1               5


<210>  78
<211>  9
<212>  PRT
<213>  Artificial Sequence

<220>
<223>  Artificial CDR3 Sequence

<400>  78

Gln Gln Val Gly Arg Tyr Pro Lys Thr
1               5


<210>  79
<211>  5

<212>  PRT
<213>  Artificial Sequence

<220>
<223>  Artificial CDR1 Sequence

<220>
<221>  MISC_FEATURE
<222>  (5)..(5)
<223>  X=Any Amino Acid


<400>  79

Phe Lys Ser Leu Xaa
1               5


<210>  80
<211>  7
<212>  PRT
<213>  Artificial Sequence

<220>
<223>  Artificial CDR2 Sequence

<400>  80

Asn Ala Ser Tyr Leu Gln Ser
1               5


<210>  81
<211>  9
<212>  PRT
<213>  Artificial Sequence

<220>
<223>  Artificial CDR3 Sequence

<400>  81

Gln Gln Val Val Tyr Trp Pro Val Thr
1               5


<210>  82
<211>  6
<212>  PRT
<213>  Artificial Sequence

<220>
<223>  Consenus CDR1 in VH Library 1

<220>
<221>  MISC_FEATURE
<222>  (1)..(2)
<223>  X=Any Amino Acid


<220>
<221>  MISC_FEATURE
<222>  (4)..(6)
<223>  X=Any Amino Acid


<400>  82

Xaa Xaa Tyr Xaa Xaa Xaa
1               5


<210>  83
<211>  17
<212>  PRT
<213>  Artificial Sequence

<220>
<223>  Consenus CDR2 in VH Library 1

<220>
<221>  MISC_FEATURE
<222>  (1)..(1)
<223>  X=Any Amino Acid


<220>
<221>  MISC_FEATURE
<222>  (3)..(5)
<223>  X=Any Amino Acid


<220>
<221>  MISC_FEATURE
<222>  (7)..(8)
<223>  X=Any Amino Acid


<220>
<221>  MISC_FEATURE
<222>  (10)..(10)
<223>  X=Any Amino Acid


<400>  83

Xaa Ile Xaa Xaa Xaa Gly Xaa Xaa Thr Xaa Tyr Ala Asp Ser Val Lys
1               5                   10                  15

Gly

<210>  84
<211>  11
<212>  PRT
<213>  Artificial Sequence

<220>
<223>  Consensus CDR3 in VH Library 1

<220>
<221>  MISC_FEATURE
<222>  (1)..(8)
<223>  X=Any Amino Acid

<400>  84

Xaa Xaa Xaa Xaa Xaa Xaa Xaa Xaa Phe Asp Tyr
1               5                   10

<210>  85
<211>  6
<212>  PRT
<213>  Artificial Sequence

<220>
<223>  Consensus CDR1 in VH Library 1

<400>  85

Trp Val Tyr Gln Met Asp
1               5

<210>  86
<211>  17
<212>  PRT
<213>  Artificial Sequence

<220>
<223>  Consensus CDR2 in VH Libarary 1

<400>  86

Ser Ile Ser Ala Phe Gly Ala Lys Thr Leu Tyr Ala Asp Ser Val Lys

1               5                   10                  15

Gly

<210>  87
<211>  7
<212>  PRT
<213>  Artificial Sequence

<220>
<223>  Consensus CDR3 in VH Library 1

<400>  87

Leu Ser Gly Lys Phe Asp Tyr
1               5

<210>  88
<211>  6
<212>  PRT
<213>  Artificial Sequence

<220>
<223>  Consensus CDR1 in VH Library 1

<400>  88

Trp Ser Tyr Gln Met Thr
1               5

<210>  89
<211>  17
<212>  PRT
<213>  Artificial Sequence

<220>
<223>  Consensus CDR2 in VH Library 1

<400>  89

Ser Ile Ser Ser Phe Gly Ser Ser Thr Leu Tyr Ala Asp Ser Val Lys
1               5                   10                  15

Gly

<210>  90
<211>  11
<212>  PRT
<213>  Artificial Sequence

<220>
<223>  Consensus CDR3 in VH Library 1

<400>  90

Gly Arg Asp His Asn Tyr Ser Leu Phe Asp Tyr
1               5                   10


<210>  91
<211>  27
<212>  DNA
<213>  Artificial Sequence

<220>
<223>  Nucleic Acid Sequence for HA Tag

<400>  91
tatccttatg atgttcctga ttatgca                                          27


<210>  92
<211>  9
<212>  PRT
<213>  Artificial Sequence

<220>
<223>  Amino Acid Sequence for HA Tag

<400>  92

Tyr Pro Tyr Asp Val Pro Asp Tyr Ala
1               5


<210>  93
<211>  108
<212>  PRT
<213>  Artificial Sequence

<220>
<223>  Synthetic Antibody Domain

<400>  93

Asp Ile Gln Met Thr Gln Ser Pro Ser Ser Leu Ser Ala Ser Val Gly
1               5                   10                  15

Asp Arg Val Thr Ile Thr Cys Arg Ala Ser Gln Pro Ile Gly Ser Phe
            20                  25                  30

Leu Trp Trp Tyr Gln Gln Lys Pro Gly Lys Ala Pro Lys Leu Leu Ile
        35                  40                  45

Tyr Tyr Ser Ser Tyr Leu Gln Ser Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Thr Tyr Tyr Cys Gln Gln Tyr Arg Trp His Pro Asn
                85                  90                  95

Thr Phe Gly Gln Gly Thr Lys Val Glu Ile Lys Arg
            100                 105

<210>  94
<211>  324
<212>  DNA
<213>  Artificial Sequence

<220>
<223>  Nucleic Acid Sequence Encoding Synthetic Antibody Domain

<400>  94
gacatccaga tgacccagtc tccatcctcc ctgtctgcat ctgtaggaga ccgtgtcacc      60

atcacttgcc gggcaagtca gcctattggg agttttttat ggtggtacca gcagaaacca      120

gggaaagccc ctaaactcct gatctattat agttcctatt tgcaaagtgg ggtcccatca      180

cgtttcagtg gcagtggatc tgggacagat ttcactctca ccatcagcag tctgcaacct      240

gaagattttg ctacgtacta ctgtcaacag tatcgttggc atcctaatac cttcggccaa      300

gggaccaagg tggaaatcaa acgg                                              324

<210>  95
<211>  108
<212>  PRT
<213>  Artificial Sequence

<220>

<223>  Synthetic Antibody Domain

<400>  95

Asp Ile Gln Met Thr Gln Ser Pro Ser Ser Leu Ser Ala Ser Val Gly
1               5                   10                  15

Asp Arg Val Thr Ile Thr Cys Arg Ala Ser Gln Ser Ile Tyr Ser Trp
            20                  25                  30

Leu Asn Trp Tyr Gln Gln Lys Pro Gly Lys Ala Pro Lys Leu Leu Ile
        35                  40                  45

Tyr Arg Ala Ser His Leu Gln Ser Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Thr Tyr Tyr Cys Gln Gln Ile Trp Asn Met Pro Phe
                85                  90                  95

Thr Phe Gly Gln Gly Thr Lys Val Glu Ile Lys Arg
            100                 105

<210>  96
<211>  324
<212>  DNA
<213>  Artificial Sequence

<220>
<223>  Nucelic Acid Sequence Encoding Synthetic Antibody Domain

<400>  96
gacatccaga tgacccagtc tccatcctcc ctgtctgcat ctgtaggaga ccgtgtcacc      60

atcacttgcc gggcaagtca gtcgatttat agttggttaa attggtacca gcagaaacca     120

gggaaagccc ctaagctcct gatctatagg gcgtcccatt tgcaaagtgg ggtcccatca     180

cgtttcagtg gcagtggatc tgggacagat ttcactctca ccatcagcag tctgcaacct     240

gaagattttg ctacgtacta ctgtcaacag atttggaata tgccttttac gttcggccaa     300

gggaccaagg tggaaatcaa acgg                                            324

<210> 97
<211> 108
<212> PRT
<213> Artificial Sequence

<220>
<223> Synthetic Antibody Domain

<400> 97

Asp Ile Gln Met Thr Gln Ser Pro Ser Ser Leu Ser Ala Ser Val Gly
1               5                   10                  15

Asp Arg Val Thr Ile Thr Cys Arg Ala Ser Gln Pro Ile Gly Tyr Asp
            20                  25                  30

Leu Phe Trp Tyr Gln Gln Lys Pro Gly Lys Ala Pro Lys Leu Leu Ile
        35                  40                  45

Tyr Arg Gly Ser Val Leu Gln Ser Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Thr Tyr Tyr Cys Gln Gln Arg Trp Arg Trp Pro Phe
                85                  90                  95

Thr Phe Gly Gln Gly Thr Lys Val Glu Ile Lys Arg
            100                 105

<210> 98
<211> 324
<212> DNA
<213> Artificial Sequence

<220>
<223> Nucleic Acid Sequence Encoding Synthetic Antibody Domain

<400> 98
gacatccaga tgacccagtc tccatcctcc ctgtctgcat ctgtaggaga ccgtgtcacc 60

atcacttgcc gggcaagtca gcctattggt tatgatttat tttggtacca gcagaaacca 120

gggaaagccc ctaagctcct gatctatcgg ggttccgtgt tgcaaagtgg ggtcccatca 180

cgtttcagtg gcagtggatc tgggacagat ttcactctca ccatcagcag tctgcaacct 240

gaagattttg ctacgtacta ctgtcaacag cggtggcgtt ggccttttac gttcggccaa 300

ggcaccaagg tggaaatcaa acgg 324

<210> 99
<211> 107
<212> PRT
<213> Artificial Sequence

<220>
<223> Synthetic Antibody Domain

<400> 99

Asp Ile Gln Met Thr Gln Ser Pro Ser Ser Leu Ser Ala Ser Val Gly
1               5                   10                  15

Asp Arg Val Thr Ile Thr Cys Arg Ala Ser Leu Pro Ile Gly Arg Asp
            20                  25                  30

Leu Trp Trp Tyr Gln Gln Lys Pro Gly Lys Ala Pro Lys Leu Leu Ile
        35                  40                  45

Tyr Arg Gly Ser Phe Leu Gln Ser Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Thr Tyr Tyr Cys Gln Gln Arg Trp Tyr Tyr Pro His
                85                  90                  95

Thr Phe Gly Gln Gly Thr Lys Val Glu Ile Lys
            100                 105

<210> 100
<211> 324
<212> DNA
<213> Artificial Sequence

<220>
<223> Nucleic Acid Sequence Encoding Synthetic Antibody Domain

<400> 100
gacatccaga tgacccagtc tccatcctcc ctgtctgcat ctgtaggaga ccgtgtcacc 60

atcacttgcc gggcaagtct gcctattggt cgtgatttat ggtggtatca gcagaaacca 120

gggaaagccc ctaagctcct gatctatcgg gggtcctttt tgcaaagtgg ggtcccatca 180

cgtttcagtg gcagtggatc tgggacagat ttcactctca ccatcagcag tctgcaacct 240

gaagattttg ctacgtacta ctgtcaacag aggtggtatt atcctcatac gttcggccaa 300

gggaccaagg tggaaatcaa acgg 324


<210> 101
<211> 324
<212> DNA
<213> Artificial Sequence

<220>
<223> Nucleic Acid Sequence Encoding Synthetic Antibody Domain

<400> 101
gacatccaga tgacccagtc tccatcctcc ctgtctgcat ctgtaggaga ccgtgtcacc 60

atcacttgcc gggcaagtca gagcattttt atgaatttat tgtggtacca gcagaaacca 120

gggaaagccc ctaagctcct gatctataat gcatccgtgt tgcaaagtgg ggtcccatca 180

cgtttcagtg gcagtggatc tgggacagat ttcactctca ccatcagcag tctgcaacct 240

gaagattttg ctacgtacta ctgtcaacag gttgtgtggc gtccttttac gttcggccaa 300

gggaccaagg tggaaatcaa acgc 324


<210> 102
<211> 323
<212> DNA
<213> Artificial Sequence

<220>
<223> Nucleic Acid Sequence Encoding Synthetic Antibody Domain

<400> 102
gacatccaga tgacccagtc tccatcctcc ctgtctgcat ctgtaggaga ccgtgtcacc 60

atcacttgcc gggcaagtca gagcatttgg acgaagttac attggtacca gcagaaacca 120

gggaaagccc ctaagctcct gatctatatg gcatccagtt tgcaaagtgg ggtcccatca 180

cgtttcagtg gcagtggatc tgggacagat ttcactctca ccatcagcag tctgcaacct 240

gaagattttg ctacgtacta ctgtcaacag tggtttagta atcctagtac gttcggccaa 300

gggaccaagg tggaaatcaa acg 323

<210> 103
<211> 324
<212> DNA
<213> Artificial Sequence

<220>
<223> Nucleic Acid Sequence Encoding Synthetic Antibody Domain

<400> 103
gacatccaga tgacccagtc tccatcctcc ctgtctgcat ctgtaggaga ccgtgtcacc 60

atcacttgcc gggcaagtca gagcattgag cattatttat ggtggtacca gcagaaacca 120

gggaaagccc ctaagctcct gatctatgct gcatcctatt tgcaaagtgg ggtcccatca 180

cgtttcagtg gcagtggatc tgggacagat ttcactctca ccatcagcag tctgcaacct 240

gaagattttg ctacgtacta ctgtcaacag agtttggcgt gtcctcctac gttcggccaa 300

gggaccaagg tggaaatcaa acgg 324

<210> 104
<211> 324
<212> DNA
<213> Artificial Sequence

<220>
<223> Nucleic Acid Sequence Encoding Synthetic Antibody

<400> 104
gacatccaga tgacccagtc tccatcctcc ctgtctgcat ctgtaggaga ccgtgtcacc 60

atcacttgcc gggcaagtca gagcatttat ggtcatttat tgtggtacca gcagaaacca 120

gggaaagccc ctaagctcct gatctatgct gcatccagtt tgcaaagtgg ggtcccatca 180

cgtttcagtg gcagtggatc tgggacagat ttcactctca ccatcagcag tctgcaacct 240

gaagattttg ctacgtacta ctgtcaacag cctttggtgc ggccttttac gttcggccaa 300

gggaccaagg tggaaatcaa acgg 324

<210> 105
<211> 324
<212> DNA

<213>  Artificial Sequence

<220>
<223>  Nucleic Acid Sequence Encoding Synthetic Antibody Domain

<400>  105
gacatccaga tgacccagtc tccatcctcc ctgtctgcat ctgtaggaga ccgtgtcacc      60

atcacttgcc gggcaagtca gagcattgct aagttgttat attggtacca gcagaaacca     120

gggaaagccc ctaagctcct gatctatgat gcatcctctt tgcaaagtgg ggtcccatca     180

cgtttcagtg gcagtggatc tgggacagat ttcactctca ccatcagcag tctgcaacct     240

gaagattttg ctacgtacta ctgtcaacag tggtgggggt atcctggtac gttcggccaa     300

gggaccaagg tggaaatcaa acgg                                            324


<210>  106
<211>  324
<212>  DNA
<213>  Artificial Sequence

<220>
<223>  Nucliec Acid Sequence Encoding Synthetic Antibody Domain

<400>  106
gacatccaga tgacccagtc tccatcctcc ctgtctgcat ctgtaggaga ccgtgtcacc      60

atcacttgcc gggcaagtca gagcattttt cctgctttac tttggtacca gcagaaacca     120

gggaaagccc ctaagctcct gatctatcat gcatccagtt tgcaaagtgg ggtcccatca     180

cgtttcagtg gcagtggatc tgggacagat ttcactctca ccatcagcag tctgcaacct     240

gaagatattg ctacgtacta ctgtcaacag gttgtgtggc gtccttttac gttcggccaa     300

gggaccaagg tggaaatcaa acgg                                            324


<210>  107
<211>  224
<212>  DNA
<213>  Artificial Sequence

<220>
<223>  Nucleic Acid Sequence Encoding Synthetic Antibody Domain

<400>  107
attggtacca gcagaaacca gggaaagccc ctaagctcct gatctatcag gcatccattt      60

tgcaaagtgg ggtcccatca cgtttcagtg gcagtggatc tgggacagat ttcactctca     120

ccatcagcag tctgcaacct gaagattttg ctacgtacta ctgtcaacag gttgtgtggc 180

gtccttttac gttcggccaa gggaccaagg tggaaatcaa acgg 224

<210> 108
<211> 324
<212> DNA
<213> Artificial Sequence

<220>
<223> Nucleic Acid Sequence Encoding Synthetic Antibody Domain

<400> 108
gacatccaga tgacccagtc tccatcctcc ctgtctgcat ctgtaggaga ccgtgtcacc 60

atcacttgcc gggcaagtca gagcattttt atgaatttat tgtggtacca gcagaaacca 120

gggaaagccc ctaagctcct gatctataat gcatccgtgt tgcaaagtgg ggtcccatca 180

cgtttcagtg gcagtggatc tgggacaggt ttcactctca ccatcagcag tctgcaacct 240

gaagattttg ctacgtacta ctgtcaacag gttgtgtggc gtccttttac gttcggccaa 300

gggaccaagg tggaaatcaa acgg 324

<210> 109
<211> 324
<212> DNA
<213> Artificial Sequence

<220>
<223> Nucleic Acid Sequence Encoding Synthetic Antibody Domain

<400> 109
gacatccaga tgacccagtc tccatcctcc ctgtctgcat ctgtaggaga ccgtgtcacc 60

atcacttgcc gggcaagtca gagcattttg aattctttac attggtacca gcagaaacca 120

gggaaagccc ctaagctcct gatctatcat gcatccactt tgcaaagtgg ggtcccatca 180

cgtttcagtg gcagtggatc tgggacagat ttcactctca ccatcagcag tctgcaacct 240

gaagattttg ctacgtacta ctgtcaacag gttgtgtggc gtccttttac gttcggccaa 300

gggaccaagg tggaaatcaa acgg 324

<210> 110
<211> 324
<212> DNA

<213>  Artificial Sequence

<220>
<223>  Nucleic Acid Sequence Encoding Synthetic Antibody Domain

<400>  110
gacatccaga tgacccagtc tccatcctcc ctgtctgcat ctgtaggaga ccgtgtcacc      60

atcacttgcc gggcaagtca gagcattttg aattctttac attggtacca gcagaaacca     120

gggaaagccc ctaagctcct gatctatcat gcatccactt tgcaaagtgg ggtcccatca     180

cgtttcagtg gcagtggatc tgggacagat ttcactctca ccatcagcag tctgcaacct     240

gaagattttg ctacgtacta ctgtcaacag gttgtgtggc gtccttttac gttcggccaa     300

gggaccaagg tggaaatcaa acgg                                            324


<210>  111
<211>  325
<212>  DNA
<213>  Artificial Sequence

<220>
<223>  Nucleic Acid Encoding Synthetic Antibody Domain

<400>  111
gacatccaga tgacccagtc tccatcctcc ctgtctgcat ctgtaggaga ccgtgtcacc      60

atcacttgcc gggcaagtca gagcattgat aattatttac attggtacca gcagaaacca     120

gggaaagccc ctaagctcct gatctattct gcatcccatt tgcaaagtgg ggtcccatca     180

cgtttcagtg gcagtggatc tgggacagat ttcactctca ccatcagcag tctgcaacct     240

gaagattttg ctacgtacta ctgtcaacag gttgtgtggc gtccttttac gttcggccaa     300

gggaccaagg tggaaatcaa acggv                                           325


<210>  112
<211>  324
<212>  DNA
<213>  Artificial Sequence

<220>
<223>  Nucleic Acid Sequence Encoding Synthetic Antibody Domain

<400>  112
gacatccaga tgacccagtc tccatcctcc ctgtctgcat ctgtaggaga ccgtgtcacc      60

atcacttgcc gggcaagtca gagcattaat gagtatttac attggtacca gcagaaacca     120

gggaaagccc ctaagctcct gatctattct gcatccgtgt tgcaaagtgg ggtcccatca 180

cgtttcagtg gcagtggatc tgggacagat ttcactctca ccatcagcag tctgcaacct 240

gaagattttg ctacgtacta ctgtcaacag gttgtgtggc gtccttttac gttcggccaa 300

gggaccaagg tggaaatcaa acgg 324

<210> 113
<211> 324
<212> DNA
<213> Artificial Sequence

<220>
<223> Nucleic Acid Sequence Encoding Synthetic Antibody Domain

<400> 113
gacatccaga tgacccagtc tccatcctcc ctgtctgcat ctgtaggaga ccgtgtcacc 60

atcacttgcc gggcaagtca gagcattaat tatgctttac attggtacca gcagaaacca 120

gggaaagccc ctaagctcct gatctatcag gcatccattt tgcaaagtgg ggtcccatca 180

cgtttcagtg gcagtggatc tgggacagat ttcactctca ccatcagcag tctgcaacct 240

gaagattttg ctacgtacta ctgtcaacag gttgtgtggc gtccttttac gttcggccaa 300

gggaccaagg tggaaatcaa acgg 324

<210> 114
<211> 324
<212> DNA
<213> Artificial Sequence

<220>
<223> Nucleic Acid Sequence Encoding Synthetic Antibody Domain

<400> 114
gacatccaga tgacccagtc tccatcctcc ctgtctgcat ctgtaggaga ccgtgtcacc 60

atcacttgcc gggcaagtca gagcattgat agttttttac attggtacca gcagaaacca 120

gggaaagccc ctaagctcct gatctatagt gcatccgagt tgcaaagtgg ggtcccatca 180

cgtttcagtg gcagtggatc tgggacagat ttcactctca ccatcagcag tctgcatcct 240

gaagattttg ctacgtacta ctgtcaacag gttgtgtggc gtccttttac gttcggccaa 300

gggaccaagg tggaaatcaa acgg 324

<210>  115
<211>  324
<212>  DNA
<213>  Artificial Sequence

<220>
<223>  Nucleic Acid Sequence Encoding Synthetic Antibody Domain

<400>  115
gacatccaga tgacccagtc tccatcctct ctgtctgcat ctgtaggaga ccgtgtcacc      60

atcacttgcc gggcaagtca gagcattgat agttatttac attggtacca gcagaaacca     120

gggaaagccc ctaagctcct gatctatagt gcatccgagt tgcaaagtgg ggtcccatca     180

cgtttcagtg gcagtggatc tgggacagat ttcactctca ccatcagcag tctgcaacct     240

gaagattttg ctacgtacta ctgtcaacag gttgtgtggc gtccttttac gttcggccaa     300

gggaccaagg tggaaatcaa acgg                                            324


<210>  116
<211>  324
<212>  DNA
<213>  Artificial Sequence

<220>
<223>  Nucleic Acid Sequence Encoding Synthetic Antibody Domain

<400>  116
gacatccaga tgacccagtc tccatcctcc ctgtctgcat ctgtaggaga ccgtgtcacc      60

atcacttgcc gggcaagtca gagcattgat cagtatttac attggtacca gcagaaacca     120

gggaaagccc ctaagctcct gatctatggt gcatccaatt tgcaaagtga ggtcccatca     180

cgtttcagtg gcagtggatc tgggacagat ttcactctca ccatcagcag tctgcaacct     240

gaagattttg ctacgtacta ctgtcaacag gttgtgtggc gtccttttac gttcggccaa     300

gggaccaagg tggaaatcaa acgg                                            324


<210>  117
<211>  324
<212>  DNA
<213>  Artificial Sequence

<220>
<223>  Nucleic Acid Sequence Encoding Synthetic Antibody Domain

<400> 117
gacatccaga tgacccagtc tccatcctcc ctgtctgcat ctgtaggaga ccgtgtcacc 60

atcacttgcc gggcaagtca gagcattgat agttttttac attggtacca gcagaaacca 120

gggaaagccc ctaagctcct gatctatagt gcatccgagt tgcaaagtgg ggtcccatca 180

cgtttcagtg gcagtggatc tgggacagat ttcactctca ccatcagcag tctgcatcct 240

gaagattttg ctacgtacta ctgtcaacag gttgtgtggc gtccttttac gttcggccaa 300

gggaccaagg tggaaatcaa acgg 324


<210> 118
<211> 324
<212> DNA
<213> Artificial Sequence

<220>
<223> Nucleic Acid Sequence Encoding Synthetic Antibody Domain

<400> 118
gacatccaga tgacccagtc tccatcctcc ctgtctgcat ctgtaggaga ccgtgtcacc 60

atcacttgcc gggcaagtca gagcattgat tcttatttac attggtacca gcagaaacca 120

gggaaagccc ctaagctcct gatctatagt gcatccctgt tgcaaagtgg ggtcccatca 180

cgtttcagtg gcagtggatc tgggacagat ttcactctca ccatcagcag tctgcaacct 240

gaagattttg ctacgtacta ctgtcaacag gttgtgtggc gtccttttac gttcggccaa 300

gggaccaagg tggaaatcaa acgg 324


<210> 119
<211> 324
<212> DNA
<213> Artificial Sequence

<220>
<223> Nucleic Acid Sequence Encoding Synthetic Antibody Domain

<400> 119
gacatccaga tgacccagtc tccatcctcc ctgtctgcat ctgtaggaga ccgtgtcacc 60

atcacttgcc gggcaagtca gagcattgat cagtatttac attggtacca gcagaaacca 120

gggaaagccc ctaagctcct gatctattct gcatcccttt tgcaaagtgg ggtcccatca 180

cgtttcagtg gcagtggatc tgggacagat ttcactctca ccatcagcag tctgcaacct 240

gaagattttg ctacatacta ctgtcaacag gttgtgtggc gtccttttac gttcggccaa 300

gggaccaagg tggaaatcaa acgg 324

<210> 120
<211> 324
<212> DNA
<213> Artificial Sequence

<220>
<223> Nucleic Acid Sequence Encoding Synthetic Antibody Domain

<400> 120
gacatccaga tgacccagtc tccatcctcc ctgtctgcat ctgtaggaga ccgtgtcacc 60

atcacttgcc gggcaagtca aagcattgat gagtttttac attggtacca gcagaaacca 120

gggaaagccc ctaagctcct gatctattgt gcatcccagt tgcaaagtgg ggtcccatca 180

cgtttcagtg gcagtggatc tgggacagat ttcactctca ccatcagcag tctacatcct 240

gaagattttg ctacgtacta ctgtcaacag gttgtgtggc gtccttttac gttcggccaa 300

gggaccaagg tggaaatcaa acgg 324

<210> 121
<211> 324
<212> DNA
<213> Artificial Sequence

<220>
<223> Nucleic Acid Sequence Encoding Synthetic Antibody Domain

<400> 121
gacatccaga tgacccagtc tccatcctcc ctgtctgcat ctgtaggaga ccgtgtcacc 60

atcacttgcc gggcaagtca gagcattgat gcgtatttac attggtacca gcagaaacca 120

gggaaagccc ctaagctcct gatctattct gcatccctgt tgcaaagtgg ggtcccatca 180

cgtttcagtg gcagtggatc tgggacagat ttcactctca ccatcagcag tctgcaacct 240

gaagattttg ctacgtacta ctgtcaacag gttgtgtggc gtccttttac gttcggccaa 300

gggaccaagg tggaaatcaa acgg 324

<210> 122
<211> 324
<212> DNA
<213> Artificial Sequence

<220>
<223>  Nucleic Acid Sequence Encoding Synthetic Antibody Domain

<400>  122
gacatccaga tgacccagtc tccatcctcc ctgtctgcat ctgtaggaga ccgtgtcacc      60

atcacttgcc gggcaagtca gagcattgat aggtatttac attggtacca gcagaaacca     120

gggaaagccc ctaagctcct gatctatagt gcatccgtgt tgcaaagtgg ggtcccatca     180

cgtttcagtg gcagtggatc tgggacagat ttcaccctca ccatcagcag tctgcagcct     240

gaagattttg ctacgtacta ctgtcaacag gttgtgtggc gtccttttac gttcggccaa     300

gggaccaagg tggaaatcaa acgg                                            324


<210>  123
<211>  324
<212>  DNA
<213>  Artificial Sequence

<220>
<223>  Nucleic Acid Sequence Encoding Synthetic Antibody Domain

<400>  123
gacatccaga tgacccagtc tccatcctcc ctgtctgcat ctgtaggaga ccgtgtcacc      60

atcacttgcc gggcaagtca gagcattgat aagtatttac attggtacca gcagaaacca     120

gggaaagccc ctaagctcct gatctatagt gcatcctcgt tgcaaagtgg ggtcccatca     180

cgtttcagtg gcagtggatc tgggacagat ttcactctca ccatcagcag tctgcaacct     240

gaagattttg ctacgtacta ctgtcaacag gttgtgtggc gtccttttac gttcggccaa     300

gggaccaagg tggaaatcaa acgg                                            324


<210>  124
<211>  324
<212>  DNA
<213>  Artificial Sequence

<220>
<223>  Nucleic Acid Sequence Encoding Synthetic Antibody Domain

<400>  124
gacatccaga tgacccagtc tccatcctcc ctgtctgcat ctgtaggaga ccgtgtcacc      60

atcacttgcc gggcaagtca gagcattgat cattatttac attggtacca gcagaaacca     120

gggaaagccc ctaagctcct gatctatagt gcatccgttt tgcaaagtgg ggtcccatca 180

cgtttcagtg gcagtggatc tgggacagat ttcactctca ccatcagcag tctgcaacct 240

gaagattttg caacgtacta ctgtcaacag gttgtgtggc gtccttttac gttcggccaa 300

gggaccaagg tggaaatcaa acgg 324

<210> 125
<211> 324
<212> DNA
<213> Artificial Sequence

<220>
<223> Nucleic Acid Sequence Encoding Synthetic Antibody Domain

<400> 125
gacatccaga tgacccagtc tccatcctcc ctgtctgcat ctgtaggaga ccgtgtcacc 60

atcacttgcc gggcaagtca gagcattgat gagtttttac attggtacca gcagaaacca 120

gggaaagccc ctaagctcct gatctatagt gcatccattt tgcaaagtgg ggtcccatca 180

cgtttcagtg gcagtggatc tgggacagat ttcactctca ccatcagcag tctgcaacct 240

gaagattttg ctacgtacta ctgtcaacag gttgtgtggc gtccttttac gttcggccaa 300

gggaccaagg tggaaatcaa acgg 324

<210> 126
<211> 324
<212> DNA
<213> Artificial Sequence

<220>
<223> Nucleic Acid Sequence Encoding Synthetic Antibody Domain

<400> 126
gacatccaga tgacccagtc tccatcctcc ctgtctgcat ctgtaggaga ccgtgtcacc 60

atcacttgcc gggcaagtca gagcattcag actgcgttac tgtggtacca gcagaaacca 120

gggaaagccc ctaagctcct gatctataat gcatccagtt tgcaaagtgg ggtcccatca 180

cgtttcagtg gcagtggatc tgggacagat ttcactctca ccatcagcag tctgcaacct 240

gaagattttg ctacatacta ctgtcaacag gttgtgtggc gtccttttac gttcggccaa 300

gggaccaagg tggaaatcaa acgg 324

<210>  127
<211>  324
<212>  DNA
<213>  Artificial Sequence

<220>
<223>  Nucleic Acid Sequence Encoding Synthetic Antibody Domain

<400>  127
gacatccaga tgacccagtc tccatcctcc ctgtctgcat ctgtaggaga ccgtgtcacc      60

atcacttgcc gggcaagtca gagcattgat cagtatttac attggtacca gcagaaacca     120

gggaaagccc ctaagctcct gatctatggt gcatccaatt tgcaaagtgg ggtcccatca     180

cgtttcagtg gcagtggatc tgggacagat ttcactctca ccatcagcag tctgcaacct     240

gaagattttg ctacgtacta ctgtcaacag gttgtgtggc gtccttttac gttcggccaa     300

gggaccaagg tggaaatcaa acgg                                            324

<210>  128
<211>  324
<212>  DNA
<213>  Artificial Sequence

<220>
<223>  Nucleic Acid Sequence Encoding Synthetic Antibody Domain

<400>  128
gacatccaga tgacccagtc tccatcctcc ctgtctgcat ctgtaggaga ccgtgtcacc      60

atcacttgcc gggcaagtca gagcattgat aattatttac attggtacca gcagaaacca     120

gggaaagccc ctaagctcct gatctatagt gcatcccagt tgcaaagtgg ggtcccatca     180

cgtttcagtg gcagtggatc tgggacagat ttcactctca ccatcagcag tctgcaacct     240

gaagattttg ctacgtacta ctgtcaacag gttgtgtggc gtccttttac gttcggccaa     300

gggaccaagg tggaaatcaa acgg                                            324

<210>  129
<211>  324
<212>  DNA
<213>  Artificial Sequence

<220>
<223>  Nucleic Acid Sequence Encoding Synthetic Antibody Domain

<400>  129

gacatccaga tgacccagtc tccatcctcc ctgtctgcat ctgtaggaga ccgtgtcacc 60

atcacttgcc gggcaagtca gagcattgat aattttttac attggtacca gcagaaacca 120

gggaaagccc ctaagctcct gatctatagt gcatccgagt tgcaaagtgg ggtcccatca 180

cgtttcagtg gcagtggatc tgggacagat ttcactctca ccatcagcag tctgcaacct 240

gaagattttg ctacgtacta ctgtcaacag gttgtgtggc gtccttttac gttcggccaa 300

gggaccaagg tggaaatcaa acgg 324

<210> 130
<211> 324
<212> DNA
<213> Artificial Sequence

<220>
<223> Nucleic Acid Sequence Encoding Synthetic Antibody Domain

<400> 130
gacatccaga tgacccagtc tccatcctcc ctgtctgcat ctgtaggaga ccgtgtcacc 60

atcacttgcc gggcaagtca gagcattgat gagtatttac attggtacca gcagaaacca 120

gggaaacccc ctaagctcct gatctattct gcatccagtt tgcaaagtgg ggtcccatca 180

cgtttcagtg gcagtggatc tgggacagat ttcactctca ccatcagcag tctgcaacct 240

gaagattttg ctacgtacta ctgtcaacag gttgtgtggc gtccttttac gttcggccaa 300

gggaccaagg tggaaatcaa acgg 324

<210> 131
<211> 324
<212> DNA
<213> Artificial Sequence

<220>
<223> Nucleic Acid Sequence Encoding Synthetic Antibody Domain

<400> 131
gacatccaga tgacccagtc tccatcctcc ctgtctgcat ctgtaggaga ccgtgtcacc 60

atcacttgcc gggcaagtca gagcattgat cattttttac attggtacca gcagaaacca 120

gggaaagccc ctaagctcct gatctatagt gcatccgagt tgcaaagtgg ggtcccatca 180

cgtttcagtg gcagtggatc tgggacagat ttcactctca ccatcagcag tctgcaacct 240

gaagattttg ctacgtacta ctgtcaacag gttgtgtggc gtccttttac gttcggccaa 300

gggaccaagg tggaaatcaa acgg                                          324

<210>  132
<211>  324
<212>  DNA
<213>  Artificial Sequence

<220>
<223>  Nucleic Acid Sequence Encoding Synthetic Antibody Domain

<400>  132
gacatccaga tgacccagtc tccatcctcc ctgtctgcat ctgtaggaga ccgtgtcacc      60

atcacttgcc gggcaagtca gagcattgat aattatttac attggtacca gcagaaacca     120

gggaaagccc ctaagctcct gatctattcg gcatccatgt tgcaaagtgg ggtcccatca     180

cgtttcagtg gcagtggatc tgggacagat ttcactctca ccatcagcag tctgcaacct     240

gaagattttg ctacgtacta ctgtcaacag gttgtgtggc gtccttttac gttcggccaa     300

gggaccaagg tggaaatcaa acgg                                          324

<210>  133
<211>  324
<212>  DNA
<213>  Artificial Sequence

<220>
<223>  Nucleic Acid Sequence Encoding Synthetic Antibody Domain

<400>  133
gacatccaga tgacccagtc tccatcctcc ctgtctgcat ctgtaggaga ccgtgtcacc      60

atcacttgcc gggcaagtca gagcattgat gagtatttac attggtacca gcagaaacca     120

gggaaagccc ccaagctcct gatctattct gcatccattt tgcaaagtgg ggtcccatca     180

cgtttcagtg gcagtggatc tgggacagat ttcactctca ccatcagcag tctgcaacct     240

gaagattttg ctacgtacta ctgtcaacag gttgtgtggc gtccttttac gttcggccaa     300

gggaccaagg tggaaatcaa acgg                                          324

<210>  134
<211>  324
<212>  DNA
<213>  Artificial Sequence

<220>
<223>  Nucleic Acid Sequence Encoding Synthetic Antibody Domain

<400>  134
gacatccaga tgacccagtc tccatcctcc ctgtctgcat ctgtaggaga ccgtgtcacc      60

atcacttgcc gggcaagtca gagcattgat gagtttttac attggtacca gcagaaacca     120

gggaaagccc ctaagctcct gatctattcg gcatccgctt tgcaaagtgg ggtcccatca     180

cgtttcagtg gcagtggatc tgggacagat ttcactctca ccatcagcag tctgcaacct     240

gaagattttg ctacgtacta ctgtcaacag gttgtgtggc gtccttttac gttcggccaa     300

gggaccaagg tggaaatcaa acgg                                            324


<210>  135
<211>  324
<212>  DNA
<213>  Artificial Sequence

<220>
<223>  Nucleic Acid Sequence Encoding Synthetic Antibody Domain

<400>  135
gacatccaga tgacccagtc tccatcctcc ctgtctgcat ctgtaggaga ccgtgtcacc      60

atcacttgcc gggcaagtca gagcattgat gagtatttac attggtacca gcagaaacca     120

gggaaagccc ctaagctcct gatctattct gcatccattt tgcaaagtgg ggtcccatca     180

cgtttcagtg gcagtggatc tgggacagat ttcactctca ccatcagcag tctgcaccct     240

gaagattttg ctacgtacta ctgtcaacag gttgtgtggc gtccttttac gttcggccaa     300

gggaccaagg tggaaatcaa acgg                                            324


<210>  136
<211>  324
<212>  DNA
<213>  Artificial Sequence

<220>
<223>  Nucleic Acid Sequence Encoding Synthetic Antibody Domain

<400>  136
gacatccaga tgacccagtc tccatcctcc ctgtctgcat ctgtaggaga ccgtgtcacc      60

atcacttgcc gggcaagtca gagcattgat aattatttac attggtacca gcagaaacca     120

gggaaagccc ctaagctcct gatctatgct gcatccagtt tgcaaagtgg ggtcccatca     180

cgtttcagtg gcagtggatc tgggacagat ttcactctca ccatcagcag tctgcaacct 240

gatgattttg ctacgtacta ctgtcaacag gttgtgtggc gtccttttgc gttcggccaa 300

gggaccaagg tggaaatcaa acgg 324

<210> 137
<211> 324
<212> DNA
<213> Artificial Sequence

<220>
<223> Nucleic Acid Sequence Encoding Synthetic Antibody Domain

<400> 137
gacatccaga tgacccagtc tccatcctcc ctgtctgcat ctgtaggaga ccgtgtcacc 60

atcacttgcc gggcaagtca gagcattgat agttatttac attggtacca gcagaaacca 120

gggaaagccc ctaagctcct gatctatagt gcatcaaatt tagaaacagg ggtcccatca 180

cgtttcagtg gcagtggatc tgggacagat ttcactctca ccatcagcag tctgcaacct 240

gaagattttg ctacgtacta ctgtcaacag gttgtgtggc gtccttttac gttcggccaa 300

gggaccaagg tggaaatcaa acgg 324

<210> 138
<211> 324
<212> DNA
<213> Artificial Sequence

<220>
<223> Nucleic Acid Sequence Encoding Synthetic Antibody Domain

<400> 138
gacatccaga tgacccagtc tccatcctcc ctgtctgcat ctgtaggaga ccgtgtcacc 60

atcacttgcc gggcaagtca ggtgatttgg gatgcgttag attggtacca gcagaaacca 120

gggaaagccc ctaagctcct gatctatagt gcgtcccgtt tgcaaagtgg ggtcccatca 180

cgtttcagtg gcagtggatc tgggacagat ttcactctca ccatcagcag tctgcatcct 240

gaagattttg ctacgtacta ctgtcaacag tatgctgtgt ttcctgtgac gttcggccaa 300

gggaccaagg tggaaatcaa acgg 324

<210> 139

<211>  324
<212>  DNA
<213>  Artificial Sequence

<220>
<223>  Nucleic Acid Sequence Encoding Synthetic Antibody Domain

<400>  139
gacatccaga tgacccagtc tccatcctcc ctgtctgcat ctgtaggaga ccgtgtcacc      60

atcacttgcc gggcaagtca gactatttat gatgcgttaa gttggtacca gcagaaacca     120

gggaaagccc ctaagctcct gatctatggt ggttccaggt tgcaaagtgg ggtcccatca     180

cgtttcagtg gcggtggatc tgggacagat ttcactctca ccatcagcag tctgcaacct     240

gaagattttg ctacgtacta ctgtcaacag tataagacta agcctttgac gttcggccaa     300

gggaccaagg tggaaatcaa acgg                                            324


<210>  140
<211>  324
<212>  DNA
<213>  Artificial Sequence

<220>
<223>  Nucleic Acid Sequence Encoding Synthetic Antibody Domain

<400>  140
gacatccaga tgacccagtc cccatcctcc ctgtctgcat ctgtaggaga ccgtgtcacc      60

atcacttgcc gggcaagtca gactatttat gatgcgttaa gttggtacca gcagaaacca     120

gggaaagccc ctaagctcct gatctatggt ggttccaggt tgcaaagtgg ggtcccatca     180

cgtttcagtg gtagtggatc tgggacagat ttcactctca ccatcagcag tctgcaaccc     240

gaagattttg ctacgtacta ctgtcaacag tatgctcgtt atcctcttac gttcggccaa     300

gggaccaagg tggaaatcaa acgg                                            324


<210>  141
<211>  108
<212>  PRT
<213>  Artificial Sequence

<220>
<223>  Synthetic Antibody Domain

<400>  141

Asp Ile Gln Met Thr Gln Ser Pro Ser Ser Leu Ser Ala Ser Val Gly
1               5                   10                  15

Asp Arg Val Thr Ile Thr Cys Arg Ala Ser Gln Asp Ile Glu Glu Trp
            20                  25                  30

Leu Met Trp Tyr Gln Gln Lys Pro Gly Lys Ala Pro Lys Leu Leu Ile
        35                  40                  45

Tyr Asn Ser Ser Thr Leu Gln Ser Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Tyr Ala Thr Tyr Tyr Cys Gln Gln Pro Leu Ser Arg Pro Phe
                85                  90                  95

Thr Phe Gly Gln Gly Thr Lys Val Glu Ile Lys Arg
            100                 105

<210>  142
<211>  108
<212>  PRT
<213>  Artificial Sequence

<220>
<223>  Synthetic Antibody Domain

<400>  142

Asp Ile Gln Met Thr Gln Ser Pro Ser Ser Leu Ser Ala Ser Val Gly
1               5                   10                  15

Asp Arg Val Thr Ile Thr Cys Arg Ala Ser Gln His Ile Asp Asp Trp
            20                  25                  30

Leu Phe Trp Tyr Gln Gln Lys Pro Gly Lys Ala Pro Lys Leu Leu Ile
        35                  40                  45

Tyr Arg Ala Ser Phe Leu Gln Ser Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Thr Tyr Tyr Cys Gln Gln Pro Leu Ser Arg Pro Phe
                85                  90                  95

Thr Phe Gly Gln Gly Thr Lys Val Glu Ile Lys Arg
            100                 105

<210>  143
<211>  108
<212>  PRT
<213>  Artificial Sequence

<220>
<223>  Synthetic Antibody Domain

<400>  143

Asp Ile Gln Met Thr Gln Ser Pro Ser Ser Leu Ser Ala Ser Val Gly
1               5                   10                  15

Asp Arg Val Thr Ile Thr Cys Arg Ala Ser Gln Phe Ile Glu Asp Trp
            20                  25                  30

Leu Phe Trp Tyr Gln Gln Lys Pro Gly Lys Ala Pro Lys Leu Leu Ile
        35                  40                  45

Tyr Gln Ala Ser Lys Leu Gln Ser Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Thr Tyr Tyr Cys Gln Gln Pro Leu Ser Arg Pro Phe
                85                  90                  95

Thr Phe Gly Gln Gly Thr Lys Val Glu Ile Lys Arg
            100                 105

<210>  144
<211>  108

<212>  PRT
<213>  Artificial Sequence

<220>
<223>  Synthetic Antibody Domain

<400>  144

Asp Ile Gln Met Thr Gln Ser Pro Ser Ser Leu Ser Ala Ser Val Gly
1               5                   10                  15

Asp Arg Val Thr Ile Thr Cys Arg Ala Ser Gln Pro Ile Asp Ser Trp
            20                  25                  30

Leu Met Trp Tyr Gln Gln Lys Pro Gly Lys Ala Pro Lys Leu Leu Ile
        35                  40                  45

Tyr Gln Ala Ser Arg Leu Gln Ser Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Thr Tyr Tyr Cys Gln Gln Pro Leu Ser Arg Pro Phe
                85                  90                  95

Thr Phe Gly Gln Gly Thr Lys Val Glu Ile Lys Arg
            100                 105

<210>  145
<211>  108
<212>  PRT
<213>  Artificial Sequence

<220>
<223>  Synthetic Antibody Domain

<400>  145

Asp Ile Gln Met Thr Gln Ser Pro Ser Ser Leu Ser Ala Ser Val Gly
1               5                   10                  15

Asp Arg Val Thr Ile Thr Cys Arg Ala Ser Gln His Ile Asp Asp Trp
            20                  25                  30

Leu Phe Trp Tyr Gln Gln Lys Pro Gly Lys Ala Pro Lys Leu Leu Ile
        35                  40                  45

Tyr Arg Ala Ser Phe Leu Gln Ser Gly Val Pro Pro Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Thr Tyr Tyr Cys Gln Gln Pro Leu Ser Arg Pro Phe
                85                  90                  95

Thr Phe Gly Gln Gly Thr Lys Val Glu Ile Lys Arg
            100                 105

<210> 146
<211> 108
<212> PRT
<213> Artificial Sequence

<220>
<223> Synthetic Antibody Domain

<400> 146

Asp Ile Gln Met Thr Gln Ser Pro Ser Ser Leu Ser Ala Ser Val Gly
1               5                   10                  15

Asp Arg Val Thr Ile Thr Cys Arg Ala Ser Gln Asn Ile Asp Asp His
            20                  25                  30

Leu Met Trp Tyr Gln Gln Lys Pro Gly Lys Ala Pro Lys Leu Leu Ile
        35                  40                  45

Tyr Ser Ser Ser Ile Leu Gln Ser Gly Val Pro Pro Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Thr Tyr Tyr Cys Gln Gln Pro Leu Ser Arg Pro Phe
                85                  90                  95

Thr Phe Gly Gln Gly Thr Lys Val Glu Ile Lys Arg
            100                 105

<210>  147
<211>  108
<212>  PRT
<213>  Artificial Sequence

<220>
<223>  Synthetic Antibody Domain

<400>  147

Asp Ile Gln Met Thr Gln Ser Pro Ser Ser Leu Ser Ala Ser Val Gly
1               5                   10                  15

Asp Arg Val Thr Ile Thr Cys Arg Ala Ser Gln Asp Ile Asp His Ala
            20                  25                  30

Leu Leu Trp Tyr Gln Gln Lys Pro Gly Lys Ala Pro Arg Leu Leu Ile
        35                  40                  45

Tyr Asn Gly Ser Met Leu Gln Ser Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Thr Tyr Tyr Cys Gln Gln Val Leu Arg Arg Pro Phe
                85                  90                  95

Thr Phe Gly Gln Gly Thr Lys Val Glu Ile Lys Arg
            100                 105

<210>  148
<211>  108
<212>  PRT
<213>  Artificial Sequence

<220>
<223>  Synthetic Antibody Domain

<400>  148

Asp Ile Gln Met Thr Gln Ser Pro Ser Ser Leu Ser Ala Ser Val Gly
1               5                   10                  15

Asp Arg Val Thr Ile Thr Cys Arg Ala Ser Gln His Ile Gly Asp Trp
            20                  25                  30

Leu Leu Trp Tyr Gln Gln Lys Pro Gly Lys Ala Pro Met Leu Leu Ile
        35                  40                  45

Tyr Gln Ser Ser Arg Leu Gln Ser Gly Val Pro Ser Arg Phe Ser Gly
    50                  55                  60

Ser Gly Ser Gly Thr Asp Phe Ile Leu Thr Ile Ser Ser Leu Gln Pro
65                  70                  75                  80

Glu Asp Phe Ala Thr Tyr Tyr Cys Gln Gln Pro Leu Ser Arg Pro Phe
                85                  90                  95

Thr Phe Gly Gln Gly Thr Lys Val Glu Ile Lys Arg
            100                 105

<210>  149
<211>  108
<212>  PRT
<213>  Artificial Sequence

<220>
<223>  Synthetic Antibody Domain

<400>  149

Asp Ile Gln Met Thr Gln Ser Pro Ser Ser Leu Ser Ala Ser Val Gly
1               5                   10                  15

Asp Arg Val Thr Ile Thr Cys Arg Ala Ser Gln His Ile Asp Ser Tyr
            20                  25                  30

Leu Met Trp Tyr Gln Gln Lys Pro Gly Lys Ala Pro Lys Leu Leu Ile
        35                  40                  45

Tyr Asn Thr Ser Val Leu Gln Ser Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Thr Tyr Tyr Cys Gln Gln Pro Leu Ser Arg Pro Phe
                85                  90                  95

Thr Phe Gly Gln Gly Thr Lys Val Glu Ile Lys Arg
            100                 105

<210>  150
<211>  108
<212>  PRT
<213>  Artificial Sequence

<220>
<223>  Synthetic Antibody Domain

<400>  150

Asp Ile Gln Met Thr Gln Ser Pro Ser Ser Leu Ser Ala Ser Val Gly
1               5                   10                  15

Asp Arg Val Thr Ile Thr Cys Arg Ala Ser Gln Trp Ile Asp Asp His
            20                  25                  30

Leu Phe Trp Tyr Gln Gln Lys Pro Gly Lys Ala Pro Lys Leu Leu Ile
        35                  40                  45

Tyr Asn Thr Ser Thr Leu Gln Ser Gly Val Pro Ser Arg Phe Ser Gly
    50                  55                  60

Ser Gly Ser Gly Thr Asp Phe Ile Leu Thr Ile Ser Ser Leu Gln Pro
65                  70                  75                  80

Glu Asp Phe Ala Thr Tyr Tyr Cys Gln Gln Pro Leu Ser Arg Pro Phe
                85                  90                  95

Thr Phe Gly Gln Gly Thr Lys Val Glu Ile Lys Arg
            100                 105

<210>  151

<211>  108
<212>  PRT
<213>  Artificial Sequence

<220>
<223>  Synthetic Antibody Domain

<400>  151

Asp Ile Gln Met Thr Gln Ser Pro Ser Ser Leu Ser Ala Ser Val Gly
1               5                   10                  15

Asp Arg Val Thr Ile Thr Cys Arg Ala Ser Gln Phe Ile Asp Glu His
            20                  25                  30

Leu Met Trp Tyr Gln Gln Lys Pro Gly Lys Ala Pro Lys Leu Leu Ile
        35                  40                  45

Tyr Arg Ser Ser Glu Leu Gln Ser Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Thr Tyr Tyr Cys Gln Gln Pro Leu Ser Arg Pro Phe
                85                  90                  95

Thr Phe Gly Gln Gly Thr Lys Val Glu Ile Lys Arg
            100                 105

<210>  152
<211>  108
<212>  PRT
<213>  Artificial Sequence

<220>
<223>  Synthetic Antibody Domain

<400>  152

Asp Ile Gln Met Thr Gln Ser Pro Ser Ser Leu Ser Ala Ser Val Gly
1               5                   10                  15

Asp Arg Val Thr Ile Thr Cys Arg Ala Ser Gln Trp Ile Asn Asn Trp
            20                  25                  30

Leu Leu Trp Tyr Gln Gln Lys Pro Gly Lys Ala Pro Lys Leu Leu Ile
        35                  40                  45

Tyr Glu Ser Ser Asn Leu Gln Ser Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Thr Tyr Tyr Cys Gln Gln Pro Leu Ser Arg Pro Phe
                85                  90                  95

Thr Phe Gly Gln Gly Thr Lys Val Glu Ile Lys Arg
            100                 105

<210>  153
<211>  107
<212>  PRT
<213>  Artificial Sequence

<220>
<223>  Synthetic Antibody Domain

<400>  153

Asp Ile Gln Met Thr Gln Ser Pro Ser Ser Leu Ser Ala Ser Val Gly
1               5                   10                  15

Asp Arg Val Thr Ile Thr Cys Arg Ala Ser Gln Leu Ile Asp Asp His
            20                  25                  30

Phe Trp Tyr Gln Gln Lys Pro Gly Lys Ala Pro Thr Leu Leu Ile Tyr
        35                  40                  45

Asn Ser Ser Val Leu Gln Ser Gly Val Pro Ser Arg Phe Ser Gly Ser
    50                  55                  60

Gly Ser Gly Thr Asp Phe Thr Leu Thr Ile Ser Ser Leu Gln Pro Glu
65                  70                  75                  80

Asp Phe Ala Thr Tyr Tyr Cys Gln Gln Pro Leu Ser Arg Pro Phe Thr

85                  90                  95

Phe Gly Gln Gly Thr Lys Val Glu Ile Lys Arg
            100                 105

<210>  154
<211>  108
<212>  PRT
<213>  Artificial Sequence

<220>
<223>  Synthetic Antibody Domain

<400>  154

Asp Ile Gln Met Thr Gln Ser Pro Ser Ser Leu Ser Ala Ser Val Gly
1               5                   10                  15

Asp Arg Val Thr Ile Thr Cys Arg Ala Ser Gln Asp Ile Asp Gln Trp
            20                  25                  30

Leu Met Trp Tyr Gln Gln Lys Pro Gly Lys Ala Pro Lys Leu Leu Ile
        35                  40                  45

Tyr Gln Ser Ser Met Leu Gln Ser Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Thr Tyr Tyr Cys Gln Gln Pro Leu Ser Arg Pro Phe
                85                  90                  95

Thr Phe Gly Gln Gly Thr Lys Val Glu Ile Lys Arg
            100                 105

<210>  155
<211>  108
<212>  PRT
<213>  Artificial Sequence

<220>
<223>  Synthetic Antibody Domain

<400> 155

Asp Ile Gln Met Thr Gln Ser Pro Ser Ser Leu Ser Ala Ser Val Gly
1               5                   10                  15

Asp Arg Val Thr Ile Thr Cys Gln Ala Ser Gln Asp Ile Asp Asn Trp
            20                  25                  30

Leu Leu Trp Tyr Gln Gln Lys Pro Gly Lys Ala Pro Lys Leu Leu Ile
        35                  40                  45

Tyr Gln Ala Ser Asn Leu Gln Ser Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Thr Tyr Tyr Cys Gln Gln Pro Leu Ser Arg Pro Phe
                85                  90                  95

Thr Phe Gly Gln Gly Thr Lys Val Glu Ile Lys Arg
            100                 105

<210>  156
<211>  108
<212>  PRT
<213>  Artificial Sequence

<220>
<223>  Synthetic Antibody Domain

<400>  156

Asp Ile Gln Met Thr Gln Ser Pro Ser Ser Leu Ser Ala Ser Val Gly
1               5                   10                  15

Asp Arg Val Thr Ile Thr Cys Arg Ala Ser Gln Pro Ile Asp Ser Trp
            20                  25                  30

Leu Met Trp Tyr Gln Gln Lys Pro Gly Lys Ala Pro Lys Leu Leu Ile
        35                  40                  45

Tyr Gln Ala Ser Arg Leu Gln Ser Gly Val Pro Ser Arg Phe Ser Gly

50                  55                  60

Ser Gly Ser Gly Thr Asp Phe Thr Leu Thr Ile Ser Ser Leu Gln Pro
65                  70                  75                  80

Glu Asp Phe Ala Thr Tyr Tyr Cys Gln Gln Pro Leu Ser Gly Pro Phe
                85                  90                  95

Thr Phe Gly Gln Gly Thr Lys Val Glu Ile Lys Arg
            100                 105

<210>  157
<211>  108
<212>  PRT
<213>  Artificial Sequence

<220>
<223>  Synthetic Antibody Domain

<400>  157

Asp Ile Gln Met Thr Gln Ser Pro Ser Ser Leu Ser Ala Ser Val Gly
1               5                   10                  15

Asp Arg Val Thr Ile Thr Cys Arg Ala Ser Gln Tyr Ile Asp Tyr Gly
            20                  25                  30

Leu Met Trp Tyr Gln Gln Lys Pro Gly Lys Ala Pro Lys Leu Leu Ile
        35                  40                  45

Tyr Arg Thr Ser Glu Leu Gln Ser Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Thr Tyr Tyr Cys Gln Gln Pro Leu Ser Arg Pro Phe
                85                  90                  95

Thr Phe Gly Gln Gly Thr Lys Val Glu Ile Lys Arg
            100                 105

<210>  158
<211>  108
<212>  PRT
<213>  Artificial Sequence

<220>
<223>  Synthetic Antibody Domain

<400>  158

Asp Ile Gln Met Thr Gln Ser Pro Ser Ser Leu Ser Ala Ser Val Gly
1               5                   10                  15

Asp Arg Val Thr Ile Thr Cys Arg Ala Ser Gln Trp Ile Asp Ser Phe
            20                  25                  30

Leu Met Trp Tyr Gln Gln Lys Pro Gly Lys Ala Pro Lys Leu Leu Ile
        35                  40                  45

Tyr Asn Gly Ser Val Leu Gln Ser Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Thr Tyr Tyr Cys Gln Gln Pro Leu Ser Arg Pro Phe
                85                  90                  95

Thr Phe Gly Gln Gly Thr Lys Val Glu Ile Lys Arg
            100                 105

<210>  159
<211>  108
<212>  PRT
<213>  Artificial Sequence

<220>
<223>  Synthetic Antibody Domain

<400>  159

Asp Ile Gln Met Thr Gln Ser Pro Ser Ser Leu Ser Ala Ser Val Gly
1               5                   10                  15

Asp Arg Val Thr Ile Thr Cys Arg Ala Ser Gln Asp Ile Gly Pro Trp

20                  25                  30

Leu Met Trp Tyr Gln Gln Lys Pro Gly Lys Ala Pro Lys Leu Leu Ile
        35                  40                  45

Tyr Gln Gly Ser Arg Leu Gln Ser Gly Val Pro Leu Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Thr Tyr Tyr Cys Gln Gln Pro Leu Ser Arg Pro Phe
                85                  90                  95

Thr Phe Gly Gln Gly Thr Lys Val Glu Ile Arg Arg
            100                 105

<210>  160
<211>  108
<212>  PRT
<213>  Artificial Sequence

<220>
<223>  Synthetic Antibody Domain

<400>  160

Asp Ile Gln Met Thr Gln Ser Pro Ser Ser Leu Ser Ala Ser Val Gly
1               5                   10                  15

Asp Arg Val Thr Ile Thr Cys Arg Ala Ser Gln His Ile Asp Ser Trp
            20                  25                  30

Leu Leu Trp Tyr Gln Gln Lys Pro Gly Lys Ala Pro Lys Leu Leu Ile
        35                  40                  45

Tyr Asn Gly Ser Val Leu Gln Ser Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Thr Tyr Tyr Cys Gln Gln Pro Leu Ser Gly Pro Phe
                85                  90                  95

Thr Phe Gly Gln Gly Thr Lys Val Glu Ile Lys Arg
            100                 105

<210>  161
<211>  108
<212>  PRT
<213>  Artificial Sequence

<220>
<223>  Synthetic Antibody Domain

<400>  161

Asp Ile Gln Met Thr Gln Ser Pro Ser Ser Leu Ser Ala Ser Val Gly
1               5                   10                  15

Asp Arg Val Thr Ile Thr Cys Arg Ala Ser Gln His Ile Asp Thr His
            20                  25                  30

Leu Phe Trp Tyr Gln Gln Lys Pro Gly Lys Ala Pro Lys Leu Leu Ile
        35                  40                  45

Tyr Asn Thr Ser Thr Leu Gln Ser Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Thr Tyr Tyr Cys Gln Gln Pro Leu Ser Arg Pro Phe
                85                  90                  95

Thr Phe Gly Gln Gly Thr Lys Val Glu Ile Lys Arg
            100                 105

<210>  162
<211>  108
<212>  PRT
<213>  Artificial Sequence

<220>
<223>  Synthetic Antibody Domain

<400>  162

Asp Ile Gln Met Thr Gln Ser Pro Ser Ser Leu Ser Ala Ser Val Gly
1               5                   10                  15

Asp Arg Val Thr Ile Thr Cys Arg Ala Ser Gln Phe Ile Asp Thr His
            20                  25                  30

Leu Met Trp Tyr Gln Gln Lys Pro Gly Lys Ala Pro Arg Leu Leu Ile
        35                  40                  45

Tyr Asn Thr Ser Thr Leu Gln Ser Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Thr Tyr Tyr Cys Gln Gln Pro Leu Ser Arg Pro Phe
                85                  90                  95

Thr Phe Gly Gln Gly Thr Lys Val Glu Ile Lys Arg
            100                 105

<210>  163
<211>  108
<212>  PRT
<213>  Artifical Sequence

<400>  163

Asp Ile Gln Met Thr Gln Ser Pro Ser Ser Leu Ser Ala Ser Val Gly
1               5                   10                  15

Asp Arg Val Thr Ile Thr Cys Arg Ala Ser Gln Asp Ile Asp Asp Trp
            20                  25                  30

Leu Leu Trp Tyr Gln Gln Lys Pro Gly Lys Ala Pro Lys Leu Leu Ile
        35                  40                  45

Tyr Gln Gly Ser Arg Leu Gln Ser Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Thr Tyr Tyr Cys Gln Gln Pro Leu Ser Arg Pro Phe
                85                  90                  95

Thr Phe Gly Gln Gly Thr Lys Val Glu Ile Lys Arg
            100                 105

<210>  164
<211>  108
<212>  PRT
<213>  Artificial Sequence

<220>
<223>  Synthetic Antibody Domain

<400>  164

Asp Ile Gln Met Thr Gln Ser Pro Ser Ser Leu Ser Ala Ser Val Gly
1               5                   10                  15

Asp Arg Val Thr Ile Thr Cys Arg Ala Ser Gln Trp Ile Asp Asp Thr
            20                  25                  30

Leu Met Trp Tyr Gln Gln Lys Pro Gly Lys Ala Pro Lys Leu Leu Ile
        35                  40                  45

Tyr Arg Ser Ser Met Leu Gln Ser Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Thr Tyr Tyr Cys Gln Gln Pro Leu Ser Arg Pro Phe
                85                  90                  95

Thr Phe Gly Gln Gly Thr Lys Val Glu Ile Lys Arg
            100                 105

<210>  165
<211>  108

<212>  PRT
<213>  Artificial Sequence

<220>
<223>  Synthetic Antiobdy Domain

<400>  165

Asp Ile Gln Met Thr Gln Ser Pro Ser Ser Leu Ser Ala Ser Val Gly
1               5                   10                  15

Asp Arg Val Thr Ile Thr Cys Arg Ala Ser Gln Tyr Ile Asp Ser His
            20                  25                  30

Leu Met Trp Tyr Gln Gln Lys Pro Gly Lys Ala Pro Lys Leu Leu Ile
        35                  40                  45

Tyr Asp Thr Ser Arg Leu Gln Ser Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Thr Tyr Tyr Cys Gln Gln Pro Leu Ser Arg Pro Phe
                85                  90                  95

Thr Phe Gly Gln Gly Thr Lys Val Glu Ile Lys Arg
            100                 105

<210>  166
<211>  108
<212>  PRT
<213>  Artificial Sequence

<220>
<223>  Synthetic Antibody Domain

<400>  166

Asp Ile Gln Met Thr Gln Ser Pro Ser Ser Leu Ser Ala Ser Val Gly
1               5                   10                  15

Asp Arg Val Thr Ile Thr Cys Arg Ala Ser Gln His Ile Asp Gln His
            20                  25                  30

Leu Phe Trp Tyr Gln Gln Lys Pro Gly Lys Ala Pro Lys Leu Leu Ile
        35                  40                  45

Tyr Asn Ser Ser Ser Leu Gln Ser Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Thr Tyr Tyr Cys Gln Gln Pro Leu Ser Arg Pro Phe
                85                  90                  95

Thr Phe Gly Gln Gly Thr Lys Val Glu Ile Lys Arg
            100                 105

<210> 167
<211> 108
<212> PRT
<213> Artificial Sequence

<220>
<223> Synthetic Antibody Domain

<400> 167

Asp Ile Gln Met Thr Gln Ser Pro Ser Ser Leu Ser Ala Ser Val Gly
1               5                   10                  15

Asp Arg Val Thr Ile Thr Cys Arg Ala Ser Gln His Ile Glu Arg Trp
            20                  25                  30

Leu Leu Trp Tyr Gln Gln Lys Pro Gly Lys Ala Pro Lys Leu Leu Ile
        35                  40                  45

Tyr Asn Ser Ser Lys Leu Gln Ser Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Thr Tyr Tyr Cys Gln Gln Pro Leu Ser Arg Pro Phe
                85                  90                  95

Thr Phe Gly Gln Gly Thr Lys Val Glu Ile Lys Arg
            100                 105

<210>  168
<211>  108
<212>  PRT
<213>  Artificial Sequence

<220>
<223>  Synthetic Antibody Domain

<400>  168

Asp Ile Gln Met Thr Gln Ser Pro Ser Ser Leu Ser Ala Ser Val Gly
1               5                   10                  15

Asp Arg Val Thr Ile Ser Cys Arg Ala Ser Gln His Ile Glu Arg Trp
            20                  25                  30

Leu Leu Trp Tyr Gln Gln Lys Pro Gly Lys Ala Pro Lys Leu Leu Ile
        35                  40                  45

Tyr Asn Ser Ser Lys Leu Gln Ser Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Thr Tyr Tyr Cys Gln Gln Pro Leu Ser Arg Pro Phe
                85                  90                  95

Thr Phe Gly Gln Gly Thr Lys Val Glu Ile Lys Arg
            100                 105

<210>  169
<211>  108
<212>  PRT
<213>  Artificial Sequence

<220>
<223>  Synthetic Antibody Domain

<400>  169

Asp Ile Gln Met Thr Gln Ser Pro Ser Ser Leu Ser Ala Ser Val Gly
1               5                   10                  15

Asp Arg Val Thr Ile Thr Cys Arg Ala Ser Gln Asp Ile Gly Ser Trp
            20                  25                  30

Leu Met Trp Tyr Gln Gln Lys Ser Gly Lys Ala Pro Lys Leu Leu Ile
        35                  40                  45

Tyr Asn Gly Ser Ala Leu Gln Ser Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Thr Tyr Tyr Cys Gln Gln Pro Leu Ser Arg Pro Phe
                85                  90                  95

Thr Phe Gly Gln Gly Thr Lys Val Glu Ile Lys Arg
            100                 105

<210>  170
<211>  108
<212>  PRT
<213>  Artificial Sequence

<220>
<223>  Synthetic Antibody Domain

<400>  170

Asp Ile Gln Met Thr Gln Ser Pro Ser Ser Leu Ser Ala Ser Val Gly
1               5                   10                  15

Asp Arg Val Thr Ile Thr Cys Arg Ala Ser Gln His Ile Asp Lys Trp
            20                  25                  30

Leu Met Trp Tyr Gln Gln Lys Pro Gly Lys Ala Pro Lys Leu Leu Ile
        35                  40                  45

Tyr Gln Ala Ser Lys Leu Gln Ser Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Thr Tyr Tyr Cys Gln Gln Pro Leu Ser Arg Pro Phe
                85                  90                  95

Thr Phe Gly Gln Gly Thr Lys Val Glu Ile Lys Arg
            100                 105

<210>  171
<211>  108
<212>  PRT
<213>  Artificial Sequence

<220>
<223>  Synthetic Antibody Domain

<400>  171

Asp Ile Gln Met Thr Gln Ser Pro Ser Ser Leu Ser Ala Ser Val Gly
1               5                   10                  15

Asp Arg Val Thr Ile Thr Cys Arg Ala Ser Gln Asp Ile Glu Glu Trp
            20                  25                  30

Leu Met Trp Tyr Gln Gln Lys Pro Gly Lys Ala Pro Lys Leu Leu Ile
        35                  40                  45

Tyr Asn Ser Ser Thr Leu Gln Ser Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Thr Tyr Tyr Cys Gln Gln Pro Leu Ser Arg Pro Phe
                85                  90                  95

Thr Phe Gly Gln Gly Thr Lys Val Glu Ile Lys Arg
            100                 105

<210>  172

<211>  100
<212>  PRT
<213>  Artificial Sequence

<220>
<223>  Synthetic Antibody Domain

<400>  172

Asp Ile Gln Met Thr Gln Ser Pro Ser Ser Leu Ser Ala Ser Val Gly
1               5                   10                  15

Asp Arg Val Thr Ile Thr Cys Arg Ala Ser Gln Tyr Ile Asp Tyr Gly
            20                  25                  30

Leu Met Trp Tyr Gln Gln Lys Pro Gly Lys Ala Pro Lys Leu Leu Ile
        35                  40                  45

Tyr Arg Thr Ser Glu Leu Gln Ser Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Thr Tyr Tyr Cys Gln Gln Pro Leu Ser Arg Pro Phe
                85                  90                  95

Thr Phe Gly Gln
            100

<210>  173
<211>  108
<212>  PRT
<213>  Artificial Sequence

<220>
<223>  Synthetic Antibody Domain

<400>  173

Asp Ile Gln Met Thr Gln Ser Pro Ser Ser Leu Ser Ala Ser Val Gly
1               5                   10                  15

Asp Arg Val Thr Ile Thr Cys Arg Ala Ser Gln Asn Ile Asp Ile His
            20                  25                  30

Leu Met Trp Tyr Gln Gln Lys Pro Gly Lys Ala Pro Lys Leu Leu Ile
        35                  40                  45

Tyr Gln Ser Ser Asn Leu Gln Ser Gly Val Pro Ser Pro Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Thr Tyr Tyr Cys Gln Gln Pro Leu Ser Arg Pro Phe
                85                  90                  95

Thr Phe Gly Gln Gly Thr Lys Val Glu Ile Lys Arg
            100                 105

<210>  174
<211>  108
<212>  PRT
<213>  Artificial Sequence

<220>
<223>  Synthetic Antibody Domain

<400>  174

Asp Ile Gln Met Thr Gln Ser Pro Ser Ser Leu Ser Ala Ser Val Gly
1               5                   10                  15

Asp Arg Val Thr Ile Thr Cys Arg Ala Ser Gln Asp Ile Gly Pro Trp
            20                  25                  30

Leu Leu Trp Tyr Gln Gln Lys Pro Gly Lys Ala Pro Lys Leu Leu Ile
        35                  40                  45

Tyr Gln Ser Ser Glu Leu Gln Ser Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Leu Ala Thr Tyr Tyr Cys Gln Gln Pro Leu Ser Arg Pro Phe

85                  90                  95

Thr Phe Gly Gln Gly Thr Lys Val Glu Ile Lys Arg
            100                 105

<210>  175
<211>  108
<212>  PRT
<213>  Artificial Sequence

<220>
<223>  Synthetic Antibody Domain

<400>  175

Asp Ile Gln Met Thr Gln Ser Pro Ser Ser Leu Ser Ala Ser Val Gly
1               5                   10                  15

Asp Arg Val Thr Ile Thr Cys Arg Ala Ser Gln Glu Ile Gly Val Trp
            20                  25                  30

Leu Met Trp Tyr Gln Gln Lys Pro Gly Lys Ala Pro Lys Leu Leu Ile
        35                  40                  45

Tyr Glu Gly Ser Arg Leu Gln Ser Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Thr Tyr Tyr Cys Gln Gln Pro Leu Ser Arg Pro Phe
                85                  90                  95

Val Phe Gly Gln Gly Thr Lys Val Glu Ile Lys Arg
            100                 105

<210>  176
<211>  108
<212>  PRT
<213>  Artificial Sequence

<220>
<223>  Synthetic Antibody Domain

<400>  176

Asp Ile Gln Met Thr Gln Ser Pro Ser Ser Leu Ser Ala Ser Val Gly
1               5                   10                  15

Asp Arg Val Thr Ile Thr Cys Arg Ala Ser Gln Ser Ile Gly Lys Trp
            20                  25                  30

Leu Met Trp Tyr Gln Gln Lys Pro Gly Lys Ala Pro Lys Leu Leu Ile
        35                  40                  45

Tyr Gln Ser Ser Leu Leu Gln Ser Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Thr Tyr Tyr Cys Gln Gln Pro Leu Ser Arg Pro Phe
                85                  90                  95

Thr Phe Gly Gln Gly Thr Lys Val Glu Ile Lys Arg
            100                 105

<210>  177
<211>  108
<212>  PRT
<213>  Artificial Sequence

<220>
<223>  Synthetic Antibody Domain

<400>  177

Asp Ile Gln Met Thr Gln Ser Pro Ser Ser Leu Ser Ala Ser Val Gly
1               5                   10                  15

Asp Arg Val Thr Ile Thr Cys Arg Ala Ser Gln Asp Ile Asp Thr Trp
            20                  25                  30

Leu Phe Trp Tyr Gln Gln Lys Pro Gly Lys Ala Pro Lys Leu Leu Ile
        35                  40                  45

Tyr Asn Gly Ser Arg Leu Gln Ser Gly Val Pro Ser Arg Phe Ser Gly

50                  55                  60

Ser Gly Ser Gly Thr Asp Phe Thr Leu Thr Ile Ser Gly Leu Gln Pro
65                  70                  75                  80

Glu Asp Phe Ala Thr Tyr Tyr Cys Gln Gln Pro Leu Ser Arg Pro Phe
                85                  90                  95

Thr Phe Gly Gln Gly Thr Lys Val Glu Ile Lys Arg
            100                 105

<210>  178
<211>  108
<212>  PRT
<213>  Artificial Sequence

<220>
<223>  Synthetic Antibody Domain

<400>  178

Asp Ile Gln Met Thr Gln Ser Pro Ser Ser Leu Ser Ala Ser Val Gly
1               5                   10                  15

Asp Arg Val Thr Ile Thr Cys Arg Ala Ser Gln Pro Ile Asp Ser Trp
            20                  25                  30

Leu Met Trp Tyr Gln Gln Lys Pro Gly Lys Ala Pro Lys Leu Leu Ile
        35                  40                  45

Tyr Gln Ala Ser Arg Leu Gln Ser Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Thr Tyr Tyr Cys Gln Gln Pro Leu Ser Arg Pro Phe
                85                  90                  95

Thr Phe Gly Gln Gly Thr Lys Val Glu Ile Lys Arg
            100                 105

<210>  179
<211>  108
<212>  PRT
<213>  Artificial Sequence

<220>
<223>  Synthetic Antibody Domain

<400>  179

Asp Ile Gln Met Thr Gln Ser Pro Ser Ser Leu Ser Ala Ser Val Gly
1               5                   10                  15

Asp Arg Val Thr Ile Thr Cys Arg Ala Ser Gln Asp Ile Glu Gly Trp
            20                  25                  30

Leu Leu Trp Tyr Gln Gln Lys Pro Gly Lys Ala Pro Lys Leu Leu Ile
        35                  40                  45

Tyr Asn Ser Ser Thr Leu Gln Ser Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Thr Tyr Tyr Cys Gln Gln Pro Leu Ser Arg Pro Phe
                85                  90                  95

Thr Phe Gly Gln Gly Thr Lys Val Glu Ile Lys Arg
            100                 105

<210>  180
<211>  108
<212>  PRT
<213>  Artificial Sequence

<220>
<223>  Synthetic Antibody Domain

<400>  180

Asp Ile Gln Met Thr Gln Ser Pro Ser Ser Leu Ser Ala Ser Val Gly
1               5                   10                  15

Asp Arg Val Thr Ile Thr Cys Arg Ala Ser Gln His Ile Asp Asp Trp

20                  25                  30

Leu Phe Trp Tyr Gln Gln Lys Pro Gly Lys Ala Pro Lys Leu Leu Ile
        35                  40                  45

Tyr Arg Ala Ser Phe Leu Gln Ser Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Thr Tyr Tyr Cys Gln Gln Pro Leu Ser Arg Pro Phe
                85                  90                  95

Thr Phe Gly Gln Gly Thr Lys Val Glu Ile Lys Arg
            100                 105

<210>  181
<211>  108
<212>  PRT
<213>  Artificial Sequence

<220>
<223>  Synthetic Antibody Domain

<400>  181

Asp Ile Gln Met Thr Gln Ser Pro Ser Ser Leu Ser Ala Ser Val Gly
1               5                   10                  15

Asp Arg Val Thr Ile Thr Cys Arg Ala Ser Gln Asp Ile Asp Thr Trp
            20                  25                  30

Leu Phe Trp Tyr Gln Gln Lys Pro Gly Lys Ala Pro Lys Leu Leu Ile
        35                  40                  45

Tyr Asn Gly Ser Arg Leu Gln Ser Gly Val Pro Ser Arg Phe Ser Gly
    50                  55                  60

Ser Gly Ser Gly Thr Asp Phe Thr Leu Thr Ile Ser Gly Leu Gln Pro
65                  70                  75                  80

Glu Asp Phe Ala Thr Tyr Tyr Cys Gln Gln Pro Leu Ser Arg Pro Phe
                85                  90                  95

Thr Phe Gly Gln Gly Thr Lys Val Glu Ile Lys Arg
            100                 105

<210>  182
<211>  108
<212>  PRT
<213>  Artificial Sequence

<220>
<223>  Synthetic Antibody Domain

<400>  182

Asp Ile Gln Met Thr Gln Ser Pro Ser Ser Leu Ser Ala Ser Val Gly
1               5                   10                  15

Asp Arg Val Thr Ile Thr Cys Arg Ala Ser Gln Pro Ile Glu Glu Trp
            20                  25                  30

Leu Leu Trp Tyr Gln Gln Lys Pro Gly Lys Ala Pro Lys Leu Leu Ile
        35                  40                  45

Tyr Asn Gly Ser His Leu Gln Ser Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Thr Tyr Tyr Cys Gln Gln Pro Leu Ser Arg Pro Phe
                85                  90                  95

Thr Phe Gly Gln Gly Thr Lys Val Glu Ile Lys Arg
            100                 105

<210>  183
<211>  108
<212>  PRT
<213>  Artificial Sequence

<220>
<223>  Synthetic Antibody Domain

<400>  183

Asp Ile Gln Met Thr Gln Ser Pro Ser Ser Leu Ser Ala Ser Val Gly
1               5                   10                  15

Asp Arg Val Thr Ile Thr Cys Arg Ala Ser Gln His Ile Asp Lys Trp
            20                  25                  30

Leu Met Trp Tyr Gln Gln Lys Pro Gly Lys Ala Pro Lys Leu Leu Ile
        35                  40                  45

Tyr Gln Ala Ser Lys Leu Gln Ser Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Thr Tyr Tyr Cys Gln Gln Pro Leu Ser Arg Pro Phe
                85                  90                  95

Thr Phe Gly Gln Gly Thr Lys Val Glu Ile Lys Arg
            100                 105


<210>  184
<211>  108
<212>  PRT
<213>  Artificial Sequence

<220>
<223>  Synthetic Antibody Domain

<400>  184

Asp Ile Gln Met Thr Gln Ser Pro Ser Ser Leu Ser Ala Ser Val Gly
1               5                   10                  15

Asp Arg Val Thr Ile Thr Cys Arg Ala Ser Gln Asp Ile Glu Glu Trp
            20                  25                  30

Leu Met Trp Tyr Gln Gln Lys Pro Gly Lys Ala Pro Lys Leu Leu Ile
        35                  40                  45

Tyr Asn Ser Ser Thr Leu Gln Ser Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Tyr Ala Thr Tyr Tyr Cys Gln Gln Pro Leu Ser Arg Pro Phe
                85                  90                  95

Thr Phe Gly Gln Gly Thr Lys Val Glu Ile Lys Arg
            100                 105

<210>  185
<211>  108
<212>  PRT
<213>  Artificial Sequence

<220>
<223>  Synthetic Antibody Domain

<400>  185

Asp Ile Gln Met Thr Gln Ser Pro Ser Ser Leu Ser Ala Ser Val Gly
1               5                   10                  15

Asp Arg Val Thr Ile Thr Cys Arg Ala Ser Gln Pro Ile Asp Tyr Gly
            20                  25                  30

Leu Met Trp Tyr Gln Gln Lys Pro Gly Lys Ala Pro Lys Leu Leu Ile
        35                  40                  45

Tyr Arg Ser Ser Gln Leu Gln Ser Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Thr Tyr Tyr Cys Gln Gln Pro Leu Ser Arg Pro Phe
                85                  90                  95

Thr Phe Gly Gln Gly Thr Lys Val Glu Ile Lys Arg
            100                 105

<210>  186
<211>  108
<212>  PRT
<213>  Artificial Sequence

<220>
<223>  Synthetic Antibody Domain

<400>  186

Asp Ile Gln Met Thr Gln Ser Pro Ser Ser Leu Ser Ala Ser Val Gly
1               5                   10                  15

Asp Arg Val Thr Ile Thr Cys Arg Ala Ser Gln Glu Ile Gly Ser Trp
            20                  25                  30

Leu Met Trp Tyr Gln Gln Lys Pro Gly Lys Ala Pro Lys Leu Leu Ile
        35                  40                  45

Tyr Gln Ser Ser Lys Leu Gln Ser Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Thr Tyr Tyr Cys Gln Gln Pro Leu Ser Arg Pro Phe
                85                  90                  95

Thr Phe Gly Gln Gly Thr Lys Val Glu Ile Lys Arg
            100                 105

<210>  187
<211>  108
<212>  PRT
<213>  Artificial Sequence

<220>
<223>  Synthetic Antibody Domain

<400>  187

Asp Ile Gln Met Thr Gln Ser Pro Ser Ser Leu Ser Ala Ser Val Gly
1               5                   10                  15

Asp Arg Val Thr Ile Thr Cys Arg Ala Ser Gln Pro Ile Asp Ser Trp
            20                  25                  30

Leu Leu Trp Tyr Gln Gln Lys Pro Gly Lys Ala Pro Lys Leu Leu Ile
        35                  40                  45

Tyr Asn Ala Ser Ser Leu Gln Ser Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Thr Tyr Tyr Cys Gln Gln Pro Leu Ser Arg Pro Phe
                85                  90                  95

Thr Phe Gly Gln Gly Thr Lys Val Glu Ile Lys Arg
            100                 105

<210>  188
<211>  108
<212>  PRT
<213>  Artificial Sequence

<220>
<223>  Synthetic Antibody Domain

<400>  188

Asp Ile Gln Met Thr Gln Ser Pro Ser Ser Leu Ser Ala Ser Val Gly
1               5                   10                  15

Asp Arg Val Thr Ile Thr Cys Arg Ala Ser Gln Asp Ile Gly Pro Trp
            20                  25                  30

Leu Met Trp Tyr Gln Gln Lys Pro Gly Lys Ala Pro Lys Leu Leu Ile
        35                  40                  45

Tyr Gln Ala Ser Ala Leu Gln Ser Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Thr Tyr Tyr Cys Gln Gln Pro Leu Ser Arg Pro Phe
                85                  90                  95

Thr Phe Gly Gln Gly Thr Lys Val Glu Ile Lys Arg
            100                 105

<210>  189
<211>  108
<212>  PRT
<213>  Artificial Sequence

<220>
<223>  Synthetic Antibody Domain

<400>  189

Asp Ile Gln Met Thr Gln Ser Pro Ser Ser Leu Ser Ala Ser Val Gly
1               5                   10                  15

Asp Arg Val Thr Ile Thr Cys Arg Ala Ser Gln Asn Ile His Glu Trp
            20                  25                  30

Leu Met Trp Tyr Gln Gln Lys Pro Gly Lys Ala Pro Lys Leu Leu Ile
        35                  40                  45

Tyr Gln Gly Ser Arg Leu Gln Ser Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Thr Tyr Tyr Cys Gln Gln Pro Leu Ser Arg Pro Phe
                85                  90                  95

Thr Phe Gly Gln Gly Thr Lys Val Glu Ile Lys Arg
            100                 105

<210>  190
<211>  108
<212>  PRT
<213>  Artificial Sequence

<220>

<223>  Synthetic Antibody Domain

<400>  190

Asp Ile Gln Met Thr Gln Ser Pro Ser Ser Leu Ser Ala Ser Val Gly
1               5                   10                  15

Asp Arg Val Thr Ile Thr Cys Arg Ala Ser Gln Asp Ile Gly Pro Trp
            20                  25                  30

Leu Met Trp Tyr Gln Gln Lys Pro Gly Lys Ala Pro Lys Leu Leu Ile
        35                  40                  45

Tyr Gln Ala Ser Ala Leu Gln Ser Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Ser Ala Thr Tyr Tyr Cys Gln Gln Pro Leu Ser Arg Pro Phe
                85                  90                  95

Thr Phe Gly Gln Gly Thr Lys Val Glu Ile Lys Arg
            100                 105

<210>  191
<211>  108
<212>  PRT
<213>  Artificial Sequence

<220>
<223>  Synthetic Antibody Domain

<400>  191

Asp Ile Gln Met Thr Gln Ser Pro Ser Ser Leu Ser Ala Ser Val Gly
1               5                   10                  15

Asp Arg Val Thr Ile Thr Cys Arg Ala Ser Gln Ser Val Lys Glu Phe
            20                  25                  30

Leu Trp Trp Tyr Gln Gln Lys Pro Gly Lys Ala Pro Lys Leu Leu Ile
        35                  40                  45

Tyr Met Ala Ser Asn Leu Gln Ser Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Thr Tyr Tyr Cys Gln Gln Lys Phe Lys Leu Pro Arg
                85                  90                  95

Thr Phe Gly Gln Gly Thr Lys Val Glu Ile Lys Arg
            100                 105

<210>  192
<211>  108
<212>  PRT
<213>  Artificial Sequence

<220>
<223>  Synthetic Antibody Domain

<400>  192

Asp Ile Gln Met Thr Gln Ser Pro Ser Ser Leu Ser Ala Ser Val Gly
1               5                   10                  15

Asp Arg Val Thr Ile Thr Cys Arg Ala Ser Gln Trp Ile Gly Pro Glu
            20                  25                  30

Leu Ser Trp Tyr Gln Gln Lys Pro Gly Lys Ala Pro Lys Leu Leu Ile
        35                  40                  45

Tyr His Gly Ser Ile Leu Gln Ser Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Thr Tyr Tyr Cys Gln Gln Arg Met Tyr Arg Pro Ala
                85                  90                  95

Thr Phe Gly Gln Gly Thr Lys Val Glu Ile Lys Arg
            100                 105

<210>  193
<211>  108
<212>  PRT
<213>  Artificial Sequence

<220>
<223>  Synthetic Antibody Domain

<400>  193

Asp Ile Gln Met Thr Gln Ser Pro Ser Ser Leu Ser Ala Ser Val Gly
1               5                   10                  15

Asp Arg Val Thr Ile Thr Cys Arg Ala Ser Gln Trp Ile Gly Arg Glu
            20                  25                  30

Leu Lys Trp Tyr Gln Gln Lys Pro Gly Lys Ala Pro Arg Leu Leu Ile
        35                  40                  45

Tyr His Gly Ser Val Leu Gln Ser Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Thr Tyr Tyr Cys Gln Gln Asp Phe Phe Val Pro Asp
                85                  90                  95

Thr Phe Gly Gln Gly Thr Lys Val Glu Ile Lys Arg
            100                 105

<210>  194
<211>  108
<212>  PRT
<213>  Artificial Sequence

<220>
<223>  Synthetic Antibody Domain

<400>  194

Asp Ile Gln Met Thr Gln Ser Pro Ser Ser Leu Ser Ala Ser Val Gly
1               5                   10                  15

Asp Arg Val Thr Ile Thr Cys Arg Ala Ser Gln Asp Ile Ala Asn Asp
            20                  25                  30

Leu Met Trp Tyr Gln Gln Lys Pro Gly Lys Ala Pro Lys Leu Leu Ile
        35                  40                  45

Tyr Arg Asn Ser Arg Leu Gln Gly Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Thr Tyr Tyr Cys Gln Gln Leu Val His Arg Pro Tyr
                85                  90                  95

Thr Ile Gly Gln Gly Thr Lys Val Glu Ile Lys Arg
            100                 105

<210>  195
<211>  108
<212>  PRT
<213>  Artificial Sequence

<220>
<223>  Synthetic Antibody Domain

<400>  195

Asp Ile Gln Met Thr Gln Ser Pro Ser Ser Leu Ser Ala Ser Val Gly
1               5                   10                  15

Asp Arg Val Thr Ile Thr Cys Arg Ala Ser Gln Phe Ile Gly Pro His
            20                  25                  30

Leu Thr Trp Tyr Gln Gln Lys Pro Gly Lys Ala Pro Lys Leu Leu Ile
        35                  40                  45

Tyr His Ser Ser Leu Leu Gln Ser Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Thr Tyr Tyr Cys Gln Gln Tyr Met Tyr Tyr Pro Ser
                85                  90                  95

Thr Phe Gly Gln Gly Thr Lys Val Lys Ile Lys Arg
            100                 105

<210>  196
<211>  108
<212>  PRT
<213>  Artificial Sequence

<220>
<223>  Synthetic Antibody Domain

<400>  196

Asp Ile Gln Met Thr Gln Ser Pro Ser Ser Leu Ser Ala Ser Val Gly
1               5                   10                  15

Asp Arg Val Thr Ile Thr Cys Arg Ala Ser Gln Trp Ile Gly Pro Glu
            20                  25                  30

Leu Ser Trp Tyr Gln Gln Lys Pro Gly Lys Ala Pro Lys Leu Leu Ile
        35                  40                  45

Tyr His Thr Ser Ile Leu Gln Ser Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Thr Tyr Tyr Cys Gln Gln Tyr Met Phe Gln Pro Arg
                85                  90                  95

Thr Phe Gly Gln Gly Thr Lys Val Glu Ile Arg Arg
            100                 105

<210>  197
<211>  108
<212>  PRT
<213>  Artificial Sequence

<220>
<223>  Synthetic Antibody Domain

<400>  197

Asp Ile Gln Met Ile Gln Ser Pro Ser Ser Leu Ser Ala Ser Val Gly
1               5                   10                  15

Asp Arg Val Thr Ile Thr Cys Arg Ala Ser Gln Phe Ile Gly Asn Glu
            20                  25                  30

Leu Ser Trp Tyr Gln Gln Lys Pro Gly Lys Ala Pro Lys Leu Leu Ile
        35                  40                  45

Tyr His Ala Ser Ser Leu Gln Ser Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Thr Tyr Tyr Cys Gln Gln Val Leu Gly Tyr Pro Tyr
                85                  90                  95

Thr Phe Gly Gln Gly Thr Lys Val Glu Ile Lys Arg
            100                 105

<210>  198
<211>  108
<212>  PRT
<213>  Artificial Sequence

<220>
<223>  Synthetic Antibody Domain

<400>  198

Asp Ile Gln Met Thr Gln Ser Pro Ser Ser Leu Ser Ala Ser Val Gly
1               5                   10                  15

Asp Arg Val Thr Ile Thr Cys Arg Ala Ser Gln Trp Ile Gly Pro Glu
            20                  25                  30

Leu Ser Trp Tyr Gln Gln Lys Pro Gly Lys Ala Pro Lys Leu Leu Ile
        35                  40                  45

Tyr His Gly Ser Ile Leu Gln Ser Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Thr Tyr Tyr Cys Gln Gln Val Leu Tyr Ser Pro Leu
                85                  90                  95

Thr Phe Gly Gln Gly Thr Lys Val Glu Ile Lys Arg
            100                 105

<210>  199
<211>  108
<212>  PRT
<213>  Artificial Sequence

<220>
<223>  Synthetic Antibody Domain

<400>  199

Asp Ile Gln Met Thr Gln Ser Pro Ser Ser Leu Ser Ala Ser Val Gly
1               5                   10                  15

Asp Arg Val Thr Ile Thr Cys Arg Ala Ser Gln Trp Ile Gly Asn Glu
            20                  25                  30

Leu Lys Trp Tyr Gln Gln Lys Pro Gly Lys Ala Pro Lys Leu Leu Ile
        35                  40                  45

Tyr Met Ser Ser Leu Leu Gln Ser Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Leu Ala Thr Tyr Tyr Cys Gln Gln Thr Leu Leu Leu Pro Phe
                85                  90                  95

Thr Phe Gly Gln Gly Thr Lys Val Glu Ile Lys Arg

            100                 105

<210> 200
<211> 108
<212> PRT
<213> Artificial Sequence

<220>
<223> Synthetic Antibody Domain

<400> 200

Asp Ile Gln Met Thr Gln Ser Pro Ser Ser Leu Ser Ala Ser Val Gly
1               5                   10                  15

Asp Arg Val Thr Ile Thr Cys Arg Ala Ser Gln Trp Ile Gly Pro Glu
            20                  25                  30

Leu Ser Trp Tyr Gln Gln Lys Pro Gly Lys Ala Pro Lys Leu Leu Ile
        35                  40                  45

Tyr His Gly Ser Ile Leu Gln Ser Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Thr Tyr Tyr Cys Gln Gln Arg Leu Tyr Tyr Pro Gly
                85                  90                  95

Thr Phe Gly Gln Gly Thr Lys Val Glu Ile Lys Arg
            100                 105

<210> 201
<211> 108
<212> PRT
<213> Artificial Sequence

<220>
<223> Synthetic Antibody Domain

<400> 201

Asp Ile Gln Met Thr Gln Ser Pro Ser Ser Leu Ser Ala Ser Val Gly
1               5                   10                  15

Asp Arg Val Thr Ile Thr Cys Arg Ala Ser Gln Ser Ile Gly Arg Glu
            20                  25                  30

Leu Ser Trp Tyr Gln Gln Lys Pro Gly Lys Ala Pro Met Leu Leu Ile
        35                  40                  45

Tyr His Ser Ser Asn Leu Gln Ser Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Thr Tyr Tyr Cys Gln Gln Gly Met Tyr Trp Pro Tyr
                85                  90                  95

Thr Phe Gly Gln Gly Thr Lys Val Glu Ile Lys Arg
            100                 105

<210> 202
<211> 108
<212> PRT
<213> Artificial Sequence

<220>
<223> Synthetic Antibody Domain

<400> 202

Asp Ile Gln Met Thr Gln Ser Pro Ser Ser Leu Ser Ala Ser Val Gly
1               5                   10                  15

Asp Arg Val Thr Ile Thr Cys Arg Ala Ser Gln Trp Ile Lys Pro Ala
            20                  25                  30

Leu His Trp Tyr Gln Gln Lys Pro Gly Lys Ala Pro Lys Leu Leu Ile
        35                  40                  45

Tyr His Gly Ser Ile Leu Gln Ser Gly Val Pro Ser Arg Phe Ser Gly
    50                  55                  60

Ser Gly Ser Gly Thr Asp Phe Thr Leu Thr Ile Ser Ser Leu Gln Pro

65                  70                  75                  80

Glu Asp Phe Ala Thr Tyr Tyr Cys Gln Gln Thr Leu Phe Met Pro Tyr
                85                  90                  95

Thr Phe Gly Gln Gly Thr Lys Val Glu Ile Lys Arg
            100                 105

<210>  203
<211>  108
<212>  PRT
<213>  Artificial Sequence

<220>
<223>  Synthetic Antibody Domain

<400>  203

Asp Ile Gln Met Thr Gln Ser Pro Ser Ser Leu Ser Ala Ser Val Gly
1               5                   10                  15

Asp Arg Val Thr Ile Thr Cys Arg Ala Ser Gln Ser Ile Ser Thr Ala
            20                  25                  30

Leu Leu Trp Tyr Gln Gln Lys Pro Gly Lys Ala Pro Lys Leu Leu Ile
        35                  40                  45

Tyr Asn Gly Ser Met Leu Pro Asn Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Thr Tyr Tyr Cys Gln Gln Thr Trp Asp Thr Pro Met
                85                  90                  95

Thr Phe Gly Gln Gly Thr Lys Val Glu Ile Lys Arg
            100                 105

<210>  204
<211>  108
<212>  PRT
<213>  Artificial Sequence

<220>
<223>  Synthetic Antibody Domain

<400>  204

Asp Ile Gln Met Thr Gln Ser Pro Ser Ser Leu Ser Ala Ser Val Gly
1               5                   10                  15

Asp Arg Val Thr Ile Thr Cys Arg Ala Ser Gln Trp Ile Gly His Asp
            20                  25                  30

Leu Ser Trp Tyr Gln Gln Lys Pro Gly Lys Ala Pro Lys Leu Leu Ile
        35                  40                  45

Tyr His Ser Ser Ser Leu Gln Ser Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Val Ala Thr Tyr Tyr Cys Gln Gln Leu Met Gly Tyr Pro Phe
                85                  90                  95

Thr Phe Gly Gln Gly Thr Lys Val Glu Ile Lys Arg
            100                 105

<210>  205
<211>  108
<212>  PRT
<213>  Artificial Sequence

<220>
<223>  Synthetic Antibody Domain

<400>  205

Asp Ile Gln Met Thr Gln Ser Pro Ser Ser Leu Ser Ala Ser Val Gly
1               5                   10                  15

Asp Arg Val Thr Ile Thr Cys Arg Ala Ser Gln Asp Ile Gly Gly Leu
            20                  25                  30

Leu Val Trp Tyr Gln Gln Lys Pro Gly Lys Ala Pro Lys Leu Leu Ile

35                  40                  45

Tyr Arg Ser Ser Tyr Leu Gln Ser Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Thr Tyr Tyr Cys Gln Gln Thr Trp Gly Ile Pro His
                85                  90                  95

Thr Phe Gly Gln Gly Thr Lys Val Glu Ile Lys Arg
            100                 105

<210>  206
<211>  108
<212>  PRT
<213>  Artificial Sequence

<220>
<223>  Synthetic Antibody Domain

<400>  206

Asp Ile Gln Met Thr Gln Ser Pro Ser Ser Leu Ser Ala Ser Val Gly
1               5                   10                  15

Asp Arg Val Thr Ile Thr Cys Arg Ala Ser Gln Lys Ile Phe Asn Gly
            20                  25                  30

Leu Ser Trp Tyr Gln Gln Lys Pro Gly Lys Ala Pro Lys Leu Leu Ile
        35                  40                  45

Tyr His Ser Ser Thr Leu Gln Ser Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Thr Tyr Tyr Cys Gln Gln Val Leu Leu Tyr Pro Tyr
                85                  90                  95

Thr Phe Gly Gln Gly Thr Lys Val Glu Ile Lys Arg
            100                 105

<210>  207
<211>  108
<212>  PRT
<213>  Artificial Sequence

<220>
<223>  Synthetic Antibody Domain

<400>  207

Asp Ile Gln Met Thr Gln Ser Pro Ser Ser Leu Ser Ala Ser Val Gly
1               5                   10                  15

Asp Arg Val Thr Ile Thr Cys Arg Ala Ser Gln Ser Ile Gly Thr Asn
            20                  25                  30

Leu Ser Trp Tyr Gln Gln Lys Pro Gly Lys Ala Pro Arg Leu Leu Ile
        35                  40                  45

Tyr Arg Thr Ser Met Leu Gln Ser Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Thr Tyr Tyr Cys Gln Gln Gln Phe Phe Trp Pro His
                85                  90                  95

Thr Phe Gly Gln Gly Thr Lys Val Glu Ile Lys Arg
            100                 105

<210>  208
<211>  118
<212>  PRT
<213>  Artificial Sequence

<220>
<223>  Synthetic Antibody Domain

<400>  208

Glu Val Gln Leu Leu Glu Ser Gly Gly Gly Leu Val Gln Pro Gly Gly

1               5                   10                  15

Ser Leu Arg Leu Ser Cys Ala Ala Ser Gly Phe Thr Phe Arg Leu Tyr
            20                  25                  30

Asp Met Val Trp Val Arg Gln Ala Pro Gly Lys Gly Leu Glu Trp Val
        35                  40                  45

Ser Tyr Ile Ser Ser Gly Gly Ser Gly Thr Tyr Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Lys Ala Gly Gly Arg Ala Ser Phe Asp Tyr Trp Gly Gln Gly Thr
            100                 105                 110

Leu Val Thr Val Ser Ser
        115

<210>  209
<211>  116
<212>  PRT
<213>  Artificial Sequence

<220>
<223>  Synthetic Antibody Domain

<400>  209

Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe His Leu Tyr
            20                  25                  30

Asp Met Met Trp Val Arg Gln Ala Pro Gly Lys Gly Leu Glu Trp Val
        35                  40                  45

Ser Phe Ile Gly Gly Asp Gly Leu Asn Thr Tyr Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Lys Ala Gly Thr Gln Phe Asp Tyr Trp Gly Gln Gly Thr Leu Val
            100                 105                 110

Thr Val Ser Ser
        115

<210> 210
<211> 119
<212> PRT
<213> Artificial Sequence

<220>
<223> Synthetic Antibody Domain

<400> 210

Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Asn Lys Tyr
            20                  25                  30

Pro Met Met Trp Val Arg Gln Ala Pro Gly Lys Gly Leu Glu Trp Val
        35                  40                  45

Ser Glu Ile Ser Pro Ser Gly Gln Asp Thr Tyr Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Lys Asn Pro Gln Ile Leu Ser Asn Phe Asp Tyr Trp Gly Gln Gly
            100                 105                 110

Thr Leu Val Thr Val Ser Ser
        115

<210> 211
<211> 124
<212> PRT
<213> Artificial Sequence

<220>
<223> Synthetic Antibody Domain

<400> 211

Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Gln Trp Tyr
            20                  25                  30

Pro Met Trp Trp Val Arg Gln Ala Pro Gly Lys Gly Leu Glu Trp Val
        35                  40                  45

Ser Leu Ile Glu Gly Gln Gly Asp Arg Thr Tyr Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Lys Ala Gly Asp Arg Thr Ala Gly Ser Arg Gly Asn Ser Phe Asp
            100                 105                 110

Tyr Trp Gly Gln Gly Thr Leu Val Thr Val Ser Ser
        115                 120

<210> 212
<211> 121

<212>  PRT
<213>  Artificial Sequence

<220>
<223>  Synthetic Antibody Domain

<400>  212

Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Lys Ala Tyr
            20                  25                  30

Glu Met Gly Trp Val Arg Gln Ala Pro Gly Lys Gly Leu Glu Trp Val
        35                  40                  45

Ser Gly Ile Ser Pro Asn Gly Gly Trp Thr Tyr Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Lys Glu Ser Ile Ser Pro Thr Pro Leu Gly Phe Asp Tyr Trp Gly
            100                 105                 110

Gln Gly Thr Leu Val Thr Val Ser Ser
        115                 120


<210>  213
<211>  116
<212>  PRT
<213>  Artificial Sequence

<220>
<223>  Synthetic Antibody Domain

<400>  213

Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Thr Gly Tyr
            20                  25                  30

Glu Met Gly Trp Val Arg Gln Ala Pro Gly Lys Gly Leu Glu Trp Val
        35                  40                  45

Ser Tyr Ile Ser Arg Gly Gly Arg Trp Thr Tyr Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Lys Ser Asp Thr Met Phe Asp Tyr Trp Gly Gln Gly Thr Leu Val
            100                 105                 110

Thr Val Ser Ser
        115

<210>  214
<211>  116
<212>  PRT
<213>  Artificial Sequence

<220>
<223>  Synthetic Antibody Domain

<400>  214

Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Ser Ala Tyr
            20                  25                  30

Glu Met Gly Trp Val Arg Gln Ala Pro Gly Lys Gly Leu Glu Trp Val
        35                  40                  45

Ser Phe Ile Ser Gly Gly Gly Arg Trp Thr Tyr Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Lys Tyr Ser Glu Asp Phe Asp Tyr Trp Gly Gln Gly Thr Leu Val
            100                 105                 110

Thr Val Ser Ser
        115

<210>  215
<211>  116
<212>  PRT
<213>  Artificial Sequence

<220>
<223>  Synthetic Antibody Domain

<400>  215

Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Gly Ala Tyr
            20                  25                  30

Pro Met Met Trp Val Arg Gln Ala Pro Gly Lys Gly Leu Glu Trp Val
        35                  40                  45

Ser Glu Ile Ser Pro Ser Gly Ser Tyr Thr Tyr Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Lys Asp Pro Arg Lys Phe Asp Tyr Trp Gly Gln Gly Thr Leu Val

            100                 105                 110

Thr Val Ser Ser
        115

<210> 216
<211> 123
<212> PRT
<213> Artificial Sequence

<220>
<223> Synthetic Antibody Domain

<400> 216

Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Gln Phe Tyr
            20                  25                  30

Lys Met Gly Trp Val Arg Gln Ala Pro Gly Lys Gly Leu Glu Trp Val
        35                  40                  45

Ser Ser Ile Ser Ser Val Gly Asp Ala Thr Tyr Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Lys Met Gly Gly Gly Pro Pro Thr Tyr Val Val Tyr Phe Asp Tyr
            100                 105                 110

Trp Gly Gln Gly Thr Leu Val Thr Val Ser Ser
        115                 120

<210> 217
<211> 123
<212> PRT
<213> Artificial Sequence

<220>
<223>  Synthetic Antibody Domain

<400>  217

Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Gly Glu Tyr
            20                  25                  30

Gly Met Tyr Trp Val Arg Gln Ala Pro Gly Lys Gly Leu Glu Trp Val
        35                  40                  45

Ser Ser Ile Ser Glu Arg Gly Arg Leu Thr Tyr Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Asn Leu Arg Ala Glu Asp Thr Ala Val Tyr Tyr Cys
                85                  90                  95

Ala Lys Ser Ala Leu Ser Ser Glu Gly Phe Ser Arg Ser Phe Asp Tyr
            100                 105                 110

Trp Gly Gln Gly Thr Leu Val Thr Val Ser Ser
        115                 120

<210>  218
<211>  123
<212>  PRT
<213>  Artificial Sequence

<220>
<223>  Synthetic Antibody Domain

<400>  218

Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Ser Asp Tyr

20                  25                  30

Ala Met Tyr Trp Val Arg Gln Ala Pro Gly Lys Gly Leu Glu Trp Val
        35                  40                  45

Ser Ser Ile Thr Ala Arg Gly Phe Ile Thr Tyr Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Lys Ser Gly Phe Pro His Lys Ser Gly Ser Asn Tyr Phe Asp Tyr
            100                 105                 110

Trp Gly Gln Gly Thr Leu Val Thr Val Ser Ser
        115                 120

<210>  219
<211>  240
<212>  PRT
<213>  Artificial Sequence

<220>
<223>  Synthetic Antibody Sequence, VH and VL joined by Gly4Ser Linker

<400>  219

Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Ser Ser Tyr
            20                  25                  30

Ala Met Ser Trp Val Arg Gln Ala Pro Gly Lys Gly Leu Glu Trp Val
        35                  40                  45

Ser Ala Ile Ser Gly Ser Gly Gly Ser Thr Tyr Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Lys Ser Tyr Gly Ala Phe Asp Tyr Trp Gly Gln Gly Thr Leu Val
            100                 105                 110

Thr Val Ser Ser Gly Gly Gly Gly Ser Gly Gly Gly Gly Ser Gly Gly
        115                 120                 125

Gly Gly Ser Thr Asp Ile Gln Met Thr Gln Ser Pro Ser Ser Leu Ser
    130                 135                 140

Ala Ser Val Gly Asp Arg Val Thr Ile Thr Cys Arg Ala Ser Gln Ser
145                 150                 155                 160

Ile Ser Ser Tyr Leu Asn Trp Tyr Gln Gln Lys Pro Gly Lys Ala Pro
                165                 170                 175

Lys Leu Leu Ile Tyr Ala Ala Ser Ser Leu Gln Ser Gly Val Pro Ser
            180                 185                 190

Arg Phe Ser Gly Ser Gly Ser Gly Thr Asp Phe Thr Leu Thr Ile Ser
        195                 200                 205

Ser Leu Gln Pro Glu Asp Phe Ala Thr Tyr Tyr Cys Gln Gln Ser Tyr
    210                 215                 220

Ser Thr Pro Asn Thr Phe Gly Gln Gly Thr Lys Val Glu Ile Lys Arg
225                 230                 235                 240

<210>  220
<211>  720
<212>  DNA
<213>  Artificial Sequence

<220>
<223>  Nucleotide Sequence Encoding Synthetic Antibody Sequence, VH and
       VL joined by Gly4Ser Linker

<400>  220
gaggtgcagc tgttggagtc tgggggaggc ttggtacagc ctggggggtc cctgcgtctc      60

tcctgtgcag cctccggatt cacctttagc agctatgcca tgagctgggt ccgccaggct     120

ccagggaagg gtctagagtg ggtctcagct attagtggta gtggtggtag cacatactac     180

gcagactccg tgaagggccg gttcaccatc tcccgtgaca attccaagaa cacgctgtat     240

ctgcaaatga acagcctgcg tgccgaggac accgcggtat attactgtgc gaaaagttat     300

ggtgcttttg actactgggg ccagggaacc ctggtcaccg tctcgagcgg tggaggcggt     360

tcaggcggag gtggcagcgg cggtggcggg tcgacggaca tccagatgac ccagtctcca     420

tcctccctgt ctgcatctgt aggagaccgt gtcaccatca cttgccgggc aagtcagagc     480

attagcagct atttaaattg gtaccagcag aaaccaggga aagcccctaa gctcctgatc     540

tatgctgcat ccagttggca aagtggggtc ccatcacgtt tcagtggcag tggatctggg     600

acagatttca ctctcaccat cagcagtctg caacctgaag attttgctac gtactactgt     660

caacagagtt acagtacccc taatacgttc ggccaaggga ccaaggtgga aatcaaacgg     720

<210>  221
<211>  359
<212>  DNA
<213>  Artificial Sequence

<220>
<223>  Phage Vector Expression Cassette Nucleotide Sequences

<400>  221
caggaaacag ctatgaccat gattacgcca agcttgcatg caaattctat ttcaaggaga      60

cagtcataat gaaataccta ttgcctacgg cagccgctgg attgttatta ctcgcggccc     120

agccggccat ggccgaggtg tttgactact ggggccaggg aaccctggtc accgtctcga     180

gcggtggagg cggttcaggc ggaggtggca gcggcggtgg cgggtcgacg gacatccaga     240

tgacccaggc ggccgcagaa caaaaactcc atcatcatca ccatcacggg gccgcaatct     300

cagaagagga tctgaatggg gccgcataga ctgttgaaag ttgtttagca aaacctcat      359

<210>  222
<211>  96
<212>  PRT
<213>  Artificial Sequence

<220>
<223>  Expression Cassette Amino Acid Sequences

<400>  222

Met Lys Tyr Leu Leu Pro Thr Ala Ala Ala Gly Leu Leu Leu Leu Ala
1               5                   10                  15

Ala Gln Pro Ala Met Ala Glu Val Phe Asp Tyr Trp Gly Gln Gly Thr
            20                  25                  30

Leu Val Thr Val Ser Ser Gly Gly Gly Gly Ser Gly Gly Gly Gly Ser
        35                  40                  45

Gly Gly Gly Gly Ser Thr Asp Ile Gln Met Thr Gln Ala Ala Ala Glu
    50                  55                  60

Gln Lys Leu His His His His His His Gly Ala Ala Ile Ser Glu Glu
65                  70                  75                  80

Asp Leu Asn Gly Ala Ala Thr Val Glu Ser Cys Leu Ala Lys Pro His
                85                  90                  95

<210>  223
<211>  116
<212>  PRT
<213>  Artificial Sequence

<220>
<223>  VH Sequence of Clone K8

<400>  223

Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Ser Ser Tyr
            20                  25                  30

Ala Met Ser Trp Val Arg Gln Ala Pro Gly Lys Gly Leu Glu Trp Val
        35                  40                  45

Ser His Ile Ser Pro Tyr Gly Ala Asn Thr Arg Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Lys Gly Leu Arg Ala Phe Asp Tyr Trp Gly Gln Gly Thr Leu Val
            100                 105                 110

Thr Val Ser Ser
        115

<210>  224
<211>  116
<212>  PRT
<213>  Artificial Sequence

<220>
<223>  VH Sequence of Clone VH2

<400>  224

Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Ser Ser Tyr
            20                  25                  30

Ala Met Ser Trp Val Arg Gln Ala Pro Gly Lys Gly Leu Glu Trp Val
        35                  40                  45

Ser Asp Ile Gly Ala Thr Gly Ser Lys Thr Gly Tyr Ala Asp Pro Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Lys Lys Val Leu Thr Phe Asp Tyr Trp Gly Gln Gly Thr Leu Val

            100                 105                 110

Thr Val Ser Ser
        115

<210> 225
<211> 115
<212> PRT
<213> Artificial Sequence

<220>
<223> VH Sequence of Clone VH4

<400> 225

Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Ser Ser Tyr
            20                  25                  30

Ala Met Ser Trp Val Arg Gln Ala Pro Gly Lys Gly Leu Glu Trp Val
        35                  40                  45

Ser Arg Ile Asn Gly Pro Gly Ala Thr Gly Tyr Ala Asp Ser Val Lys
    50                  55                  60

Gly Arg Phe Thr Ile Ser Arg Asp Asn Ser Lys Asn Thr Leu Tyr Leu
65                  70                  75                  80

Gln Ile Asn Ser Leu Arg Ala Glu Asp Thr Ala Val Tyr Tyr Cys Ala
                85                  90                  95

Lys His Gly Ala Pro Phe Asp Tyr Trp Gly Gln Gly Thr Leu Val Thr
            100                 105                 110

Val Ser Ser
        115

<210> 226
<211> 116
<212> PRT
<213> Artificial Sequence

<220>
<223>  VH Sequence of Clone VHC11

<400>  226

Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Ser Ser Tyr
            20                  25                  30

Ala Met Asn Trp Val Arg Gln Ala Pro Gly Lys Gly Leu Glu Trp Val
        35                  40                  45

Ser Ser Ile Pro Ala Ser Gly Leu His Thr Arg Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Lys Pro Gly Leu Gly Phe Asp Tyr Trp Gly Gln Gly Thr Leu Val
            100                 105                 110

Thr Val Ser Ser
        115

<210>  227
<211>  115
<212>  PRT
<213>  Artificial Sequence

<220>
<223>  VH Sequence of Clone VHA10sd

<400>  227

Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Ser Ser Tyr

20                  25                  30

Ala Met Ser Trp Val Arg Gln Ala Pro Gly Lys Gly Leu Glu Trp Val
        35                  40                  45

Ser Asp Ile Glu Arg Thr Gly Tyr Thr Arg Tyr Ala Asp Ser Val Lys
    50                  55                  60

Gly Arg Phe Thr Ile Ser Arg Asp Asn Ser Lys Asn Thr Leu Tyr Leu
65                  70                  75                  80

Gln Met Asn Ser Leu Arg Ala Glu Asp Thr Ala Val Tyr Tyr Cys Ala
                85                  90                  95

Lys Lys Val Leu Val Phe Asp Tyr Trp Gly Gln Gly Thr Leu Val Thr
            100                 105                 110

Val Ser Ser
        115

<210>  228
<211>  116
<212>  PRT
<213>  Artificial Sequence

<220>
<223>  VH Sequence of clone VHA1sd

<400>  228

Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Ser Ser Tyr
            20                  25                  30

Ala Met Ser Trp Val Arg Gln Ala Pro Gly Lys Gly Leu Glu Trp Val
        35                  40                  45

Ser Glu Ile Ser Ala Asn Gly Ser Lys Thr Gln Tyr Ala Asp Ser Val
    50                  55                  60

Lys Gly Arg Leu Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Lys Lys Val Leu Gln Phe Asp Tyr Trp Gly Gln Gly Thr Leu Val
            100                 105                 110

Thr Val Ser Ser
        115

<210>  229
<211>  115
<212>  PRT
<213>  Artificial Sequence

<220>
<223>  VH Sequence of Clone VHA5sd

<400>  229

Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Ser Ser Tyr
            20                  25                  30

Ala Met Ser Trp Val Arg Gln Ala Pro Gly Lys Gly Leu Glu Trp Val
        35                  40                  45

Ser Thr Ile Pro Ala Asn Gly Val Thr Arg Tyr Ala Asp Ser Val Lys
    50                  55                  60

Gly Arg Phe Thr Ile Ser Arg Asp Asn Ser Lys Asn Thr Leu Tyr Leu
65                  70                  75                  80

Gln Met Asn Ser Leu Arg Ala Glu Asp Thr Ala Val Tyr Tyr Cys Ala
                85                  90                  95

Lys Ser Leu Leu Gln Phe Asp Tyr Trp Gly Gln Gly Thr Leu Val Thr
            100                 105                 110

Val Ser Ser
        115

<210>  230
<211>  116
<212>  PRT
<213>  Artificial Sequence

<220>
<223>  VH Sequence of Clone VHC5sd

<400>  230

Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Ser Ser Tyr
            20                  25                  30

Ala Met Ser Trp Val Arg Gln Ala Pro Gly Lys Gly Leu Glu Trp Val
        35                  40                  45

Ser Asp Ile Ala Ala Thr Gly Ser Ala Thr Ser Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Lys Lys Ile Leu Lys Phe Asp Tyr Trp Gly Gln Gly Thr Leu Val
            100                 105                 110

Thr Val Ser Ser
        115

<210>  231
<211>  116
<212>  PRT
<213>  Artificial Sequence

<220>

<223>  VH Sequence of Clone VHC11sd

<400>  231

Glu Val Gln Leu Leu Glu Ser Gly Gly Gly Leu Val Gln Pro Gly Gly
1               5                   10                  15

Ser Leu Arg Leu Ser Cys Ala Ala Ser Gly Ser Thr Phe Ser Ser Tyr
            20                  25                  30

Ala Met Ser Trp Val Arg Gln Ala Pro Gly Lys Gly Leu Glu Trp Val
        35                  40                  45

Ser Thr Ile Ser Ser Val Gly Gln Ser Thr Arg Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Lys Asn Leu Met Ser Phe Asp Tyr Trp Gly Gln Gly Thr Leu Val
            100                 105                 110

Thr Val Ser Ser
        115

<210>  232
<211>  108
<212>  PRT
<213>  Artificial Sequence

<220>
<223>  Vk Sequence of Clone K8

<400>  232

Asp Ile Gln Met Thr Gln Ser Pro Ser Ser Leu Ser Ala Ser Val Gly
1               5                   10                  15

Asp Arg Val Thr Ile Thr Cys Arg Ala Ser Gln Ser Ile Ser Ser Tyr
            20                  25                  30

Leu Asn Trp Tyr Gln Gln Lys Pro Gly Lys Ala Pro Lys Leu Leu Ile
        35                  40                  45

Tyr Arg Ala Ser His Leu Gln Ser Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Thr Tyr Tyr Cys Gln Gln Pro Trp Arg Ser Pro Gly
                85                  90                  95

Thr Phe Gly Gln Gly Thr Lys Val Glu Ile Lys Arg
            100                 105

<210>  233
<211>  108
<212>  PRT
<213>  Artificial Sequence

<220>
<223>  Vk Sequence of Clone E5sd

<400>  233

Asp Ile Gln Met Thr Gln Ser Pro Ser Ser Leu Ser Ala Ser Val Gly
1               5                   10                  15

Asp Arg Val Thr Ile Thr Cys Arg Ala Ser Gln Ser Val Ser Ser Tyr
            20                  25                  30

Leu Asn Trp Tyr Gln Gln Lys Pro Gly Lys Ala Pro Lys Leu Leu Ile
        35                  40                  45

Tyr Leu Ala Ser Arg Leu Gln Ser Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Thr Tyr Tyr Cys Gln Gln Asn Trp Trp Leu Pro Pro
                85                  90                  95

Thr Phe Gly Gln Gly Thr Lys Val Glu Ile Lys Arg
            100                 105

<210>  234
<211>  107
<212>  PRT
<213>  Artificial Sequence

<220>
<223>  Vk Sequence of Clone C3

<400>  234

Asp Ile Gln Met Thr Gln Ser Pro Ser Ser Leu Ser Ala Ser Val Gly
1               5                   10                  15

Asp Arg Val Thr Ile Thr Cys Arg Ala Ser Gln Ser Ile Ser Ser Tyr
            20                  25                  30

Leu Asn Trp Tyr Gln Gln Lys Pro Gly Lys Ala Pro Lys Leu Leu Ile
        35                  40                  45

Tyr Ala Ser Leu Leu Gln Ser Gly Val Pro Ser Arg Phe Ser Gly Ser
    50                  55                  60

Gly Ser Gly Thr Asp Phe Thr Leu Thr Ile Ser Ser Leu Gln Pro Glu
65                  70                  75                  80

Asp Phe Ala Thr Tyr Tyr Cys Gln Gln Arg Val Tyr Asp Pro Leu Thr
                85                  90                  95

Phe Gly Gln Gly Thr Lys Val Glu Ile Lys Arg
            100                 105

<210>  235
<211>  120
<212>  PRT
<213>  Artificial Sequence

<220>
<223>  Dummy VH for Library

<220>

<221>  MISC_FEATURE
<222>  (103)..(106)
<223>  Xaa at position 103-106 is an amino acid encoded by the NNK codon
       , where N is any of G, A, T or C.

<400>  235

Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Ser Ser Tyr
            20                  25                  30

Ala Met Ser Trp Val Arg Gln Ala Pro Gly Lys Gly Leu Glu Trp Val
        35                  40                  45

Ser Ala Ile Ser Gly Ser Gly Gly Ser Thr Tyr Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Lys Ser Tyr Gly Ala Xaa Xaa Xaa Xaa Phe Asp Tyr Trp Gly Gln
            100                 105                 110

Gly Thr Leu Val Thr Val Ser Ser
        115                 120

<210>  236
<211>  360
<212>  DNA
<213>  Artificial Sequence

<220>
<223>  Nucleotide Sequence for Dummy VH for Library

<220>
<221>  misc_feature
<222>  (307)..(317)
<223>  N is G,A,T, or C

<400> 236
gaggtgcagc tgttggagtc tgggggaggc ttggtacagc ctggggggtc cctgcgtctc 60

tcctgtgcag cctccggatt cacctttagc agctatgcca tgagctgggt ccgccaggct 120

ccagggaagg gtctagagtg ggtctcagct attagtggta gtggtggtag cacatactac 180

gcagactccg tgaagggccg gttcaccatc tcccgtgaca attccaagaa cacgctgtat 240

ctgcaaatga acagcctgcg tgccgaggac accgcggtat attactgtgc gaaaagttat 300

ggtgctnnkn nknnknnktt tgactactgg ggccagggaa ccctggtcac cgtctcgagc 360


<210> 237
<211> 108
<212> PRT
<213> Artificial Sequence

<220>
<223> Sequence of Anti-Murine Serum Albumin Domain Antibody

<400> 237

Asp Ile Gln Met Thr Gln Ser Pro Ser Ser Leu Ser Ala Ser Val Gly
1               5                   10                  15


Asp Arg Val Thr Ile Thr Cys Arg Ala Ser Gln Ser Ile Ile Lys His
            20                  25                  30


Leu Lys Trp Tyr Gln Gln Lys Pro Gly Lys Ala Pro Lys Leu Leu Ile
        35                  40                  45


Tyr Gly Ala Ser Arg Leu Gln Ser Gly Val Pro Ser Arg Phe Ser Gly
    50                  55                  60


Ser Gly Ser Gly Thr Asp Phe Thr Leu Thr Ile Ser Ser Leu Gln Pro
65                  70                  75                  80


Glu Asp Phe Ala Thr Tyr Tyr Cys Gln Gln Gly Ala Arg Trp Pro Gln
                85                  90                  95


Thr Phe Gly Gln Gly Thr Lys Val Glu Ile Lys Arg
            100                 105

<210> 238
<211> 324
<212> DNA
<213> Artificial Sequence

<220>
<223> Nucleotide Sequence Encoding Anti-Murine Serum Albumin Domain Ani
      tbody

<400> 238
gacatccaga tgacccagtc tccatcctcc ctgtctgcat ctgtaggaga ccgtgtcacc     60

atcacttgcc gggcaagtca gagcattatt aagcatttaa agtggtacca gcagaaacca    120

gggaaagccc ctaagctcct gatctatggt gcatcccggt tgcaaagtgg ggtcccatca    180

cgtttcagtg gcagtggatc tgggacagat ttcactctca ccatcagcag tctgcaacct    240

gaagattttg ctacgtacta ctgtcaacag ggggctcggt ggcctcagac gttcggccaa    300

gggaccaagg tggaaatcaa acgg                                           324


<210> 239
<211> 108
<212> PRT
<213> Artificial Sequence

<220>
<223> Sequence of Anti-Murine Serum Albumin Domain Antibody

<400> 239

Asp Ile Gln Met Thr Gln Ser Pro Ser Ser Leu Ser Ala Ser Val Gly
1               5                   10                  15


Asp Arg Val Thr Ile Thr Cys Arg Ala Ser Gln Ser Ile Tyr Tyr His
            20                  25                  30


Leu Lys Trp Tyr Gln Gln Lys Pro Gly Lys Ala Pro Lys Leu Leu Ile
        35                  40                  45


Tyr Lys Ala Ser Thr Leu Gln Ser Gly Val Pro Ser Arg Phe Ser Gly
    50                  55                  60


Ser Gly Ser Gly Thr Asp Phe Thr Leu Thr Ile Ser Ser Leu Gln Pro
65                  70                  75                  80

Glu Asp Phe Ala Thr Tyr Tyr Cys Gln Gln Val Arg Lys Val Pro Arg
                85                  90                  95

Thr Phe Gly Gln Gly Thr Lys Val Glu Ile Lys Arg
            100                 105

<210>  240
<211>  324
<212>  DNA
<213>  Artificial Sequence

<220>
<223>  Nucleotide Sequence Encoding Anti-Murine Serum Albumin Domain Ant
       ibody

<400>  240
gacatccaga tgacccagtc tccatcctcc ctgtctgcat ctgtaggaga ccgtgtcacc      60

atcacttgcc gggcaagtca gagcatttat tatcatttaa agtggtacca gcagaaacca     120

gggaaagccc ctaagctcct gatctataag gcatccacgt tgcaaagtgg ggtcccatca     180

cgtttcagtg gcagtggatc tgggacagat ttcactctca ccatcagcag tctgcaacct     240

gaagattttg ctacgtacta ctgtcaacag gttcggaagg tgcctcggac gttcggccaa     300

gggaccaagg tggaaatcaa acgg                                            324

<210>  241
<211>  324
<212>  DNA
<213>  Artificial Sequence

<220>
<223>  Nucleotide Sequence of Anti-Murine Serum Albumin Domain Antibody

<400>  241
gacatccaga tgacccagtc tccatcctcc ctgtctgcat ctgtaggaga ccgtgtcacc      60

atcacttgcc gggcaagtca gagcattttt atgaatttat tgtggtacca gcagaaacca     120

gggaaagccc ctaagctcct gatctataat gcatccgtgt tgcaaagtgg ggtcccatca     180

cgtttcagtg gcagtggatc tgggacagat ttcactctca ccatcagcag tctgcaacct     240

gaagattttg ctacgtacta ctgtcaacag gttgtgtggc gtccttttac gttcggccaa      300

gggaccaagg tggaaatcaa acgc                                            324

<210>  242
<211>  323
<212>  DNA
<213>  Artificial Sequence

<220>
<223>  Nucleotide Sequence of Anti-Murine Serum Albumin Domain Antibody

<400>  242
gacatccaga tgacccagtc tccatcctcc ctgtctgcat ctgtaggaga ccgtgtcacc      60

atcacttgcc gggcaagtca gagcatttgg acgaagttac attggtacca gcagaaacca     120

gggaaagccc ctaagctcct gatctatatg gcatccagtt tgcaaagtgg ggtcccatca     180

cgtttcagtg gcagtggatc tgggacagat ttcactctca ccatcagcag tctgcaacct     240

gaagattttg ctacgtacta ctgtcaacag tggtttagta atcctagtac gttcggccaa     300

gggaccaagg tggaaatcaa acg                                             323


<210>  243
<211>  324
<212>  DNA
<213>  Artificial Sequence

<220>
<223>  Nucleotide Sequence of Anti-Murine Serum Albumin Domain Antibody

<400>  243
gacatccaga tgacccagtc tccatcctcc ctgtctgcat ctgtaggaga ccgtgtcacc      60

atcacttgcc gggcaagtca gagcattgag cattatttat ggtggtacca gcagaaacca     120

gggaaagccc ctaagctcct gatctatgct gcatcctatt tgcaaagtgg ggtcccatca     180

cgtttcagtg gcagtggatc tgggacagat ttcactctca ccatcagcag tctgcaacct     240

gaagattttg ctacgtacta ctgtcaacag agtttggcgt gtcctcctac gttcggccaa     300

gggaccaagg tggaaatcaa acgg                                            324


<210>  244
<211>  324
<212>  DNA
<213>  Artificial Sequence

<220>
<223>  Nucleotide Sequence of Anti-Murine Serum Albumin Domain Antibody

<400>  244

gacatccaga tgacccagtc tccatcctcc ctgtctgcat ctgtaggaga ccgtgtcacc 60

atcacttgcc gggcaagtca gagcatttat ggtcatttat tgtggtacca gcagaaacca 120

gggaaagccc ctaagctcct gatctatgct gcatccagtt tgcaaagtgg ggtcccatca 180

cgtttcagtg gcagtggatc tgggacagat ttcactctca ccatcagcag tctgcaacct 240

gaagattttg ctacgtacta ctgtcaacag cctttggtgc ggccttttac gttcggccaa 300

gggaccaagg tggaaatcaa acgg 324

<210> 245
<211> 324
<212> DNA
<213> Artificial Sequence

<220>
<223> Nucleotide Sequence of Anti-Murine Serum Albumin Domain Antibody

<400> 245
gacatccaga tgacccagtc tccatcctcc ctgtctgcat ctgtaggaga ccgtgtcacc 60

atcacttgcc gggcaagtca gagcattgct aagttgttat attggtacca gcagaaacca 120

gggaaagccc ctaagctcct gatctatgat gcatcctctt tgcaaagtgg ggtcccatca 180

cgtttcagtg gcagtggatc tgggacagat ttcactctca ccatcagcag tctgcaacct 240

gaagattttg ctacgtacta ctgtcaacag tggtgggggt atcctggtac gttcggccaa 300

gggaccaagg tggaaatcaa acgg 324

<210> 246
<211> 324
<212> DNA
<213> Artificial Sequence

<220>
<223> Nucleotide Sequence of Anti-Murine Serum Albumin Domain Antibody

<400> 246
gacatccaga tgacccagtc tccatcctcc ctgtctgcat ctgtaggaga ccgtgtcacc 60

atcacttgcc gggcaagtca gagcattttt cctgctttac tttggtacca gcagaaacca 120

gggaaagccc ctaagctcct gatctatcat gcatccagtt tgcaaagtgg ggtcccatca 180

cgtttcagtg gcagtggatc tgggacagat ttcactctca ccatcagcag tctgcaacct 240

gaagatattg ctacgtacta ctgtcaacag gttgtgtggc gtccttttac gttcggccaa 300

gggaccaagg tggaaatcaa acgg 324

<210> 247
<211> 324
<212> DNA
<213> Artificial Sequence

<220>
<223> Nucleotide Sequence of Anti-Murine Serum Albumin Domain Antibody

<400> 247
gacatccaga tgacccagtc tccatcctcc ctgtctgcat ctgtaggaga ccgtgtcacc 60

atcacttgcc gggcaagtca gagcattgat aatgcgttac attggtacca gcagaaacca 120

gggaaagccc ctaagctcct gatctatcag gcatccattt tgcaaagtgg ggtcccatca 180

cgtttcagtg gcagtggatc tgggacagat ttcactctca ccatcagcag tctgcaacct 240

gaagattttg ctacgtacta ctgtcaacag gttgtgtggc gtccttttac gttcggccaa 300

gggaccaagg tggaaatcaa acgg 324

<210> 248
<211> 324
<212> DNA
<213> Artificial Sequence

<220>
<223> Nucleotide Sequence of Anti-Murine Serum Albumin Domain Antibody

<400> 248
gacatccaga tgacccagtc tccatcctcc ctgtctgcat ctgtaggaga ccgtgtcacc 60

atcacttgcc gggcaagtca gagcattttt atgaatttat tgtggtacca gcagaaacca 120

gggaaagccc ctaagctcct gatctataat gcatccgtgt tgcaaagtgg ggtcccatca 180

cgtttcagtg gcagtggatc tgggacaggt ttcactctca ccatcagcag tctgcaacct 240

gaagattttg ctacgtacta ctgtcaacag gttgtgtggc gtccttttac gttcggccaa 300

gggaccaagg tggaaatcaa acgg 324

<210> 249
<211> 324
<212> DNA
<213> Artificial Sequence

<220>
<223>  Nucleotide Sequence of Anti-Murine Serum Albumin Domain Antibody

<400>  249
gacatccaga tgacccagtc tccatcctcc ctgtctgcat ctgtaggaga ccgtgtcacc      60

atcacttgcc gggcaagtca gagcattttg aattctttac attggtacca gcagaaacca     120

gggaaagccc ctaagctcct gatctatcat gcatccactt tgcaaagtgg ggtcccatca     180

cgtttcagtg gcagtggatc tgggacagat ttcactctca ccatcagcag tctgcaacct     240

gaagattttg ctacgtacta ctgtcaacag gttgtgtggc gtccttttac gttcggccaa     300

gggaccaagg tggaaatcaa acgg                                            324


<210>  250
<211>  324
<212>  DNA
<213>  Artificial Sequence

<220>
<223>  Nucleotide Sequence of Anti-Murine Serum Albumin Domain Antibody

<400>  250
gacatccaga tgacccagtc tccatcctcc ctgtctgcat ctgtaggaga ccgtgtcacc      60

atcacttgcc gggcaagtca gagcattttg aattctttac attggtacca gcagaaacca     120

gggaaagccc ctaagctcct gatctatcat gcatccactt tgcaaagtgg ggtcccatca     180

cgtttcagtg gcagtggatc tgggacagat ttcactctca ccatcagcag tctgcaacct     240

gaagattttg ctacgtacta ctgtcaacag gttgtgtggc gtccttttac gttcggccaa     300

gggaccaagg tggaaatcaa acgg                                            324


<210>  251
<211>  324
<212>  DNA
<213>  Artificial Sequence

<220>
<223>  Nucleotide Sequence of Anti-Murine Serum Albumin Domain Antibody

<400>  251
gacatccaga tgacccagtc tccatcctcc ctgtctgcat ctgtaggaga ccgtgtcacc      60

atcacttgcc gggcaagtca gagcattgat aattatttac attggtacca gcagaaacca     120

gggaaagccc ctaagctcct gatctattct gcatcccatt tgcaaagtgg ggtcccatca     180

cgtttcagtg gcagtggatc tgggacagat ttcactctca ccatcagcag tctgcaacct 240

gaagattttg ctacgtacta ctgtcaacag gttgtgtggc gtccttttac gttcggccaa 300

gggaccaagg tggaaatcaa acgg 324

<210> 252
<211> 324
<212> DNA
<213> Artificial Sequence

<220>
<223> Nucleotide Sequence of Anti-Murine Serum Albumin Domain Antibody

<400> 252
gacatccaga tgacccagtc tccatcctcc ctgtctgcat ctgtaggaga ccgtgtcacc 60

atcacttgcc gggcaagtca gagcattaat gagtatttac attggtacca gcagaaacca 120

gggaaagccc ctaagctcct gatctattct gcatccgtgt tgcaaagtgg ggtcccatca 180

cgtttcagtg gcagtggatc tgggacagat ttcactctca ccatcagcag tctgcaacct 240

gaagattttg ctacgtacta ctgtcaacag gttgtgtggc gtccttttac gttcggccaa 300

gggaccaagg tggaaatcaa acgg 324

<210> 253
<211> 324
<212> DNA
<213> Artificial Sequence

<220>
<223> Nucleotide Sequence of Anti-Murine Serum Albumin Domain Antibody

<400> 253
gacatccaga tgacccagtc tccatcctcc ctgtctgcat ctgtaggaga ccgtgtcacc 60

atcacttgcc gggcaagtca gagcattaat tatgctttac attggtacca gcagaaacca 120

gggaaagccc ctaagctcct gatctatcag gcatccattt tgcaaagtgg ggtcccatca 180

cgtttcagtg gcagtggatc tgggacagat ttcactctca ccatcagcag tctgcaacct 240

gaagattttg ctacgtacta ctgtcaacag gttgtgtggc gtccttttac gttcggccaa 300

gggaccaagg tggaaatcaa acgg 324

<210> 254

<211>  324
<212>  DNA
<213>  Artificial Sequence

<220>
<223>  Nucleotide Sequence of Anti-Murine Serum Albumin Domain Antibody

<400>  254
gacatccaga tgacccagtc tccatcctcc ctgtctgcat ctgtaggaga ccgtgtcacc      60

atcacttgcc gggcaagtca gagcattgat agttttttac attggtacca gcagaaacca      120

gggaaagccc ctaagctcct gatctatagt gcatccgagt tgcaaagtgg ggtcccatca      180

cgtttcagtg gcagtggatc tgggacagat ttcactctca ccatcagcag tctgcatcct      240

gaagattttg ctacgtacta ctgtcaacag gttgtgtggc gtccttttac gttcggccaa      300

gggaccaagg tggaaatcaa acgg                                              324


<210>  255
<211>  324
<212>  DNA
<213>  Artificial Sequence

<220>
<223>  Nucleotide Sequence of Anti-Murine Serum Albumin Domain Antibody

<400>  255
gacatccaga tgacccagtc tccatcctcc ctgtctgcat ctgtaggaga ccgtgtcacc      60

atcacttgcc gggcaagtca gagcattgat cagtatttac attggtacca gcagaaacca      120

gggaaagccc ctaagctcct gatctatggt gcatccaatt tgcaaagtga ggtcccatca      180

cgtttcagtg gcagtggatc tgggacagat ttcactctca ccatcagcag tctgcaacct      240

gaagattttg ctacgtacta ctgtcaacag gttgtgtggc gtccttttac gttcggccaa      300

gggaccaagg tggaaatcaa acgg                                              324


<210>  256
<211>  324
<212>  DNA
<213>  Artificial Sequence

<220>
<223>  Nucleotide Sequence of Anti-Murine Serum Albumin Domain Antibody

<400>  256
gacatccaga tgacccagtc tccatcctcc ctgtctgcat ctgtaggaga ccgtgtcacc      60

atcacttgcc gggcaagtca gagcattgat agttttttac attggtacca gcagaaacca 120

gggaaagccc ctaagctcct gatctatagt gcatccgagt tgcaaagtgg ggtcccatca 180

cgtttcagtg gcagtggatc tgggacagat ttcactctca ccatcagcag tctgcatcct 240

gaagattttg ctacgtacta ctgtcaacag gttgtgtggc gtccttttac gttcggccaa 300

gggaccaagg tggaaatcaa acgg 324

<210> 257
<211> 324
<212> DNA
<213> Artificial Sequence

<220>
<223> Nucleotide Sequence of Anti-Murine Serum Albumin Domain Antibody

<400> 257
gacatccaga tgacccagtc tccatcctcc ctgtctgcat ctgtaggaga ccgtgtcacc 60

atcacttgcc gggcaagtca gagcattgat tcttatttac attggtacca gcagaaacca 120

gggaaagccc ctaagctcct gatctatagt gcatccctgt tgcaaagtgg ggtcccatca 180

cgtttcagtg gcagtggatc tgggacagat ttcactctca ccatcagcag tctgcaacct 240

gaagattttg ctacgtacta ctgtcaacag gttgtgtggc gtccttttac gttcggccaa 300

gggaccaagg tggaaatcaa acgg 324

<210> 258
<211> 324
<212> DNA
<213> Artificial Sequence

<220>
<223> Nucleotide Sequence of Anti-Murine Serum Albumin Domain Antibody

<400> 258
gacatccaga tgacccagtc tccatcctcc ctgtctgcat ctgtaggaga ccgtgtcacc 60

atcacttgcc gggcaagtca gagcattgat cagtatttac attggtacca gcagaaacca 120

gggaaagccc ctaagctcct gatctattct gcatcccttt tgcaaagtgg ggtcccatca 180

cgtttcagtg gcagtggatc tgggacagat ttcactctca ccatcagcag tctgcaacct 240

gaagattttg ctacatacta ctgtcaacag gttgtgtggc gtccttttac gttcggccaa 300

gggaccaagg tggaaatcaa acgg 324

<210> 259
<211> 324
<212> DNA
<213> Artificial Sequence

<220>
<223> Nucleotide Sequence of Anti-Murine Serum Albumin Domain Antibody

<400> 259
gacatccaga tgacccagtc tccatcctcc ctgtctgcat ctgtaggaga ccgtgtcacc 60

atcacttgcc gggcaagtca aagcattgat gagtttttac attggtacca gcagaaacca 120

gggaaagccc ctaagctcct gatctattgt gcatcccagt tgcaaagtgg ggtcccatca 180

cgtttcagtg gcagtggatc tgggacagat ttcactctca ccatcagcag tctacatcct 240

gaagattttg ctacgtacta ctgtcaacag gttgtgtggc gtccttttac gttcggccaa 300

gggaccaagg tggaaatcaa acgg 324

<210> 260
<211> 324
<212> DNA
<213> Artificial Sequence

<220>
<223> Nucleotide Sequence of Anti-Murine Serum Albumin Domain Antibody

<400> 260
gacatccaga tgacccagtc tccatcctcc ctgtctgcat ctgtaggaga ccgtgtcacc 60

atcacttgcc gggcaagtca gagcattgat gcgtatttac attggtacca gcagaaacca 120

gggaaagccc ctaagctcct gatctattct gcatccctgt tgcaaagtgg ggtcccatca 180

cgtttcagtg gcagtggatc tgggacagat ttcactctca ccatcagcag tctgcaacct 240

gaagattttg ctacgtacta ctgtcaacag gttgtgtggc gtccttttac gttcggccaa 300

gggaccaagg tggaaatcaa acgg 324

<210> 261
<211> 324
<212> DNA
<213> Artificial Sequence

<220>

<223>  Nucleotide Sequence of Anti-Murine Serum Albumin Domain Antibody

<400>  261
gacatccaga tgacccagtc tccatcctcc ctgtctgcat ctgtaggaga ccgtgtcacc      60

atcacttgcc gggcaagtca gagcattgat aggtatttac attggtacca gcagaaacca     120

gggaaagccc ctaagctcct gatctatagt gcatccgtgt tgcaaagtgg ggtcccatca     180

cgtttcagtg gcagtggatc tgggacagat ttcaccctca ccatcagcag tctgcagcct     240

gaagattttg ctacgtacta ctgtcaacag gttgtgtggc gtccttttac gttcggccaa     300

gggaccaagg tggaaatcaa acgg                                            324


<210>  262
<211>  324
<212>  DNA
<213>  Artificial Sequence

<220>
<223>  Nucleotide Sequence of Anti-Murine Serum Albumin Domain Antibody

<400>  262
gacatccaga tgacccagtc tccatcctcc ctgtctgcat ctgtaggaga ccgtgtcacc      60

atcacttgcc gggcaagtca gagcattgat aagtatttac attggtacca gcagaaacca     120

gggaaagccc ctaagctcct gatctatagt gcatcctcgt tgcaaagtgg ggtcccatca     180

cgtttcagtg gcagtggatc tgggacagat ttcactctca ccatcagcag tctgcaacct     240

gaagattttg ctacgtacta ctgtcaacag gttgtgtggc gtccttttac gttcggccaa     300

gggaccaagg tggaaatcaa acgg                                            324


<210>  263
<211>  324
<212>  DNA
<213>  Artificial Sequence

<220>
<223>  Nucleotide Sequence of Anti-Murine Serum Albumin Domain Antibody

<400>  263
gacatccaga tgacccagtc tccatcctcc ctgtctgcat ctgtaggaga ccgtgtcacc      60

atcacttgcc gggcaagtca gagcattgat cattatttac attggtacca gcagaaacca     120

gggaaagccc ctaagctcct gatctatagt gcatccgttt tgcaaagtgg ggtcccatca     180

cgtttcagtg gcagtggatc tgggacagat ttcactctca ccatcagcag tctgcaacct 240

gaagattttg caacgtacta ctgtcaacag gttgtgtggc gtccttttac gttcggccaa 300

gggaccaagg tggaaatcaa acgg 324

<210> 264
<211> 324
<212> DNA
<213> Artificial Sequence

<220>
<223> Nucleotide Sequence of Anti-Murine Serum Albumin Domain Antibody

<400> 264
gacatccaga tgacccagtc tccatcctcc ctgtctgcat ctgtaggaga ccgtgtcacc 60

atcacttgcc gggcaagtca gagcattgat gagtttttac attggtacca gcagaaacca 120

gggaaagccc ctaagctcct gatctatagt gcatccattt tgcaaagtgg ggtcccatca 180

cgtttcagtg gcagtggatc tgggacagat ttcactctca ccatcagcag tctgcaacct 240

gaagattttg ctacgtacta ctgtcaacag gttgtgtggc gtccttttac gttcggccaa 300

gggaccaagg tggaaatcaa acgg 324

<210> 265
<211> 324
<212> DNA
<213> Artificial Sequence

<220>
<223> Nucleotide Sequence of Anti-Murine Serum Albumin Domain Antibody

<400> 265
gacatccaga tgacccagtc tccatcctcc ctgtctgcat ctgtaggaga ccgtgtcacc 60

atcacttgcc gggcaagtca gagcattcag actgcgttac tgtggtacca gcagaaacca 120

gggaaagccc ctaagctcct gatctataat gcatccagtt tgcaaagtgg ggtcccatca 180

cgtttcagtg gcagtggatc tgggacagat ttcactctca ccatcagcag tctgcaacct 240

gaagattttg ctacatacta ctgtcaacag gttgtgtggc gtccttttac gttcggccaa 300

gggaccaagg tggaaatcaa acgg 324

<210> 266
<211> 324

<212>  DNA
<213>  Artificial Sequence

<220>
<223>  Nucleotide Sequence of Anti-Murine Serum Albumin Domain Antibody

<400>  266
gacatccaga tgacccagtc tccatcctcc ctgtctgcat ctgtaggaga ccgtgtcacc      60

atcacttgcc gggcaagtca gagcattgat cagtatttac attggtacca gcagaaacca     120

gggaaagccc ctaagctcct gatctatggt gcatccaatt tgcaaagtgg ggtcccatca     180

cgtttcagtg gcagtggatc tgggacagat ttcactctca ccatcagcag tctgcaacct     240

gaagattttg ctacgtacta ctgtcaacag gttgtgtggc gtccttttac gttcggccaa     300

gggaccaagg tggaaatcaa acgg                                            324


<210>  267
<211>  324
<212>  DNA
<213>  Artificial Sequence

<220>
<223>  Nucleotide Sequence of Anti-Murine Serum Albumin Domain Antibody

<400>  267
gacatccaga tgacccagtc tccatcctcc ctgtctgcat ctgtaggaga ccgtgtcacc      60

atcacttgcc gggcaagtca gagcattgat aattatttac attggtacca gcagaaacca     120

gggaaagccc ctaagctcct gatctatagt gcatcccagt tgcaaagtgg ggtcccatca     180

cgtttcagtg gcagtggatc tgggacagat ttcactctca ccatcagcag tctgcaacct     240

gaagattttg ctacgtacta ctgtcaacag gttgtgtggc gtccttttac gttcggccaa     300

gggaccaagg tggaaatcaa acgg                                            324


<210>  268
<211>  324
<212>  DNA
<213>  Artificial Sequence

<220>
<223>  Nucleotide Sequence of Anti-Murine Serum Albumin Domain Antibody

<400>  268
gacatccaga tgacccagtc tccatcctcc ctgtctgcat ctgtaggaga ccgtgtcacc      60

atcacttgcc gggcaagtca gagcattgat aattttttac attggtacca gcagaaacca 120

gggaaagccc ctaagctcct gatctatagt gcatccgagt tgcaaagtgg ggtcccatca 180

cgtttcagtg gcagtggatc tgggacagat ttcactctca ccatcagcag tctgcaacct 240

gaagattttg ctacgtacta ctgtcaacag gttgtgtggc gtccttttac gttcggccaa 300

gggaccaagg tggaaatcaa acgg 324

<210> 269
<211> 324
<212> DNA
<213> Artificial Sequence

<220>
<223> Nucleotide Sequence of Anti-Murine Serum Albumin Domain Antibody

<400> 269
gacatccaga tgacccagtc tccatcctcc ctgtctgcat ctgtaggaga ccgtgtcacc 60

atcacttgcc gggcaagtca gagcattgat gagtatttac attggtacca gcagaaacca 120

gggaaacccc ctaagctcct gatctattct gcatccagtt tgcaaagtgg ggtcccatca 180

cgtttcagtg gcagtggatc tgggacagat ttcactctca ccatcagcag tctgcaacct 240

gaagattttg ctacgtacta ctgtcaacag gttgtgtggc gtccttttac gttcggccaa 300

gggaccaagg tggaaatcaa acgg 324

<210> 270
<211> 324
<212> DNA
<213> Artificial Sequence

<220>
<223> Nucleotide Sequence of Anti-Murine Serum Albumin Domain Antibody

<400> 270
gacatccaga tgacccagtc tccatcctcc ctgtctgcat ctgtaggaga ccgtgtcacc 60

atcacttgcc gggcaagtca gagcattgat cattttttac attggtacca gcagaaacca 120

gggaaagccc ctaagctcct gatctatagt gcatccgagt tgcaaagtgg ggtcccatca 180

cgtttcagtg gcagtggatc tgggacagat ttcactctca ccatcagcag tctgcaacct 240

gaagattttg ctacgtacta ctgtcaacag gttgtgtggc gtccttttac gttcggccaa 300

gggaccaagg tggaaatcaa acgg 324

<210>  271
<211>  324
<212>  DNA
<213>  Artificial Sequence

<220>
<223>  Nucleotide Sequence of Anti-Murine Serum Albumin Domain Antibody

<400>  271
gacatccaga tgacccagtc tccatcctcc ctgtctgcat ctgtaggaga ccgtgtcacc      60

atcacttgcc gggcaagtca gagcattgat aattatttac attggtacca gcagaaacca     120

gggaaagccc ctaagctcct gatctattcg gcatccatgt tgcaaagtgg ggtcccatca     180

cgtttcagtg gcagtggatc tgggacagat ttcactctca ccatcagcag tctgcaacct     240

gaagattttg ctacgtacta ctgtcaacag gttgtgtggc gtccttttac gttcggccaa     300

gggaccaagg tggaaatcaa acgg                                            324


<210>  272
<211>  324
<212>  DNA
<213>  Artificial Sequence

<220>
<223>  Nucleotide Sequence of Anti-Murine Serum Albumin Domain Antibody

<400>  272
gacatccaga tgacccagtc tccatcctcc ctgtctgcat ctgtaggaga ccgtgtcacc      60

atcacttgcc gggcaagtca gagcattgat gagtatttac attggtacca gcagaaacca     120

gggaaagccc ccaagctcct gatctattct gcatccattt tgcaaagtgg ggtcccatca     180

cgtttcagtg gcagtggatc tgggacagat ttcactctca ccatcagcag tctgcaacct     240

gaagattttg ctacgtacta ctgtcaacag gttgtgtggc gtccttttac gttcggccaa     300

gggaccaagg tggaaatcaa acgg                                            324


<210>  273
<211>  324
<212>  DNA
<213>  Artificial Sequence

<220>
<223>  Nucleotide Sequence of Anti-Murine Serum Albumin Domain Antibody

<400>  273
gacatccaga tgacccagtc tccatcctcc ctgtctgcat ctgtaggaga ccgtgtcacc      60

atcacttgcc gggcaagtca gagcattgat gagtttttac attggtacca gcagaaacca     120

gggaaagccc ctaagctcct gatctattcg gcatccgctt tgcaaagtgg ggtcccatca     180

cgtttcagtg gcagtggatc tgggacagat ttcactctca ccatcagcag tctgcaacct     240

gaagattttg ctacgtacta ctgtcaacag gttgtgtggc gtccttttac gttcggccaa     300

gggaccaagg tggaaatcaa acgg                                            324


<210>  274
<211>  324
<212>  DNA
<213>  Artificial Sequence

<220>
<223>  Nucleotide Sequence of Anti-Murine Serum Albumin Domain Antibody

<400>  274
gacatccaga tgacccagtc tccatcctcc ctgtctgcat ctgtaggaga ccgtgtcacc      60

atcacttgcc gggcaagtca gagcattgat gagtatttac attggtacca gcagaaacca     120

gggaaagccc ctaagctcct gatctattct gcatccattt tgcaaagtgg ggtcccatca     180

cgtttcagtg gcagtggatc tgggacagat ttcactctca ccatcagcag tctgcaccct     240

gaagattttg ctacgtacta ctgtcaacag gttgtgtggc gtccttttac gttcggccaa     300

gggaccaagg tggaaatcaa acgg                                            324


<210>  275
<211>  324
<212>  DNA
<213>  Artificial Sequence

<220>
<223>  Nucleotide Sequence of Anti-Murine Serum Albumin Domain Antibody

<400>  275
gacatccaga tgacccagtc tccatcctcc ctgtctgcat ctgtaggaga ccgtgtcacc      60

atcacttgcc gggcaagtca gagcattgat aattatttac attggtacca gcagaaacca     120

gggaaagccc ctaagctcct gatctatgct gcatccagtt tgcaaagtgg ggtcccatca     180

cgtttcagtg gcagtggatc tgggacagat ttcactctca ccatcagcag tctgcaacct     240

gatgattttg ctacgtacta ctgtcaacag gttgtgtggc gtccttttgc gttcggccaa 300

gggaccaagg tggaaatcaa acgg 324

<210> 276
<211> 324
<212> DNA
<213> Artificial Sequence

<220>
<223> Nucleotide Sequence of Anti-Murine Serum Albumin Domain Antibody

<400> 276
gacatccaga tgacccagtc tccatcctcc ctgtctgcat ctgtaggaga ccgtgtcacc 60

atcacttgcc gggcaagtca gagcattgat agttatttac attggtacca gcagaaacca 120

gggaaagccc ctaagctcct gatctatagt gcatcaaatt tagaaacagg ggtcccatca 180

cgtttcagtg gcagtggatc tgggacagat ttcactctca ccatcagcag tctgcaacct 240

gaagattttg ctacgtacta ctgtcaacag gttgtgtggc gtccttttac gttcggccaa 300

gggaccaagg tggaaatcaa acgg 324

<210> 277
<211> 324
<212> DNA
<213> Artificial Sequence

<220>
<223> Nucleotide Sequence of Anti-Murine Serum Albumin Domain Antibody

<400> 277
gacatccaga tgacccagtc tccatcctcc ctgtctgcat ctgtaggaga ccgtgtcacc 60

atcacttgcc gggcaagtca ggtgatttgg gatgcgttag attggtacca gcagaaacca 120

gggaaagccc ctaagctcct gatctatagt gcgtcccgtt tgcaaagtgg ggtcccatca 180

cgtttcagtg gcagtggatc tgggacagat ttcactctca ccatcagcag tctgcatcct 240

gaagattttg ctacgtacta ctgtcaacag tatgctgtgt ttcctgtgac gttcggccaa 300

gggaccaagg tggaaatcaa acgg 324

<210> 278
<211> 324
<212> DNA

<213>  Artificial Sequence

<220>
<223>  Nucleotide Sequence of Anti-Murine Serum Albumin Domain Antibody

<400>  278
gacatccaga tgacccagtc tccatcctcc ctgtctgcat ctgtaggaga ccgtgtcacc      60

atcacttgcc gggcaagtca gactatttat gatgcgttaa gttggtacca gcagaaacca     120

gggaaagccc ctaagctcct gatctatggt ggttccaggt tgcaaagtgg ggtcccatca     180

cgtttcagtg gcggtggatc tgggacagat ttcactctca ccatcagcag tctgcaacct     240

gaagattttg ctacgtacta ctgtcaacag tataagacta agcctttgac gttcggccaa     300

gggaccaagg tggaaatcaa acgg                                            324


<210>  279
<211>  324
<212>  DNA
<213>  Artificial Sequence

<220>
<223>  Nucleotide Sequence of Anti-Murine Serum Albumin Domain Antibody

<400>  279
gacatccaga tgacccagtc cccatcctcc ctgtctgcat ctgtaggaga ccgtgtcacc      60

atcacttgcc gggcaagtca gactatttat gatgcgttaa gttggtacca gcagaaacca     120

gggaaagccc ctaagctcct gatctatggt ggttccaggt tgcaaagtgg ggtcccatca     180

cgtttcagtg gtagtggatc tgggacagat ttcactctca ccatcagcag tctgcaaccc     240

gaagattttg ctacgtacta ctgtcaacag tatgctcgtt atcctcttac gttcggccaa     300

gggaccaagg tggaaatcaa acgg                                            324


<210>  280
<211>  120
<212>  PRT
<213>  Artificial Sequence

<220>
<223>  Amino Acid Sequence of Domain Antibody

<400>  280

Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Arg Ile Ser Asp Glu
            20                  25                  30

Asp Met Gly Trp Val Arg Gln Ala Pro Gly Lys Gly Leu Glu Trp Val
        35                  40                  45

Ser Ser Ile Tyr Gly Pro Ser Gly Ser Thr Tyr Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Ser Ala Leu Glu Pro Leu Ser Glu Pro Leu Gly Phe Trp Gly Gln
            100                 105                 110

Gly Thr Leu Val Thr Val Ser Ser
        115                 120

<210>  281
<211>  360
<212>  DNA
<213>  Artificial Sequence

<220>
<223>  Nucleotide sequence of domain antibody

<400>  281
gaggtgcagc tgttggagtc tgggggaggc ttggtacagc ctggggggtc cctgcgtctc      60

tcctgtgcag cctccggatt taggattagc gatgaggata tgggctgggt ccgccaggct     120

ccagggaagg gtctagagtg ggtatcaagc atttatggcc ctagcggtag cacatactac     180

gcagactccg tgaagggccg gttcaccatc tcccgtgaca attccaagaa cacgctgtat     240

ctgcaaatga acagcctgcg tgccgaggac accgcggtat attattgcgc gagtgctttg     300

gagccgcttt cggagcccct gggcttttgg ggtcagggaa ccctggtcac cgtctcgagc     360

<210>  282

<211>  116
<212>  PRT
<213>  Artificial Sequence

<220>
<223>  Amino Acid Sequence of Domain Antibody

<400>  282

Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Asp Leu Tyr
            20                  25                  30

Asn Met Phe Trp Val Arg Gln Ala Pro Gly Lys Gly Leu Glu Trp Val
        35                  40                  45

Ser Phe Ile Ser Gln Thr Gly Arg Leu Thr Trp Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Lys Thr Leu Glu Asp Phe Asp Tyr Trp Gly Gln Gly Thr Leu Val
            100                 105                 110

Thr Val Ser Ser
        115

<210>  283
<211>  348
<212>  DNA
<213>  Artificial Sequence

<220>
<223>  Nucleotide Sequence of Domain Antibody

<400>  283
gaggtgcagc tgttggagtc tgggggaggc ttggtacagc ctggggggtc cctgcgtctc      60

tcctgtgcag cctccggatt cacctttgat ctttataata tgttttgggt ccgccaggct     120

ccagggaagg gtctagagtg ggtctcattt attagtcaga ctggtaggct tacatggtac 180

gcagactccg tgaagggccg gttcaccatc tcccgcgaca attccaagaa cacgctgtat 240

ctgcaaatga acagcctgcg tgccgaggac accgcggtat attactgtgc gaaaacgctg 300

gaggattttg actactgggg ccagggaacc ctggtcaccg tctcgagc 348

<210> 284
<211> 108
<212> PRT
<213> Artificial Sequence

<220>
<223> Amino Acid Sequence of Domain Antibody

<400> 284

Asp Ile Gln Met Thr Gln Ser Pro Ser Ser Leu Ser Ala Ser Val Gly
1               5                   10                  15

Asp Arg Val Thr Ile Thr Cys Arg Ala Ser Gln Ser Val Lys Glu Phe
            20                  25                  30

Leu Trp Trp Tyr Gln Gln Lys Pro Gly Lys Ala Pro Lys Leu Leu Ile
        35                  40                  45

Tyr Met Ala Ser Asn Leu Gln Ser Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Thr Tyr Tyr Cys Gln Gln Lys Phe Lys Leu Pro Arg
                85                  90                  95

Thr Phe Gly Gln Gly Thr Lys Val Glu Ile Lys Arg
            100                 105

<210> 285
<211> 324
<212> DNA
<213> Artificial Sequence

<220>
<223>  Nucleotide Sequence of Domain Antibody

<400>  285
gacatccaga tgacccagtc tccatcctcc ctgtctgcat ctgtaggaga ccgtgtcacc      60

atcacttgcc gggcaagtca gagcgttaag gagtttttat ggtggtacca gcagaaacca     120

gggaaagccc ctaagctcct gatctatatg gcatccaatt tgcaaagtgg ggtcccatca     180

cgtttcagtg gcagtggatc tgggacagat ttcactctca ccatcagcag tctgcaacct     240

gaagattttg ctacgtacta ctgtcaacag aagtttaagc tgcctcgtac gttcggccaa     300

gggaccaagg tggaaatcaa acgg                                            324


<210>  286
<211>  120
<212>  PRT
<213>  Artificial Sequence

<220>
<223>  Amino Acid Sequence of Domain Antibody

<400>  286

Glu Val Gln Leu Leu Glu Ser Gly Gly Gly Leu Val Gln Pro Gly Gly
1               5                   10                  15


Ser Leu Arg Leu Ser Cys Ala Ala Ser Gly Phe Thr Phe Glu Trp Tyr
            20                  25                  30


Trp Met Gly Trp Val Arg Gln Ala Pro Gly Lys Gly Leu Glu Trp Val
        35                  40                  45


Ser Ala Ile Ser Gly Ser Gly Gly Ser Thr Tyr Tyr Ala Asp Ser Val
    50                  55                  60


Lys Gly Arg Phe Thr Ile Ser Arg Asp Asn Ser Lys Asn Thr Leu Tyr
65                  70                  75                  80


Leu Gln Met Asn Ser Leu Arg Ala Glu Asp Thr Ala Val Tyr Tyr Cys
                85                  90                  95


Ala Lys Val Lys Leu Gly Gly Gly Pro Asn Phe Asp Tyr Trp Gly Gln
            100                 105                 110

Gly Thr Leu Val Thr Val Ser Ser
        115                 120

<210>  287
<211>  362
<212>  DNA
<213>  Artificial Sequence

<220>
<223>  Nucleotide Sequence of Domain Antibody

<400>  287
gaggtgcagc tgttggagtc tgggggaggc ttggtacagc ctggggggtc cctgcgtctc      60

tcctgtgcag cctccggatt cacctttgag tggtattgga tgggttgggt ccgccaggct     120

ccagggaagg gtctagagtg ggtctcagct attagtggta gtggtggtag cacatactac     180

gcagactccg tgaagggccg gttcaccatc tcccgcgaca attccaagaa cacgctgtat     240

ctgcaaatga acagcctgcg tgccgaggac accgcggtat attactgtgc gaaagttaag     300

ttgggggggg ggcctaattt tgactactgg ggccagggaa ccctggtcac cgtctcgagc     360

gc                                                                    362

<210>  288
<211>  108
<212>  PRT
<213>  Artificial Sequence

<220>
<223>  Amino Acid Sequence of Domain Antibody

<400>  288

Asp Ile Gln Met Thr Gln Ser Pro Ser Ser Leu Ser Ala Ser Val Gly
1               5                   10                  15

Asp Arg Val Thr Ile Thr Cys Arg Ala Ser Gln Ser Ile Asp Ser Tyr
            20                  25                  30

Leu His Trp Tyr Gln Gln Lys Pro Gly Lys Ala Pro Lys Leu Leu Ile
        35                  40                  45

Tyr Ser Ala Ser Glu Leu Gln Ser Gly Val Pro Ser Arg Phe Ser Gly

50                  55                  60

Ser Gly Ser Gly Thr Asp Phe Thr Leu Thr Ile Ser Ser Leu Gln Pro
65                  70                  75                  80

Glu Asp Phe Ala Thr Tyr Tyr Cys Gln Gln Val Val Trp Arg Pro Phe
                85                  90                  95

Thr Phe Gly Gln Gly Thr Lys Val Glu Ile Lys Arg
            100                 105

<210>  289
<211>  324
<212>  DNA
<213>  Artificial Sequence

<220>
<223>  Nucleotide Sequence of Domain Antibody

<400>  289
gacatccaga tgacccagtc tccatcctct ctgtctgcat ctgtaggaga ccgtgtcacc      60

atcacttgcc gggcaagtca gagcattgat agttatttac attggtacca gcagaaacca     120

gggaaagccc ctaagctcct gatctatagt gcatccgagt tgcaaagtgg ggtcccatca     180

cgtttcagtg gcagtggatc tgggacagat ttcactctca ccatcagcag tctgcaacct     240

gaagattttg ctacgtacta ctgtcaacag gttgtgtggc gtccttttac gttcggccaa     300

gggaccaagg tggaaatcaa acgc                                            324

<210>  290
<211>  108
<212>  PRT
<213>  Artificial Sequence

<220>
<223>  Amino Acid Sequence of Domain Antibody

<400>  290

Asp Ile Gln Met Thr Gln Ser Pro Ser Ser Leu Ser Ala Ser Val Gly
1               5                   10                  15

Asp Arg Val Thr Ile Thr Cys Arg Ala Ser Gln Ser Ile Phe Met Asn
            20                  25                  30

Leu Leu Trp Tyr Gln Gln Lys Pro Gly Lys Ala Pro Lys Leu Leu Ile
        35                  40                  45

Tyr Asn Ala Ser Val Leu Gln Ser Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Thr Tyr Tyr Cys Gln Gln Val Val Trp Arg Pro Phe
                85                  90                  95

Thr Phe Gly Gln Gly Thr Lys Val Glu Ile Lys Arg
            100                 105

<210>  291
<211>  324
<212>  DNA
<213>  Artificial Sequence

<220>
<223>  Nucleotide Sequence of Domain Antibody

<400>  291
gacatccaga tgacccagtc tccatcctcc ctgtctgcat ctgtaggaga ccgtgtcacc      60

atcacttgcc gggcaagtca gagcattttt atgaatttat tgtggtacca gcagaaacca     120

gggaaagccc ctaagctcct gatctataat gcatccgtgt tgcaaagtgg ggtcccatca     180

cgtttcagtg gcagtggatc tgggacagat ttcactctca ccatcagcag tctgcaacct     240

gaagattttg ctacgtacta ctgtcaacag gttgtgtggc gtccttttac gttcggccaa     300

gggaccaagg tggaaatcaa acgg                                            324

<210>  292
<211>  108
<212>  PRT
<213>  Artificial Sequence

<220>
<223>  Amino Acid Sequence of Domain Antibody

<400>  292

Asp Ile Gln Met Thr Gln Ser Pro Ser Ser Leu Ser Ala Ser Val Gly
1               5                   10                  15

Asp Arg Val Thr Ile Thr Cys Arg Ala Ser Gln Ser Ile Tyr Asp Ala
            20                  25                  30

Leu Glu Trp Tyr Gln Gln Lys Pro Gly Lys Ala Pro Lys Leu Leu Ile
        35                  40                  45

Tyr Thr Ala Ser Arg Leu Gln Ser Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Thr Tyr Tyr Cys Gln Gln Val Met Gln Arg Pro Val
                85                  90                  95

Thr Phe Gly Gln Gly Thr Lys Val Glu Ile Lys Arg
            100                 105

<210>  293
<211>  324
<212>  DNA
<213>  Artificial Sequence

<220>
<223>  Nucleotide Sequence of Domain Antibody

<400>  293
gacatccaga tgacccagtc tccatcctcc ctgtctgcat ctgtaggaga ccgtgtcacc      60

atcacttgcc gggcaagtca gagcatttat gatgcgttag agtggtacca gcagaaacca     120

gggaaagccc ctaagctcct gatctatact gcatcccggt tgcaaagtgg ggtcccatca     180

cgtttcagtg gcagtggatc tgggacagat ttcactctca ccatcagcag tctgcaacct     240

gaagattttg ctacgtacta ctgtcaacag gttatgcagc gtcctgttac gttcggccaa     300

gggaccaagg tggaaatcaa acgg                                            324

<210>  294
<211>  108

<212>  PRT
<213>  Artificial Sequence

<220>
<223>  Amino Acid Sequence of Domain Antibody

<220>
<221>  MISC_FEATURE
<222>  (34)..(34)
<223>  Stop Codon

<400>  294

Asp Ile Gln Met Thr Gln Ser Pro Ser Ser Leu Ser Ala Ser Val Gly
1               5                   10                  15

Asp Arg Val Thr Ile Thr Cys Arg Ala Ser Gln Ser Ile Tyr Asp Ala
            20                  25                  30

Leu Xaa Trp Tyr Gln Gln Lys Pro Gly Lys Ala Pro Lys Leu Leu Ile
        35                  40                  45

Tyr Thr Ala Ser Arg Leu Gln Ser Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Thr Tyr His Cys Gln Gln Val Met Gln Arg Pro Val
                85                  90                  95

Thr Phe Gly Gln Gly Thr Lys Val Glu Ile Lys Arg
            100                 105

<210>  295
<211>  324
<212>  DNA
<213>  Artificial Sequence

<220>
<223>  Nucleotide Sequence of Domain Antibody

<400>  295
gacatccaga tgacccagtc tccatcctcc ctgtctgcat ctgtaggaga ccgtgtcacc      60

atcacttgcc gggcaagtca gagcatttat gatgctttac agtggtacca gcagaaacca     120

gggaaagccc ctaagctcct gatctatact gcatcccggt tgcaaagtgg ggtcccatca     180

cgtttcagtg gcagtggatc tgggacagat ttcactctca ccatcagcag tctgcaacct     240

gaagattttg ctacgtacca ctgtcaacag gttatgcagc gtcctgttac gttcggccaa     300

gggaccaagg tggaaatcaa acgg                                            324

<210>  296
<211>  108
<212>  PRT
<213>  Artificial Sequence

<220>
<223>  Amino Acid Sequence of Domain Antibody

<400>  296

Asp Ile Gln Met Thr Gln Ser Pro Ser Ser Leu Ser Ala Ser Val Gly
1               5                   10                  15

Asp Arg Val Thr Ile Thr Cys Arg Ala Ser Gln Ser Val Lys Glu Phe
            20                  25                  30

Leu Trp Trp Tyr Gln Gln Lys Pro Gly Lys Ala Pro Lys Leu Leu Ile
        35                  40                  45

Tyr Met Ala Ser Asn Leu Gln Ser Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Thr Tyr Tyr Cys Gln Gln Lys Phe Lys Leu Pro Arg
                85                  90                  95

Thr Phe Gly Gln Gly Thr Lys Val Glu Ile Lys Arg
            100                 105

<210>  297
<211>  324
<212>  DNA
<213>  Artificial Sequence

<220>
<223>  Nucleotide Sequence of Domain Antibody

<400>  297
gacatccaga tgacccagtc tccatcctcc ctgtctgcat ctgtaggaga ccgtgtcacc      60

atcacttgcc gggcaagtca gagcgttaag gagtttttat ggtggtacca gcagaaacca     120

gggaaagccc ctaagctcct gatctatatg gcatccaatt tgcaaagtgg ggtcccatca     180

cgtttcagtg gcagtggatc tgggacagat ttcactctca ccatcagcag tctgcaacct     240

gaagattttg ctacgtacta ctgtcaacag aagtttaagc tgcctcgtac gttcggccaa     300

gggaccaagg tggaaatcaa acgg                                            324


<210>  298
<211>  108
<212>  PRT
<213>  Artificial Sequence

<220>
<223>  Amino Acid Sequence of Domain Antibody

<400>  298

Asp Ile Gln Met Thr Gln Ser Pro Ser Ser Leu Ser Ala Ser Val Gly
1               5                   10                  15


Asp Arg Val Thr Ile Thr Cys Arg Ala Ser Gln Ser Ile Trp Thr Lys
            20                  25                  30


Leu His Trp Tyr Gln Gln Lys Pro Gly Lys Ala Pro Lys Leu Leu Ile
        35                  40                  45


Tyr Met Ala Ser Ser Leu Gln Ser Gly Val Pro Ser Arg Phe Ser Gly
    50                  55                  60


Ser Gly Ser Gly Thr Asp Phe Thr Leu Thr Ile Ser Ser Leu Gln Pro
65                  70                  75                  80


Glu Asp Phe Ala Thr Tyr Tyr Cys Gln Gln Trp Phe Ser Asn Pro Ser
                85                  90                  95


Thr Phe Gly Gln Gly Thr Lys Val Glu Ile Lys Arg

            100                 105

<210> 299
<211> 324
<212> DNA
<213> Artificial Sequence

<220>
<223> Nucleotide Sequence of Domain Antibody

<400> 299
gacatccaga tgacccagtc tccatcctcc ctgtctgcat ctgtaggaga ccgtgtcacc 60

atcacttgcc gggcaagtca gagcatttgg acgaagttac attggtacca gcagaaacca 120

gggaaagccc ctaagctcct gatctatatg gcatccagtt tgcaaagtgg ggtcccatca 180

cgtttcagtg gcagtggatc tgggacagat ttcactctca ccatcagcag tctgcaacct 240

gaagattttg ctacgtacta ctgtcaacag tggtttagta atcctagtac gttcggccaa 300

gggaccaagg tggaaatcaa acgc 324

<210> 300
<211> 108
<212> PRT
<213> Artificial Sequence

<220>
<223> Amino Acid Sequence of Domain Antibody

<220>
<221> MISC_FEATURE
<222> (30)..(30)
<223> Stop Codon

<400> 300

Asp Ile Gln Met Thr Gln Ser Pro Ser Ser Leu Ser Ala Ser Val Gly
1               5                   10                  15

Asp Arg Val Thr Ile Thr Cys Arg Ala Ser Gln Ser Ile Xaa Pro Ile
            20                  25                  30

Leu Cys Trp Tyr Gln Gln Lys Pro Gly Lys Ala Pro Lys Leu Leu Ile
        35                  40                  45

Tyr Ala Ala Ser Ser Leu Gln Ser Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Thr Tyr Tyr Cys Gln Gln Ile Gln His Ile Pro Val
                85                  90                  95

Thr Phe Gly Gln Gly Thr Lys Val Glu Ile Lys Arg
            100                 105

<210>  301
<211>  324
<212>  DNA
<213>  Artificial Sequence

<220>
<223>  Nucleotide Sequence of Domain Antibody

<400>  301
gacatccaga tgacccagtc tccatcctcc ctgtctgcat ctgtaggaga ccgtgtcacc      60

atcacttgcc gggcaagtca gagcatttag ccgattttat gttggtacca gcagaaacca     120

gggaaagccc ctaagctcct gatctatgct gcatccagtt tgcaaagtgg ggtcccatca     180

cgtttcagtg gcagtggatc tgggacagat ttcactctca ccatcagcag tctgcaacct     240

gaagattttg ctacgtacta ctgtcaacag attcagcata ttcctgtgac gttcggccaa     300

gggaccaagg tggaaatcaa acgg                                            324

<210>  302
<211>  108
<212>  PRT
<213>  Artificial Sequence

<220>
<223>  Amino Acid Sequence of Domain Antibody

<220>
<221>  MISC_FEATURE
<222>  (31)..(31)
<223>  Stop Codon

<400>  302

Asp Ile Gln Met Thr Gln Ser Pro Ser Ser Leu Ser Ala Ser Val Gly
1               5                   10                  15

Asp Arg Val Thr Ile Thr Cys Arg Ala Ser Gln Ser Ile Gly Xaa Asp
            20                  25                  30

Leu His Trp Tyr Gln Gln Lys Pro Gly Lys Ala Pro Lys Leu Leu Ile
        35                  40                  45

Tyr Thr Ala Ser Leu Leu Gln Ser Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Thr Tyr Tyr Cys Gln Gln Gln Ser Ala Phe Pro Asn
                85                  90                  95

Thr Leu Gly Gln Gly Thr Lys Val Glu Ile Lys Arg
            100                 105

<210>  303
<211>  324
<212>  DNA
<213>  Artificial Sequence

<220>
<223>  Nucleotide Sequence of Domain Antibody

<400>  303
gacatccaga tgacccagtc tccatcctcc ctgtctgcat ctgtaggaga ccgtgtcacc      60

atcacttgcc gggcaagtca gagcattggg taggatttac attggtacca gcagaaacca     120

gggaaagccc ctaagctcct gatctatacg gcatcccttt tgcaaagtgg ggtcccatca     180

cgtttcagtg gcagtggatc tgggacagat ttcactctca ccatcagcag tctgcaacct     240

gaagattttg ctacgtacta ctgtcaacag cagagtgctt ttcctaatac gctcggccaa     300

gggaccaagg tggaaatcaa acgg                                            324

<210>  304
<211>  108

<212>  PRT
<213>  Artificial Sequence

<220>
<223>  Amino Acid Sequence of Domain Antibody

<220>
<221>  MISC_FEATURE
<222>  (50)..(50)
<223>  Stop Codon


<400>  304

Asp Ile Gln Met Thr Gln Ser Pro Ser Ser Leu Ser Ala Ser Val Gly
1               5                   10                  15


Asp Arg Val Thr Ile Thr Cys Arg Ala Ser Gln Ser Ile Thr Lys Asn
            20                  25                  30


Leu Leu Trp Tyr Gln Gln Lys Pro Gly Lys Ala Pro Lys Leu Leu Ile
        35                  40                  45


Tyr Xaa Ala Ser Ser Leu Gln Ser Gly Val Pro Ser Arg Phe Ser Gly
    50                  55                  60


Ser Gly Ser Gly Thr Asp Phe Thr Leu Thr Ile Ser Ser Leu Gln Pro
65                  70                  75                  80


Glu Asp Phe Ala Thr Tyr Tyr Cys Gln Gln Leu Arg His Lys Pro Pro
                85                  90                  95


Thr Phe Gly Gln Gly Thr Lys Val Glu Ile Lys Arg
            100                 105


<210>  305
<211>  324
<212>  DNA
<213>  Artificial Sequence

<220>
<223>  Nucleotide Sequence of Domain Antibody

<400>  305
gacatccaga tgacccagtc tccatcctcc ctgtctgcat ccgtaggaga ccgtgtcacc      60

atcacttgcc gggcaagtca gagcataacg aagaatttac tttggtacca gcagaaacca 120

gggaaagccc ctaagctcct gatctattag gcatcctctt tgcaaagtgg ggtcccatca 180

cgtttcagtg gcagtggatc tgggacagat ttcactctca ccatcagcag tctgcaacct 240

gaagattttg ctacgtacta ctgtcaacag cttcgtcata agcctccgac gttcggccaa 300

gggaccaagg tggaaatcaa acgg 324

<210> 306
<211> 108
<212> PRT
<213> Artificial Sequence

<220>
<223> Amino Acid Sequence of Domain Antibody

<220>
<221> MISC_FEATURE
<222> (30)..(30)
<223> Stop Codon

<400> 306

Asp Ile Gln Met Thr Gln Ser Pro Ser Ser Leu Ser Ala Ser Val Gly
1               5                   10                  15

Asp Arg Val Thr Ile Thr Cys Arg Ala Ser Gln Ser Ile Xaa Lys Ser
            20                  25                  30

Leu Arg Trp Tyr Gln Gln Lys Pro Gly Lys Ala Pro Lys Leu Leu Ile
        35                  40                  45

Tyr His Ala Ser Asp Leu Gln Ser Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Thr Tyr Tyr Cys Gln Gln Met Val Asn Ser Pro Val
                85                  90                  95

Thr Phe Gly Gln Gly Thr Lys Val Glu Ile Lys Arg
            100                 105

<210>  307
<211>  324
<212>  DNA
<213>  Artificial Sequence

<220>
<223>  Nucleotide Sequence of Domain Antibody

<400>  307
gacatccaga tgacccagtc tccatcctcc ctgtctgcat ctgtaggaga ccgtgtcacc      60

atcacttgcc gggcaagtca gagcatttag aagtctttaa ggtggtacca gcagaaacca     120

gggaaagccc ctaagctcct gatctatcat gcatccgatt tgcaaagtgg ggtcccatca     180

cgtttcagtg gcagtggatc tgggacagat ttcactctca ccatcagcag tctgcaacct     240

gaagattttg ctacgtacta ctgtcaacag atggttaata gtcctgttac gttcggccaa     300

gggaccaagg tggaaatcaa acgg                                            324


<210>  308
<211>  108
<212>  PRT
<213>  Artificial Sequence

<220>
<223>  Amino Acid Sequence of Domain Antibody

<220>
<221>  MISC_FEATURE
<222>  (30)..(30)
<223>  Stop Codon


<400>  308

Asp Ile Gln Met Thr Gln Ser Pro Ser Ser Leu Ser Ala Ser Val Gly
1               5                   10                  15


Asp Arg Val Thr Ile Thr Cys Arg Ala Ser Gln Ser Ile Xaa Thr Ala
            20                  25                  30


Leu His Trp Tyr Gln Gln Lys Pro Gly Lys Ala Pro Lys Leu Leu Ile
        35                  40                  45


Tyr Ser Ala Ser Ser Leu Gln Ser Gly Val Pro Ser Arg Phe Ser Gly

50                  55                  60

Ser Gly Ser Gly Thr Asp Phe Thr Leu Thr Ile Ser Ser Leu Gln Pro
65                  70                  75                  80

Glu Asp Phe Ala Thr Tyr Tyr Cys Gln Gln Ser Ser Phe Leu Pro Phe
                85                  90                  95

Thr Phe Gly Gln Gly Thr Lys Val Glu Ile Lys Arg
            100                 105

<210>  309
<211>  324
<212>  DNA
<213>  Artificial Sequence

<220>
<223>  Nucleotide Sequence of Domain Antibody

<400>  309
gacatccaga tgacccagtc tccatcctcc ctgtctgcat ctgtaggaga ccgtgtcacc      60

atcacttgcc gggcaagtca gagcatttag acggcgttac attggtacca gcagaaacca     120

gggaaagccc ctaagctcct gatctattct gcatccagtt tgcaaagtgg ggtcccatca     180

cgtttcagtg gcagtggatc tgggacagat ttcactctca ccatcagcag tctgcaacct     240

gaagattttg ctacgtacta ctgtcaacag tcgagttttt tgccttttac gttcggccaa     300

gggaccaagg tggaaatcaa acgg                                            324

<210>  310
<211>  108
<212>  PRT
<213>  Artificial Sequence

<220>
<223>  Amino Acid Sequence of Domain Antibody

<400>  310

Asp Ile Gln Met Thr Gln Ser Pro Ser Ser Leu Ser Ala Ser Val Gly
1               5                   10                  15

Asp Arg Val Thr Ile Thr Cys Arg Ala Ser Gln Ser Ile Gly Pro Asn
            20                  25                  30

Leu Glu Trp Tyr Gln Gln Lys Pro Gly Lys Ala Pro Lys Leu Leu Ile
        35                  40                  45

Tyr Ala Ala Ser Ser Leu Gln Ser Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Thr Tyr Tyr Cys Gln Gln Gln Met Gly Arg Pro Arg
                85                  90                  95

Thr Phe Gly Gln Gly Thr Lys Val Glu Ile Lys Arg
            100                 105

<210>  311
<211>  324
<212>  DNA
<213>  Artificial Sequence

<220>
<223>  Nucleotide Sequence of Domain Antibody

<400>  311
gacatccaga tgacccagtc tccatcctcc ctgtctgcat ctgtaggaga ccgtgtcacc      60

atcacttgcc gggcaagtca gagcattggg ccgaatttag agtggtacca gcagaaacca     120

gggaaagccc ctaagctcct gatctatgct gcatccagtt tgcaaagtgg ggtcccatca     180

cgtttcagtg gcagtggatc tgggacagat ttcactctca ccatcagcag tctgcaacct     240

gaagattttg ctacgtacta ctgtcaacag cagatggggc gtcctcggac gttcggccaa     300

gggaccaagg tggaaatcaa acgg                                            324

<210>  312
<211>  108
<212>  PRT
<213>  Artificial Sequence

<220>
<223>  Amino Acid Sequence of Domain Antibody

<220>

<221>  MISC_FEATURE
<222>  (32)..(32)
<223>  Stop Codon

<400>  312

Asp Ile Gln Met Thr Gln Ser Pro Ser Ser Leu Ser Ala Ser Val Gly
1               5                   10                  15

Asp Arg Val Thr Ile Thr Cys Arg Ala Ser Gln Ser Ile Lys His Xaa
            20                  25                  30

Leu Ala Trp Tyr Gln Gln Lys Pro Gly Lys Ala Pro Lys Leu Leu Ile
        35                  40                  45

Tyr Lys Ala Ser Val Leu Gln Ser Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Thr Tyr Tyr Cys Gln Gln Leu Arg Arg Arg Pro Thr
                85                  90                  95

Thr Phe Gly Gln Gly Thr Lys Val Glu Ile Lys Arg
            100                 105

<210>  313
<211>  324
<212>  DNA
<213>  Artificial Sequence

<220>
<223>  Nucleotide Sequence of Domain Antibody

<400>  313
gacatccaga tgacccagtc tccatcctcc ctgtctgcat ctgtaggaga ccgtgtcacc      60

atcacttgcc gggcaagtca gagcattaag cattagttag cttggtacca gcagaaacca     120

gggaaagccc ctaagctcct gatctataag gcatccgtgt tgcaaagtgg ggtcccatca     180

cgtttcagtg gcagtggatc tgggacagat ttcactctca ccatcagcag tctgcaacct     240

gaagattttg ctacgtacta ctgtcaacag cttaggcgtc gtcctactac gttcggccaa     300

gggaccaagg tggaaatcaa acgg 324

<210> 314
<211> 108
<212> PRT
<213> Artificial Sequence

<220>
<223> Amino Acid Sequence of Domain Antibody

<220>
<221> MISC_FEATURE
<222> (32)..(32)
<223> Stop Codon

<400> 314

Asp Ile Gln Met Thr Gln Ser Pro Ser Ser Leu Ser Ala Ser Val Gly
1               5                   10                  15

Asp Arg Val Thr Ile Thr Cys Arg Ala Ser Gln Ser Val Lys Ala Xaa
            20                  25                  30

Leu Thr Trp Tyr Gln Gln Lys Pro Gly Lys Ala Pro Lys Leu Leu Ile
        35                  40                  45

Tyr Lys Ala Ser Thr Leu Gln Ser Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Thr Tyr Tyr Cys Gln Gln His Ser Ser Arg Pro Tyr
                85                  90                  95

Thr Phe Gly Gln Gly Thr Lys Val Glu Ile Lys Arg
            100                 105

<210> 315
<211> 324
<212> DNA
<213> Artificial Sequence

<220>
<223>  Nucleotide Sequence of Domain Antibody

<400>  315
gacatccaga tgacccagtc tccatcctcc ctgtctgcat ctgtaggaga ccgtgtcacc      60

atcacttgcc gggcaagtca gagcgttaag gcttagttaa cttggtacca gcagaaacca     120

gggaaagccc ctaagctcct gatctataag gcatccactt tgcaaagtgg ggtcccatca     180

cgtttcagtg gcagtggatc tgggacagat ttcactctca ccatcagcag tctgcaacct     240

gaagattttg ctacgtacta ctgtcaacag catagttcta ggccttatac gttcggccaa     300

gggaccaagg tggaaatcaa acgg                                            324


<210>  316
<211>  108
<212>  PRT
<213>  Artificial Sequence

<220>
<223>  Amino Acid Sequence of Domain Antibody

<220>
<221>  MISC_FEATURE
<222>  (50)..(50)
<223>  Stop Codon


<400>  316

Asp Ile Gln Met Thr Gln Ser Pro Ser Ser Leu Ser Ala Ser Val Gly
1               5                   10                  15


Asp Arg Val Thr Ile Thr Cys Arg Ala Ser Gln Ser Ile Glu Asn Arg
            20                  25                  30


Leu Gly Glu Tyr Gln Gln Lys Pro Gly Lys Ala Pro Lys Leu Leu Ile
        35                  40                  45


Tyr Xaa Ala Ser Leu Leu Gln Ser Gly Val Pro Ser Arg Phe Ser Gly
    50                  55                  60


Ser Gly Ser Gly Thr Asp Phe Thr Leu Thr Ile Ser Ser Leu Gln Pro
65                  70                  75                  80

Glu Asp Phe Ala Thr Tyr Tyr Cys Gln Gln Asp Ser Tyr Phe Pro Arg
                85                  90                  95

Thr Phe Gly Gln Gly Thr Lys Val Glu Ile Lys Arg
            100                 105

<210>  317
<211>  324
<212>  DNA
<213>  Artificial Sequence

<220>
<223>  Nucleotide Sequence of Domain Antibody

<400>  317
gacatccaga tgacccagtc tccatcctcc ctgtctgcat ctgtaggaga ccgtgtcacc      60

atcacttgcc gggcaagtca gagcattgag aatcggttag gttggtacca gcagaaacca     120

gggaaagccc ctaagctcct gatctattag gcgtccttgt tgcaaagtgg ggtcccatca     180

cgtttcagtg gcagtggatc tgggacagat ttcactctca ccatcagcag tctgcaacct     240

gaagattttg ctacgtacta ctgtcaacag gattcgtatt ttcctcgtac gttcggccaa     300

gggaccaagg tggaaatcaa acgg                                            324

<210>  318
<211>  108
<212>  PRT
<213>  Artificial Sequence

<220>
<223>  Amino Acid Sequence of Domain Antibody

<220>
<221>  MISC_FEATURE
<222>  (50)..(50)
<223>  Stop Codon

<400>  318

Asp Ile Gln Met Thr Gln Ser Pro Ser Ser Leu Ser Ala Ser Val Gly
1               5                   10                  15

Asp Arg Val Thr Ile Thr Cys Arg Ala Ser Gln Ser Ile Met Asp Lys
            20                  25                  30

Leu Lys Trp Tyr Gln Gln Lys Pro Gly Lys Ala Pro Lys Leu Leu Ile
        35                  40                  45

Tyr Xaa Ala Ser Ile Leu Gln Ser Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Thr Tyr Tyr Cys Gln Gln Asp Ser Gly Gly Pro Asn
                85                  90                  95

Thr Phe Gly Gln Gly Thr Lys Val Glu Ile Lys Arg
            100                 105

<210> 319
<211> 324
<212> DNA
<213> Artificial Sequence

<220>
<223> Nucleotide Sequence of Domain Antibody

<400> 319
gacatccaga tgacccagtc tccatcctcc ctgtctgcat ctgtaggaga ccgtgtcacc 60

atcacttgcc gggcaagtca gagcattatg gataagttaa agtggtacca gcagaaacca 120

gggaaagccc ctaagctcct gatctattag gcatccattt tgcaaagtgg ggtcccatca 180

cgtttcagtg gcagtggatc tgggacagat ttcactctca ccatcagcag tctgcaacct 240

gaagattttg ctacgtacta ctgtcaacag gatagtgggg gtcctaatac gttcggccaa 300

gggaccaagg tggaaatcaa acgg 324

<210> 320
<211> 108
<212> PRT
<213> Artificial Sequence

<220>
<223> Amino Acid Sequence of Domain Antibody

<400> 320

Asp Ile Gln Met Thr Gln Ser Pro Ser Ser Leu Ser Ala Ser Val Gly
1               5                   10                  15

Asp Arg Val Thr Ile Thr Cys Arg Ala Ser Gln Ser Ile Gly Arg Asn
            20                  25                  30

Leu Glu Trp Tyr Gln Gln Lys Pro Gly Lys Ala Pro Lys Leu Leu Ile
        35                  40                  45

Tyr Asp Ala Ser His Leu Gln Ser Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Thr Tyr Tyr Cys Gln Gln Ser Arg Glu Leu Pro Arg
                85                  90                  95

Thr Phe Gly Gln Gly Thr Lys Val Glu Ile Lys Arg
            100                 105

<210>  321
<211>  324
<212>  DNA
<213>  Artificial Sequence

<220>
<223>  Nucleotide Sequence of Domain Antibody

<400>  321
gacatccaga tgacccagtc tccatcctcc ctgtctgcat ctgtaggaga ccgtgtcacc      60

atcacttgcc gggcaagtca gagcattggg aggaatttag agtggtacca gcagaaacca     120

gggaaagccc ctaagctcct gatctatgat gcatcccatt tgcaaagtgg ggtcccatca     180

cgtttcagtg gcagtggatc tgggacagat ttcactctca ccatcagcag tctgcaacct     240

gaagattttg ctacgtacta ctgtcaacag tcgcgttggc ttcctcgtac gttcggccaa     300

gggaccaagg tggaaatcaa acgg                                            324

<210>  322
<211>  108
<212>  PRT

<213>  Artificial Sequence

<220>
<223>  Amino Acid Sequence of Domain Antibody

<400>  322

Asp Ile Gln Met Thr Gln Ser Pro Ser Ser Leu Ser Ala Ser Val Gly
1               5                   10                  15

Asp Arg Val Thr Ile Thr Cys Arg Ala Ser Gln Ser Ile Arg Lys Met
            20                  25                  30

Leu Val Trp Tyr Gln Gln Lys Pro Gly Lys Ala Pro Lys Leu Leu Ile
        35                  40                  45

Tyr Arg Ala Ser Tyr Leu Gln Ser Gly Val Pro Ser Arg Phe Ser Gly
    50                  55                  60

Ser Gly Ser Gly Thr Asp Phe Thr Leu Thr Ile Ser Ser Leu Gln Pro
65                  70                  75                  80

Glu Asp Phe Ala Thr Tyr Tyr Cys Gln Gln Ala Phe Arg Arg Pro Arg
                85                  90                  95

Thr Phe Gly Gln Gly Thr Lys Val Glu Ile Lys Arg
            100                 105

<210>  323
<211>  324
<212>  DNA
<213>  Artificial Sequence

<220>
<223>  Nucleotide Sequence of Domain Antibody

<400>  323
gacatccaga tgacccagtc tccatcctcc ctgtctgcat ctgtaggaga ccgtgtcacc      60

atcacttgcc gggcaagtca gagcattagg aagatgttag tttggtacca gcagaaacca     120

gggaaagccc ctaagctcct gatctatcgg gcatcctatt tgcaaagtgg ggtcccatca     180

cgtttcagtg gcagtggatc tgggacagat ttcactctca ccatcagcag tctgcaacct     240

gaagattttg ctacgtacta ctgtcaacag gcttttcggc ggcctaggac gttcggccaa     300

gggaccaagg tggaaatcaa acgg                                                324

<210>  324
<211>  115
<212>  PRT
<213>  Artificial Sequence

<220>
<223>  Amino Acid Sequence of Domain Antibody

<400>  324

Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Asp Leu Tyr
            20                  25                  30

Asn Met Phe Trp Val Arg Gln Ala Pro Gly Lys Gly Leu Glu Trp Val
        35                  40                  45

Ser Phe Ile Ser Gln Thr Gly Arg Leu Thr Trp Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Lys Thr Leu Glu Asp Phe Asp Tyr Trp Gly Gln Gly Thr Leu Val
            100                 105                 110

Thr Val Ser
        115

<210>  325
<211>  345
<212>  DNA
<213>  Artificial Sequence

<220>
<223>  Nucleotide Sequence of Domain Antibody

<400>  325
gaggtgcagc tgttggagtc tgggggaggc ttggtacagc ctggggggtc cctgcgtctc      60

tcctgtgcag cctccggatt cacctttgat ctttataata tgttttgggt ccgccaggct     120

ccagggaagg gtctagagtg ggtctcattt attagtcaga ctggtaggct tacatggtac     180

gcagactccg tgaagggccg gttcaccatc tcccgcgaca attccaagaa cacgctgtat     240

ctgcaaatga acagcctgcg tgccgaggac accgcggtat attactgtgc gaaaacgctg     300

gaggattttg actactgggg ccagggaacc ctggtcaccg tctcg                     345

<210>  326
<211>  119
<212>  PRT
<213>  Artificial Sequence

<220>
<223>  Amino Acid Sequence of Domain Antibody

<400>  326

Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Pro Val Tyr
            20                  25                  30

Met Met Gly Trp Val Arg Gln Ala Pro Gly Lys Gly Leu Glu Trp Val
        35                  40                  45

Ser Ser Ile Asp Ala Leu Gly Gly Arg Thr Gly Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Lys Thr Met Ser Asn Lys Thr His Thr Phe Asp Tyr Trp Gly Gln
            100                 105                 110

Gly Thr Leu Val Thr Val Ser
        115

<210>  327
<211>  357
<212>  DNA
<213>  Artificial Sequence

<220>
<223>  Nucleotide Sequence of Domain Antibody

<400>  327
gaggtgcagc tgttggagtc tgggggaggc ttggtacagc ctggggggtc cctgcgtctc      60

tcctgtgcag cctccggatt cacctttccg gtttatatga tgggttgggt ccgccaggct     120

ccagggaagg gtctagagtg ggtctcatcg attgatgctc ttggtgggcg gacaggttac     180

gcagactccg tgaagggccg gttcaccatc tcccgcgaca attccaagaa cacgctgtat     240

ctgcaaatga acagcctgcg tgccgaggac accgcggtat attactgtgc gaaaactatg     300

tcgaataaga cgcatacgtt tgactactgg ggccagggaa ccctggtcac cgtctcg        357

<210>  328
<211>  115
<212>  PRT
<213>  Artificial Sequence

<220>
<223>  Amino Acid Sequence of Domain Antibody

<220>
<221>  MISC_FEATURE
<222>  (57)..(57)
<223>  Stop Codon

<400>  328

Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Val Ala Tyr
            20                  25                  30

Asn Met Thr Trp Val Arg Gln Ala Pro Gly Lys Gly Leu Glu Trp Val
        35                  40                  45

Ser Ser Ile Asn Thr Phe Gly Asn Xaa Thr Arg Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Lys Gly Ser Arg Pro Phe Asp Tyr Trp Gly Gln Gly Thr Leu Val
            100                 105                 110

Thr Val Ser
        115

<210>  329
<211>  345
<212>  DNA
<213>  Artificial Sequence

<220>
<223>  Nucleotide Sequence of Domain Antibody

<400>  329
gaggtgcagc tgttggagtc tgggggaggc ttggtacagc ctggggggtc cctgcgtctc      60

tcctgtgcag cctccggatt cacctttgtg gcttataata tgacttgggt ccgccaggct     120

ccagggaagg gtctagagtg ggtctcaagt attaatactt ttggtaatta gacaaggtac     180

gcagactccg tgaagggccg gttcaccatc tcccgcgaca attccaagaa cacgctgtat     240

ctgcaaatga acagcctgcg tgccgaggac accgcggtat attactgtgc gaaaggtagt     300

aggccttttg actactgggg ccagggaacc ctggtcaccg tctcg                     345

<210>  330
<211>  119
<212>  PRT
<213>  Artificial Sequence

<220>
<223>  Amino Acid Sequence of Domain Antibody

<220>
<221>  MISC_FEATURE

<222>  (30)..(30)
<223>  Stop Codon

<400>  330

Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Xaa Gly Tyr
            20                  25                  30

Arg Met Gly Trp Val Arg Gln Ala Pro Gly Lys Gly Leu Glu Trp Val
        35                  40                  45

Ser Trp Ile Thr Arg Thr Gly Gly Thr Thr Gln Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Lys Pro Ala Lys Leu Val Gly Val Gly Phe Asp Tyr Trp Gly Gln
            100                 105                 110

Gly Thr Leu Val Thr Val Ser
        115

<210>  331
<211>  357
<212>  DNA
<213>  Artificial Sequence

<220>
<223>  Nucleotide Sequence of Domain Antibody

<400>  331
gaggtgcagc tgttggagtc tgggggaggc ttggtacagc ctggggggtc cctgcgtctc      60

tcctgtgcag cctccggatt caccttttag gggtatcgta tgggttgggt ccgccaggct      120

ccagggaagg gtctagagtg ggtctcatgg attacgcgta ctggtgggac gacacagtac      180

gcagactccg tgaagggccg gttcaccatc tcccgcgaca attccaagaa cacgctgtat 240

ctgcaaatga acagcctgcg tgccgaggac accgcggtat attactgtgc gaaaccggcg 300

aagcttgttg gggttgggtt tgactactgg ggccagggaa ccctggtcac cgtctcg 357

<210> 332
<211> 119
<212> PRT
<213> Artificial Sequence

<220>
<223> Amino Acid Sequence of Domain Antibody

<220>
<221> MISC_FEATURE
<222> (33)..(33)
<223> Stop Codon

<400> 332

Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Arg Lys Tyr
            20                  25                  30

Xaa Met Gly Trp Val Arg Gln Ala Pro Gly Lys Gly Leu Glu Trp Val
        35                  40                  45

Ser Gln Ile Gly Ala Lys Gly Gln Ser Thr Asp Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Lys Lys Lys Arg Gly Glu Asn Tyr Phe Phe Asp Tyr Trp Gly Gln
            100                 105                 110

Gly Thr Leu Val Thr Val Ser
        115

<210>  333
<211>  357
<212>  DNA
<213>  Artificial Sequence

<220>
<223>  Nucleotide Sequence of Domain Antibody

<400>  333
gaggtgcagc tgttggagtc tgggggaggc ttggtacagc ctggggggtc cctgcgtctc      60

tcctgtgcag cctccggatt cacctttcgg aagtattaga tggggtgggt ccgccaggct     120

ccagggaagg gtctagagtg ggtctcacag attggtgcga agggtcagtc tacagattac     180

gcagactccg tgaagggccg gttcaccatc tcccgcgaca attccaagaa cacgctgtat     240

ctgcaaatga acagcctgcg tgccgaggac accgcggtat attactgtgc gaaaaagaag     300

aggggggaga attatttttt tgactactgg ggccagggaa ccctggtcac cgtctcg        357


<210>  334
<211>  119
<212>  PRT
<213>  Artificial Sequence

<220>
<223>  Amino Acid Sequence of Domain Antibody

<400>  334

Glu Val Gln Leu Leu Glu Ser Gly Gly Gly Leu Val Gln Pro Gly Gly
1               5                   10                  15


Ser Leu Arg Leu Ser Cys Ala Ala Ser Gly Phe Thr Phe Arg Arg Tyr
            20                  25                  30


Ser Met Ser Trp Val Arg Gln Ala Pro Gly Lys Gly Leu Glu Trp Val
        35                  40                  45


Ser Asp Ile Ser Arg Ser Gly Arg Tyr Thr His Tyr Ala Asp Ser Val
    50                  55                  60


Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Lys Arg Ile Asp Ser Ser Gln Asn Gly Phe Asp Tyr Trp Gly Gln
            100                 105                 110

Gly Thr Leu Val Thr Val Ser
        115

<210>  335
<211>  357
<212>  DNA
<213>  Artificial Sequence

<220>
<223>  Nucleotide Sequence of Domain Antibody

<400>  335
gaggtgcagc tgttggagtc tgggggaggc ttggtacagc ctggggggtc cctgcgtctc      60

tcctgtgcag cctccggatt cacctttcgg cggtatagta tgtcgtgggt ccgccaggct     120

ccagggaagg gtctagagtg ggtctcagat atttctcgtt ctggtcggta tacacattac     180

gcagactccg tgaagggccg gttcaccatc tcccgcgaca attccaagaa cacgctgtat     240

ctgcaaatga acagcctgcg tgccgaggac accgcggtat attactgtgc gaaacgtatt     300

gattcttctc agaatgggtt tgactactgg ggccagggaa ccctggtcac cgtctcg        357

<210>  336
<211>  115
<212>  PRT
<213>  Artificial Sequence

<220>
<223>  Amino Acid Sequence of Domain Antibody

<220>
<221>  MISC_FEATURE
<222>  (30)..(30)
<223>  Stop Codon

<400>  336

Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Xaa Gly Tyr
            20                  25                  30

Lys Met Phe Trp Val Arg Gln Ala Pro Gly Lys Gly Leu Glu Trp Val
        35                  40                  45

Ser Ala Ile Ser Gly Ser Gly Gly Ser Thr Tyr Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Lys Gln Lys Glu Asn Phe Asp Tyr Trp Gly Gln Gly Thr Leu Val
            100                 105                 110

Thr Val Ser
        115

<210>  337
<211>  345
<212>  DNA
<213>  Artificial Sequence

<220>
<223>  Nucleotide Sequence of Domain Antibody

<400>  337
   gaggtgcagc tgttggagtc tgggggaggc ttggtacagc ctggggggtc cctgcgtctc      60
   tcctgtgcag cctccggatt caccttttag gggtataaga tgttttgggt ccgccaggct      120
   ccagggaagg gtctagagtg ggtctcagct attagtggta gtggtggtag cacatactac      180
   gcagactccg tgaagggccg gttcaccatc tcccgcgaca attccaagaa cacgctgtat      240
   ctgcaaatga acagcctgcg tgccgaggac accgcggtat attactgtgc gaaacagaag      300
   gagaattttg actactgggg ccagggaacc ctggtcaccg tctcg                      345

<210>  338

<211>  119
<212>  PRT
<213>  Artificial Sequence

<220>
<223>  Amino Acid Sequence of Domain Antibody

<400>  338

Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Gly Asp Tyr
            20                  25                  30

Ala Met Trp Trp Val Arg Gln Ala Pro Gly Lys Gly Leu Glu Trp Val
        35                  40                  45

Ser Val Ile Ser Ser Asn Gly Gly Ser Thr Phe Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Lys Arg Val Arg Lys Arg Thr Pro Glu Phe Asp Tyr Trp Gly Gln
            100                 105                 110

Gly Thr Leu Val Thr Val Ser
        115

<210>  339
<211>  357
<212>  DNA
<213>  Artificial Sequence

<220>
<223>  Nucleotide Sequence of Domain Antibody

<400>  339
gaggtgcagc tgttggagtc tgggggaggc ttggtacagc ctggggggtc cctgcgtctc      60

tcctgtgcag cctccggatt cacctttggg gattatgcta tgtggtgggt ccgccaggct     120

ccagggaagg gtctagagtg ggtctcagtg attagttcga atggtgggag tacattttac 180

gcagactccg tgaagggccg gttcaccatc tcccgcgaca attccaagaa cacgctgtat 240

ctgcaaatga acagcctgcg tgccgaggac accgcggtat attactgtgc gaaacgtgtt 300

cgtaagagga ctcctgagtt tgactactgg ggccagggaa ccctggtcac cgtctcg 357

<210> 340
<211> 119
<212> PRT
<213> Artificial Sequence

<220>
<223> Amino Acid Sequence of Domain Antibody

<400> 340

Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Arg Arg Tyr
            20                  25                  30

Lys Met Gly Trp Val Arg Gln Ala Pro Gly Lys Gly Leu Glu Trp Val
        35                  40                  45

Ser Ala Ile Gly Arg Asn Gly Thr Lys Thr Asn Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Lys Ile Tyr Thr Gly Lys Pro Ala Ala Phe Asp Tyr Trp Gly Gln
            100                 105                 110

Gly Thr Leu Val Thr Val Ser
        115

<210> 341

<211>  357
<212>  DNA
<213>  Artificial Sequence

<220>
<223>  Nucleotide Sequence of Domain Antibody

<400>  341
gaggtgcagc tgttggagtc tgggggaggc ttggtacagc ctggggggtc cctgcgtctc      60

tcctgtgcag cctccggatt cacctttagg aggtataaga tgggttgggt ccgccaggct     120

ccagggaagg gtctagagtg ggtctcagcg attgggagga atggtacgaa gacaaattac     180

gcagactccg tgaagggccg gttcaccatc tcccgcgaca attccaagaa cacgctgtat     240

ctgcaaatga acagcctgcg tgccgaggac accgcggtat attactgtgc gaaaatttat     300

acggggaagc ctgctgcgtt tgactactgg ggccagggaa ccctggtcac cgtctcg        357


<210>  342
<211>  119
<212>  PRT
<213>  Artificial Sequence

<220>
<223>  Amino Acid Sequence of Domain Antibody

<220>
<221>  MISC_FEATURE
<222>  (33)..(33)
<223>  Stop Codon


<400>  342

Glu Val Gln Leu Leu Glu Ser Gly Gly Gly Leu Val Gln Pro Gly Gly
1               5                   10                  15


Ser Leu Arg Leu Ser Cys Ala Ala Ser Gly Phe Thr Phe Lys Lys Tyr
            20                  25                  30


Xaa Met Ser Trp Val Arg Gln Ala Pro Gly Lys Gly Leu Glu Trp Val
        35                  40                  45


Ser Ala Ile Ser Gly Ser Gly Gly Ser Thr Tyr Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Lys Met Leu Arg Thr Lys Asn Lys Val Phe Asp Tyr Trp Gly Gln
            100                 105                 110

Gly Thr Leu Val Thr Val Ser
        115

<210>  343
<211>  357
<212>  DNA
<213>  Artificial Sequence

<220>
<223>  Nucleotide Sequence of Domain Antibody

<400>  343
gaggtgcagc tgttggagtc tgggggaggc ttggtacagc ctggggggtc cctgcgtctc      60

tcctgtgcag cctccggatt cacctttaag aagtattaga tgtcttgggt ccgccaggct     120

ccagggaagg gtctagagtg ggtctcagct attagtggta gtggtggtag cacatactac     180

gcagactccg tgaagggccg gttcaccatc tcccgcgaca attccaagaa cacgctgtat     240

ctgcaaatga acagcctgcg tgccgaggac accgcggtat attactgtgc gaaaatgctg     300

aggactaaga ataaggtgtt tgactactgg ggccagggaa ccctggtcac cgtctcg        357

<210>  344
<211>  119
<212>  PRT
<213>  Artificial Sequence

<220>
<223>  Amino Acid Sequence of Domain Antibody

<400>  344

Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Arg Arg Tyr

20                  25                  30

Lys Met Gly Trp Val Arg Gln Ala Pro Gly Lys Gly Leu Glu Trp Val
        35                  40                  45

Ser Ala Ile Gly Arg Asn Gly Thr Lys Thr Asn Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Lys Ile Tyr Thr Gly Lys Pro Ala Ala Phe Asp Tyr Trp Gly Gln
            100                 105                 110

Gly Thr Leu Val Thr Val Ser
        115

<210>  345
<211>  357
<212>  DNA
<213>  Artificial Sequence

<220>
<223>  Nucleotide Sequence of Domain Antibody

<400>  345
gaggtgcagc tgttggagtc tgggggaggc ttggtacagc ctggggggtc cctgcgtctc      60

tcctgtgcag cctccggatt cacctttagg aggtataaga tgggttgggt ccgccaggct     120

ccagggaagg gtctagagtg ggtctcagcg attgggagga atggtacgaa gacaaattac     180

gcagactccg tgaagggccg gttcaccatc tcccgcgaca attccaagaa cacgctgtat     240

ctgcaaatga acagcctgcg tgccgaggac accgcggtat attactgtgc gaaaatttat     300

acggggaagc ctgctgcgtt tgactactgg ggccagggaa ccctggtcac cgtctcg        357

<210>  346
<211>  119
<212>  PRT
<213>  Artificial Sequence

<220>
<223>  Amino Acid Sequence of Domain Antibody

<220>
<221>  MISC_FEATURE
<222>  (30)..(30)
<223>  Stop Codon


<400>  346

Glu Val Gln Leu Leu Glu Ser Gly Gly Gly Leu Val Gln Pro Gly Gly
1               5                   10                  15


Ser Leu Arg Leu Ser Cys Ala Ala Ser Gly Phe Thr Phe Xaa Ser Tyr
            20                  25                  30


Arg Met Gly Trp Val Arg Gln Ala Pro Gly Lys Gly Leu Glu Trp Val
        35                  40                  45


Ser Ser Ile Ser Ser Arg Gly Arg His Thr Ser Tyr Ala Asp Ser Val
    50                  55                  60


Lys Gly Arg Phe Thr Ile Ser Arg Asp Asn Ser Lys Asn Thr Leu Tyr
65                  70                  75                  80


Leu Gln Met Asn Ser Leu Arg Ala Glu Asp Thr Ala Val Tyr Tyr Cys
                85                  90                  95


Ala Lys Arg Val Pro Gly Arg Gly Arg Ser Phe Asp Tyr Trp Gly Gln
            100                 105                 110


Gly Thr Leu Val Thr Val Ser
        115


<210>  347
<211>  357
<212>  DNA
<213>  Artificial Sequence

<220>
<223>  Nucleotide Sequence of Domain Antibody

<400>  347

gaggtgcagc tgttggagtc tgggggaggc ttggtacagc ctggggggtc cctgcgtctc 60

tcctgtgcag cctccggatt caccttttag agttatcgga tgggttgggt ccgccaggct 120

ccagggaagg gtctagagtg ggtctcaagt atttcgtcga ggggtaggca tacatcttac 180

gcagactccg tgaagggccg gttcaccatc tcccgcgaca attccaagaa cacgctgtat 240

ctgcaaatga acagcctgcg tgccgaggac accgcggtat attactgtgc gaaaagggtt 300

ccgggtcggg ggcgttcttt tgactactgg ggccagggaa ccctggtcac cgtctcg 357

<210> 348
<211> 119
<212> PRT
<213> Artificial Sequence

<220>
<223> Amino Acid Sequence of Domain Antibody

<400> 348

Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Pro Phe Arg Arg Tyr
            20                  25                  30

Arg Met Arg Trp Val Arg Gln Ala Pro Gly Lys Gly Leu Glu Trp Val
        35                  40                  45

Ser Gly Ile Ser Pro Gly Gly Lys His Thr Thr Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Lys Gly Glu Gly Gly Ala Ser Ser Ala Phe Asp Tyr Trp Gly Gln
            100                 105                 110

Gly Thr Leu Val Thr Val Ser
        115

<210>  349
<211>  357
<212>  DNA
<213>  Artificial Sequence

<220>
<223>  Nucleotide Sequence of Domain Antibody

<400>  349
gaggtgcagc tgttggagtc tgggggaggc ttggtacagc ctggggggtc cctgcgtctc      60

tcctgtgcag cctccggatt cccctttcgt cggtatcgga tgaggtgggt ccgccaggct     120

ccagggaagg gtctagagtg ggtctcaggt atttctccgg gtggtaagca tacaacgtac     180

gcagactccg tgaagggccg gttcaccatc tcccgcgaca attccaagaa cacgctgtat     240

ctgcaaatga acagcctgcg tgccgaggac accgcggtat attactgtgc gaaaggtgag     300

gggggggcga gttctgcgtt tgactactgg ggccagggaa ccctggtcac cgtctcg        357


<210>  350
<211>  119
<212>  PRT
<213>  Artificial Sequence

<220>
<223>  Amino Acid Sequence of Domain Antibody

<220>
<221>  MISC_FEATURE
<222>  (30)..(30)
<223>  Stop Codon


<400>  350

Glu Val Gln Leu Leu Glu Ser Gly Gly Gly Leu Val Gln Pro Gly Gly
1               5                   10                  15


Ser Leu Arg Leu Ser Cys Ala Ala Ser Gly Phe Thr Phe Xaa Arg Tyr
            20                  25                  30


Gly Met Val Trp Val Arg Gln Ala Pro Gly Lys Gly Leu Glu Trp Val
        35                  40                  45


Ser Ala Ile Ser Gly Ser Gly Gly Ser Thr Tyr Tyr Ala Asp Ser Val

50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Lys Arg His Ser Ser Glu Ala Arg Gln Phe Asp Tyr Trp Gly Gln
            100                 105                 110

Gly Thr Leu Val Thr Val Ser
        115

<210>  351
<211>  357
<212>  DNA
<213>  Artificial Sequence

<220>
<223>  Nucleotide Sequence of Domain Antibody

<400>  351
gaggtgcagc tgttggagtc tgggggaggc ttggtacagc ctggggggtc cctgcgtctc      60

tcctgtgcag cctccggatt caccttttag cggtatggga tggtttgggt ccgccaggct      120

ccagggaagg gtctagagtg ggtctcagct attagtggta gtggtggtag cacatactac      180

gcagactccg tgaagggccg gttcaccatc tcccgcgaca attccaagaa cacgctgtat      240

ctgcaaatga acagcctgcg tgccgaggac accgcggtat attactgtgc gaaacggcat      300

agttctgagg ctaggcagtt tgactactgg ggccagggaa ccctggtcac cgtctcg      357